(19) Europäisches Patentamt
European Patent Office
Office européen des brevets

(11) EP 3 167 079 B1

(12) # EUROPEAN PATENT SPECIFICATION

(45) Date of publication and mention of the grant of the patent:
**05.08.2020 Bulletin 2020/32**

(21) Application number: **15753461.1**

(22) Date of filing: **07.07.2015**

(51) Int Cl.:
***C12Q 1/6886*** *(2018.01)* ***C12Q 1/70*** *(2006.01)*

(86) International application number:
**PCT/IB2015/055136**

(87) International publication number:
**WO 2016/005905 (14.01.2016 Gazette 2016/02)**

(54) **BROAD RANGE GENE AND GENOTYPE PAPILLOMAVIRUS TRANSCRIPTOME AS A BIOMARKER OF PAPILLOMAVIRUS-ASSOCIATED CANCER STAGES**

PAPILLOMAVIRUSTRANSKRIPTOM MIT BREITEM GEN- UND GENOTYPSPEKTRUM ALS EIN BIOMARKER FÜR KREBS IM ZUSAMMENHANG MIT DEM PAPILLOMAVIRUS

TRANSCRIPTOME DE PAPILLOMAVIRUS À LARGE SPECTRE GÉNÉTIQUE ET GÉNOTYPIQUE UTILE EN TANT QUE BIOMARQUEUR DES STADES DU CANCER ASSOCIÉS AU PAPILLOMAVIRUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.07.2014 PCT/IB2014/062926**

(43) Date of publication of application:
**17.05.2017 Bulletin 2017/20**

(60) Divisional application:
**20183824.0**

(73) Proprietors:
- **Institut Pasteur**
  **75015 Paris (FR)**
- **Ecole Nationale Vétérinaire D'Alfort**
  **94704 Maisons-Alfort (FR)**
- **PathoQuest**
  **75013 Paris (FR)**
- **Assistance Publique - Hôpitaux de Paris**
  **75004 Paris (FR)**

(72) Inventors:
- **ELOIT, Marc**
  **F-75006 Paris (FR)**
- **TORNO, Nicolas**
  **F-75724 Paris Cedex 15 (FR)**
- **DEREGNAUCOURT, Jean**
  **13910 Maillane (FR)**
- **MOUTON, Marion**
  **F-75724 Paris Cedex 15 (FR)**
- **HEARD, Isabelle**
  **F-75003 Paris (FR)**
- **PEROT, Philippe**
  **F-16710 Saint-Yrieix (FR)**

(74) Representative: **Gevers & Orès**
**Immeuble le Palatin 2**
**3 Cours du Triangle**
**CS 80165**
**92939 Paris La Défense Cedex (FR)**

(56) References cited:
**EP-A1- 2 184 368**
**WO-A1-91/08312**
**WO-A1-98/27225**
**WO-A1-98/27225**
**WO-A1-2010/129941**
**WO-A1-2011/131192**
**WO-A2-00/75336**
**WO-A2-00/75336**
**WO-A2-99/29890**
**WO-A2-03/057914**
**WO-A2-2012/116220**
**US-A1- 2006 222 656**
**US-A1- 2006 222 656**
**US-A1- 2006 222 656**

- **DATABASE MEDLINE [Online] US NATIONAL LIBRARY OF MEDICINE (NLM), BETHESDA, MD, US; 16 February 2005 (2005-02-16), CHEN QING-YUN ET AL: "[Detection of integration status of human papillomavirus 16 in cervical precancerous lesions].", XP002733700, Database accession no. NLM15854531 & ZHONGHUA YI XUE ZA ZHI 16 FEB 2005, vol. 85, no. 6, 16 February 2005 (2005-02-16), pages 400-404, ISSN: 0376-2491**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 167 079 B1

- DATABASE MEDLINE [Online] US NATIONAL LIBRARY OF MEDICINE (NLM), BETHESDA, MD, US; September 2003 (2003-09), LUKASZUK KRZYSZTOF ET AL: "[Quantity estimation of the E2/E6 HPV gene products ratio can be a prognostic marker for the stage of the cervical cancer carcinogenesis].", XP002733701, Database accession no. NLM14674126 & GINEKOLOGIA POLSKA SEP 2003, vol. 74, no. 9, September 2003 (2003-09), pages 793-798, ISSN: 0017-0011
- DATABASE MEDLINE [Online] US NATIONAL LIBRARY OF MEDICINE (NLM), BETHESDA, MD, US; August 1990 (1990-08), NAGAI N: "[Molecular biologic study on the carcinogenesis of HPV in uterine cervical cancer and related lesions--analysis of HPV types 16, 18 E6/E7 gene mRNA].", XP002733702, Database accession no. NLM2172419 & NIHON SANKA FUJINKA GAKKAI ZASSHI AUG 1990, vol. 42, no. 8, August 1990 (1990-08), pages 823-833, ISSN: 0300-9165

SEQUENCE LISTING

<110>

INSTITUT PASTEUR

ECOLE NATIONALE VETERINAIRE D'ALFORT PATHOQUEST

ELOIT, Marc

TORNO, Nicolas

DEREGNAUCOURT, Jean

MOUTON, Marion

PEROT, Philippe

HEARD, Isabelle

<120> BROAD RANGE GENE AND GENOTYPE PAPILLOMAVIRUS TRANSCRIPTOME AS A BIOMARKER OF PAPILLOMAVIRUS-ASSOCIATED CANCER STAGES

<130> XRNcc-F226/205WO2

<160> 1816

<170> PatentIn version 3.5

<210> 1
<211> 23
<212> DNA
<213> Artificial Sequence

<220>
<223> Primer Forward E6 alpha1

<400> 1
rgtacwtctg cctcatcaca gcc 23

<210> 2
<211> 21
<212> DNA
<213> Artificial Sequence

<220>
<223> Primer Reverse E6 alpha1

<400> 2
ctctgcamtg sgtacascga c 21

<210> 3
<211> 23
<212> DNA
<213> Artificial Sequence

<220>
<223> Primer Forward E7 alpha1

<400> 3
ggarasrcrc cwacsctaaa gga 23

<210> 4
<211> 21
<212> DNA
<213> Artificial Sequence

<220>
<223> Primer Reverse E7 alpha 1

<400> 4
cacgcrggca cacaawggac a 21

<210> 5
<211> 20
<212> DNA
<213> Artificial Sequence

<220>
<223> Primer Forward L1 alpha1

<400> 5
gcggcctagt gacracaagg 20

<210> 6
<211> 20
<212> DNA
<213> Artificial Sequence

<220>
<223> Primer Reverse L1 alpha1

<400> 6
gcacgyaacc crgcytgcag 20

<210> 7
<211> 21
<212> DNA

<213> Artificial Sequence

<220>
<223> Primer Forward 1 E6 alpha 2

<400> 7
ghghgccmta ygstgcctgt g 21

<210> 8
<211> 19
<212> DNA
<213> Artificial Sequence

<220>
<223> Primer Forward 2 E6 alpha 2

<400> 8
ckccstacgg tgcwtgtgc 19

<210> 9
<211> 21
<212> DNA
<213> Artificial Sequence

<220>
<223> Primer Reverse 1 E6 alpha2

<400> 9
gcggaccgtg catcktrwcc a 21

<210> 10
<211> 20
<212> DNA
<213> Artificial Sequence

<220>
<223> Primer Reverse 2 E6 alpha2

<400> 10
ggctttggcc catgcatcgt 20

<210> 11
<211> 21
<212> DNA
<213> Artificial Sequence

<220>
<223> Primer Reverse 3 E6 alpha2

<400> 11
gtgcatcgtg accagcagta c 21

<210> 12
<211> 23
<212> DNA
<213> Artificial Sequence

<220>

<223> Primer Forward 1 E7 alpha2

<400> 12
ttgrdtcttg caccagaggm cgt 23

<210> 13
<211> 19
<212> DNA
<213> Artificial Sequence

<220>
<223> Primer Forward 2 E7 alpha2

<400> 13
tgcacggtcc gcatcccac 19

<210> 14
<211> 23
<212> DNA
<213> Artificial Sequence

<220>
<223> Primer Forward 3 E7 alpha2

<400> 14
tgtctatggg tgcacaagaa ccc 23

<210> 15
<211> 23
<212> DNA
<213> Artificial Sequence

<220>
<223> Primer Reverse 1 E7 alpha2

<400> 15
cccttatatc tgcktsgctg cws 23

<210> 16
<211> 19
<212> DNA
<213> Artificial Sequence

<220>
<223> Primer Reverse 2 E7 alpha2

<400> 16
gcagcgaggr cacacgasc 19

<210> 17
<211> 19
<212> DNA
<213> Artificial Sequence

<220>
<223> Primer Reverse 3 E7 alpha2

<400> 17

ggaccgtgca tcgtgacca 19

<210> 18
<211> 22
<212> DNA
<213> Artificial Sequence

<220>
<223> Primer Forward L1 alpha2

<400> 18
atggcwytst ggcgcyctag tg 22

<210> 19
<211> 23
<212> DNA
<213> Artificial Sequence

<220>
<223> Primer Reverse 1 L1 alpha2

<400> 19
cctccargct agtrgayggy ggy 23

<210> 20
<211> 23
<212> DNA
<213> Artificial Sequence

<220>
<223> Primer Reverse 2 L1 alpha2

<400> 20
gggracyacy gaacgmcgkc gcg 23

<210> 21
<211> 23
<212> DNA
<213> Artificial Sequence

<220>
<223> Primer Forward 1 E6 alpha3

<400> 21
agtggacrgg raagtgcwgc aac 23

<210> 22
<211> 22
<212> DNA
<213> Artificial Sequence

<220>
<223> Primer Forward 2 E6 alpha3

<400> 22
ytgtgcaaag actgcgasgt gg 22

<210> 23

<211> 22
<212> DNA
<213> Artificial Sequence

<220>
<223> Primer Forward 3 E6 alpha3

<400> 23
actggccatt tggagtmtgc gc 22

<210> 24
<211> 23
<212> DNA
<213> Artificial Sequence

<220>
<223> Primer Reverse 1 E6 alpha3

<400> 24
ggccrygcat gttrcyctac agt 23

<210> 25
<211> 23
<212> DNA
<213> Artificial Sequence

<220>
<223> Primer Reverse 2 E6 alpha3

<400> 25
cacyktcctg tccactbycc wgc 23

<210> 26
<211> 22
<212> DNA
<213> Artificial Sequence

<220>
<223> Primer Reverse 3 E6 alpha3

<400> 26
ccagtgycgt agctcycgyr yc 22

<210> 27
<211> 20
<212> DNA
<213> Artificial Sequence

<220>
<223> Primer Reverse 4 E6 alpha3

<400> 27
ctggccgtgc atrsycctct 20

<210> 28
<211> 23
<212> DNA
<213> Artificial Sequence

<220>
<223> Primer Forward 1 E7 alpha3

<400> 28
vagcamagcw ggccywtagg gtg 23

<210> 29
<211> 22
<212> DNA
<213> Artificial Sequence

<220>
<223> Primer Forward 2 E7 alpha3

<400> 29
kgywgaacrr gcacagcagg cc 22

<210> 30
<211> 23
<212> DNA
<213> Artificial Sequence

<220>
<223> Primer Reverse 1 E7 alpha3

<400> 30
ggccacyrck tccacyataa gct 23

<210> 31
<211> 23
<212> DNA
<213> Artificial Sequence

<220>
<223> Primer Reverse 2 E7 alpha3

<400> 31
cagcygggac acactatrtc cac 23

<210> 32
<211> 20
<212> DNA
<213> Artificial Sequence

<220>
<223> Primer Reverse 3 E7 alpha3

<400> 32
gcgcagcsvg gacacactat 20

<210> 33
<211> 23
<212> DNA
<213> Artificial Sequence

<220>
<223> Primer Forward L1 alpha3

<400> 33
ctwtgtggcg rcmtggtgay ggc 23

<210> 34
<211> 21
<212> DNA
<213> Artificial Sequence

<220>
<223> Primer Reverse 1 L1 alpha3

<400> 34
ggarggaggg ggcamwacmc c 21

<210> 35
<211> 21
<212> DNA
<213> Artificial Sequence

<220>
<223> Primer Reverse 2 L1 alpha3

<220>
<221> misc_feature
<222> (12)..(12)
<223> n is a, c, g, or t

<400> 35
ccctgbgcvc gntgyagcca r 21

<210> 36
<211> 23
<212> DNA
<213> Artificial Sequence

<220>
<223> Primer Forward E6 alpha4

<400> 36
sagtatggty tggagctaga gga 23

<210> 37
<211> 19
<212> DNA
<213> Artificial Sequence

<220>
<223> Primer Reverse E6 alpha4

<400> 37
gtccsgtcca cyggcckgm 19

<210> 38
<211> 19
<212> DNA
<213> Artificial Sequence

<220>

<223> Primer Forward E7 alpha4

<400> 38
mcgmcccagc ctsrmggac 19

<210> 39
<211> 21
<212> DNA
<213> Artificial Sequence

<220>
<223> Primer Reverse E7 alpha4

<400> 39
cctccatrac gctabgcgca g 21

<210> 40
<211> 23
<212> DNA
<213> Artificial Sequence

<220>
<223> Primer Forward 1 L1 alpha4

<400> 40
tggcctaaac gacgtaaacg tgt 23

<210> 41
<211> 23
<212> DNA
<213> Artificial Sequence

<220>
<223> Primer Forward 2 L1 alpha4

<400> 41
ttctttgcag atggctwtgt ggc 23

<210> 42
<211> 21
<212> DNA
<213> Artificial Sequence

<220>
<223> Primer Reverse 1 L1 alpha4

<400> 42
gcggygcgyt tkcgagacac r 21

<210> 43
<211> 23
<212> DNA
<213> Artificial Sequence

<220>
<223> Primer Reverse 2 L1 alpha4

<400> 43

ccccgctgca ryaaraactt gcg 23

<210> 44
<211> 21
<212> DNA
<213> Artificial Sequence

<220>
<223> Primer Forward 1 E6 alpha5

<400> 44
grgaaagacc acgaacgctg c 21

<210> 45
<211> 23
<212> DNA
<213> Artificial Sequence

<220>
<223> Primer Forward 2 E6 alpha5

<400> 45
aatagcaggg yastggaaag ggt 23

<210> 46
<211> 23
<212> DNA
<213> Artificial Sequence

<220>
<223> Primer Reverse 1 E6 alpha5

<400> 46
gcaattwgcr caytgycccg tcc 23

<210> 47
<211> 23
<212> DNA
<213> Artificial Sequence

<220>
<223> Primer Reverse 2 E6 alpha5

<400> 47
ttgtgtttct gtttggcgcc ttg 23

<210> 48
<211> 21
<212> DNA
<213> Artificial Sequence

<220>
<223> Primer Reverse 3 E6 alpha5

<400> 48
gccttggtct ccagcagttt g 21

<210> 49

<211> 22
<212> DNA
<213> Artificial Sequence

<220>
<223> Primer Forwward 1 E7 alpha5

<400> 49
ytagatytgg tgccgcaacc cg 22

<210> 50
<211> 23
<212> DNA
<213> Artificial Sequence

<220>
<223> Primer Forward 2 E7 alpha5

<400> 50
mgccatgcgt ggtaatgtac cac 23

<210> 51
<211> 22
<212> DNA
<213> Artificial Sequence

<220>
<223> Primer Reverse 1 E7 alpha5

<400> 51
ctccascrct cgracgttct gt 22

<210> 52
<211> 21
<212> DNA
<213> Artificial Sequence

<220>
<223> Primer Reverse 2 E7 alpha5

<400> 52
cacgggcama ccaggcttag k 21

<210> 53
<211> 23
<212> DNA
<213> Artificial Sequence

<220>
<223> Primer Forward 1 L1 alpha5

<400> 53
kcagatggcy ttgyggcgta cta 23

<210> 54
<211> 23
<212> DNA
<213> Artificial Sequence

<220>
<223> Primer Forward 2 L1 alpha5

<400> 54
tggcyttgyg gcgtactagt gac 23

<210> 55
<211> 23
<212> DNA
<213> Artificial Sequence

<220>
<223> Primer Forward 3 L1 alpha5

<400> 55
tgtatttrcc acctgcaccw gtg 23

<210> 56
<211> 23
<212> DNA
<213> Artificial Sequence

<220>
<223> Primer Reverse 1 L1 alpha5

<400> 56
ggggcrtyrc gytgacakgt agt 23

<210> 57
<211> 22
<212> DNA
<213> Artificial Sequence

<220>
<223> Primer Reverse 2 L1 alpha5

<400> 57
ggcmggsckt ttaaggcctg gt 22

<210> 58
<211> 22
<212> DNA
<213> Artificial Sequence

<220>
<223> Primer Forward 1 E6 alpha6

<400> 58
garcghccac gwashbtgca cc 22

<210> 59
<211> 23
<212> DNA
<213> Artificial Sequence

<220>
<223> Primer Forward 2 E6 alpha6

<400> 59
aatacagrmg agcgmccacg tac 23

<210> 60
<211> 23
<212> DNA
<213> Artificial Sequence

<220>
<223> Primer Forward 3 E6 alpha6

<400> 60
rcaatmcaca ggaacgtcca cga 23

<210> 61
<211> 23
<212> DNA
<213> Artificial Sequence

<220>
<223> Primer Reverse 1 E6 alpha6

<400> 61
cctctggtgt caacggmtgt tga 23

<210> 62
<211> 23
<212> DNA
<213> Artificial Sequence

<220>
<223> Primer Reverse 2 E6 alpha6

<400> 62
tctccarcac yscaaacatg acc 23

<210> 63
<211> 23
<212> DNA
<213> Artificial Sequence

<220>
<223> Primer Forward 1 E7 alpha6

<400> 63
gracagctca gaggawgagg atg 23

<210> 64
<211> 21
<212> DNA
<213> Artificial Sequence

<220>
<223> Primer Forward 2 E7 alpha6

<400> 64
gctcagagga wgaggatgag g 21

<210> 65
<211> 23
<212> DNA
<213> Artificial Sequence

<220>
<223> Primer Forward 3 E7 alpha6

<400> 65
ytrcwgragc rgccacagca agc 23

<210> 66
<211> 21
<212> DNA
<213> Artificial Sequence

<220>
<223> Primer Forward 4 E7 alpha6

<400> 66
gragcrgcca cagcaagcta g 21

<210> 67
<211> 23
<212> DNA
<213> Artificial Sequence

<220>
<223> Primer Forward 5 E7 alpha6

<400> 67
gaacagctca gaggawgagg atg 23

<210> 68
<211> 23
<212> DNA
<213> Artificial Sequence

<220>
<223> Primer Forward 6 E7 alpha6

<400> 68
artagaccat ttgcwggagc ggc 23

<210> 69
<211> 19
<212> DNA
<213> Artificial Sequence

<220>
<223> Primer Reverse 1 E7 alpha6

<400> 69
gccttgttgc rcasagggg 19

<210> 70
<211> 20
<212> DNA

<213> Artificial Sequence

<220>
<223> Primer Reverse 2 E7 alpha6

<400> 70
cgcagagtgg gcacgttact 20

<210> 71
<211> 20
<212> DNA
<213> Artificial Sequence

<220>
<223> Primer Forward L1 alpha6

<400> 71
ttgcagatgg cgrygtggcg 20

<210> 72
<211> 21
<212> DNA
<213> Artificial Sequence

<220>
<223> Primer Reverse L1 alpha6

<400> 72
cacctaaagg ytgdccdcgg c 21

<210> 73
<211> 22
<212> DNA
<213> Artificial Sequence

<220>
<223> Primer Forward 1 E6 alpha7

<400> 73
tasaggacag tgycgmcrst gc 22

<210> 74
<211> 23
<212> DNA
<213> Artificial Sequence

<220>
<223> Primer Forward 2 E6 alpha7

<400> 74
tcmcaaycct gmrgaacggc cat 23

<210> 75
<211> 20
<212> DNA
<213> Artificial Sequence

<220>

<223> Primer Forward 3 E6 alpha7

<400> 75
asaggacagt gtcgysggtg 20

<210> 76
<211> 23
<212> DNA
<213> Artificial Sequence

<220>
<223> Primer Forward 4 E6 alpha7

<400> 76
tgccagaaac crttgaaycc agc 23

<210> 77
<211> 22
<212> DNA
<213> Artificial Sequence

<220>
<223> Primer Reverse 1 E6 alpha7

<400> 77
gtctgcggtc ctcycgbttd st 22

<210> 78
<211> 22
<212> DNA
<213> Artificial Sequence

<220>
<223> Primer Reverse 2 E6 alpha7

<400> 78
ctgscctckr tastgcccag ct 22

<210> 79
<211> 21
<212> DNA
<213> Artificial Sequence

<220>
<223> Primer Reverse 3 E6 alpha7

<400> 79
caccagtgtt tcactacgcg c 21

<210> 80
<211> 21
<212> DNA
<213> Artificial Sequence

<220>
<223> Primer Reverse 4 E6 alpha7

<400> 80

gccttgctgt tcttgtgcac g 21

<210> 81
<211> 23
<212> DNA
<213> Artificial Sequence

<220>
<223> Primer Reverse 5 E6 alpha7

<400> 81
gtctggaaag cctttcttgc cgt 23

<210> 82
<211> 23
<212> DNA
<213> Artificial Sequence

<220>
<223> Primer Forward 1 E7 alpha7

<400> 82
gacgrgmhga acmacarcgt cac 23

<210> 83
<211> 23
<212> DNA
<213> Artificial Sequence

<220>
<223> Primer Forward 2 E7 alpha7

<400> 83
gacgrgmhga acmacagcgt cac 23

<210> 84
<211> 23
<212> DNA
<213> Artificial Sequence

<220>
<223> Primer Forward 3 E7 alpha7

<400> 84
arcaccytgt cctttgtgtg tcc 23

<210> 85
<211> 23
<212> DNA
<213> Artificial Sequence

<220>
<223> Primer Reverse 1 E7 alpha7

<400> 85
gtgwstccat aaacagcwgc wgt 23

<210> 86

<211> 23
<212> DNA
<213> Artificial Sequence

<220>
<223> Primer Reverse 2 E7 alpha7

<400> 86
cacaccamgg acacacaaag gac 23

<210> 87
<211> 23
<212> DNA
<213> Artificial Sequence

<220>
<223> Primer Forward 1 L1 alpha7

<400> 87
gcgbtctagy gacarcahgg tgt 23

<210> 88
<211> 23
<212> DNA
<213> Artificial Sequence

<220>
<223> Primer Forward 2 L1 alpha7

<400> 88
hcctgctatt ggkgarcayt ggg 23

<210> 89
<211> 23
<212> DNA
<213> Artificial Sequence

<220>
<223> Primer Reverse 1 L1 alpha7

<400> 89
ccagtgytcy ccmatrgcrg gwa 23

<210> 90
<211> 23
<212> DNA
<213> Artificial Sequence

<220>
<223> Primer Reverse 2 L1 alpha7

<220>
<221> misc_feature
<222> (17)..(17)
<223> n is a, c, g, or t

<400> 90
tagasccact dggwganggr gaa 23

<210> 91
<211> 22
<212> DNA
<213> Artificial Sequence

<220>
<223> Primer Forward E6 alpha8

<400> 91
watgwctgca cgkwgckgct cc 22

<210> 92
<211> 23
<212> DNA
<213> Artificial Sequence

<220>
<223> Primer Reverse 1 E6 alpha8

<400> 92
gtaggcarta tccyttccac rcg 23

<210> 93
<211> 19
<212> DNA
<213> Artificial Sequence

<220>
<223> Primer Reverse 2 E6 alpha8

<400> 93
ctccgagcgt tggcctttc 19

<210> 94
<211> 20
<212> DNA
<213> Artificial Sequence

<220>
<223> Primer Forward E7 alpha8

<400> 94
gcgtgagcaa yccacgcaac 20

<210> 95
<211> 23
<212> DNA
<213> Artificial Sequence

<220>
<223> Primer Reverse 1 E7 alpha8

<400> 95
cagccatkgy agtcacacmg ctg 23

<210> 96
<211> 23
<212> DNA

<213> Artificial Sequence

<220>
<223> Primer Reverse 2 E7 alpha8

<400> 96
tgccattgtt gtcackctgt agc 23

<210> 97
<211> 23
<212> DNA
<213> Artificial Sequence

<220>
<223> Primer Forward L1 alpha8

<220>
<221> misc_feature
<222> (12)..(12)
<223> n is a, c, g, or t

<400> 97
ccycchatkg gngaatattg ggg 23

<210> 98
<211> 19
<212> DNA
<213> Artificial Sequence

<220>
<223> Primer Reverse 1 L1 alpha8

<400> 98
ggaggatggt gcwgmacgc 19

<210> 99
<211> 23
<212> DNA
<213> Artificial Sequence

<220>
<223> Primer Reverse 2 L1 alpha8

<400> 99
gggtgactgr cyyagaagag gaa 23

<210> 100
<211> 23
<212> DNA
<213> Artificial Sequence

<220>
<223> Primer Forward 1 E6 alpha9

<400> 100
agtrmaratg cctccacgyc tgc 23

<210> 101

<211> 19
<212> DNA
<213> Artificial Sequence

<220>
<223> Primer Forward 2 E6 alpha9

<400> 101
ctgcacagga ccagatggc 19

<210> 102
<211> 23
<212> DNA
<213> Artificial Sequence

<220>
<223> Primer Reverse 1 E6 alpha9

<400> 102
tccatgcatg wtgwccagca rtg 23

<210> 103
<211> 22
<212> DNA
<213> Artificial Sequence

<220>
<223> Primer Reverse 2 E6 alpha9

<400> 103
gcagcgmccy ttccaggtrt ck 22

<210> 104
<211> 23
<212> DNA
<213> Artificial Sequence

<220>
<223> Primer Reverse 3 E6 alpha9

<400> 104
ggcatttcgc ccaccattgt tat 23

<210> 105
<211> 23
<212> DNA
<213> Artificial Sequence

<220>
<223> Primer Forward 1 E7 alpha9

<400> 105
gcytacactg ctggacaaca tgc 23

<210> 106
<211> 23
<212> DNA
<213> Artificial Sequence

<220>
<223> Primer Forward 2 E7 alpha9

<400> 106
agacagctca gaagabgagg tgg 23

<210> 107
<211> 23
<212> DNA
<213> Artificial Sequence

<220>
<223> Primer Forward 3 E7 alpha9

<400> 107
aacaatggtg ggcgaaatgc cag 23

<210> 108
<211> 23
<212> DNA
<213> Artificial Sequence

<220>
<223> Primer Reverse 1 E7 alpha9

<400> 108
cgtccgccat csttgttatg kyt 23

<210> 109
<211> 22
<212> DNA
<213> Artificial Sequence

<220>
<223> Primer Reverse 2 E7 alpha9

<400> 109
cctgtrcact scacmacmag cc 22

<210> 110
<211> 20
<212> DNA
<213> Artificial Sequence

<220>
<223> Primer Reverse 3 E7 alpha9

<400> 110
ctgtcgctgt agggtgcaca 20

<210> 111
<211> 23
<212> DNA
<213> Artificial Sequence

<220>
<223> Primer Forward 1 L1 alpha9

<400> 111
atgtgcctcc tccyrmcccw gta 23

<210> 112
<211> 22
<212> DNA
<213> Artificial Sequence

<220>
<223> Primer Forward 2 L1 alpha9

<400> 112
agatggctgt ctggttacca gc 22

<210> 113
<211> 23
<212> DNA
<213> Artificial Sequence

<220>
<223> Primer Reverse 1 L1 alpha9

<400> 113
ccatawggrt cygcagccat ttg 23

<210> 114
<211> 19
<212> DNA
<213> Artificial Sequence

<220>
<223> Primer Reverse 2 L1 alpha9

<400> 114
gccttacgcc tgcgcttgg 19

<210> 115
<211> 23
<212> DNA
<213> Artificial Sequence

<220>
<223> Primer Forward 1 E6 alpha10

<400> 115
ccsarstgta awcatgcrtg gag 23

<210> 116
<211> 23
<212> DNA
<213> Artificial Sequence

<220>
<223> Primer Forward 2 E6 alpha10

<400> 116
mcgsamcctg cacgaattgt gtg 23

<210> 117
<211> 23
<212> DNA
<213> Artificial Sequence

<220>
<223> Primer Forward 3 E6 alpha10

<400> 117
cargacrcwg aggaraaacc acg 23

<210> 118
<211> 21
<212> DNA
<213> Artificial Sequence

<220>
<223> Primer Reverse 1 E6 alpha10

<400> 118
ccaacacwct gaacascgyc c 21

<210> 119
<211> 23
<212> DNA
<213> Artificial Sequence

<220>
<223> Primer Reverse 2 E6 alpha10

<400> 119
ccatgcatga ttacasctsg gtt 23

<210> 120
<211> 21
<212> DNA
<213> Artificial Sequence

<220>
<223> Primer Reverse 3 E6 alpha10

<400> 120
gtcgggryct ccaacacrcy g 21

<210> 121
<211> 23
<212> DNA
<213> Artificial Sequence

<220>
<223> Primer Reverse 4 E6 alpha10

<400> 121
ctccacgcat gtttacactt ggg 23

<210> 122
<211> 23
<212> DNA

<213> Artificial Sequence

<220>
<223> Primer Forward E7 alpha10

<400> 122
gcwcaytwgg aathgtgtgc ccc 23

<210> 123
<211> 23
<212> DNA
<213> Artificial Sequence

<220>
<223> Primer Forward 2 E7 alpha10

<400> 123
cstgtaamaa cgccatgaga gga 23

<210> 124
<211> 22
<212> DNA
<213> Artificial Sequence

<220>
<223> Primer Forward 3 E7 alpha10

<400> 124
cgccatgaga ggamacaasc ca 22

<210> 125
<211> 23
<212> DNA
<213> Artificial Sequence

<220>
<223> Primer Reverse 1 E7 alpha10

<400> 125
ggcacacdat tccwartgwg ccc 23

<210> 126
<211> 23
<212> DNA
<213> Artificial Sequence

<220>
<223> Primer Reverse 2 E7 alpha10

<400> 126
ggttcgtasg tcrsttgytg tac 23

<210> 127
<211> 23
<212> DNA
<213> Artificial Sequence

<220>

<223> Primer Reverse 3 E7 alpha10

<400> 127
gtgcacagsy gggrcacacw ayt 23

<210> 128
<211> 22
<212> DNA
<213> Artificial Sequence

<220>
<223> Primer Forward 1 L1 alpha10

<400> 128
gargccacwg tstacytgcc tc 22

<210> 129
<211> 20
<212> DNA
<213> Artificial Sequence

<220>
<223> Primer Forward 2 L1 alpha10

<400> 129
acagatgtct ctgtggcggc 20

<210> 130
<211> 23
<212> DNA
<213> Artificial Sequence

<220>
<223> Primer Reverse 1 L1 alpha10

<220>
<221> misc_feature
<222> (6) .. (6)
<223> n is a, c, g, or t

<400> 130
ggatgnccac twayrcchac dcc 23

<210> 131
<211> 23
<212> DNA
<213> Artificial Sequence

<220>
<223> Primer Reverse 2 L1 alpha10

<400> 131
gaggwwacca tagarccact rgg 23

<210> 132
<211> 23
<212> DNA
<213> Artificial Sequence

<220>
<223> Primer Reverse 3 L1 alpha10

<400> 132
gtgcacgytg tagccaataw ggc 23

<210> 133
<211> 23
<212> DNA
<213> Artificial Sequence

<220>
<223> Primer Reverse 4 L1 alpha10

<400> 133
tcctgtaaac trgcagaygg agg 23

<210> 134
<211> 23
<212> DNA
<213> Artificial Sequence

<220>
<223> Primer Reverse 5 L1 alpha10

<400> 134
ggccytgtgc wcgttgyaac caa 23

<210> 135
<211> 23
<212> DNA
<213> Artificial Sequence

<220>
<223> Primer Forward E6 alpha11

<400> 135
gaacgrccat acaagctacm agc 23

<210> 136
<211> 20
<212> DNA
<213> Artificial Sequence

<220>
<223> Primer Reverse E6 alpha11

<400> 136
gcagatggtc tccagcacyg 20

<210> 137
<211> 23
<212> DNA
<213> Artificial Sequence

<220>
<223> Primer Forward E7 alpha11

<400> 137
wattgtgtgc cccaactgtt cca 23

<210> 138
<211> 21
<212> DNA
<213> Artificial Sequence

<220>
<223> Primer Reverse E7 alpha11

<400> 138
ctggaacagt tggggcacac a 21

<210> 139
<211> 22
<212> DNA
<213> Artificial Sequence

<220>
<223> Primer Forward L1 alpha11

<400> 139
agttctatct tcctccccag cc 22

<210> 140
<211> 20
<212> DNA
<213> Artificial Sequence

<220>
<223> Primer Reverse L1 alpha11

<400> 140
ggacgkgcac gcataccwag 20

<210> 141
<211> 22
<212> DNA
<213> Artificial Sequence

<220>
<223> Primer Forward E6 alpha13

<400> 141
tgtctgctac tgaaccccac ac 22

<210> 142
<211> 22
<212> DNA
<213> Artificial Sequence

<220>
<223> Primer Reverse E6 alpha13

<400> 142
ggcttccagc aatgtagaca cc 22

<210> 143
<211> 22
<212> DNA
<213> Artificial Sequence

<220>
<223> Primer Forward E7 alpha13

<400> 143
gtttgacctg tactgcaggg ag 22

<210> 144
<211> 21
<212> DNA
<213> Artificial Sequence

<220>
<223> Primer Reverse E7 alpha13

<400> 144
gtgaagcaca ggtgggacac a 21

<210> 145
<211> 23
<212> DNA
<213> Artificial Sequence

<220>
<223> Primer Forward L1 alpha13

<400> 145
aaagtatacc tgcctcctac ccc 23

<210> 146
<211> 19
<212> DNA
<213> Artificial Sequence

<220>
<223> Primer Reverse L1 alpha13

<400> 146
gcacgcttgc gcgctgtac 19

<210> 147
<211> 22
<212> DNA
<213> Artificial Sequence

<220>
<223> Primer Forward E6 alpha14

<400> 147
taysamstgg acctgcagga cc 22

<210> 148
<211> 23
<212> DNA

<213> Artificial Sequence

<220>
<223> Primer Reverse E6 alpha14

<400> 148
ggccwygcat grtktccaac act 23

<210> 149
<211> 23
<212> DNA
<213> Artificial Sequence

<220>
<223> Primer Forward E7 alpha14

<400> 149
caattwgcca gctcagamga gga 23

<210> 150
<211> 23
<212> DNA
<213> Artificial Sequence

<220>
<223> Primer Reverse E7 alpha14

<400> 150
ccaccacmag cctwactgya crv 23

<210> 151
<211> 21
<212> DNA
<213> Artificial Sequence

<220>
<223> Primer Forward L1 alpha14

<400> 151
argtatacct gcctccygcc c 21

<210> 152
<211> 20
<212> DNA
<213> Artificial Sequence

<220>
<223> Primer Reverse L1 alpha14

<400> 152
cctgtgcwcg ttgyagccag 20

<210> 153
<211> 19
<212> DNA
<213> Artificial Sequence

<220>

<223> Primer HPV16-early

<400> 153
cagcggacgt attaatagg 19

<210> 154
<211> 19
<212> DNA
<213> Artificial Sequence

<220>
<223> Primer HPV16-late

<400> 154
tcatattcct ccccatgtc 19

<210> 155
<211> 18
<212> DNA
<213> Artificial Sequence

<220>
<223> Primer HPV18-early-pop1

<400> 155
aggggacgtt attaccac 18

<210> 156
<211> 19
<212> DNA
<213> Artificial Sequence

<220>
<223> Primer HPV18-early-pop2

<400> 156
caggggacgt tattatcac 19

<210> 157
<211> 20
<212> DNA
<213> Artificial Sequence

<220>
<223> Primer HPV18-late-pop1

<400> 157
atattcctca acatgtctgc 20

<210> 158
<211> 21
<212> DNA
<213> Artificial Sequence

<220>
<223> Primer HPV18-late-pop2

<400> 158

catattcttc aacatgtctg c 21

<210> 159
<211> 22
<212> DNA
<213> Artificial Sequence

<220>
<223> Primer

<400> 159
ccaggtcatc accattggca at 22

<210> 160
<211> 21
<212> DNA
<213> Artificial Sequence

<220>
<223> Primer

<400> 160
cgtacaggtc tttgcggatg t 21

<210> 161
<211> 20
<212> DNA
<213> Artificial Sequence

<220>
<223> Primer

<400> 161
ccatgagcga cgtggctatt 20

<210> 162
<211> 20
<212> DNA
<213> Artificial Sequence

<220>
<223> Primer

<400> 162
ctcacgttgg tccacatcct 20

<210> 163
<211> 18
<212> DNA
<213> Artificial Sequence

<220>
<223> Primer

<400> 163
ctgtgctcgc gctactct 18

<210> 164

<211> 26
<212> DNA
<213> Artificial Sequence

<220>
<223> Primer

<400> 164
caacttcaat gtcggatgga tgaaac 26

<210> 165
<211> 23
<212> DNA
<213> Artificial Sequence

<220>
<223> Primer

<400> 165
gtggatgact gagtacctga acc 23

<210> 166
<211> 21
<212> DNA
<213> Artificial Sequence

<220>
<223> Primer

<400> 166
ggccaaactg agcagagtct t 21

<210> 167
<211> 21
<212> DNA
<213> Artificial Sequence

<220>
<223> Primer

<400> 167
cgggactcga gtgatgattg g 21

<210> 168
<211> 20
<212> DNA
<213> Artificial Sequence

<220>
<223> Primer

<400> 168
ctgaggtgta ggtgctgtca 20

<210> 169
<211> 24
<212> DNA
<213> Artificial Sequence

<220>
<223> Primer

<400> 169
ctcctgaaaa gagagtggaa gtgt 24

<210> 170
<211> 22
<212> DNA
<213> Artificial Sequence

<220>
<223> Primer

<400> 170
ccggattaat ctccagccag tt 22

<210> 171
<211> 21
<212> DNA
<213> Artificial Sequence

<220>
<223> Primer

<400> 171
aacgcaccga atagttacgg t 21

<210> 172
<211> 17
<212> DNA
<213> Artificial Sequence

<220>
<223> Primer

<400> 172
acgggtcggg tgagagt 17

<210> 173
<211> 19
<212> DNA
<213> Artificial Sequence

<220>
<223> Primer

<400> 173
cggatcccaa cggagtcaa 19

<210> 174
<211> 17
<212> DNA
<213> Artificial Sequence

<220>
<223> Primer

<400> 174
accggtcggg tgagagt 17

<210> 175
<211> 24
<212> DNA
<213> Artificial Sequence

<220>
<223> Primer

<400> 175
tcttccagaa cctgcaagta atcc 24

<210> 176
<211> 21
<212> DNA
<213> Artificial Sequence

<220>
<223> Primer

<400> 176
ggtgggtgtt atggtggatg a 21

<210> 177
<211> 25
<212> DNA
<213> Artificial Sequence

<220>
<223> Primer

<400> 177
aggagaatcc gaagggaaag gaata 25

<210> 178
<211> 20
<212> DNA
<213> Artificial Sequence

<220>
<223> Primer

<400> 178
tccttcagca ggttggcaat 20

<210> 179
<211> 19
<212> DNA
<213> Artificial Sequence

<220>
<223> Primer

<400> 179
agtccactgg cgtcttcac 19

<210> 180
<211> 26
<212> DNA
<213> Artificial Sequence

<220>
<223> Primer

<400> 180
tgatcttgag gctgttgtca tacttc 26

<210> 181
<211> 21
<212> DNA
<213> Artificial Sequence

<220>
<223> Primer

<400> 181
gcgagtatgg agcagaaacg a 21

<210> 182
<211> 24
<212> DNA
<213> Artificial Sequence

<220>
<223> Primer

<400> 182
aattccaaat gagctctcca acca 24

<210> 183
<211> 22
<212> DNA
<213> Artificial Sequence

<220>
<223> Primer

<400> 183
cggaatataa gctggtggtg gt 22

<210> 184
<211> 20
<212> DNA
<213> Artificial Sequence

<220>
<223> Primer

<400> 184
gcacgtctcc ccatcaatga 20

<210> 185
<211> 21
<212> DNA

<213> Artificial Sequence

<220>
<223> Primer

<400> 185
gtgcaatgag ggaccagtac a 21

<210> 186
<211> 28
<212> DNA
<213> Artificial Sequence

<220>
<223> Primer

<400> 186
ctactaggac cataggtaca tcttcaga 28

<210> 187
<211> 20
<212> DNA
<213> Artificial Sequence

<220>
<223> Primer

<400> 187
gatgagctga ccctgaccaa 20

<210> 188
<211> 24
<212> DNA
<213> Artificial Sequence

<220>
<223> Primer

<400> 188
ggcagcattc atttccacat tcac 24

<210> 189
<211> 19
<212> DNA
<213> Artificial Sequence

<220>
<223> Primer

<400> 189
aggagctggc ctacctgaa 19

<210> 190
<211> 23
<212> DNA
<213> Artificial Sequence

<220>

<223> Primer

<400> 190
cttctcatac tggtcacgca tct 23

<210> 191
<211> 21
<212> DNA
<213> Artificial Sequence

<220>
<223> Primer

<400> 191
aacactgagc tggaggtgaa g 21

<210> 192
<211> 22
<212> DNA
<213> Artificial Sequence

<220>
<223> Primer

<400> 192
ctgtagcagg atgttggcat tg 22

<210> 193
<211> 28
<212> DNA
<213> Artificial Sequence

<220>
<223> Primer

<400> 193
tgtgtgcatt ccctatcaaa tatgtcaa 28

<210> 194
<211> 19
<212> DNA
<213> Artificial Sequence

<220>
<223> Primer

<400> 194
gcgcttcaca gcctgatga 19

<210> 195
<211> 23
<212> DNA
<213> Artificial Sequence

<220>
<223> Primer

<400> 195

cgtcgtgtct caagatctag ctt 23

<210> 196
<211> 22
<212> DNA
<213> Artificial Sequence

<220>
<223> Primer

<400> 196
tgagtcatct gcggtactgt ct 22

<210> 197
<211> 22
<212> DNA
<213> Artificial Sequence

<220>
<223> Primer

<400> 197
gcttctctga aaggctctcc tt 22

<210> 198
<211> 19
<212> DNA
<213> Artificial Sequence

<220>
<223> Primer

<400> 198
aaatacggct gcaccgagt 19

<210> 199
<211> 19
<212> DNA
<213> Artificial Sequence

<220>
<223> Primer

<400> 199
ccgacgcaca aggtgtctt 19

<210> 200
<211> 20
<212> DNA
<213> Artificial Sequence

<220>
<223> Primer

<400> 200
gtcggcgtgt gagttgatga 20

<210> 201

<211> 24
<212> DNA
<213> Artificial Sequence

<220>
<223> Primer

<400> 201
gacggagtga aattttctgc aagt 24

<210> 202
<211> 24
<212> DNA
<213> Artificial Sequence

<220>
<223> Primer

<400> 202
gaagttcagg tacctcagtg caaa 24

<210> 203
<211> 23
<212> DNA
<213> Artificial Sequence

<220>
<223> Primer

<400> 203
agcgtgcaga taatgacaag gaa 23

<210> 204
<211> 22
<212> DNA
<213> Artificial Sequence

<220>
<223> Primer

<400> 204
gatttgacgg ctcctctact gt 22

<210> 205
<211> 21
<212> DNA
<213> Artificial Sequence

<220>
<223> Primer

<400> 205
cggtcttcat gcagagactg a 21

<210> 206
<211> 28
<212> DNA
<213> Artificial Sequence

<220>
<223> Primer

<400> 206
gtgaaatata gatgttccct ccaggaat 28

<210> 207
<211> 22
<212> DNA
<213> Artificial Sequence

<220>
<223> Primer

<400> 207
gacggattac accttcccac tt 22

<210> 208
<211> 24
<212> DNA
<213> Artificial Sequence

<220>
<223> Primer

<400> 208
gactcttcct tggcttcaac ctta 24

<210> 209
<211> 20
<212> DNA
<213> Artificial Sequence

<220>
<223> Primer

<400> 209
cgatgggctc agctttcaga 20

<210> 210
<211> 21
<212> DNA
<213> Artificial Sequence

<220>
<223> Primer

<400> 210
acaaaacctc gtccacggaa t 21

<210> 211
<211> 20
<212> DNA
<213> Artificial Sequence

<220>
<223> Primer

<400> 211
tcctgcgttt ggtggatgat 20

<210> 212
<211> 24
<212> DNA
<213> Artificial Sequence

<220>
<223> Primer

<400> 212
cctcgtcttc tacagggaag ttca 24

<210> 213
<211> 20
<212> DNA
<213> Artificial Sequence

<220>
<223> Primer

<400> 213
tgggtggtcc tgcaaaatcc 20

<210> 214
<211> 27
<212> DNA
<213> Artificial Sequence

<220>
<223> Primer

<400> 214
acatattgat ttggagccag ttcttca 27

<210> 215
<211> 20
<212> DNA
<213> Artificial Sequence

<220>
<223> Primer

<400> 215
ctggcccctg tcatcttctg 20

<210> 216
<211> 21
<212> DNA
<213> Artificial Sequence

<220>
<223> Primer

<400> 216
cttggccagt tggcaaaaca t 21

<210> 217
<211> 19
<212> DNA
<213> Artificial Sequence

<220>
<223> Primer

<400> 217
ctggaactgt cccactgct 19

<210> 218
<211> 23
<212> DNA
<213> Artificial Sequence

<220>
<223> Primer

<400> 218
caggattcga tggaaccttc tga 23

<210> 219
<211> 25
<212> DNA
<213> Artificial Sequence

<220>
<223> Primer

<400> 219
cacagagctg caaacaacta tacat 25

<210> 220
<211> 27
<212> DNA
<213> Artificial Sequence

<220>
<223> Primer

<400> 220
cacatacagc atatggattc ccatctc 27

<210> 221
<211> 28
<212> DNA
<213> Artificial Sequence

<220>
<223> Primer

<400> 221
ggaacaacat tagaacagca atacaaca 28

<210> 222
<211> 26
<212> DNA

<213> Artificial Sequence

<220>
<223> Primer

<400> 222
tgtccagatg tctttgcttt tcttca 26

<210> 223
<211> 17
<212> DNA
<213> Artificial Sequence

<220>
<223> Primer

<400> 223
cggtggaccg gtcgatg 17

<210> 224
<211> 24
<212> DNA
<213> Artificial Sequence

<220>
<223> Primer

<400> 224
tcagttgtct ctggttgcaa atct 24

<210> 225
<211> 26
<212> DNA
<213> Artificial Sequence

<220>
<223> Primer

<400> 225
ctcagaggag gaggatgaaa tagatg 26

<210> 226
<211> 26
<212> DNA
<213> Artificial Sequence

<220>
<223> Primer

<400> 226
ccattaacag gtcttccaaa gtacga 26

<210> 227
<211> 21
<212> DNA
<213> Artificial Sequence

<220>

<223> Primer

<400> 227
ggaattgtgt gccccatctg t 21

<210> 228
<211> 22
<212> DNA
<213> Artificial Sequence

<220>
<223> Primer

<400> 228
catccattac atcccgtacc ct 22

<210> 229
<211> 26
<212> DNA
<213> Artificial Sequence

<220>
<223> Primer

<400> 229
ggttttatgt agaggctgta gtggaa 26

<210> 230
<211> 22
<212> DNA
<213> Artificial Sequence

<220>
<223> Primer

<400> 230
tgtgcagtaa acaacgcatg tg 22

<210> 231
<211> 22
<212> DNA
<213> Artificial Sequence

<220>
<223> Primer

<400> 231
gcgggtatgg caatactgaa gt 22

<210> 232
<211> 26
<212> DNA
<213> Artificial Sequence

<220>
<223> Primer

<400> 232

tggtgtttgg catatagtgt gtcttt 26

<210> 233
<211> 20
<212> DNA
<213> Artificial Sequence

<220>
<223> Primer

<400> 233
atcaacgtgt tgcgattggt 20

<210> 234
<211> 28
<212> DNA
<213> Artificial Sequence

<220>
<223> Primer

<400> 234
ctaatagtaa cacaaccatt ccccatga 28

<210> 235
<211> 27
<212> DNA
<213> Artificial Sequence

<220>
<223> Primer

<400> 235
gaggtgattg gaagcaaatt gttatgt 27

<210> 236
<211> 28
<212> DNA
<213> Artificial Sequence

<220>
<223> Primer

<400> 236
cagacccttg cagaaatttc attaaact 28

<210> 237
<211> 27
<212> DNA
<213> Artificial Sequence

<220>
<223> Primer

<400> 237
ggatgtaaag catagaccat tggtaca 27

<210> 238

<211> 28
<212> DNA
<213> Artificial Sequence

<220>
<223> Primer

<400> 238
gttttcgtca aatggaaact cattagga 28

<210> 239
<211> 28
<212> DNA
<213> Artificial Sequence

<220>
<223> Primer

<400> 239
cggaaatcca gtgtatgagc ttaatgat 28

<210> 240
<211> 26
<212> DNA
<213> Artificial Sequence

<220>
<223> Primer

<400> 240
tgacacacat ttaaacgttg gcaaag 26

<210> 241
<211> 19
<212> DNA
<213> Artificial Sequence

<220>
<223> Primer

<400> 241
catgcgggtg gtcaggtaa 19

<210> 242
<211> 19
<212> DNA
<213> Artificial Sequence

<220>
<223> Primer

<400> 242
aaggcgacgg ctttggtat 19

<210> 243
<211> 23
<212> DNA
<213> Artificial Sequence

<220>
<223> Primer

<400> 243
gctcacacaa aggacggatt aac 23

<210> 244
<211> 22
<212> DNA
<213> Artificial Sequence

<220>
<223> Primer

<400> 244
ccaatgccat gtagacgaca ct 22

<210> 245
<211> 22
<212> DNA
<213> Artificial Sequence

<220>
<223> Primer

<400> 245
gcgtgctttt tgctttgctt tg 22

<210> 246
<211> 24
<212> DNA
<213> Artificial Sequence

<220>
<223> Primer

<400> 246
cagaggctgc tgttatccac aata 24

<210> 247
<211> 22
<212> DNA
<213> Artificial Sequence

<220>
<223> Primer

<400> 247
tgggcccttc tgatccttct at 2

<210> 248
<211> 28
<212> DNA
<213> Artificial Sequence

<220>
<223> Primer

<400> 248
ggtcagtgaa agtgggatta ttatgtgt 28

<210> 249
<211> 24
<212> DNA
<213> Artificial Sequence

<220>
<223> Primer

<400> 249
ctgcttttgt aaccactccc acta 24

<210> 250
<211> 24
<212> DNA
<213> Artificial Sequence

<220>
<223> Primer

<400> 250
cctagaggtt aatgctggcc tatg 24

<210> 251
<211> 21
<212> DNA
<213> Artificial Sequence

<220>
<223> Primer

<400> 251
cttcacatgc agcctcacct a 21

<210> 252
<211> 26
<212> DNA
<213> Artificial Sequence

<220>
<223> Primer

<400> 252
ggaatattgt atgcaccacc aaaagg 26

<210> 253
<211> 24
<212> DNA
<213> Artificial Sequence

<220>
<223> Primer

<400> 253
cctatagttc cagggtctcc acaa 24

<210> 254
<211> 26
<212> DNA
<213> Artificial Sequence

<220>
<223> Primer

<400> 254
atccgtgctt acaaccttag atactg 26

<210> 255
<211> 27
<212> DNA
<213> Artificial Sequence

<220>
<223> Primer

<400> 255
ggatgacaca gaaaatgcta gtgctta 27

<210> 256
<211> 24
<212> DNA
<213> Artificial Sequence

<220>
<223> Primer

<400> 256
cacctggatt tactgcaaca ttgg 24

<210> 257
<211> 24
<212> DNA
<213> Artificial Sequence

<220>
<223> Primer

<400> 257
acctccagca cctaaagaag atga 24

<210> 258
<211> 28
<212> DNA
<213> Artificial Sequence

<220>
<223> Primer

<400> 258
ggtgtagctt ttcgttttcc taatgtaa 28

<210> 259
<211> 25
<212> DNA

<213> Artificial Sequence

<220>
<223> Primer

<400> 259
cacagagctg caaacaacta tacat 25

<210> 260
<211> 26
<212> DNA
<213> Artificial Sequence

<220>
<223> Primer

<400> 260
tgtccagatg tctttgcttt tcttca 26

<210> 261
<211> 25
<212> DNA
<213> Artificial Sequence

<220>
<223> Primer

<400> 261
cacagagctg caaacaacta tacat 25

<210> 262
<211> 24
<212> DNA
<213> Artificial Sequence

<220>
<223> Primer

<400> 262
tcagttgtct ctggttgcaa atct 24

<210> 263
<211> 25
<212> DNA
<213> Artificial Sequence

<220>
<223> Primer

<400> 263
cacagagctg caaacaacta tacat 25

<210> 264
<211> 26
<212> DNA
<213> Artificial Sequence

<220>

<223> Primer

<400> 264
ccattaacag gtcttccaaa gtacga 26

<210> 265
<211> 25
<212> DNA
<213> Artificial Sequence

<220>
<223> Primer

<400> 265
cacagagctg caaacaacta tacat 25

<210> 266
<211> 28
<212> DNA
<213> Artificial Sequence

<220>
<223> Primer

<400> 266
cactaagtgg actaccaaat actttcgt 28

<210> 267
<211> 25
<212> DNA
<213> Artificial Sequence

<220>
<223> Primer

<400> 267
cacagagctg caaacaacta tacat 25

<210> 268
<211> 28
<212> DNA
<213> Artificial Sequence

<220>
<223> Primer

<400> 268
gttttcgtca aatggaaact cattagga 28

<210> 269
<211> 25
<212> DNA
<213> Artificial Sequence

<220>
<223> Primer

<400> 269

cacagagctg caaacaacta tacat 25

<210> 270
<211> 26
<212> DNA
<213> Artificial Sequence

<220>
<223> Primer

<400> 270
tgacacacat ttaaacgttg gcaaag 26

<210> 271
<211> 25
<212> DNA
<213> Artificial Sequence

<220>
<223> Primer

<400> 271
cacagagctg caaacaacta tacat 25

<210> 272
<211> 19
<212> DNA
<213> Artificial Sequence

<220>
<223> Primer

<400> 272
aaggcgacgg ctttggtat 19

<210> 273
<211> 25
<212> DNA
<213> Artificial Sequence

<220>
<223> Primer

<400> 273
cacagagctg caaacaacta tacat 25

<210> 274
<211> 28
<212> DNA
<213> Artificial Sequence

<220>
<223> Primer

<400> 274
ggtcagtgaa agtgggatta ttatgtgt 28

<210> 275

<211> 25
<212> DNA
<213> Artificial Sequence

<220>
<223> Primer

<400> 275
cacagagctg caaacaacta tacat 25

<210> 276
<211> 26
<212> DNA
<213> Artificial Sequence

<220>
<223> Primer

<400> 276
ggaatattgt atgcaccacc aaaagg 26

<210> 277
<211> 25
<212> DNA
<213> Artificial Sequence

<220>
<223> Primer

<400> 277
cacagagctg caaacaacta tacat 25

<210> 278
<211> 26
<212> DNA
<213> Artificial Sequence

<220>
<223> Primer

<400> 278
atccgtgctt acaaccttag atactg 26

<210> 279
<211> 25
<212> DNA
<213> Artificial Sequence

<220>
<223> Primer

<400> 279
cacagagctg caaacaacta tacat 25

<210> 280
<211> 28
<212> DNA
<213> Artificial Sequence

<220>
<223> Primer

<400> 280
ggtgtagctt ttcgttttcc taatgtaa 28

<210> 281
<211> 21
<212> DNA
<213> Artificial Sequence

<220>
<223> Primer

<400> 281
ggaattgtgt gccccatctg t 21

<210> 282
<211> 28
<212> DNA
<213> Artificial Sequence

<220>
<223> Primer

<400> 282
cactaagtgg actaccaaat actttcgt 28

<210> 283
<211> 21
<212> DNA
<213> Artificial Sequence

<220>
<223> Primer

<400> 283
ggaattgtgt gccccatctg t 21

<210> 284
<211> 28
<212> DNA
<213> Artificial Sequence

<220>
<223> Primer

<400> 284
gttttcgtca aatggaaact cattagga 28

<210> 285
<211> 21
<212> DNA
<213> Artificial Sequence

<220>
<223> Primer

<400> 285
ggaattgtgt gccccatctg t 21

<210> 286
<211> 26
<212> DNA
<213> Artificial Sequence

<220>
<223> Primer

<400> 286
tgacacacat ttaaacgttg gcaaag 26

<210> 287
<211> 21
<212> DNA
<213> Artificial Sequence

<220>
<223> Primer

<400> 287
ggaattgtgt gccccatctg t 21

<210> 288
<211> 19
<212> DNA
<213> Artificial Sequence

<220>
<223> Primer

<400> 288
aaggcgacgg ctttggtat 19

<210> 289
<211> 21
<212> DNA
<213> Artificial Sequence

<220>
<223> Primer

<400> 289
ggaattgtgt gccccatctg t 21

<210> 290
<211> 28
<212> DNA
<213> Artificial Sequence

<220>
<223> Primer

<400> 290
ggtcagtgaa agtgggatta ttatgtgt 28

<210> 291
<211> 21
<212> DNA
<213> Artificial Sequence

<220>
<223> Primer

<400> 291
ggaattgtgt gccccatctg t 21

<210> 292
<211> 26
<212> DNA
<213> Artificial Sequence

<220>
<223> Primer

<400> 292
ggaatattgt atgcaccacc aaaagg 26

<210> 293
<211> 21
<212> DNA
<213> Artificial Sequence

<220>
<223> Primer

<400> 293
ggaattgtgt gccccatctg t 21

<210> 294
<211> 26
<212> DNA
<213> Artificial Sequence

<220>
<223> Primer

<400> 294
atccgtgctt acaaccttag atactg 26

<210> 295
<211> 21
<212> DNA
<213> Artificial Sequence

<220>
<223> Primer

<400> 295
ggaattgtgt gccccatctg t 21

<210> 296
<211> 28
<212> DNA

<213> Artificial Sequence

<220>
<223> Primer

<400> 296
ggtgtagctt ttcgttttcc taatgtaa 28

<210> 297
<211> 26
<212> DNA
<213> Artificial Sequence

<220>
<223> Primer

<400> 297
ggttttatgt agaggctgta gtggaa 26

<210> 298
<211> 28
<212> DNA
<213> Artificial Sequence

<220>
<223> Primer

<400> 298
cactaagtgg actaccaaat actttcgt 28

<210> 299
<211> 26
<212> DNA
<213> Artificial Sequence

<220>
<223> Primer

<400> 299
ggttttatgt agaggctgta gtggaa 26

<210> 300
<211> 28
<212> DNA
<213> Artificial Sequence

<220>
<223> Primer

<400> 300
gttttcgtca aatggaaact cattagga 28

<210> 301
<211> 26
<212> DNA
<213> Artificial Sequence

<220>

<223> Primer

<400> 301
ggttttatgt agaggctgta gtggaa 26

<210> 302
<211> 26
<212> DNA
<213> Artificial Sequence

<220>
<223> Primer

<400> 302
tgacacacat ttaaacgttg gcaaag 26

<210> 303
<211> 26
<212> DNA
<213> Artificial Sequence

<220>
<223> Primer

<400> 303
ggttttatgt agaggctgta gtggaa 26

<210> 304
<211> 19
<212> DNA
<213> Artificial Sequence

<220>
<223> Primer

<400> 304
aaggcgacgg ctttggtat 19

<210> 305
<211> 26
<212> DNA
<213> Artificial Sequence

<220>
<223> Primer

<400> 305
ggttttatgt agaggctgta gtggaa 26

<210> 306
<211> 28
<212> DNA
<213> Artificial Sequence

<220>
<223> Primer

<400> 306

ggtcagtgaa agtgggatta ttatgtgt 28

<210> 307
<211> 26
<212> DNA
<213> Artificial Sequence

<220>
<223> Primer

<400> 307
ggttttatgt agaggctgta gtggaa 26

<210> 308
<211> 26
<212> DNA
<213> Artificial Sequence

<220>
<223> Primer

<400> 308
ggaatattgt atgcaccacc aaaagg 26

<210> 309
<211> 26
<212> DNA
<213> Artificial Sequence

<220>
<223> Primer

<400> 309
ggttttatgt agaggctgta gtggaa 26

<210> 310
<211> 26
<212> DNA
<213> Artificial Sequence

<220>
<223> Primer

<400> 310
atccgtgctt acaaccttag atactg 26

<210> 311
<211> 26
<212> DNA
<213> Artificial Sequence

<220>
<223> Primer

<400> 311
ggttttatgt agaggctgta gtggaa 26

<210> 312

<211> 28
<212> DNA
<213> Artificial Sequence

<220>
<223> Primer

<400> 312
ggtgtagctt ttcgttttcc taatgtaa 28

<210> 313
<211> 22
<212> DNA
<213> Artificial Sequence

<220>
<223> Primer

<400> 313
gcgggtatgg caatactgaa gt 22

<210> 314
<211> 28
<212> DNA
<213> Artificial Sequence

<220>
<223> Primer

<400> 314
gttttcgtca aatggaaact cattagga 28

<210> 315
<211> 22
<212> DNA
<213> Artificial Sequence

<220>
<223> Primer

<400> 315
gcgggtatgg caatactgaa gt 22

<210> 316
<211> 26
<212> DNA
<213> Artificial Sequence

<220>
<223> Primer

<400> 316
tgacacacat ttaaacgttg gcaaag 26

<210> 317
<211> 22
<212> DNA
<213> Artificial Sequence

<220>
<223> Primer

<400> 317
gcgggtatgg caatactgaa gt 22

<210> 318
<211> 19
<212> DNA
<213> Artificial Sequence

<220>
<223> Primer

<400> 318
aaggcgacgg ctttggtat 19

<210> 319
<211> 22
<212> DNA
<213> Artificial Sequence

<220>
<223> Primer

<400> 319
gcgggtatgg caatactgaa gt 22

<210> 320
<211> 26
<212> DNA
<213> Artificial Sequence

<220>
<223> Primer

<400> 320
atccgtgctt acaaccttag atactg 26

<210> 321
<211> 22
<212> DNA
<213> Artificial Sequence

<220>
<223> Primer

<400> 321
gcgggtatgg caatactgaa gt 22

<210> 322
<211> 28
<212> DNA
<213> Artificial Sequence

<220>
<223> Primer

<400> 322
ggtcagtgaa agtgggatta ttatgtgt 28

<210> 323
<211> 22
<212> DNA
<213> Artificial Sequence

<220>
<223> Primer

<400> 323
gcgggtatgg caatactgaa gt 22

<210> 324
<211> 26
<212> DNA
<213> Artificial Sequence

<220>
<223> Primer

<400> 324
ggaatattgt atgcaccacc aaaagg 26

<210> 325
<211> 22
<212> DNA
<213> Artificial Sequence

<220>
<223> Primer

<400> 325
gcgggtatgg caatactgaa gt 22

<210> 326
<211> 28
<212> DNA
<213> Artificial Sequence

<220>
<223> Primer

<400> 326
ggtgtagctt ttcgttttcc taatgtaa 28

<210> 327
<211> 27
<212> DNA
<213> Artificial Sequence

<220>
<223> Primer

<400> 327
gaggtgattg gaagcaaatt gttatgt 27

<210> 328
<211> 26
<212> DNA
<213> Artificial Sequence

<220>
<223> Primer

<400> 328
tgacacacat ttaaacgttg gcaaag 26

<210> 329
<211> 27
<212> DNA
<213> Artificial Sequence

<220>
<223> Primer

<400> 329
gaggtgattg gaagcaaatt gttatgt 27

<210> 330
<211> 19
<212> DNA
<213> Artificial Sequence

<220>
<223> Primer

<400> 330
aaggcgacgg ctttggtat 19

<210> 331
<211> 27
<212> DNA
<213> Artificial Sequence

<220>
<223> Primer

<400> 331
gaggtgattg gaagcaaatt gttatgt 27

<210> 332
<211> 26
<212> DNA
<213> Artificial Sequence

<220>
<223> Primer

<400> 332
atccgtgctt acaaccttag atactg 26

<210> 333
<211> 27
<212> DNA

<213> Artificial Sequence

<220>
<223> Primer

<400> 333
gaggtgattg gaagcaaatt gttatgt 27

<210> 334
<211> 28
<212> DNA
<213> Artificial Sequence

<220>
<223> Primer

<400> 334
ggtcagtgaa agtgggatta ttatgtgt 28

<210> 335
<211> 27
<212> DNA
<213> Artificial Sequence

<220>
<223> Primer

<400> 335
gaggtgattg gaagcaaatt gttatgt 27

<210> 336
<211> 26
<212> DNA
<213> Artificial Sequence

<220>
<223> Primer

<400> 336
ggaatattgt atgcaccacc aaaagg 26

<210> 337
<211> 27
<212> DNA
<213> Artificial Sequence

<220>
<223> Primer

<400> 337
gaggtgattg gaagcaaatt gttatgt 27

<210> 338
<211> 28
<212> DNA
<213> Artificial Sequence

<220>

<223> Primer

<400> 338
ggtgtagctt ttcgttttcc taatgtaa 28

<210> 339
<211> 23
<212> DNA
<213> Artificial Sequence

<220>
<223> Primer

<400> 339
gctcacacaa aggacggatt aac 23

<210> 340
<211> 26
<212> DNA
<213> Artificial Sequence

<220>
<223> Primer

<400> 340
atccgtgctt acaaccttag atactg 26

<210> 341
<211> 23
<212> DNA
<213> Artificial Sequence

<220>
<223> Primer

<400> 341
gctcacacaa aggacggatt aac 23

<210> 342
<211> 28
<212> DNA
<213> Artificial Sequence

<220>
<223> Primer

<400> 342
ggtcagtgaa agtgggatta ttatgtgt 28

<210> 343
<211> 23
<212> DNA
<213> Artificial Sequence

<220>
<223> Primer

<400> 343

gctcacacaa aggacggatt aac 23

<210> 344
<211> 26
<212> DNA
<213> Artificial Sequence

<220>
<223> Primer

<400> 344
ggaatattgt atgcaccacc aaaagg 26

<210> 345
<211> 23
<212> DNA
<213> Artificial Sequence

<220>
<223> Primer

<400> 345
gctcacacaa aggacggatt aac 23

<210> 346
<211> 28
<212> DNA
<213> Artificial Sequence

<220>
<223> Primer

<400> 346
ggtgtagctt ttcgttttcc taatgtaa 28

<210> 347
<211> 24
<212> DNA
<213> Artificial Sequence

<220>
<223> Primer

<400> 347
ctgcttttgt aaccactccc acta 24

<210> 348
<211> 26
<212> DNA
<213> Artificial Sequence

<220>
<223> Primer

<400> 348
ggaatattgt atgcaccacc aaaagg 26

<210> 349

<211> 24
<212> DNA
<213> Artificial Sequence

<220>
<223> Primer

<400> 349
ctgcttttgt aaccactccc acta 24

<210> 350
<211> 26
<212> DNA
<213> Artificial Sequence

<220>
<223> Primer

<400> 350
atccgtgctt acaaccttag atactg 26

<210> 351
<211> 24
<212> DNA
<213> Artificial Sequence

<220>
<223> Primer

<400> 351
ctgcttttgt aaccactccc acta 24

<210> 352
<211> 28
<212> DNA
<213> Artificial Sequence

<220>
<223> Primer

<400> 352
ggtgtagctt ttcgttttcc taatgtaa 28

<210> 353
<211> 21
<212> DNA
<213> Artificial Sequence

<220>
<223> Primer

<400> 353
ggaattgtgt gccccatctg t 21

<210> 354
<211> 19
<212> DNA
<213> Artificial Sequence

<220>
<223> Primer

<400> 354
ctgagaagcc ctgcccttc 19

<210> 355
<211> 21
<212> DNA
<213> Artificial Sequence

<220>
<223> Primer

<400> 355
ggaattgtgt gccccatctg t 21

<210> 356
<211> 19
<212> DNA
<213> Artificial Sequence

<220>
<223> Primer

<400> 356
aaatacggct gcaccgagt 19

<210> 357
<211> 21
<212> DNA
<213> Artificial Sequence

<220>
<223> Primer

<400> 357
ggaattgtgt gccccatctg t 21

<210> 358
<211> 24
<212> DNA
<213> Artificial Sequence

<220>
<223> Primer

<400> 358
ggtgatccag actctgacct tttg 24

<210> 359
<211> 21
<212> DNA
<213> Artificial Sequence

<220>
<223> Primer

<400> 359
ggaattgtgt gccccatctg t 21

<210> 360
<211> 22
<212> DNA
<213> Artificial Sequence

<220>
<223> Primer

<400> 360
atcatgatgg ctgtatgtgc ca 22

<210> 361
<211> 21
<212> DNA
<213> Artificial Sequence

<220>
<223> Primer

<400> 361
ggaattgtgt gccccatctg t 21

<210> 362
<211> 21
<212> DNA
<213> Artificial Sequence

<220>
<223> Primer

<400> 362
catggttcca ccagcgttat t 21

<210> 363
<211> 21
<212> DNA
<213> Artificial Sequence

<220>
<223> Primer

<400> 363
ggaattgtgt gccccatctg t 21

<210> 364
<211> 18
<212> DNA
<213> Artificial Sequence

<220>
<223> Primer

<400> 364
tctttgctcg cagctcgt 18

<210> 365
<211> 27
<212> DNA
<213> Artificial Sequence

<220>
<223> Primer

<400> 365
agtacagacc tacgtgacca tatagac 27

<210> 366
<211> 19
<212> DNA
<213> Artificial Sequence

<220>
<223> Primer

<400> 366
ctgagaagcc ctgcccttc 19

<210> 367
<211> 27
<212> DNA
<213> Artificial Sequence

<220>
<223> Primer

<400> 367
agtacagacc tacgtgacca tatagac 27

<210> 368
<211> 19
<212> DNA
<213> Artificial Sequence

<220>
<223> Primer

<400> 368
aaatacggct gcaccgagt 19

<210> 369
<211> 27
<212> DNA
<213> Artificial Sequence

<220>
<223> Primer

<400> 369
agtacagacc tacgtgacca tatagac 27

<210> 370
<211> 24
<212> DNA

<213> Artificial Sequence

<220>
<223> Primer

<400> 370
ggtgatccag actctgacct tttg 24

<210> 371
<211> 27
<212> DNA
<213> Artificial Sequence

<220>
<223> Primer

<400> 371
agtacagacc tacgtgacca tatagac 27

<210> 372
<211> 22
<212> DNA
<213> Artificial Sequence

<220>
<223> Primer

<400> 372
atcatgatgg ctgtatgtgc ca 22

<210> 373
<211> 27
<212> DNA
<213> Artificial Sequence

<220>
<223> Primer

<400> 373
agtacagacc tacgtgacca tatagac 27

<210> 374
<211> 21
<212> DNA
<213> Artificial Sequence

<220>
<223> Primer

<400> 374
catggttcca ccagcgttat t 21

<210> 375
<211> 27
<212> DNA
<213> Artificial Sequence

<220>

<223> Primer

<400> 375
agtacagacc tacgtgacca tatagac 27

<210> 376
<211> 18
<212> DNA
<213> Artificial Sequence

<220>
<223> Primer

<400> 376
tctttgctcg cagctcgt 18

<210> 377
<211> 27
<212> DNA
<213> Artificial Sequence

<220>
<223> Primer

<400> 377
agtacagacc tacgtgacca tatagac 27

<210> 378
<211> 18
<212> DNA
<213> Artificial Sequence

<220>
<223> Primer

<400> 378
tctttgctcg cagctcgt 18

<210> 379
<211> 22
<212> DNA
<213> Artificial Sequence

<220>
<223> Primer

<400> 379
gcgggtatgg caatactgaa gt 22

<210> 380
<211> 28
<212> DNA
<213> Artificial Sequence

<220>
<223> Primer

<400> 380

gttttcgtca aatggaaact cattagga 28

<210> 381
<211> 25
<212> DNA
<213> Artificial Sequence

<220>
<223> Primer

<400> 381
cacagagctg caaacaacta tacat 25

<210> 382
<211> 26
<212> DNA
<213> Artificial Sequence

<220>
<223> Primer

<400> 382
ccattaacag gtcttccaaa gtacga 26

<210> 383
<211> 17
<212> DNA
<213> Artificial Sequence

<220>
<223> Primer

<400> 383
cggtggaccg gtcgatg 17

<210> 384
<211> 24
<212> DNA
<213> Artificial Sequence

<220>
<223> Primer

<400> 384
tcagttgtct ctggttgcaa atct 24

<210> 385
<211> 22
<212> DNA
<213> Artificial Sequence

<220>
<223> Primer

<400> 385
gcgggtatgg caatactgaa gt 22

<210> 386

<211> 26
<212> DNA
<213> Artificial Sequence

<220>
<223> Primer

<400> 386
tggtgtttgg catatagtgt gtcttt 26

<210> 387
<211> 21
<212> DNA
<213> Artificial Sequence

<220>
<223> Primer

<400> 387
ggaattgtgt gccccatctg t 21

<210> 388
<211> 28
<212> DNA
<213> Artificial Sequence

<220>
<223> Primer

<400> 388
gttttcgtca aatggaaact cattagga 28

<210> 389
<211> 27
<212> DNA
<213> Artificial Sequence

<220>
<223> Primer

<400> 389
agtacagacc tacgtgacca tatagac 27

<210> 390
<211> 19
<212> DNA
<213> Artificial Sequence

<220>
<223> Primer

<400> 390
aaatacggct gcaccgagt 19

<210> 391
<211> 23
<212> DNA
<213> Artificial Sequence

<220>
<223> Primer

<400> 391
gctcacacaa aggacggatt aac 23

<210> 392
<211> 18
<212> DNA
<213> Artificial Sequence

<220>
<223> Primer

<400> 392
tctttgctcg cagctcgt 18

<210> 393
<211> 21
<212> DNA
<213> Artificial Sequence

<220>
<223> Primer

<400> 393
ggaattgtgt gccccatctg t 21

<210> 394
<211> 18
<212> DNA
<213> Artificial Sequence

<220>
<223> Primer

<400> 394
tctttgctcg cagctcgt 18

<210> 395
<211> 25
<212> DNA
<213> Artificial Sequence

<220>
<223> Primer

<400> 395
cacagagctg caaacaacta tacat 25

<210> 396
<211> 27
<212> DNA
<213> Artificial Sequence

<220>
<223> Primer

<400> 396
cacatacagc atatggattc ccatctc 27

<210> 397
<211> 25
<212> DNA
<213> Artificial Sequence

<220>
<223> Primer

<400> 397
cacagagctg caaacaacta tacat 25

<210> 398
<211> 26
<212> DNA
<213> Artificial Sequence

<220>
<223> Primer

<400> 398
tgtccagatg tctttgcttt tcttca 26

<210> 399
<211> 21
<212> DNA
<213> Artificial Sequence

<220>
<223> Primer

<400> 399
ggaattgtgt gccccatctg t 21

<210> 400
<211> 19
<212> DNA
<213> Artificial Sequence

<220>
<223> Primer

<400> 400
aaggcgacgg ctttggtat 19

<210> 401
<211> 27
<212> DNA
<213> Artificial Sequence

<220>
<223> Primer

<400> 401
ggatgtaaag catagaccat tggtaca 27

<210> 402
<211> 28
<212> DNA
<213> Artificial Sequence

<220>
<223> Primer

<400> 402
gttttcgtca aatggaaact cattagga 28

<210> 403
<211> 27
<212> DNA
<213> Artificial Sequence

<220>
<223> Primer

<400> 403
agtacagacc tacgtgacca tatagac 27

<210> 404
<211> 24
<212> DNA
<213> Artificial Sequence

<220>
<223> Primer

<400> 404
ggtgatccag actctgacct tttg 24

<210> 405
<211> 27
<212> DNA
<213> Artificial Sequence

<220>
<223> Primer

<400> 405
agtacagacc tacgtgacca tatagac 27

<210> 406
<211> 22
<212> DNA
<213> Artificial Sequence

<220>
<223> Primer

<400> 406
atcatgatgg ctgtatgtgc ca 22

<210> 407
<211> 23
<212> DNA

<213> Artificial Sequence

<220>
<223> Primer

<400> 407
gctcacacaa aggacggatt aac 23

<210> 408
<211> 22
<212> DNA
<213> Artificial Sequence

<220>
<223> Primer

<400> 408
ccaatgccat gtagacgaca ct 22

<210> 409
<211> 23
<212> DNA
<213> Artificial Sequence

<220>
<223> Primer

<400> 409
gctcacacaa aggacggatt aac 23

<210> 410
<211> 21
<212> DNA
<213> Artificial Sequence

<220>
<223> Primer

<400> 410
catggttcca ccagcgttat t 21

<210> 411
<211> 21
<212> DNA
<213> Artificial Sequence

<220>
<223> Primer

<400> 411
ggaattgtgt gccccatctg t 21

<210> 412
<211> 24
<212> DNA
<213> Artificial Sequence

<220>

<223> Primer

<400> 412
ggtgatccag actctgacct tttg 24

<210> 413
<211> 25
<212> DNA
<213> Artificial Sequence

<220>
<223> Primer

<400> 413
cacagagctg caaacaacta tacat 25

<210> 414
<211> 28
<212> DNA
<213> Artificial Sequence

<220>
<223> Primer

<400> 414
gttttcgtca aatggaaact cattagga 28

<210> 415
<211> 22
<212> DNA
<213> Artificial Sequence

<220>
<223> Primer

<400> 415
gcgggtatgg caatactgaa gt 22

<210> 416
<211> 26
<212> DNA
<213> Artificial Sequence

<220>
<223> Primer

<400> 416
tgacacacat ttaaacgttg gcaaag 26

<210> 417
<211> 21
<212> DNA
<213> Artificial Sequence

<220>
<223> Primer

<400> 417

ggaattgtgt gccccatctg t 21

<210> 418
<211> 22
<212> DNA
<213> Artificial Sequence

<220>
<223> Primer

<400> 418
atcatgatgg ctgtatgtgc ca 22

<210> 419
<211> 23
<212> DNA
<213> Artificial Sequence

<220>
<223> Primer

<400> 419
gctcacacaa aggacggatt aac 23

<210> 420
<211> 26
<212> DNA
<213> Artificial Sequence

<220>
<223> Primer

<400> 420
atccgtgctt acaaccttag atactg 26

<210> 421
<211> 19
<212> DNA
<213> Artificial Sequence

<220>
<223> Primer

<400> 421
catgcgggtg gtcaggtaa 19

<210> 422
<211> 19
<212> DNA
<213> Artificial Sequence

<220>
<223> Primer

<400> 422
aaggcgacgg ctttggtat 19

<210> 423

<211> 24
<212> DNA
<213> Artificial Sequence

<220>
<223> Primer

<400> 423
cctatagttc cagggtctcc acaa 24

<210> 424
<211> 26
<212> DNA
<213> Artificial Sequence

<220>
<223> Primer

<400> 424
atccgtgctt acaaccttag atactg 26

<210> 425
<211> 21
<212> DNA
<213> Artificial Sequence

<220>
<223> Primer

<400> 425
ggaattgtgt gccccatctg t 21

<210> 426
<211> 21
<212> DNA
<213> Artificial Sequence

<220>
<223> Primer

<400> 426
catggttcca ccagcgttat t 21

<210> 427
<211> 27
<212> DNA
<213> Artificial Sequence

<220>
<223> Primer

<400> 427
agtacagacc tacgtgacca tatagac 27

<210> 428
<211> 19
<212> DNA
<213> Artificial Sequence

<220>
<223> Primer

<400> 428
ctgagaagcc ctgcccttc 19

<210> 429
<211> 28
<212> DNA
<213> Artificial Sequence

<220>
<223> Primer

<400> 429
cggaaatcca gtgtatgagc ttaatgat 28

<210> 430
<211> 26
<212> DNA
<213> Artificial Sequence

<220>
<223> Primer

<400> 430
tgacacacat ttaaacgttg gcaaag 26

<210> 431
<211> 25
<212> DNA
<213> Artificial Sequence

<220>
<223> Primer

<400> 431
cacagagctg caaacaacta tacat 25

<210> 432
<211> 19
<212> DNA
<213> Artificial Sequence

<220>
<223> Primer

<400> 432
aaggcgacgg ctttggtat 19

<210> 433
<211> 23
<212> DNA
<213> Artificial Sequence

<220>
<223> Primer

<400> 433
gctcacacaa aggacggatt aac 23

<210> 434
<211> 19
<212> DNA
<213> Artificial Sequence

<220>
<223> Primer

<400> 434
ctgagaagcc ctgcccttc 19

<210> 435
<211> 22
<212> DNA
<213> Artificial Sequence

<220>
<223> Primer

<400> 435
gcgggtatgg caatactgaa gt 22

<210> 436
<211> 19
<212> DNA
<213> Artificial Sequence

<220>
<223> Primer

<400> 436
aaggcgacgg ctttggtat 19

<210> 437
<211> 21
<212> DNA
<213> Artificial Sequence

<220>
<223> Primer

<400> 437
ggaattgtgt gccccatctg t 21

<210> 438
<211> 26
<212> DNA
<213> Artificial Sequence

<220>
<223> Primer

<400> 438
atccgtgctt acaaccttag atactg 26

<210> 439
<211> 26
<212> DNA
<213> Artificial Sequence

<220>
<223> Primer

<400> 439
ctcagaggag gaggatgaaa tagatg 26

<210> 440
<211> 26
<212> DNA
<213> Artificial Sequence

<220>
<223> Primer

<400> 440
ccattaacag gtcttccaaa gtacga 26

<210> 441
<211> 21
<212> DNA
<213> Artificial Sequence

<220>
<223> Primer

<400> 441
ggaattgtgt gccccatctg t 21

<210> 442
<211> 19
<212> DNA
<213> Artificial Sequence

<220>
<223> Primer

<400> 442
aaatacggct gcaccgagt 19

<210> 443
<211> 23
<212> DNA
<213> Artificial Sequence

<220>
<223> Primer

<400> 443
gctcacacaa aggacggatt aac 23

<210> 444
<211> 24
<212> DNA

<213> Artificial Sequence

<220>
<223> Primer

<400> 444
ggtgatccag actctgacct tttg 24

<210> 445
<211> 21
<212> DNA
<213> Artificial Sequence

<220>
<223> Primer

<400> 445
ggaattgtgt gccccatctg t 21

<210> 446
<211> 19
<212> DNA
<213> Artificial Sequence

<220>
<223> Primer

<400> 446
ctgagaagcc ctgcccttc 19

<210> 447
<211> 28
<212> DNA
<213> Artificial Sequence

<220>
<223> Primer

<400> 447
ggaacaacat tagaacagca atacaaca 28

<210> 448
<211> 26
<212> DNA
<213> Artificial Sequence

<220>
<223> Primer

<400> 448
tgtccagatg tctttgcttt tcttca 26

<210> 449
<211> 21
<212> DNA
<213> Artificial Sequence

<220>

<223> Primer

<400> 449
ggaattgtgt gccccatctg t 21

<210> 450
<211> 22
<212> DNA
<213> Artificial Sequence

<220>
<223> 16_unsp_880_881

<400> 450
catccattac atcccgtacc ct 22

<210> 451
<211> 23
<212> DNA
<213> Artificial Sequence

<220>
<223> Primer

<400> 451
gctcacacaa aggacggatt aac 23

<210> 452
<211> 22
<212> DNA
<213> Artificial Sequence

<220>
<223> Primer

<400> 452
atcatgatgg ctgtatgtgc ca 22

<210> 453
<211> 25
<212> DNA
<213> Artificial Sequence

<220>
<223> Primer

<400> 453
cacagagctg caaacaacta tacat 25

<210> 454
<211> 26
<212> DNA
<213> Artificial Sequence

<220>
<223> Primer

<400> 454

tgacacacat ttaaacgttg gcaaag 26

<210> 455
<211> 21
<212> DNA
<213> Artificial Sequence

<220>
<223> Primer

<400> 455
ggaattgtgt gccccatctg t 21

<210> 456
<211> 26
<212> DNA
<213> Artificial Sequence

<220>
<223> Primer

<400> 456
tgacacacat ttaaacgttg gcaaag 26

<210> 457
<211> 23
<212> DNA
<213> Artificial Sequence

<220>
<223> Primer

<400> 457
gctcacacaa aggacggatt aac 23

<210> 458
<211> 19
<212> DNA
<213> Artificial Sequence

<220>
<223> Primer

<400> 458
aaatacggct gcaccgagt 19

<210> 459
<211> 25
<212> DNA
<213> Artificial Sequence

<220>
<223> Primer

<400> 459
cacagagctg caaacaacta tacat 25

<210> 460

<211> 24
<212> DNA
<213> Artificial Sequence

<220>
<223> Primer

<400> 460
tcagttgtct ctggttgcaa atct 24

<210> 461
<211> 27
<212> DNA
<213> Artificial Sequence

<220>
<223> Primer

<400> 461
agtacagacc tacgtgacca tatagac 27

<210> 462
<211> 21
<212> DNA
<213> Artificial Sequence

<220>
<223> Primer

<400> 462
catggttcca ccagcgttat t 21

<210> 463
<211> 22
<212> DNA
<213> Artificial Sequence

<220>
<223> Primer

<400> 463
cgtgcttttt gctttgcttt gt 22

<210> 464
<211> 27
<212> DNA
<213> Artificial Sequence

<220>
<223> Primer

<400> 464
gaggctgctg ttatccacaa tagtaat 27

<210> 465
<211> 23
<212> DNA
<213> Artificial Sequence

<220>
<223> Primer

<400> 465
cctgtgtagg tgttgaggta ggt 23

<210> 466
<211> 25
<212> DNA
<213> Artificial Sequence

<220>
<223> Primer

<400> 466
tctattatcc acacctgcat ttgct 25

<210> 467
<211> 23
<212> DNA
<213> Artificial Sequence

<220>
<223> Primer

<400> 467
aacgtgttgc gattggtgta ttg 23

<210> 468
<211> 27
<212> DNA
<213> Artificial Sequence

<220>
<223> Primer

<400> 468
cattccccat gaacatgcta aactttg 27

<210> 469
<211> 20
<212> DNA
<213> Artificial Sequence

<220>
<223> Primer

<400> 469
ccaggcccat tttgtagctt 20

<210> 470
<211> 23
<212> DNA
<213> Artificial Sequence

<220>
<223> Primer

<400> 470
aggtcaggaa aacagggatt tgg 23

<210> 471
<211> 31
<212> DNA
<213> Artificial Sequence

<220>
<223> Primer

<400> 471
ctaaaatgtc ctccaatact actaaccaca a 31

<210> 472
<211> 28
<212> DNA
<213> Artificial Sequence

<220>
<223> Primer

<400> 472
gtcatttatt tcatatactg gattgcca 28

<210> 473
<211> 20
<212> DNA
<213> Artificial Sequence

<220>
<223> Primer

<400> 473
tgcatcccag cagtaagcaa 20

<210> 474
<211> 28
<212> DNA
<213> Artificial Sequence

<220>
<223> Primer

<400> 474
gtcatttatt tcatatactg gattgcca 28

<210> 475
<211> 22
<212> DNA
<213> Artificial Sequence

<220>
<223> Primer

<400> 475
ggattggaca ctgcaagaca ca 22

<210> 476
<211> 28
<212> DNA
<213> Artificial Sequence

<220>
<223> Primer

<400> 476
cccatgctac ataggtcata caattgtc 28

<210> 477
<211> 22
<212> DNA
<213> Artificial Sequence

<220>
<223> Primer

<400> 477
ggattggaca ctgcaagaca ca 22

<210> 478
<211> 18
<212> DNA
<213> Artificial Sequence

<220>
<223> Primer

<400> 478
acgtctggcc gtaggtct 18

<210> 479
<211> 28
<212> DNA
<213> Artificial Sequence

<220>
<223> Primer

<400> 479
cagaggaaga aaacgatgaa atagatgg 28

<210> 480
<211> 21
<212> DNA
<213> Artificial Sequence

<220>
<223> Primer

<400> 480
agaaacagct gctggaatgc t 21

<210> 481
<211> 25
<212> DNA

<213> Artificial Sequence

<220>
<223> Primer

<400> 481
tcctaagaaa cgtaaacgtg ttccc 25

<210> 482
<211> 27
<212> DNA
<213> Artificial Sequence

<220>
<223> Primer

<400> 482
gtatttacaa ctcttgccac agaagga 27

<210> 483
<211> 24
<212> DNA
<213> Artificial Sequence

<220>
<223> Primer

<400> 483
tcagatagtg gctatggctg ttct 24

<210> 484
<211> 28
<212> DNA
<213> Artificial Sequence

<220>
<223> Primer

<400> 484
gtcatttatt tcatatactg gattgcca 28

<210> 485
<211> 22
<212> DNA
<213> Artificial Sequence

<220>
<223> Primer

<400> 485
cagctacacc tacaggcaac aa 22

<210> 486
<211> 24
<212> DNA
<213> Artificial Sequence

<220>

<223> Primer

<400> 486
ggtgatccag actctgacct tttg 24

<210> 487
<211> 28
<212> DNA
<213> Artificial Sequence

<220>
<223> Primer

<400> 487
aatgacagta aagacataga cagccaaa 28

<210> 488
<211> 19
<212> DNA
<213> Artificial Sequence

<220>
<223> Primer

<400> 488
aaatacggct gcaccgagt 19

<210> 489
<211> 28
<212> DNA
<213> Artificial Sequence

<220>
<223> Primer

<400> 489
ttcactgcaa gacatagaaa taacctgt 28

<210> 490
<211> 18
<212> DNA
<213> Artificial Sequence

<220>
<223> Primer

<400> 490
acgtctggcc gtaggtct 18

<210> 491
<211> 22
<212> DNA
<213> Artificial Sequence

<220>
<223> Primer

<400> 491

cagctacacc tacaggcaac aa 22

<210> 492
<211> 22
<212> DNA
<213> Artificial Sequence

<220>
<223> Primer

<400> 492
atcatgatgg ctgtatgtgc ca 22

<210> 493
<211> 24
<212> DNA
<213> Artificial Sequence

<220>
<223> Primer

<400> 493
tcagatagtg gctatggctg ttct 24

<210> 494
<211> 21
<212> DNA
<213> Artificial Sequence

<220>
<223> Primer

<400> 494
ggtttccttc ggtgtctgca t 21

<210> 495
<211> 22
<212> DNA
<213> Artificial Sequence

<220>
<223> Primer

<400> 495
cagctacacc tacaggcaac aa 22

<210> 496
<211> 28
<212> DNA
<213> Artificial Sequence

<220>
<223> Primer

<400> 496
tcaggtaact gcaccctaaa tactctat 28

<210> 497

<211> 28
<212> DNA
<213> Artificial Sequence

<220>
<223> Primer

<400> 497
aatgacagta aagacataga cagccaaa 28

<210> 498
<211> 21
<212> DNA
<213> Artificial Sequence

<220>
<223> Primer

<400> 498
catggttcca ccagcgttat t 21

<210> 499
<211> 22
<212> DNA
<213> Artificial Sequence

<220>
<223> Primer

<400> 499
cagctacacc tacaggcaac aa 22

<210> 500
<211> 19
<212> DNA
<213> Artificial Sequence

<220>
<223> Primer

<400> 500
aaatacggct gcaccgagt 19

<210> 501
<211> 22
<212> DNA
<213> Artificial Sequence

<220>
<223> Primer

<400> 501
cagctacacc tacaggcaac aa 22

<210> 502
<211> 18
<212> DNA
<213> Artificial Sequence

<220>
<223> Primer

<400> 502
tctttgctcg cagctcgt 18

<210> 503
<211> 27
<212> DNA
<213> Artificial Sequence

<220>
<223> Primer

<400> 503
gcatatttta tcatgctggc agctcta 27

<210> 504
<211> 28
<212> DNA
<213> Artificial Sequence

<220>
<223> Primer

<400> 504
tcaggtaact gcaccctaaa tactctat 28

<210> 505
<211> 20
<212> DNA
<213> Artificial Sequence

<220>
<223> Primer

<400> 505
tgcatcccag cagtaagcaa 20

<210> 506
<211> 27
<212> DNA
<213> Artificial Sequence

<220>
<223> Primer

<400> 506
gtatttacaa ctcttgccac agaagga 27

<210> 507
<211> 20
<212> DNA
<213> Artificial Sequence

<220>
<223> Primer

<400> 507
tgcatcccag cagtaagcaa 20

<210> 508
<211> 18
<212> DNA
<213> Artificial Sequence

<220>
<223> Primer

<400> 508
tctttgctcg cagctcgt 18

<210> 509
<211> 28
<212> DNA
<213> Artificial Sequence

<220>
<223> Primer

<400> 509
aatgacagta aagacataga cagccaaa 28

<210> 510
<211> 22
<212> DNA
<213> Artificial Sequence

<220>
<223> Primer

<400> 510
atcatgatgg ctgtatgtgc ca 22

<210> 511
<211> 24
<212> DNA
<213> Artificial Sequence

<220>
<223> Primer

<400> 511
tcagatagtg gctatggctg ttct 24

<210> 512
<211> 18
<212> DNA
<213> Artificial Sequence

<220>
<223> Primer

<400> 512
acgtctggcc gtaggtct 18

<210> 513
<211> 20
<212> DNA
<213> Artificial Sequence

<220>
<223> Primer

<400> 513
tgcatcccag cagtaagcaa 20

<210> 514
<211> 19
<212> DNA
<213> Artificial Sequence

<220>
<223> Primer

<400> 514
aaatacggct gcaccgagt 19

<210> 515
<211> 20
<212> DNA
<213> Artificial Sequence

<220>
<223> Primer

<400> 515
tgcatcccag cagtaagcaa 20

<210> 516
<211> 19
<212> DNA
<213> Artificial Sequence

<220>
<223> Primer

<400> 516
ctgagaagcc ctgcccttc 19

<210> 517
<211> 28
<212> DNA
<213> Artificial Sequence

<220>
<223> Primer

<400> 517
aatgacagta aagacataga cagccaaa 28

<210> 518
<211> 18
<212> DNA

<213> Artificial Sequence

<220>
<223> Primer

<400> 518
tctttgctcg cagctcgt 18

<210> 519
<211> 28
<212> DNA
<213> Artificial Sequence

<220>
<223> Primer

<400> 519
aatgacagta aagacataga cagccaaa 28

<210> 520
<211> 24
<212> DNA
<213> Artificial Sequence

<220>
<223> Primer

<400> 520
ggtgatccag actctgacct tttg 24

<210> 521
<211> 28
<212> DNA
<213> Artificial Sequence

<220>
<223> Primer

<400> 521
ttcactgcaa gacatagaaa taacctgt 28

<210> 522
<211> 22
<212> DNA
<213> Artificial Sequence

<220>
<223> Primer

<400> 522
cccagctatg ttgtgaaatc gt 22

<210> 523
<211> 20
<212> DNA
<213> Artificial Sequence

<220>

<223> Primer

<400> 523
tgcatcccag cagtaagcaa 20

<210> 524
<211> 21
<212> DNA
<213> Artificial Sequence

<220>
<223> Primer

<400> 524
catggttcca ccagcgttat t 21

<210> 525
<211> 24
<212> DNA
<213> Artificial Sequence

<220>
<223> Primer

<400> 525
tcagatagtg gctatggctg ttct 24

<210> 526
<211> 20
<212> DNA
<213> Artificial Sequence

<220>
<223> Primer

<400> 526
ccgttgtcta tagcctccgt 20

<210> 527
<211> 22
<212> DNA
<213> Artificial Sequence

<220>
<223> Primer

<400> 527
cagctacacc tacaggcaac aa 22

<210> 528
<211> 27
<212> DNA
<213> Artificial Sequence

<220>
<223> Primer

<400> 528

gtatttacaa ctcttgccac agaagga 27

<210> 529
<211> 20
<212> DNA
<213> Artificial Sequence

<220>
<223> Primer

<400> 529
tgcatcccag cagtaagcaa 20

<210> 530
<211> 21
<212> DNA
<213> Artificial Sequence

<220>
<223> Primer

<400> 530
ggtttccttc ggtgtctgca t 21

<210> 531
<211> 20
<212> DNA
<213> Artificial Sequence

<220>
<223> Primer

<400> 531
tgcatcccag cagtaagcaa 20

<210> 532
<211> 18
<212> DNA
<213> Artificial Sequence

<220>
<223> Primer

<400> 532
acgtctggcc gtaggtct 18

<210> 533
<211> 28
<212> DNA
<213> Artificial Sequence

<220>
<223> Primer

<400> 533
ttcactgcaa gacatagaaa taacctgt 28

<210> 534

<211> 26
<212> DNA
<213> Artificial Sequence

<220>
<223> Primer

<400> 534
ctatacattt atggcatgca gcatgg 26

<210> 535
<211> 25
<212> DNA
<213> Artificial Sequence

<220>
<223> Primer

<400> 535
tcagactctg tgtatggaga cacat 25

<210> 536
<211> 22
<212> DNA
<213> Artificial Sequence

<220>
<223> Primer

<400> 536
cccagctatg ttgtgaaatc gt 22

<210> 537
<211> 28
<212> DNA
<213> Artificial Sequence

<220>
<223> Primer

<400> 537
aatgacagta aagacataga cagccaaa 28

<210> 538
<211> 19
<212> DNA
<213> Artificial Sequence

<220>
<223> Primer

<400> 538
ctgagaagcc ctgcccttc 19

<210> 539
<211> 20
<212> DNA
<213> Artificial Sequence

<220>
<223> Primer

<400> 539
tgcatcccag cagtaagcaa 20

<210> 540
<211> 24
<212> DNA
<213> Artificial Sequence

<220>
<223> Primer

<400> 540
ggtgatccag actctgacct tttg 24

<210> 541
<211> 28
<212> DNA
<213> Artificial Sequence

<220>
<223> Primer

<400> 541
ttcactgcaa gacatagaaa taacctgt 28

<210> 542
<211> 21
<212> DNA
<213> Artificial Sequence

<220>
<223> Primer

<400> 542
agaaacagct gctggaatgc t 21

<210> 543
<211> 22
<212> DNA
<213> Artificial Sequence

<220>
<223> Primer

<400> 543
cagctacacc tacaggcaac aa 22

<210> 544
<211> 21
<212> DNA
<213> Artificial Sequence

<220>
<223> Primer

<400> 544
catggttcca ccagcgttat t 21

<210> 545
<211> 20
<212> DNA
<213> Artificial Sequence

<220>
<223> Primer

<400> 545
tgcatcccag cagtaagcaa 20

<210> 546
<211> 28
<212> DNA
<213> Artificial Sequence

<220>
<223> Primer

<400> 546
tcaggtaact gcaccctaaa tactctat 28

<210> 547
<211> 20
<212> DNA
<213> Artificial Sequence

<220>
<223> Primer

<400> 547
tgcatcccag cagtaagcaa 20

<210> 548
<211> 22
<212> DNA
<213> Artificial Sequence

<220>
<223> Primer

<400> 548
atcatgatgg ctgtatgtgc ca 22

<210> 549
<211> 28
<212> DNA
<213> Artificial Sequence

<220>
<223> Primer

<400> 549
ttcactgcaa gacatagaaa taacctgt 28

<210> 550
<211> 21
<212> DNA
<213> Artificial Sequence

<220>
<223> Primer

<400> 550
ggtttccttc ggtgtctgca t 21

<210> 551
<211> 28
<212> DNA
<213> Artificial Sequence

<220>
<223> Primer

<400> 551
ttcactgcaa gacatagaaa taacctgt 28

<210> 552
<211> 28
<212> DNA
<213> Artificial Sequence

<220>
<223> Primer

<400> 552
gtcatttatt tcatatactg gattgcca 28

<210> 553
<211> 22
<212> DNA
<213> Artificial Sequence

<220>
<223> Primer

<400> 553
cagctacacc tacaggcaac aa 22

<210> 554
<211> 19
<212> DNA
<213> Artificial Sequence

<220>
<223> Primer

<400> 554
ctgagaagcc ctgcccttc 19

<210> 555
<211> 20
<212> DNA

<213> Artificial Sequence

<220>
<223> Primer

<400> 555
tgcatcccag cagtaagcaa 20

<210> 556
<211> 28
<212> DNA
<213> Artificial Sequence

<220>
<223> Primer

<400> 556
ctcgtcatct gatattacat ctcctgtt 28

<210> 557
<211> 27
<212> DNA
<213> Artificial Sequence

<220>
<223> Primer

<400> 557
cgaaaacata gcgaccacta tagagat 27

<210> 558
<211> 28
<212> DNA
<213> Artificial Sequence

<220>
<223> Primer

<400> 558
tcaggtaact gcaccctaaa tactctat 28

<210> 559
<211> 20
<212> DNA
<213> Artificial Sequence

<220>
<223> Primer

<400> 559
tgacgacacg gtatccgcta 20

<210> 560
<211> 18
<212> DNA
<213> Artificial Sequence

<220>

<223> Primer

<400> 560
acgtctggcc gtaggtct 18

<210> 561
<211> 27
<212> DNA
<213> Artificial Sequence

<220>
<223> Primer

<400> 561
cgaaaacata gcgaccacta tagagat 27

<210> 562
<211> 27
<212> DNA
<213> Artificial Sequence

<220>
<223> Primer

<400> 562
ttgtacacta tctggaattg caacagt 27

<210> 563
<211> 22
<212> DNA
<213> Artificial Sequence

<220>
<223> Primer

<400> 563
cagctacacc tacaggcaac aa 22

<210> 564
<211> 24
<212> DNA
<213> Artificial Sequence

<220>
<223> Primer

<400> 564
gtcgctatgt tttcgcaatc tgta 24

<210> 565
<211> 26
<212> DNA
<213> Artificial Sequence

<220>
<223> Primer

<400> 565

tggagtaaac ccaacaatag cagaag 26

<210> 566
<211> 24
<212> DNA
<213> Artificial Sequence

<220>
<223> Primer

<400> 566
catttgtaac gcaacagggc taat 24

<210> 567
<211> 27
<212> DNA
<213> Artificial Sequence

<220>
<223> Primer

<400> 567
cgaaaacata gcgaccacta tagagat 27

<210> 568
<211> 27
<212> DNA
<213> Artificial Sequence

<220>
<223> Primer

<400> 568
gtatttacaa ctcttgccac agaagga 27

<210> 569
<211> 26
<212> DNA
<213> Artificial Sequence

<220>
<223> Primer

<400> 569
cgccctagtg agtaacaact gtattt 26

<210> 570
<211> 27
<212> DNA
<213> Artificial Sequence

<220>
<223> Primer

<400> 570
ggaggattgt aggataaaat ggatgct 27

<210> 571

<211> 22
<212> DNA
<213> Artificial Sequence

<220>
<223> Primer

<400> 571
gaggacgtta gggacaatgt gt 22

<210> 572
<211> 23
<212> DNA
<213> Artificial Sequence

<220>
<223> Primer

<400> 572
ccctgtgata aaggacgcga ttt 23

<210> 573
<211> 26
<212> DNA
<213> Artificial Sequence

<220>
<223> Primer

<400> 573
cgtatgcatg ggtattggta tttgtg 26

<210> 574
<211> 28
<212> DNA
<213> Artificial Sequence

<220>
<223> Primer

<400> 574
catgtatatg caatagtaac atgggcaa 28

<210> 575
<211> 26
<212> DNA
<213> Artificial Sequence

<220>
<223> Primer

<400> 575
gccacaagtg tctatttttg ttgatg 26

<210> 576
<211> 23
<212> DNA
<213> Artificial Sequence

<220>
<223> Primer

<400> 576
tttagacact gggacaggtg gta 23

<210> 577
<211> 26
<212> DNA
<213> Artificial Sequence

<220>
<223> Primer

<400> 577
cagatgagga ggatgtcata gacagt 26

<210> 578
<211> 28
<212> DNA
<213> Artificial Sequence

<220>
<223> Primer

<400> 578
cattaacagc tcttgcaata tgcgaata 28

<210> 579
<211> 19
<212> DNA
<213> Artificial Sequence

<220>
<223> Primer

<400> 579
cagctgcatg cacaaacca 19

<210> 580
<211> 24
<212> DNA
<213> Artificial Sequence

<220>
<223> Primer

<400> 580
ggtgatccag actctgacct tttg 24

<210> 581
<211> 27
<212> DNA
<213> Artificial Sequence

<220>
<223> Primer

<400> 581
caacgtttaa atgtgtgtca ggacaaa 27

<210> 582
<211> 19
<212> DNA
<213> Artificial Sequence

<220>
<223> Primer

<400> 582
aaatacggct gcaccgagt 19

<210> 583
<211> 27
<212> DNA
<213> Artificial Sequence

<220>
<223> Primer

<400> 583
caacgtttaa atgtgtgtca ggacaaa 27

<210> 584
<211> 21
<212> DNA
<213> Artificial Sequence

<220>
<223> Primer

<400> 584
catggttcca ccagcgttat t 21

<210> 585
<211> 19
<212> DNA
<213> Artificial Sequence

<220>
<223> Primer

<400> 585
cagctgcatg cacaaacca 19

<210> 586
<211> 21
<212> DNA
<213> Artificial Sequence

<220>
<223> Primer

<400> 586
catggttcca ccagcgttat t 21

<210> 587
<211> 19
<212> DNA
<213> Artificial Sequence

<220>
<223> Primer

<400> 587
cagctgcatg cacaaacca 19

<210> 588
<211> 23
<212> DNA
<213> Artificial Sequence

<220>
<223> Primer

<400> 588
tttagacact gggacaggtg gta 23

<210> 589
<211> 27
<212> DNA
<213> Artificial Sequence

<220>
<223> Primer

<400> 589
caacgtttaa atgtgtgtca ggacaaa 27

<210> 590
<211> 22
<212> DNA
<213> Artificial Sequence

<220>
<223> Primer

<400> 590
atcatgatgg ctgtatgtgc ca 22

<210> 591
<211> 22
<212> DNA
<213> Artificial Sequence

<220>
<223> Primer

<400> 591
gcgggtatgg caatactgaa gt 22

<210> 592
<211> 27
<212> DNA

<213> Artificial Sequence

<220>
<223> Primer

<400> 592
tggagtttca ttctctcgtt cactatg 27

<210> 593
<211> 22
<212> DNA
<213> Artificial Sequence

<220>
<223> Primer

<400> 593
gcgggtatgg caatactgaa gt 22

<210> 594
<211> 26
<212> DNA
<213> Artificial Sequence

<220>
<223> Primer

<400> 594
cgttgagaaa gagtctccat cgtttt 26

<210> 595
<211> 23
<212> DNA
<213> Artificial Sequence

<220>
<223> Primer

<400> 595
cggcattgga aataccctac gat 23

<210> 596
<211> 24
<212> DNA
<213> Artificial Sequence

<220>
<223> Primer

<400> 596
gaattcgatg tggtggtgtt gttg 24

<210> 597
<211> 27
<212> DNA
<213> Artificial Sequence

<220>

<223> Primer

<400> 597
caacgtttaa atgtgtgtca ggacaaa 27

<210> 598
<211> 19
<212> DNA
<213> Artificial Sequence

<220>
<223> Primer

<400> 598
ctgagaagcc ctgcccttc 19

<210> 599
<211> 23
<212> DNA
<213> Artificial Sequence

<220>
<223> Primer

<400> 599
cggcattgga aataccctac gat 23

<210> 600
<211> 28
<212> DNA
<213> Artificial Sequence

<220>
<223> Primer

<400> 600
cattaacagc tcttgcaata tgcgaata 28

<210> 601
<211> 27
<212> DNA
<213> Artificial Sequence

<220>
<223> Primer

<400> 601
caacgtttaa atgtgtgtca ggacaaa 27

<210> 602
<211> 24
<212> DNA
<213> Artificial Sequence

<220>
<223> Primer

<400> 602

ggtgatccag actctgacct tttg 24

<210> 603
<211> 19
<212> DNA
<213> Artificial Sequence

<220>
<223> Primer

<400> 603
aatcgtgtgc cccaactgt 19

<210> 604
<211> 21
<212> DNA
<213> Artificial Sequence

<220>
<223> Primer

<400> 604
catggttcca ccagcgttat t 21

<210> 605
<211> 28
<212> DNA
<213> Artificial Sequence

<220>
<223> Primer

<400> 605
ctggtggttt ttacatttcc aaatccat 28

<210> 606
<211> 26
<212> DNA
<213> Artificial Sequence

<220>
<223> Primer

<400> 606
cgttgagaaa gagtctccat cgtttt 26

<210> 607
<211> 19
<212> DNA
<213> Artificial Sequence

<220>
<223> Primer

<400> 607
aatcgtgtgc cccaactgt 19

<210> 608

<211> 23
<212> DNA
<213> Artificial Sequence

<220>
<223> Primer

<400> 608
tttagacact gggacaggtg gta 23

<210> 609
<211> 23
<212> DNA
<213> Artificial Sequence

<220>
<223> Primer

<400> 609
cggcattgga aataccctac gat 23

<210> 610
<211> 24
<212> DNA
<213> Artificial Sequence

<220>
<223> Primer

<400> 610
ttttcttctg gacacaacgg tctt 24

<210> 611
<211> 22
<212> DNA
<213> Artificial Sequence

<220>
<223> Primer

<400> 611
gcgggtatgg caatactgaa gt 22

<210> 612
<211> 28
<212> DNA
<213> Artificial Sequence

<220>
<223> Primer

<400> 612
aatgtaaaaa ccaccagtct gctatgta 28

<210> 613
<211> 23
<212> DNA
<213> Artificial Sequence

<220>
<223> Primer

<400> 613
cggcattgga aataccctac gat 23

<210> 614
<211> 26
<212> DNA
<213> Artificial Sequence

<220>
<223> Primer

<400> 614
cgttgagaaa gagtctccat cgtttt 26

<210> 615
<211> 19
<212> DNA
<213> Artificial Sequence

<220>
<223> Primer

<400> 615
aatcgtgtgc cccaactgt 19

<210> 616
<211> 28
<212> DNA
<213> Artificial Sequence

<220>
<223> Primer

<400> 616
aatgtaaaaa ccaccagtct gctatgta 28

<210> 617
<211> 19
<212> DNA
<213> Artificial Sequence

<220>
<223> Primer

<400> 617
cagctgcatg cacaaacca 19

<210> 618
<211> 18
<212> DNA
<213> Artificial Sequence

<220>
<223> Primer

<400> 618
tctttgctcg cagctcgt 18

<210> 619
<211> 23
<212> DNA
<213> Artificial Sequence

<220>
<223> Primer

<400> 619
cggcattgga aataccctac gat 23

<210> 620
<211> 28
<212> DNA
<213> Artificial Sequence

<220>
<223> Primer

<400> 620
tcttaaacat tttgtacaca ctccgtgt 28

<210> 621
<211> 19
<212> DNA
<213> Artificial Sequence

<220>
<223> Primer

<400> 621
cagctgcatg cacaaacca 19

<210> 622
<211> 19
<212> DNA
<213> Artificial Sequence

<220>
<223> Primer

<400> 622
aaatacggct gcaccgagt 19

<210> 623
<211> 19
<212> DNA
<213> Artificial Sequence

<220>
<223> Primer

<400> 623
aatcgtgtgc cccaactgt 19

<210> 624
<211> 22
<212> DNA
<213> Artificial Sequence

<220>
<223> Primer

<400> 624
atcatgatgg ctgtatgtgc ca 22

<210> 625
<211> 19
<212> DNA
<213> Artificial Sequence

<220>
<223> Primer

<400> 625
aatcgtgtgc cccaactgt 19

<210> 626
<211> 19
<212> DNA
<213> Artificial Sequence

<220>
<223> Primer

<400> 626
ctgagaagcc ctgcccttc 19

<210> 627
<211> 19
<212> DNA
<213> Artificial Sequence

<220>
<223> Primer

<400> 627
aatcgtgtgc cccaactgt 19

<210> 628
<211> 24
<212> DNA
<213> Artificial Sequence

<220>
<223> Primer

<400> 628
cccctgtctg tctgtcaatt actg 24

<210> 629
<211> 19
<212> DNA

<213> Artificial Sequence

<220>
<223> Primer

<400> 629
aatcgtgtgc cccaactgt 19

<210> 630
<211> 26
<212> DNA
<213> Artificial Sequence

<220>
<223> Primer

<400> 630
cgttgagaaa gagtctccat cgtttt 26

<210> 631
<211> 19
<212> DNA
<213> Artificial Sequence

<220>
<223> Primer

<400> 631
cagctgcatg cacaaacca 19

<210> 632
<211> 28
<212> DNA
<213> Artificial Sequence

<220>
<223> Primer

<400> 632
gccatgtaga tgacacttgt tcatacaa 28

<210> 633
<211> 23
<212> DNA
<213> Artificial Sequence

<220>
<223> Primer

<400> 633
cggcattgga aataccctac gat 23

<210> 634
<211> 25
<212> DNA
<213> Artificial Sequence

<220>

<223> Primer

<400> 634
acatagtctt gcaacgtagg tgttt 25

<210> 635
<211> 32
<212> DNA
<213> Artificial Sequence

<220>
<223> Primer

<400> 635
ggaacaacat tagaaaaatt gacaaacaaa gg 32

<210> 636
<211> 24
<212> DNA
<213> Artificial Sequence

<220>
<223> Primer

<400> 636
ttttcttctg gacacaacgg tctt 24

<210> 637
<211> 23
<212> DNA
<213> Artificial Sequence

<220>
<223> Primer

<400> 637
cggcattgga aataccctac gat 23

<210> 638
<211> 28
<212> DNA
<213> Artificial Sequence

<220>
<223> Primer

<400> 638
aatgtaaaaa ccaccagtct gctatgt 28

<210> 639
<211> 19
<212> DNA
<213> Artificial Sequence

<220>
<223> Primer

<400> 639

cagctgcatg cacaaacca 19

<210> 640
<211> 22
<212> DNA
<213> Artificial Sequence

<220>
<223> Primer

<400> 640
atcatgatgg ctgtatgtgc ca 22

<210> 641
<211> 19
<212> DNA
<213> Artificial Sequence

<220>
<223> Primer

<400> 641
aatcgtgtgc cccaactgt 19

<210> 642
<211> 18
<212> DNA
<213> Artificial Sequence

<220>
<223> Primer

<400> 642
tctttgctcg cagctcgt 18

<210> 643
<211> 23
<212> DNA
<213> Artificial Sequence

<220>
<223> Primer

<400> 643
cactagatgg caaccctgta tct 23

<210> 644
<211> 28
<212> DNA
<213> Artificial Sequence

<220>
<223> Primer

<400> 644
aatgtaaaaa ccaccagtct gctatgta 28

<210> 645

<211> 19
<212> DNA
<213> Artificial Sequence

<220>
<223> Primer

<400> 645
aatcgtgtgc cccaactgt 19

<210> 646
<211> 24
<212> DNA
<213> Artificial Sequence

<220>
<223> Primer

<400> 646
gaattcgatg tggtggtgtt gttg 24

<210> 647
<211> 19
<212> DNA
<213> Artificial Sequence

<220>
<223> Primer

<400> 647
aatcgtgtgc cccaactgt 19

<210> 648
<211> 24
<212> DNA
<213> Artificial Sequence

<220>
<223> Primer

<400> 648
ggtgatccag actctgacct tttg 24

<210> 649
<211> 19
<212> DNA
<213> Artificial Sequence

<220>
<223> Primer

<400> 649
catgcgggtg gtcaggtaa 19

<210> 650
<211> 24
<212> DNA
<213> Artificial Sequence

<220>
<223> Primer

<400> 650
gaattcgatg tggtggtgtt gttg 24

<210> 651
<211> 19
<212> DNA
<213> Artificial Sequence

<220>
<223> Primer

<400> 651
aatcgtgtgc cccaactgt 19

<210> 652
<211> 19
<212> DNA
<213> Artificial Sequence

<220>
<223> Primer

<400> 652
aaatacggct gcaccgagt 19

<210> 653
<211> 25
<212> DNA
<213> Artificial Sequence

<220>
<223> Primer

<400> 653
gaaacgattc cacaacatag gagga 25

<210> 654
<211> 25
<212> DNA
<213> Artificial Sequence

<220>
<223> Primer

<400> 654
acatagtctt gcaacgtagg tgttt 25

<210> 655
<211> 22
<212> DNA
<213> Artificial Sequence

<220>
<223> Primer

<400> 655
gcgggtatgg caatactgaa gt 22

<210> 656
<211> 24
<212> DNA
<213> Artificial Sequence

<220>
<223> Primer

<400> 656
gaattcgatg tggtggtgtt gttg 24

<210> 657
<211> 19
<212> DNA
<213> Artificial Sequence

<220>
<223> Primer

<400> 657
cagctgcatg cacaaacca 19

<210> 658
<211> 19
<212> DNA
<213> Artificial Sequence

<220>
<223> Primer

<400> 658
ctgagaagcc ctgcccttc 19

<210> 659
<211> 27
<212> DNA
<213> Artificial Sequence

<220>
<223> Primer

<400> 659
caacgtttaa atgtgtgtca ggacaaa 27

<210> 660
<211> 18
<212> DNA
<213> Artificial Sequence

<220>
<223> Primer

<400> 660
tctttgctcg cagctcgt 18

<210> 661
<211> 25
<212> DNA
<213> Artificial Sequence

<220>
<223> Primer

<400> 661
tgtgtgtgtt gtgtatgttg tcctt 25

<210> 662
<211> 26
<212> DNA
<213> Artificial Sequence

<220>
<223> Primer

<400> 662
caacttttac tatggcgtga caccta 26

<210> 663
<211> 20
<212> DNA
<213> Artificial Sequence

<220>
<223> Primer

<400> 663
gcttagtttg ggcctgtgtt 20

<210> 664
<211> 27
<212> DNA
<213> Artificial Sequence

<220>
<223> Primer

<400> 664
accaccggca tatctattag agttttc 27

<210> 665
<211> 25
<212> DNA
<213> Artificial Sequence

<220>
<223> Primer

<400> 665
gcattgtgct atgctttttg ctttg 25

<210> 666
<211> 25
<212> DNA

<213> Artificial Sequence

<220>
<223> Primer

<400> 666
acaacgtaat ggagaggttg caata 25

<210> 667
<211> 27
<212> DNA
<213> Artificial Sequence

<220>
<223> Primer

<400> 667
gtgaaacacc agaatggata gaaagac 27

<210> 668
<211> 25
<212> DNA
<213> Artificial Sequence

<220>
<223> Primer

<400> 668
tgcacatgca ttactatcac tgtca 25

<210> 669
<211> 23
<212> DNA
<213> Artificial Sequence

<220>
<223> Primer

<400> 669
acgtactgca actaactgca caa 23

<210> 670
<211> 24
<212> DNA
<213> Artificial Sequence

<220>
<223> Primer

<400> 670
ggtgatccag actctgacct tttg 24

<210> 671
<211> 23
<212> DNA
<213> Artificial Sequence

<220>

<223> Primer

<400> 671
acgtactgca actaactgca caa 23

<210> 672
<211> 21
<212> DNA
<213> Artificial Sequence

<220>
<223> Primer

<400> 672
catggttcca ccagcgttat t 21

<210> 673
<211> 25
<212> DNA
<213> Artificial Sequence

<220>
<223> Primer

<400> 673
gatgagctag aagacagcgg atatg 25

<210> 674
<211> 21
<212> DNA
<213> Artificial Sequence

<220>
<223> Primer

<400> 674
gtggtggtcg gttatcgttg t 21

<210> 675
<211> 19
<212> DNA
<213> Artificial Sequence

<220>
<223> Primer

<400> 675
gtgccctacc tgtgcacaa 19

<210> 676
<211> 27
<212> DNA
<213> Artificial Sequence

<220>
<223> Primer

<400> 676

ttcttctctc tatgactgct tctacct 27

<210> 677
<211> 28
<212> DNA
<213> Artificial Sequence

<220>
<223> Primer

<400> 677
tgtgaaacat agggcattag tgcaatta 28

<210> 678
<211> 28
<212> DNA
<213> Artificial Sequence

<220>
<223> Primer

<400> 678
catacactgg gttaccattt tcatcaaa 28

<210> 679
<211> 19
<212> DNA
<213> Artificial Sequence

<220>
<223> Primer

<400> 679
gtgccctacc tgtgcacaa 19

<210> 680
<211> 22
<212> DNA
<213> Artificial Sequence

<220>
<223> Primer

<400> 680
atcatgatgg ctgtatgtgc ca 22

<210> 681
<211> 19
<212> DNA
<213> Artificial Sequence

<220>
<223> Primer

<400> 681
gtgccctacc tgtgcacaa 19

<210> 682

<211> 19
<212> DNA
<213> Artificial Sequence

<220>
<223> Primer

<400> 682
ctgagaagcc ctgcccttc 19

<210> 683
<211> 23
<212> DNA
<213> Artificial Sequence

<220>
<223> Primer

<400> 683
acgtactgca actaactgca caa 23

<210> 684
<211> 27
<212> DNA
<213> Artificial Sequence

<220>
<223> Primer

<400> 684
atcagtgctg acaactttag atacagg 27

<210> 685
<211> 23
<212> DNA
<213> Artificial Sequence

<220>
<223> Primer

<400> 685
acgtactgca actaactgca caa 23

<210> 686
<211> 18
<212> DNA
<213> Artificial Sequence

<220>
<223> Primer

<400> 686
tctttgctcg cagctcgt 18

<210> 687
<211> 28
<212> DNA
<213> Artificial Sequence

<220>
<223> Primer

<400> 687
agcattggag acaactatac acaacatt 28

<210> 688
<211> 26
<212> DNA
<213> Artificial Sequence

<220>
<223> Primer

<400> 688
catattcctt taacgttggc ttgtgt 26

<210> 689
<211> 33
<212> DNA
<213> Artificial Sequence

<220>
<223> Primer

<400> 689
attctgtata tggaaataca ttagaacaaa cag 33

<210> 690
<211> 28
<212> DNA
<213> Artificial Sequence

<220>
<223> Primer

<400> 690
tcgtttgttt aaatccacat gtcgtttt 28

<210> 691
<211> 23
<212> DNA
<213> Artificial Sequence

<220>
<223> Primer

<400> 691
acgtactgca actaactgca caa 23

<210> 692
<211> 19
<212> DNA
<213> Artificial Sequence

<220>
<223> Primer

<400> 692
ctgagaagcc ctgcccttc 19

<210> 693
<211> 23
<212> DNA
<213> Artificial Sequence

<220>
<223> Primer

<400> 693
acgtactgca actaactgca caa 23

<210> 694
<211> 19
<212> DNA
<213> Artificial Sequence

<220>
<223> Primer

<400> 694
aaatacggct gcaccgagt 19

<210> 695
<211> 28
<212> DNA
<213> Artificial Sequence

<220>
<223> Primer

<400> 695
agcattggag acaactatac acaacatt 28

<210> 696
<211> 28
<212> DNA
<213> Artificial Sequence

<220>
<223> Primer

<400> 696
tcgtttgttt aaatccacat gtcgtttt 28

<210> 697
<211> 29
<212> DNA
<213> Artificial Sequence

<220>
<223> Primer

<400> 697
gtgcaggaga aaatactaga tctttacga 29

<210> 698
<211> 19
<212> DNA
<213> Artificial Sequence

<220>
<223> Primer

<400> 698
ctgagaagcc ctgcccttc 19

<210> 699
<211> 25
<212> DNA
<213> Artificial Sequence

<220>
<223> Primer

<400> 699
gatgagctag aagacagcgg atatg 25

<210> 700
<211> 20
<212> DNA
<213> Artificial Sequence

<220>
<223> Primer

<400> 700
catcccccac cccactagat 20

<210> 701
<211> 23
<212> DNA
<213> Artificial Sequence

<220>
<223> Primer

<400> 701
acgtactgca actaactgca caa 23

<210> 702
<211> 22
<212> DNA
<213> Artificial Sequence

<220>
<223> Primer

<400> 702
atcatgatgg ctgtatgtgc ca 22

<210> 703
<211> 29
<212> DNA

<213> Artificial Sequence

<220>
<223> Primer

<400> 703
gtgcaggaga aaatactaga tctttacga 29

<210> 704
<211> 22
<212> DNA
<213> Artificial Sequence

<220>
<223> Primer

<400> 704
atcatgatgg ctgtatgtgc ca 22

<210> 705
<211> 28
<212> DNA
<213> Artificial Sequence

<220>
<223> Primer

<400> 705
agcattggag acaactatac acaacatt 28

<210> 706
<211> 28
<212> DNA
<213> Artificial Sequence

<220>
<223> Primer

<400> 706
cgcaaacaca gtttacatat tccaaatg 28

<210> 707
<211> 19
<212> DNA
<213> Artificial Sequence

<220>
<223> Primer

<400> 707
gtgccctacc tgtgcacaa 19

<210> 708
<211> 18
<212> DNA
<213> Artificial Sequence

<220>

<223> Primer

<400> 708
tctttgctcg cagctcgt 18

<210> 709
<211> 19
<212> DNA
<213> Artificial Sequence

<220>
<223> Primer

<400> 709
gtgccctacc tgtgcacaa 19

<210> 710
<211> 19
<212> DNA
<213> Artificial Sequence

<220>
<223> Primer

<400> 710
aaatacggct gcaccgagt 19

<210> 711
<211> 29
<212> DNA
<213> Artificial Sequence

<220>
<223> Primer

<400> 711
gtgcaggaga aaatactaga tctttacga 29

<210> 712
<211> 24
<212> DNA
<213> Artificial Sequence

<220>
<223> Primer

<400> 712
ggtgatccag actctgacct tttg 24

<210> 713
<211> 28
<212> DNA
<213> Artificial Sequence

<220>
<223> Primer

<400> 713

agcattggag acaactatac acaacatt 28

<210> 714
<211> 28
<212> DNA
<213> Artificial Sequence

<220>
<223> Primer

<400> 714
catacactgg gttaccattt tcatcaaa 28

<210> 715
<211> 29
<212> DNA
<213> Artificial Sequence

<220>
<223> Primer

<400> 715
gtgcaggaga aaatactaga tctttacga 29

<210> 716
<211> 21
<212> DNA
<213> Artificial Sequence

<220>
<223> Primer

<400> 716
catggttcca ccagcgttat t 21

<210> 717
<211> 19
<212> DNA
<213> Artificial Sequence

<220>
<223> Primer

<400> 717
gtgccctacc tgtgcacaa 19

<210> 718
<211> 28
<212> DNA
<213> Artificial Sequence

<220>
<223> Primer

<400> 718
tgatatttcc tccatggttt tccttgtc 28

<210> 719

<211> 28
<212> DNA
<213> Artificial Sequence

<220>
<223> Primer

<400> 719
agcattggag acaactatac acaacatt 28

<210> 720
<211> 21
<212> DNA
<213> Artificial Sequence

<220>
<223> Primer

<400> 720
gtggtggtcg gttatcgttg t 21

<210> 721
<211> 25
<212> DNA
<213> Artificial Sequence

<220>
<223> Primer

<400> 721
gatgagctag aagacagcgg atatg 25

<210> 722
<211> 28
<212> DNA
<213> Artificial Sequence

<220>
<223> Primer

<400> 722
catacactgg gttaccattt tcatcaaa 28

<210> 723
<211> 19
<212> DNA
<213> Artificial Sequence

<220>
<223> Primer

<400> 723
gtgccctacc tgtgcacaa 19

<210> 724
<211> 24
<212> DNA
<213> Artificial Sequence

<220>
<223> Primer

<400> 724
ggtgatccag actctgacct tttg 24

<210> 725
<211> 29
<212> DNA
<213> Artificial Sequence

<220>
<223> Primer

<400> 725
gtgcaggaga aaatactaga tctttacga 29

<210> 726
<211> 18
<212> DNA
<213> Artificial Sequence

<220>
<223> Primer

<400> 726
tctttgctcg cagctcgt 18

<210> 727
<211> 19
<212> DNA
<213> Artificial Sequence

<220>
<223> Primer

<400> 727
gtgccctacc tgtgcacaa 19

<210> 728
<211> 21
<212> DNA
<213> Artificial Sequence

<220>
<223> Primer

<400> 728
gtggtggtcg gttatcgttg t 21

<210> 729
<211> 19
<212> DNA
<213> Artificial Sequence

<220>
<223> Primer

<400> 729
gtgccctacc tgtgcacaa 19

<210> 730
<211> 21
<212> DNA
<213> Artificial Sequence

<220>
<223> Primer

<400> 730
catggttcca ccagcgttat t 21

<210> 731
<211> 29
<212> DNA
<213> Artificial Sequence

<220>
<223> Primer

<400> 731
gtgcaggaga aaatactaga tctttacga 29

<210> 732
<211> 19
<212> DNA
<213> Artificial Sequence

<220>
<223> Primer

<400> 732
aaatacggct gcaccgagt 19

<210> 733
<211> 25
<212> DNA
<213> Artificial Sequence

<220>
<223> Primer

<400> 733
gatgagctag aagacagcgg atatg 25

<210> 734
<211> 28
<212> DNA
<213> Artificial Sequence

<220>
<223> Primer

<400> 734
tgatatttcc tccatggttt tccttgtc 28

<210> 735
<211> 28
<212> DNA
<213> Artificial Sequence

<220>
<223> Primer

<400> 735
ggatgctgca aagtattcta aaacacaa 28

<210> 736
<211> 21
<212> DNA
<213> Artificial Sequence

<220>
<223> Primer

<400> 736
gtggtggtcg gttatcgttg t 21

<210> 737
<211> 27
<212> DNA
<213> Artificial Sequence

<220>
<223> Primer

<400> 737
cgatttcata atatttcggg tcgttgg 27

<210> 738
<211> 26
<212> DNA
<213> Artificial Sequence

<220>
<223> Primer

<400> 738
catattcctt taacgttggc ttgtgt 26

<210> 739
<211> 19
<212> DNA
<213> Artificial Sequence

<220>
<223> Primer

<400> 739
gtgccctacc tgtgcacaa 19

<210> 740
<211> 27
<212> DNA

<213> Artificial Sequence

<220>
<223> Primer

<400> 740
atcagtgctg acaactttag atacagg 27

<210> 741
<211> 23
<212> DNA
<213> Artificial Sequence

<220>
<223> Primer

<400> 741
acgtactgca actaactgca caa 23

<210> 742
<211> 27
<212> DNA
<213> Artificial Sequence

<220>
<223> Primer

<400> 742
gccaggtgga tgacatagaa ctataca 27

<210> 743
<211> 23
<212> DNA
<213> Artificial Sequence

<220>
<223> Primer

<400> 743
ttgttgtaga cggtgctgac ttt 23

<210> 744
<211> 27
<212> DNA
<213> Artificial Sequence

<220>
<223> Primer

<400> 744
atcagtgctg acaactttag atacagg 27

<210> 745
<211> 19
<212> DNA
<213> Artificial Sequence

<220>

<223> Primer

<400> 745
gtgccctacc tgtgcacaa 19

<210> 746
<211> 28
<212> DNA
<213> Artificial Sequence

<220>
<223> Primer

<400> 746
catacactgg gttaccattt tcatcaaa 28

<210> 747
<211> 28
<212> DNA
<213> Artificial Sequence

<220>
<223> Primer

<400> 747
agcattggag acaactatac acaacatt 28

<210> 748
<211> 28
<212> DNA
<213> Artificial Sequence

<220>
<223> Primer

<400> 748
tgatatttcc tccatggttt tccttgtc 28

<210> 749
<211> 25
<212> DNA
<213> Artificial Sequence

<220>
<223> Primer

<400> 749
ccatttctac ctatgcttgg ttgct 25

<210> 750
<211> 25
<212> DNA
<213> Artificial Sequence

<220>
<223> Primer

<400> 750

gttgtgtcat atgctgtgca tgaaa 25

<210> 751
<211> 19
<212> DNA
<213> Artificial Sequence

<220>
<223> Primer

<400> 751
cttgccctac cctgcattg 19

<210> 752
<211> 27
<212> DNA
<213> Artificial Sequence

<220>
<223> Primer

<400> 752
cggttaggca tacaaaatgg aggaaat 27

<210> 753
<211> 21
<212> DNA
<213> Artificial Sequence

<220>
<223> Primer

<400> 753
cggagccaaa catgtgcatt g 21

<210> 754
<211> 26
<212> DNA
<213> Artificial Sequence

<220>
<223> Primer

<400> 754
cgttatcata tgcccactgt accatt 26

<210> 755
<211> 27
<212> DNA
<213> Artificial Sequence

<220>
<223> Primer

<400> 755
catgtgtagg ccttgaaata ggtagag 27

<210> 756

<211> 22
<212> DNA
<213> Artificial Sequence

<220>
<223> Primer

<400> 756
cctattatca gcacccggtt gt 22

<210> 757
<211> 27
<212> DNA
<213> Artificial Sequence

<220>
<223> Primer

<400> 757
cgaggtagaa gaaagcatcc atgaaat 27

<210> 758
<211> 23
<212> DNA
<213> Artificial Sequence

<220>
<223> Primer

<400> 758
catactccat atggctggcc ttc 23

<210> 759
<211> 26
<212> DNA
<213> Artificial Sequence

<220>
<223> Primer

<400> 759
tctacatctg actgcacaaa caaaga 26

<210> 760
<211> 27
<212> DNA
<213> Artificial Sequence

<220>
<223> Primer

<400> 760
ccatctccat gtagatgaag catcttg 27

<210> 761
<211> 27
<212> DNA
<213> Artificial Sequence

<220>
<223> Primer

<400> 761
cgaggtagaa gaaagcatcc atgaaat 27

<210> 762
<211> 26
<212> DNA
<213> Artificial Sequence

<220>
<223> Primer

<400> 762
ggaaagcgtc tccatcattt tctttg 26

<210> 763
<211> 27
<212> DNA
<213> Artificial Sequence

<220>
<223> Primer

<400> 763
attacgagac tgatagcaca tgtttgt 27

<210> 764
<211> 24
<212> DNA
<213> Artificial Sequence

<220>
<223> Primer

<400> 764
ggtgatccag actctgacct tttg 24

<210> 765
<211> 26
<212> DNA
<213> Artificial Sequence

<220>
<223> Primer

<400> 765
tctacatctg actgcacaaa caaaga 26

<210> 766
<211> 24
<212> DNA
<213> Artificial Sequence

<220>
<223> Primer

<400> 766
ggtgatccag actctgacct tttg 24

<210> 767
<211> 23
<212> DNA
<213> Artificial Sequence

<220>
<223> Primer

<400> 767
cggctgttca cagagagcat aat 23

<210> 768
<211> 19
<212> DNA
<213> Artificial Sequence

<220>
<223> Primer

<400> 768
aaatacggct gcaccgagt 19

<210> 769
<211> 27
<212> DNA
<213> Artificial Sequence

<220>
<223> Primer

<400> 769
attacgagac tgatagcaca tgtttgt 27

<210> 770
<211> 21
<212> DNA
<213> Artificial Sequence

<220>
<223> Primer

<400> 770
catggttcca ccagcgttat t 21

<210> 771
<211> 26
<212> DNA
<213> Artificial Sequence

<220>
<223> Primer

<400> 771
gggtgacttt tatttacacc ctagtt 26

<210> 772
<211> 26
<212> DNA
<213> Artificial Sequence

<220>
<223> Primer

<400> 772
catcagtgct aacaacctta gacact 26

<210> 773
<211> 27
<212> DNA
<213> Artificial Sequence

<220>
<223> Primer

<400> 773
attacgagac tgatagcaca tgtttgt 27

<210> 774
<211> 18
<212> DNA
<213> Artificial Sequence

<220>
<223> Primer

<400> 774
tctttgctcg cagctcgt 18

<210> 775
<211> 26
<212> DNA
<213> Artificial Sequence

<220>
<223> Primer

<400> 775
tctacatctg actgcacaaa caaaga 26

<210> 776
<211> 26
<212> DNA
<213> Artificial Sequence

<220>
<223> Primer

<400> 776
catcagtgct aacaacctta gacact 26

<210> 777
<211> 23
<212> DNA

<213> Artificial Sequence

<220>
<223> Primer

<400> 777
cggctgttca cagagagcat aat 23

<210> 778
<211> 26
<212> DNA
<213> Artificial Sequence

<220>
<223> Primer

<400> 778
ggaaagcgtc tccatcattt tctttg 26

<210> 779
<211> 23
<212> DNA
<213> Artificial Sequence

<220>
<223> Primer

<400> 779
cggctgttca cagagagcat aat 23

<210> 780
<211> 25
<212> DNA
<213> Artificial Sequence

<220>
<223> Primer

<400> 780
cccgtacgtc tactaactac tgctt 25

<210> 781
<211> 27
<212> DNA
<213> Artificial Sequence

<220>
<223> Primer

<400> 781
attacgagac tgatagcaca tgtttgt 27

<210> 782
<211> 22
<212> DNA
<213> Artificial Sequence

<220>

<223> Primer

<400> 782
atcatgatgg ctgtatgtgc ca 22

<210> 783
<211> 23
<212> DNA
<213> Artificial Sequence

<220>
<223> Primer

<400> 783
cggctgttca cagagagcat aat 23

<210> 784
<211> 24
<212> DNA
<213> Artificial Sequence

<220>
<223> Primer

<400> 784
ggtgatccag actctgacct tttg 24

<210> 785
<211> 23
<212> DNA
<213> Artificial Sequence

<220>
<223> Primer

<400> 785
cggctgttca cagagagcat aat 23

<210> 786
<211> 26
<212> DNA
<213> Artificial Sequence

<220>
<223> Primer

<400> 786
catcagtgct aacaacctta gacact 26

<210> 787
<211> 23
<212> DNA
<213> Artificial Sequence

<220>
<223> Primer

<400> 787

cggctgttca cagagagcat aat 23

<210> 788
<211> 19
<212> DNA
<213> Artificial Sequence

<220>
<223> Primer

<400> 788
ctgagaagcc ctgcccttc 19

<210> 789
<211> 26
<212> DNA
<213> Artificial Sequence

<220>
<223> Primer

<400> 789
tctacatctg actgcacaaa caaaga 26

<210> 790
<211> 18
<212> DNA
<213> Artificial Sequence

<220>
<223> Primer

<400> 790
tctttgctcg cagctcgt 18

<210> 791
<211> 27
<212> DNA
<213> Artificial Sequence

<220>
<223> Primer

<400> 791
attacgagac tgatagcaca tgtttgt 27

<210> 792
<211> 19
<212> DNA
<213> Artificial Sequence

<220>
<223> Primer

<400> 792
aaatacggct gcaccgagt 19

<210> 793

<211> 27
<212> DNA
<213> Artificial Sequence

<220>
<223> Primer

<400> 793
cgaggtagaa gaaagcatcc atgaaat 27

<210> 794
<211> 28
<212> DNA
<213> Artificial Sequence

<220>
<223> Primer

<400> 794
tcattgtgaa atgtaaagac cactaccc 28

<210> 795
<211> 27
<212> DNA
<213> Artificial Sequence

<220>
<223> Primer

<400> 795
attacgagac tgatagcaca tgtttgt 27

<210> 796
<211> 19
<212> DNA
<213> Artificial Sequence

<220>
<223> Primer

<400> 796
ctgagaagcc ctgcccttc 19

<210> 797
<211> 25
<212> DNA
<213> Artificial Sequence

<220>
<223> Primer

<400> 797
catctactat catgcaggca gttct 25

<210> 798
<211> 27
<212> DNA
<213> Artificial Sequence

<220>
<223> Primer

<400> 798
actctgtatt gcaaaccaga taccttg 27

<210> 799
<211> 24
<212> DNA
<213> Artificial Sequence

<220>
<223> Primer

<400> 799
ggaaacccag tgtatgggct taat 24

<210> 800
<211> 26
<212> DNA
<213> Artificial Sequence

<220>
<223> Primer

<400> 800
ggaaagcgtc tccatcattt tctttg 26

<210> 801
<211> 23
<212> DNA
<213> Artificial Sequence

<220>
<223> Primer

<400> 801
cggctgttca cagagagcat aat 23

<210> 802
<211> 22
<212> DNA
<213> Artificial Sequence

<220>
<223> Primer

<400> 802
atcatgatgg ctgtatgtgc ca 22

<210> 803
<211> 26
<212> DNA
<213> Artificial Sequence

<220>
<223> Primer

<400> 803
tctacatctg actgcacaaa caaaga 26

<210> 804
<211> 21
<212> DNA
<213> Artificial Sequence

<220>
<223> Primer

<400> 804
catggttcca ccagcgttat t 21

<210> 805
<211> 26
<212> DNA
<213> Artificial Sequence

<220>
<223> Primer

<400> 805
tctacatctg actgcacaaa caaaga 26

<210> 806
<211> 22
<212> DNA
<213> Artificial Sequence

<220>
<223> Primer

<400> 806
atcatgatgg ctgtatgtgc ca 22

<210> 807
<211> 23
<212> DNA
<213> Artificial Sequence

<220>
<223> Primer

<400> 807
cggctgttca cagagagcat aat 23

<210> 808
<211> 18
<212> DNA
<213> Artificial Sequence

<220>
<223> Primer

<400> 808
tctttgctcg cagctcgt 18

<210> 809
<211> 26
<212> DNA
<213> Artificial Sequence

<220>
<223> Primer

<400> 809
tctacatctg actgcacaaa caaaga 26

<210> 810
<211> 19
<212> DNA
<213> Artificial Sequence

<220>
<223> Primer

<400> 810
ctgagaagcc ctgcccttc 19

<210> 811
<211> 23
<212> DNA
<213> Artificial Sequence

<220>
<223> Primer

<400> 811
cggctgttca cagagagcat aat 23

<210> 812
<211> 20
<212> DNA
<213> Artificial Sequence

<220>
<223> Primer

<400> 812
gctttggtat gggtctcggt 20

<210> 813
<211> 26
<212> DNA
<213> Artificial Sequence

<220>
<223> Primer

<400> 813
attatttgaa ctaccagaca gcggtt 26

<210> 814
<211> 20
<212> DNA

<213> Artificial Sequence

<220>
<223> Primer

<400> 814
gctttggtat gggtctcggt 20

<210> 815
<211> 23
<212> DNA
<213> Artificial Sequence

<220>
<223> Primer

<400> 815
cggctgttca cagagagcat aat 23

<210> 816
<211> 28
<212> DNA
<213> Artificial Sequence

<220>
<223> Primer

<400> 816
tcattgtgaa atgtaaagac cactaccc 28

<210> 817
<211> 26
<212> DNA
<213> Artificial Sequence

<220>
<223> Primer

<400> 817
attatttgaa ctaccagaca gcggtt 26

<210> 818
<211> 26
<212> DNA
<213> Artificial Sequence

<220>
<223> Primer

<400> 818
ggaaagcgtc tccatcattt tctttg 26

<210> 819
<211> 27
<212> DNA
<213> Artificial Sequence

<220>

<223> Primer

<400> 819
cgaggtagaa gaaagcatcc atgaaat 27

<210> 820
<211> 25
<212> DNA
<213> Artificial Sequence

<220>
<223> Primer

<400> 820
tccaccgatg ttatggaatc gtttt 25

<210> 821
<211> 26
<212> DNA
<213> Artificial Sequence

<220>
<223> Primer

<400> 821
tctacatctg actgcacaaa caaaga 26

<210> 822
<211> 19
<212> DNA
<213> Artificial Sequence

<220>
<223> Primer

<400> 822
aaatacggct gcaccgagt 19

<210> 823
<211> 23
<212> DNA
<213> Artificial Sequence

<220>
<223> Primer

<400> 823
cggctgttca cagagagcat aat 23

<210> 824
<211> 21
<212> DNA
<213> Artificial Sequence

<220>
<223> Primer

<400> 824

catggttcca ccagcgttat t 21

<210> 825
<211> 28
<212> DNA
<213> Artificial Sequence

<220>
<223> Primer

<400> 825
ggagaaacgt tagaaaaaca atgcaaca 28

<210> 826
<211> 25
<212> DNA
<213> Artificial Sequence

<220>
<223> Primer

<400> 826
tccaccgatg ttatggaatc gtttt 25

<210> 827
<211> 27
<212> DNA
<213> Artificial Sequence

<220>
<223> Primer

<400> 827
cgaggtagaa gaaagcatcc atgaaat 27

<210> 828
<211> 20
<212> DNA
<213> Artificial Sequence

<220>
<223> Primer

<400> 828
gctttggtat gggtctcggt 20

<210> 829
<211> 26
<212> DNA
<213> Artificial Sequence

<220>
<223> Primer

<400> 829
attatttgaa ctaccagaca gcggtt 26

<210> 830

<211> 27
<212> DNA
<213> Artificial Sequence

<220>
<223> Primer

<400> 830
gctactagag gttatactat ccccact 27

<210> 831
<211> 23
<212> DNA
<213> Artificial Sequence

<220>
<223> Primer

<400> 831
cggctgttca cagagagcat aat 23

<210> 832
<211> 27
<212> DNA
<213> Artificial Sequence

<220>
<223> Primer

<400> 832
actctgtatt gcaaaccaga taccttg 27

<210> 833
<211> 26
<212> DNA
<213> Artificial Sequence

<220>
<223> Primer

<400> 833
attatttgaa ctaccagaca gcggtt 26

<210> 834
<211> 28
<212> DNA
<213> Artificial Sequence

<220>
<223> Primer

<400> 834
tcattgtgaa atgtaaagac cactaccc 28

<210> 835
<211> 27
<212> DNA
<213> Artificial Sequence

<220>
<223> Primer

<400> 835
cattagtgca attaaaatgc ccacctt 27

<210> 836
<211> 28
<212> DNA
<213> Artificial Sequence

<220>
<223> Primer

<400> 836
tcattgtgaa atgtaaagac cactaccc 28

<210> 837
<211> 26
<212> DNA
<213> Artificial Sequence

<220>
<223> Primer

<400> 837
tctacatctg actgcacaaa caaaga 26

<210> 838
<211> 27
<212> DNA
<213> Artificial Sequence

<220>
<223> Primer

<400> 838
actctgtatt gcaaaccaga taccttg 27

<210> 839
<211> 26
<212> DNA
<213> Artificial Sequence

<220>
<223> Primer

<400> 839
aaaatatatg ggaagtgcat gtgggt 26

<210> 840
<211> 20
<212> DNA
<213> Artificial Sequence

<220>
<223> Primer

<400> 840
gctttggtat gggtctcggt 20

<210> 841
<211> 27
<212> DNA
<213> Artificial Sequence

<220>
<223> Primer

<400> 841
cgttcgctat tgctatctgt gtcatta 27

<210> 842
<211> 27
<212> DNA
<213> Artificial Sequence

<220>
<223> Primer

<400> 842
gccaaatatt gtgcatgagc gttaatc 27

<210> 843
<211> 24
<212> DNA
<213> Artificial Sequence

<220>
<223> Primer

<400> 843
aacattccta cctcagcaga acac 24

<210> 844
<211> 23
<212> DNA
<213> Artificial Sequence

<220>
<223> Primer

<400> 844
tgggtggacc acaagtatga aaa 23

<210> 845
<211> 27
<212> DNA
<213> Artificial Sequence

<220>
<223> Primer

<400> 845
ggtacagata acagggaatg catttct 27

<210> 846
<211> 26
<212> DNA
<213> Artificial Sequence

<220>
<223> Primer

<400> 846
gacattctcc tgcttttacc tggtta 26

<210> 847
<211> 27
<212> DNA
<213> Artificial Sequence

<220>
<223> Primer

<400> 847
gctatgtatt tcagctgcaa gtatgct 27

<210> 848
<211> 25
<212> DNA
<213> Artificial Sequence

<220>
<223> Primer

<400> 848
cattctggtg tttctccatc aacct 25

<210> 849
<211> 18
<212> DNA
<213> Artificial Sequence

<220>
<223> Primer

<400> 849
cgtggtgtgc aactgcaa 18

<210> 850
<211> 28
<212> DNA
<213> Artificial Sequence

<220>
<223> Primer

<400> 850
ctgttttggt caaatggaaa tgcattag 28

<210> 851
<211> 25
<212> DNA

<213> Artificial Sequence

<220>
<223> Primer

<400> 851
gcaataacca ttcagggttc caatt 25

<210> 852
<211> 22
<212> DNA
<213> Artificial Sequence

<220>
<223> Primer

<400> 852
agtattgaca accttcgcca ca 22

<210> 853
<211> 20
<212> DNA
<213> Artificial Sequence

<220>
<223> Primer

<400> 853
ggtgtattcc gtgccagaca 20

<210> 854
<211> 19
<212> DNA
<213> Artificial Sequence

<220>
<223> Primer

<400> 854
gtacactgcc gccatgttc 19

<210> 855
<211> 20
<212> DNA
<213> Artificial Sequence

<220>
<223> Primer

<400> 855
ggtgtattcc gtgccagaca 20

<210> 856
<211> 21
<212> DNA
<213> Artificial Sequence

<220>

<223> Primer

<400> 856
ggtcgcggtg gtgtttgata a 21

<210> 857
<211> 25
<212> DNA
<213> Artificial Sequence

<220>
<223> Primer

<400> 857
caccaccttg caggacatta caata 25

<210> 858
<211> 28
<212> DNA
<213> Artificial Sequence

<220>
<223> CTGTTTTGGTCAAATGGAAATGCATTAG

<400> 858
ctgttttggt caaatggaaa tgcattag 28

<210> 859
<211> 22
<212> DNA
<213> Artificial Sequence

<220>
<223> Primer

<400> 859
cacagtaaca gtacaggcca ca 22

<210> 860
<211> 21
<212> DNA
<213> Artificial Sequence

<220>
<223> Primer

<400> 860
catggttcca ccagcgttat t 21

<210> 861
<211> 22
<212> DNA
<213> Artificial Sequence

<220>
<223> Primer

<400> 861

cacagtaaca gtacaggcca ca 22

<210> 862
<211> 22
<212> DNA
<213> Artificial Sequence

<220>
<223> Primer

<400> 862
atcatgatgg ctgtatgtgc ca 22

<210> 863
<211> 26
<212> DNA
<213> Artificial Sequence

<220>
<223> Primer

<400> 863
catgcagtta atcaccaaca tcaact 26

<210> 864
<211> 23
<212> DNA
<213> Artificial Sequence

<220>
<223> Primer

<400> 864
tgctgtagtt gtcgcagagt atc 23

<210> 865
<211> 25
<212> DNA
<213> Artificial Sequence

<220>
<223> Primer

<400> 865
caccaccttg caggacatta caata 25

<210> 866
<211> 27
<212> DNA
<213> Artificial Sequence

<220>
<223> Primer

<400> 866
ctgtcctgta tagcttcctg ctatttt 27

<210> 867

<211> 18
<212> DNA
<213> Artificial Sequence

<220>
<223> Primer

<400> 867
cgtggtgtgc aactgcaa 18

<210> 868
<211> 23
<212> DNA
<213> Artificial Sequence

<220>
<223> Primer

<400> 868
cactgtgtcg cctgtttgtt tat 23

<210> 869
<211> 18
<212> DNA
<213> Artificial Sequence

<220>
<223> Primer

<400> 869
cgtggtgtgc aactgcaa 18

<210> 870
<211> 19
<212> DNA
<213> Artificial Sequence

<220>
<223> Primer

<400> 870
aaatacggct gcaccgagt 19

<210> 871
<211> 31
<212> DNA
<213> Artificial Sequence

<220>
<223> Primer

<400> 871
attagatggg tatgcaataa gtttagatag g 31

<210> 872
<211> 28
<212> DNA
<213> Artificial Sequence

<220>
<223> Primer

<400> 872
ctgttttggt caaatggaaa tgcattag 28

<210> 873
<211> 26
<212> DNA
<213> Artificial Sequence

<220>
<223> Primer

<400> 873
acaacgttta aatgtgttac aggaca 26

<210> 874
<211> 19
<212> DNA
<213> Artificial Sequence

<220>
<223> Primer

<400> 874
aaatacggct gcaccgagt 19

<210> 875
<211> 26
<212> DNA
<213> Artificial Sequence

<220>
<223> Primer

<400> 875
acaacgttta aatgtgttac aggaca 26

<210> 876
<211> 22
<212> DNA
<213> Artificial Sequence

<220>
<223> Primer

<400> 876
atcatgatgg ctgtatgtgc ca 22

<210> 877
<211> 18
<212> DNA
<213> Artificial Sequence

<220>
<223> Primer

<400> 877
cgtggtgtgc aactgcaa 18

<210> 878
<211> 22
<212> DNA
<213> Artificial Sequence

<220>
<223> Primer

<400> 878
atcatgatgg ctgtatgtgc ca 22

<210> 879
<211> 18
<212> DNA
<213> Artificial Sequence

<220>
<223> Primer

<400> 879
cgtggtgtgc aactgcaa 18

<210> 880
<211> 18
<212> DNA
<213> Artificial Sequence

<220>
<223> Primer

<400> 880
tctttgctcg cagctcgt 18

<210> 881
<211> 18
<212> DNA
<213> Artificial Sequence

<220>
<223> Primer

<400> 881
cgtggtgtgc aactgcaa 18

<210> 882
<211> 24
<212> DNA
<213> Artificial Sequence

<220>
<223> Primer

<400> 882
ggtgatccag actctgacct tttg 24

<210> 883
<211> 26
<212> DNA
<213> Artificial Sequence

<220>
<223> Primer

<400> 883
acaacgttta aatgtgttac aggaca 26

<210> 884
<211> 18
<212> DNA
<213> Artificial Sequence

<220>
<223> Primer

<400> 884
tctttgctcg cagctcgt 18

<210> 885
<211> 18
<212> DNA
<213> Artificial Sequence

<220>
<223> Primer

<400> 885
cgtggtgtgc aactgcaa 18

<210> 886
<211> 19
<212> DNA
<213> Artificial Sequence

<220>
<223> Primer

<400> 886
ctgagaagcc ctgcccttc 19

<210> 887
<211> 18
<212> DNA
<213> Artificial Sequence

<220>
<223> Primer

<400> 887
cgtggtgtgc aactgcaa 18

<210> 888
<211> 22
<212> DNA

<213> Artificial Sequence

<220>
<223> Primer

<400> 888
agtattgaca accttcgcca ca 22

<210> 889
<211> 22
<212> DNA
<213> Artificial Sequence

<220>
<223> Primer

<400> 889
cacagtaaca gtacaggcca ca 22

<210> 890
<211> 25
<212> DNA
<213> Artificial Sequence

<220>
<223> Primer

<400> 890
cgtatccaat gccaggtaca tgaaa 25

<210> 891
<211> 22
<212> DNA
<213> Artificial Sequence

<220>
<223> Primer

<400> 891
cacagtaaca gtacaggcca ca 22

<210> 892
<211> 22
<212> DNA
<213> Artificial Sequence

<220>
<223> Primer

<400> 892
agtattgaca accttcgcca ca 22

<210> 893
<211> 22
<212> DNA
<213> Artificial Sequence

<220>

<223> Primer

<400> 893
cacagtaaca gtacaggcca ca 22

<210> 894
<211> 18
<212> DNA
<213> Artificial Sequence

<220>
<223> Primer

<400> 894
tctttgctcg cagctcgt 18

<210> 895
<211> 20
<212> DNA
<213> Artificial Sequence

<220>
<223> Primer

<400> 895
ctcggactcg gtgtatgcaa 20

<210> 896
<211> 27
<212> DNA
<213> Artificial Sequence

<220>
<223> Primer

<400> 896
ctgtcctgta tagcttcctg ctatttt 27

<210> 897
<211> 18
<212> DNA
<213> Artificial Sequence

<220>
<223> Primer

<400> 897
cgtggtgtgc aactgcaa 18

<210> 898
<211> 21
<212> DNA
<213> Artificial Sequence

<220>
<223> Primer

<400> 898

catggttcca ccagcgttat t 21

<210> 899
<211> 18
<212> DNA
<213> Artificial Sequence

<220>
<223> Primer

<400> 899
cgtggtgtgc aactgcaa 18

<210> 900
<211> 21
<212> DNA
<213> Artificial Sequence

<220>
<223> Primer

<400> 900
ggtcgcggtg gtgtttgata a 21

<210> 901
<211> 25
<212> DNA
<213> Artificial Sequence

<220>
<223> Primer

<400> 901
caccaccttg caggacatta caata 25

<210> 902
<211> 20
<212> DNA
<213> Artificial Sequence

<220>
<223> Primer

<400> 902
gattggcatg cagctagtgg 20

<210> 903
<211> 25
<212> DNA
<213> Artificial Sequence

<220>
<223> Primer

<400> 903
caccaccttg caggacatta caata 25

<210> 904

<211> 23
<212> DNA
<213> Artificial Sequence

<220>
<223> Primer

<400> 904
tgctgtagtt gtcgcagagt atc 23

<210> 905
<211> 26
<212> DNA
<213> Artificial Sequence

<220>
<223> Primer

<400> 905
acaacgttta aatgtgttac aggaca 26

<210> 906
<211> 24
<212> DNA
<213> Artificial Sequence

<220>
<223> Primer

<400> 906
ggtgatccag actctgacct tttg 24

<210> 907
<211> 26
<212> DNA
<213> Artificial Sequence

<220>
<223> Primer

<400> 907
acaacgttta aatgtgttac aggaca 26

<210> 908
<211> 19
<212> DNA
<213> Artificial Sequence

<220>
<223> Primer

<400> 908
ctgagaagcc ctgcccttc 19

<210> 909
<211> 22
<212> DNA
<213> Artificial Sequence

<220>
<223> Primer

<400> 909
cacagtaaca gtacaggcca ca 22

<210> 910
<211> 19
<212> DNA
<213> Artificial Sequence

<220>
<223> Primer

<400> 910
aaatacggct gcaccgagt 19

<210> 911
<211> 20
<212> DNA
<213> Artificial Sequence

<220>
<223> Primer

<400> 911
ggtgtattcc gtgccagaca 20

<210> 912
<211> 28
<212> DNA
<213> Artificial Sequence

<220>
<223> Primer

<400> 912
ctgttttggt caaatggaaa tgcattag 28

<210> 913
<211> 22
<212> DNA
<213> Artificial Sequence

<220>
<223> Primer

<400> 913
cacagtaaca gtacaggcca ca 22

<210> 914
<211> 19
<212> DNA
<213> Artificial Sequence

<220>
<223> Primer

<400> 914
ctgagaagcc ctgcccttc 19

<210> 915
<211> 26
<212> DNA
<213> Artificial Sequence

<220>
<223> Primer

<400> 915
acaacgttta aatgtgttac aggaca 26

<210> 916
<211> 21
<212> DNA
<213> Artificial Sequence

<220>
<223> Primer

<400> 916
catggttcca ccagcgttat t 21

<210> 917
<211> 22
<212> DNA
<213> Artificial Sequence

<220>
<223> Primer

<400> 917
cacagtaaca gtacaggcca ca 22

<210> 918
<211> 24
<212> DNA
<213> Artificial Sequence

<220>
<223> Primer

<400> 918
ggtgatccag actctgacct tttg 24

<210> 919
<211> 25
<212> DNA
<213> Artificial Sequence

<220>
<223> Primer

<400> 919
caccaccttg caggacatta caata 25

<210> 920
<211> 21
<212> DNA
<213> Artificial Sequence

<220>
<223> Primer

<400> 920
ggtcgcggtg gtgtttgata a 21

<210> 921
<211> 25
<212> DNA
<213> Artificial Sequence

<220>
<223> Primer

<400> 921
ttggtgtggt ttggtgtgtg tatat 25

<210> 922
<211> 26
<212> DNA
<213> Artificial Sequence

<220>
<223> Primer

<400> 922
ctccaatggt gtggtacgta taagaa 26

<210> 923
<211> 20
<212> DNA
<213> Artificial Sequence

<220>
<223> Primer

<400> 923
cattttgtgg cgaccgaagt 20

<210> 924
<211> 26
<212> DNA
<213> Artificial Sequence

<220>
<223> Primer

<400> 924
cctggacagg atgatgagta ataagg 26

<210> 925
<211> 27
<212> DNA

<213> Artificial Sequence

<220>
<223> Primer

<400> 925
agggttactg taggaaaggg attaagt 27

<210> 926
<211> 28
<212> DNA
<213> Artificial Sequence

<220>
<223> Primer

<400> 926
cgtatcccct gttaccacac taatattg 28

<210> 927
<211> 20
<212> DNA
<213> Artificial Sequence

<220>
<223> Primer

<400> 927
ccagccattg ggtgttggta 20

<210> 928
<211> 26
<212> DNA
<213> Artificial Sequence

<220>
<223> Primer

<400> 928
gcctataatg cacaactgtg tctgtt 26

<210> 929
<211> 21
<212> DNA
<213> Artificial Sequence

<220>
<223> Primer

<400> 929
agcaccttgt cctttgtgtg t 21

<210> 930
<211> 24
<212> DNA
<213> Artificial Sequence

<220>

<223> Primer

<400> 930
ggtgatccag actctgacct tttg 24

<210> 931
<211> 28
<212> DNA
<213> Artificial Sequence

<220>
<223> Primer

<400> 931
cgttacagga caaaatacta gaccacta 28

<210> 932
<211> 21
<212> DNA
<213> Artificial Sequence

<220>
<223> Primer

<400> 932
catggttcca ccagcgttat t 21

<210> 933
<211> 21
<212> DNA
<213> Artificial Sequence

<220>
<223> Primer

<400> 933
agcaccttgt cctttgtgtg t 21

<210> 934
<211> 28
<212> DNA
<213> Artificial Sequence

<220>
<223> Primer

<400> 934
gaaatgcatg tggaaatgta aataccgt 28

<210> 935
<211> 28
<212> DNA
<213> Artificial Sequence

<220>
<223> Primer

<400> 935

tcctgtgttc aagtacaagt aacaacaa 28

<210> 936
<211> 19
<212> DNA
<213> Artificial Sequence

<220>
<223> Primer

<400> 936
aaatacggct gcaccgagt 19

<210> 937
<211> 24
<212> DNA
<213> Artificial Sequence

<220>
<223> Primer

<400> 937
tcagatagtg gctatggctg ttct 24

<210> 938
<211> 28
<212> DNA
<213> Artificial Sequence

<220>
<223> Primer

<400> 938
gaaatgcatg tggaaatgta aataccgt 28

<210> 939
<211> 28
<212> DNA
<213> Artificial Sequence

<220>
<223> Primer

<400> 939
tcctgtgttc aagtacaagt aacaacaa 28

<210> 940
<211> 24
<212> DNA
<213> Artificial Sequence

<220>
<223> Primer

<400> 940
ggtgatccag actctgacct tttg 24

<210> 941

<211> 26
<212> DNA
<213> Artificial Sequence

<220>
<223> Primer

<400> 941
ctacaagacg tatctattgc ctgtgt 26

<210> 942
<211> 25
<212> DNA
<213> Artificial Sequence

<220>
<223> Primer

<400> 942
tcaaaaacag ctgctgtagt gttct 25

<210> 943
<211> 21
<212> DNA
<213> Artificial Sequence

<220>
<223> Primer

<400> 943
agcaccttgt cctttgtgtg t 21

<210> 944
<211> 18
<212> DNA
<213> Artificial Sequence

<220>
<223> Primer

<400> 944
cccacggatg cggttttg 18

<210> 945
<211> 27
<212> DNA
<213> Artificial Sequence

<220>
<223> Primer

<400> 945
aaactctgta tatggagaga cactgga 27

<210> 946
<211> 27
<212> DNA
<213> Artificial Sequence

<220>
<223> Primer

<400> 946
cgtttgtcct taaggtgtct acgtttt 27

<210> 947
<211> 26
<212> DNA
<213> Artificial Sequence

<220>
<223> Primer

<400> 947
ctacaagacg tatctattgc ctgtgt 26

<210> 948
<211> 27
<212> DNA
<213> Artificial Sequence

<220>
<223> Primer

<400> 948
cgtttgtcct taaggtgtct acgtttt 27

<210> 949
<211> 26
<212> DNA
<213> Artificial Sequence

<220>
<223> Primer

<400> 949
ctacaagacg tatctattgc ctgtgt 26

<210> 950
<211> 21
<212> DNA
<213> Artificial Sequence

<220>
<223> Primer

<400> 950
ggattccttc ggtgtctgca t 21

<210> 951
<211> 28
<212> DNA
<213> Artificial Sequence

<220>
<223> Primer

<400> 951
cgttacagga caaaatacta gaccacta 28

<210> 952
<211> 19
<212> DNA
<213> Artificial Sequence

<220>
<223> Primer

<400> 952
ctgagaagcc ctgcccttc 19

<210> 953
<211> 26
<212> DNA
<213> Artificial Sequence

<220>
<223> Primer

<400> 953
ctacaagacg tatctattgc ctgtgt 26

<210> 954
<211> 29
<212> DNA
<213> Artificial Sequence

<220>
<223> Primer

<400> 954
aagtctatac atttatggca tgcagcata 29

<210> 955
<211> 21
<212> DNA
<213> Artificial Sequence

<220>
<223> Primer

<400> 955
agcaccttgt cctttgtgtg t 21

<210> 956
<211> 21
<212> DNA
<213> Artificial Sequence

<220>
<223> Primer

<400> 956
catggttcca ccagcgttat t 21

<210> 957
<211> 24
<212> DNA
<213> Artificial Sequence

<220>
<223> Primer

<400> 957
tcagatagtg gctatggctg ttct 24

<210> 958
<211> 18
<212> DNA
<213> Artificial Sequence

<220>
<223> Primer

<400> 958
cccacggatg cggttttg 18

<210> 959
<211> 21
<212> DNA
<213> Artificial Sequence

<220>
<223> Primer

<400> 959
agcaccttgt cctttgtgtg t 21

<210> 960
<211> 19
<212> DNA
<213> Artificial Sequence

<220>
<223> Primer

<400> 960
aaatacggct gcaccgagt 19

<210> 961
<211> 21
<212> DNA
<213> Artificial Sequence

<220>
<223> Primer

<400> 961
agcaccttgt cctttgtgtg t 21

<210> 962
<211> 18
<212> DNA

<213> Artificial Sequence

<220>
<223> Primer

<400> 962
tctttgctcg cagctcgt 18

<210> 963
<211> 26
<212> DNA
<213> Artificial Sequence

<220>
<223> Primer

<400> 963
ctacaagacg tatctattgc ctgtgt 26

<210> 964
<211> 18
<212> DNA
<213> Artificial Sequence

<220>
<223> Primer

<400> 964
cccacggatg cggttttg 18

<210> 965
<211> 30
<212> DNA
<213> Artificial Sequence

<220>
<223> Primer

<400> 965
cattattaca gctaaaatgt cctccaatcc 30

<210> 966
<211> 28
<212> DNA
<213> Artificial Sequence

<220>
<223> Primer

<400> 966
gaaatgcatg tggaaatgta aataccgt 28

<210> 967
<211> 28
<212> DNA
<213> Artificial Sequence

<220>

<223> Primer

<400> 967
tcctgtgttc aagtacaagt aacaacaa 28

<210> 968
<211> 22
<212> DNA
<213> Artificial Sequence

<220>
<223> Primer

<400> 968
atcatgatgg ctgtatgtgc ca 22

<210> 969
<211> 28
<212> DNA
<213> Artificial Sequence

<220>
<223> Primer

<400> 969
cgttacagga caaaatacta gaccacta 28

<210> 970
<211> 22
<212> DNA
<213> Artificial Sequence

<220>
<223> Primer

<400> 970
atcatgatgg ctgtatgtgc ca 22

<210> 971
<211> 28
<212> DNA
<213> Artificial Sequence

<220>
<223> Primer

<400> 971
cgttacagga caaaatacta gaccacta 28

<210> 972
<211> 19
<212> DNA
<213> Artificial Sequence

<220>
<223> Primer

<400> 972

aaatacggct gcaccgagt 19

<210> 973
<211> 24
<212> DNA
<213> Artificial Sequence

<220>
<223> Primer

<400> 973
ggagttagtc atgcacaact acca 24

<210> 974
<211> 25
<212> DNA
<213> Artificial Sequence

<220>
<223> Primer

<400> 974
tcaaaaacag ctgctgtagt gttct 25

<210> 975
<211> 21
<212> DNA
<213> Artificial Sequence

<220>
<223> Primer

<400> 975
agcaccttgt cctttgtgtg t 21

<210> 976
<211> 21
<212> DNA
<213> Artificial Sequence

<220>
<223> Primer

<400> 976
ggattccttc ggtgtctgca t 21

<210> 977
<211> 28
<212> DNA
<213> Artificial Sequence

<220>
<223> Primer

<400> 977
tcctgtgttc aagtacaagt aacaacaa 28

<210> 978

<211> 18
<212> DNA
<213> Artificial Sequence

<220>
<223> Primer

<400> 978
tctttgctcg cagctcgt 18

<210> 979
<211> 21
<212> DNA
<213> Artificial Sequence

<220>
<223> Primer

<400> 979
agcaccttgt cctttgtgtg t 21

<210> 980
<211> 19
<212> DNA
<213> Artificial Sequence

<220>
<223> Primer

<400> 980
gctgacaact ctggccaca 19

<210> 981
<211> 26
<212> DNA
<213> Artificial Sequence

<220>
<223> Primer

<400> 981
ctacaagacg tatctattgc ctgtgt 26

<210> 982
<211> 28
<212> DNA
<213> Artificial Sequence

<220>
<223> Primer

<400> 982
gaaatgcatg tggaaatgta aataccgt 28

<210> 983
<211> 28
<212> DNA
<213> Artificial Sequence

<220>
<223> Primer

<400> 983
gcacacaata ttatttatgg ccatggta 28

<210> 984
<211> 19
<212> DNA
<213> Artificial Sequence

<220>
<223> Primer

<400> 984
gctgacaact ctggccaca 19

<210> 985
<211> 28
<212> DNA
<213> Artificial Sequence

<220>
<223> Primer

<400> 985
cgttacagga caaaatacta gaccacta 28

<210> 986
<211> 18
<212> DNA
<213> Artificial Sequence

<220>
<223> Primer

<400> 986
tctttgctcg cagctcgt 18

<210> 987
<211> 21
<212> DNA
<213> Artificial Sequence

<220>
<223> Primer

<400> 987
agcaccttgt cctttgtgtg t 21

<210> 988
<211> 28
<212> DNA
<213> Artificial Sequence

<220>
<223> Primer

<400> 988
caattgtttc tacaaagaac cagccatt 28

<210> 989
<211> 28
<212> DNA
<213> Artificial Sequence

<220>
<223> Primer

<400> 989
cgttacagga caaaatacta gaccacta 28

<210> 990
<211> 24
<212> DNA
<213> Artificial Sequence

<220>
<223> Primer

<400> 990
ggtgatccag actctgacct tttg 24

<210> 991
<211> 21
<212> DNA
<213> Artificial Sequence

<220>
<223> Primer

<400> 991
agcaccttgt cctttgtgtg t 21

<210> 992
<211> 19
<212> DNA
<213> Artificial Sequence

<220>
<223> Primer

<400> 992
ctgagaagcc ctgcccttc 19

<210> 993
<211> 24
<212> DNA
<213> Artificial Sequence

<220>
<223> Primer

<400> 993
tcagatagtg gctatggctg ttct 24

<210> 994
<211> 21
<212> DNA
<213> Artificial Sequence

<220>
<223> Primer

<400> 994
ggattccttc ggtgtctgca t 21

<210> 995
<211> 24
<212> DNA
<213> Artificial Sequence

<220>
<223> Primer

<400> 995
tcagatagtg gctatggctg ttct 24

<210> 996
<211> 25
<212> DNA
<213> Artificial Sequence

<220>
<223> Primer

<400> 996
actatcccca ccactacttt gtgta 25

<210> 997
<211> 28
<212> DNA
<213> Artificial Sequence

<220>
<223> Primer

<400> 997
tcctgtgttc aagtacaagt aacaacaa 28

<210> 998
<211> 21
<212> DNA
<213> Artificial Sequence

<220>
<223> Primer

<400> 998
catggttcca ccagcgttat t 21

<210> 999
<211> 28
<212> DNA

<213> Artificial Sequence

<220>
<223> Primer

<400> 999
tcctgtgttc aagtacaagt aacaacaa 28

<210> 1000
<211> 19
<212> DNA
<213> Artificial Sequence

<220>
<223> Primer

<400> 1000
gctgacaact ctggccaca 19

<210> 1001
<211> 28
<212> DNA
<213> Artificial Sequence

<220>
<223> Primer

<400> 1001
tcctgtgttc aagtacaagt aacaacaa 28

<210> 1002
<211> 19
<212> DNA
<213> Artificial Sequence

<220>
<223> Primer

<400> 1002
ctgagaagcc ctgcccttc 19

<210> 1003
<211> 21
<212> DNA
<213> Artificial Sequence

<220>
<223> Primer

<400> 1003
agcaccttgt cctttgtgtg t 21

<210> 1004
<211> 22
<212> DNA
<213> Artificial Sequence

<220>

<223> Primer

<400> 1004
atcatgatgg ctgtatgtgc ca 22

<210> 1005
<211> 21
<212> DNA
<213> Artificial Sequence

<220>
<223> Primer

<400> 1005
tgcttttgct tggttgttgg t 21

<210> 1006
<211> 27
<212> DNA
<213> Artificial Sequence

<220>
<223> Primer

<400> 1006
catcacaggt atgttacact gtactgt 27

<210> 1007
<211> 28
<212> DNA
<213> Artificial Sequence

<220>
<223> Primer

<400> 1007
tcctgtgttc aagtacaagt aacaacaa 28

<210> 1008
<211> 25
<212> DNA
<213> Artificial Sequence

<220>
<223> Primer

<400> 1008
ggtctgcata tttgcgtagc ctata 25

<210> 1009
<211> 23
<212> DNA
<213> Artificial Sequence

<220>
<223> Primer

<400> 1009

atttcggttg cctgtggctt ata 23

<210> 1010
<211> 25
<212> DNA
<213> Artificial Sequence

<220>
<223> Primer

<400> 1010
cagttgtgca agccattgtt ttagt 25

<210> 1011
<211> 27
<212> DNA
<213> Artificial Sequence

<220>
<223> Primer

<400> 1011
cgcaaatatg cagaccatta ctcagaa 27

<210> 1012
<211> 19
<212> DNA
<213> Artificial Sequence

<220>
<223> Primer

<400> 1012
gctgacaact ctggccaca 19

<210> 1013
<211> 25
<212> DNA
<213> Artificial Sequence

<220>
<223> Primer

<400> 1013
gcaacgttat acgcccatat ccaat 25

<210> 1014
<211> 23
<212> DNA
<213> Artificial Sequence

<220>
<223> Primer

<400> 1014
ggtacgtgca acaatgtgct taa 23

<210> 1015

<211> 27
<212> DNA
<213> Artificial Sequence

<220>
<223> Primer

<400> 1015
cgcaaatatg cagaccatta ctcagaa 27

<210> 1016
<211> 24
<212> DNA
<213> Artificial Sequence

<220>
<223> Primer

<400> 1016
cccaccgaga tttgtacact gtta 24

<210> 1017
<211> 25
<212> DNA
<213> Artificial Sequence

<220>
<223> Primer

<400> 1017
ggcatgtgta ggtatggaaa ttggt 25

<210> 1018
<211> 25
<212> DNA
<213> Artificial Sequence

<220>
<223> Primer

<400> 1018
acatcctgcg taataacagc tgtag 25

<210> 1019
<211> 20
<212> DNA
<213> Artificial Sequence

<220>
<223> Primer

<400> 1019
tgacgacacg gtatccgcta 20

<210> 1020
<211> 18
<212> DNA
<213> Artificial Sequence

<220>
<223> Primer

<400> 1020
cccacggatg cggttttg 18

<210> 1021
<211> 31
<212> DNA
<213> Artificial Sequence

<220>
<223> Primer

<400> 1021
agtatgtcca ccattactaa taacgtcaaa c 31

<210> 1022
<211> 28
<212> DNA
<213> Artificial Sequence

<220>
<223> Primer

<400> 1022
tcattcaatg tatacacagc attcccat 28

<210> 1023
<211> 21
<212> DNA
<213> Artificial Sequence

<220>
<223> Primer

<400> 1023
ctaacactgg agggcaccaa a 21

<210> 1024
<211> 19
<212> DNA
<213> Artificial Sequence

<220>
<223> Primer

<400> 1024
aaatacggct gcaccgagt 19

<210> 1025
<211> 20
<212> DNA
<213> Artificial Sequence

<220>
<223> GGGCGAACTAAGCCTGGTTT

<400> 1025
gggcgaacta agcctggttt 20

<210> 1026
<211> 22
<212> DNA
<213> Artificial Sequence

<220>
<223> Primer

<400> 1026
atcatgatgg ctgtatgtgc ca 22

<210> 1027
<211> 20
<212> DNA
<213> Artificial Sequence

<220>
<223> Primer

<400> 1027
gggcgaacta agcctggttt 20

<210> 1028
<211> 19
<212> DNA
<213> Artificial Sequence

<220>
<223> Primer

<400> 1028
aaatacggct gcaccgagt 19

<210> 1029
<211> 21
<212> DNA
<213> Artificial Sequence

<220>
<223> CTAACACTGGAGGGCACCAAA

<400> 1029
ctaacactgg agggcaccaa a 21

<210> 1030
<211> 18
<212> DNA
<213> Artificial Sequence

<220>
<223> Primer

<400> 1030
tctttgctcg cagctcgt 18

<210> 1031
<211> 28
<212> DNA
<213> Artificial Sequence

<220>
<223> Primer

<400> 1031
gtgccaggag aaaatactag actgttat 28

<210> 1032
<211> 19
<212> DNA
<213> Artificial Sequence

<220>
<223> Primer

<400> 1032
ctgagaagcc ctgcccttc 19

<210> 1033
<211> 21
<212> DNA
<213> Artificial Sequence

<220>
<223> Primer

<400> 1033
ctaacactgg agggcaccaa a 21

<210> 1034
<211> 26
<212> DNA
<213> Artificial Sequence

<220>
<223> Primer

<400> 1034
atgccaggtt gaggatacgt ttttat 26

<210> 1035
<211> 28
<212> DNA
<213> Artificial Sequence

<220>
<223> Primer

<400> 1035
gtgccaggag aaaatactag actgttat 28

<210> 1036
<211> 21
<212> DNA

<213> Artificial Sequence

<220>
<223> Primer

<400> 1036
catggttcca ccagcgttat t 21

<210> 1037
<211> 21
<212> DNA
<213> Artificial Sequence

<220>
<223> Primer

<400> 1037
cggacagcgg atatggcaat a 21

<210> 1038
<211> 26
<212> DNA
<213> Artificial Sequence

<220>
<223> Primer

<400> 1038
tctgttgttt ccacatccat aacact 26

<210> 1039
<211> 21
<212> DNA
<213> Artificial Sequence

<220>
<223> Primer

<400> 1039
ctaacactgg agggcaccaa a 21

<210> 1040
<211> 22
<212> DNA
<213> Artificial Sequence

<220>
<223> Primer

<400> 1040
atcatgatgg ctgtatgtgc ca 22

<210> 1041
<211> 21
<212> DNA
<213> Artificial Sequence

<220>

<223> Primer

<400> 1041
ctaacactgg agggcaccaa a 21

<210> 1042
<211> 21
<212> DNA
<213> Artificial Sequence

<220>
<223> Primer

<400> 1042
caattcgaga cacaggtgca g 21

<210> 1043
<211> 28
<212> DNA
<213> Artificial Sequence

<220>
<223> Primer

<400> 1043
gagagtatag acgttatagc aggtctgt 28

<210> 1044
<211> 24
<212> DNA
<213> Artificial Sequence

<220>
<223> Primer

<400> 1044
tcccgctatt tcatggaacc tttt 24

<210> 1045
<211> 21
<212> DNA
<213> Artificial Sequence

<220>
<223> Primer

<400> 1045
cggacagcgg atatggcaat a 21

<210> 1046
<211> 18
<212> DNA
<213> Artificial Sequence

<220>
<223> Primer

<400> 1046

ccacgcaggt ggtaaggg 18

<210> 1047
<211> 27
<212> DNA
<213> Artificial Sequence

<220>
<223> Primer

<400> 1047
ctgcatgaat tatgtgaagc tttgaac 27

<210> 1048
<211> 21
<212> DNA
<213> Artificial Sequence

<220>
<223> Primer

<400> 1048
catctgctgt acaacgcgaa g 21

<210> 1049
<211> 20
<212> DNA
<213> Artificial Sequence

<220>
<223> Primer

<400> 1049
gggcgaacta agcctggttt 20

<210> 1050
<211> 24
<212> DNA
<213> Artificial Sequence

<220>
<223> Primer

<400> 1050
ggtgatccag actctgacct tttg 24

<210> 1051
<211> 21
<212> DNA
<213> Artificial Sequence

<220>
<223> Primer

<400> 1051
ctaacactgg agggcaccaa a 21

<210> 1052

<211> 21
<212> DNA
<213> Artificial Sequence

<220>
<223> Primer

<400> 1052
catggttcca ccagcgttat t 21

<210> 1053
<211> 21
<212> DNA
<213> Artificial Sequence

<220>
<223> Primer

<400> 1053
cggacagcgg atatggcaat a 21

<210> 1054
<211> 28
<212> DNA
<213> Artificial Sequence

<220>
<223> Primer

<400> 1054
tcattcaatg tatacacagc attcccat 28

<210> 1055
<211> 20
<212> DNA
<213> Artificial Sequence

<220>
<223> Primer

<400> 1055
gggcgaacta agcctggttt 20

<210> 1056
<211> 27
<212> DNA
<213> Artificial Sequence

<220>
<223> Primer

<400> 1056
ctcatcatcc gaaacattat ctcctgt 27

<210> 1057
<211> 28
<212> DNA
<213> Artificial Sequence

<220>
<223> Primer

<400> 1057
gtgccaggag aaaatactag actgttat 28

<210> 1058
<211> 22
<212> DNA
<213> Artificial Sequence

<220>
<223> Primer

<400> 1058
atcatgatgg ctgtatgtgc ca 22

<210> 1059
<211> 24
<212> DNA
<213> Artificial Sequence

<220>
<223> Primer

<400> 1059
gcacaacagt gggaggtcta tatg 24

<210> 1060
<211> 18
<212> DNA
<213> Artificial Sequence

<220>
<223> Primer

<400> 1060
ccacgcaggt ggtaaggg 18

<210> 1061
<211> 28
<212> DNA
<213> Artificial Sequence

<220>
<223> Primer

<400> 1061
gtgccaggag aaaatactag actgttat 28

<210> 1062
<211> 19
<212> DNA
<213> Artificial Sequence

<220>
<223> Primer

<400> 1062
aaatacggct gcaccgagt 19

<210> 1063
<211> 20
<212> DNA
<213> Artificial Sequence

<220>
<223> Primer

<400> 1063
gggcgaacta agcctggttt 20

<210> 1064
<211> 21
<212> DNA
<213> Artificial Sequence

<220>
<223> Primer

<400> 1064
caattcgaga cacaggtgca g 21

<210> 1065
<211> 21
<212> DNA
<213> Artificial Sequence

<220>
<223> Primer

<400> 1065
ctaacactgg agggcaccaa a 21

<210> 1066
<211> 24
<212> DNA
<213> Artificial Sequence

<220>
<223> Primer

<400> 1066
ggtgatccag actctgacct tttg 24

<210> 1067
<211> 27
<212> DNA
<213> Artificial Sequence

<220>
<223> Primer

<400> 1067
ctgcatgaat tatgtgaagc tttgaac 27

<210> 1068
<211> 24
<212> DNA
<213> Artificial Sequence

<220>
<223> Primer

<400> 1068
tcccgctatt tcatggaacc tttt 24

<210> 1069
<211> 20
<212> DNA
<213> Artificial Sequence

<220>
<223> Primer

<400> 1069
gcgtgaccag ctaccagaaa 20

<210> 1070
<211> 21
<212> DNA
<213> Artificial Sequence

<220>
<223> Primer

<400> 1070
catctgctgt acaacgcgaa g 21

<210> 1071
<211> 20
<212> DNA
<213> Artificial Sequence

<220>
<223> Primer

<400> 1071
gggcgaacta agcctggttt 20

<210> 1072
<211> 21
<212> DNA
<213> Artificial Sequence

<220>
<223> Primer

<400> 1072
catggttcca ccagcgttat t 21

<210> 1073
<211> 21
<212> DNA

<213> Artificial Sequence

<220>
<223> Primer

<400> 1073
ctaacactgg agggcaccaa a 21

<210> 1074
<211> 19
<212> DNA
<213> Artificial Sequence

<220>
<223> Primer

<400> 1074
ctgagaagcc ctgcccttc 19

<210> 1075
<211> 27
<212> DNA
<213> Artificial Sequence

<220>
<223> Primer

<400> 1075
ctgcatgaat tatgtgaagc tttgaac 27

<210> 1076
<211> 29
<212> DNA
<213> Artificial Sequence

<220>
<223> Primer

<400> 1076
gtaaacattg tttgcatact gcatatgga 29

<210> 1077
<211> 20
<212> DNA
<213> Artificial Sequence

<220>
<223> Primer

<400> 1077
gggcgaacta agcctggttt 20

<210> 1078
<211> 19
<212> DNA
<213> Artificial Sequence

<220>

<223> Primer

<400> 1078
ctgagaagcc ctgcccttc 19

<210> 1079
<211> 20
<212> DNA
<213> Artificial Sequence

<220>
<223> Primer

<400> 1079
gggcgaacta agcctggttt 20

<210> 1080
<211> 18
<212> DNA
<213> Artificial Sequence

<220>
<223> Primer

<400> 1080
ccacgcaggt ggtaaggg 18

<210> 1081
<211> 28
<212> DNA
<213> Artificial Sequence

<220>
<223> Primer

<400> 1081
gtgccaggag aaaatactag actgttat 28

<210> 1082
<211> 24
<212> DNA
<213> Artificial Sequence

<220>
<223> Primer

<400> 1082
ggtgatccag actctgacct tttg 24

<210> 1083
<211> 28
<212> DNA
<213> Artificial Sequence

<220>
<223> Primer

<400> 1083

gtgccaggag aaaatactag actgttat 28

<210> 1084
<211> 18
<212> DNA
<213> Artificial Sequence

<220>
<223> Primer

<400> 1084
tctttgctcg cagctcgt 18

<210> 1085
<211> 20
<212> DNA
<213> Artificial Sequence

<220>
<223> Primer

<400> 1085
gggcgaacta agcctggttt 20

<210> 1086
<211> 18
<212> DNA
<213> Artificial Sequence

<220>
<223> Primer

<400> 1086
tctttgctcg cagctcgt 18

<210> 1087
<211> 26
<212> DNA
<213> Artificial Sequence

<220>
<223> Primer

<400> 1087
ggccctatac acatttacta cgcaaa 26

<210> 1088
<211> 21
<212> DNA
<213> Artificial Sequence

<220>
<223> Primer

<400> 1088
caattcgaga cacaggtgca g 21

<210> 1089

<211> 27
<212> DNA
<213> Artificial Sequence

<220>
<223> Primer

<400> 1089
ctgcatgaat tatgtgaagc tttgaac 27

<210> 1090
<211> 18
<212> DNA
<213> Artificial Sequence

<220>
<223> Primer

<400> 1090
ccacgcaggt ggtaaggg 18

<210> 1091
<211> 20
<212> DNA
<213> Artificial Sequence

<220>
<223> Primer

<400> 1091
gggcgaacta agcctggttt 20

<210> 1092
<211> 28
<212> DNA
<213> Artificial Sequence

<220>
<223> Primer

<400> 1092
tcattcaatg tatacacagc attcccat 28

<210> 1093
<211> 27
<212> DNA
<213> Artificial Sequence

<220>
<223> Primer

<400> 1093
ctgcatgaat tatgtgaagc tttgaac 27

<210> 1094
<211> 28
<212> DNA
<213> Artificial Sequence

<220>
<223> Primer

<400> 1094
tcattcaatg tatacacagc attcccat 28

<210> 1095
<211> 27
<212> DNA
<213> Artificial Sequence

<220>
<223> Primer

<400> 1095
gggtattaca ttatccccgt aggtcaa 27

<210> 1096
<211> 23
<212> DNA
<213> Artificial Sequence

<220>
<223> Primer

<400> 1096
gctgcagctg taacaaaatg gaa 23

<210> 1097
<211> 25
<212> DNA
<213> Artificial Sequence

<220>
<223> Primer

<400> 1097
aagccaatat gtgctgctaa ttgta 25

<210> 1098
<211> 23
<212> DNA
<213> Artificial Sequence

<220>
<223> Primer

<400> 1098
aacacgtatt gggacagcag tag 23

<210> 1099
<211> 24
<212> DNA
<213> Artificial Sequence

<220>
<223> Primer

<400> 1099
gatggaggca actggagaga aatt 24

<210> 1100
<211> 26
<212> DNA
<213> Artificial Sequence

<220>
<223> Primer

<400> 1100
gtgtttggtg ggccatatat gactat 26

<210> 1101
<211> 19
<212> DNA
<213> Artificial Sequence

<220>
<223> Primer

<400> 1101
acacccctcc acaggctaa 19

<210> 1102
<211> 20
<212> DNA
<213> Artificial Sequence

<220>
<223> Primer

<400> 1102
tgtacgccaa cctgcaacaa 20

<210> 1103
<211> 27
<212> DNA
<213> Artificial Sequence

<220>
<223> Primer

<400> 1103
gacatgttaa tgcaaacaag cgatttc 27

<210> 1104
<211> 28
<212> DNA
<213> Artificial Sequence

<220>
<223> Primer

<400> 1104
tcagttgttt caggttgcag atctaata 28

<210> 1105
<211> 22
<212> DNA
<213> Artificial Sequence

<220>
<223> Primer

<400> 1105
gctgttgggc acattacaag tt 22

<210> 1106
<211> 19
<212> DNA
<213> Artificial Sequence

<220>
<223> Primer

<400> 1106
ctgagaagcc ctgcccttc 19

<210> 1107
<211> 28
<212> DNA
<213> Artificial Sequence

<220>
<223> Primer

<400> 1107
gctgatagta atgacctaaa cgcacaaa 28

<210> 1108
<211> 18
<212> DNA
<213> Artificial Sequence

<220>
<223> Primer

<400> 1108
tctttgctcg cagctcgt 18

<210> 1109
<211> 22
<212> DNA
<213> Artificial Sequence

<220>
<223> Primer

<400> 1109
gctgttgggc acattacaag tt 22

<210> 1110
<211> 21
<212> DNA

<213> Artificial Sequence

<220>
<223> Primer

<400> 1110
catggttcca ccagcgttat t 21

<210> 1111
<211> 24
<212> DNA
<213> Artificial Sequence

<220>
<223> Primer

<400> 1111
agaatcggtg catgaaataa ggct 24

<210> 1112
<211> 28
<212> DNA
<213> Artificial Sequence

<220>
<223> Primer

<400> 1112
tcagttgttt caggttgcag atctaata 28

<210> 1113
<211> 21
<212> DNA
<213> Artificial Sequence

<220>
<223> Primer

<400> 1113
tcactgcaac tgagtgcaca a 21

<210> 1114
<211> 26
<212> DNA
<213> Artificial Sequence

<220>
<223> Primer

<400> 1114
tgcttacaac cttagagaca ggtaca 26

<210> 1115
<211> 21
<212> DNA
<213> Artificial Sequence

<220>

<223> Primer

<400> 1115
tcactgcaac tgagtgcaca a 21

<210> 1116
<211> 18
<212> DNA
<213> Artificial Sequence

<220>
<223> Primer

<400> 1116
tctttgctcg cagctcgt 18

<210> 1117
<211> 22
<212> DNA
<213> Artificial Sequence

<220>
<223> Primer

<400> 1117
ttttactacg tcgcaggcgt aa 22

<210> 1118
<211> 26
<212> DNA
<213> Artificial Sequence

<220>
<223> Primer

<400> 1118
tgcttacaac cttagagaca ggtaca 26

<210> 1119
<211> 24
<212> DNA
<213> Artificial Sequence

<220>
<223> Primer

<400> 1119
agaatcggtg catgaaataa ggct 24

<210> 1120
<211> 27
<212> DNA
<213> Artificial Sequence

<220>
<223> Primer

<400> 1120

cgcttgtttg cattaacatg tctttct 27

<210> 1121
<211> 21
<212> DNA
<213> Artificial Sequence

<220>
<223> Primer

<400> 1121
tcactgcaac tgagtgcaca a 21

<210> 1122
<211> 28
<212> DNA
<213> Artificial Sequence

<220>
<223> Primer

<400> 1122
tgccaggtag atgaaatttg aacataca 28

<210> 1123
<211> 22
<212> DNA
<213> Artificial Sequence

<220>
<223> Primer

<400> 1123
gctgttgggc acattacaag tt 22

<210> 1124
<211> 24
<212> DNA
<213> Artificial Sequence

<220>
<223> Primer

<400> 1124
ggtgatccag actctgacct tttg 24

<210> 1125
<211> 22
<212> DNA
<213> Artificial Sequence

<220>
<223> Primer

<400> 1125
gctgttgggc acattacaag tt 22

<210> 1126

<211> 19
<212> DNA
<213> Artificial Sequence

<220>
<223> Primer

<400> 1126
aaatacggct gcaccgagt 19

<210> 1127
<211> 22
<212> DNA
<213> Artificial Sequence

<220>
<223> Primer

<400> 1127
gctgttgggc acattacaag tt 22

<210> 1128
<211> 19
<212> DNA
<213> Artificial Sequence

<220>
<223> Primer

<400> 1128
gcggaggtct tggaggttt 19

<210> 1129
<211> 21
<212> DNA
<213> Artificial Sequence

<220>
<223> Primer

<400> 1129
tcactgcaac tgagtgcaca a 21

<210> 1130
<211> 22
<212> DNA
<213> Artificial Sequence

<220>
<223> Primer

<400> 1130
atcatgatgg ctgtatgtgc ca 22

<210> 1131
<211> 28
<212> DNA
<213> Artificial Sequence

<220>
<223> Primer

<400> 1131
gctgatagta atgacctaaa cgcacaaa 28

<210> 1132
<211> 24
<212> DNA
<213> Artificial Sequence

<220>
<223> Primer

<400> 1132
ggtgatccag actctgacct tttg 24

<210> 1133
<211> 22
<212> DNA
<213> Artificial Sequence

<220>
<223> Primer

<400> 1133
gctgttgggc acattacaag tt 22

<210> 1134
<211> 22
<212> DNA
<213> Artificial Sequence

<220>
<223> Primer

<400> 1134
atcatgatgg ctgtatgtgc ca 22

<210> 1135
<211> 24
<212> DNA
<213> Artificial Sequence

<220>
<223> Primer

<400> 1135
agaatcggtg catgaaataa ggct 24

<210> 1136
<211> 24
<212> DNA
<213> Artificial Sequence

<220>
<223> Primer

<400> 1136
gcatttgctg tagagtacga aggt 24

<210> 1137
<211> 24
<212> DNA
<213> Artificial Sequence

<220>
<223> Primer

<400> 1137
agaatcggtg catgaaataa ggct 24

<210> 1138
<211> 19
<212> DNA
<213> Artificial Sequence

<220>
<223> Primer

<400> 1138
gcggaggtct tggaggttt 19

<210> 1139
<211> 24
<212> DNA
<213> Artificial Sequence

<220>
<223> Primer

<400> 1139
caaaccatgt cacgtagaag acag 24

<210> 1140
<211> 28
<212> DNA
<213> Artificial Sequence

<220>
<223> Primer

<400> 1140
gggtttttga aatgaaacac aaccaatc 28

<210> 1141
<211> 24
<212> DNA
<213> Artificial Sequence

<220>
<223> Primer

<400> 1141
gatgaggagg atacagatgg tgtg 24

<210> 1142
<211> 24
<212> DNA
<213> Artificial Sequence

<220>
<223> Primer

<400> 1142
gcatttgctg tagagtacga aggt 24

<210> 1143
<211> 24
<212> DNA
<213> Artificial Sequence

<220>
<223> Primer

<400> 1143
caaaccatgt cacgtagaag acag 24

<210> 1144
<211> 19
<212> DNA
<213> Artificial Sequence

<220>
<223> Primer

<400> 1144
ccccacccca cttgattga 19

<210> 1145
<211> 24
<212> DNA
<213> Artificial Sequence

<220>
<223> Primer

<400> 1145
caaaccatgt cacgtagaag acag 24

<210> 1146
<211> 23
<212> DNA
<213> Artificial Sequence

<220>
<223> Primer

<400> 1146
cggtatcgac tccatcgttt tcc 23

<210> 1147
<211> 28
<212> DNA

<213> Artificial Sequence

<220>
<223> Primer

<400> 1147
gctgatagta atgacctaaa cgcacaaa 28

<210> 1148
<211> 19
<212> DNA
<213> Artificial Sequence

<220>
<223> Primer

<400> 1148
ctgagaagcc ctgcccttc 19

<210> 1149
<211> 22
<212> DNA
<213> Artificial Sequence

<220>
<223> Primer

<400> 1149
gctgttgggc acattacaag tt 22

<210> 1150
<211> 20
<212> DNA
<213> Artificial Sequence

<220>
<223> Primer

<400> 1150
cctgtattgc aggccagaca 20

<210> 1151
<211> 24
<212> DNA
<213> Artificial Sequence

<220>
<223> Primer

<400> 1151
agaatcggtg catgaaataa ggct 24

<210> 1152
<211> 27
<212> DNA
<213> Artificial Sequence

<220>

<223> Primer

<400> 1152
cacacgccat atggattatt gtctcta 27

<210> 1153
<211> 22
<212> DNA
<213> Artificial Sequence

<220>
<223> Primer

<400> 1153
gctgttgggc acattacaag tt 22

<210> 1154
<211> 23
<212> DNA
<213> Artificial Sequence

<220>
<223> Primer

<400> 1154
cggtatcgac tccatcgttt tcc 23

<210> 1155
<211> 21
<212> DNA
<213> Artificial Sequence

<220>
<223> Primer

<400> 1155
tcactgcaac tgagtgcaca a 21

<210> 1156
<211> 24
<212> DNA
<213> Artificial Sequence

<220>
<223> Primer

<400> 1156
ggtgatccag actctgacct tttg 24

<210> 1157
<211> 22
<212> DNA
<213> Artificial Sequence

<220>
<223> Primer

<400> 1157

gctgttgggc acattacaag tt 22

<210> 1158
<211> 28
<212> DNA
<213> Artificial Sequence

<220>
<223> Primer

<400> 1158
tcctctgaaa tgttatctcc tgtttgtt 28

<210> 1159
<211> 27
<212> DNA
<213> Artificial Sequence

<220>
<223> Primer

<400> 1159
ccttagtaca aataaaatgc ccaccat 27

<210> 1160
<211> 28
<212> DNA
<213> Artificial Sequence

<220>
<223> Primer

<400> 1160
gggtttttga aatgaaacac aaccaatc 28

<210> 1161
<211> 28
<212> DNA
<213> Artificial Sequence

<220>
<223> Primer

<400> 1161
gctgatagta atgacctaaa cgcacaaa 28

<210> 1162
<211> 22
<212> DNA
<213> Artificial Sequence

<220>
<223> Primer

<400> 1162
atcatgatgg ctgtatgtgc ca 22

<210> 1163

<211> 24
<212> DNA
<213> Artificial Sequence

<220>
<223> Primer

<400> 1163
agaatcggtg catgaaataa ggct 24

<210> 1164
<211> 23
<212> DNA
<213> Artificial Sequence

<220>
<223> Primer

<400> 1164
cggtatcgac tccatcgttt tcc 23

<210> 1165
<211> 28
<212> DNA
<213> Artificial Sequence

<220>
<223> Primer

<400> 1165
gctgatagta atgacctaaa cgcacaaa 28

<210> 1166
<211> 19
<212> DNA
<213> Artificial Sequence

<220>
<223> Primer

<400> 1166
aaatacggct gcaccgagt 19

<210> 1167
<211> 22
<212> DNA
<213> Artificial Sequence

<220>
<223> Primer

<400> 1167
gctgttgggc acattacaag tt 22

<210> 1168
<211> 18
<212> DNA
<213> Artificial Sequence

<220>
<223> Primer

<400> 1168
tctttgctcg cagctcgt 18

<210> 1169
<211> 27
<212> DNA
<213> Artificial Sequence

<220>
<223> Primer

<400> 1169
gtaacaggag tatgggaagt acatgtg 27

<210> 1170
<211> 19
<212> DNA
<213> Artificial Sequence

<220>
<223> Primer

<400> 1170
gcggaggtct tggaggttt 19

<210> 1171
<211> 27
<212> DNA
<213> Artificial Sequence

<220>
<223> Primer

<400> 1171
gtatgggaaa acattagaag agagggt 27

<210> 1172
<211> 27
<212> DNA
<213> Artificial Sequence

<220>
<223> Primer

<400> 1172
cgcttgtttg cattaacatg tctttct 27

<210> 1173
<211> 24
<212> DNA
<213> Artificial Sequence

<220>
<223> Primer

<400> 1173
caaaccatgt cacgtagaag acag 24

<210> 1174
<211> 19
<212> DNA
<213> Artificial Sequence

<220>
<223> Primer

<400> 1174
gcggaggtct tggaggttt 19

<210> 1175
<211> 21
<212> DNA
<213> Artificial Sequence

<220>
<223> Primer

<400> 1175
tcactgcaac tgagtgcaca a 21

<210> 1176
<211> 19
<212> DNA
<213> Artificial Sequence

<220>
<223> Primer

<400> 1176
aaatacggct gcaccgagt 19

<210> 1177
<211> 22
<212> DNA
<213> Artificial Sequence

<220>
<223> Primer

<400> 1177
gctgttgggc acattacaag tt 22

<210> 1178
<211> 28
<212> DNA
<213> Artificial Sequence

<220>
<223> Primer

<400> 1178
gggtttttga aatgaaacac aaccaatc 28

<210> 1179
<211> 24
<212> DNA
<213> Artificial Sequence

<220>
<223> Primer

<400> 1179
agaatcggtg catgaaataa ggct 24

<210> 1180
<211> 28
<212> DNA
<213> Artificial Sequence

<220>
<223> Primer

<400> 1180
gggtttttga aatgaaacac aaccaatc 28

<210> 1181
<211> 21
<212> DNA
<213> Artificial Sequence

<220>
<223> Primer

<400> 1181
tcactgcaac tgagtgcaca a 21

<210> 1182
<211> 21
<212> DNA
<213> Artificial Sequence

<220>
<223> Primer

<400> 1182
catggttcca ccagcgttat t 21

<210> 1183
<211> 21
<212> DNA
<213> Artificial Sequence

<220>
<223> Primer

<400> 1183
tcactgcaac tgagtgcaca a 21

<210> 1184
<211> 20
<212> DNA

<213> Artificial Sequence

<220>
<223> Primer

<400> 1184
cctgtattgc aggccagaca 20

<210> 1185
<211> 22
<212> DNA
<213> Artificial Sequence

<220>
<223> Primer

<400> 1185
gctgttgggc acattacaag tt 22

<210> 1186
<211> 26
<212> DNA
<213> Artificial Sequence

<220>
<223> Primer

<400> 1186
tgcttacaac cttagagaca ggtaca 26

<210> 1187
<211> 28
<212> DNA
<213> Artificial Sequence

<220>
<223> Primer

<400> 1187
aagcatctat tattatgcag gcagttct 28

<210> 1188
<211> 20
<212> DNA
<213> Artificial Sequence

<220>
<223> Primer

<400> 1188
cctgtattgc aggccagaca 20

<210> 1189
<211> 21
<212> DNA
<213> Artificial Sequence

<220>

<223> Primer

<400> 1189
tcactgcaac tgagtgcaca a 21

<210> 1190
<211> 19
<212> DNA
<213> Artificial Sequence

<220>
<223> Primer

<400> 1190
ctgagaagcc ctgcccttc 19

<210> 1191
<211> 28
<212> DNA
<213> Artificial Sequence

<220>
<223> Primer

<400> 1191
gctgatagta atgacctaaa cgcacaaa 28

<210> 1192
<211> 21
<212> DNA
<213> Artificial Sequence

<220>
<223> Primer

<400> 1192
catggttcca ccagcgttat t 21

<210> 1193
<211> 27
<212> DNA
<213> Artificial Sequence

<220>
<223> Primer

<400> 1193
ggactatatg ttttgggagg tggattt 27

<210> 1194
<211> 22
<212> DNA
<213> Artificial Sequence

<220>
<223> Primer

<400> 1194

gatgcagggc gttttagttt gg 22

<210> 1195
<211> 27
<212> DNA
<213> Artificial Sequence

<220>
<223> Primer

<400> 1195
caccatcagt tgcagaagga ttaaaag 27

<210> 1196
<211> 25
<212> DNA
<213> Artificial Sequence

<220>
<223> Primer

<400> 1196
ctgtgacatt agtttggaca ctgtt 25

<210> 1197
<211> 19
<212> DNA
<213> Artificial Sequence

<220>
<223> Primer

<400> 1197
tcggttggtc ttggcacaa 19

<210> 1198
<211> 21
<212> DNA
<213> Artificial Sequence

<220>
<223> Primer

<400> 1198
tttaggcggg acaacaagtg t 21

<210> 1199
<211> 24
<212> DNA
<213> Artificial Sequence

<220>
<223> Primer

<400> 1199
caacacaagc caatattgct gcta 24

<210> 1200

<211> 23
<212> DNA
<213> Artificial Sequence

<220>
<223> Primer

<400> 1200
cctgcgcata caccgatata gat 23

<210> 1201
<211> 23
<212> DNA
<213> Artificial Sequence

<220>
<223> Primer

<400> 1201
gttaacagta acgtgcccac tct 23

<210> 1202
<211> 28
<212> DNA
<213> Artificial Sequence

<220>
<223> Primer

<400> 1202
ttctacaatt gcctctactt caaaccat 28

<210> 1203
<211> 23
<212> DNA
<213> Artificial Sequence

<220>
<223> Primer

<400> 1203
acaacaacca ccctggtgat aag 23

<210> 1204
<211> 19
<212> DNA
<213> Artificial Sequence

<220>
<223> Primer

<400> 1204
aaatacggct gcaccgagt 19

<210> 1205
<211> 27
<212> DNA
<213> Artificial Sequence

<220>
<223> Primer

<400> 1205
gtgccagaac aaaatactag actgttt 27

<210> 1206
<211> 22
<212> DNA
<213> Artificial Sequence

<220>
<223> Primer

<400> 1206
atcatgatgg ctgtatgtgc ca 22

<210> 1207
<211> 23
<212> DNA
<213> Artificial Sequence

<220>
<223> Primer

<400> 1207
gttaacagta acgtgcccac tct 23

<210> 1208
<211> 24
<212> DNA
<213> Artificial Sequence

<220>
<223> Primer

<400> 1208
ggtgatccag actctgacct tttg 24

<210> 1209
<211> 23
<212> DNA
<213> Artificial Sequence

<220>
<223> Primer

<400> 1209
gttaacagta acgtgcccac tct 23

<210> 1210
<211> 30
<212> DNA
<213> Artificial Sequence

<220>
<223> Primer

<400> 1210
tgaaactgaa acactaacat tctactgtgt 30

<210> 1211
<211> 27
<212> DNA
<213> Artificial Sequence

<220>
<223> Primer

<400> 1211
gtgccagaac aaaatactag actgttt 27

<210> 1212
<211> 18
<212> DNA
<213> Artificial Sequence

<220>
<223> Primer

<400> 1212
tctttgctcg cagctcgt 18

<210> 1213
<211> 27
<212> DNA
<213> Artificial Sequence

<220>
<223> Primer

<400> 1213
gtgccagaac aaaatactag actgttt 27

<210> 1214
<211> 21
<212> DNA
<213> Artificial Sequence

<220>
<223> Primer

<400> 1214
catggttcca ccagcgttat t 21

<210> 1215
<211> 23
<212> DNA
<213> Artificial Sequence

<220>
<223> Primer

<400> 1215
acaacaacca ccctggtgat aag 23

<210> 1216
<211> 19
<212> DNA
<213> Artificial Sequence

<220>
<223> Primer

<400> 1216
ctgagaagcc ctgcccttc 19

<210> 1217
<211> 23
<212> DNA
<213> Artificial Sequence

<220>
<223> Primer

<400> 1217
acaacaacca ccctggtgat aag 23

<210> 1218
<211> 28
<212> DNA
<213> Artificial Sequence

<220>
<223> Primer

<400> 1218
tattgtctgt acttgtccaa tgatatgt 28

<210> 1219
<211> 27
<212> DNA
<213> Artificial Sequence

<220>
<223> Primer

<400> 1219
tgcattgtga cagaaaaaga cgatttc 27

<210> 1220
<211> 19
<212> DNA
<213> Artificial Sequence

<220>
<223> Primer

<400> 1220
acgtcttgca gcgttggta 19

<210> 1221
<211> 29
<212> DNA

<213> Artificial Sequence

<220>
<223> Primer

<400> 1221
agaatgttag tgtttcagtt tcaaaatcc 29

<210> 1222
<211> 26
<212> DNA
<213> Artificial Sequence

<220>
<223> Primer

<400> 1222
ttttctttgt cctcgtcgtt atccaa 26

<210> 1223
<211> 23
<212> DNA
<213> Artificial Sequence

<220>
<223> Primer

<400> 1223
acaacaacca ccctggtgat aag 23

<210> 1224
<211> 22
<212> DNA
<213> Artificial Sequence

<220>
<223> Primer

<400> 1224
atcatgatgg ctgtatgtgc ca 22

<210> 1225
<211> 27
<212> DNA
<213> Artificial Sequence

<220>
<223> Primer

<400> 1225
gtgccagaac aaaatactag actgttt 27

<210> 1226
<211> 19
<212> DNA
<213> Artificial Sequence

<220>

<223> Primer

<400> 1226
ctgagaagcc ctgcccttc 19

<210> 1227
<211> 25
<212> DNA
<213> Artificial Sequence

<220>
<223> Primer

<400> 1227
gcaccacttg agtgaggtat tagaa 25

<210> 1228
<211> 30
<212> DNA
<213> Artificial Sequence

<220>
<223> Primer

<400> 1228
tgaaactgaa acactaacat tctactgtgt 30

<210> 1229
<211> 25
<212> DNA
<213> Artificial Sequence

<220>
<223> Primer

<400> 1229
acagcaagct agacaagcta aacaa 25

<210> 1230
<211> 20
<212> DNA
<213> Artificial Sequence

<220>
<223> Primer

<400> 1230
tgtacaacac gcaggtcctc 20

<210> 1231
<211> 23
<212> DNA
<213> Artificial Sequence

<220>
<223> Primer

<400> 1231

gttaacagta acgtgcccac tct 23

<210> 1232
<211> 19
<212> DNA
<213> Artificial Sequence

<220>
<223> Primer

<400> 1232
ctgagaagcc ctgcccttc 19

<210> 1233
<211> 23
<212> DNA
<213> Artificial Sequence

<220>
<223> Primer

<400> 1233
gttaacagta acgtgcccac tct 23

<210> 1234
<211> 24
<212> DNA
<213> Artificial Sequence

<220>
<223> Primer

<400> 1234
acaacctttg aaacaggtgt tgga 24

<210> 1235
<211> 25
<212> DNA
<213> Artificial Sequence

<220>
<223> Primer

<400> 1235
gcaccacttg agtgaggtat tagaa 25

<210> 1236
<211> 19
<212> DNA
<213> Artificial Sequence

<220>
<223> Primer

<400> 1236
acgtcttgca gcgttggta 19

<210> 1237

<211> 27
<212> DNA
<213> Artificial Sequence

<220>
<223> Primer

<400> 1237
gtgccagaac aaaatactag actgttt 27

<210> 1238
<211> 24
<212> DNA
<213> Artificial Sequence

<220>
<223> Primer

<400> 1238
ggtgatccag actctgacct tttg 24

<210> 1239
<211> 20
<212> DNA
<213> Artificial Sequence

<220>
<223> Primer

<400> 1239
atcatgcagg cagttcacga 20

<210> 1240
<211> 28
<212> DNA
<213> Artificial Sequence

<220>
<223> Primer

<400> 1240
caaccgtacc ctaaataccc tatattga 28

<210> 1241
<211> 22
<212> DNA
<213> Artificial Sequence

<220>
<223> Primer

<400> 1241
caagacagcg ggtatggcaa ta 22

<210> 1242
<211> 18
<212> DNA
<213> Artificial Sequence

<220>
<223> Primer

<400> 1242
ggtggtggtg gtggtctt 18

<210> 1243
<211> 22
<212> DNA
<213> Artificial Sequence

<220>
<223> Primer

<400> 1243
caagacagcg ggtatggcaa ta 22

<210> 1244
<211> 26
<212> DNA
<213> Artificial Sequence

<220>
<223> Primer

<400> 1244
ttttctttgt cctcgtcgtt atccaa 26

<210> 1245
<211> 25
<212> DNA
<213> Artificial Sequence

<220>
<223> Primer

<400> 1245
gcaccacttg agtgaggtat tagaa 25

<210> 1246
<211> 28
<212> DNA
<213> Artificial Sequence

<220>
<223> Primer

<400> 1246
acaataaaca tactctgcac actgcata 28

<210> 1247
<211> 23
<212> DNA
<213> Artificial Sequence

<220>
<223> Primer

<400> 1247
acaacaacca ccctggtgat aag 23

<210> 1248
<211> 24
<212> DNA
<213> Artificial Sequence

<220>
<223> Primer

<400> 1248
acaacctttg aaacaggtgt tgga 24

<210> 1249
<211> 25
<212> DNA
<213> Artificial Sequence

<220>
<223> Primer

<400> 1249
gcaccacttg agtgaggtat tagaa 25

<210> 1250
<211> 26
<212> DNA
<213> Artificial Sequence

<220>
<223> Primer

<400> 1250
ttttctttgt cctcgtcgtt atccaa 26

<210> 1251
<211> 22
<212> DNA
<213> Artificial Sequence

<220>
<223> Primer

<400> 1251
caagacagcg ggtatggcaa ta 22

<210> 1252
<211> 30
<212> DNA
<213> Artificial Sequence

<220>
<223> Primer

<400> 1252
tgaaactgaa acactaacat tctactgtgt 30

<210> 1253
<211> 23
<212> DNA
<213> Artificial Sequence

<220>
<223> Primer

<400> 1253
gttaacagta acgtgcccac tct 23

<210> 1254
<211> 18
<212> DNA
<213> Artificial Sequence

<220>
<223> Primer

<400> 1254
ggtggtggtg gtggtctt 18

<210> 1255
<211> 22
<212> DNA
<213> Artificial Sequence

<220>
<223> Primer

<400> 1255
caagacagcg ggtatggcaa ta 22

<210> 1256
<211> 25
<212> DNA
<213> Artificial Sequence

<220>
<223> Primer

<400> 1256
ggtactgttt tgtgagcctc cattt 25

<210> 1257
<211> 23
<212> DNA
<213> Artificial Sequence

<220>
<223> Primer

<400> 1257
gttaacagta acgtgcccac tct 23

<210> 1258
<211> 19
<212> DNA

<213> Artificial Sequence

<220>
<223> Primer

<400> 1258
aaatacggct gcaccgagt 19

<210> 1259
<211> 23
<212> DNA
<213> Artificial Sequence

<220>
<223> Primer

<400> 1259
acaacaacca ccctggtgat aag 23

<210> 1260
<211> 24
<212> DNA
<213> Artificial Sequence

<220>
<223> Primer

<400> 1260
ggtgatccag actctgacct tttg 24

<210> 1261
<211> 23
<212> DNA
<213> Artificial Sequence

<220>
<223> Primer

<400> 1261
acaacaacca ccctggtgat aag 23

<210> 1262
<211> 21
<212> DNA
<213> Artificial Sequence

<220>
<223> Primer

<400> 1262
catggttcca ccagcgttat t 21

<210> 1263
<211> 27
<212> DNA
<213> Artificial Sequence

<220>

<223> Primer

<400> 1263
gtgccagaac aaaatactag actgttt 27

<210> 1264
<211> 19
<212> DNA
<213> Artificial Sequence

<220>
<223> Primer

<400> 1264
aaatacggct gcaccgagt 19

<210> 1265
<211> 23
<212> DNA
<213> Artificial Sequence

<220>
<223> Primer

<400> 1265
agggatcctc ctttgcatta tgg 23

<210> 1266
<211> 24
<212> DNA
<213> Artificial Sequence

<220>
<223> Primer

<400> 1266
acaacctttg aaacaggtgt tgga 24

<210> 1267
<211> 25
<212> DNA
<213> Artificial Sequence

<220>
<223> Primer

<400> 1267
gcaccacttg agtgaggtat tagaa 25

<210> 1268
<211> 24
<212> DNA
<213> Artificial Sequence

<220>
<223> Primer

<400> 1268

caattgcttt tcctccggag ttaa 24

<210> 1269
<211> 23
<212> DNA
<213> Artificial Sequence

<220>
<223> Primer

<400> 1269
gttaacagta acgtgcccac tct 23

<210> 1270
<211> 28
<212> DNA
<213> Artificial Sequence

<220>
<223> Primer

<400> 1270
caaccgtacc ctaaataccc tatattga 28

<210> 1271
<211> 23
<212> DNA
<213> Artificial Sequence

<220>
<223> Primer

<400> 1271
gttaacagta acgtgcccac tct 23

<210> 1272
<211> 22
<212> DNA
<213> Artificial Sequence

<220>
<223> Primer

<400> 1272
atcatgatgg ctgtatgtgc ca 22

<210> 1273
<211> 23
<212> DNA
<213> Artificial Sequence

<220>
<223> Primer

<400> 1273
gttaacagta acgtgcccac tct 23

<210> 1274

<211> 26
<212> DNA
<213> Artificial Sequence

<220>
<223> Primer

<400> 1274
ttttctttgt cctcgtcgtt atccaa 26

<210> 1275
<211> 27
<212> DNA
<213> Artificial Sequence

<220>
<223> Primer

<400> 1275
tcagtgtatg gagctacact agaaagt 27

<210> 1276
<211> 24
<212> DNA
<213> Artificial Sequence

<220>
<223> Primer

<400> 1276
caattgcttt tcctccggag ttaa 24

<210> 1277
<211> 23
<212> DNA
<213> Artificial Sequence

<220>
<223> Primer

<400> 1277
gttaacagta acgtgcccac tct 23

<210> 1278
<211> 18
<212> DNA
<213> Artificial Sequence

<220>
<223> Primer

<400> 1278
tctttgctcg cagctcgt 18

<210> 1279
<211> 23
<212> DNA
<213> Artificial Sequence

<220>
<223> Primer

<400> 1279
acaacaacca ccctggtgat aag 23

<210> 1280
<211> 28
<212> DNA
<213> Artificial Sequence

<220>
<223> Primer

<400> 1280
caaccgtacc ctaaataccc tatattga 28

<210> 1281
<211> 23
<212> DNA
<213> Artificial Sequence

<220>
<223> Primer

<400> 1281
gttaacagta acgtgcccac tct 23

<210> 1282
<211> 21
<212> DNA
<213> Artificial Sequence

<220>
<223> Primer

<400> 1282
catggttcca ccagcgttat t 21

<210> 1283
<211> 23
<212> DNA
<213> Artificial Sequence

<220>
<223> Primer

<400> 1283
acaacaacca ccctggtgat aag 23

<210> 1284
<211> 18
<212> DNA
<213> Artificial Sequence

<220>
<223> Primer

<400> 1284
tctttgctcg cagctcgt 18

<210> 1285
<211> 25
<212> DNA
<213> Artificial Sequence

<220>
<223> Primer

<400> 1285
gcaccacttg agtgaggtat tagaa 25

<210> 1286
<211> 18
<212> DNA
<213> Artificial Sequence

<220>
<223> Primer

<400> 1286
ggtggtggtg gtggtctt 18

<210> 1287
<211> 25
<212> DNA
<213> Artificial Sequence

<220>
<223> Primer

<400> 1287
gcaccacttg agtgaggtat tagaa 25

<210> 1288
<211> 20
<212> DNA
<213> Artificial Sequence

<220>
<223> Primer

<400> 1288
tgtacaacac gcaggtcctc 20

<210> 1289
<211> 28
<212> DNA
<213> Artificial Sequence

<220>
<223> Primer

<400> 1289
tgctacgcat atatattgca accattga 28

<210> 1290
<211> 28
<212> DNA
<213> Artificial Sequence

<220>
<223> Primer

<400> 1290
ggatgtggct ataacaaacc aaaacaat 28

<210> 1291
<211> 21
<212> DNA
<213> Artificial Sequence

<220>
<223> Primer

<400> 1291
aattcggttg catggcctag t 21

<210> 1292
<211> 26
<212> DNA
<213> Artificial Sequence

<220>
<223> Primer

<400> 1292
gggtgcggta ctgtacataa ttcaag 26

<210> 1293
<211> 21
<212> DNA
<213> Artificial Sequence

<220>
<223> Primer

<400> 1293
tgtactcccg ctatgggtga a 21

<210> 1294
<211> 25
<212> DNA
<213> Artificial Sequence

<220>
<223> Primer

<400> 1294
gtgtctatca tgtccccatc ctcta 25

<210> 1295
<211> 25
<212> DNA

<213> Artificial Sequence

<220>
<223> Primer

<400> 1295
cagatgatag ccaaattgcg tttca 25

<210> 1296
<211> 27
<212> DNA
<213> Artificial Sequence

<220>
<223> Primer

<400> 1296
gctgttgtgc ccttttataa tgtctac 27

<210> 1297
<211> 23
<212> DNA
<213> Artificial Sequence

<220>
<223> Primer

<400> 1297
tgcttatggg cacatgtacc att 23

<210> 1298
<211> 23
<212> DNA
<213> Artificial Sequence

<220>
<223> Primer

<400> 1298
gcttacaacc ttagacacag gca 23

<210> 1299
<211> 27
<212> DNA
<213> Artificial Sequence

<220>
<223> Primer

<400> 1299
gaggaggact acacagtaca actaact 27

<210> 1300
<211> 22
<212> DNA
<213> Artificial Sequence

<220>

<223> Primer

<400> 1300
atcatgatgg ctgtatgtgc ca 22

<210> 1301
<211> 23
<212> DNA
<213> Artificial Sequence

<220>
<223> Primer

<400> 1301
tgcttatggg cacatgtacc att 23

<210> 1302
<211> 24
<212> DNA
<213> Artificial Sequence

<220>
<223> Primer

<400> 1302
ggtgatccag actctgacct tttg 24

<210> 1303
<211> 28
<212> DNA
<213> Artificial Sequence

<220>
<223> Primer

<400> 1303
aaaattattg agctagaaga cagcggat 28

<210> 1304
<211> 23
<212> DNA
<213> Artificial Sequence

<220>
<223> Primer

<400> 1304
ccctgtgtac tttcgttgtt ggt 23

<210> 1305
<211> 28
<212> DNA
<213> Artificial Sequence

<220>
<223> Primer

<400> 1305

aaaattattg agctagaaga cagcggat 28

<210> 1306
<211> 25
<212> DNA
<213> Artificial Sequence

<220>
<223> Primer

<400> 1306
ccccactaga ctccgagtca tttaa 25

<210> 1307
<211> 27
<212> DNA
<213> Artificial Sequence

<220>
<223> Primer

<400> 1307
gaggaggact acacagtaca actaact 27

<210> 1308
<211> 24
<212> DNA
<213> Artificial Sequence

<220>
<223> Primer

<400> 1308
ggtgatccag actctgacct tttg 24

<210> 1309
<211> 28
<212> DNA
<213> Artificial Sequence

<220>
<223> Primer

<400> 1309
gcaggacaaa atcctagaca tatacgaa 28

<210> 1310
<211> 18
<212> DNA
<213> Artificial Sequence

<220>
<223> Primer

<400> 1310
tctttgctcg cagctcgt 18

<210> 1311

<211> 27
<212> DNA
<213> Artificial Sequence

<220>
<223> Primer

<400> 1311
gaggaggact acacagtaca actaact 27

<210> 1312
<211> 21
<212> DNA
<213> Artificial Sequence

<220>
<223> Primer

<400> 1312
catggttcca ccagcgttat t 21

<210> 1313
<211> 23
<212> DNA
<213> Artificial Sequence

<220>
<223> Primer

<400> 1313
tgcttatggg cacatgtacc att 23

<210> 1314
<211> 27
<212> DNA
<213> Artificial Sequence

<220>
<223> Primer

<400> 1314
ctgttcttcg ttctattacc gcttcta 27

<210> 1315
<211> 23
<212> DNA
<213> Artificial Sequence

<220>
<223> Primer

<400> 1315
tgcttatggg cacatgtacc att 23

<210> 1316
<211> 22
<212> DNA
<213> Artificial Sequence

<220>
<223> Primer

<400> 1316
atcatgatgg ctgtatgtgc ca 22

<210> 1317
<211> 30
<212> DNA
<213> Artificial Sequence

<220>
<223> Primer

<400> 1317
attagatggt aacgacattt caatagatgt 30

<210> 1318
<211> 31
<212> DNA
<213> Artificial Sequence

<220>
<223> Primer

<400> 1318
tgcatcaaat ggaaatggat tgttaaattc a 31

<210> 1319
<211> 20
<212> DNA
<213> Artificial Sequence

<220>
<223> Primer

<400> 1319
gtcaggcgtt ggagacatct 20

<210> 1320
<211> 31
<212> DNA
<213> Artificial Sequence

<220>
<223> Primer

<400> 1320
tgcatcaaat ggaaatggat tgttaaattc a 31

<210> 1321
<211> 28
<212> DNA
<213> Artificial Sequence

<220>
<223> Primer

<400> 1321
gcaggacaaa atcctagaca tatacgaa 28

<210> 1322
<211> 24
<212> DNA
<213> Artificial Sequence

<220>
<223> Primer

<400> 1322
ggtgatccag actctgacct tttg 24

<210> 1323
<211> 23
<212> DNA
<213> Artificial Sequence

<220>
<223> Primer

<400> 1323
tgcttatggg cacatgtacc att 23

<210> 1324
<211> 19
<212> DNA
<213> Artificial Sequence

<220>
<223> Primer

<400> 1324
aaatacggct gcaccgagt 19

<210> 1325
<211> 20
<212> DNA
<213> Artificial Sequence

<220>
<223> Primer

<400> 1325
gtcaggcgtt ggagacatct 20

<210> 1326
<211> 28
<212> DNA
<213> Artificial Sequence

<220>
<223> Primer

<400> 1326
cgacccgaaa tattatgaaa ccttttgt 28

<210> 1327
<211> 23
<212> DNA
<213> Artificial Sequence

<220>
<223> Primer

<400> 1327
tgcttatggg cacatgtacc att 23

<210> 1328
<211> 18
<212> DNA
<213> Artificial Sequence

<220>
<223> Primer

<400> 1328
tctttgctcg cagctcgt 18

<210> 1329
<211> 20
<212> DNA
<213> Artificial Sequence

<220>
<223> Primer

<400> 1329
gtcaggcgtt ggagacatct 20

<210> 1330
<211> 28
<212> DNA
<213> Artificial Sequence

<220>
<223> Primer

<400> 1330
tgatatttcc tccatcgttt tccttgtc 28

<210> 1331
<211> 29
<212> DNA
<213> Artificial Sequence

<220>
<223> Primer

<400> 1331
cgctatatgg agacacatta gaacaaaca 29

<210> 1332
<211> 28
<212> DNA

<213> Artificial Sequence

<220>
<223> Primer

<400> 1332
cgacccgaaa tattatgaaa ccttttgt 28

<210> 1333
<211> 26
<212> DNA
<213> Artificial Sequence

<220>
<223> Primer

<400> 1333
acaattatgg gaggtacatg tgggta 26

<210> 1334
<211> 23
<212> DNA
<213> Artificial Sequence

<220>
<223> Primer

<400> 1334
ccctgtgtac tttcgttgtt ggt 23

<210> 1335
<211> 28
<212> DNA
<213> Artificial Sequence

<220>
<223> Primer

<400> 1335
aaaattattg agctagaaga cagcggat 28

<210> 1336
<211> 31
<212> DNA
<213> Artificial Sequence

<220>
<223> Primer

<400> 1336
tgcatcaaat ggaaatggat tgttaaattc a 31

<210> 1337
<211> 28
<212> DNA
<213> Artificial Sequence

<220>

<223> Primer

<400> 1337
aaaattattg agctagaaga cagcggat 28

<210> 1338
<211> 28
<212> DNA
<213> Artificial Sequence

<220>
<223> Primer

<400> 1338
tgatatttcc tccatcgttt tccttgtc 28

<210> 1339
<211> 28
<212> DNA
<213> Artificial Sequence

<220>
<223> Primer

<400> 1339
gcaggacaaa atcctagaca tatacgaa 28

<210> 1340
<211> 19
<212> DNA
<213> Artificial Sequence

<220>
<223> Primer

<400> 1340
aaatacggct gcaccgagt 19

<210> 1341
<211> 20
<212> DNA
<213> Artificial Sequence

<220>
<223> Primer

<400> 1341
gtcaggcgtt ggagacatct 20

<210> 1342
<211> 21
<212> DNA
<213> Artificial Sequence

<220>
<223> Primer

<400> 1342

gcgttgggtt gtttcctctc a 21

<210> 1343
<211> 23
<212> DNA
<213> Artificial Sequence

<220>
<223> Primer

<400> 1343
tgcttatggg cacatgtacc att 23

<210> 1344
<211> 21
<212> DNA
<213> Artificial Sequence

<220>
<223> Primer

<400> 1344
catggttcca ccagcgttat t 21

<210> 1345
<211> 23
<212> DNA
<213> Artificial Sequence

<220>
<223> Primer

<400> 1345
tgcttatggg cacatgtacc att 23

<210> 1346
<211> 19
<212> DNA
<213> Artificial Sequence

<220>
<223> Primer

<400> 1346
ctgagaagcc ctgcccttc 19

<210> 1347
<211> 23
<212> DNA
<213> Artificial Sequence

<220>
<223> Primer

<400> 1347
tgcttatggg cacatgtacc att 23

<210> 1348

<211> 28
<212> DNA
<213> Artificial Sequence

<220>
<223> Primer

<400> 1348
tgatatttcc tccatcgttt tccttgtc 28

<210> 1349
<211> 27
<212> DNA
<213> Artificial Sequence

<220>
<223> Primer

<400> 1349
gaggaggact acacagtaca actaact 27

<210> 1350
<211> 18
<212> DNA
<213> Artificial Sequence

<220>
<223> Primer

<400> 1350
tctttgctcg cagctcgt 18

<210> 1351
<211> 28
<212> DNA
<213> Artificial Sequence

<220>
<223> Primer

<400> 1351
gcaggacaaa atcctagaca tatacgaa 28

<210> 1352
<211> 21
<212> DNA
<213> Artificial Sequence

<220>
<223> Primer

<400> 1352
catggttcca ccagcgttat t 21

<210> 1353
<211> 20
<212> DNA
<213> Artificial Sequence

<220>
<223> Primer

<400> 1353
gtcaggcgtt ggagacatct 20

<210> 1354
<211> 23
<212> DNA
<213> Artificial Sequence

<220>
<223> Primer

<400> 1354
ccctgtgtac tttcgttgtt ggt 23

<210> 1355
<211> 28
<212> DNA
<213> Artificial Sequence

<220>
<223> Primer

<400> 1355
gcaggacaaa atcctagaca tatacgaa 28

<210> 1356
<211> 22
<212> DNA
<213> Artificial Sequence

<220>
<223> Primer

<400> 1356
atcatgatgg ctgtatgtgc ca 22

<210> 1357
<211> 27
<212> DNA
<213> Artificial Sequence

<220>
<223> Primer

<400> 1357
gaggaggact acacagtaca actaact 27

<210> 1358
<211> 19
<212> DNA
<213> Artificial Sequence

<220>
<223> Primer

<400> 1358
aaatacggct gcaccgagt 19

<210> 1359
<211> 27
<212> DNA
<213> Artificial Sequence

<220>
<223> Primer

<400> 1359
gaggaggact acacagtaca actaact 27

<210> 1360
<211> 27
<212> DNA
<213> Artificial Sequence

<220>
<223> Primer

<400> 1360
ccaatgccat gtggatgaca tattaca 27

<210> 1361
<211> 28
<212> DNA
<213> Artificial Sequence

<220>
<223> Primer

<400> 1361
ctgattttat gttgcaccct agctattt 28

<210> 1362
<211> 23
<212> DNA
<213> Artificial Sequence

<220>
<223> Primer

<400> 1362
gcttacaacc ttagacacag gca 23

<210> 1363
<211> 27
<212> DNA
<213> Artificial Sequence

<220>
<223> Primer

<400> 1363
gaggaggact acacagtaca actaact 27

<210> 1364
<211> 19
<212> DNA
<213> Artificial Sequence

<220>
<223> Primer

<400> 1364
ctgagaagcc ctgcccttc 19

<210> 1365
<211> 20
<212> DNA
<213> Artificial Sequence

<220>
<223> Primer

<400> 1365
gtcaggcgtt ggagacatct 20

<210> 1366
<211> 27
<212> DNA
<213> Artificial Sequence

<220>
<223> Primer

<400> 1366
tcgtaagcac actttacata ctgcaaa 27

<210> 1367
<211> 23
<212> DNA
<213> Artificial Sequence

<220>
<223> Primer

<400> 1367
tgcttatggg cacatgtacc att 23

<210> 1368
<211> 31
<212> DNA
<213> Artificial Sequence

<220>
<223> Primer

<400> 1368
tgcatcaaat ggaaatggat tgttaaattc a 31

<210> 1369
<211> 23
<212> DNA

<213> Artificial Sequence

<220>
<223> Primer

<400> 1369
tgcttatggg cacatgtacc att 23

<210> 1370
<211> 23
<212> DNA
<213> Artificial Sequence

<220>
<223> Primer

<400> 1370
ccctgtgtac tttcgttgtt ggt 23

<210> 1371
<211> 27
<212> DNA
<213> Artificial Sequence

<220>
<223> Primer

<400> 1371
gaggaggact acacagtaca actaact 27

<210> 1372
<211> 23
<212> DNA
<213> Artificial Sequence

<220>
<223> Primer

<400> 1372
gcttacaacc ttagacacag gca 23

<210> 1373
<211> 28
<212> DNA
<213> Artificial Sequence

<220>
<223> Primer

<400> 1373
gcaggacaaa atcctagaca tatacgaa 28

<210> 1374
<211> 19
<212> DNA
<213> Artificial Sequence

<220>

<223> Primer

<400> 1374
ctgagaagcc ctgcccttc 19

<210> 1375
<211> 21
<212> DNA
<213> Artificial Sequence

<220>
<223> Primer

<400> 1375
cgtttggtct gggcatgtgt a 21

<210> 1376
<211> 23
<212> DNA
<213> Artificial Sequence

<220>
<223> Primer

<400> 1376
gctgtgcggg atatctgtta ctg 23

<210> 1377
<211> 27
<212> DNA
<213> Artificial Sequence

<220>
<223> Primer

<400> 1377
tctatatatg cttggttgct ggtgttg 27

<210> 1378
<211> 27
<212> DNA
<213> Artificial Sequence

<220>
<223> Primer

<400> 1378
catgtgcaga accagtatac agttagt 27

<210> 1379
<211> 27
<212> DNA
<213> Artificial Sequence

<220>
<223> Primer

<400> 1379

caatgggaca atggatacaa agtaggt 27

<210> 1380
<211> 23
<212> DNA
<213> Artificial Sequence

<220>
<223> Primer

<400> 1380
gggccacaca gtaacataca act 23

<210> 1381
<211> 27
<212> DNA
<213> Artificial Sequence

<220>
<223> Primer

<400> 1381
tctatgagta aggtgctgtc cctaaat 27

<210> 1382
<211> 26
<212> DNA
<213> Artificial Sequence

<220>
<223> Primer

<400> 1382
ggaggtaaag taaaatggag gcagta 26

<210> 1383
<211> 19
<212> DNA
<213> Artificial Sequence

<220>
<223> Primer

<400> 1383
tccgtttgca tccaggcaa 19

<210> 1384
<211> 28
<212> DNA
<213> Artificial Sequence

<220>
<223> Primer

<400> 1384
ccaatgccag gtagaggaaa tattttca 28

<210> 1385

<211> 27
<212> DNA
<213> Artificial Sequence

<220>
<223> Primer

<400> 1385
gcgtttaagt gtgttacagg atcaaat 27

<210> 1386
<211> 19
<212> DNA
<213> Artificial Sequence

<220>
<223> Primer

<400> 1386
ctgagaagcc ctgcccttc 19

<210> 1387
<211> 27
<212> DNA
<213> Artificial Sequence

<220>
<223> Primer

<400> 1387
aaagaaggtt aataacagtg ccagaca 27

<210> 1388
<211> 19
<212> DNA
<213> Artificial Sequence

<220>
<223> Primer

<400> 1388
cccaagtacg tggcttcgg 19

<210> 1389
<211> 28
<212> DNA
<213> Artificial Sequence

<220>
<223> Primer

<400> 1389
agatagaaag cataggcacc tagtacaa 28

<210> 1390
<211> 31
<212> DNA
<213> Artificial Sequence

<220>
<223> Primer

<400> 1390
tctatttttg tcaaatggca atttgtttgg a 31

<210> 1391
<211> 28
<212> DNA
<213> Artificial Sequence

<220>
<223> Primer

<400> 1391
cagatggagt taatcatcct ttgctact 28

<210> 1392
<211> 19
<212> DNA
<213> Artificial Sequence

<220>
<223> Primer

<400> 1392
tgtaaggctc gcaatccgt 19

<210> 1393
<211> 25
<212> DNA
<213> Artificial Sequence

<220>
<223> Primer

<400> 1393
actatccttt gtgtgtcctt tgtgt 25

<210> 1394
<211> 21
<212> DNA
<213> Artificial Sequence

<220>
<223> Primer

<400> 1394
catggttcca ccagcgttat t 21

<210> 1395
<211> 27
<212> DNA
<213> Artificial Sequence

<220>
<223> Primer

<400> 1395
gcgtttaagt gtgttacagg atcaaat 27

<210> 1396
<211> 21
<212> DNA
<213> Artificial Sequence

<220>
<223> Primer

<400> 1396
catggttcca ccagcgttat t 21

<210> 1397
<211> 25
<212> DNA
<213> Artificial Sequence

<220>
<223> Primer

<400> 1397
actatccttt gtgtgtcctt tgtgt 25

<210> 1398
<211> 22
<212> DNA
<213> Artificial Sequence

<220>
<223> Primer

<400> 1398
atcatgatgg ctgtatgtgc ca 22

<210> 1399
<211> 19
<212> DNA
<213> Artificial Sequence

<220>
<223> Primer

<400> 1399
tccgtttgca tccaggcaa 19

<210> 1400
<211> 19
<212> DNA
<213> Artificial Sequence

<220>
<223> Primer

<400> 1400
ctgagaagcc ctgcccttc 19

<210> 1401
<211> 27
<212> DNA
<213> Artificial Sequence

<220>
<223> Primer

<400> 1401
aaagaaggtt aataacagtg ccagaca 27

<210> 1402
<211> 21
<212> DNA
<213> Artificial Sequence

<220>
<223> Primer

<400> 1402
ggtgtccatc actgtctgca t 21

<210> 1403
<211> 19
<212> DNA
<213> Artificial Sequence

<220>
<223> Primer

<400> 1403
tccgtttgca tccaggcaa 19

<210> 1404
<211> 25
<212> DNA
<213> Artificial Sequence

<220>
<223> Primer

<400> 1404
tgacatactc atcagtgctg acaac 25

<210> 1405
<211> 19
<212> DNA
<213> Artificial Sequence

<220>
<223> Primer

<400> 1405
tccgtttgca tccaggcaa 19

<210> 1406
<211> 19
<212> DNA

<213> Artificial Sequence

<220>
<223> Primer

<400> 1406
aaatacggct gcaccgagt 19

<210> 1407
<211> 27
<212> DNA
<213> Artificial Sequence

<220>
<223> Primer

<400> 1407
gcgtttaagt gtgttacagg atcaaat 27

<210> 1408
<211> 24
<212> DNA
<213> Artificial Sequence

<220>
<223> Primer

<400> 1408
ggtgatccag actctgacct tttg 24

<210> 1409
<211> 28
<212> DNA
<213> Artificial Sequence

<220>
<223> Primer

<400> 1409
gtatgtcacc cgtaccagta ttttctac 28

<210> 1410
<211> 27
<212> DNA
<213> Artificial Sequence

<220>
<223> Primer

<400> 1410
gccaaattta ttgggatcag gtaactt 27

<210> 1411
<211> 25
<212> DNA
<213> Artificial Sequence

<220>

<223> Primer

<400> 1411
actatccttt gtgtgtcctt tgtgt 25

<210> 1412
<211> 19
<212> DNA
<213> Artificial Sequence

<220>
<223> Primer

<400> 1412
cccaagtacg tggcttcgg 19

<210> 1413
<211> 25
<212> DNA
<213> Artificial Sequence

<220>
<223> Primer

<400> 1413
gcatcaattg tgtgttttgc aaagg 25

<210> 1414
<211> 31
<212> DNA
<213> Artificial Sequence

<220>
<223> Primer

<400> 1414
tctatttttg tcaaatggca atttgtttgg a 31

<210> 1415
<211> 19
<212> DNA
<213> Artificial Sequence

<220>
<223> Primer

<400> 1415
tccgtttgca tccaggcaa 19

<210> 1416
<211> 24
<212> DNA
<213> Artificial Sequence

<220>
<223> Primer

<400> 1416

ggtgatccag actctgacct tttg 24

<210> 1417
<211> 25
<212> DNA
<213> Artificial Sequence

<220>
<223> Primer

<400> 1417
actatccttt gtgtgtcctt tgtgt 25

<210> 1418
<211> 24
<212> DNA
<213> Artificial Sequence

<220>
<223> Primer

<400> 1418
ggtgatccag actctgacct tttg 24

<210> 1419
<211> 19
<212> DNA
<213> Artificial Sequence

<220>
<223> Primer

<400> 1419
tccgtttgca tccaggcaa 19

<210> 1420
<211> 18
<212> DNA
<213> Artificial Sequence

<220>
<223> Primer

<400> 1420
tctttgctcg cagctcgt 18

<210> 1421
<211> 27
<212> DNA
<213> Artificial Sequence

<220>
<223> Primer

<400> 1421
gcgtttaagt gtgttacagg atcaaat 27

<210> 1422

<211> 19
<212> DNA
<213> Artificial Sequence

<220>
<223> Primer

<400> 1422
aaatacggct gcaccgagt 19

<210> 1423
<211> 25
<212> DNA
<213> Artificial Sequence

<220>
<223> Primer

<400> 1423
actatccttt gtgtgtcctt tgtgt 25

<210> 1424
<211> 25
<212> DNA
<213> Artificial Sequence

<220>
<223> Primer

<400> 1424
tgacatactc atcagtgctg acaac 25

<210> 1425
<211> 25
<212> DNA
<213> Artificial Sequence

<220>
<223> Primer

<400> 1425
actatccttt gtgtgtcctt tgtgt 25

<210> 1426
<211> 19
<212> DNA
<213> Artificial Sequence

<220>
<223> Primer

<400> 1426
aaatacggct gcaccgagt 19

<210> 1427
<211> 25
<212> DNA
<213> Artificial Sequence

<220>
<223> Primer

<400> 1427
actatccttt gtgtgtcctt tgtgt 25

<210> 1428
<211> 27
<212> DNA
<213> Artificial Sequence

<220>
<223> Primer

<400> 1428
gccaaattta ttgggatcag gtaactt 27

<210> 1429
<211> 25
<212> DNA
<213> Artificial Sequence

<220>
<223> Primer

<400> 1429
actatccttt gtgtgtcctt tgtgt 25

<210> 1430
<211> 28
<212> DNA
<213> Artificial Sequence

<220>
<223> Primer

<400> 1430
cgtcatctga aattttgtca cctgtttt 28

<210> 1431
<211> 27
<212> DNA
<213> Artificial Sequence

<220>
<223> Primer

<400> 1431
gcgtttaagt gtgttacagg atcaaat 27

<210> 1432
<211> 22
<212> DNA
<213> Artificial Sequence

<220>
<223> Primer

<400> 1432
atcatgatgg ctgtatgtgc ca 22

<210> 1433
<211> 19
<212> DNA
<213> Artificial Sequence

<220>
<223> Primer

<400> 1433
tccgtttgca tccaggcaa 19

<210> 1434
<211> 27
<212> DNA
<213> Artificial Sequence

<220>
<223> Primer

<400> 1434
gccaaattta ttgggatcag gtaactt 27

<210> 1435
<211> 27
<212> DNA
<213> Artificial Sequence

<220>
<223> Primer

<400> 1435
aaagaaggtt aataacagtg ccagaca 27

<210> 1436
<211> 31
<212> DNA
<213> Artificial Sequence

<220>
<223> Primer

<400> 1436
tctatttttg tcaaatggca atttgtttgg a 31

<210> 1437
<211> 25
<212> DNA
<213> Artificial Sequence

<220>
<223> Primer

<400> 1437
actatccttt gtgtgtcctt tgtgt 25

<210> 1438
<211> 31
<212> DNA
<213> Artificial Sequence

<220>
<223> Primer

<400> 1438
tctatttttg tcaaatggca atttgtttgg a 31

<210> 1439
<211> 27
<212> DNA
<213> Artificial Sequence

<220>
<223> Primer

<400> 1439
aaagaaggtt aataacagtg ccagaca 27

<210> 1440
<211> 26
<212> DNA
<213> Artificial Sequence

<220>
<223> Primer

<400> 1440
gtctatttga ctgtcgctac aaacac 26

<210> 1441
<211> 23
<212> DNA
<213> Artificial Sequence

<220>
<223> Primer

<400> 1441
cctcgtaaac gtaaacgtgt tcc 23

<210> 1442
<211> 25
<212> DNA
<213> Artificial Sequence

<220>
<223> Primer

<400> 1442
tgacatactc atcagtgctg acaac 25

<210> 1443
<211> 19
<212> DNA

<213> Artificial Sequence

<220>
<223> Primer

<400> 1443
tccgtttgca tccaggcaa 19

<210> 1444
<211> 22
<212> DNA
<213> Artificial Sequence

<220>
<223> Primer

<400> 1444
atcatgatgg ctgtatgtgc ca 22

<210> 1445
<211> 19
<212> DNA
<213> Artificial Sequence

<220>
<223> Primer

<400> 1445
tccgtttgca tccaggcaa 19

<210> 1446
<211> 21
<212> DNA
<213> Artificial Sequence

<220>
<223> Primer

<400> 1446
catggttcca ccagcgttat t 21

<210> 1447
<211> 27
<212> DNA
<213> Artificial Sequence

<220>
<223> Primer

<400> 1447
gcgtttaagt gtgttacagg atcaaat 27

<210> 1448
<211> 18
<212> DNA
<213> Artificial Sequence

<220>

<223> Primer

<400> 1448
tctttgctcg cagctcgt 18

<210> 1449
<211> 25
<212> DNA
<213> Artificial Sequence

<220>
<223> Primer

<400> 1449
gcatcaattg tgtgttttgc aaagg 25

<210> 1450
<211> 19
<212> DNA
<213> Artificial Sequence

<220>
<223> Primer

<400> 1450
cccaagtacg tggcttcgg 19

<210> 1451
<211> 25
<212> DNA
<213> Artificial Sequence

<220>
<223> Primer

<400> 1451
actatccttt gtgtgtcctt tgtgt 25

<210> 1452
<211> 21
<212> DNA
<213> Artificial Sequence

<220>
<223> Primer

<400> 1452
ggtgtccatc actgtctgca t 21

<210> 1453
<211> 25
<212> DNA
<213> Artificial Sequence

<220>
<223> Primer

<400> 1453

gcatcaattg tgtgttttgc aaagg 25

<210> 1454
<211> 21
<212> DNA
<213> Artificial Sequence

<220>
<223> Primer

<400> 1454
ggtgtccatc actgtctgca t 21

<210> 1455
<211> 25
<212> DNA
<213> Artificial Sequence

<220>
<223> Primer

<400> 1455
actatccttt gtgtgtcctt tgtgt 25

<210> 1456
<211> 19
<212> DNA
<213> Artificial Sequence

<220>
<223> Primer

<400> 1456
ctgagaagcc ctgcccttc 19

<210> 1457
<211> 25
<212> DNA
<213> Artificial Sequence

<220>
<223> Primer

<400> 1457
actatccttt gtgtgtcctt tgtgt 25

<210> 1458
<211> 18
<212> DNA
<213> Artificial Sequence

<220>
<223> Primer

<400> 1458
tctttgctcg cagctcgt 18

<210> 1459

<211> 25
<212> DNA
<213> Artificial Sequence

<220>
<223> Primer

<400> 1459
gcatcaattg tgtgttttgc aaagg 25

<210> 1460
<211> 19
<212> DNA
<213> Artificial Sequence

<220>
<223> Primer

<400> 1460
tgtaaggctc gcaatccgt 19

<210> 1461
<211> 25
<212> DNA
<213> Artificial Sequence

<220>
<223> Primer

<400> 1461
gcatcaattg tgtgttttgc aaagg 25

<210> 1462
<211> 22
<212> DNA
<213> Artificial Sequence

<220>
<223> Primer

<400> 1462
gcatttcaga cacgctgcat ac 22

<210> 1463
<211> 20
<212> DNA
<213> Artificial Sequence

<220>
<223> Primer

<400> 1463
gttgcaatgt cccgcttctg 20

<210> 1464
<211> 27
<212> DNA
<213> Artificial Sequence

<220>
<223> Primer

<400> 1464
catgggcata tagtagtaac agtggaa 27

<210> 1465
<211> 23
<212> DNA
<213> Artificial Sequence

<220>
<223> Primer

<400> 1465
ggttgcaccc aatgagtaag gta 23

<210> 1466
<211> 25
<212> DNA
<213> Artificial Sequence

<220>
<223> Primer

<400> 1466
gcaaaactgg acattcagga caaaa 25

<210> 1467
<211> 20
<212> DNA
<213> Artificial Sequence

<220>
<223> Primer

<400> 1467
gctgtgtacc tgccattgga 20

<210> 1468
<211> 26
<212> DNA
<213> Artificial Sequence

<220>
<223> Primer

<400> 1468
ctgtgtctac catatcacca tcttca 26

<210> 1469
<211> 25
<212> DNA
<213> Artificial Sequence

<220>
<223> Primer

<400> 1469
gtgcatgtta attgaaccac ccaaa 25

<210> 1470
<211> 25
<212> DNA
<213> Artificial Sequence

<220>
<223> Primer

<400> 1470
tcaaacacgc tatcatcaac tccat 25

<210> 1471
<211> 26
<212> DNA
<213> Artificial Sequence

<220>
<223> Primer

<400> 1471
cgtgccagaa caaaatacta gactgt 26

<210> 1472
<211> 22
<212> DNA
<213> Artificial Sequence

<220>
<223> Primer

<400> 1472
atcatgatgg ctgtatgtgc ca 22

<210> 1473
<211> 23
<212> DNA
<213> Artificial Sequence

<220>
<223> Primer

<400> 1473
caccatctga gcgaggtatt aca 23

<210> 1474
<211> 21
<212> DNA
<213> Artificial Sequence

<220>
<223> Primer

<400> 1474
tgtaccacac gtagctcctc t 21

<210> 1475
<211> 23
<212> DNA
<213> Artificial Sequence

<220>
<223> Primer

<400> 1475
gttaacagta acgtgcccac tct 23

<210> 1476
<211> 19
<212> DNA
<213> Artificial Sequence

<220>
<223> Primer

<400> 1476
ctgagaagcc ctgcccttc 19

<210> 1477
<211> 23
<212> DNA
<213> Artificial Sequence

<220>
<223> Primer

<400> 1477
caccatctga gcgaggtatt aca 23

<210> 1478
<211> 31
<212> DNA
<213> Artificial Sequence

<220>
<223> Primer

<400> 1478
acaataaaca taccctacat actgcatatg g 31

<210> 1479
<211> 23
<212> DNA
<213> Artificial Sequence

<220>
<223> Primer

<400> 1479
gttaacagta acgtgcccac tct 23

<210> 1480
<211> 24
<212> DNA

<213> Artificial Sequence

<220>
<223> Primer

<400> 1480
ggtgatccag actctgacct tttg 24

<210> 1481
<211> 26
<212> DNA
<213> Artificial Sequence

<220>
<223> Primer

<400> 1481
cgtgccagaa caaaatacta gactgt 26

<210> 1482
<211> 19
<212> DNA
<213> Artificial Sequence

<220>
<223> Primer

<400> 1482
ctgagaagcc ctgcccttc 19

<210> 1483
<211> 22
<212> DNA
<213> Artificial Sequence

<220>
<223> Primer

<400> 1483
gaagacagcg ggtatggcaa ta 22

<210> 1484
<211> 26
<212> DNA
<213> Artificial Sequence

<220>
<223> Primer

<400> 1484
ttttctttgt cctcgtcgtt atccaa 26

<210> 1485
<211> 26
<212> DNA
<213> Artificial Sequence

<220>

<223> Primer

<400> 1485
cgtgccagaa caaaatacta gactgt 26

<210> 1486
<211> 24
<212> DNA
<213> Artificial Sequence

<220>
<223> Primer

<400> 1486
ggtgatccag actctgacct tttg 24

<210> 1487
<211> 22
<212> DNA
<213> Artificial Sequence

<220>
<223> Primer

<400> 1487
gaagacagcg ggtatggcaa ta 22

<210> 1488
<211> 31
<212> DNA
<213> Artificial Sequence

<220>
<223> Primer

<400> 1488
cattacttaa ttcatacaca ggattaccat t 31

<210> 1489
<211> 23
<212> DNA
<213> Artificial Sequence

<220>
<223> Primer

<400> 1489
gtatcaacac acaaagccac tgt 23

<210> 1490
<211> 18
<212> DNA
<213> Artificial Sequence

<220>
<223> Primer

<400> 1490

tctttgctcg cagctcgt 18

<210> 1491
<211> 23
<212> DNA
<213> Artificial Sequence

<220>
<223> Primer

<400> 1491
gttaacagta acgtgcccac tct 23

<210> 1492
<211> 24
<212> DNA
<213> Artificial Sequence

<220>
<223> Primer

<400> 1492
acaacctttg aaacaggtgt tgga 24

<210> 1493
<211> 23
<212> DNA
<213> Artificial Sequence

<220>
<223> Primer

<400> 1493
gtatcaacac acaaagccac tgt 23

<210> 1494
<211> 24
<212> DNA
<213> Artificial Sequence

<220>
<223> Primer

<400> 1494
ggtgatccag actctgacct tttg 24

<210> 1495
<211> 23
<212> DNA
<213> Artificial Sequence

<220>
<223> Primer

<400> 1495
gttaacagta acgtgcccac tct 23

<210> 1496

<211> 18
<212> DNA
<213> Artificial Sequence

<220>
<223> Primer

<400> 1496
ggtggtggtg gtcctgtg 18

<210> 1497
<211> 23
<212> DNA
<213> Artificial Sequence

<220>
<223> Primer

<400> 1497
gtatcaacac acaaagccac tgt 23

<210> 1498
<211> 21
<212> DNA
<213> Artificial Sequence

<220>
<223> Primer

<400> 1498
catggttcca ccagcgttat t 21

<210> 1499
<211> 26
<212> DNA
<213> Artificial Sequence

<220>
<223> Primer

<400> 1499
cgtgccagaa caaaatacta gactgt 26

<210> 1500
<211> 19
<212> DNA
<213> Artificial Sequence

<220>
<223> Primer

<400> 1500
aaatacggct gcaccgagt 19

<210> 1501
<211> 23
<212> DNA
<213> Artificial Sequence

<220>
<223> Primer

<400> 1501
caccatctga gcgaggtatt aca 23

<210> 1502
<211> 26
<212> DNA
<213> Artificial Sequence

<220>
<223> Primer

<400> 1502
ttttctttgt cctcgtcgtt atccaa 26

<210> 1503
<211> 23
<212> DNA
<213> Artificial Sequence

<220>
<223> Primer

<400> 1503
caccatctga gcgaggtatt aca 23

<210> 1504
<211> 22
<212> DNA
<213> Artificial Sequence

<220>
<223> Primer

<400> 1504
aacctcttgc aacgttggta ct 22

<210> 1505
<211> 22
<212> DNA
<213> Artificial Sequence

<220>
<223> Primer

<400> 1505
acagcaagct agacaagctg aa 22

<210> 1506
<211> 21
<212> DNA
<213> Artificial Sequence

<220>
<223> Primer

<400> 1506
tgtaccacac gtagctcctc t 21

<210> 1507
<211> 23
<212> DNA
<213> Artificial Sequence

<220>
<223> Primer

<400> 1507
caccatctga gcgaggtatt aca 23

<210> 1508
<211> 28
<212> DNA
<213> Artificial Sequence

<220>
<223> Primer

<400> 1508
gaaatcgtct tttatgttca cagtgcaa 28

<210> 1509
<211> 23
<212> DNA
<213> Artificial Sequence

<220>
<223> Primer

<400> 1509
caccatctga gcgaggtatt aca 23

<210> 1510
<211> 31
<212> DNA
<213> Artificial Sequence

<220>
<223> Primer

<400> 1510
cattacttaa ttcatacaca ggattaccat t 31

<210> 1511
<211> 23
<212> DNA
<213> Artificial Sequence

<220>
<223> Primer

<400> 1511
gtatcaacac acaaagccac tgt 23

<210> 1512
<211> 24
<212> DNA
<213> Artificial Sequence

<220>
<223> Primer

<400> 1512
acaacctttg aaacaggtgt tgga 24

<210> 1513
<211> 26
<212> DNA
<213> Artificial Sequence

<220>
<223> Primer

<400> 1513
cgtgccagaa caaaatacta gactgt 26

<210> 1514
<211> 21
<212> DNA
<213> Artificial Sequence

<220>
<223> Primer

<400> 1514
catggttcca ccagcgttat t 21

<210> 1515
<211> 23
<212> DNA
<213> Artificial Sequence

<220>
<223> Primer

<400> 1515
gttaacagta acgtgcccac tct 23

<210> 1516
<211> 22
<212> DNA
<213> Artificial Sequence

<220>
<223> Primer

<400> 1516
atcatgatgg ctgtatgtgc ca 22

<210> 1517
<211> 23
<212> DNA

<213> Artificial Sequence

<220>
<223> Primer

<400> 1517
gttaacagta acgtgcccac tct 23

<210> 1518
<211> 28
<212> DNA
<213> Artificial Sequence

<220>
<223> Primer

<400> 1518
ttctacaatt gcttctacct gaaaccat 28

<210> 1519
<211> 23
<212> DNA
<213> Artificial Sequence

<220>
<223> Primer

<400> 1519
caccatctga gcgaggtatt aca 23

<210> 1520
<211> 18
<212> DNA
<213> Artificial Sequence

<220>
<223> Primer

<400> 1520
ggtggtggtg gtcctgtg 18

<210> 1521
<211> 23
<212> DNA
<213> Artificial Sequence

<220>
<223> Primer

<400> 1521
gttaacagta acgtgcccac tct 23

<210> 1522
<211> 31
<212> DNA
<213> Artificial Sequence

<220>

<223> Primer

<400> 1522
cattacttaa ttcatacaca ggattaccat t 31

<210> 1523
<211> 23
<212> DNA
<213> Artificial Sequence

<220>
<223> Primer

<400> 1523
gtatcaacac acaaagccac tgt 23

<210> 1524
<211> 19
<212> DNA
<213> Artificial Sequence

<220>
<223> Primer

<400> 1524
aaatacggct gcaccgagt 19

<210> 1525
<211> 26
<212> DNA
<213> Artificial Sequence

<220>
<223> Primer

<400> 1525
cgtgccagaa caaaatacta gactgt 26

<210> 1526
<211> 18
<212> DNA
<213> Artificial Sequence

<220>
<223> Primer

<400> 1526
tctttgctcg cagctcgt 18

<210> 1527
<211> 23
<212> DNA
<213> Artificial Sequence

<220>
<223> Primer

<400> 1527

gtatcaacac acaaagccac tgt 23

<210> 1528
<211> 22
<212> DNA
<213> Artificial Sequence

<220>
<223> Primer

<400> 1528
atcatgatgg ctgtatgtgc ca 22

<210> 1529
<211> 22
<212> DNA
<213> Artificial Sequence

<220>
<223> Primer

<400> 1529
gaagacagcg ggtatggcaa ta 22

<210> 1530
<211> 18
<212> DNA
<213> Artificial Sequence

<220>
<223> Primer

<400> 1530
ggtggtggtg gtcctgtg 18

<210> 1531
<211> 23
<212> DNA
<213> Artificial Sequence

<220>
<223> Primer

<400> 1531
gttaacagta acgtgcccac tct 23

<210> 1532
<211> 26
<212> DNA
<213> Artificial Sequence

<220>
<223> Primer

<400> 1532
ttttctttgt cctcgtcgtt atccaa 26

<210> 1533

<211> 23
<212> DNA
<213> Artificial Sequence

<220>
<223> Primer

<400> 1533
gttaacagta acgtgcccac tct 23

<210> 1534
<211> 19
<212> DNA
<213> Artificial Sequence

<220>
<223> Primer

<400> 1534
aaatacggct gcaccgagt 19

<210> 1535
<211> 23
<212> DNA
<213> Artificial Sequence

<220>
<223> Primer

<400> 1535
gtatcaacac acaaagccac tgt 23

<210> 1536
<211> 19
<212> DNA
<213> Artificial Sequence

<220>
<223> Primer

<400> 1536
ctgagaagcc ctgcccttc 19

<210> 1537
<211> 22
<212> DNA
<213> Artificial Sequence

<220>
<223> Primer

<400> 1537
gaagacagcg ggtatggcaa ta 22

<210> 1538
<211> 27
<212> DNA
<213> Artificial Sequence

<220>
<223> Primer

<400> 1538
gataccgagt gctcactaca attactg 27

<210> 1539
<211> 23
<212> DNA
<213> Artificial Sequence

<220>
<223> Primer

<400> 1539
gttaacagta acgtgcccac tct 23

<210> 1540
<211> 21
<212> DNA
<213> Artificial Sequence

<220>
<223> Primer

<400> 1540
catggttcca ccagcgttat t 21

<210> 1541
<211> 23
<212> DNA
<213> Artificial Sequence

<220>
<223> Primer

<400> 1541
gttaacagta acgtgcccac tct 23

<210> 1542
<211> 18
<212> DNA
<213> Artificial Sequence

<220>
<223> Primer

<400> 1542
tctttgctcg cagctcgt 18

<210> 1543
<211> 23
<212> DNA
<213> Artificial Sequence

<220>
<223> Primer

<400> 1543
gtatcaacac acaaagccac tgt 23

<210> 1544
<211> 29
<212> DNA
<213> Artificial Sequence

<220>
<223> Primer

<400> 1544
tctgtacttg tccaatgata tgttgttgt 29

<210> 1545
<211> 24
<212> DNA
<213> Artificial Sequence

<220>
<223> Primer

<400> 1545
gctacatttg cactatggcc tgta 24

<210> 1546
<211> 24
<212> DNA
<213> Artificial Sequence

<220>
<223> Primer

<400> 1546
acaacctttg aaacaggtgt tgga 24

<210> 1547
<211> 31
<212> DNA
<213> Artificial Sequence

<220>
<223> Primer

<400> 1547
gggcaacatt agaaagtata actaaaaaac a 31

<210> 1548
<211> 28
<212> DNA
<213> Artificial Sequence

<220>
<223> Primer

<400> 1548
gaaatcgtct tttatgttca cagtgcaa 28

<210> 1549
<211> 26
<212> DNA
<213> Artificial Sequence

<220>
<223> Primer

<400> 1549
ggttaggtgg tgttccttac tgttta 26

<210> 1550
<211> 22
<212> DNA
<213> Artificial Sequence

<220>
<223> Primer

<400> 1550
caaaaggcta ggcaaccgaa tt 22

<210> 1551
<211> 25
<212> DNA
<213> Artificial Sequence

<220>
<223> Primer

<400> 1551
agacatagat agcaatgcac aagca 25

<210> 1552
<211> 27
<212> DNA
<213> Artificial Sequence

<220>
<223> Primer

<400> 1552
atcacccccct tcatctactt tactaca 27

<210> 1553
<211> 22
<212> DNA
<213> Artificial Sequence

<220>
<223> Primer

<400> 1553
gtttgtctgt gtgtgtgcca tt 22

<210> 1554
<211> 27
<212> DNA

<213> Artificial Sequence

<220>
<223> Primer

<400> 1554
gcatggcaat atatacacag tgtaggt 27

<210> 1555
<211> 21
<212> DNA
<213> Artificial Sequence

<220>
<223> Primer

<400> 1555
gtaggccgag gtcaaccttt a 21

<210> 1556
<211> 25
<212> DNA
<213> Artificial Sequence

<220>
<223> Primer

<400> 1556
gtgcacatcc cacaatacat aactg 25

<210> 1557
<211> 26
<212> DNA
<213> Artificial Sequence

<220>
<223> Primer

<400> 1557
gacagggaga cagctcaaca attatt 26

<210> 1558
<211> 25
<212> DNA
<213> Artificial Sequence

<220>
<223> Primer

<400> 1558
ctctcggtac acagtttgct gatta 25

<210> 1559
<211> 23
<212> DNA
<213> Artificial Sequence

<220>

<223> Primer

<400> 1559
gtgggtggtg taaagtgtca tca 23

<210> 1560
<211> 26
<212> DNA
<213> Artificial Sequence

<220>
<223> Primer

<400> 1560
ggacagtaaa tactctcggt ttccat 26

<210> 1561
<211> 25
<212> DNA
<213> Artificial Sequence

<220>
<223> Primer

<400> 1561
gacattggac actacattgc atgac 25

<210> 1562
<211> 19
<212> DNA
<213> Artificial Sequence

<220>
<223> Primer

<400> 1562
tcgcggtggt gttctgtag 19

<210> 1563
<211> 25
<212> DNA
<213> Artificial Sequence

<220>
<223> Primer

<400> 1563
gacattggac actacattgc atgac 25

<210> 1564
<211> 28
<212> DNA
<213> Artificial Sequence

<220>
<223> Primer

<400> 1564

ctgttttggt caaatggaaa tgcattag 28

<210> 1565
<211> 28
<212> DNA
<213> Artificial Sequence

<220>
<223> Primer

<400> 1565
acaggacagt aaatgtatac aggaccat 28

<210> 1566
<211> 19
<212> DNA
<213> Artificial Sequence

<220>
<223> Primer

<400> 1566
ctgagaagcc ctgcccttc 19

<210> 1567
<211> 28
<212> DNA
<213> Artificial Sequence

<220>
<223> Primer

<400> 1567
tacaaccttt gccataacta tatatggt 28

<210> 1568
<211> 21
<212> DNA
<213> Artificial Sequence

<220>
<223> Primer

<400> 1568
attgacaacc ttcgccactg a 21

<210> 1569
<211> 23
<212> DNA
<213> Artificial Sequence

<220>
<223> Primer

<400> 1569
agtagaagtg caggccaaaa caa 23

<210> 1570

<211> 21
<212> DNA
<213> Artificial Sequence

<220>
<223> Primer

<400> 1570
catggttcca ccagcgttat t 21

<210> 1571
<211> 19
<212> DNA
<213> Artificial Sequence

<220>
<223> Primer

<400> 1571
tccgtggtgt gcaactgaa 19

<210> 1572
<211> 19
<212> DNA
<213> Artificial Sequence

<220>
<223> Primer

<400> 1572
ctgagaagcc ctgcccttc 19

<210> 1573
<211> 23
<212> DNA
<213> Artificial Sequence

<220>
<223> Primer

<400> 1573
agtagaagtg caggccaaaa caa 23

<210> 1574
<211> 24
<212> DNA
<213> Artificial Sequence

<220>
<223> Primer

<400> 1574
ggtgatccag actctgacct tttg 24

<210> 1575
<211> 28
<212> DNA
<213> Artificial Sequence

<220>
<223> Primer

<400> 1575
acaggacagt aaatgtatac aggaccat 28

<210> 1576
<211> 22
<212> DNA
<213> Artificial Sequence

<220>
<223> Primer

<400> 1576
atcatgatgg ctgtatgtgc ca 22

<210> 1577
<211> 28
<212> DNA
<213> Artificial Sequence

<220>
<223> Primer

<400> 1577
acaggacagt aaatgtatac aggaccat 28

<210> 1578
<211> 19
<212> DNA
<213> Artificial Sequence

<220>
<223> Primer

<400> 1578
aaatacggct gcaccgagt 19

<210> 1579
<211> 19
<212> DNA
<213> Artificial Sequence

<220>
<223> Primer

<400> 1579
tccgtggtgt gcaactgaa 19

<210> 1580
<211> 21
<212> DNA
<213> Artificial Sequence

<220>
<223> Primer

<400> 1580
catggttcca ccagcgttat t 21

<210> 1581
<211> 21
<212> DNA
<213> Artificial Sequence

<220>
<223> Primer

<400> 1581
agacaaccgg cgtatacagt g 21

<210> 1582
<211> 24
<212> DNA
<213> Artificial Sequence

<220>
<223> Primer

<400> 1582
cacactacta cagtcctccc gtat 24

<210> 1583
<211> 23
<212> DNA
<213> Artificial Sequence

<220>
<223> Primer

<400> 1583
agtagaagtg caggccaaaa caa 23

<210> 1584
<211> 19
<212> DNA
<213> Artificial Sequence

<220>
<223> Primer

<400> 1584
ctgagaagcc ctgcccttc 19

<210> 1585
<211> 28
<212> DNA
<213> Artificial Sequence

<220>
<223> Primer

<400> 1585
acaggacagt aaatgtatac aggaccat 28

<210> 1586
<211> 21
<212> DNA
<213> Artificial Sequence

<220>
<223> Primer

<400> 1586
catggttcca ccagcgttat t 21

<210> 1587
<211> 25
<212> DNA
<213> Artificial Sequence

<220>
<223> Primer

<400> 1587
gacattggac actacattgc atgac 25

<210> 1588
<211> 22
<212> DNA
<213> Artificial Sequence

<220>
<223> Primer

<400> 1588
gattggcatg cagcaaatgg ta 22

<210> 1589
<211> 28
<212> DNA
<213> Artificial Sequence

<220>
<223> Primer

<400> 1589
acaggacagt aaatgtatac aggaccat 28

<210> 1590
<211> 24
<212> DNA
<213> Artificial Sequence

<220>
<223> Primer

<400> 1590
ggtgatccag actctgacct tttg 24

<210> 1591
<211> 21
<212> DNA

<213> Artificial Sequence

<220>
<223> Primer

<400> 1591
agacaaccgg cgtatacagt g 21

<210> 1592
<211> 28
<212> DNA
<213> Artificial Sequence

<220>
<223> Primer

<400> 1592
ctgttttggt caaatggaaa tgcattag 28

<210> 1593
<211> 25
<212> DNA
<213> Artificial Sequence

<220>
<223> Primer

<400> 1593
ccaccaacat ctactactag ccaga 25

<210> 1594
<211> 21
<212> DNA
<213> Artificial Sequence

<220>
<223> Primer

<400> 1594
ctgttgtagt gtccgcaggt t 21

<210> 1595
<211> 30
<212> DNA
<213> Artificial Sequence

<220>
<223> Primer

<400> 1595
cctaatacaa ataaagtgtc caccaatgct 30

<210> 1596
<211> 28
<212> DNA
<213> Artificial Sequence

<220>

<223> Primer

<400> 1596
ctgttttggt caaatggaaa tgcattag 28

<210> 1597
<211> 25
<212> DNA
<213> Artificial Sequence

<220>
<223> Primer

<400> 1597
gacattggac actacattgc atgac 25

<210> 1598
<211> 26
<212> DNA
<213> Artificial Sequence

<220>
<223> Primer

<400> 1598
cttcgttttg ttgttaggtg ccttag 26

<210> 1599
<211> 23
<212> DNA
<213> Artificial Sequence

<220>
<223> Primer

<400> 1599
agtagaagtg caggccaaaa caa 23

<210> 1600
<211> 22
<212> DNA
<213> Artificial Sequence

<220>
<223> Primer

<400> 1600
atcatgatgg ctgtatgtgc ca 22

<210> 1601
<211> 23
<212> DNA
<213> Artificial Sequence

<220>
<223> Primer

<400> 1601

agtagaagtg caggccaaaa caa 23

<210> 1602
<211> 18
<212> DNA
<213> Artificial Sequence

<220>
<223> Primer

<400> 1602
tctttgctcg cagctcgt 18

<210> 1603
<211> 19
<212> DNA
<213> Artificial Sequence

<220>
<223> Primer

<400> 1603
tccgtggtgt gcaactgaa 19

<210> 1604
<211> 28
<212> DNA
<213> Artificial Sequence

<220>
<223> Primer

<400> 1604
ctgttttggt caaatggaaa tgcattag 28

<210> 1605
<211> 19
<212> DNA
<213> Artificial Sequence

<220>
<223> Primer

<400> 1605
tccgtggtgt gcaactgaa 19

<210> 1606
<211> 28
<212> DNA
<213> Artificial Sequence

<220>
<223> Primer

<400> 1606
gactgtgtca cctgtttgtt tatctact 28

<210> 1607

<211> 19
<212> DNA
<213> Artificial Sequence

<220>
<223> Primer

<400> 1607
tccgtggtgt gcaactgaa 19

<210> 1608
<211> 21
<212> DNA
<213> Artificial Sequence

<220>
<223> Primer

<400> 1608
attgacaacc ttcgccactg a 21

<210> 1609
<211> 23
<212> DNA
<213> Artificial Sequence

<220>
<223> Primer

<400> 1609
agtagaagtg caggccaaaa caa 23

<210> 1610
<211> 19
<212> DNA
<213> Artificial Sequence

<220>
<223> Primer

<400> 1610
aaatacggct gcaccgagt 19

<210> 1611
<211> 19
<212> DNA
<213> Artificial Sequence

<220>
<223> Primer

<400> 1611
tccgtggtgt gcaactgaa 19

<210> 1612
<211> 22
<212> DNA
<213> Artificial Sequence

<220>
<223> Primer

<400> 1612
atcatgatgg ctgtatgtgc ca 22

<210> 1613
<211> 19
<212> DNA
<213> Artificial Sequence

<220>
<223> Primer

<400> 1613
tccgtggtgt gcaactgaa 19

<210> 1614
<211> 18
<212> DNA
<213> Artificial Sequence

<220>
<223> Primer

<400> 1614
tctttgctcg cagctcgt 18

<210> 1615
<211> 19
<212> DNA
<213> Artificial Sequence

<220>
<223> Primer

<400> 1615
tccgtggtgt gcaactgaa 19

<210> 1616
<211> 19
<212> DNA
<213> Artificial Sequence

<220>
<223> Primer

<400> 1616
tcgcggtggt gttctgtag 19

<210> 1617
<211> 23
<212> DNA
<213> Artificial Sequence

<220>
<223> Primer

<400> 1617
agtagaagtg caggccaaaa caa 23

<210> 1618
<211> 21
<212> DNA
<213> Artificial Sequence

<220>
<223> Primer

<400> 1618
attgacaacc ttcgccactg a 21

<210> 1619
<211> 19
<212> DNA
<213> Artificial Sequence

<220>
<223> Primer

<400> 1619
tccgtggtgt gcaactgaa 19

<210> 1620
<211> 19
<212> DNA
<213> Artificial Sequence

<220>
<223> Primer

<400> 1620
aaatacggct gcaccgagt 19

<210> 1621
<211> 25
<212> DNA
<213> Artificial Sequence

<220>
<223> Primer

<400> 1621
gacattggac actacattgc atgac 25

<210> 1622
<211> 21
<212> DNA
<213> Artificial Sequence

<220>
<223> Primer

<400> 1622
ctgttgtagt gtccgcaggt t 21

<210> 1623
<211> 27
<212> DNA
<213> Artificial Sequence

<220>
<223> Primer

<400> 1623
ctagtggaaa atgggacgtg cattata 27

<210> 1624
<211> 19
<212> DNA
<213> Artificial Sequence

<220>
<223> Primer

<400> 1624
tcgcggtggt gttctgtag 19

<210> 1625
<211> 23
<212> DNA
<213> Artificial Sequence

<220>
<223> Primer

<400> 1625
agtagaagtg caggccaaaa caa 23

<210> 1626
<211> 27
<212> DNA
<213> Artificial Sequence

<220>
<223> Primer

<400> 1626
aagcgttatg tttttgcaac ctatacc 27

<210> 1627
<211> 21
<212> DNA
<213> Artificial Sequence

<220>
<223> Primer

<400> 1627
agacaaccgg cgtatacagt g 21

<210> 1628
<211> 19
<212> DNA

<213> Artificial Sequence

<220>
<223> Primer

<400> 1628
tcgcggtggt gttctgtag 19

<210> 1629
<211> 19
<212> DNA
<213> Artificial Sequence

<220>
<223> Primer

<400> 1629
tccgtggtgt gcaactgaa 19

<210> 1630
<211> 24
<212> DNA
<213> Artificial Sequence

<220>
<223> Primer

<400> 1630
ggtgatccag actctgacct tttg 24

<210> 1631
<211> 28
<212> DNA
<213> Artificial Sequence

<220>
<223> Primer

<400> 1631
acaggacagt aaatgtatac aggaccat 28

<210> 1632
<211> 18
<212> DNA
<213> Artificial Sequence

<220>
<223> Primer

<400> 1632
tctttgctcg cagctcgt 18

<210> 1633
<211> 28
<212> DNA
<213> Artificial Sequence

<220>

<223> Primer

<400> 1633
ggaatcggtg tatgcaacta cattagaa 28

<210> 1634
<211> 26
<212> DNA
<213> Artificial Sequence

<220>
<223> Primer

<400> 1634
cttcgttttg ttgttaggtg ccttag 26

<210> 1635
<211> 25
<212> DNA
<213> Artificial Sequence

<220>
<223> Primer

<400> 1635
ccctgtgact aacatatgtc cttgt 25

<210> 1636
<211> 24
<212> DNA
<213> Artificial Sequence

<220>
<223> Primer

<400> 1636
ccacacggta tagtttgcaa ccat 24

<210> 1637
<211> 25
<212> DNA
<213> Artificial Sequence

<220>
<223> Primer

<400> 1637
gcctgtgttg gtgttgaaat aggta 25

<210> 1638
<211> 26
<212> DNA
<213> Artificial Sequence

<220>
<223> Primer

<400> 1638

tgcaacattg tccctactgt ctttag 26

<210> 1639
<211> 23
<212> DNA
<213> Artificial Sequence

<220>
<223> Primer

<400> 1639
ggtgtggttt tgtgtatgca tgt 23

<210> 1640
<211> 26
<212> DNA
<213> Artificial Sequence

<220>
<223> Primer

<400> 1640
ggtatacagc aaacacctca aatggt 26

<210> 1641
<211> 20
<212> DNA
<213> Artificial Sequence

<220>
<223> Primer

<400> 1641
cgacacgccg gaatggataa 20

<210> 1642
<211> 26
<212> DNA
<213> Artificial Sequence

<220>
<223> Primer

<400> 1642
cgctgcagca ttactattac aatctg 26

<210> 1643
<211> 28
<212> DNA
<213> Artificial Sequence

<220>
<223> Primer

<400> 1643
tgggtcaggt tttatattac accctagt 28

<210> 1644

<211> 25
<212> DNA
<213> Artificial Sequence

<220>
<223> Primer

<400> 1644
gcttacaacc ttagacacag acaca 25

<210> 1645
<211> 26
<212> DNA
<213> Artificial Sequence

<220>
<223> Primer

<400> 1645
gtatgaacgt gacagtgtac acctaa 26

<210> 1646
<211> 19
<212> DNA
<213> Artificial Sequence

<220>
<223> Primer

<400> 1646
ctgagaagcc ctgcccttc 19

<210> 1647
<211> 23
<212> DNA
<213> Artificial Sequence

<220>
<223> Primer

<400> 1647
aaacgaagac tgtttgagga gca 23

<210> 1648
<211> 19
<212> DNA
<213> Artificial Sequence

<220>
<223> Primer

<400> 1648
tggtgttggt ggttgtggt 19

<210> 1649
<211> 21
<212> DNA
<213> Artificial Sequence

<220>
<223> Primer

<400> 1649
acctacatcc caccacagag t 21

<210> 1650
<211> 19
<212> DNA
<213> Artificial Sequence

<220>
<223> Primer

<400> 1650
aaatacggct gcaccgagt 19

<210> 1651
<211> 27
<212> DNA
<213> Artificial Sequence

<220>
<223> Primer

<400> 1651
tgcttatggg tacactaggt attgtgt 27

<210> 1652
<211> 24
<212> DNA
<213> Artificial Sequence

<220>
<223> Primer

<400> 1652
gggttcccat tactgtcaaa tgga 24

<210> 1653
<211> 27
<212> DNA
<213> Artificial Sequence

<220>
<223> Primer

<400> 1653
tgcttatggg tacactaggt attgtgt 27

<210> 1654
<211> 24
<212> DNA
<213> Artificial Sequence

<220>
<223> Primer

<400> 1654
ggtgatccag actctgacct tttg 24

<210> 1655
<211> 28
<212> DNA
<213> Artificial Sequence

<220>
<223> Primer

<400> 1655
agcgttatgt gacgaagtga atatttct 28

<210> 1656
<211> 28
<212> DNA
<213> Artificial Sequence

<220>
<223> Primer

<400> 1656
ctgttctgct atttgatgaa accgtttt 28

<210> 1657
<211> 28
<212> DNA
<213> Artificial Sequence

<220>
<223> Primer

<400> 1657
agcgttatgt gacgaagtga atatttct 28

<210> 1658
<211> 24
<212> DNA
<213> Artificial Sequence

<220>
<223> Primer

<400> 1658
ttcggttgtt ggtttcaggt ctaa 24

<210> 1659
<211> 27
<212> DNA
<213> Artificial Sequence

<220>
<223> Primer

<400> 1659
tgcttatggg tacactaggt attgtgt 27

<210> 1660
<211> 19
<212> DNA
<213> Artificial Sequence

<220>
<223> Primer

<400> 1660
tggtgttggt ggttgtggt 19

<210> 1661
<211> 26
<212> DNA
<213> Artificial Sequence

<220>
<223> Primer

<400> 1661
gggtaaaagg catatgggaa gtacat 26

<210> 1662
<211> 19
<212> DNA
<213> Artificial Sequence

<220>
<223> Primer

<400> 1662
tggtgttggt ggttgtggt 19

<210> 1663
<211> 29
<212> DNA
<213> Artificial Sequence

<220>
<223> Primer

<400> 1663
caagttaaat gccctccatt actgataac 29

<210> 1664
<211> 24
<212> DNA
<213> Artificial Sequence

<220>
<223> Primer

<400> 1664
gggttcccat tactgtcaaa tgga 24

<210> 1665
<211> 27
<212> DNA

<213> Artificial Sequence

<220>
<223> Primer

<400> 1665
tgcttatggg tacactaggt attgtgt 27

<210> 1666
<211> 21
<212> DNA
<213> Artificial Sequence

<220>
<223> Primer

<400> 1666
catggttcca ccagcgttat t 21

<210> 1667
<211> 21
<212> DNA
<213> Artificial Sequence

<220>
<223> Primer

<400> 1667
acctacatcc caccacagag t 21

<210> 1668
<211> 24
<212> DNA
<213> Artificial Sequence

<220>
<223> Primer

<400> 1668
gtccaatgcc atgttgttgt taca 24

<210> 1669
<211> 21
<212> DNA
<213> Artificial Sequence

<220>
<223> Primer

<400> 1669
acctacatcc caccacagag t 21

<210> 1670
<211> 22
<212> DNA
<213> Artificial Sequence

<220>

<223> Primer

<400> 1670
atcatgatgg ctgtatgtgc ca 22

<210> 1671
<211> 27
<212> DNA
<213> Artificial Sequence

<220>
<223> Primer

<400> 1671
tgcttatggg tacactaggt attgtgt 27

<210> 1672
<211> 24
<212> DNA
<213> Artificial Sequence

<220>
<223> Primer

<400> 1672
acgaagccta aacaccctgt attg 24

<210> 1673
<211> 26
<212> DNA
<213> Artificial Sequence

<220>
<223> Primer

<400> 1673
gtatgaacgt gacagtgtac acctaa 26

<210> 1674
<211> 22
<212> DNA
<213> Artificial Sequence

<220>
<223> Primer

<400> 1674
atcatgatgg ctgtatgtgc ca 22

<210> 1675
<211> 25
<212> DNA
<213> Artificial Sequence

<220>
<223> Primer

<400> 1675

actcagagga tgaggatgaa acaga 25

<210> 1676
<211> 28
<212> DNA
<213> Artificial Sequence

<220>
<223> Primer

<400> 1676
cctagtgtac ccataagcaa ctcttcta 28

<210> 1677
<211> 21
<212> DNA
<213> Artificial Sequence

<220>
<223> Primer

<400> 1677
acctacatcc caccacagag t 21

<210> 1678
<211> 25
<212> DNA
<213> Artificial Sequence

<220>
<223> Primer

<400> 1678
gcttacaacc ttagacacag acaca 25

<210> 1679
<211> 23
<212> DNA
<213> Artificial Sequence

<220>
<223> Primer

<400> 1679
aaacgaagac tgtttgagga gca 23

<210> 1680
<211> 28
<212> DNA
<213> Artificial Sequence

<220>
<223> Primer

<400> 1680
gacacaattt ggttgccttc ttcattaa 28

<210> 1681

<211> 27
<212> DNA
<213> Artificial Sequence

<220>
<223> Primer

<400> 1681
tgcttatggg tacactaggt attgtgt 27

<210> 1682
<211> 19
<212> DNA
<213> Artificial Sequence

<220>
<223> Primer

<400> 1682
aaatacggct gcaccgagt 19

<210> 1683
<211> 20
<212> DNA
<213> Artificial Sequence

<220>
<223> Primer

<400> 1683
tgcaggtagc acacgtttgt 20

<210> 1684
<211> 24
<212> DNA
<213> Artificial Sequence

<220>
<223> Primer

<400> 1684
acgaagccta aacaccctgt attg 24

<210> 1685
<211> 21
<212> DNA
<213> Artificial Sequence

<220>
<223> Primer

<400> 1685
acctacatcc caccacagag t 21

<210> 1686
<211> 18
<212> DNA
<213> Artificial Sequence

<220>
<223> Primer

<400> 1686
tctttgctcg cagctcgt 18

<210> 1687
<211> 27
<212> DNA
<213> Artificial Sequence

<220>
<223> Primer

<400> 1687
tgcttatggg tacactaggt attgtgt 27

<210> 1688
<211> 26
<212> DNA
<213> Artificial Sequence

<220>
<223> Primer

<400> 1688
tggaattgga tcccctgttt ttcttt 26

<210> 1689
<211> 27
<212> DNA
<213> Artificial Sequence

<220>
<223> Primer

<400> 1689
tgcttatggg tacactaggt attgtgt 27

<210> 1690
<211> 18
<212> DNA
<213> Artificial Sequence

<220>
<223> Primer

<400> 1690
tctttgctcg cagctcgt 18

<210> 1691
<211> 27
<212> DNA
<213> Artificial Sequence

<220>
<223> Primer

<400> 1691
tgcttatggg tacactaggt attgtgt 27

<210> 1692
<211> 19
<212> DNA
<213> Artificial Sequence

<220>
<223> Primer

<400> 1692
ctgagaagcc ctgcccttc 19

<210> 1693
<211> 27
<212> DNA
<213> Artificial Sequence

<220>
<223> Primer

<400> 1693
tgcttatggg tacactaggt attgtgt 27

<210> 1694
<211> 25
<212> DNA
<213> Artificial Sequence

<220>
<223> Primer

<400> 1694
gcttacaacc ttagacacag acaca 25

<210> 1695
<211> 26
<212> DNA
<213> Artificial Sequence

<220>
<223> Primer

<400> 1695
gtatgaacgt gacagtgtac acctaa 26

<210> 1696
<211> 21
<212> DNA
<213> Artificial Sequence

<220>
<223> Primer

<400> 1696
catggttcca ccagcgttat t 21

<210> 1697
<211> 28
<212> DNA
<213> Artificial Sequence

<220>
<223> Primer

<400> 1697
agcgttatgt gacgaagtga atatttct 28

<210> 1698
<211> 27
<212> DNA
<213> Artificial Sequence

<220>
<223> Primer

<400> 1698
aaaattttaa acacggttga catacac 27

<210> 1699
<211> 26
<212> DNA
<213> Artificial Sequence

<220>
<223> Primer

<400> 1699
gtatgaacgt gacagtgtac acctaa 26

<210> 1700
<211> 19
<212> DNA
<213> Artificial Sequence

<220>
<223> Primer

<400> 1700
aaatacggct gcaccgagt 19

<210> 1701
<211> 26
<212> DNA
<213> Artificial Sequence

<220>
<223> Primer

<400> 1701
gtatgaacgt gacagtgtac acctaa 26

<210> 1702
<211> 18
<212> DNA

<213> Artificial Sequence

<220>
<223> Primer

<400> 1702
tctttgctcg cagctcgt 18

<210> 1703
<211> 27
<212> DNA
<213> Artificial Sequence

<220>
<223> Primer

<400> 1703
tgcttatggg tacactaggt attgtgt 27

<210> 1704
<211> 22
<212> DNA
<213> Artificial Sequence

<220>
<223> Primer

<400> 1704
atcatgatgg ctgtatgtgc ca 22

<210> 1705
<211> 29
<212> DNA
<213> Artificial Sequence

<220>
<223> Primer

<400> 1705
agacaatcag tatatggcac tacgttaga 29

<210> 1706
<211> 28
<212> DNA
<213> Artificial Sequence

<220>
<223> Primer

<400> 1706
ctgttctgct atttgatgaa accgtttt 28

<210> 1707
<211> 21
<212> DNA
<213> Artificial Sequence

<220>

<223> Primer

<400> 1707
acctacatcc caccacagag t 21

<210> 1708
<211> 21
<212> DNA
<213> Artificial Sequence

<220>
<223> Primer

<400> 1708
catggttcca ccagcgttat t 21

<210> 1709
<211> 28
<212> DNA
<213> Artificial Sequence

<220>
<223> Primer

<400> 1709
agcgttatgt gacgaagtga atatttctv 28

<210> 1710
<211> 24
<212> DNA
<213> Artificial Sequence

<220>
<223> Primer

<400> 1710
gggttcccat tactgtcaaa tgga 24

<210> 1711
<211> 21
<212> DNA
<213> Artificial Sequence

<220>
<223> Primer

<400> 1711
acctacatcc caccacagag t 21

<210> 1712
<211> 19
<212> DNA
<213> Artificial Sequence

<220>
<223> Primer

<400> 1712

ctgagaagcc ctgcccttc 19

<210> 1713
<211> 21
<212> DNA
<213> Artificial Sequence

<220>
<223> Primer

<400> 1713
acctacatcc caccacagag t 21

<210> 1714
<211> 24
<212> DNA
<213> Artificial Sequence

<220>
<223> Primer

<400> 1714
ggtgatccag actctgacct tttg 24

<210> 1715
<211> 23
<212> DNA
<213> Artificial Sequence

<220>
<223> Primer

<400> 1715
aaacgaagac tgtttgagga gca 23

<210> 1716
<211> 24
<212> DNA
<213> Artificial Sequence

<220>
<223> Primer

<400> 1716
gggttcccat tactgtcaaa tgga 24

<210> 1717
<211> 28
<212> DNA
<213> Artificial Sequence

<220>
<223> Primer

<400> 1717
agcgttatgt gacgaagtga atatttct 28

<210> 1718

<211> 28
<212> DNA
<213> Artificial Sequence

<220>
<223> Primer

<400> 1718
cctagtgtac ccataagcaa ctcttcta 28

<210> 1719
<211> 28
<212> DNA
<213> Artificial Sequence

<220>
<223> Primer

<400> 1719
agcgttatgt gacgaagtga atatttct 28

<210> 1720
<211> 19
<212> DNA
<213> Artificial Sequence

<220>
<223> Primer

<400> 1720
tggtgttggt ggttgtggt 19

<210> 1721
<211> 26
<212> DNA
<213> Artificial Sequence

<220>
<223> Primer

<400> 1721
gtatgaacgt gacagtgtac acctaa 26

<210> 1722
<211> 24
<212> DNA
<213> Artificial Sequence

<220>
<223> Primer

<400> 1722
ggtgatccag actctgacct tttg 24

<210> 1723
<211> 21
<212> DNA
<213> Artificial Sequence

<220>
<223> Primer

<400> 1723
acctacatcc caccacagag t 21

<210> 1724
<211> 24
<212> DNA
<213> Artificial Sequence

<220>
<223> Primer

<400> 1724
acgaagccta aacaccctgt attg 24

<210> 1725
<211> 25
<212> DNA
<213> Artificial Sequence

<220>
<223> Primer

<400> 1725
tcgcttgcag tgtctgtgta tattt 25

<210> 1726
<211> 26
<212> DNA
<213> Artificial Sequence

<220>
<223> Primer

<400> 1726
catggtaatg tacaagtgcc atagga 26

<210> 1727
<211> 25
<212> DNA
<213> Artificial Sequence

<220>
<223> Primer

<400> 1727
gaacgcatgt taattgaacc tccaa 25

<210> 1728
<211> 27
<212> DNA
<213> Artificial Sequence

<220>
<223> Primer

<400> 1728
gctgcactaa cgtttgtctt ttaatcc 27

<210> 1729
<211> 27
<212> DNA
<213> Artificial Sequence

<220>
<223> Primer

<400> 1729
tgtattttag gttgtaggcc tccctta 27

<210> 1730
<211> 27
<212> DNA
<213> Artificial Sequence

<220>
<223> Primer

<400> 1730
ctccaaagcc aacatctatc atatcac 27

<210> 1731
<211> 25
<212> DNA
<213> Artificial Sequence

<220>
<223> Primer

<400> 1731
gtcgccattt tacatgcatt aaggt 25

<210> 1732
<211> 22
<212> DNA
<213> Artificial Sequence

<220>
<223> Primer

<400> 1732
aggaaacaaa ccctgccaag tt 22

<210> 1733
<211> 21
<212> DNA
<213> Artificial Sequence

<220>
<223> Primer

<400> 1733
tgcgaccacc aaatacactg t 21

<210> 1734
<211> 21
<212> DNA
<213> Artificial Sequence

<220>
<223> Primer

<400> 1734
gtgttgacaa tgcgtgacac t 21

<210> 1735
<211> 21
<212> DNA
<213> Artificial Sequence

<220>
<223> Primer

<400> 1735
tgcgaccacc aaatacactg t 21

<210> 1736
<211> 19
<212> DNA
<213> Artificial Sequence

<220>
<223> Primer

<400> 1736
ctgagaagcc ctgcccttc 19

<210> 1737
<211> 28
<212> DNA
<213> Artificial Sequence

<220>
<223> Primer

<400> 1737
gtgccaggag aaaatactag actgttat 28

<210> 1738
<211> 24
<212> DNA
<213> Artificial Sequence

<220>
<223> Primer

<400> 1738
ggtgatccag actctgacct tttg 24

<210> 1739
<211> 28
<212> DNA

<213> Artificial Sequence

<220>
<223> Primer

<400> 1739
gtgccaggag aaaatactag actgttat 28

<210> 1740
<211> 18
<212> DNA
<213> Artificial Sequence

<220>
<223> Primer

<400> 1740
tctttgctcg cagctcgt 18

<210> 1741
<211> 28
<212> DNA
<213> Artificial Sequence

<220>
<223> Primer

<400> 1741
gtgccaggag aaaatactag actgttat 28

<210> 1742
<211> 19
<212> DNA
<213> Artificial Sequence

<220>
<223> Primer

<400> 1742
ctgagaagcc ctgcccttc 19

<210> 1743
<211> 22
<212> DNA
<213> Artificial Sequence

<220>
<223> Primer

<400> 1743
ccggacagtg gatatggcaa ta 22

<210> 1744
<211> 25
<212> DNA
<213> Artificial Sequence

<220>

<223> Primer

<400> 1744
ggtctatctc tgtacttctg tcgct 25

<210> 1745
<211> 23
<212> DNA
<213> Artificial Sequence

<220>
<223> Primer

<400> 1745
cctgcaatac gtctatgcac aat 23

<210> 1746
<211> 27
<212> DNA
<213> Artificial Sequence

<220>
<223> Primer

<400> 1746
ccagtaacat ttgctgaaat atgcgaa 27

<210> 1747
<211> 28
<212> DNA
<213> Artificial Sequence

<220>
<223> Primer

<400> 1747
ggaggatgaa gtagataata tgcgtgac 28

<210> 1748
<211> 27
<212> DNA
<213> Artificial Sequence

<220>
<223> Primer

<400> 1748
ccagtaacat ttgctgaaat atgcgaa 27

<210> 1749
<211> 20
<212> DNA
<213> Artificial Sequence

<220>
<223> Primer

<400> 1749

gggcacaaca atgggaggta 20

<210> 1750
<211> 21
<212> DNA
<213> Artificial Sequence

<220>
<223> Primer

<400> 1750
gggtgttcga tagctgttca a 21

<210> 1751
<211> 21
<212> DNA
<213> Artificial Sequence

<220>
<223> Primer

<400> 1751
tgcgaccacc aaatacactg t 21

<210> 1752
<211> 28
<212> DNA
<213> Artificial Sequence

<220>
<223> Primer

<400> 1752
caatgccagg tagatgacac ttctttaa 28

<210> 1753
<211> 25
<212> DNA
<213> Artificial Sequence

<220>
<223> Primer

<400> 1753
gggattacta ctttgtggcc gtata 25

<210> 1754
<211> 21
<212> DNA
<213> Artificial Sequence

<220>
<223> Primer

<400> 1754
gtgttgacaa tgcgtgacac t 21

<210> 1755

<211> 23
<212> DNA
<213> Artificial Sequence

<220>
<223> Primer

<400> 1755
cctgcaatac gtctatgcac aat 23

<210> 1756
<211> 24
<212> DNA
<213> Artificial Sequence

<220>
<223> Primer

<400> 1756
tttttgtcg tccaccacct tttg 24

<210> 1757
<211> 21
<212> DNA
<213> Artificial Sequence

<220>
<223> Primer

<400> 1757
tgcgaccacc aaatacactg t 21

<210> 1758
<211> 24
<212> DNA
<213> Artificial Sequence

<220>
<223> Primer

<400> 1758
ggtgatccag actctgacct tttg 24

<210> 1759
<211> 20
<212> DNA
<213> Artificial Sequence

<220>
<223> Primer

<400> 1759
cgtggtgtgc gaccaactaa 20

<210> 1760
<211> 19
<212> DNA
<213> Artificial Sequence

<220>
<223> Primer

<400> 1760
aaatacggct gcaccgagt 19

<210> 1761
<211> 20
<212> DNA
<213> Artificial Sequence

<220>
<223> Primer

<400> 1761
cgtggtgtgc gaccaactaa 20

<210> 1762
<211> 19
<212> DNA
<213> Artificial Sequence

<220>
<223> Primer

<400> 1762
ctgagaagcc ctgcccttc 19

<210> 1763
<211> 23
<212> DNA
<213> Artificial Sequence

<220>
<223> Primer

<400> 1763
cctgcaatac gtctatgcac aat 23

<210> 1764
<211> 21
<212> DNA
<213> Artificial Sequence

<220>
<223> Primer

<400> 1764
gggtgttcga tagctgttca a 21

<210> 1765
<211> 23
<212> DNA
<213> Artificial Sequence

<220>
<223> Primer

<400> 1765
cctgcaatac gtctatgcac aat 23

<210> 1766
<211> 23
<212> DNA
<213> Artificial Sequence

<220>
<223> Primer

<400> 1766
catgctgcat atggcgtatt gtc 23

<210> 1767
<211> 25
<212> DNA
<213> Artificial Sequence

<220>
<223> Primer

<400> 1767
gtaggtctgt gtatggtgct acatt 25

<210> 1768
<211> 24
<212> DNA
<213> Artificial Sequence

<220>
<223> Primer

<400> 1768
tttttgtcg tccaccacct tttg 24

<210> 1769
<211> 21
<212> DNA
<213> Artificial Sequence

<220>
<223> Primer

<400> 1769
tgcgaccacc aaatacactg t 21

<210> 1770
<211> 22
<212> DNA
<213> Artificial Sequence

<220>
<223> Primer

<400> 1770
atcatgatgg ctgtatgtgc ca 22

<210> 1771
<211> 21
<212> DNA
<213> Artificial Sequence

<220>
<223> Primer

<400> 1771
tgcgaccacc aaatacactg t 21

<210> 1772
<211> 18
<212> DNA
<213> Artificial Sequence

<220>
<223> Primer

<400> 1772
tctttgctcg cagctcgt 18

<210> 1773
<211> 22
<212> DNA
<213> Artificial Sequence

<220>
<223> Primer

<400> 1773
ccggacagtg gatatggcaa ta 22

<210> 1774
<211> 21
<212> DNA
<213> Artificial Sequence

<220>
<223> Primer

<400> 1774
gggtgttcga tagctgttca a 21

<210> 1775
<211> 20
<212> DNA
<213> Artificial Sequence

<220>
<223> Primer

<400> 1775
cgtggtgtgc gaccaactaa 20

<210> 1776
<211> 21
<212> DNA

<213> Artificial Sequence

<220>
<223> Primer

<400> 1776
gtgttgacaa tgcgtgacac t 21

<210> 1777
<211> 20
<212> DNA
<213> Artificial Sequence

<220>
<223> Primer

<400> 1777
cgtggtgtgc gaccaactaa 20

<210> 1778
<211> 21
<212> DNA
<213> Artificial Sequence

<220>
<223> Primer

<400> 1778
catggttcca ccagcgttat t 21

<210> 1779
<211> 20
<212> DNA
<213> Artificial Sequence

<220>
<223> Primer

<400> 1779
cgtggtgtgc gaccaactaa 20

<210> 1780
<211> 28
<212> DNA
<213> Artificial Sequence

<220>
<223> Primer

<400> 1780
catcatttag tgcatataca ggattccc 28

<210> 1781
<211> 20
<212> DNA
<213> Artificial Sequence

<220>

<223> Primer

<400> 1781
cgtggtgtgc gaccaactaa 20

<210> 1782
<211> 21
<212> DNA
<213> Artificial Sequence

<220>
<223> Primer

<400> 1782
gggtgttcga tagctgttca a 21

<210> 1783
<211> 28
<212> DNA
<213> Artificial Sequence

<220>
<223> Primer

<400> 1783
gtgccaggag aaaatactag actgttat 28

<210> 1784
<211> 21
<212> DNA
<213> Artificial Sequence

<220>
<223> Primer

<400> 1784
catggttcca ccagcgttat t 21

<210> 1785
<211> 22
<212> DNA
<213> Artificial Sequence

<220>
<223> Primer

<400> 1785
ccggacagtg gatatggcaa ta 22

<210> 1786
<211> 28
<212> DNA
<213> Artificial Sequence

<220>
<223> Primer

<400> 1786

catcatttag tgcatataca ggattccc 28

<210> 1787
<211> 21
<212> DNA
<213> Artificial Sequence

<220>
<223> Primer

<400> 1787
tgcgaccacc aaatacactg t 21

<210> 1788
<211> 21
<212> DNA
<213> Artificial Sequence

<220>
<223> Primer

<400> 1788
catggttcca ccagcgttat t 21

<210> 1789
<211> 20
<212> DNA
<213> Artificial Sequence

<220>
<223> Primer

<400> 1789
cgtggtgtgc gaccaactaa 20

<210> 1790
<211> 18
<212> DNA
<213> Artificial Sequence

<220>
<223> Primer

<400> 1790
tctttgctcg cagctcgt 18

<210> 1791
<211> 23
<212> DNA
<213> Artificial Sequence

<220>
<223> Primer

<400> 1791
cctgcaatac gtctatgcac aat 23

<210> 1792

<211> 28
<212> DNA
<213> Artificial Sequence

<220>
<223> Primer

<400> 1792
catcatttag tgcatataca ggattccc 28

<210> 1793
<211> 20
<212> DNA
<213> Artificial Sequence

<220>
<223> Primer

<400> 1793
cgtggtgtgc gaccaactaa 20

<210> 1794
<211> 24
<212> DNA
<213> Artificial Sequence

<220>
<223> Primer

<400> 1794
ggtgatccag actctgacct tttg 24

<210> 1795
<211> 20
<212> DNA
<213> Artificial Sequence

<220>
<223> Primer

<400> 1795
cgtggtgtgc gaccaactaa 20

<210> 1796
<211> 26
<212> DNA
<213> Artificial Sequence

<220>
<223> Primer

<400> 1796
ttgtcaacta ctgcctccac ataaaa 26

<210> 1797
<211> 23
<212> DNA
<213> Artificial Sequence

<220>
<223> Primer

<400> 1797
cctgcaatac gtctatgcac aat 23

<210> 1798
<211> 27
<212> DNA
<213> Artificial Sequence

<220>
<223> Primer

<400> 1798
tccaacacta tgtcctttaa ttgtggt 27

<210> 1799
<211> 21
<212> DNA
<213> Artificial Sequence

<220>
<223> Primer

<400> 1799
tgcgaccacc aaatacactg t 21

<210> 1800
<211> 19
<212> DNA
<213> Artificial Sequence

<220>
<223> Primer

<400> 1800
aaatacggct gcaccgagt 19

<210> 1801
<211> 28
<212> DNA
<213> Artificial Sequence

<220>
<223> Primer

<400> 1801
gtgccaggag aaaatactag actgttat 28

<210> 1802
<211> 19
<212> DNA
<213> Artificial Sequence

<220>
<223> Primer

<400> 1802
aaatacggct gcaccgagt 19

<210> 1803
<211> 28
<212> DNA
<213> Artificial Sequence

<220>
<223> Primer

<400> 1803
gtgccaggag aaaatactag actgttat 28

<210> 1804
<211> 22
<212> DNA
<213> Artificial Sequence

<220>
<223> Primer

<400> 1804
atcatgatgg ctgtatgtgc ca 22

<210> 1805
<211> 20
<212> DNA
<213> Artificial Sequence

<220>
<223> Primer

<400> 1805
cgtggtgtgc gaccaactaa 20

<210> 1806
<211> 22
<212> DNA
<213> Artificial Sequence

<220>
<223> Primer

<400> 1806
atcatgatgg ctgtatgtgc ca 22

<210> 1807
<211> 20
<212> DNA
<213> Artificial Sequence

<220>
<223> Primer

<400> 1807
acacagaagc ctgctgcaaa 20

<210> 1808
<211> 28
<212> DNA
<213> Artificial Sequence

<220>
<223> Primer

<400> 1808
catcatttag tgcatataca ggattccc 28

<210> 1809
<211> 22
<212> DNA
<213> Artificial Sequence

<220>
<223> Primer

<400> 1809
cctgtaggtt aagggtggtg tt 22

<210> 1810
<211> 21
<212> DNA
<213> Artificial Sequence

<220>
<223> Primer

<400> 1810
aaatcggtcg ccacaaaatg g 21

<210> 1811
<211> 21
<212> DNA
<213> Artificial Sequence

<220>
<223> Primer

<400> 1811
cgtagtacag ccgttgcatt g 21

<210> 1812
<211> 24
<212> DNA
<213> Artificial Sequence

<220>
<223> Primer

<400> 1812
cccattgtac catttgcgat agtt 24

<210> 1813
<211> 25
<212> DNA

<213> Artificial Sequence

<220>
<223> Primer

<400> 1813
ggatgtgttg gtgttgaagt aggta 25

<210> 1814
<211> 20
<212> DNA
<213> Artificial Sequence

<220>
<223> Primer

<400> 1814
tcctgttggt cgttgccatt 20

<210> 1815
<211> 27
<212> DNA
<213> Artificial Sequence

<220>
<223> Primer

<400> 1815
gctgctaagt gtatatagtt actcgca 27

<210> 1816
<211> 24
<212> DNA
<213> Artificial Sequence

<220>
<223> Primer

<400> 1816
ctgctgcaaa cacatattgg gatt 24

## Description

### Background of the invention

HPV infections are associated with the development of cervix carcinoma and possibly other cancers like head and neck cancers. For example, cancer of the cervix is one of the most common cancers among women in all countries. Human papillomaviruses (HPV) are the etiologic agents responsible for over 99% of all cancers of the cervix. HPVs are DNA viruses commonly transmitted through sexual contact, which include more than 100 genotypes. Human Papillomaviruses are small, non-enveloped DNA viruses, approximately 55 nm in diameter, that infect basal cells and replicate in the nucleus of squamous epithelial cells. The genomic organization of each of the papillomaviruses is similar and can be divided into three functional regions. Following infection, the early HPV genes (E6, E7, E1, E2, E4 and E5) are expressed and the viral DNA replicates from the episomal form of the virus. In the upper layer of the epithelium the viral genome is replicated further, and the late genes (L1 and L2) and E4 are expressed. The shed virus can then initiate new infections.

Human papillomaviruses (HPV) are viruses displaying a high genetic diversity. About one hundred HPV types which are classified in different genus, mainly the *alpha, beta* and *gamma* genus. Within these genus, many species have been identified. HPV classification is based on the genomic sequence of the L1 gene which encodes the major capsid protein. The different HPV types are characterized by their tissue tropism, and HPV types with either cutaneous or mucosal tropism can be distinguished. They are also characterized by their oncogenic potential and one can distinguish between highly oncogenic HPV types (high-risk HPV) and weakly oncogenic HPV types (low-risk HPV).

HPV infections are very common and depending on the HPV types and host immune defense, the infection disappears in 6-12 months in 90% of women. According to a recent CDC report, there are 14 million new HPV infections each year in the USA alone, which account for 50% of sexually transmitted infections (STIs). This means that 1.4 million individuals are each year at risk of developing HPV induced cancer. Two HPV vaccines have been approved, but they are not broadly used in the total population. In addition, these vaccines only cover several types such as HPV6, 11, 16 and 18 and leave unprotected a significant part of the population.

Since HPV are common viruses that can cause usually warts and because there are more than 100 types of HPV, diagnosis and disease management are complex. It is even further complicated taken that most HPVs are deemed harmless, and so far only about 14 types have been shown to be associated with increased risk of cancer. These HPV types affect the genital tract and are transmitted through sexual contact with an infected partner. As of today, HPV types have been classified as low-risk or high-risk HPVs according to observations in clinical cohorts. Low-risk HPVs have been classified according to their association with genital warts ; whereas High-risk HPVs (HR HPVs) are identified as a limited number of types which are shown to induce cancers of the cervix, vulva, vagina, and anus in women. In men, these High-risk HPVs can lead to cancers of the anus and penis.

Cancer biomarkers in HPV-related cancers are greatly needed for a better diagnostic of pre-cancer and cancer stages of the disease, prognosis and therapeutic management.

Despite the responsibility of HR HPVs in most cervix cancers, screening tests of cancer remain mainly based on the Pap cytology test and not on HPV tests. This is largely due to the limitations of current molecular tests. HPV DNA identification of HR HPVs is not fully predictive of cancer: only high loads of HPV16 and possibility persistence for months of HR-HPVs are associated with an increased risk of cancer development. Thus, the usage of DNA HPV tests, as a screening assay, shows low positive predictive value for CIN2/3 lesions. Expression of E6 and E7 mRNAs of HR HPVs has been proposed as a better marker of cancer development, but E6 and E7 are expressed during HPV acute infection, so it remains difficult to define a threshold of expression associated to persistence and cancer development.

Low-grade intraepithelial lesions are a site of productive viral replication. Progression to high-grade intraepithelial lesions and invasive carcinomas is associated with a persistent high-risk HPV infection and often integration of the HPV genome into the host chromosomes, loss or disruption of E2 and subsequent upregulation of E6 and E7 expression. E6 and E7 are the oncogenes of the virus and expression of these genes is required for malignant transformation. Among others, E6 and E7 mediate degradation of the tumor suppressors p53 and RB, respectively, and interfere with cell-cycle regulation. E6 and E7 proteins from low-risk types are less competent in interfering with p53 and pRb functions than E6/E7 proteins from high-risk types. Therefore, low-risk HPV infections are associated with benign proliferations, such as genital warts and low-grade intraepithelial lesions prone to regress.

Different techniques are available today for detecting HPV based on DNA typing. For example, the COBAS (Roche) and APTIMA (GEN-PROBE) kits are PCR tests of specific targets intended for the qualitative *in vitro* detection of mRNA of the L1 gene from 14 types of human papillomavirus (HPV) virus considered High risk (HPV 16, 18, 31, 33, 35, 39, 45, 51, 52, 56, 58, 59, 66 and 68). However, both of these HPV Assays do not distinguish the differences between the 14 High-risk types. In fact, only 6 different results are obtainable: HPV16 positive or negative, HPV18 positive or negative, others 12 HPVs positive or negative. LINEAR ARRAY HPV Genotyping Test (Roche) is a qualitative test that detects 37 high- and low-risk human papillomavirus genotypes, including those considered a significant risk factor for High-grade Squamous Intraepithelial (HSIL) progression to cervical cancer. This test is a qualitative *in vitro* test for the detection of Human Papilloma Virus in clinical specimens. The test utilizes amplification of target DNAs by PCR of the late gene L1 of HPV DNA genotypes 6, 11, 16, 18, 26, 31, 33, 35, 39, 40, 42, 45, 51, 52, 53, 54, 55, 56, 58, 59, 61, 62, 64, 66, 67, 68, 69, 70, 71, 72, 73 (MM9) (novel type related to *HPV73),* 81, 82 (MM4) (novel type related to *HPV82),* 83 (MM7) (novel type related to *HPV83),* 84 (MM8) (novel type related to *HPV84),* IS39 and CP6108. The *digene* HC2 HPV DNA Test, developed by Qiagen, is based on Capture Hybridization of HPV DNAs (L1 gene) for the qualitative detection of 18 types (HPV 16, 18, 26, 31, 33, 35, 39, 45, 51, 52, 53, 56, 58, 59, 66, 68 [68a], 73, 82MM4 [82IS39]) in cervical specimens.

More recently, NucliSENS EasyQ HPV was made available to qualitative detection of oncogenes E6 / E7 mRNAs of 5 specific High risk HPVs 16, 18, 31, 33 and 45. Detection of HPV E6 and E7 has been proposed as a better correlate of cancer development than HPV DNA.

In addition, WO2011/088573 (Her Majesty The Queen In Right of Canada as represented by The Minister of Health), describes a set of probes to detect and Identify 46 specifically targeted species of mucosal human papillomaviruses (HPV). These probes are used as a multiplex assay based on nested PCR amplification and the Luminex xMAP technology for genotyping DNA of L1 genes of HPV types 6, 11, 13, 16, 18, 26, 30, 31, 32, 33, 35, 39, 40, 42 , 43, 44, 45, 51, 52, 53, 54, 56, 58, 59, 61, 62, 66, 67, 68, 69, 70, 71, 72, 73, 74, 81, 82, 83, 84, 85, 86, 87, 89, 90, 91 and 97.

US2006222656 discloses methods of treating squamous cell carcinoma of the head and neck (SCCHN) using Trojan antigens, and immunogenic compositions comprising the Trojan antigens. WO2010129941 discloses a method for determining an increased likelihood of developing cervical cancer by analysing the expression levels of a human papillomavirus (HPV) E6 mRNA and/or E7 mRNA.

All the molecular tests currently described for HPV identification use molecular techniques based on species/genus genotype specific oligonucleotides binding to specifically amplify and/or probe papillomavirus nucleic acids. In addition, these tests all share a specific qualitative detection restricted to some specific HPVs, but not a general and broad range of HPVs. Finally, the presence of HPV nucleic acid, especially DNA, does not mean the presence of cervical dysplasia or cervical cancer.

The inventors analyzed clinical data of cohorts of HPV infected individuals and considered that about 15% of patients are not positive for one of the classified High-risk HPVs but yet, they are at risk of developing HPV induced cancers. Therefore, there is a need to design a new HPV assay with a transversal and broad approach not limited to few specific HPV types. Moreover, the profile expression of the viral genes must be characterized. In fact, HPV infection during cancer development is associated with a shift from productive infection towards non-productive infection characterized by a high level of E6 and E7 and low levels of expression E2 and of late genes, often associated with an integration of HPV DNA in the host chromosomes, at least for HPV16. In connection with the present invention, the inventors investigated shared homology of sequences of all HPV types and no clear global homologies are found within features between polypeptide or nucleic acid sequences.

The inventors designed consensus primers within all intra subgroup alpha, and HPV-species specific composition of primers encompassing splice junctions, genomic and unspliced regions, and human fusion transcript regions of each HPV transcript. It is therefore provided hereafter several compositions of primers depending on the desired specificity and coverage of the test. The inventors propose here an approach for detecting cancer or risk of developing cancer not limited to the specific HPVs known today to be classified as High-risk. Thus, to bypass the limitation of the current tests above, the present invention does not rely on the above 14 HR species/genotype specific oligonucleotides binding to specifically amplify and/or probe papillomavirus few DNAs or few transcripts. It relies in one aspect on identification of the different gene transcripts based on High-Throughput sequencing, which allows for further transcripts/species/genotype identification based on sequence comparison with known alpha-papillomavirus sequences in relevant databases. This has the advantage of being capable of testing simultaneously the different transcripts of a great number of papillomavirus genotypes/species and to identify relevant biomarkers along the wide range of HR and LR HPVs.

The invention also provides generic consensus primers allowing a broad amplification or pre-amplification of relevant genes of alpha HPV, not depending on the specific transcripts of the 14 HR HPVs, which are amenable to PCR testing or enhance signal/noise ratio in connection with the High-Throughput sequencing mentioned above.

More particularly, the invention relates to identification of all E6/E7 transcripts in a given sample, and recognizing to which species/genotypes they belong to, sorting the reads corresponding to other viral transcripts of the same genotype/species so as to compute ratios defining relative molecular abundance of transcripts within this (these) given genotype(s) as biomarker of cancer development.

The inventors therefore designed a kit for HPV diagnosis based on a broad screening of the level of E6 and/or E7 mRNAs within the group alpha of HPVs. In the test described in details below, the inventors designed consensus primers allowing amplification or pre-amplification E6 and/or E7 mRNAs of genotypes of sub-group alpha to detect the level of expression of, wherein a significant expression level of E6 and/or E7 of group alpha HPVs in a single time point or over time is indicative of risk of developing HPV induced cancers. Following broad range amplification with consensus HPV primers or with HPV primers designed to perform a first step of HPV specific Reverse transcription reaction, the inventors also propose to quantitate E6 and or E7 as reads delivered by next generation sequencing techniques.

The present invention also provides a method for determining the level of expression of structural or late viral proteins such as L1 or L2. In such embodiment, a ratio R1 between E6 and/or E7 and L1 and/or L2 is determined, and compared to a ratio R found in low risk or non-persistent HPV infections, wherein a ratio R1 below a reference value R is indicative of HPV infections associated with higher risk of developing genital neoplasia and cancer.

**General description of the invention**

The invention provides a high through put sequencing method allowing relative quantification of reads across oncogenic viruses, such as polyoma virus or group alpha HPV, preferably group alpha HPV, comprising enrichment of the viral RNAs in a sample using random or consensus pre-amplification and/or specific reverse transcriptase reaction, determining the number of reads matching said viruses based on species discrimination, comparing the most prevalent high risk species, further determining within said most prevalent high risk species the relative number of reads matching at least one oncogenic gene, preferably two oncogenic genes, compared to at least one non oncogenic gene, preferably several non oncogenic genes. From these discrimination steps on species and interspecies read numbers, ratios are calculated to detect increase in relative level of high risk species versus low risk species and ratios within said high risk species of reads matching oncogenic genes versus structural or regulatory genes. Applied to HPV, this test encompasses determining the level of HR HPVs reads versus LR HPVs reads through group alpha HPVs, determining the ratio of early versus late genes (E6 and or E7 versus L1 and/or L2) within the most prevalent HR HPVs, and assessing risk of developing HPV induced cancer in patients which said ratio tend towards infinity. Refined ratios can be obtained using a filter applied on reads mapping specifically RNA spliced events.

The scope of the invention is defined by the terms of the appended claims.

In one embodiment, the method is for assessing risk of developing HPV induced cancer in patients infected with at least two different HPV species of group alpha.

In one embodiment, the method is for assessing HPV virus clearance in patient receiving HPV preventive or curative HPV vaccine.

In a first aspect, the present invention relates to a method for determining a patient risk of developing oncogenic virus induced cancer according to claims 1-6.

It is disclosed herein a method for determining a patient risk of developing oncogenic virus induced cancer, such as polyoma virus or group alpha HPV comprising:

a) enrichment of the viral RNAs in a sample using random or consensus pre-amplification and/or specific reverse transcriptase reaction, preferably consensus pre-amplification;
b) sequencing cDNA produced in step a), and generating reads of said cDNA;
c) determining the number of reads matching said viruses based on species discrimination and determining the most prevalent high risk species present in the sample relative to other species;
d) determining within said most prevalent high risk species the relative number of reads matching least one oncogenic gene compared to at least one non oncogenic gene, preferably oncogenic genes compared to non oncogenic genes;
e) computing ratios within said high risk species of reads matching at least one oncogenic gene versus corresponding interspecies structural or regulatory gene, preferably oncogenic genes versus corresponding interspecies structural or regulatory genes;
f) determining risk of developing oncogenic virus induced cancer in patients wherein said ratio tend towards infinity, such as for example when ratio R is between 0.25, 0.4, 0.5, 1 to infinity.

This method is suitable for diagnosis or prognosis of risk to develop virus induced cancer in a human subject.

This method is particularly suited for assessing risk of developing HPV induced cancer in patients infected with at least two different HPV species of group alpha, for example multiple infected with HPV16, HPV35 and HPV6.

This method is also specifically suitable for assessing HPV virus clearance in patient receiving HPV preventive or curative HPV vaccine. It can be performed before vaccination to confirm vaccine potential for clearing existing infections or post-vaccination for follow-up.

In a preferred embodiment of step a), the enrichment of the viral RNAs is performed by a reverse transcription of the viral RNAs, and an amplification of the produced cDNA by multiplex-PCR with a group alpha HPV-specific composition of primers encompassing splice junctions, genomic and unspliced regions, and human fusion transcript regions of each HPV transcript.

In another embodiment, the reverse transcription is performed with random hexamers.

In another embodiment, the reverse transcription is performed with HPV-specific primers.

In another embodiment, the reverse transcription (RT) and the multiplex amplification are performed in the same tube (one-step RT-PCR).

In another preferred embodiment of step a) above, consensus pre-amplification comprises random reverse transcription of the viral RNAs followed by a multiplex amplification of the HPV transcripts.
Advantageously, the random reverse transcription is performed with random hexamers.
Advantageously, the multiplex amplification of the HPV transcripts is performed with HPV-specific primers.
In a preferred embodiment of step b) above, the sequencing is a High throughput sequencing method.
Ratio (R) is calculated as the number of reads of at least one early HPV16 transcript to the number of reads of at least one late HPV16 transcript, with a higher ratio (R) correlating with an increased risk of developing high-grade malignant HPV-induced cancer. This method further include correlating a higher number of reads of HPV16 transcripts relative to reads of transcripts of another HPV species with an increased risk of developing high-grade malignant HPV-induced cancer. To obtain sufficient number of reads, the cDNA is generated using random primers or using HPV-specific primers.
For example, the ratio is calculated by calculating a ratio (R1) of the number of reads of one HPV HR E6 and/or E7 transcripts to the number of reads of said one HPV HR L1 and/or L2 transcripts and the ratio is calculated by calculating a ratio (R2) of the number of reads of a second HPV HR E6 and E7 transcripts to the number of reads of said second HPV HR L1 and L2 transcripts. This method is applicable to determining the number of HPV sequence reads of at least 2 Alpha group HPV species, including for example HPV16.
In a specific embodiment but applied to oncogenic viruses in general such as polyoma or HPV, the method of the invention comprises:

a) optionally, pretreating nucleic acids to remove human genomic DNA,
b) optionally, pre-amplify viral mRNAs, wherein said viral mRNAs comprises oncogenic mRNAs and at least one other mRNA,
c) sequencing mRNAs, or cDNAs thereof, obtained after steps a) and b), in the sample of a human subject,
d) identifying the reads corresponding to said oncogenic mRNAs,
e) identifying to which species or genotypes said oncogenic mRNAs of step d) belong to,
f) sorting the reads corresponding to said at least one other viral mRNAs, or cDNAs thereof obtained after steps a) and b), of the same genotype or species identified in step e),
g) optionally, identifying fusion transcripts as a signature of viral DNA integrations events in the host chromosome and/or additional human cancer cell biomarkers,
h) optionally, deleting all other sequences including human sequences which are not sequences identified and sorted following steps d), e), f) and g),
i) computing ratios R defining molecular abundance of said oncogenic mRNAs relative to said at least one other viral mRNAs of the same genotype or species of step f),

wherein an increase of ratios R correlate with an increased risk of developing viral induced cancer.
By virus induced cancer, it is more particularly contemplated herein Papova virus induced cancer, more specifically Papilloma or Polyoma virus induced cancer, preferably Papilloma virus induced cancer.
By other viral mRNAs in step f) it is meant mRNAs of viral genes selected from structural genes, for example capsid genes as well as from regulatory genes, and replication/transcription genes.
The present invention relates to a method for diagnosis or prognosis of risk to develop HPV induced cancer according to claims 7-12.
It is disclosed herein a method for diagnosis or prognosis of risk to develop HPV induced cancer in a human subject comprising:

a) optionally, pretreating nucleic acids to remove human genomic DNA,
b) optionally, pre-amplify HPVs mRNAs, wherein said mRNAs comprises E6 and/or E7 HPV RNAs and at least one other HPV mRNAs,
c) sequencing nucleic acids in the sample of a human subject or obtain after steps a) and b),
d) identifying the reads corresponding to E6 and/or E7 HPV RNAs,
e) identifying to which species or genotypes E6 and/or E7 HPV mRNAs of step d)belong to,
f) sorting the reads corresponding to other viral HPV mRNAs of the same genotype or species identified in step e),
g) optionally, identifying fusion transcripts as a signature of HPV DNA integrations events in the host chromosome and/or additional human cancer cell biomarkers,
h) optionally, deleting all other sequences including human sequences which are not sequences identified and sorted following steps d), e), f) and g),
i) computing ratios defining molecular abundance of E6 and/or E7 HPV mRNAs relative to said other viral transcripts of the same genotype or species of step f),

wherein an increased level of said ratios correlates with an increased risk of developing viral induced cancer.

By other viral mRNAs in step f), it is more particularly referred to selected mRNAs from genes coding for capsid proteins (L1 and L2), gene coding for the growth stimulation protein (E5), genes coding for replication or transcription proteins (E4, E2 and E1, E8). In step g), additional human cancer cell biomarkers can be selected for example from PRC1, CCNB2, SYCP2 CDKN3, NUSAP1, CDC20, p16INK4a, Ki-67.

In one specific embodiment, step f) comprises sorting the reads of L1 and/or L2 HPV mRNAs corresponding to the species or genotype of E6 and/or E7 HPV mRNAs identified in step d). In this embodiment, step h) comprises computing ratios defining relative molecular abundance of E6 and/or E7 HPV mRNAs relative to the reads of L1 and/or at least one other viral mRNAs corresponding to the species or genotype of E6 and/or E7 HPV mRNAs. In such embodiment, step b) optionally comprises pre-amplifying HPVs mRNAs, wherein said mRNAs comprises E6 and/or E7 HPV RNAs and L1 and/or at least one other viral HPV mRNAs.

It is disclosed herein, in a second aspect, a method for diagnosis risk to develop HPV induced cancer comprising:

(a) determining the level of at least a first marker selected from E6 mRNAs of group alpha HPVs, E7 mRNAs of group alpha HPVs, or both, in the sample of a patient or in the sample of an individual suspected to be infected by HPV,
(b) comparing the levels determined in step (a) to a reference value of E6 mRNAs of group alpha HPVs, E7 mRNAs of group alpha HPVs, or both in low risk individuals infected with HPVs,
(c) wherein an increased level as determined in step a) compared to the reference level in step b) is indicative of higher risk to develop HPV cancer induced.

It must be contemplated that these biomarkers are not restricted to E6 or E7 mRNAs of HPV16, 18, 31, 33, 35, 39, 45, 51, 52, 56, 58, 59, 66, 68 but extend to all E6 or E7 mRNAs of HPVs of several genus alpha, comprising and covering HPVs of groups $\alpha$5, 6, 7, 10; optionally extending to additional alpha group HPVs as desired.

In this second aspect, the levels of E6 mRNAs of group alpha HPVs, E7 mRNAs of group alpha HPVs, or both are determined inter alia by hybridization with a labeled probe, amplification, including PCR, nucleic acid microarrays, high-throughput sequencing with or without pre-amplification. The measure may be carried out directly on an extracted messenger RNA (mRNA) sample, or on reverse transcribed complementary DNA (cDNA) prepared from extracted mRNA. From the mRNA or cDNA sample, the amount of nucleic acid transcripts is determined using nucleic acid microarrays, quantitative PCR, hybridization with a labeled probe, or directly by counting corresponding reads following high-throughput sequencing.

For both first and second aspect, amplification or pre-amplification is depicted in details below with specifically designed consensus primers allowing generic pre-amplification of all or desired HPVs belonging to group alpha, in particular pre-amplification of the specific domains of the group alpha, preferably of the HR-$\alpha$HPV. According to the invention, primers depicted below are provided to amplify and detect the amount of E6 mRNAs and E7 mRNAs of all or several group alpha HPVs depending on the desired scope of the test. Therefore, the invention provides a much broader test extending beyond types such as HPV 16, 18, 31, 33, 35, 39, 45, 51, 52, 56, 58, 59, 66, 68 which are today classified as High-risk HPVs; as it allows the determination of high level expression of E6 and/E7 of several alpha subgroups of HPVs and even covering the all HPVs of the alpha group.

For example, In the above method, the quantification is performed on E6 and/or E7 mRNAs of papillomaviruses $\alpha$6 comprising HPV 30, HPV 53, HPV 56 and HPV 66, papillomaviruses $\alpha$7 comprising HPV 68, HPV 39, HPV 70, HPV 85, HPV 59, HPV 45, HPV 18, HPV 97, papillomaviruses $\alpha$10 comprising HPV 16, HPV 35, HPV 31, HPV 52, HPV 67, HPV 33, HPV 58 and papillomaviruses $\alpha$5 comprising HPV 26, HPV, 69, HPV 51, HPV 82. The combined generic consensus primers to generically amplify alpha subgroups are described herein-after.

In a specific embodiment, the method of the invention further refines the above method aiming at the quantitative determination of expression levels of a panel of biomarkers in biological samples of patients or individuals suspected to be infected with HPVs, which combined biomarkers are indicative and /or predictive, in a single time-point, of patients at risk of developing HPV induced cancers.

The panel provided by the invention comprises the following biomarkers:

- At least a first marker selected from E6 mRNAs of group alpha HPVs, E7 mRNAs of group alpha HPVs, or both,
- At least a second marker selected from L1 mRNAs of group alpha HPVs, L2 mRNAs of group alpha HPVs, or both,
- wherein said E6, E7, L1 and L2 mRNAs have corresponding intragenetic sequences,
- optionally, at least one host cellular marker indicative of neoplasia or cancer.

For example, the panel is composed of at least 5, 10, 20, 30 or 50 different mRNAs of said E6, E7, L1 and L2 mRNAs of group alpha HPVs. In one specific embodiment, the panel is composed of all group alpha HPVs mRNAs of E6 and/or E7, and L1 and/or L2. The combined generic consensus primers to generically amplify alpha subgroups depending on the desired scope of the test are provided herein-after. Alternatively, the invention is performed using sequence-independent-amplified or direct HT-sequencing for quantitative detection of individual mRNA of E6 and/or E7 HPV sequences belonging to the HPV alpha group.

Such method according to the invention is also provided for predicting the progression of HPV infection in a patient suffering from HPV infection.

In one embodiment, the invention encompasses a method for assessing a human papilloma virus (HPV) infected patient comprising: generating cDNA from a patient sample comprising RNA; sequencing the cDNA; generating reads of sequence of the cDNA; discriminating HPV sequence reads on the basis of HPV specie; discriminating HPV sequence reads on the basis of HPV gene transcript; quantitating the number of HPV sequence reads according to HPV species and HPV gene transcript, determining the number of HPV sequence reads of at least 2 HPV gene transcripts; and determining the number of HPV sequence reads of at least 2 HPV species; wherein the patient sample contains 2 or more HPV species.

In a further embodiment, the method comprises calculating a ratio (R) of the number of reads of at least one early HPV16 transcript to the number of reads of at least one late HPV16 transcript, with a higher ratio (R) correlating with an increased risk of developing high-grade malignant HPV-induced cancer.

In another embodiment, the method comprises correlating a higher number of reads of HPV16 transcripts relative to reads of transcripts of another HPV species with an increased risk of developing high-grade malignant HPV-induced cancer.

In one embodiment, the cDNA is generated using random primers. In one embodiment, the cDNA is generated using HPV-specific primers (*i.e.,* primers specific to domains of a HPV, such as a HR-$\alpha$HPV, comprising splice junctions, genomic and unspliced regions, and human fusion transcript regions of each HPV transcript).

In one embodiment, the ratio is calculated by calculating the ratio (R) of the number of reads of HPV16 E6 and/or E7 transcripts to the number of reads of HPV16 L1 and/or L2 transcripts. In one embodiment, the ratio is calculated by calculating the ratio (R) of the number of reads of HPV16 E6 and E7 transcripts to the number of reads of L1 and L2 transcripts.

In one embodiment, the method comprises determining the number of HPV sequence reads of at least 2 alpha group HPV species. In one embodiment, the method comprises generating at least $10^6$ reads of sequence of the cDNA. In one embodiment, the method comprises generating at least $10^7$ reads of sequence of the cDNA.

In one embodiment, the invention encompasses a method for assessing a human papilloma virus (HPV) infected patient comprising generating cDNA from a patient sample comprising RNA; sequencing the cDNA; generating reads of sequence of the cDNA; discriminating HPV sequence reads on the basis of HPV gene transcript; quantitating the level of HPV sequence reads according to HPV gene transcript; determining the number of HPV sequence reads of at least one HPV early gene transcript; determining the number of HPV sequence reads of at least one HPV late gene transcript; and determining the ratio of the number of HPV sequence reads of at least one HPV early gene transcript to the number of HPV sequence reads of at least one HPV late gene transcript.

In one embodiment, the method comprises calculating a ratio (R) of the number of reads of at least one early HPV16 transcript to the number of reads of at least one late HPV16 transcript, with a higher ratio (R) correlating with an increased risk of developing high-grade malignant HPV-induced cancer.

In one embodiment, the at least one early transcript is HPV E6 or E7 and the at least one late transcript is L1 or L2. In one embodiment, the at least one early transcript is HPV E6 and E7 and the at least one late transcript is L1 and L2.

In one embodiment, the cDNA is generated using random primers. In one embodiment, the cDNA is generated using HPV specific primers.

Some of the terms used throughout the specification are specifically defined here below:

**Definitions**

Biological samples as referred herein include, without limitation, mammalian bodily fluids, especially oral fluids or scrapings, genital scrapings, in particular cervix scrapings.

HPV alpha group: HPVs are contained within five evolutionary groups. HPV types that infect the cervix come from the Alpha group which contains over 60 members. HPV types from the Beta, Gamma, Mu and Nu groups or genus primarily infect cutaneous sites. Alpha papillomaviruses can be subdivided into three categories (high risk, low risk and cutaneous), depending on their prevalence in the general population and on the frequency with which they cause cervical cancer. High-risk types come from the Alpha 5, 6, 7, and 10 groups.

Primers encompassed by the invention are not limited to the sequences defined in the primers depicted below but they can comprise extra bases at the 5' end, for example from 1 to 5 extra bases as extension corresponding to sequences of the corresponding HPVs E6 or E7. Also, primers shall be understood as embracing shorter sequences of at least 12, 15, 20 or 25 consecutive bases of the primers featured below. In some embodiments, it shall be understood that the invention also contemplates generic probes which have the sequences of the primers depicted herein and which are directly or indirectly labeled. The probes and primers can be extended or swifted from 1 to 15 bases depending on the desired specificity of the PCR amplification step and/or on the specificity of the detection step using standard parameters such as the nucleic acid size and GC contents, stringent hybridization conditions and temperature reactions. For example, low stringency conditions are used when it is desired to obtain broad positive results on a range of homologous targets whereas high stringency conditions are preferred to obtain positive results only if the specific target nucleic is present in the sample. As used herein, the term "stringent hybridization conditions" refers to conditions under which the primer or probe will hybridize only to that exactly complementary target(s). The hybridization conditions affect the stability of hybrids, e.g., temperature, salt concentration, pH, formamide concentration and the like. These conditions are optimized to maximize specific binding and minimize non-specific binding of primer or probe to its target nucleic acid sequence. Stringent conditions are sequence dependent and will be different in different circumstances. Longer sequences hybridize specifically at higher temperatures. Generally, stringent conditions are selected to be about 5°C lower than the thermal melting point (Tm) for the specific sequences at a defined ionic strength and pH. The Tm is the temperature (under defined ionic strength and pH) at which 50% of a complementary target sequence hybridizes to a perfectly matched probe or primer. Typically, stringent conditions will be those in which the salt concentration is less than about 1.0 M Na+, typically about 0.01 to 1.0 M Na+ concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions include hybridization with a buffer solution of 20-30% formamide, 1 M NaCl, 1% SDS at 37°C and a wash in 2*SSC at 40°C. Exemplary high stringency conditions include hybridization in 40-50% formamide, 1 M NaCl, 1 % SDS at 37°C, and a wash in 0.1*SSC at 60°C. Determination of particular hybridization conditions relating to a specified nucleic acid is routine and is well known in the art, for instance, as described in J. Sambrook and D.W. Russell, Molecular Cloning: A Laboratory Manual, Cold Spring Harbor Laboratory Press; 3rd Ed., 2001; and F.M. Ausubel, Ed., Short Protocols in Molecular Biology, Current Protocols; 5th Ed., 2002.

Preferred PCR primers, which can be used separately or together as a set to amplify a HPV nucleic acid sequence comprise the primers.

α1:

- E6

  Forward: 5'- RGTACWTCTGCCTCATCACAGCC -3' (SEQ ID NO. 1)
  Reverse: 3'- CTCTGCAMTGSGTACASCGAC -5' (SEQ ID NO. 2)

- E7

  Forward: 5'- GGARASRCRCCWACSCTAAAGGA -3' (SEQ ID NO. 3)
  Reverse: 3'- CACGCRGGCACACAAWGGACA -5'(5EQ ID NO. 4)

- L1

  Forward: 5'- GCGGCCTAGTGACRACAAGG -3' (SEQ ID NO. 5)
  Reverse: 3'- GCACGYAACCCRGCYTGCAG -5'(5EQ ID NO. 6)

α2:

- E6

  Forward: 5'- GHGHGCCMTAYGSTGCCTGTG -3' (SEQ ID NO. 7)
  Forward: 5'- CKCCSTACGGTGCWTGTGC -3' (SEQ ID NO. 8)
  Reverse: 3'- GCGGACCGTGCATCKTRWCCA -5' (SEQ ID NO. 9)
  Reverse: 3'- GGCTTTGGCCCATGCATCGT -5' (SEQ ID NO. 10)
  Reverse: 3'- GTGCATCGTGACCAGCAGTAC -5' (SEQ ID NO. 11)

- E7

  Forward: 5'- TTGRDTCTTGCACCAGAGGMCGT -3' (SEQ ID NO. 12)
  Forward: 5'- TGCACGGTCCGCATCCCAC -3' (SEQ ID NO. 13)
  Forward: 5'- TGTCTATGGGTGCACAAGAACCC -3' (SEQ ID NO. 14)
  Reverse: 3'- CCCTTATATCTGCKTSGCTGCWS -5' (SEQ ID NO. 15)

Reverse: 3'- GCAGCGAGGRCACACGASC -5' (SEQ ID NO. 16)
Reverse: 3'- GGACCGTGCATCGTGACCA -5' (SEQ ID NO. 17)

- L1

Forward: 5'- ATGGCWYTSTGGCGCYCTAGTG -3' (SEQ ID NO. 18)
Reverse: 3'- CCTCCARGCTAGTRGAYGGYGGY -5' (SEQ ID NO. 19)
Reverse: 3'- GGGRACYACYGAACGMCGKCGCG -5' (SEQ ID NO. 20)

α3:

- E6

Forward: 5'- AGTGGACRGGRAAGTGCWGCAAC -3' (SEQ ID NO. 21)
Forward: 5'- YTGTGCAAAGACTGCGASGTGG -3' (SEQ ID NO. 22)
Forward: 5'- ACTGGCCATTTGGAGTMTGCGC -3' (SEQ ID NO. 23)
Reverse: 3'- GGCCRYGCATGTTRCYCTACAGT -5' (SEQ ID NO. 24)
Reverse: 3'- CACYKTCCTGTCCACTBYCCWGC -5' (SEQ ID NO. 25)
Reverse: 3'- CCAGTGYCGTAGCTCYCGYRYC -5' (SEQ ID NO. 26)
Reverse: 3'- CTGGCCGTGCATRSYCCTCT -5' (SEQ ID NO. 27)

- E7

Forward: 5'- VAGCAMAGCWGGCCYWTAGGGTG -3' (SEQ ID NO. 28)
Forward: 5'- KGYWGAACRRGCACAGCAGGCC -3' (SEQ ID NO. 29)
Reverse: 3'- GGCCACYRCKTCCACYATAAGCT -5' (SEQ ID NO. 30)
Reverse: 3'- CAGCYGGGACACACTATRTCCAC -5' (SEQ ID NO. 31)
Reverse: 3'- GCGCAGCSVGGACACACTAT -5' (SEQ ID NO. 32)

- L1

Forward: 5'- CTWTGTGGCGRCMTGGTGAYGGC -3' (SEQ ID NO. 33)
Reverse: 3'- GGARGGAGGGGGCAMWACMCC -5' (SEQ ID NO. 34)
Reverse: 3'- CCCTGBGCVCGNTGYAGCCAR -5' (SEQ ID NO. 35)

α4:

- E6

Forward: 5'- SAGTATGGTYTGGAGCTAGAGGA -3' (SEQ ID NO. 36)
Reverse: 3'- GTCCSGTCCACYGGCCKGM -5' (SEQ ID NO. 37)

- E7

Forward: 5'- MCGMCCCAGCCTSRMGGAC -3' (SEQ ID NO. 38)
Reverse: 3'- CCTCCATRACGCTABGCGCAG -5'(5EQ ID NO. 39)

- L1

Forward: 5'- TGGCCTAAACGACGTAAACGTGT -3' (SEQ ID NO. 40)
Forward: 5'- TTCTTTGCAGATGGCTWTGTGGC -3'(5EQ ID NO. 41)
Reverse: 5'- YGTGTCTCGMAARCGCRCCGC -3' :: 3'- GCGGYGCGYTTKCGAGACACR -5'(5EQ ID NO. 42)
Reverse: 5'- CGCAAGTTYTTRYTGCAGCGGGG -3' :: 3'- CCCCGCTGCARYAARAACTTGCG -5'(5EQ ID NO. 43)

α5:

- E6

Forward: 5'- GRGAAAGACCACGAACGCTGC -3' (SEQ ID NO. 44)
Forward: 5'- AATAGCAGGGYASTGGAAAGGGT -3' (SEQ ID NO. 45)
Reverse: 3'- GCAATTWGCRCAYTGYCCCGTCC -5' (SEQ ID NO. 46)
Reverse: 3'- TTGTGTTTCTGTTTGGCGCCTTG -5' (SEQ ID NO. 47)
Reverse: 3'- GCCTTGGTCTCCAGCAGTTTG -5'(5EQ ID NO. 48)

- E7

Forward: 5'- YTAGATYTGGTGCCGCAACCCG -3' (SEQ ID NO. 49)
Forward: 5'- MGCCATGCGTGGTAATGTACCAC -3' (SEQ ID NO. 50)
Reverse: 3'- CTCCASCRCTCGRACGTTCTGT -5' (SEQ ID NO. 51)
Reverse: 3'- CACGGGCAMACCAGGCTTAGK -5' (SEQ ID NO. 52)

- L1

Forward: 5'- KCAGATGGCYTTGYGGCGTACTA -3' (SEQ ID NO. 53)
Forward: 5'- TGGCYTTGYGGCGTACTAGTGAC -3' (SEQ ID NO. 54)
Forward: 5'- TGTATTTRCCACCTGCACCWGTG -3' (SEQ ID NO. 55)
Reverse: 3'- GGGGCRTYRCGYTGACAKGTAGT -5' (SEQ ID NO. 56)
Reverse: 3'- GGCMGGSCKTTTAAGGCCTGGT -5' (SEQ ID NO. 57)

α6:

- E6

Forward: 5'- GARCGHCCACGWASHBTGCACC -3' (SEQ ID NO. 58)
Forward: 5'- AATACAGRMGAGCGMCCACGTAC -3' (SEQ ID NO. 59)
Forward: 5'- RCAATMCACAGGAACGTCCACGA -3' (SEQ ID NO. 60)
Reverse: 3'- CCTCTGGTGTCAACGGMTGTTGA -5' (SEQ ID NO. 61)
Reverse: 3'- TCTCCARCACY5CAAACATGACC -5' (SEQ ID NO. 62)

- E7

Forward: 5'- GRACAGCTCAGAGGAWGAGGATG -3' (SEQ ID NO. 63)
Forward: 5'- GCTCAGAGGAWGAGGATGAGG -3' (SEQ ID NO. 64)
Forward: 5'- YTRCWGRAGCRGCCACAGCAAGC -3' (SEQ ID NO. 65)
Forward: 5'- GRAGCRGCCACAGCAAGCTAG -3' (SEQ ID NO. 66)
Forward: 5'- GAACAGCTCAGAGGAWGAGGATG -3' (SEQ ID NO. 67)
Forward: 5'- ARTAGACCATTTGCWGGAGCGGC -3' (SEQ ID NO. 68)
Reverse: 3'- GCCTTGTTGCRCASAGGGG -5' (SEQ ID NO. 69)
Reverse: 3'- CGCAGAGTGGGCACGTTACT -5' (SEQ ID NO. 70)

- L1

Forward: 5'- TTGCAGATGGCGRYGTGGCG -3' (SEQ ID NO. 71)
Reverse: 3'- CACCTAAAGGYTGDCCDCGGC -5'(5EQ ID NO. 72)

α7:

- E6

Forward: 5'- TASAGGACAGTGYCGMCRSTGC -3' (SEQ ID NO. 73)
Forward: 5'- TCMCAAYCCTGMRGAACGGCCAT -3' (SEQ ID NO. 74)
Forward: 5'- ASAGGACAGTGTCGYSGGTG -3' (SEQ ID NO. 75)
Forward: 5'- TGCCAGAAACCRTTGAAYCCAGC -3' (SEQ ID NO. 76)
Reverse: 3'- GTCTGCGGTCCTCYCGBTTDST -5' (SEQ ID NO. 77)

Reverse: 3'- CTGSCCTCKRTASTGCCCAGCT -5' (SEQ ID NO. 78)
Reverse: 3'- CACCAGTGTTTCACTACGCGC -5'(5EQ ID NO. 79)
Reverse: 3'- GCCTTGCTGTTCTTGTGCACG -5' (SEQ ID NO. 80)
Reverse: 3'- GTCTGGAAAGCCTTTCTTGCCGT -5' (SEQ ID NO. 81)

- E7

Forward: 5'- GACGRGMHGAACMACARCGTCAC -3' (SEQ ID NO. 82)
Forward: 5'- GACGRGMHGAACMACAGCGTCAC -3' (SEQ ID NO. 83)
Forward: 5'- ARCACCYTGTCCTTTGTGTGTCC -3' (SEQ ID NO. 84)
Reverse: 3'- GTGWSTCCATAAACAGCWGCWGT -5' (SEQ ID NO. 85)
Reverse: 3'- CACACCAMGGACACACAAAGGAC -5' (SEQ ID NO. 86)

- L1

Forward: 5'- GCGBTCTAGYGACARCAHGGTGT -3' (SEQ ID NO. 87)
Forward: 5'- HCCTGCTATTGGKGARCAYTGGG -3' (SEQ ID NO. 88)
Reverse: 3'- CCAGTGYTCYCCMATRGCRGGWA -5' (SEQ ID NO. 89)
Reverse: 3'- TAGASCCACTDGGWGANGGRGAA -5' (SEQ ID NO. 90)

α8:

- E6

Forward: 5'- WATGWCTGCACGKWGCKGCTCC -3' (SEQ ID NO. 91)
Reverse: 3'- GTAGGCARTATCCYTTCCACRCG -5' (SEQ ID NO. 92)
Reverse: 3'- CTCCGAGCGTTGGCCTTTC -5' (SEQ ID NO. 93)

- E7

Forward: 5'- GCGTGAGCAAYCCACGCAAC -3' (SEQ ID NO. 94)
Reverse: 3'- CAGCCATKGYAGTCACACMGCTG -5' (SEQ ID NO. 95)
Reverse: 3'- TGCCATTGTTGTCACKCTGTAGC -5' (SEQ ID NO. 96)

- L1

Forward: 5'- CCYCCHATKGGNGAATATTGGGG -3' (SEQ ID NO. 97)
Reverse: 3'- GGAGGATGGTGCWGMACGC -5' (SEQ ID NO. 98)
Reverse: 3'- GGGTGACTGRCYYAGAAGAGGAA -5' (SEQ ID NO. 99)

α9:

- E6

Forward: 5'- AGTRMARATGCCTCCACGYCTGC -3' (SEQ ID NO. 100)
Forward: 5'- CTGCACAGGACCAGATGGC -3' (SEQ ID NO. 101)
Reverse: 3'- TCCATGCATGWTGWCCAGCARTG -5' (SEQ ID NO. 102)
Reverse: 3'- GCAGCGMCCYTTCCAGGTRTCK -5' (SEQ ID NO. 103)
Reverse: 3'- GGCATTTCGCCCACCATTGTTAT -5' (SEQ ID NO. 104)

- E7

Forward: 5'- GCYTACACTGCTGGACAACATGC -3' (SEQ ID NO. 105)
Forward: 5'- AGACAGCTCAGAAGABGAGGTGG -3' (SEQ ID NO. 106)
Forward: 5'- AACAATGGTGGGCGAAATGCCAG -3' (SEQ ID NO. 107)
Reverse: 3'- CGTCCGCCATCSTTGTTATGKYT -5' (SEQ ID NO. 108)
Reverse: 3'- CCTGTRCACTSCACMACMAGCC -5' (SEQ ID NO. 109)
Reverse: 3'- CTGTCGCTGTAGGGTGCACA -5'(5EQ ID NO. 110)

- L1

Forward: 5'- ATGTGCCTCCTCCYRMCCCWGTA -3' (SEQ ID NO. 111)
Forward: 5'- AGATGGCTGTCTGGTTACCAGC -3' (SEQ ID NO. 112)
Reverse: 3'- CCATAWGGRTCYGCAGCCATTTG -5' (SEQ ID NO. 113)
Reverse: 3'- GCCTTACGCCTGCGCTTGG -5' (SEQ ID NO. 114)

α10:

- E6

Forward: 5'- CCSARSTGTAAWCATGCRTGGAG -3' (SEQ ID NO. 115)
Forward: 5'- MCGSAMCCTGCACGAATTGTGTG -3' (SEQ ID NO. 116)
Forward: 5'- CARGACRCWGAGGARAAACCACG -3' (SEQ ID NO. 117)
Reverse: 3'- CCAACACWCTGAACASCGYCC -5' (SEQ ID NO. 118)
Reverse: 3'- CCATGCATGATTACASCTSGGTT -5' (SEQ ID NO. 119)
Reverse: 3'- GTCGGGRYCTCCAACACRCYG -5' (SEQ ID NO. 120)
Reverse: 3'- CTCCACGCATGTTTACACTTGGG -5' (SEQ ID NO. 121)

- E7

Forward: 5'- GCWCAYTWGGAATHGTGTGCCCC -3' (SEQ ID NO. 122)
Forward: 5'- CSTGTAAMAACGCCATGAGAGGA -3' (SEQ ID NO. 123)
Forward: 5'- CGCCATGAGAGGAMACAASCCA -3' (SEQ ID NO. 124)
Reverse: 3'- GGCACACDATTCCWARTGWGCCC -5' (SEQ ID NO. 125)
Reverse: 3'- GGTTCGTASGTCRSTTGYTGTAC -5' (SEQ ID NO. 126)
Reverse: 3'- GTGCACAGSYGGGRCACACWAYT -5' (SEQ ID NO. 127)

- L1

Forward: 5'- GARGCCACWGTSTACYTGCCTC -3' (SEQ ID NO. 128)
Forward: 5'- ACAGATGTCTCTGTGGCGGC -3' (SEQ ID NO. 129)
Reverse: 3'- GGATGNCCACTWAYRCCHACDCC -5' (SEQ ID NO. 130)
Reverse: 3'- GAGGWWACCATAGARCCACTRGG -5' (SEQ ID NO. 131)
Reverse: 3'- GTGCACGYTGTAGCCAATAWGGC -5' (SEQ ID NO. 132)
Reverse: 3'- TCCTGTAAACTRGCAGAYGGAGG -5'(5EQ ID NO. 133)
Reverse: 3'- GGCCYTGTGCWCGTTGYAACCAA -5' (SEQ ID NO. 134)

α11:

- E6

Forward:5'- GAACGRCCATACAAGCTACMAGC -3' (SEQ ID NO. 135)
Reverse: 3'- GCAGATGGTCTCCAGCACYG -5' (SEQ ID NO. 136)

- E7

Forward: 5'- WATTGTGTGCCCCAACTGTTCCA -3' (SEQ ID NO. 137)
Reverse: 3'- CTGGAACAGTTGGGGCACACA -5' (SEQ ID NO. 138)

- L1

Forward: 5'- AGTTCTATCTTCCTCCCCAGCC -3' (SEQ ID NO. 139)
Reverse: 3'- GGACGKGCACGCATACCWAG -5' (SEQ ID NO. 140)

α13:

- E6

Forward: 5'- TGTCTGCTACTGAACCCCACAC -3' (SEQ ID NO. 141)
Reverse: 3'- GGCTTCCAGCAATGTAGACACC -5' (SEQ ID NO. 142)

- E7

Forward: 5'- GTTTGACCTGTACTGCAGGGAG -3' (SEQ ID NO. 143)
Reverse: 3'- GTGAAGCACAGGTGGGACACA -5' (SEQ ID NO. 144)

- L1

Forward: 5'- AAAGTATACCTGCCTCCTACCCC -3' (SEQ ID NO. 145)
Reverse: 3'- GCACGCTTGCGCGCTGTAC -5' (SEQ ID NO. 146)

α14:

- E6

Forward: 5'- TAYSAMSTGGACCTGCAGGACC -3' (SEQ ID NO. 147)
Reverse: 3'- GGCCWYGCATGRTKTCCAACACT -5'(5EQ ID NO. 148)

- E7

Forward: 5'- CAATTWGCCAGCTCAGAMGAGGA -3' (SEQ ID NO. 149)
Reverse: 3'- CCACCACMAGCCTWACTGYACRV -5' (SEQ ID NO. 150)

- L1

Forward: 5'- ARGTATACCTGCCTCCYGCCC -3' (SEQ ID NO. 151)
Reverse: 3'- CCTGTGCWCGTTGYAGCCAG -5' (SEQ ID NO. 152)

As used herein, G is used to designate Guanine, A is used to designate Adenine, T is used to designate a Thymine, C is used to designate a Cytosine. R is commonly used to designate a Purine (A or G), Y is commonly used to designate a Pyrimidine (T or C), W is commonly used to designate A or T, S is commonly used to designate C or G, K is commonly used to designate G or T, H is commonly used to designate A or T or C, B is commonly used to designate G or C or T, V is commonly used to designate G or A or T, D is commonly used to designate G or A or T, N is commonly used to designate any nucleotide (A or T or C or G).

Addition of indices and sequencing adapters are needed for sequencing technologies and can be added by standard procedures. For example, said primers can be used in solution or linked to a solid support. To permit its covalent coupling to the support, the primer is generally functionalized. Thus, it may be modified by a thiol, amine or carboxyl terminal group at the 5 ' or 3' position. In particular, the addition of a thiol, amine or carboxyl group makes it possible, for example, to couple the oligonucleotide to a support bearing disulphide, maleimide, amine, carboxyl, ester, epoxide, cyanogen bromide or aldehyde functions. These couplings form by establishment of disulphide, thioether, ester, amide or amine links between the primer and the support. Any other method known to a person skilled in the art may be used, such as bifunctional coupling reagents, for example.

Moreover, to improve the hybridization with the coupled oligonucleotide, it can be advantageous for the oligonucleotide to contain an "arm" and a "spacer" sequence of bases. The use of an arm makes it possible, in effect, to bind the primer at a chosen distance from the support, enabling its conditions of interaction with the DNA to be improved. The arm advantageously consists of a linear carbon chain, comprising 1 to 18 and preferably 6 or 12 ($CH_2$) groups, and an amine which permits binding to the column. The arm is linked to a phosphate of the oligonucleotide or of a "spacer" composed of bases which do not interfere with the hybridization. Thus, the "spacer" can comprise purine bases. As an example, the "spacer" can comprise the sequence GAGG. The arm is advantageously composed of a linear carbon chain comprising 6 or 12 carbon atoms.

For implementation of the present invention, different types of support may be used. These can be functionalized chromatographic supports, in bulk or prepacked in a column, functionalized plastic surfaces or functionalized latex beads, magnetic or otherwise. Chromatographic supports are preferably used. As an example, the chromatographic supports capable of being used are agarose, acrylamide or dextran as well as their derivatives (such as Sephadex, Sepharose, Superose, etc.), polymers such as poly(styrene/divinylbenzene), or grafted or ungrafted silica, for example. The chromatography columns can operate in the diffusion or perfusion mode.

As used herein, the term "sequencing" is used in a broad sense and refers to any technique known by the skilled person including but not limited to Sanger dideoxy termination sequencing, whole-genome sequencing, sequencing by hybridization, pyrosequencing, capillary electrophoresis, cycle sequencing, single-base extension sequencing, solid- phase sequencing, high-throughput sequencing, massively parallel signature sequencing (MPSS), sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD(R) sequencing, MS-PET sequencing, mass spectrometry, and a combination thereof. In specific embodiments, the method and kit of the invention is adapted to run on ABI PRISM(R) 377 DNA Sequencer, an ABI PRISM(R) 310, 3100, 3100-Avant, 3730, or 3730x1 Genetic Analyzer, an ABI PRISM(R) 3700 DNA Analyzer, or an Applied Biosystems SOLiD(TM) System (all from Applied Biosystems), a Genome Sequencer 20 System (Roche Applied Science).
For all technologies described herein, although the said primers can be used in solution, in another embodiment the said primers are linked to a solid support.
To permit its covalent coupling to the support, the primer is generally functionalized. Thus, it may be modified by a thiol, amine or carboxyl terminal group at the 5 ’ or 3’ position. In particular, the addition of a thiol, amine or carboxyl group makes it possible, for example, to couple the oligonucleotide to a support bearing disulphide, maleimide, amine, carboxyl, ester, epoxide, cyanogen bromide or aldehyde functions. These couplings form by establishment of disulphide, thioether, ester, amide or amine links between the primer and the support. Any other method known to a person skilled in the art may be used, such as bifunctional coupling reagents, for example.
Moreover, to improve the hybridization with the coupled oligonucleotide, it can be advantageous for the oligonucleotide to contain an "arm" and a "spacer" sequence of bases. The use of an arm makes it possible, in effect, to bind the primer at a chosen distance from the support, enabling its conditions of interaction with the DNA to be improved. The arm advantageously consists of a linear carbon chain, comprising 1 to 18 and preferably 6 or 12 (CH2) groups, and an amine which permits binding to the column. The arm is linked to a phosphate of the oligonucleotide or of a "spacer" composed of bases which do not interfere with the hybridization. Thus, the "spacer" can comprise purine bases. As an example, the "spacer" can comprise the sequence GAGG. The arm is advantageously composed of a linear carbon chain comprising 6 or 12 carbon atoms.
For implementation of the present invention, different types of support may be used. These can be functionalized chromatographic supports, in bulk or prepacked in a column, functionalized plastic surfaces or functionalized latex beads, magnetic or otherwise. Chromatographic supports are preferably used. As an example, the chromatographic supports capable of being used are agarose, acrylamide or dextran as well as their derivatives (such as Sephadex, Sepharose, Superose, etc.), polymers such as poly(styrene/divinylbenzene), or grafted or ungrafted silica, for example. The chromatography columns can operate in the diffusion or perfusion mode.

As used herein, "oncogenic genes or oncogenic mRNAs" refers to genes or mRNAs which are directly or indirectly inducing cell transformation into cancer cells development. For example, oncogenic genes are used to designated E6 genes and /or E7 genes.

As used herein, "other viral mRNAs" refers to mRNAs coding for capsid proteins (L1 and L2), mRNAs coding for the growth stimulation (E5), mRNAs coding for replication/transcription (E4 and E2) and mRNAs coding for replication (E1 and E8), which are not oncogenic genes.

R ratios as used herein are defined as the relative level of an oncogenic mRNA, for example E6, E7 or oncogenic mRNAs, for example E6 + E7, compared to other viral mRNAs of the same genotype or species, more particularly compared to selected mRNAs from genes coding for capsid proteins (L1 and L2), gene coding for the growth stimulation protein (E5), genes coding for replication or transcription proteins (E4, E2 and E1, E8).
For example, a reference Ratio R can be defined as

$$R = \sum(xE6 \text{ and/or } xE7) / \sum(xL1 \text{ and/or } xL2 \text{ and/or } xE2....)$$

Wherein x is a factor in the range 0-1000000,
Wherein xE6 is for example either the number of reads mapped to the gene Ex, or the number of times each nucleotide of the gene Ex is sequenced,
and wherein xL1 is for example the number of reads mapped to the gene Lx, or the number of times each nucleotide of the gene Lx is sequenced.

## Drawings

FIG. 1 represents percentage homology between oncogenic proteins E6 HPVs HR and LR. The x-axis corresponds to HR-HPVs and y-axis represents the percentage of the nucleotide sequences homology of HPVs compared to LR HR HPVs.

FIG. 2 represents percentage homology between oncogenic proteins E7 HPVs HR and LR. The x-axis corresponds to HR-HPVs and y-axis represents the percentage of the nucleotide sequences homology of HPVs compared to LR HR HPVs.

FIG. 3 represents percentage homology between oncogenic proteins E6 HPVs HR. The x-axis corresponds to HR-HPVs and y-axis represents the percentage of the nucleotide sequences homology of HR HPVs.

FIG. 4 represents percentage homology between oncogenic proteins E7 HPVs HR. The x-axis corresponds to HR-HPVs and y-axis represents the percentage of the nucleotide sequences homology of H HPVs.

FIG. 5 represents percentage homology between oncogenic proteins E6 HPVs LR. The x-axis corresponds to HR-HPVs and y-axis represents the percentage of the nucleotide sequences homology of HR HPVs.

FIG. 6 represents percentage homology between oncogenic proteins E7 HPVs LR. The x-axis corresponds to HR-HPVs and y-axis represents the percentage of the nucleotide sequences homology of HR HPVs.

FIG. 7 represents transcription map of HR αHPV. Upper part: genomic coordinates of splice donor (SD) and acceptor (SA) sites are indicated for each HR αHPV (light grey for previously documented sites, dark grey for sites identified by analogy). Additional polyA (pA) and putative breakpoint sites (put bkpt) are added. Lower part: overview of HR αHPV splice events (black line: sequences found in mRNA; dot line: splice events) delineating splice isoforms that compose the αHPV transcripts database.

**Detailed description**

Referring to both first and second aspect, and in a first specific embodiment, the method is practiced to include at least the group consisting of papillomaviruses α6 comprising HPV 30, HPV 53, HPV 56 and HPV 66, papillomaviruses α7 comprising HPV 68, HPV 39, HPV 70, HPV 85, HPV 59, HPV 45, HPV 18, HPV 97, papillomaviruses α10 comprising HPV 16, HPV 35, HPV 31, HPV 52, HPV 67, HPV 33, HPV 58 and papillomaviruses α5 comprising HPV 26, HPV, 69, HPV 51, HPV 82.

The invention also relates to a composition according to claim 13.

In this regard, the invention also contemplates a composition of primers comprising for E6: α5: both SEQ ID NO. 44 and SEQ ID NO. 45, and all three SEQ ID NO. 46, SEQ ID NO. 47 and SEQ ID NO. 48 and; α6: SEQ ID NO. 58 or both SEQ ID NO. 59 and SEQ ID NO. 60, and both SEQ ID NO. 61 and SEQ ID NO. 62and; α7: all three SEQ ID NO. 73, SEQ ID NO. 75 and SEQ ID NO. 76 or all three EQ ID NO. 74, SEQ ID NO. 75 and SEQ ID NO. 76, and all five SEQ ID NO. 77, SEQ ID NO. 78, SEQ ID NO. 79, SEQ ID NO. 80 and SEQ ID NO. 81 and ; α10: all three SEQ ID NO. 115, SEQ ID NO. 116, SEQ ID NO. 117 and all four SEQ ID NO. 118, SEQ ID NO. 119, SEQ ID NO. 120, SEQ ID NO. 121,

And comprising for L1 α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134.

Or, in this regard, the invention also contemplates a composition of primers comprising for E7: α5: both SEQ ID NO. 49, SEQ ID NO. 50 and both SEQ ID NO. 51, SEQ ID NO. 52; and α6: SEQ ID NO. 63 or SEQ ID NO. 64 or SEQ ID NO. 65 SEQ ID NO. 66 or both SEQ ID NO. 67 and SEQ ID NO. 68, and both SEQ ID NO. 69 and SEQ ID NO. 70; and α7: SEQ ID NO. 82 or both SEQ ID NO. 83, SEQ ID NO. 84, and both SEQ ID NO. 85, SEQ ID NO. 86; and α10: all three SEQ ID NO. 122, SEQ ID NO. 123 and SEQ ID NO. 124, and all three SEQ ID NO. 125, SEQ ID NO. 126 and SEQ ID NO. 127,

And comprising for L1 α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134.

In a second specific embodiment the method is practiced to include at least the group consisting of papillomaviruses α6 comprising HPV 30, HPV 53, HPV 56 and HPV 66, papillomaviruses α7 comprising HPV 68, HPV 39, HPV 70, HPV 85, HPV 59, HPV 45, HPV 18, HPV 97, papillomaviruses α10 comprising HPV 16, HPV 35, HPV 31, HPV 52, HPV 67, HPV 33, HPV 58, papillomaviruses α5 comprising HPV 26, HPV, 69, HPV 51, HPV 82 and papillomaviruses α9 comprising HPV 6, HPV 11, HPV 13, HPV 1, HPV 74, HPV 44.

In this regard, the invention also contemplates a composition of primers comprising for E6: α5: both SEQ ID NO. 44 and SEQ ID NO. 45, and all three SEQ ID NO. 46, SEQ ID NO. 47 and SEQ ID NO. 48and; α6: SEQ ID NO. 58 or both SEQ ID NO. 59 and SEQ ID NO. 60, and both SEQ ID NO. 61 and SEQ ID NO. 62and; α7: all three SEQ ID NO. 73, SEQ ID NO. 75 and SEQ ID NO. 76 or all three EQ ID NO. 74, SEQ ID NO. 75 and SEQ ID NO. 76, and all five SEQ ID NO. 77, SEQ ID NO. 78, SEQ ID NO. 79, SEQ ID NO. 80 and SEQ ID NO. 81 and ; α10: all three SEQ ID NO.

115, SEQ ID NO. 116, SEQ ID NO. 117 and all four SEQ ID NO. 118, SEQ ID NO. 119, SEQ ID NO. 120, SEQ ID NO. 121; and α9: both SEQ ID NO. 100 and SEQ ID NO. 101 and all three SEQ ID NO. 102, SEQ ID NO. 103 and SEQ ID NO. 104,

And comprising for L1 α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114.

Or, in this regard, the invention also contemplates a composition of primers comprising for E7: α5: both SEQ ID NO. 49, SEQ ID NO. 50 and both SEQ ID NO. 51, SEQ ID NO. 52; and α6: SEQ ID NO. 63 or SEQ ID NO. 64 or SEQ ID NO. 65 SEQ ID NO. 66 or both SEQ ID NO. 67 and SEQ ID NO. 68, and both SEQ ID NO. 69 and SEQ ID NO. 70; and α7: SEQ ID NO. 82 or both SEQ ID NO. 83, SEQ ID NO. 84, and both SEQ ID NO. 85, SEQ ID NO. 86; and α10: all three SEQ ID NO. 122, SEQ ID NO. 123 and SEQ ID NO. 124, and all three SEQ ID NO. 125, SEQ ID NO. 126 and SEQ ID NO. 127; and α9: all three SEQ ID NO. 105, SEQ ID NO. 106 and SEQ ID NO. 107, and all three SEQ ID NO. 108, SEQ ID NO. 109 and 110, And comprising for L1 α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114.

In a third specific embodiment, the above defined invention is practiced to include at least the group consisting of papillomaviruses α6 comprising HPV 30, HPV 53, HPV 56 and HPV 66, papillomaviruses α7 comprising HPV 68, HPV 39, HPV 70, HPV 85, HPV 59, HPV 45, HPV 18, HPV 97, papillomaviruses α10 comprising HPV 16, HPV 35, HPV 31, HPV 52, HPV 67, HPV 33, HPV 58, papillomaviruses α5 comprising HPV 26, HPV, 69, HPV 51, HPV 82, papillomaviruses α9 comprising HPV 6, HPV 11, HPV 13, HPV 1, HPV 74, HPV 44 and papillomaviruses α8 comprising HPV 91, HPV 43, HPV 7, HPV 40.

In this regard, the invention also contemplates a composition of primers comprising for E6: α5: both SEQ ID NO. 44 and SEQ ID NO. 45, and all three SEQ ID NO. 46, SEQ ID NO. 47 and SEQ ID NO. 48and; α6: SEQ ID NO. 58 or both SEQ ID NO. 59 and SEQ ID NO. 60, and both SEQ ID NO. 61 and SEQ ID NO. 62 and; α7: all three SEQ ID NO. 73, SEQ ID NO. 75 and SEQ ID NO. 76 or all three EQ ID NO. 74, SEQ ID NO. 75 and SEQ ID NO. 76, and all five SEQ ID NO. 77, SEQ ID NO. 78, SEQ ID NO. 79, SEQ ID NO. 80 and SEQ ID NO. 81 and ; α10: all three SEQ ID NO. 115, SEQ ID NO. 116, SEQ ID NO. 117 and all four SEQ ID NO. 118, SEQ ID NO. 119, SEQ ID NO. 120, SEQ ID NO. 121; and α9: both SEQ ID NO. 100 and SEQ ID NO. 101 and all three SEQ ID NO. 102, SEQ ID NO. 103 and SEQ ID NO. 104; and α8: SEQ ID NO. 91, and both SEQ ID NO. 92 and SEQ ID NO. 93.

And comprising for L1 α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114; and α8: SEQ ID NO. 97, and both SEQ ID NO. 98 and SEQ ID NO. 99.

Or, in this regard, the invention also contemplates a composition of primers comprising for E7 : α5: both SEQ ID NO. 49, SEQ ID NO. 50 and both SEQ ID NO. 51, SEQ ID NO. 52; and α6: SEQ ID NO. 63 or SEQ ID NO. 64 or SEQ ID NO. 65 SEQ ID NO. 66 or both SEQ ID NO. 67 and SEQ ID NO. 68, and both SEQ ID NO. 69 and SEQ ID NO. 70; and α7: SEQ ID NO. 82 or both SEQ ID NO. 83, SEQ ID NO. 84, and both SEQ ID NO. 85, SEQ ID NO. 86; and α10: all three SEQ ID NO. 122, SEQ ID NO. 123 and SEQ ID NO. 124, and all three SEQ ID NO. 125, SEQ ID NO. 126 and SEQ ID NO. 127; and α9: all three SEQ ID NO. 105, SEQ ID NO. 106 and SEQ ID NO. 107, and all three SEQ ID NO. 108, SEQ ID NO. 109 and 110; and α8: SEQ ID NO. 94, and both SEQ ID NO. 95 and SEQ ID NO. 96

And comprising for L1 α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114; and α8: SEQ ID NO. 97, and both SEQ ID NO. 98 and SEQ ID NO. 99.

In a forth specific embodiment, the above defined invention is practiced to include at least the group consisting of papillomaviruses α6 comprising HPV 30, HPV 53, HPV 56 and HPV 66, papillomaviruses α7 comprising HPV 68, HPV 39, HPV 70, HPV 85, HPV 59, HPV 45, HPV 18, HPV 97, papillomaviruses α10 comprising HPV 16, HPV 35, HPV 31, HPV 52, HPV 67, HPV 33, HPV 58, papillomaviruses α5 comprising HPV 26, HPV, 69, HPV 51, HPV 82, papillomaviruses α9 comprising HPV 6, HPV 11, HPV 13, HPV 1, HPV 74, HPV 44, papillomaviruses α8 comprising HPV 91, HPV 43, HPV 7, HPV 40 and papillomaviruses α1 comprising HPV 42, HPV 32.

In this regard, the invention also contemplates a composition of primers comprising for E6: α5: both SEQ ID NO. 44 and SEQ ID NO. 45, and all three SEQ ID NO. 46, SEQ ID NO. 47 and SEQ ID NO. 48and; α6: SEQ ID NO. 58 or both SEQ ID NO. 59 and SEQ ID NO. 60, and both SEQ ID NO. 61 and SEQ ID NO. 62and; α7: all three SEQ ID NO. 73, SEQ ID NO. 75 and SEQ ID NO. 76 or all three EQ ID NO. 74, SEQ ID NO. 75 and SEQ ID NO. 76, and all five SEQ ID NO. 77, SEQ ID NO. 78, SEQ ID NO. 79, SEQ ID NO. 80 and SEQ ID NO. 81 and ; α10: all three SEQ ID NO. 115, SEQ ID NO. 116, SEQ ID NO. 117 and all four SEQ ID NO. 118, SEQ ID NO. 119, SEQ ID NO. 120, SEQ ID NO. 121; and α9: both SEQ ID NO. 100 and SEQ ID NO. 101 and all three SEQ ID NO. 102, SEQ ID NO. 103 and SEQ ID NO. 104; and α8: SEQ ID NO. 91, and both SEQ ID NO. 92 and SEQ ID NO. 93; and α1: SEQ ID NO. 1 and SEQ ID NO. 2

And comprising for L1 α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114; and α8: SEQ ID NO. 97, and both SEQ ID NO. 98 and SEQ ID NO. 99; and α1: SEQ ID NO. 5 and SEQ ID NO. 6.

Or, in this regard, the invention also contemplates a composition of primers comprising for E7: α5: both SEQ ID NO. 49, SEQ ID NO. 50 and both SEQ ID NO. 51, SEQ ID NO. 52; and α6: SEQ ID NO. 63 or SEQ ID NO. 64 or SEQ ID NO. 65 SEQ ID NO. 66 or both SEQ ID NO. 67 and SEQ ID NO. 68, and both SEQ ID NO. 69 and SEQ ID NO. 70; and α7: SEQ ID NO. 82 or both SEQ ID NO. 83, SEQ ID NO. 84, and both SEQ ID NO. 85, SEQ ID NO. 86; and α10: all three SEQ ID NO. 122, SEQ ID NO. 123 and SEQ ID NO. 124, and all three SEQ ID NO. 125, SEQ ID NO. 126 and SEQ ID NO. 127; and α9: all three SEQ ID NO. 105, SEQ ID NO. 106 and SEQ ID NO. 107, and all three SEQ ID NO. 108, SEQ ID NO. 109 and 110; and α8: SEQ ID NO. 94, and both SEQ ID NO. 95 and SEQ ID NO. 96; and α1: SEQ ID NO. 3 and SEQ ID NO. 4

And comprising for L1 α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114; and α8: SEQ ID NO. 97, and both SEQ ID NO. 98 and SEQ ID NO. 99; and α1: SEQ ID NO. 5 and SEQ ID NO. 6.

In a fifth specific embodiment, the above defined invention is practiced to include at least the group consisting of papillomaviruses α6 comprising HPV 30, HPV 53, HPV 56 and HPV 66, papillomaviruses α7 comprising HPV 68, HPV 39, HPV 70, HPV 85, HPV 59, HPV 45, HPV 18, HPV 97, papillomaviruses α10 comprising HPV 16, HPV 35, HPV 31, HPV 52, HPV 67, HPV 33, HPV 58, papillomaviruses α5 comprising HPV 26, HPV, 69, HPV 51, HPV 82, papillomaviruses α9 comprising HPV 6, HPV 11, HPV 13, HPV 1, HPV 74, HPV 44, papillomaviruses α8 comprising HPV 91, HPV 43, HPV 7, HPV 40 papillomaviruses α1 comprising HPV 42, HPV 32 and papillomavirus α3 comprising HPV 114, HPV 84, HPV 86, HPV87, HPV 102, HPV83, HPV89, HPV 61, HPV 72, HPV 62.

In this regard, the invention also contemplates a composition of primers comprising for E6: α5: both SEQ ID NO. 44 and SEQ ID NO. 45, and all three SEQ ID NO. 46, SEQ ID NO. 47 and SEQ ID NO. 48and; α6: SEQ ID NO. 58 or both SEQ ID NO. 59 and SEQ ID NO. 60, and both SEQ ID NO. 61 and SEQ ID NO. 62and; α7: all three SEQ ID NO. 73, SEQ ID NO. 75 and SEQ ID NO. 76 or all three EQ ID NO. 74, SEQ ID NO. 75 and SEQ ID NO. 76, and all five SEQ ID NO. 77, SEQ ID NO. 78, SEQ ID NO. 79, SEQ ID NO. 80 and SEQ ID NO. 81 and ; α10: all three SEQ ID NO. 115, SEQ ID NO. 116, SEQ ID NO. 117 and all four SEQ ID NO. 118, SEQ ID NO. 119, SEQ ID NO. 120, SEQ ID NO. 121; and α9: both SEQ ID NO. 100 and SEQ ID NO. 101 and all three SEQ ID NO. 102, SEQ ID NO. 103 and SEQ ID NO. 104; and α8: SEQ ID NO. 91, and both SEQ ID NO. 92 and SEQ ID NO. 93; and α1: SEQ ID NO. 1 and SEQ ID NO. 2; and α3: all three SEQ ID NO. 21, SEQ ID NO. 22 and SEQ ID NO. 23, and all four SEQ ID NO. 24, SEQ ID NO. 25, SEQ ID NO. 26 and SEQ ID NO. 27

And comprising for L1 α5: SEQ ID NO. 53 or both SEQ ID NO. 54 and SEQ ID NO. 55, and both SEQ ID NO. 56 and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114; and α8: SEQ ID NO. 97, and both SEQ ID NO. 98 and SEQ ID NO. 99; and α1: SEQ ID NO. 5 and SEQ ID NO. 6; and α3 SEQ ID NO. 33 and both SEQ ID NO. 34 and SEQ ID NO. 35.

Or, in this regard, the invention also contemplates a composition of primers comprising for E7 : α5: both SEQ ID NO. 49, SEQ ID NO. 50 and both SEQ ID NO. 51, SEQ ID NO. 52; and α6: SEQ ID NO. 63 or SEQ ID NO. 64 or SEQ ID NO. 65 or SEQ ID NO. 66 or both SEQ ID NO. 67 and SEQ ID NO. 68, and both SEQ ID NO. 69 and SEQ ID NO. 70; and α7: SEQ ID NO. 82 or both SEQ ID NO. 83, SEQ ID NO. 84, and both SEQ ID NO. 85, SEQ ID NO. 86; and α10: all three SEQ ID NO. 122, SEQ ID NO. 123 and SEQ ID NO. 124, and all three SEQ ID NO. 125, SEQ ID NO. 126 and SEQ ID NO. 127; and α9: all three SEQ ID NO. 105, SEQ ID NO. 106 and SEQ ID NO. 107, and all three SEQ ID NO. 108, SEQ ID NO. 109 and 110; and α8: SEQ ID NO. 94, and both SEQ ID NO. 95 and SEQ ID NO. 96; and α1: SEQ ID NO. 3 and SEQ ID NO. 4;and α3: both SEQ ID NO. 28 and SEQ ID NO. 29, and all three SEQ ID NO. 30, SEQ ID NO. 31, SEQ ID NO. 32,

And comprising for L1 α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114; and α8: SEQ ID NO. 97, and both SEQ ID NO. 98 and SEQ ID NO. 99; and α1: SEQ ID NO. 5 and SEQ ID NO. 6; and α3 SEQ ID NO. 33 and both SEQ ID NO. 34 and SEQ ID NO. 35.

In a sixth specific embodiment, the above defined invention is practiced to include at least the group consisting of papillomaviruses α6 comprising HPV 30, HPV 53, HPV 56 and HPV 66, papillomaviruses α7 comprising HPV 68, HPV 39, HPV 70, HPV 85, HPV 59, HPV 45, HPV 18, HPV 97, papillomaviruses α10 comprising HPV 16, HPV 35, HPV 31, HPV 52, HPV 67, HPV 33, HPV 58, papillomaviruses α5 comprising HPV 26, HPV, 69, HPV 51, HPV 82, papillomaviruses α9 comprising HPV 6, HPV 11, HPV 13, HPV 1, HPV 74, HPV 44, papillomaviruses α8 comprising HPV 91, HPV 43, HPV 7, HPV 40 papillomaviruses α1 comprising HPV 42, HPV 32, papillomavirus α3 comprising HPV 114, HPV 84, HPV 86, HPV87, HPV 102, HPV83, HPV89, HPV 61, HPV 72, HPV 62 and papillomavirus α2 comprising HPV 117, HPV 10, HPV 94, HPV 28, HPV125, HPV 3, HPV 78, HPV 160, HPV 29, HPV 77.

In this regard, the invention also contemplates a composition of primers comprising for E6: α5: both SEQ ID NO. 44 and SEQ ID NO. 45, and all three SEQ ID NO. 46, SEQ ID NO. 47 and SEQ ID NO. 48and; α6: SEQ ID NO. 58 or both SEQ ID NO. 59 and SEQ ID NO. 60, and both SEQ ID NO. 61 and SEQ ID NO. 62and; α7: all three SEQ ID NO. 73, SEQ ID NO. 75 and SEQ ID NO. 76 or all three EQ ID NO. 74, SEQ ID NO. 75 and SEQ ID NO. 76, and all five SEQ ID NO. 77, SEQ ID NO. 78, SEQ ID NO. 79, SEQ ID NO. 80 and SEQ ID NO. 81 and ; α10: all three SEQ ID NO. 115, SEQ ID NO. 116, SEQ ID NO. 117 and all four SEQ ID NO. 118, SEQ ID NO. 119, SEQ ID NO. 120, SEQ ID NO. 121; and α9: both SEQ ID NO. 100 and SEQ ID NO. 101 and all three SEQ ID NO. 102, SEQ ID NO. 103 and SEQ ID NO. 104; and α8: SEQ ID NO. 91, and both SEQ ID NO. 92 and SEQ ID NO. 93; and α1: SEQ ID NO. 1 and SEQ ID NO. 2; and α3: all three SEQ ID NO. 21, SEQ ID NO. 22 and SEQ ID NO. 23, and all four SEQ ID NO. 24, SEQ ID NO. 25, SEQ ID NO. 26 and SEQ ID NO. 27; and α2 and both SEQ ID NO. 7 and SEQ ID NO. 8, and all three SEQ ID NO. 9, SEQ ID NO. 10 and SEQ ID NO. 11

And comprising for L1 α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114; and α8: SEQ ID NO. 97, and both SEQ ID NO. 98 and SEQ ID NO. 99; and α1: SEQ ID NO. 5 and SEQ ID NO. 6; and α3 SEQ ID NO. 33 and both SEQ ID NO. 34 and SEQ ID NO. 35; and α2 SEQ ID NO. 18, and both SEQ ID NO. 19 and SEQ ID NO. 20.

Or, in this regard, the invention also contemplates a composition of primers comprising for E7: α5: both SEQ ID NO. 49, SEQ ID NO. 50 and both SEQ ID NO. 51, SEQ ID NO. 52; and α6: SEQ ID NO. 63 or SEQ ID NO. 64 or SEQ ID NO. 65 SEQ ID NO. 66 or both SEQ ID NO. 67 and SEQ ID NO. 68, and both SEQ ID NO. 69 and SEQ ID NO. 70; and α7: SEQ ID NO. 82 or both SEQ ID NO. 83, SEQ ID NO. 84, and both SEQ ID NO. 85, SEQ ID NO. 86; and α10: all three SEQ ID NO. 122, SEQ ID NO. 123 and SEQ ID NO. 124, and all three SEQ ID NO. 125, SEQ ID NO. 126 and SEQ ID NO. 127; and α9: all three SEQ ID NO. 105, SEQ ID NO. 106 and SEQ ID NO. 107, and all three SEQ ID NO. 108, SEQ ID NO. 109 and 110; and α8: SEQ ID NO. 94, and both SEQ ID NO. 95 and SEQ ID NO. 96; and α1: SEQ ID NO. 3 and SEQ ID NO. 4;and α3: both SEQ ID NO. 28 and SEQ ID NO. 29, and all three SEQ ID NO. 30, SEQ ID NO. 31, SEQ ID NO. 32; and α2: all three SEQ ID NO. 12, SEQ ID NO. 13 and SEQ ID NO. 14, and all three SEQ ID NO. 15, SEQ ID NO. 16 and SEQ ID NO. 17,

And comprising for L1 α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114; and α8: SEQ ID NO. 97, and both SEQ ID NO. 98 and SEQ ID NO. 99; and α1: SEQ ID NO. 5 and SEQ ID NO. 6; and α3 SEQ ID NO. 33 and both SEQ ID NO. 34 and SEQ ID NO. 35; and α2 SEQ ID NO. 18, and both SEQ ID NO. 19 and SEQ ID NO. 20.

In a seventh specific embodiment, the above defined invention is practiced to include at least the group consisting of papillomaviruses α6 comprising HPV 30, HPV 53, HPV 56 and HPV 66, papillomaviruses α7 comprising HPV 68, HPV 39, HPV 70, HPV 85, HPV 59, HPV 45, HPV 18, HPV 97 , papillomaviruses α10 comprising HPV 16, HPV 35, HPV 31, HPV 52, HPV 67, HPV 33, HPV 58, papillomaviruses α5 comprising HPV 26, HPV, 69, HPV 51, HPV 82, papillomaviruses α9 comprising HPV 6, HPV 11, HPV 13, HPV 1, HPV 74, HPV 44, papillomaviruses α8 comprising HPV 91, HPV 43, HPV 7, HPV 40 papillomaviruses α1 comprising HPV 42, HPV 32, papillomavirus α3 comprising HPV 114, HPV 84, HPV 86, HPV87, HPV 102, HPV83, HPV89, HPV 61, HPV 72, HPV 62, papillomavirus α2 comprising HPV 117, HPV 10, HPV 94, HPV 28, HPV125, HPV 3, HPV 78, HPV 160, HPV 29, HPV 77 and papillomaviruses α4 comprising HPV 2, HPV 27, HPV 57.

In this regard, the invention also contemplates a composition of primers comprising for E6: α5: both SEQ ID NO. 44 and SEQ ID NO. 45, and all three SEQ ID NO. 46, SEQ ID NO. 47 and SEQ ID NO. 48and; α6: SEQ ID NO. 58 or both SEQ ID NO. 59 and SEQ ID NO. 60, and both SEQ ID NO. 61 and SEQ ID NO. 62and; α7: all three SEQ ID NO. 73, SEQ ID NO. 75 and SEQ ID NO. 76 or all three EQ ID NO. 74, SEQ ID NO. 75 and SEQ ID NO. 76, and all five SEQ ID NO. 77, SEQ ID NO. 78, SEQ ID NO. 79, SEQ ID NO. 80 and SEQ ID NO. 81 and ; α10: all three SEQ ID NO. 115, SEQ ID NO. 116, SEQ ID NO. 117 and all four SEQ ID NO. 118, SEQ ID NO. 119, SEQ ID NO. 120, SEQ ID NO. 121; and α9: both SEQ ID NO. 100 and SEQ ID NO. 101 and all three SEQ ID NO. 102, SEQ ID NO. 103 and SEQ ID NO. 104; and α8: SEQ ID NO. 91, and both SEQ ID NO. 92 and SEQ ID NO. 93; and α1: SEQ ID NO. 1 and SEQ ID NO. 2; and α3: all three SEQ ID NO. 21, SEQ ID NO. 22 and SEQ ID NO. 23, and all four SEQ ID NO. 24, SEQ ID NO. 25, SEQ ID NO. 26 and SEQ ID NO. 27; and α2 and both SEQ ID NO. 7 and SEQ ID NO. 8, and all three SEQ ID NO. 9, SEQ ID NO. 10 and SEQ ID NO. 11; and α4: SEQ ID NO. 36, and SEQ ID NO. 37

And comprising for L1 α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114; and α8: SEQ ID NO. 97, and both SEQ ID NO. 98 and SEQ ID NO. 99; and α1: SEQ ID NO. 5 and SEQ ID NO. 6; and α3 SEQ ID NO. 33 and both SEQ ID NO. 34 and SEQ ID NO. 35; and α2 SEQ ID NO. 18, and both SEQ ID NO. 19 and SEQ ID NO. 20; and α4: SEQ ID NO. 40 or SEQ ID NO. 41, and SEQ ID NO. 42 or SEQ ID NO. 43.

Or, in this regard, the invention also contemplates a composition of primers comprising for E7 : α5: both SEQ ID NO. 49, SEQ ID NO. 50 and both SEQ ID NO. 51, SEQ ID NO. 52; and α6: SEQ ID NO. 63 or SEQ ID NO. 64 or SEQ ID NO. 65 SEQ ID NO. 66 or both SEQ ID NO. 67 and SEQ ID NO. 68, and both SEQ ID NO. 69 and SEQ ID NO. 70; and α7: SEQ ID NO. 82 or both SEQ ID NO. 83, SEQ ID NO. 84, and both SEQ ID NO. 85, SEQ ID NO. 86; and α10: all three SEQ ID NO. 122, SEQ ID NO. 123 and SEQ ID NO. 124, and all three SEQ ID NO. 125, SEQ ID NO. 126 and SEQ ID NO. 127; and α9: all three SEQ ID NO. 105, SEQ ID NO. 106 and SEQ ID NO. 107, and all three SEQ ID NO. 108, SEQ ID NO. 109 and 110; and α8: SEQ ID NO. 94, and both SEQ ID NO. 95 and SEQ ID NO. 96; and α1: SEQ ID NO. 3 and SEQ ID NO. 4;and α3: both SEQ ID NO. 28 and SEQ ID NO. 29, and all three SEQ ID NO. 30, SEQ ID NO. 31, SEQ ID NO. 32; and α2: all three SEQ ID NO. 12, SEQ ID NO. 13 and SEQ ID NO. 14, and all three SEQ ID NO. 15, SEQ ID NO. 16 and SEQ ID NO. 17;, and α4: SEQ ID NO. 38, and SEQ ID NO. 39

And comprising for L1 α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114; and α8: SEQ ID NO. 97, and both SEQ ID NO. 98 and SEQ ID NO. 99; and α1: SEQ ID NO. 5 and SEQ ID NO. 6; and α3 SEQ ID NO. 33 and both SEQ ID NO. 34 and SEQ ID NO. 35; and α2 SEQ ID NO. 18, and both SEQ ID NO. 19 and SEQ ID NO. 20; and α4: SEQ ID NO. 40 or SEQ ID NO. 41, and SEQ ID NO. 42 or SEQ ID NO. 43.

In an eighth specific embodiment, the above defined invention is practiced to include at least the group consisting of papillomaviruses α6 comprising HPV 30, HPV 53, HPV 56 and HPV 66, papillomaviruses α7 comprising HPV 68, HPV 39, HPV 70, HPV 85, HPV 59, HPV 45, HPV 18, HPV 97 , papillomaviruses α10 comprising HPV 16, HPV 35, HPV 31, HPV 52, HPV 67, HPV 33, HPV 58, papillomaviruses α5 comprising HPV 26, HPV, 69, HPV 51, HPV 82, papillomaviruses α9 comprising HPV 6, HPV 11, HPV 13, HPV 1, HPV 74, HPV 44, papillomaviruses α8 comprising HPV 91, HPV 43, HPV 7, HPV 40 papillomaviruses α1 comprising HPV 42, HPV 32, papillomavirus α3 comprising HPV 114, HPV 84, HPV 86, HPV87, HPV 102, HPV83, HPV89, HPV 61, HPV 72, HPV 62, papillomavirus α2 comprising HPV 117, HPV 10, HPV 94, HPV 28, HPV125, HPV 3, HPV 78, HPV 160, HPV 29, HPV 77, papillomaviruses α4 comprising HPV 2, HPV 27, HPV 57 and papillomaviruses α11 comprising HPV 73, HPV 34.

In this regard, the invention also contemplates a composition of primers comprising for E6: α5: both SEQ ID NO. 44 and SEQ ID NO. 45, and all three SEQ ID NO. 46, SEQ ID NO. 47 and SEQ ID NO. 48and; α6: SEQ ID NO. 58 or both SEQ ID NO. 59 and SEQ ID NO. 60, and both SEQ ID NO. 61 and SEQ ID NO. 62and; α7: all three SEQ ID NO. 73, SEQ ID NO. 75 and SEQ ID NO. 76 or all three EQ ID NO. 74, SEQ ID NO. 75 and SEQ ID NO. 76, and all five SEQ ID NO. 77, SEQ ID NO. 78, SEQ ID NO. 79, SEQ ID NO. 80 and SEQ ID NO. 81 and ; α10: all three SEQ ID NO. 115, SEQ ID NO. 116, SEQ ID NO. 117 and all four SEQ ID NO. 118, SEQ ID NO. 119, SEQ ID NO. 120, SEQ ID NO. 121; and α9: both SEQ ID NO. 100 and SEQ ID NO. 101 and all three SEQ ID NO. 102, SEQ ID NO. 103 and SEQ ID NO. 104; and α8: SEQ ID NO. 91, and both SEQ ID NO. 92 and SEQ ID NO. 93; and α1: SEQ ID NO. 1 and SEQ ID NO. 2; and α3: all three SEQ ID NO. 21, SEQ ID NO. 22 and SEQ ID NO. 23, and all four SEQ ID NO. 24, SEQ ID NO. 25, SEQ ID NO. 26 and SEQ ID NO. 27; and α2 and both SEQ ID NO. 7 and SEQ ID NO. 8, and all three SEQ ID NO. 9, SEQ ID NO. 10 and SEQ ID NO. 11; and α4: SEQ ID NO. 36, and SEQ ID NO. 37; and α11: SEQ ID NO. 135, and SEQ ID NO. 136,

And comprising for L1 α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114; and α8: SEQ ID NO. 97, and both SEQ ID NO. 98 and SEQ ID NO. 99; and α1: SEQ ID NO. 5 and SEQ ID NO. 6; and α3 SEQ ID NO. 33 and both SEQ ID NO. 34 and SEQ ID NO. 35; and α2 SEQ ID NO. 18, and both SEQ ID NO. 19 and SEQ ID NO. 20; and α4: SEQ ID NO. 40 or SEQ ID NO. 41, and SEQ ID NO. 42 or SEQ ID NO. 43; and α11: SEQ ID NO. 139, and SEQ ID NO. 140.

Or, in this regard, the invention also contemplates a composition of primers comprising for E7 : α5: both SEQ ID NO. 49, SEQ ID NO. 50 and both SEQ ID NO. 51, SEQ ID NO. 52; and α6: SEQ ID NO. 63 or SEQ ID NO. 64 or SEQ ID NO. 65 SEQ ID NO. 66 or both SEQ ID NO. 67 and SEQ ID NO. 68, and both SEQ ID NO. 69 and SEQ ID NO. 70; and α7: SEQ ID NO. 82 or both SEQ ID NO. 83, SEQ ID NO. 84, and both SEQ ID NO. 85, SEQ ID NO. 86; and α10: all three SEQ ID NO. 122, SEQ ID NO. 123 and SEQ ID NO. 124, and all three SEQ ID NO. 125, SEQ ID NO. 126 and SEQ ID NO. 127; and α9: all three SEQ ID NO. 105, SEQ ID NO. 106 and SEQ ID NO. 107, and all three SEQ ID NO. 108, SEQ ID NO. 109 and 110; and α8: SEQ ID NO. 94, and both SEQ ID NO. 95 and SEQ ID NO. 96; and α1: SEQ ID NO. 3 and SEQ ID NO. 4;and α3: both SEQ ID NO. 28 and SEQ ID NO. 29, and all three SEQ ID NO. 30, SEQ ID NO. 31, SEQ ID NO. 32; and α2: all three SEQ ID NO. 12, SEQ ID NO. 13 and SEQ ID NO. 14, and all three SEQ ID NO. 15, SEQ ID NO. 16 and SEQ ID NO. 17; and α4: SEQ ID NO. 38, and SEQ ID NO. 39; and α11 SEQ ID NO. 137, and SEQ ID NO. 138,

And comprising for L1 α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114; and α8: SEQ ID NO. 97, and both SEQ ID NO. 98 and SEQ ID NO. 99; and α1: SEQ ID NO. 5 and SEQ ID NO. 6; and α3 SEQ ID NO. 33 and both SEQ ID NO. 34 and SEQ ID NO. 35; and α2 SEQ ID NO. 18, and both SEQ ID NO. 19 and SEQ ID NO. 20; and α4: SEQ ID NO. 40 or SEQ ID NO. 41, and SEQ ID NO. 42 or SEQ ID NO. 43; and α11: SEQ ID NO. 139, and SEQ ID NO. 140.

In a ninth specific embodiment, the above defined invention is practiced to include at least the group consisting of papillomaviruses α6 comprising HPV 30, HPV 53, HPV 56 and HPV 66, papillomaviruses α7 comprising HPV 68, HPV 39, HPV 70, HPV 85, HPV 59, HPV 45, HPV 18, HPV 97 , papillomaviruses α10 comprising HPV 16, HPV 35, HPV 31, HPV 52, HPV 67, HPV 33, HPV 58, papillomaviruses α5 comprising HPV 26, HPV, 69, HPV 51, HPV 82, papillomaviruses α9 comprising HPV 6, HPV 11, HPV 13, HPV 1, HPV 74, HPV 44, papillomaviruses α8 comprising HPV 91, HPV 43, HPV 7, HPV 40, papillomaviruses α1 comprising HPV 42, HPV 32, papillomavirus α3 comprising HPV 114, HPV 84, HPV 86, HPV87, HPV 102, HPV83, HPV89, HPV 61, HPV 72, HPV 62, papillomavirus α2 comprising HPV 117, HPV 10, HPV 94, HPV 28, HPV125, HPV 3, HPV 78, HPV 160, HPV 29, HPV 77, papillomaviruses α4 comprising HPV 2, HPV 27, HPV 57, papillomaviruses α11 comprising HPV 73, HPV 34 and papillomaviruses α13 comprising HPV 54.

In this regard, the invention also contemplates a composition of primers comprising for E6: α5: both SEQ ID NO. 44 and SEQ ID NO. 45, and all three SEQ ID NO. 46, SEQ ID NO. 47 and SEQ ID NO. 48and; α6: SEQ ID NO. 58 or both SEQ ID NO. 59 and SEQ ID NO. 60, and both SEQ ID NO. 61 and SEQ ID NO. 62and; α7: all three SEQ ID NO. 73, SEQ ID NO. 75 and SEQ ID NO. 76 or all three EQ ID NO. 74, SEQ ID NO. 75 and SEQ ID NO. 76, and all five SEQ ID NO. 77, SEQ ID NO. 78, SEQ ID NO. 79, SEQ ID NO. 80 and SEQ ID NO. 81 and ; α10: all three SEQ ID NO. 115, SEQ ID NO. 116, SEQ ID NO. 117 and all four SEQ ID NO. 118, SEQ ID NO. 119, SEQ ID NO. 120, SEQ ID NO. 121; and α9: both SEQ ID NO. 100 and SEQ ID NO. 101 and all three SEQ ID NO. 102, SEQ ID NO. 103 and SEQ ID NO. 104; and α8: SEQ ID NO. 91, and both SEQ ID NO. 92 and SEQ ID NO. 93; and α1: SEQ ID NO. 1 and SEQ ID NO. 2; and α3: all three SEQ ID NO. 21, SEQ ID NO. 22 and SEQ ID NO. 23, and all four SEQ ID NO. 24, SEQ ID NO. 25, SEQ ID NO. 26 and SEQ ID NO. 27; and α2 and both SEQ ID NO. 7 and SEQ ID NO. 8, and all three SEQ ID NO. 9, SEQ ID NO. 10 and SEQ ID NO. 11; and α4: SEQ ID NO. 36, and SEQ ID NO. 37; and α11: SEQ ID NO. 135, and SEQ ID NO. 136; and α13 SEQ ID NO. 141, and SEQ ID NO. 142,

And comprising for L1 α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114; and α8: SEQ ID NO. 97, and both SEQ ID NO. 98 and SEQ ID NO. 99; and α1: SEQ ID NO. 5 and SEQ ID NO. 6; and α3 SEQ ID NO. 33 and both SEQ ID NO. 34 and SEQ ID NO. 35; and α2 SEQ ID NO. 18, and both SEQ ID NO. 19 and SEQ ID NO. 20; and α4: SEQ ID NO. 40 or SEQ ID NO. 41, and SEQ ID NO. 42 or SEQ ID NO. 43; and α11: SEQ ID NO. 139, and SEQ ID NO. 140; and α13 SEQ ID NO. 145, and SEQ ID NO. 146.

Or, in this regard, the invention also contemplates a composition of primers comprising for E7 : α5: both SEQ ID NO. 49, SEQ ID NO. 50 and both SEQ ID NO. 51, SEQ ID NO. 52; and α6: SEQ ID NO. 63 or SEQ ID NO. 64 or SEQ ID NO. 65 SEQ ID NO. 66 or both SEQ ID NO. 67 and SEQ ID NO. 68, and both SEQ ID NO. 69 and SEQ ID NO. 70; and α7: SEQ ID NO. 82 or both SEQ ID NO. 83, SEQ ID NO. 84, and both SEQ ID NO. 85, SEQ ID NO. 86; and α10: all three SEQ ID NO. 122, SEQ ID NO. 123 and SEQ ID NO. 124, and all three SEQ ID NO. 125, SEQ ID NO. 126 and SEQ ID NO. 127; and α9: all three SEQ ID NO. 105, SEQ ID NO. 106 and SEQ ID NO. 107, and all three SEQ ID NO. 108, SEQ ID NO. 109 and 110; and α8: SEQ ID NO. 94, and both SEQ ID NO. 95 and SEQ ID NO. 96; and α1: SEQ ID NO. 3 and SEQ ID NO. 4;and α3: both SEQ ID NO. 28 and SEQ ID NO. 29, and all three SEQ ID NO. 30, SEQ ID NO. 31, SEQ ID NO. 32; and α2: all three SEQ ID NO. 12, SEQ ID NO. 13 and SEQ ID NO. 14, and all three SEQ ID NO. 15, SEQ ID NO. 16 and SEQ ID NO. 17;, and α4: SEQ ID NO. 38, and SEQ ID NO. 39; and α11 SEQ ID NO. 137, and SEQ ID NO. 138; and α13: SEQ ID NO. 143, and SEQ ID NO. 144,

And comprising for L1 α5: SEQ ID NO. 53 or both SEQ ID NO. 54 and SEQ ID NO. 55, and both SEQ ID NO. 56and SEQ ID NO. 57; and α6 : SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114; and α8: SEQ ID NO. 97, and both SEQ ID NO. 98 and SEQ ID NO. 99; and α1: SEQ ID NO. 5 and SEQ ID NO. 6; and α3 SEQ ID NO. 33 and both SEQ ID NO. 34 and SEQ ID NO. 35; and α2 SEQ ID NO. 18, and both SEQ ID NO. 19 and SEQ ID NO. 20; and α4: SEQ ID NO. 40 or SEQ ID NO. 41, and SEQ ID NO. 42 or SEQ ID NO. 43; and α11: SEQ ID NO. 139, and SEQ ID NO. 140; and α13 SEQ ID NO. 145, and SEQ ID NO. 146.

In a tenth specific embodiment, the above defined invention is practiced to include at least the group consisting of papillomaviruses α6 comprising HPV 30, HPV 53, HPV 56 and HPV 66, papillomaviruses α7 comprising HPV 68, HPV 39, HPV 70, HPV 85, HPV 59, HPV 45, HPV 18, HPV 97, papillomaviruses α10 comprising HPV 16, HPV 35, HPV 31, HPV 52, HPV 67, HPV 33, HPV 58, papillomaviruses α5 comprising HPV 26, HPV, 69, HPV 51, HPV 82, papillomaviruses α9 comprising HPV 6, HPV 11, HPV 13, HPV 1, HPV 74, HPV 44, papillomaviruses α8 comprising HPV 91, HPV 43, HPV 7, HPV 40, papillomaviruses α1 comprising HPV 42, HPV 32, papillomavirus α3 comprising HPV 114, HPV 84, HPV 86, HPV87, HPV 102, HPV83, HPV89, HPV 61, HPV 72, HPV 62, papillomavirus α2 comprising HPV 117, HPV 10, HPV 94, HPV 28, HPV125, HPV 3, HPV 78, HPV 160, HPV 29, HPV 77, papillomaviruses α4 comprising HPV 2, HPV 27, HPV 57, papillomaviruses α11 comprising HPV 73, HPV 34, papillomaviruses α13 comprising HPV 54 and papillomaviruses α14 comprising HPV 106, HPV 90, HPV 71.

In this regard, the invention also contemplates a composition of primers comprising for E6: α5: both SEQ ID NO. 44 and SEQ ID NO. 45, and all three SEQ ID NO. 46, SEQ ID NO. 47 and SEQ ID NO. 48and; α6: SEQ ID NO. 58 or both SEQ ID NO. 59 and SEQ ID NO. 60, and both SEQ ID NO. 61 and SEQ ID NO. 62and; α7: all three SEQ ID NO. 73, SEQ ID NO. 75 and SEQ ID NO. 76 or all three EQ ID NO. 74, SEQ ID NO. 75 and SEQ ID NO. 76, and all five SEQ ID NO. 77, SEQ ID NO. 78, SEQ ID NO. 79, SEQ ID NO. 80 and SEQ ID NO. 81 and ; α10: all three SEQ ID NO. 115, SEQ ID NO. 116, SEQ ID NO. 117 and all four SEQ ID NO. 118, SEQ ID NO. 119, SEQ ID NO. 120, SEQ ID NO. 121; and α9: both SEQ ID NO. 100 and SEQ ID NO. 101 and all three SEQ ID NO. 102, SEQ ID NO. 103 and SEQ ID NO. 104; and α8: SEQ ID NO. 91, and both SEQ ID NO. 92 and SEQ ID NO. 93; and α1: SEQ ID NO. 1 and SEQ ID NO. 2; and α3: all three SEQ ID NO. 21, SEQ ID NO. 22 and SEQ ID NO. 23, and all four SEQ ID NO. 24, SEQ ID NO. 25, SEQ ID NO. 26 and SEQ ID NO. 27; and α2 and both SEQ ID NO. 7 and SEQ ID NO. 8, and all three SEQ ID NO. 9, SEQ ID NO. 10 and SEQ ID NO. 11; and α4: SEQ ID NO. 36, and SEQ ID NO. 37; and α11: SEQ ID NO. 135, and SEQ ID NO. 136; and α13 SEQ ID NO. 141, and SEQ ID NO. 142; and α14: SEQ ID NO. 147, and SEQ ID NO. 148,

And comprising for L1, α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114; and α8: SEQ ID NO. 97, and both SEQ ID NO. 98 and SEQ ID NO. 99; and α1: SEQ ID NO. 5 and SEQ ID NO. 6; and α3 SEQ ID NO. 33 and both SEQ ID NO. 34 and SEQ ID NO. 35; and α2 SEQ ID NO. 18, and both SEQ ID NO. 19 and SEQ ID NO. 20; and α4: SEQ ID NO. 40 or SEQ ID NO. 41, and SEQ ID NO. 42 or SEQ ID NO. 43; and α11: SEQ ID NO. 139, and SEQ ID NO. 140; and α13 SEQ ID NO. 145, and SEQ ID NO. 146; and α14: SEQ ID NO. 151 and SEQ ID NO. 152.

Or, in this regard, the invention also contemplates a composition of primers comprising for E7: α5: both SEQ ID NO. 49, SEQ ID NO. 50 and both SEQ ID NO. 51, SEQ ID NO. 52; and α6: SEQ ID NO. 63 or SEQ ID NO. 64 or SEQ ID NO. 65 SEQ ID NO. 66 or both SEQ ID NO. 67 and SEQ ID NO. 68, and both SEQ ID NO. 69 and SEQ ID NO.

70; and α7: SEQ ID NO. 82 or both SEQ ID NO. 83, SEQ ID NO. 84, and both SEQ ID NO. 85, SEQ ID NO. 86; and α10: all three SEQ ID NO. 122, SEQ ID NO. 123 and SEQ ID NO. 124, and all three SEQ ID NO. 125, SEQ ID NO. 126 and SEQ ID NO. 127; and α9: all three SEQ ID NO. 105, SEQ ID NO. 106 and SEQ ID NO. 107, and all three SEQ ID NO. 108, SEQ ID NO. 109 and 110; and α8: SEQ ID NO. 94, and both SEQ ID NO. 95 and SEQ ID NO. 96; and α1: SEQ ID NO. 3 and SEQ ID NO. 4;and α3: both SEQ ID NO. 28 and SEQ ID NO. 29, and all three SEQ ID NO. 30, SEQ ID NO. 31, SEQ ID NO. 32; and α2: all three SEQ ID NO. 12, SEQ ID NO. 13 and SEQ ID NO. 14, and all three SEQ ID NO. 15, SEQ ID NO. 16 and SEQ ID NO. 17;, and α4: SEQ ID NO. 38, and SEQ ID NO. 39; and α11 SEQ ID NO. 137, and SEQ ID NO. 138; and α13: SEQ ID NO. 143, and SEQ ID NO. 144; and α14: SEQ ID NO. 149,and SEQ ID NO. 150 And comprising for L1 α5: SEQ ID NO. 53 or both SEQ ID NO. 54 and SEQ ID NO. 55, and both SEQ ID NO. 56and SEQ ID NO. 57; and α6: SEQ ID NO. 71 and SEQ ID NO. 72; and α7: both SEQ ID NO. 87 and SEQ ID NO. 88, and both SEQ ID NO. 89and SEQ ID NO. 90; and α10: both SEQ ID NO. 128, SEQ ID NO. 129 and SEQ ID NO. 130 or all four SEQ ID NO. 131, SEQ ID NO. 132, SEQ ID NO. 133, SEQ ID NO. 134; and α9: both SEQ ID NO. 111 and SEQ ID NO. 112, and both SEQ ID NO. 113 and SEQ ID NO. 114; and α8: SEQ ID NO. 97, and both SEQ ID NO. 98 and SEQ ID NO. 99; and α1: SEQ ID NO. 5 and SEQ ID NO. 6; and α3 SEQ ID NO. 33 and both SEQ ID NO. 34 and SEQ ID NO. 35; and α2 SEQ ID NO. 18, and both SEQ ID NO. 19 and SEQ ID NO. 20; and α4: SEQ ID NO. 40 or SEQ ID NO. 41, and SEQ ID NO. 42 or SEQ ID NO. 43; and α11: SEQ ID NO. 139, and SEQ ID NO. 140; and α13 SEQ ID NO. 145, and SEQ ID NO. 146; and α14: SEQ ID NO. 151 and SEQ ID NO. 152.

It is disclosed herein the use of composition of primers describe above for diagnosis or prognosis of risk to develop HPV induced cancer in a human subject.

Said primers can further comprise at least one of:

- a functional group for covalent coupling at the 5' or 3' end, such as a terminal group comprising a thiol, amine or carboxyl group,
- a spacer molecule or sequence at the 5' or 3' end,
- additional sequences as index or tag sequences to perform pre or post additional and general amplification steps not depending on the target sequences to be quantified.

It is disclosed herein a composition of primers comprising at least one primer selected from SEQ ID No 153 to 158. Said composition can comprise 1, 2, 3, 4, 5 or the 6 primers selected from SEQ ID No 153 to 158.

In another embodiment, the present invention relates to a kit for diagnosis or prognosis risk to develop HPV induced cancer according to claim 14 or 15.

It is disclosed herein a kit for diagnosis or prognosis risk to develop HPV induced cancer comprising:

a) a composition of primers,
b) reagents to detect amplification products.

In a specific embodiment, the present invention relates to a kit for diagnosis or prognosis risk to develop HPV induced cancer comprising :

a) primers or probes for detecting at least a first marker selected from E6 mRNAs of group alpha HPVs, E7 mRNAs of group alpha HPVs, or both,
b) primers or probes for detecting at least a second marker selected from L1 mRNAs of group alpha HPVs, L2 mRNAs of group alpha HPVs, or both,
wherein said E6, E7, L1 and L2 mRNAs have corresponding intragenetic sequences,
c) and optionally, primers or probes for detecting at least one host cellular marker indicative of neoplasia or cancer.

In various embodiments, the primers can be selected from primers comprising or consisting of the nucleic acid sequence of any of SEQ ID NOs: 1-152. Preferably the primers comprise or consist of at least 15, 16, 17, 18, 19, 20, 21, 22, 23, 24, 25, 26, 27, 28, 29, or 30 consecutive nucleotides of any of SEQ ID NOs:1-152. The kit can contain any of the compositions of primers described herein.

The kit can further contain at least 1, 2, 3, 4, or more controls for the determination of R. The controls can contain a known ratio of E6 and/or E7 to L1 and/or L2. Preferably, the controls contain a known ratio of E6 and E7 to L1 and L2.

In various embodiments, the kit contains at least 1 or at least 2 controls indicating a low risk or non-persistent HPV infection. In various embodiments, the kit contains at least 1 or at least 2 controls indicative of a high risk infection or associated with higher risk of developing genital neoplasia and cancer. In preferred embodiments, the kit contains at least 1 or at least 2 controls indicating a low risk or non-persistent HPV infection and at least 1 or at least 2 controls indicating a high risk infection or associated with higher risk of developing genital neoplasia and cancer.

In various embodiments, the invention encompasses a method for assessing a human papilloma virus (HPV) infected patient. In one embodiment, the method comprises generating cDNA from a patient sample comprising RNA and sequencing the cDNA to generate reads of sequence of the cDNA.

In various embodiments, the number of reads is at least $10^6$, $5x10^6$, $10^7$, $2x10^7$, or $5x10^7$ reads.

In one embodiment, the cDNA is generated using random primers. In one embodiment, the cDNA is generated using HPV-specific primers. In preferred embodiments, at least one of the primers comprises or consists of the nucleic acid sequences in Table 3.

In various embodiments, the method comprises discriminating HPV sequence reads on the basis of HPV species including any of the specific species referenced herein. In various embodiments, the method comprises discriminating HPV sequence reads on the basis of HPV gene transcript, including E1, E2, E4, E5, E6, E7, E8, L1, and L2 transcripts. The transcripts can be spliced transcripts.

In various embodiments, the cDNA or the sequencing can be performed with HPV-specific or random primers, preferably HPV-specific primers.

In various embodiments, the primers comprise or consist of any of the nucleic acid sequences of SEQ ID NOs: 1-158. In various embodiments, the cDNA is generated with HPV-specific primers and the sequencing performed randomly or specifically for HPV sequences.

In various embodiments, the cDNA is generated with random primers and the sequencing performed randomly or specifically for HPV sequences.

According to a preferred embodiment, the method comprises:

a) enrichment of the viral RNAs, preferably HPV16 RNAs, in a sample,
b) random reverse transcription reaction, advantageously performed with random hexamers,
c) amplification of the cDNA produced in step a), advantageously performed by multiplex PCR with HPV-specific primers (to generate a DNA sequence library),
d) high throughput sequencing of the DNA library produced in step c) and generating reads of said cDNA,
e) determining the number of reads matching said viruses based on species discrimination and determining the most prevalent high risk species present in the sample relative to other species,
f) determining within said most prevalent high risk species the relative number of reads matching at least one oncogenic gene compared to at least one non oncogenic genes, preferably oncogenic genes compared to non oncogenic genes,
g) computing ratios within said high risk species of reads matching at least one oncogenic gene versus at least one versus corresponding at least one interspecies structural or regulatory gene, preferably oncogenic genes versus corresponding interspecies structural or regulatory genes,
h) determining risk of developing oncogenic virus induced cancer in patients in which said ratio tend towards infinity.

In advantageous embodiments, the HPV-specific primers comprise at least one of, preferably all, the following groups of pairs of primers:

- the HPV16-specific primers comprising or consisting of the primers of SEQ ID NOs: 219-258 for HPV16 genomic and unspliced transcripts, SEQ ID NOs: 259-352 for HPV16 spliced transcripts and SEQ ID NOs: 353-376 for HPV16-human fusion transcripts (including the pairs of primers of SEQ ID NO: 219-220; 221-222; 223-224; 225-226; 227-228; 229-230; 231-232; 233-234; 235-236; 237-238; 239-240; 241-242; 243-244; 245-246; 247-248; 249-250; 251-252; 253-254; 255-256; 257-258; 259-260; 261-262; 263-264; 265-266; 267-268; 269-270; 271-272; 273-274; 275-276; 277-278; 279-280; 281-282; 283-284; 285-286; 287-288; 289-290; 291-292; 293-294; 295-296; 297-298; 299-300; 301-302; 303-304; 305-306; 307-308; 309-310; 311-312; 313-314; 315-316; 317-318; 319-320; 321-322; 323-324; 325-326; 327-328; 329-330; 331-332; 333-334; 335-336; 337-338; 339-340; 341-342; 343-344; 345-346; 347-348; 349-350; 351-352; 353-354; 355-356; 357-358; 359-360; 361-362; 363-364; 365-366; 367-368; 369-370; 371-372; 373-374; 375-376) or 377-470 (including the pairs of primers of SEQ ID NO. 377-378; 379-380; 381-382; 383-384; 385- 386; 387-388; 389-390; 391-392; 393-394; 395-396; 397-398; 399-400; 401-402; 403-404; 405-406; 407-408; 409-410; 411-412; 413-414; 415-416; 417-418; 419-420; 421-422; 423-424; 425-426; 427-428; 429-430; 431-432; 433-434; 435-436; 437-438; 439-440; 441-442; 443-444; 445-446; 447-448; 449-450; 451-452; 453-454; 455-456; 457-458; 459-460; 461-462; 463-464; 465-466; 467-468 and; 469-470); and/or,

- the HPV18-specific primers comprising or consisting of the primers of SEQ ID NO. 471-574 (including the pairs of primers of SEQ ID NO.: 471-472; 473-474; 475-476; 477-478; 479-480; 481-482; 483-484; 485-486; 487-488; 489-490; 491-492; 493-494; 495-496; 497-498; 499-500; 501-502; 503-504; 505-506; 507-508; 509-510; 511-512; 513-514; 515-516; 517-518; 519-520; 521-522; 523-524; 525-526; 527-528; 529-530; 531-532; 533-534; 535-536; 537-538; 539-540; 541-542; 543-544; 545-546; 547-548; 549-550; 551-552; 553-554; 555-556; 557-558; 559-560;

561-562; 563-564; 565-566; 567-568; 569-570; 571-572; 573-574); and/or,

- the HPV31-specific primers comprising or consisting of the primers of SEQ ID NO. 575-668 (including the pairs of primers of SEQ ID NO.: 575-576; 577-578; 579-580; 581-582; 583-584; 585-586; 587-588; 589-590; 591-592; 593-594; 595-596; 597-598; 599-600; 601-602; 603-604; 605-606; 607-608; 609-610; 611-612; 613-614; 615-616; 617-618; 619-620; 621-622; 623-624; 625-626; 627-628; 629-630; 631-632; 633-634; 635-636; 637-638; 639-640; 641-642; 643-644; 645-646; 647-648; 649-650; 651-652; 653-654; 655-656; 657-658; 659-660; 661-662; 663-664; 665-666; 667-668); and/or,

- the HPV33-specific primers comprising or consisting of SEQ ID NO. 669-756 (including the pairs of primers of SEQ ID NO.: 669-670; 671-672; 673-674; 675-676; 677-678; 679-680; 681-682; 683-684; 685-686; 687-688; 689-690; 691-692; 693-694; 695-696; 697-698; 699-700; 701-702; 703-704; 705-706; 707-708; 709-710; 711-712; 713-714; 715-716; 717-718; 719-720; 721-722; 723-724; 725-726; 727-728; 729-730; 731-732; 733-734; 735-736; 737-738; 739-740; 741-742; 743-744; 745-746; 747-748; 749-750; 751-752; 753-754; 755-756); and/or,

- the HPV35-specific primers comprising or consisting of the primers of SEQ ID NO. 757-848 (including the pairs of primers of SEQ ID NO.: 757-758; 759-760; 761-762; 763-764; 765-766; 767-768; 769-770; 771-772; 773-774; 775-776; 777-778; 779-780; 781-782; 783-784; 785-786; 787-788; 789-790; 791-792; 793-794; 795-796; 797-798; 799-800; 801-802; 803-804; 805-806; 807-808; 809-810; 811-812; 813-814; 815-816; 817-818; 819-820; 821-822; 823-824; 825-826; 827-828; 829-830; 831-832; 833-834; 835-836; 837-838; 839-840; 841-842; 843-844; 845-846; 847-848); and/or,

- the HPV39-specific primers comprising or consisting of the primers of SEQ ID NO. 849-928 (including the pairs of primers of SEQ ID NO.: 849-850; 851-852; 853-854; 855-856; 857-858; 859-860; 861-862; 863-864; 865-866; 867-868; 869-870; 871-872; 873-874; 875-876; 877-878; 879-880; 881-882; 883-884; 885-886; 887-888; 889-890; 891-892; 893-894; 895-896; 897-898; 899-900; 901-902; 903-904; 905-906; 907-908; 909-910; 911-912; 913-914; 915-916; 917-918; 919-920; 921-922; 923-924; 925-926; 927-928); and/or,

- the HPV45-specific primers comprising or consisting of the primers of SEQ ID NO. 929-1020 (including the pairs of primers of SEQ ID NO.: 929-930; 931-932; 933-934; 935-936; 937-938; 939-940; 941-942; 943-944; 945-946; 947-948; 949-950; 951-952; 953-954; 955-956; 957-958; 959-960; 961-962; 963-964; 965-966; 967-968; 969-970; 971-972; 973-974; 975-976; 977-978; 979-980; 981-982; 983-984; 985-986; 987-988; 989-990; 991-992; 993-994; 995-996; 997-998; 999-1000; 1001-1002; 1003-1004; 1005-1006; 1007-1008; 1009-1010; 1011-1012; 1013-1014; 1015-1016; 1017-1018; 1019-1020); and/or,

- the HPV51-specific primers comprising or consisting of the primers of SEQ ID NO. 1021-1102 (including the pairs of primers of SEQ ID NO.: 1021-1022; 1023-1024; 1025-1026; 1027-1028; 1029-1030; 1031-1032; 1033-1034; 1035-1036; 1037-1038; 1039-1040; 1041-1042; 1043-1044; 1045-1046; 1047-1048; 1049-1050; 1051-1052; 1053-1054; 1055-1056; 1057-1058; 1059-1060; 1061-1062; 1063-1064; 1065-1066; 1067-1068; 1069-1070; 1071-1072; 1073-1074; 1075-1076; 1077-1078; 1079-1080; 1081-1082; 1083-1084; 1085-1086; 1087-1088; 1089-1090; 1091-1092; 1093-1094; 1095-1096; 1097-1098; 1099-1100; 1101-1102); and/or,

- the HPV52-specific primers comprising or consisting of the primers of SEQ ID NO. 1103-1200 (including the pairs of primers of SEQ ID NO.: 1103-1104; 1105-1106; 1107-1108; 1109-1110; 1111-1112; 1113-1114; 1115-1116; 1117-1118; 1119-1120; 1121-1122; 1123-1124; 1125-1126; 1127-1128; 1129-1130; 1131-1132; 1133-1134; 1135-1136; 1137-1138; 1139-1140; 1141-1142; 1143-1144; 1145-1146; 1147-1148; 1149-1150; 1151-1152; 1153-1154; 1155-1156; 1157-1158; 1159-1160; 1161-1162; 1163-1164; 1165-1166; 1167-1168; 1169-1170; 1171-1172; 1173-1174; 1175-1176; 1177-1178; 1179-1180; 1181-1182; 1183-1184; 1185-1186; 1187-1188; 1189-1190; 1191-1192; 1193-1194; 1195-1196; 1197-1198; 1199-1200); and/or,

- the HPV56-specific primers comprising or consisting of the primers of SEQ ID NO. 1201-1296 (including the pairs of primers of SEQ ID NO.: 1201-1202; 1203-1204; 1205-1206; 1207-1208; 1209-1210; 1211-1212; 1213-1214; 1215-1216; 1217-1218; 1219-1220; 1221-1222; 1223-1224; 1225-1226; 1227-1228; 1229-1230; 1231-1232; 1233-1234; 1235-1236; 1237-1238; 1239-1240; 1241-1242; 1243-1244; 1245-1246; 1247-1248; 1249-1250; 1251-1252; 1253-1254; 1255-1256; 1257-1258; 1259-1260; 1261-1262; 1263-1264; 1265-1266; 1267-1268; 1269-1270; 1271-1272; 1273-1274; 1275-1276; 1277-1278; 1279-1280; 1281-1282; 1283-1284; 1285-1286; 1287-1288; 1289-1290; 1291-1292; 1293-1294; 1295-1296); and/or,

- the HPV58-specific primers comprising or consisting of the primers of SEQ ID NO. 1297-1382 (including the pairs of primers of SEQ ID NO.: 1297-1298; 1299-1300; 1301-1302; 1303-1304; 1305-1306; 1307-1308; 1309-1310; 1311-1312; 1313-1314; 1315-1316; 1317-1318; 1319-1320; 1321-1322; 1323-1324; 1325-1326; 1327-1328; 1329-1330; 1331-1332; 1333-1334; 1335-1336; 1337-1338; 1339-1340; 1341-1342; 1343-1344; 1345-1346; 1347-1348; 1349-1350; 1351-1352; 1353-1354; 1355-1356; 1357-1358; 1359-1360; 1361-1362; 1363-1364; 1365-1366; 1367-1368; 1369-1370; 1371-1372; 1373-1374; 1375-1376; 1377-1378; 1379-1380; 1381-1382); and/or,

- the HPV59-specific primers comprising or consisting of the primers of SEQ ID NO. 1383-1470 (including the pairs of primers of SEQ ID NO.: 1383-1384; 1385-1386; 1387-1388; 1389-1390; 1391-1392; 1393-1394; 1395-1396; 1397-1398; 1399-1400; 1401-1402; 1403-1404; 1405-1406; 1407-1408; 1409-1410; 1411-1412; 1413-1414; 1415-1416; 1417-1418; 1419-1420; 1421-1422; 1423-1424; 1425-1426; 1427-1428; 1429-1430; 1431-1432; 1433-1434; 1435-1436; 1437-1438; 1439-1440; 1441-1442; 1443-1444; 1445-1446; 1447-1448; 1449-1450; 1451-1452; 1453-1454; 1455-1456; 1457-1458; 1459-1460; 1461-1462; 1463-1464; 1465-1466; 1467-1468; 1469-1470); and/or,

- the HPV66-specific primers comprising or consisting of the primers of SEQ ID NO. 1471-1560 (including the pairs of primers of SEQ ID NO.: 1471-1472; 1473-1474; 1475-1476; 1477-1478; 1479-1480; 1481-1482; 1483-1484; 1485-1486; 1487-1488; 1489-1490; 1491-1492; 1493-1494; 1495-1496; 1497-1498; 1499-1500; 1501-1502; 1503-1504; 1505-1506; 1507-1508; 1509-1510; 1511-1512; 1513-1514; 1515-1516; 1517-1518; 1519-1520; 1521-1522; 1523-1524; 1525-1526; 1527-1528; 1529-1530; 1531-1532; 1533-1534; 1535-1536; 1537-1538; 1539-1540; 1541-1542; 1543-1544; 1545-1546; 1547-1548; 1549-1550; 1551-1552; 1553-1554; 1555-1556; 1557-1558; 1559-1560; and/or,

- the HPV68-specific primers comprising or consisting of the primers of SEQ ID NO. 1561-1642 (including the pairs of primers of SEQ ID NO.: 1561-1562; 1563-1564; 1565-1566; 1567-1568; 1569-1570; 1571-1572; 1573-1574; 1575-1576; 1577-1578; 1579-1580; 1581-1582; 1583-1584; 1585-1586; 1587-1588; 1589-1590; 1591-1592; 1593-1594; 1595-1596; 1597-1598; 1599-1600; 1601-1602; 1603-1604; 1605-1606; 1607-1608; 1609-1610; 1611-1612; 1613-1614; 1615-1616; 1617-1618; 1619-1620; 1621-1622; 1623-1624; 1625-1626; 1627-1628; 1629-1630; 1631-1632; 1633-1634; 1635-1636; 1637-1638; 1639-1640; 1641-1642); and/or,

- the HPV73-specific primers comprising or consisting of the primers of SEQ ID NO. 1643-1732 (including the pairs of primers of SEQ ID NO.: 1643-1644; 1645-1646; 1647-1648; 1649-1650; 1651-1652; 1653-1654; 1655-1656; 1657-1658; 1659-1660; 1661-1662; 1663-1664; 1665-1666; 1667-1668; 1669-1670; 1671-1672; 1673-1674; 1675-1676; 1677-1678; 1679-1680; 1681-1682; 1683-1684; 1685-1686; 1687-1688; 1689-1690; 1691-1692; 1693-1694; 1695-1696; 1697-1698; 1699-1700; 1701-1702; 1703-1704; 1705-1706; 1707-1708; 1709-1710; 1711-1712; 1713-1714; 1715-1716; 1717-1718; 1719-1720; 1721-1722; 1723-1724; 1725-1726; 1727-1728; 1729-1730; 1731-1732); and/or,

- the HPV82-specific primers comprising or consisting of the primers of SEQ ID NO. 1733-1816 (including the pairs of primers of SEQ ID NO.: 1733-1734; 1735-1736; 1737-1738; 1739-1740; 1741-1742; 1743-1744; 1745-1746; 1747-1748; 1749-1750; 1751-1752; 1753-1754; 1755-1756; 1757-1758; 1759-1760; 1761-1762; 1763-1764; 1765-1766; 1767-1768; 1769-1770; 1771-1772; 1773-1774; 1775-1776; 1777-1778; 1779-1780; 1781-1782; 1783-1784; 1785-1786; 1787-1788; 1789-1790; 1791-1792; 1793-1794; 1795-1796; 1797-1798; 1799-1800; 1801-1802; 1803-1804; 1805-1806; 1807-1808; 1809-1810; 1811-1812; 1813-1814; 1815-1816).

In other advantageous embodiments, the HPV-specific primers comprise at least one of, preferably all, the following groups of pairs of primers:

- SD1-SA1 group consisting of the pairs of primers of SEQ ID NO: 397-398; 521-522; 609-610; 695-696; 819-820; 865-866; 947-948; 1067-1068; 1119-1120; 1267-1268; 1325-1326; 1507-1508; 1597-1598; 1655-1656; 1755-1756; and/or,
- SD1-SA2 group consisting of the pairs of primers of SEQ ID NO: 459-460; 633-634; 687-688; 1111-1112; 1235-1236; 1341-1342; 1503-1504; 1657-1658; 1797-1798; and/or,
- SD1-SA3 group consisting of the pairs of primers of SEQ ID NO: 381-382; 541-542; 599-600; 903-904; 941-942; 1047-1048; 1135-1136; 1287-1288; 1459-1460; 1473-1474; 1621-1622; 1717-1718; 1745-1746; and/or,
- SD1-SA4 group consisting of the pairs of primers of SEQ ID NO: 413-414; 551-552; 637-638; 713-714; 793-794; 857-858; 981-982; 1093-1094; 1179-1180; 1227-1228; 1319-1320; 1413-1414; 1509-1510; 1563-1564; 1709-1710;

1791-1792 ; and/or,

- SD1-SA5 group consisting of the pairs of primers of SEQ ID NO: 453-454; 549-550; 613-614; 747-748; 761-762; 949-950; 1163-1164; 1249-1250; 1329-1330; 1453-1454; 1501-1502; and/or,
- SD1-SA6 group consisting of the pairs of primers of SEQ ID NO: 431-432; 595-596; 719-720; 827-828; 1089-1090; 1137-1138; 1285-1286; 1353-1354; 1561-1562; 1719-1720; 1763-1764; and/or,
- SD1-SA7 group consisting of the pairs of primers of SEQ ID NO: 919-920; 1449-1450; and/or,
- SD1-SA8 group consisting of the pairs of primers of SEQ ID NO: 489-490; 963-964; 1519-1520 ; and/or,
- SD2-SA4 group consisting of the pairs of primers of SEQ ID NO: 387-388; 473-474; 615-616; 745-746; 815-816; 849-850; 933-934; 1091-1092; 1177-1178; 1209-1210; 1367-1368; 1437-1438; 1521-1522; 1603-1604; 1651-1652; 1779-1780; and/or,
- SD2-SA5 group consisting of the pairs of primers of SEQ ID NO: 455-456; 529-530; 629-630; 717-718; 777-778; 975-976; 1153-1154; 1273-1274; 1347-1348; 1451-1452; 1531-1532; and/or,
- SD2-SA6 group consisting of the pairs of primers of SEQ ID NO: 399-400; 645-646; 727-728; 811-812; 1079-1080; 1127-1128; 1253-1254; 1369-1370; 1615-1616; 1659-1660; 1781-1782; and/or,
- SD2-SA7 group consisting of the pairs of primers of SEQ ID NO: 531-532; 899-900; 943-944; 1411-1412; 1495-1496; and/or,
- SD2-SA9 group consisting of the pairs of primers of SEQ ID NO: 437-438; 505-506; 607-608; 739-740; 785-786; 887-888; 979-980; 1063-1064; 1185-1186; 1233-1234; 1297-1298; 1423-1424; 1491-1492; 1607-1608; 1693-1694; 1775-1776; and/or,
- SD2-SA10 group consisting of the pairs of primers of SEQ ID NO: 545-546; 831-832; 1149-1150; 1269-1270; 1427-1428; 1671-1672 ; and/or,
- SD3-SA4 group consisting of the pairs of primers of SEQ ID NO: 379-380; 483-484; 611-612; 721-722; 833-834; 911-912; 937-938; 1053-1054; 1139-1140; 1251-1252; 1335-1336; 1435-1436; 1487-1488; 1591-1592; 1715-1716; 1785-1786; and/or,
- SD3-SA5 group consisting of the pairs of primers of SEQ ID NO: 415-416; 493-494; 593-594; 733-734; 817-818; 993-994; 1145-1146; 1243-1244; 1337-1338; 1401-1402; 1483-1484 ; and/or,
- SD3-SA6 group consisting of the pairs of primers of SEQ ID NO: 435-436; 655-656; 673-674; 813-814; 1045-1046; 1173-1174; 1241-1242; 1303-1304; 1557-1558; 1627-1628; 1647-1648; 1773-1774; and/or,
- SD3-SA7 group consisting of the pairs of primers of SEQ ID NO: 855-856; 1387-1388 ; and/or,
- SD3-SA8 group consisting of the pairs of primers of SEQ ID NO: 511-512; 957-958; 1529-1530; and/or,
- SD5-SA9 group consisting of the pairs of primers of SEQ ID NO: 419-420; 527-528; 567-568; 587-588; 683-684; 775-776; 891-892; 999-1000; 1041-1042; 1113-1114; 1247-1248; 1371-1372; 1403-1404; 1511-1512; 1617-1618; 1677-1678; 1733-1734; and/or,
- SD5-SA10 group consisting of the pairs of primers of SEQ ID NO: 495-496; 837-838; 1183-1184; 1279-1280; 1433-1434; 1723-1724.

In other embodiments, the HPV-specific primers comprise one of the following groups of pairs of primers:

- the group of pairs of primers of SEQ ID NO: 397-398; 521-522; 609-610; 695-696; 819-820; 865-866; 947-948; 1067-1068; 1119-1120; 1267-1268; 1325-1326; 1507-1508; 1597-1598; 1655-1656; 1755-1756; 459-460; 633-634; 687-688; 1111-1112; 1235-1236; 1341-1342; 1503-1504; 1657-1658; 1797-1798; 381-382; 541-542; 599-600; 903-904; 941-942; 1047-1048; 1135-1136; 1287-1288; 1459-1460; 1473-1474; 1621-1622; 1717-1718; 1745-1746; 413-414; 551-552; 637-638; 713-714; 793-794; 857-858; 981-982; 1093-1094; 1179-1180; 1227-1228; 1319-1320; 1413-1414; 1509-1510; 1563-1564; 1709-1710; 1791-1792; 453-454; 549-550; 613-614; 747-748; 761-762; 949-950; 1163-1164; 1249-1250; 1329-1330; 1453-1454; 1501-1502; 431-432; 595-596; 719-720; 827-828; 1089-1090; 1137-1138; 1285-1286; 1353-1354; 1561-1562; 1719-1720; 1763-1764; 919-920; 1449-1450; 489-490; 963-964; 1519-1520; 387-388; 473-474; 615-616; 745-746; 815-816; 849-850; 933-934; 1091-1092; 1177-1178; 1209-1210; 1367-1368; 1437-1438; 1521-1522; 1603-1604; 1651-1652; 1779-1780; 455-456; 529-530; 629-630; 717-718; 777-778; 975-976; 1153-1154; 1273-1274; 1347-1348; 1451-1452; 1531-1532; 399-400; 645-646; 727-728; 811-812; 1079-1080; 1127-1128; 1253-1254; 1369-1370; 1615-1616; 1659-1660; 1781-1782; 531-532; 899-900; 943-944; 1411-1412; 1495-1496; 437-438; 505-506; 607-608; 739-740; 785-786; 887-888; 979-980; 1063-1064; 1185-1186; 1233-1234; 1297-1298; 1423-1424; 1491-1492; 1607-1608; 1693-1694; 1775-1776; 545-546; 831-832; 1149-1150; 1269-1270; 1427-1428; 1671-1672; 379-380; 483-484; 611-612; 721-722; 833-834; 911-912; 937-938; 1053-1054; 1139-1140; 1251-1252; 1335-1336; 1435-1436; 1487-1488; 1591-1592; 1715-1716; 1785-1786; 415-416; 493-494; 593-594; 733-734; 817-818; 993-994; 1145-1146; 1243-1244; 1337-1338; 1401-1402; 1483-1484; 435-436; 655-656; 673-674; 813-814; 1045-1046; 1173-1174; 1241-1242; 1303-1304; 1557-1558; 1627-1628; 1647-1648; 1773-1774; 855-856; 1387-1388; 511-512; 957-958; 1529-1530; 477-478; 419-420; 527-528; 567-568; 587-588; 683-684; 775-776; 891-892; 999-1000; 1041-1042; 1113-1114; 1247-1248;

1371-1372; 1403-1404; 1511-1512; 1617-1618; 1677-1678; 1733-1734; 495-496; 837-838; 1183-1184; 1279-1280; 1433-1434; 1723-1724; 1011-1012; 557-558; or,

- the group of pairs of primers of SEQ ID NO: 229-230; 233-234; 235-236; 245-246; 247-248; 249-250; 251-252; 255-256; 257-258; 265-266; 273-274; 275-276; 277-278; 279-280; 281-282; 289-290; 291-292; 295-296; 297-298; 299-300; 301-302; 303-304; 305-306; 307-308; 309-310; 311-312; 319-320; 321-322; 323-324; 325-326; 327-328; 329-330; 331-332; 333-334; 335-336; 337-338; 341-342; 343-344; 345-346; 347-348; 349-350; 351-352; 377-378; 379-380; 381-382; 383-384; 385-386; 387-388; 389-390; 391-392; 393-394; 395-396; 397-398; 399-400; 401-402; 403-404; 405-406; 407-408; 409-410; 411-412; 413-414; 415-416; 417-418; 419-420; 421-422; 423-424; 425-426; 427-428; 429-430; 431-432; 433-434; 435-436; 437-438; 439-440; 441-442; 443-444; 445-446; 447-448; 449-450; 451-452; 453-454; 455-456; 457-458; 459-460; 461-462; 463-464; 465-466; 467-468; 469-470; 471-472; 473-474; 475-476; 477-478; 479-480; 481-482; 483-484; 485-486; 487-488; 489-490; 491-492; 493-494; 495-496; 497-498; 499-500; 501-502; 503-504; 505-506; 507-508; 509-510; 511-512; 513-514; 515-516; 517-518; 519-520; 521-522; 523-524; 525-526; 527-528; 529-530; 531-532; 533-534; 535-536; 537-538; 539-540; 541-542; 543-544; 545-546; 547-548; 549-550; 551-552; 553-554; 555-556; 557-558; 559-560; 561-562; 563-564; 565-566; 567-568; 569-570; 571-572; 573-574; 575-576; 577-578; 579-580; 581-582; 583-584; 585-586; 587-588; 589-590; 591-592; 593-594; 595-596; 597-598; 599-600; 601-602; 603-604; 605-606; 607-608; 609-610; 611-612; 613-614; 615-616; 617-618; 619-620; 621-622; 623-624; 625-626; 627-628; 629-630; 631-632; 633-634; 635-636; 637-638; 639-640; 641-642; 643-644; 645-646; 647-648; 649-650; 651-652; 653-654; 655-656; 657-658; 659-660; 661-662; 663-664; 665-666; 667-668; 669-670; 671-672; 673-674; 675-676; 677-678; 679-680; 681-682; 683-684; 685-686; 687-688; 689-690; 691-692; 693-694; 695-696; 697-698; 699-700; 701-702; 703-704; 705-706; 707-708; 709-710; 711-712; 713-714; 715-716; 717-718; 719-720; 721-722; 723-724; 725-726; 727-728; 729-730; 731-732; 733-734; 735-736; 737-738; 739-740; 741-742; 743-744; 745-746; 747-748; 749-750; 751-752; 753-754; 755-756; 757-758; 759-760; 761-762; 763-764; 765-766; 767-768; 769-770; 771-772; 773-774; 775-776; 777-778; 779-780; 781-782; 783-784; 785-786; 787-788; 789-790; 791-792; 793-794; 795-796; 797-798; 799-800; 801-802; 803-804; 805-806; 807-808; 809-810; 811-812; 813-814; 815-816; 817-818; 819-820; 821-822; 823-824; 825-826; 827-828; 829-830; 831-832; 833-834; 835-836; 837-838; 839-840; 841-842; 843-844; 845-846; 847-848; 849-850; 851-852; 853-854; 855-856; 857-858; 859-860; 861-862; 863-864; 865-866; 867-868; 869-870; 871-872; 873-874; 875-876; 877-878; 879-880; 881-882; 883-884; 885-886; 887-888; 889-890; 891-892; 893-894; 895-896; 897-898; 899-900; 901-902; 903-904; 905-906; 907-908; 909-910; 911-912; 913-914; 915-916; 917-918; 919-920; 921-922; 923-924; 925-926; 927-928; 929-930; 931-932; 933-934; 935-936; 937-938; 939-940; 941-942; 943-944; 945-946; 947-948; 949-950; 951-952; 953-954; 955-956; 957-958; 959-960; 961-962; 963-964; 965-966; 967-968; 969-970; 971-972; 973-974; 975-976; 977-978; 979-980; 981-982; 983-984; 985-986; 987-988; 989-990; 991-992; 993-994; 995-996; 997-998; 999-1000; 1001-1002; 1003-1004; 1005-1006; 1007-1008; 1009-1010; 1011-1012; 1013-1014; 1015-1016; 1017-1018; 1019-1020; 1021-1022; 1023-1024; 1025-1026; 1027-1028; 1029-1030; 1031-1032; 1033-1034; 1035-1036; 1037-1038; 1039-1040; 1041-1042; 1043-1044; 1045-1046; 1047-1048; 1049-1050; 1051-1052; 1053-1054; 1055-1056; 1057-1058; 1059-1060; 1061-1062; 1063-1064; 1065-1066; 1067-1068; 1069-1070; 1071-1072; 1073-1074; 1075-1076; 1077-1078; 1079-1080; 1081-1082; 1083-1084; 1085-1086; 1087-1088; 1089-1090; 1091-1092; 1093-1094; 1095-1096; 1097-1098; 1099-1100; 1101-1102; 1103-1104; 1105-1106; 1107-1108; 1109-1110; 1111-1112; 1113-1114; 1115-1116; 1117-1118; 1119-1120; 1121-1122; 1123-1124; 1125-1126; 1127-1128; 1129-1130; 1131-1132; 1133-1134; 1135-1136; 1137-1138; 1139-1140; 1141-1142; 1143-1144; 1145-1146; 1147-1148; 1149-1150; 1151-1152; 1153-1154; 1155-1156; 1157-1158; 1159-1160; 1161-1162; 1163-1164; 1165-1166; 1167-1168; 1169-1170; 1171-1172; 1173-1174; 1175-1176; 1177-1178; 1179-1180; 1181-1182; 1183-1184; 1185-1186; 1187-1188; 1189-1190; 1191-1192; 1193-1194; 1195-1196; 1197-1198; 1199-1200; 1201-1202; 1203-1204; 1205-1206; 1207-1208; 1209-1210; 1211-1212; 1213-1214; 1215-1216; 1217-1218; 1219-1220; 1221-1222; 1223-1224; 1225-1226; 1227-1228; 1229-1230; 1231-1232; 1233-1234; 1235-1236; 1237-1238; 1239-1240; 1241-1242; 1243-1244; 1245-1246; 1247-1248; 1249-1250; 1251-1252; 1253-1254; 1255-1256; 1257-1258; 1259-1260; 1261-1262; 1263-1264; 1265-1266; 1267-1268; 1269-1270; 1271-1272; 1273-1274; 1275-1276; 1277-1278; 1279-1280; 1281-1282; 1283-1284; 1285-1286; 1287-1288; 1289-1290; 1291-1292; 1293-1294; 1295-1296; 1297-1298; 1299-1300; 1301-1302; 1303-1304; 1305-1306; 1307-1308; 1309-1310; 1311-1312; 1313-1314; 1315-1316; 1317-1318; 1319-1320; 1321-1322; 1323-1324; 1325-1326; 1327-1328; 1329-1330; 1331-1332; 1333-1334; 1335-1336; 1337-1338; 1339-1340; 1341-1342; 1343-1344; 1345-1346; 1347-1348; 1349-1350; 1351-1352; 1353-1354; 1355-1356; 1357-1358; 1359-1360; 1361-1362; 1363-1364; 1365-1366; 1367-1368; 1369-1370; 1371-1372; 1373-1374; 1375-1376; 1377-1378; 1379-1380; 1381-1382; 1383-1384; 1385-1386; 1387-1388; 1389-1390; 1391-1392; 1393-1394; 1395-1396; 1397-1398; 1399-1400; 1401-1402; 1403-1404; 1405-1406; 1407-1408; 1409-1410; 1411-1412; 1413-1414; 1415-1416; 1417-1418; 1419-1420; 1421-1422; 1423-1424; 1425-1426; 1427-1428; 1429-1430; 1431-1432; 1433-1434; 1435-1436; 1437-1438; 1439-1440; 1441-1442; 1443-1444; 1445-1446; 1447-1448; 1449-1450; 1451-1452; 1453-1454; 1455-1456; 1457-1458; 1459-1460; 1461-1462; 1463-1464; 1465-1466; 1467-1468;

1469-1470; 1471-1472; 1473-1474; 1475-1476; 1477-1478; 1479-1480; 1481-1482; 1483-1484; 1485-1486; 1487-1488; 1489-1490; 1491-1492; 1493-1494; 1495-1496; 1497-1498; 1499-1500; 1501-1502; 1503-1504; 1505-1506; 1507-1508; 1509-1510; 1511-1512; 1513-1514; 1515-1516; 1517-1518; 1519-1520; 1521-1522; 1523-1524; 1525-1526; 1527-1528; 1529-1530; 1531-1532; 1533-1534; 1535-1536; 1537-1538; 1539-1540; 1541-1542; 1543-1544; 1545-1546; 1547-1548; 1549-1550; 1551-1552; 1553-1554; 1555-1556; 1557-1558; 1559-1560; 1561-1562; 1563-1564; 1565-1566; 1567-1568; 1569-1570; 1571-1572; 1573-1574; 1575-1576; 1577-1578; 1579-1580; 1581-1582; 1583-1584; 1585-1586; 1587-1588; 1589-1590; 1591-1592; 1593-1594; 1595-1596; 1597-1598; 1599-1600; 1601-1602; 1603-1604; 1605-1606; 1607-1608; 1609-1610; 1611-1612; 1613-1614; 1615-1616; 1617-1618; 1619-1620; 1621-1622; 1623-1624; 1625-1626; 1627-1628; 1629-1630; 1631-1632; 1633-1634; 1635-1636; 1637-1638; 1639-1640; 1641-1642; 1643-1644; 1645-1646; 1647-1648; 1649-1650; 1651-1652; 1653-1654; 1655-1656; 1657-1658; 1659-1660; 1661-1662; 1663-1664; 1665-1666; 1667-1668; 1669-1670; 1671-1672; 1673-1674; 1675-1676; 1677-1678; 1679-1680; 1681-1682; 1683-1684; 1685-1686; 1687-1688; 1689-1690; 1691-1692; 1693-1694; 1695-1696; 1697-1698; 1699-1700; 1701-1702; 1703-1704; 1705-1706; 1707-1708; 1709-1710; 1711-1712; 1713-1714; 1715-1716; 1717-1718; 1719-1720; 1721-1722; 1723-1724; 1725-1726; 1727-1728; 1729-1730; 1731-1732; 1733-1734; 1735-1736; 1737-1738; 1739-1740; 1741-1742; 1743-1744; 1745-1746; 1747-1748; 1749-1750; 1751-1752; 1753-1754; 1755-1756; 1757-1758; 1759-1760; 1761-1762; 1763-1764; 1765-1766; 1767-1768; 1769-1770; 1771-1772; 1773-1774; 1775-1776; 1777-1778; 1779-1780; 1781-1782; 1783-1784; 1785-1786; 1787-1788; 1789-1790; 1791-1792; 1793-1794; 1795-1796; 1797-1798; 1799-1800; 1801-1802; 1803-1804; 1805-1806; 1807-1808; 1809-1810; 1811-1812; 1813-1814; 1815-1816.

In various embodiments, the number of HPV sequence reads according to HPV species and/or HPV gene transcript can be determined.

In one embodiment, the method comprises determining the number of HPV sequence reads of at least 1, 2, 3, 4, 5, 6, 7, 8, 9, or 10 HPV species. In one embodiment, the method comprises determining the number of HPV sequence reads of at least 1, 2, 3, 4, 5, 6, 7, 8, 9, or 10 Alpha group HPV species.

In a further embodiment, the method comprises calculating a ratio (R) of the number of reads of at least one early HPV transcript to the number of reads of at least one late HPV transcript. Preferably the HPV transcripts are Alpha group HPV species HPV transcripts, most preferably HPV16 or HPV18 transcripts.

R values can be determined using any of the various formulas presented in Table 9. In one embodiment, the ratio is calculated by calculating a ratio (R) of the number of reads of HPV E6 and/or E7 transcripts to the number of reads of HPV L1 and/or L2 transcripts. In one embodiment, the ratio is calculated by calculating a ratio (R) of the number of reads of HPV E6 and E7 transcripts to the number of reads of HPV L1 and L2 transcripts.

In some embodiments, a higher ratio (R) correlates with an increased risk of developing high-grade malignant HPV-induced cancer. In various embodiments, an R value tending towards high values above 0.5, 1, 25, 50, 100 and tending towards infinity, indicates that the viral cycle is integrated, non replicating viruses expressing high level of oncogenes E6 and E7; whereas, an R value below 0.25, 0.2, 0.1, 0.05, and tending towards 0, indicates that the viral lifecycle is replicative and expressing low levels of E6 and E7.

In some embodiments, a higher number of reads of transcripts of HPV16, HPV18, or another high-risk HPV relative to reads of transcripts of a lower risk HPV species correlates with an increased risk of developing high-grade malignant HPV-induced cancer.

In some embodiments, the method comprises determining the number of reads matching the viruses based on species discrimination and determining the most prevalent high risk species present in the sample relative to other species and /or determining within said most prevalent high risk species the relative number of reads matching oncogenic genes compared to non oncogenic genes.

In one embodiment, the invention encompasses methods for assessing a human papilloma virus (HPV) infected patient comprising generating cDNA from a patient sample comprising RNA; sequencing the cDNA; generating reads of sequence of the cDNA; discriminating HPV sequence reads on the basis of HPV gene transcript; quantitating the level of HPV sequence reads according to HPV gene transcript; determining the number of HPV sequence reads of at least one HPV early gene transcript; determining the number of HPV sequence reads of at least one HPV late gene transcript; and determining the ratio of the number of HPV sequence reads of at least one HPV early gene transcript to the number of HPV sequence reads of at least one HPV late gene transcript.

The invention also contemplates a composition of group alpha HPV-specific primers comprising at least one of, preferably all, the following groups of pairs of primers:

- the HPV16-specific primers comprising or consisting of the primers of SEQ ID NOs: 219-258 for HPV16 genomic and unspliced transcripts, SEQ ID NOs: 259-352 for HPV16 spliced transcripts and SEQ ID NOs: 353-376 for HPV16-human fusion transcripts (including the pairs of primers of SEQ ID NO: 219-220; 221-222; 223-224; 225-226; 227-228;

229-230; 231-232; 233-234; 235-236; 237-238; 239-240; 241-242; 243-244; 245-246; 247-248; 249-250; 251-252; 253-254; 255-256; 257-258; 259-260; 261-262; 263-264; 265-266; 267-268; 269-270; 271-272; 273-274; 275-276; 277-278; 279-280; 281-282; 283-284; 285-286; 287-288; 289-290; 291-292; 293-294; 295-296; 297-298; 299-300; 301-302; 303-304; 305-306; 307-308; 309-310; 311-312; 313-314; 315-316; 317-318; 319-320; 321-322; 323-324; 325-326; 327-328; 329-330; 331-332; 333-334; 335-336; 337-338; 339-340; 341-342; 343-344; 345-346; 347-348; 349-350; 351-352; 353-354; 355-356; 357-358; 359-360; 361-362; 363-364; 365-366; 367-368; 369-370; 371-372; 373-374; 375-376) or 377-470 (including the pairs of primers of SEQ ID NO. 377-378; 379-380; 381-382; 383-384; 385- 386; 387-388; 389-390; 391-392; 393-394; 395-396; 397-398; 399-400; 401-402; 403-404; 405-406; 407-408; 409-410; 411-412; 413-414; 415-416; 417-418; 419-420; 421-422; 423-424; 425-426; 427-428; 429-430; 431-432; 433-434; 435-436; 437-438; 439-440; 441-442; 443-444; 445-446; 447-448; 449-450; 451-452; 453-454; 455-456; 457-458; 459-460; 461-462; 463-464; 465-466; 467-468 and; 469-470); and/or,

- the HPV18-specific primers comprising or consisting of the primers of SEQ ID NO. 471-574 (including the pairs of primers of SEQ ID NO.: 471-472; 473-474; 475-476; 477-478; 479-480; 481-482; 483-484; 485-486; 487-488; 489-490; 491-492; 493-494; 495-496; 497-498; 499-500; 501-502; 503-504; 505-506; 507-508; 509-510; 511-512; 513-514; 515-516; 517-518; 519-520; 521-522; 523-524; 525-526; 527-528; 529-530; 531-532; 533-534; 535-536; 537-538; 539-540; 541-542; 543-544; 545-546; 547-548; 549-550; 551-552; 553-554; 555-556; 557-558; 559-560; 561-562; 563-564; 565-566; 567-568; 569-570; 571-572; 573-574); and/or,

- the HPV31-specific primers comprising or consisting of the primers of SEQ ID NO. 575-668 (including the pairs of primers of SEQ ID NO.: 575-576; 577-578; 579-580; 581-582; 583-584; 585-586; 587-588; 589-590; 591-592; 593-594; 595-596; 597-598; 599-600; 601-602; 603-604; 605-606; 607-608; 609-610; 611-612; 613-614; 615-616; 617-618; 619-620; 621-622; 623-624; 625-626; 627-628; 629-630; 631-632; 633-634; 635-636; 637-638; 639-640; 641-642; 643-644; 645-646; 647-648; 649-650; 651-652; 653-654; 655-656; 657-658; 659-660; 661-662; 663-664; 665-666; 667-668); and/or,

- the HPV33-specific primers comprising or consisting of SEQ ID NO. 669-756 (including the pairs of primers of SEQ ID NO.: 669-670; 671-672; 673-674; 675-676; 677-678; 679-680; 681-682; 683-684; 685-686; 687-688; 689-690; 691-692; 693-694; 695-696; 697-698; 699-700; 701-702; 703-704; 705-706; 707-708; 709-710; 711-712; 713-714; 715-716; 717-718; 719-720; 721-722; 723-724; 725-726; 727-728; 729-730; 731-732; 733-734; 735-736; 737-738; 739-740; 741-742; 743-744; 745-746; 747-748; 749-750; 751-752; 753-754; 755-756); and/or,

- the HPV35-specific primers comprising or consisting of the primers of SEQ ID NO. 757-848 (including the pairs of primers of SEQ ID NO.: 757-758; 759-760; 761-762; 763-764; 765-766; 767-768; 769-770; 771-772; 773-774; 775-776; 777-778; 779-780; 781-782; 783-784; 785-786; 787-788; 789-790; 791-792; 793-794; 795-796; 797-798; 799-800; 801-802; 803-804; 805-806; 807-808; 809-810; 811-812; 813-814; 815-816; 817-818; 819-820; 821-822; 823-824; 825-826; 827-828; 829-830; 831-832; 833-834; 835-836; 837-838; 839-840; 841-842; 843-844; 845-846; 847-848); and/or,

- the HPV39-specific primers comprising or consisting of the primers of SEQ ID NO. 849-928 (including the pairs of primers of SEQ ID NO.: 849-850; 851-852; 853-854; 855-856; 857-858; 859-860; 861-862; 863-864; 865-866; 867-868; 869-870; 871-872; 873-874; 875-876; 877-878; 879-880; 881-882; 883-884; 885-886; 887-888; 889-890; 891-892; 893-894; 895-896; 897-898; 899-900; 901-902; 903-904; 905-906; 907-908; 909-910; 911-912; 913-914; 915-916; 917-918; 919-920; 921-922; 923-924; 925-926; 927-928); and/or,

- the HPV45-specific primers comprising or consisting of the primers of SEQ ID NO. 929-1020 (including the pairs of primers of SEQ ID NO.: 929-930; 931-932; 933-934; 935-936; 937-938; 939-940; 941-942; 943-944; 945-946; 947-948; 949-950; 951-952; 953-954; 955-956; 957-958; 959-960; 961-962; 963-964; 965-966; 967-968; 969-970; 971-972; 973-974; 975-976; 977-978; 979-980; 981-982; 983-984; 985-986; 987-988; 989-990; 991-992; 993-994; 995-996; 997-998; 999-1000; 1001-1002; 1003-1004; 1005-1006; 1007-1008; 1009-1010; 1011-1012; 1013-1014; 1015-1016; 1017-1018; 1019-1020); and/or,

- the HPV51-specific primers comprising or consisting of the primers of SEQ ID NO. 1021-1102 (including the pairs of primers of SEQ ID NO.: 1021-1022; 1023-1024; 1025-1026; 1027-1028; 1029-1030; 1031-1032; 1033-1034; 1035-1036; 1037-1038; 1039-1040; 1041-1042; 1043-1044; 1045-1046; 1047-1048; 1049-1050; 1051-1052; 1053-1054; 1055-1056; 1057-1058; 1059-1060; 1061-1062; 1063-1064; 1065-1066; 1067-1068; 1069-1070; 1071-1072; 1073-1074; 1075-1076; 1077-1078; 1079-1080; 1081-1082; 1083-1084; 1085-1086; 1087-1088; 1089-1090; 1091-1092; 1093-1094; 1095-1096; 1097-1098; 1099-1100; 1101-1102); and/or,

- the HPV52-specific primers comprising or consisting of the primers of SEQ ID NO. 1103-1200 (including the pairs of primers of SEQ ID NO.: 1103-1104; 1105-1106; 1107-1108; 1109-1110; 1111-1112; 1113-1114; 1115-1116; 1117-1118; 1119-1120; 1121-1122; 1123-1124; 1125-1126; 1127-1128; 1129-1130; 1131-1132; 1133-1134; 1135-1136; 1137-1138; 1139-1140; 1141-1142; 1143-1144; 1145-1146; 1147-1148; 1149-1150; 1151-1152; 1153-1154; 1155-1156; 1157-1158; 1159-1160; 1161-1162; 1163-1164; 1165-1166; 1167-1168; 1169-1170; 1171-1172; 1173-1174; 1175-1176; 1177-1178; 1179-1180; 1181-1182; 1183-1184; 1185-1186; 1187-1188; 1189-1190; 1191-1192; 1193-1194; 1195-1196; 1197-1198; 1199-1200); and/or,

- the HPV56-specific primers comprising or consisting of the primers of SEQ ID NO. 1201-1296 (including the pairs of primers of SEQ ID NO.: 1201-1202; 1203-1204; 1205-1206; 1207-1208; 1209-1210; 1211-1212; 1213-1214; 1215-1216; 1217-1218; 1219-1220; 1221-1222; 1223-1224; 1225-1226; 1227-1228; 1229-1230; 1231-1232; 1233-1234; 1235-1236; 1237-1238; 1239-1240; 1241-1242; 1243-1244; 1245-1246; 1247-1248; 1249-1250; 1251-1252; 1253-1254; 1255-1256; 1257-1258; 1259-1260; 1261-1262; 1263-1264; 1265-1266; 1267-1268; 1269-1270; 1271-1272; 1273-1274; 1275-1276; 1277-1278; 1279-1280; 1281-1282; 1283-1284; 1285-1286; 1287-1288; 1289-1290; 1291-1292; 1293-1294; 1295-1296); and/or,

- the HPV58-specific primers comprising or consisting of the primers of SEQ ID NO. 1297-1382 (including the pairs of primers of SEQ ID NO.: 1297-1298; 1299-1300; 1301-1302; 1303-1304; 1305-1306; 1307-1308; 1309-1310; 1311-1312; 1313-1314; 1315-1316; 1317-1318; 1319-1320; 1321-1322; 1323-1324; 1325-1326; 1327-1328; 1329-1330; 1331-1332; 1333-1334; 1335-1336; 1337-1338; 1339-1340; 1341-1342; 1343-1344; 1345-1346; 1347-1348; 1349-1350; 1351-1352; 1353-1354; 1355-1356; 1357-1358; 1359-1360; 1361-1362; 1363-1364; 1365-1366; 1367-1368; 1369-1370; 1371-1372; 1373-1374; 1375-1376; 1377-1378; 1379-1380; 1381-1382); and/or,

- the HPV59-specific primers comprising or consisting of the primers of SEQ ID NO. 1383-1470 (including the pairs of primers of SEQ ID NO.: 1383-1384; 1385-1386; 1387-1388; 1389-1390; 1391-1392; 1393-1394; 1395-1396; 1397-1398; 1399-1400; 1401-1402; 1403-1404; 1405-1406; 1407-1408; 1409-1410; 1411-1412; 1413-1414; 1415-1416; 1417-1418; 1419-1420; 1421-1422; 1423-1424; 1425-1426; 1427-1428; 1429-1430; 1431-1432; 1433-1434; 1435-1436; 1437-1438; 1439-1440; 1441-1442; 1443-1444; 1445-1446; 1447-1448; 1449-1450; 1451-1452; 1453-1454; 1455-1456; 1457-1458; 1459-1460; 1461-1462; 1463-1464; 1465-1466; 1467-1468; 1469-1470); and/or,

- the HPV66-specific primers comprising or consisting of the primers of SEQ ID NO. 1471-1560 (including the pairs of primers of SEQ ID NO.: 1471-1472; 1473-1474; 1475-1476; 1477-1478; 1479-1480; 1481-1482; 1483-1484; 1485-1486; 1487-1488; 1489-1490; 1491-1492; 1493-1494; 1495-1496; 1497-1498; 1499-1500; 1501-1502; 1503-1504; 1505-1506; 1507-1508; 1509-1510; 1511-1512; 1513-1514; 1515-1516; 1517-1518; 1519-1520; 1521-1522; 1523-1524; 1525-1526; 1527-1528; 1529-1530; 1531-1532; 1533-1534; 1535-1536; 1537-1538; 1539-1540; 1541-1542; 1543-1544; 1545-1546; 1547-1548; 1549-1550; 1551-1552; 1553-1554; 1555-1556; 1557-1558; 1559-1560; and/or,

- the HPV68-specific primers comprising or consisting of the primers of SEQ ID NO. 1561-1642 (including the pairs of primers of SEQ ID NO.: 1561-1562; 1563-1564; 1565-1566; 1567-1568; 1569-1570; 1571-1572; 1573-1574; 1575-1576; 1577-1578; 1579-1580; 1581-1582; 1583-1584; 1585-1586; 1587-1588; 1589-1590; 1591-1592; 1593-1594; 1595-1596; 1597-1598; 1599-1600; 1601-1602; 1603-1604; 1605-1606; 1607-1608; 1609-1610; 1611-1612; 1613-1614; 1615-1616; 1617-1618; 1619-1620; 1621-1622; 1623-1624; 1625-1626; 1627-1628; 1629-1630; 1631-1632; 1633-1634; 1635-1636; 1637-1638; 1639-1640; 1641-1642); and/or,

- the HPV73-specific primers comprising or consisting of the primers of SEQ ID NO. 1643-1732 (including the pairs of primers of SEQ ID NO.: 1643-1644; 1645-1646; 1647-1648; 1649-1650; 1651-1652; 1653-1654; 1655-1656; 1657-1658; 1659-1660; 1661-1662; 1663-1664; 1665-1666; 1667-1668; 1669-1670; 1671-1672; 1673-1674; 1675-1676; 1677-1678; 1679-1680; 1681-1682; 1683-1684; 1685-1686; 1687-1688; 1689-1690; 1691-1692; 1693-1694; 1695-1696; 1697-1698; 1699-1700; 1701-1702; 1703-1704; 1705-1706; 1707-1708; 1709-1710; 1711-1712; 1713-1714; 1715-1716; 1717-1718; 1719-1720; 1721-1722; 1723-1724; 1725-1726; 1727-1728; 1729-1730; 1731-1732); and/or,

- the HPV82-specific primers comprising or consisting of the primers of SEQ ID NO. 1733-1816 (including the pairs of primers of SEQ ID NO.: 1733-1734; 1735-1736; 1737-1738; 1739-1740; 1741-1742; 1743-1744; 1745-1746; 1747-1748; 1749-1750; 1751-1752; 1753-1754; 1755-1756; 1757-1758; 1759-1760; 1761-1762; 1763-1764;

1765-1766; 1767-1768; 1769-1770; 1771-1772; 1773-1774; 1775-1776; 1777-1778; 1779-1780; 1781-1782; 1783-1784; 1785-1786; 1787-1788; 1789-1790; 1791-1792; 1793-1794; 1795-1796; 1797-1798; 1799-1800; 1801-1802; 1803-1804; 1805-1806; 1807-1808; 1809-1810; 1811-1812; 1813-1814; 1815-1816).

The invention also contemplates a composition of pairs of group alpha HPV-specific primers comprising at least one of, preferably all, the following groups of pairs of primers:

- SD1-SA1 group consisting of the pairs of primers of SEQ ID NO: 397-398; 521-522; 609-610; 695-696; 819-820; 865-866; 947-948; 1067-1068; 1119-1120; 1267-1268; 1325-1326; 1507-1508; 1597-1598; 1655-1656; 1755-1756; and/or,
- SD1-SA2 group consisting of the pairs of primers of SEQ ID NO: 459-460; 633-634; 687-688; 1111-1112; 1235-1236; 1341-1342; 1503-1504; 1657-1658; 1797-1798; and/or,
- SD1-SA3 group consisting of the pairs of primers of SEQ ID NO: 381-382; 541-542; 599-600; 903-904; 941-942; 1047-1048; 1135-1136; 1287-1288; 1459-1460; 1473-1474; 1621-1622; 1717-1718; 1745-1746; and/or,
- SD1-SA4 group consisting of the pairs of primers of SEQ ID NO: 413-414; 551-552; 637-638; 713-714; 793-794; 857-858; 981-982; 1093-1094; 1179-1180; 1227-1228; 1319-1320; 1413-1414; 1509-1510; 1563-1564; 1709-1710; 1791-1792 ; and/or,
- SD1-SA5 group consisting of the pairs of primers of SEQ ID NO: 453-454; 549-550; 613-614; 747-748; 761-762; 949-950; 1163-1164; 1249-1250; 1329-1330; 1453-1454; 1501-1502; and/or,
- SD1-SA6 group consisting of the pairs of primers of SEQ ID NO: 431-432; 595-596; 719-720; 827-828; 1089-1090; 1137-1138; 1285-1286; 1353-1354; 1561-1562; 1719-1720; 1763-1764; and/or,
- SD1-SA7 group consisting of the pairs of primers of SEQ ID NO: 919-920; 1449-1450; and/or,
- SD1-SA8 group consisting of the pairs of primers of SEQ ID NO: 489-490; 963-964; 1519-1520 ; and/or,
- SD2-SA4 group consisting of the pairs of primers of SEQ ID NO: 387-388; 473-474; 615-616; 745-746; 815-816; 849-850; 933-934; 1091-1092; 1177-1178; 1209-1210; 1367-1368; 1437-1438; 1521-1522; 1603-1604; 1651-1652; 1779-1780; and/or,
- SD2-SA5 group consisting of the pairs of primers of SEQ ID NO: 455-456; 529-530; 629-630; 717-718; 777-778; 975-976; 1153-1154; 1273-1274; 1347-1348; 1451-1452; 1531-1532; and/or,
- SD2-SA6 group consisting of the pairs of primers of SEQ ID NO: 399-400; 645-646; 727-728; 811-812; 1079-1080; 1127-1128; 1253-1254; 1369-1370; 1615-1616; 1659-1660; 1781-1782; and/or,
- SD2-SA7 group consisting of the pairs of primers of SEQ ID NO: 531-532; 899-900; 943-944; 1411-1412; 1495-1496; and/or,
- SD2-SA9 group consisting of the pairs of primers of SEQ ID NO: 437-438; 505-506; 607-608; 739-740; 785-786; 887-888; 979-980; 1063-1064; 1185-1186; 1233-1234; 1297-1298; 1423-1424; 1491-1492; 1607-1608; 1693-1694; 1775-1776; and/or,
- SD2-SA10 group consisting of the pairs of primers of SEQ ID NO: 545-546; 831-832; 1149-1150; 1269-1270; 1427-1428; 1671-1672 ; and/or,
- SD3-SA4 group consisting of the pairs of primers of SEQ ID NO: 379-380; 483-484; 611-612; 721-722; 833-834; 911-912; 937-938; 1053-1054; 1139-1140; 1251-1252; 1335-1336; 1435-1436; 1487-1488; 1591-1592; 1715-1716; 1785-1786; and/or,
- SD3-SA5 group consisting of the pairs of primers of SEQ ID NO: 415-416; 493-494; 593-594; 733-734; 817-818; 993-994; 1145-1146; 1243-1244; 1337-1338; 1401-1402; 1483-1484 ; and/or,
- SD3-SA6 group consisting of the pairs of primers of SEQ ID NO: 435-436; 655-656; 673-674; 813-814; 1045-1046; 1173-1174; 1241-1242; 1303-1304; 1557-1558; 1627-1628; 1647-1648; 1773-1774; and/or,
- SD3-SA7 group consisting of the pairs of primers of SEQ ID NO: 855-856; 1387-1388 ; and/or,
- SD3-SA8 group consisting of the pairs of primers of SEQ ID NO: 511-512; 957-958; 1529-1530; and/or,
- SD5-SA9 group consisting of the pairs of primers of SEQ ID NO: 419-420; 527-528; 567-568; 587-588; 683-684; 775-776; 891-892; 999-1000; 1041-1042; 1113-1114; 1247-1248; 1371-1372; 1403-1404; 1511-1512; 1617-1618; 1677-1678; 1733-1734; and/or,
- SD5-SA10 group consisting of the pairs of primers of SEQ ID NO: 495-496; 837-838; 1183-1184; 1279-1280; 1433-1434; 1723-1724.

The invention also contemplates a composition of group alpha HPV-specific primers comprising one of the following groups of pairs of primers:

- the group of pairs of primers of SEQ ID NO: 397-398; 521-522; 609-610; 695-696; 819-820; 865-866; 947-948; 1067-1068; 1119-1120; 1267-1268; 1325-1326; 1507-1508; 1597-1598; 1655-1656; 1755-1756; 459-460; 633-634; 687-688; 1111-1112; 1235-1236; 1341-1342; 1503-1504; 1657-1658; 1797-1798; 381-382; 541-542; 599-600;

903-904; 941-942; 1047-1048; 1135-1136; 1287-1288; 1459-1460; 1473-1474; 1621-1622; 1717-1718; 1745-1746; 413-414; 551-552; 637-638; 713-714; 793-794; 857-858; 981-982; 1093-1094; 1179-1180; 1227-1228; 1319-1320; 1413-1414; 1509-1510; 1563-1564; 1709-1710; 1791-1792; 453-454; 549-550; 613-614; 747-748; 761-762; 949-950; 1163-1164; 1249-1250; 1329-1330; 1453-1454; 1501-1502; 431-432; 595-596; 719-720; 827-828; 1089-1090; 1137-1138; 1285-1286; 1353-1354; 1561-1562; 1719-1720; 1763-1764; 919-920; 1449-1450; 489-490; 963-964; 1519-1520; 387-388; 473-474; 615-616; 745-746; 815-816; 849-850; 933-934; 1091-1092; 1177-1178; 1209-1210; 1367-1368; 1437-1438; 1521-1522; 1603-1604; 1651-1652; 1779-1780; 455-456; 529-530; 629-630; 717-718; 777-778; 975-976; 1153-1154; 1273-1274; 1347-1348; 1451-1452; 1531-1532; 399-400; 645-646; 727-728; 811-812; 1079-1080; 1127-1128; 1253-1254; 1369-1370; 1615-1616; 1659-1660; 1781-1782; 531-532; 899-900; 943-944; 1411-1412; 1495-1496; 437-438; 505-506; 607-608; 739-740; 785-786; 887-888; 979-980; 1063-1064; 1185-1186; 1233-1234; 1297-1298; 1423-1424; 1491-1492; 1607-1608; 1693-1694; 1775-1776; 545-546; 831-832; 1149-1150; 1269-1270; 1427-1428; 1671-1672; 379-380; 483-484; 611-612; 721-722; 833-834; 911-912; 937-938; 1053-1054; 1139-1140; 1251-1252; 1335-1336; 1435-1436; 1487-1488; 1591-1592; 1715-1716; 1785-1786; 415-416; 493-494; 593-594; 733-734; 817-818; 993-994; 1145-1146; 1243-1244; 1337-1338; 1401-1402; 1483-1484; 435-436; 655-656; 673-674; 813-814; 1045-1046; 1173-1174; 1241-1242; 1303-1304; 1557-1558; 1627-1628; 1647-1648; 1773-1774; 855-856; 1387-1388; 511-512; 957-958; 1529-1530; 477-478; 419-420; 527-528; 567-568; 587-588; 683-684; 775-776; 891-892; 999-1000; 1041-1042; 1113-1114; 1247-1248; 1371-1372; 1403-1404; 1511-1512; 1617-1618; 1677-1678; 1733-1734; 495-496; 837-838; 1183-1184; 1279-1280; 1433-1434; 1723-1724; 1011-1012; 557-558; or,

- the group of pairs of primers of SEQ ID NO: 229-230; 233-234; 235-236; 245-246; 247-248; 249-250; 251-252; 255-256; 257-258; 265-266; 273-274; 275-276; 277-278; 279-280; 281-282; 289-290; 291-292; 295-296; 297-298; 299-300; 301-302; 303-304; 305-306; 307-308; 309-310; 311-312; 319-320; 321-322; 323-324; 325-326; 327-328; 329-330; 331-332; 333-334; 335-336; 337-338; 341-342; 343-344; 345-346; 347-348; 349-350; 351-352; 377-378; 379-380; 381-382; 383-384; 385-386; 387-388; 389-390; 391-392; 393-394; 395-396; 397-398; 399-400; 401-402; 403-404; 405-406; 407-408; 409-410; 411-412; 413-414; 415-416; 417-418; 419-420; 421-422; 423-424; 425-426; 427-428; 429-430; 431-432; 433-434; 435-436; 437-438; 439-440; 441-442; 443-444; 445-446; 447-448; 449-450; 451-452; 453-454; 455-456; 457-458; 459-460; 461-462; 463-464; 465-466; 467-468; 469-470; 471-472; 473-474; 475-476; 477-478; 479-480; 481-482; 483-484; 485-486; 487-488; 489-490; 491-492; 493-494; 495-496; 497-498; 499-500; 501-502; 503-504; 505-506; 507-508; 509-510; 511-512; 513-514; 515-516; 517-518; 519-520; 521-522; 523-524; 525-526; 527-528; 529-530; 531-532; 533-534; 535-536; 537-538; 539-540; 541-542; 543-544; 545-546; 547-548; 549-550; 551-552; 553-554; 555-556; 557-558; 559-560; 561-562; 563-564; 565-566; 567-568; 569-570; 571-572; 573-574; 575-576; 577-578; 579-580; 581-582; 583-584; 585-586; 587-588; 589-590; 591-592; 593-594; 595-596; 597-598; 599-600; 601-602; 603-604; 605-606; 607-608; 609-610; 611-612; 613-614; 615-616; 617-618; 619-620; 621-622; 623-624; 625-626; 627-628; 629-630; 631-632; 633-634; 635-636; 637-638; 639-640; 641-642; 643-644; 645-646; 647-648; 649-650; 651-652; 653-654; 655-656; 657-658; 659-660; 661-662; 663-664; 665-666; 667-668; 669-670; 671-672; 673-674; 675-676; 677-678; 679-680; 681-682; 683-684; 685-686; 687-688; 689-690; 691-692; 693-694; 695-696; 697-698; 699-700; 701-702; 703-704; 705-706; 707-708; 709-710; 711-712; 713-714; 715-716; 717-718; 719-720; 721-722; 723-724; 725-726; 727-728; 729-730; 731-732; 733-734; 735-736; 737-738; 739-740; 741-742; 743-744; 745-746; 747-748; 749-750; 751-752; 753-754; 755-756; 757-758; 759-760; 761-762; 763-764; 765-766; 767-768; 769-770; 771-772; 773-774; 775-776; 777-778; 779-780; 781-782; 783-784; 785-786; 787-788; 789-790; 791-792; 793-794; 795-796; 797-798; 799-800; 801-802; 803-804; 805-806; 807-808; 809-810; 811-812; 813-814; 815-816; 817-818; 819-820; 821-822; 823-824; 825-826; 827-828; 829-830; 831-832; 833-834; 835-836; 837-838; 839-840; 841-842; 843-844; 845-846; 847-848; 849-850; 851-852; 853-854; 855-856; 857-858; 859-860; 861-862; 863-864; 865-866; 867-868; 869-870; 871-872; 873-874; 875-876; 877-878; 879-880; 881-882; 883-884; 885-886; 887-888; 889-890; 891-892; 893-894; 895-896; 897-898; 899-900; 901-902; 903-904; 905-906; 907-908; 909-910; 911-912; 913-914; 915-916; 917-918; 919-920; 921-922; 923-924; 925-926; 927-928; 929-930; 931-932; 933-934; 935-936; 937-938; 939-940; 941-942; 943-944; 945-946; 947-948; 949-950; 951-952; 953-954; 955-956; 957-958; 959-960; 961-962; 963-964; 965-966; 967-968; 969-970; 971-972; 973-974; 975-976; 977-978; 979-980; 981-982; 983-984; 985-986; 987-988; 989-990; 991-992; 993-994; 995-996; 997-998; 999-1000; 1001-1002; 1003-1004; 1005-1006; 1007-1008; 1009-1010; 1011-1012; 1013-1014; 1015-1016; 1017-1018; 1019-1020; 1021-1022; 1023-1024; 1025-1026; 1027-1028; 1029-1030; 1031-1032; 1033-1034; 1035-1036; 1037-1038; 1039-1040; 1041-1042; 1043-1044; 1045-1046; 1047-1048; 1049-1050; 1051-1052; 1053-1054; 1055-1056; 1057-1058; 1059-1060; 1061-1062; 1063-1064; 1065-1066; 1067-1068; 1069-1070; 1071-1072; 1073-1074; 1075-1076; 1077-1078; 1079-1080; 1081-1082; 1083-1084; 1085-1086; 1087-1088; 1089-1090; 1091-1092; 1093-1094; 1095-1096; 1097-1098; 1099-1100; 1101-1102; 1103-1104; 1105-1106; 1107-1108; 1109-1110; 1111-1112; 1113-1114; 1115-1116; 1117-1118; 1119-1120; 1121-1122; 1123-1124; 1125-1126; 1127-1128; 1129-1130; 1131-1132; 1133-1134; 1135-1136; 1137-1138; 1139-1140; 1141-1142; 1143-1144; 1145-1146; 1147-1148; 1149-1150; 1151-1152; 1153-1154; 1155-1156; 1157-1158; 1159-1160; 1161-1162;

1163-1164; 1165-1166; 1167-1168; 1169-1170; 1171-1172; 1173-1174; 1175-1176; 1177-1178; 1179-1180; 1181-1182; 1183-1184; 1185-1186; 1187-1188; 1189-1190; 1191-1192; 1193-1194; 1195-1196; 1197-1198; 1199-1200; 1201-1202; 1203-1204; 1205-1206; 1207-1208; 1209-1210; 1211-1212; 1213-1214; 1215-1216; 1217-1218; 1219-1220; 1221-1222; 1223-1224; 1225-1226; 1227-1228; 1229-1230; 1231-1232; 1233-1234; 1235-1236; 1237-1238; 1239-1240; 1241-1242; 1243-1244; 1245-1246; 1247-1248; 1249-1250; 1251-1252; 1253-1254; 1255-1256; 1257-1258; 1259-1260; 1261-1262; 1263-1264; 1265-1266; 1267-1268; 1269-1270; 1271-1272; 1273-1274; 1275-1276; 1277-1278; 1279-1280; 1281-1282; 1283-1284; 1285-1286; 1287-1288; 1289-1290; 1291-1292; 1293-1294; 1295-1296; 1297-1298; 1299-1300; 1301-1302; 1303-1304; 1305-1306; 1307-1308; 1309-1310; 1311-1312; 1313-1314; 1315-1316; 1317-1318; 1319-1320; 1321-1322; 1323-1324; 1325-1326; 1327-1328; 1329-1330; 1331-1332; 1333-1334; 1335-1336; 1337-1338; 1339-1340; 1341-1342; 1343-1344; 1345-1346; 1347-1348; 1349-1350; 1351-1352; 1353-1354; 1355-1356; 1357-1358; 1359-1360; 1361-1362; 1363-1364; 1365-1366; 1367-1368; 1369-1370; 1371-1372; 1373-1374; 1375-1376; 1377-1378; 1379-1380; 1381-1382; 1383-1384; 1385-1386; 1387-1388; 1389-1390; 1391-1392; 1393-1394; 1395-1396; 1397-1398; 1399-1400; 1401-1402; 1403-1404; 1405-1406; 1407-1408; 1409-1410; 1411-1412; 1413-1414; 1415-1416; 1417-1418; 1419-1420; 1421-1422; 1423-1424; 1425-1426; 1427-1428; 1429-1430; 1431-1432; 1433-1434; 1435-1436; 1437-1438; 1439-1440; 1441-1442; 1443-1444; 1445-1446; 1447-1448; 1449-1450; 1451-1452; 1453-1454; 1455-1456; 1457-1458; 1459-1460; 1461-1462; 1463-1464; 1465-1466; 1467-1468; 1469-1470; 1471-1472; 1473-1474; 1475-1476; 1477-1478; 1479-1480; 1481-1482; 1483-1484; 1485-1486; 1487-1488; 1489-1490; 1491-1492; 1493-1494; 1495-1496; 1497-1498; 1499-1500; 1501-1502; 1503-1504; 1505-1506; 1507-1508; 1509-1510; 1511-1512; 1513-1514; 1515-1516; 1517-1518; 1519-1520; 1521-1522; 1523-1524; 1525-1526; 1527-1528; 1529-1530; 1531-1532; 1533-1534; 1535-1536; 1537-1538; 1539-1540; 1541-1542; 1543-1544; 1545-1546; 1547-1548; 1549-1550; 1551-1552; 1553-1554; 1555-1556; 1557-1558; 1559-1560; 1561-1562; 1563-1564; 1565-1566; 1567-1568; 1569-1570; 1571-1572; 1573-1574; 1575-1576; 1577-1578; 1579-1580; 1581-1582; 1583-1584; 1585-1586; 1587-1588; 1589-1590; 1591-1592; 1593-1594; 1595-1596; 1597-1598; 1599-1600; 1601-1602; 1603-1604; 1605-1606; 1607-1608; 1609-1610; 1611-1612; 1613-1614; 1615-1616; 1617-1618; 1619-1620; 1621-1622; 1623-1624; 1625-1626; 1627-1628; 1629-1630; 1631-1632; 1633-1634; 1635-1636; 1637-1638; 1639-1640; 1641-1642; 1643-1644; 1645-1646; 1647-1648; 1649-1650; 1651-1652; 1653-1654; 1655-1656; 1657-1658; 1659-1660; 1661-1662; 1663-1664; 1665-1666; 1667-1668; 1669-1670; 1671-1672; 1673-1674; 1675-1676; 1677-1678; 1679-1680; 1681-1682; 1683-1684; 1685-1686; 1687-1688; 1689-1690; 1691-1692; 1693-1694; 1695-1696; 1697-1698; 1699-1700; 1701-1702; 1703-1704; 1705-1706; 1707-1708; 1709-1710; 1711-1712; 1713-1714; 1715-1716; 1717-1718; 1719-1720; 1721-1722; 1723-1724; 1725-1726; 1727-1728; 1729-1730; 1731-1732; 1733-1734; 1735-1736; 1737-1738; 1739-1740; 1741-1742; 1743-1744; 1745-1746; 1747-1748; 1749-1750; 1751-1752; 1753-1754; 1755-1756; 1757-1758; 1759-1760; 1761-1762; 1763-1764; 1765-1766; 1767-1768; 1769-1770; 1771-1772; 1773-1774; 1775-1776; 1777-1778; 1779-1780; 1781-1782; 1783-1784; 1785-1786; 1787-1788; 1789-1790; 1791-1792; 1793-1794; 1795-1796; 1797-1798; 1799-1800; 1801-1802; 1803-1804; 1805-1806; 1807-1808; 1809-1810; 1811-1812; 1813-1814; 1815-1816.

The following examples are not limitative.

**Example 1: General amplification α5, α6, α7 and α10 HPVs**

We developed a NGS test for quantifying oncogenic HPV E7 mRNAs relative their respective L1 mRNAs. We searched and designed consensus primers in this regard for a quantitative pre-amplification of the oncogenic E7 HPVs mRNAs of all alpha papillomavirus (high and low risk).

We completed the test with the identification of L1 mRNA level and determine the ratio between the expression E7 (early gene) and L1 (late gene) to determine the risk of a patient developing cancer. This new test allows determining the inherent risk of any HPVs types regardless of the current classification regardless of whether the patient is infected with HPV species 16 or 18, etc. (HR) or 30...etc. (BR).

Furthermore, this assay allows identifications of multiple HPV infections in single individuals.

First, we analyzed the feasibility of generating consensus sequences for different HPVs type (HR and LR).

We started to determine the percentage protein homology of the different HPVs Type, using NCBI Blastn. We observed that a high heterogeneity exists between the gene sequence of E7 from one type of HPV to another even among HR species (FIGURE 1, 2, 3, 4, 5, 6). This observation did not reveal obvious features in sequences of HPVs explaining the consensus designing partners by current classification as LR or HR. this observation reinforces the hypothesis of a lack of correlation between sequence of a whole E7 gene and the classification based on the risk: this impair a possibility to derive tests based on consensus sequence to differentiate HT=R and LR.

Then, we did a sequence alignment based on oncogenic E7 HPVs HR and LR.

We focus on global comparison of HR and LR, then only within HR and finally only within LR. We observed that no specific consensus sequence emerged from the sequence alignment of HR, HR and LR / LR overall on E7 genes species. There is very little global sequence homology, the locus of homology being very punctual.

Nonetheless, we were led to design consensus primers based on subgroup α for E7 using ClustalW for alignments. We found some homologies between HPVs types sequences contained in a subgroup. After having generated all the FASTA files alignment, we searched to generate consensus sequences using GEMI program. Sometimes, we couldn't find any consensus sequences in all the HPV subgroup. We divided the subgroup to generate the degenerated consensus sequences. We then selected more particularly combined primers which cover all subgroup parameters such as minimizing the number of required primers selecting primers to obtain the largest possible amplicons, further selecting primers for relative conserved 3' sequences and selecting primers which do not cross hybridize.

We did these selections for all the E7 and L1 HPVs subgroup. After analyzing all the sequences we elected the best set of primers for α5, α6, α7 and α10 E7 and α5, α6, α7 and α10 L1 to amplify all the genes.

Finally, we provide here a new diagnosis test comprising a set of probes for the pre amplification of E7 α5, α6, α7 and α10 HPVs mRNAs and L1 α5, α6, α7 and α10 HPVs mRNAs level and which allows to assess the ratio between the expression of E7 (early gene) and L1 (late gene) as a marker of the risk for a patient developing cancer.

One preferred set of primers for the pre-amplification comprises the following sequences:

α5:

- E7

  Forward: 5'- YTAGATYTGGTGCCGCAACCCG -3' (SEQ ID NO. 49)
  Forward: 5'- MGCCATGCGTGGTAATGTACCAC -3' (SEQ ID NO. 50)
  Reverse: 3'- CTCCASCRCTCGRACGTTCTGT -5' (SEQ ID NO. 51)
  Reverse: 3'- CACGGGCAMACCAGGCTTAGK -5' (SEQ ID NO. 52)

- L1

  Forward: 5'- KCAGATGGCYTTGYGGCGTACTA -3' (SEQ ID NO. 53)
  Reverse: 3'- GGGGCRTYRCGYTGACAKGTAGT -5'(SEQ ID NO. 56)
  Reverse: 3'- GGCMGGSCKTTTAAGGCCTGGT -5'(SEQ ID NO. 57)

α6:

- E7

  Forward: 5'- GCTCAGAGGAWGAGGATGAGG -3'(5EQ ID NO. 64)
  Reverse: 3'- GCCTTGTTGCRCASAGGGG -5'(SEQ ID NO. 69)
  Reverse: 3'- CGCAGAGTGGGCACGTTACT -5'(SEQ ID NO. 70)

- L1

  Forward: 5'- TTGCAGATGGCGRYGTGGCG -3'(SEQ ID NO. 71)
  Reverse: 3'- CACCTAAAGGYTGDCCDCGGC -5'(SEQ ID NO. 72)

α7:

- E7

  Forward: 5'- GACGRGMHGAACMACARCGTCAC -3'(SEQ ID NO. 82)
  Reverse: 3'- GTGWSTCCATAAACAGCWGCWGT -5'(SEQ ID NO. 85)
  Reverse: 3'- CACACCAMGGACACACAAAGGAC -5'(SEQ ID NO. 86)

- L1

  Forward: 5'- GCGBTCTAGYGACARCAHGGTGT -3'(SEQ ID NO. 87)
  Forward: 5'- HCCTGCTATTGGKGARCAYTGGG -3' (SEQ ID NO. 88)
  Reverse: 3'- CCAGTGYTCYCCMATRGCRGGWA -5' (SEQ ID NO. 89)

Reverse: 3'- TAGASCCACTDGGWGANGGRGAA -5' (SEQ ID NO. 90)

α10:

- E7

Forward: 5'- GCWCAYTWGGAATHGTGTGCCCC -3' (SEQ ID NO. 122)
Forward: 5'- CSTGTAAMAACGCCATGAGAGGA -3'(5EQ ID NO. 123)
Forward: 5'- CGCCATGAGAGGAMACAASCCA -3' (SEQ ID NO. 124)
Reverse: 3'- GGCACACDATTCCWARTGWGCCC -5' (SEQ ID NO. 125)
Reverse: 3'- GGTTCGTASGTCRSTTGYTGTAC -5' (SEQ ID NO. 126)
Reverse: 3'- GTGCACAGSYGGGRCACACWAYT -5'(SEQ ID NO. 127)

- L1

Forward: 5'- GARGCCACWGTSTACYTGCCTC -3' (SEQ ID NO. 128)
Forward: 5'- ACAGATGTCTCTGTGGCGGC -3' (SEQ ID NO. 129)
Reverse: 3'- GGATGNCCACTWAYRCCHACDCC -5' (SEQ ID NO. 130)

**Example 2: Quantifying E6 and E7 reads versus other viral reads**

A sample of cells is collected from the cervix using a spatula or small brush and put in a conservative solution. RNAs are extracted from the cells using standard procedure and polyA mRNAs are selected using standard procedures like using poly dT beads. Librairies are prepared using standard library preparation (RNA fragmentation and reverse transcription into double-stranded complementary DNA primed by random hexamer followed by adapter selection, or reverse transcription to single strand cDNA, ligation of cDNA and random amplification by phi 29 polymerase followed by fragmentation and adpaterligation). Alternatively RT-PCR is conducted using set of primers for E6 and E7 and at least one another late gene as described. After sequencing using several million reads of at least 100nt, reads are mapped on a database of E6 and E7 genes: HPV genotypes expressing E6/E7 are identified. The other reads are mapped on the subset of genomes corresponding to the corresponding genotypes. Within each genotype, ratio of the number of E6/E7 reads to the reads mapped to at least one anther gene is calculated and compared to thresholds.

**Example 3: Biological samples**

Two high grade lesions (HSIL) samples of the cervix from two donor women, hereinafter referred to as 117 and 119, were collected in PreservCyt medium (Hologic) and kept at room temperature for a couple of days. After homogenization, 1mL aliquots were collected from the 20μL total liquid medium for HPV genotyping (Papillocheck, Greiner Bio-One). Results of HPV typing are given in table 1. The remaining samples were centrifuged at 4,500xg for 10 min and the pellets were stabilized in 1mL RNAProtect Cell (Qiagen) for storage at -80°C before RNA extraction.

**Example 4: HPV database**

Sixty four (64) reference sequences representing the entire HPV alpha genus were retrieved from the International Human Papillomavirus Reference Center (; updated May 2014). Additional nine (9) sublineage sequences corresponding to HPV16, plus nine (9) sublineage sequences corresponding to HPV18 (described in Burk et al. Virology 2013) were added. The resulting eighty two (82) HPV genomes (listed in table 2) were aligned using ClustalW2 (default parameters) and the output file was analyzed using the Geneious software (Geneious 7.1.5, Biomatters Ldt).

**Example 5: Design of HPV reverse transcription primers**

A dedicated strategy for the design of HPV reverse transcription (RT)-primers was set up with the goal to carry out a specific enrichment of HVP sequences during the reverse transcription step within a ballast of viral and non-viral RNA sequences. The overall approach consists in targeting the entire early and late populations of HPV transcripts starting from a limited number of specific RT primers. The design is achieved by taking advantage of the sequence shared by all early and late transcripts, located in the 5' vicinity of the early and late polyA signals, respectively (figure 1). In order to minimize the number of RT primers required to cover all kind of alpha HPV, the degree of similarity between the 82 HPV genomes was taken into account. Additional criteria for the design of RT primers were as follows: (i) an overall good specificity of the primer aligned against all existing sequences databases (BLAST NCBI) with special attention paid in considering the 3' part of the primer, (ii) a GC content around 50% (+/-12%), (iii) a melting temperature (Tm) > 50°C (assuming 0,2μM primers and 50mM salt), (iv) no T tracts, (v) no or low GC content in the 3' part of the primer and (vi) no or limited number of putative secondary structures. This approach was implemented manually as a proof of principle to design one (1) RT-specific primer targeting the early transcripts of the 9 HPV16 sequences, one (1) RT-specific primer targeting the late transcripts of the 9 HPV16 sequences, two (2) RT-specific primers targeting the early transcripts of the 9 HPV18 sequences and two (2) RT-specific primers targeting the late transcripts of the 9 HPV18 sequences. The resulting six (6) RT-specific primers targeting both HPV16 and HPV18 sequences and including sublineages are given in table 3. HPSF-purified primers (0.01 μmol) were ordered at Eurofins genomics (http://www.eurofinsgenomics.eu).

**Example 6: RNA extraction and characterization**

Total RNA from samples 117 and 119 were extracted using the PicoPure RNA isolation kit (Life Technologies), adding a DNAse treatment step directly on column (RNAse-free DNAse set, Qiagen) as recommended by the supplier. Elution was achieved in 30μL elution buffer. Assessment of RNA quantity and quality was done with a Nanodrop 1000 (Thermo Scientist) and a Bioanalyzer 2100 using the RNA Nano chips (Agilent).

**Example 7: Random reverse transcription**

Random reverse transcription of total RNA was carried out using the SuperScript III First-Strand cDNA Synthesis kit (Invitrogen). Briefly, 8μL of total RNA was used for template and the reaction was performed in the presence of 50nM random hexamers (provided by Invitrogen), incubated 10min at 25°C, 50min at 50°C and 5min at 85°C before a final RNAse H treatment 20min at 37°C. The resulting cDNA were stored at -20°C.

**Example 8: HPV-specific reverse transcription**

HPV-specific reverse transcription was carried out using the SuperScript III First-Strand cDNA Synthesis kit (Invitrogen) and primed with the HPV-specific RT-primers described above. Briefly, 8μL of total RNA was used for template and reaction was performed with a 0,2μM mixture of the 6 HPV-specific RT primers, incubated 50min at 50°C and 5min at 85°C before a final RNAse H treatment 20min at 37°C. The resulting cDNA were stored at -20°C.

**Example 9: Control PCR**

The HPV16 E7 and the human cellular beta-actin (ACTB) genes were used as controls of the random and HPV-specific reverse-transcription steps, respectively. 1μL of reverse-transcribed cDNA was used as PCR templates in 20μL final volume, working with LightCycler DNA Master SybrGreen I reagents (Roche Diagnostics). 45 amplification cycles were achieved on a Light Cycler 480 (Roche) as follows: 95°C 10sec, 56°C 10sec, 72°C 30sec. Fusion curves and electrophoresis gels served for validation. A comparison of Ct values obtained by following either the random RT or the HPV RT protocol is given in table 4.

**Example 10: Whole transcriptome random amplification**

cDNA were randomly amplified using the Multiple Displacement Amplification (MDA) protocol with phi29 polymerase and random hexamers (Whole Transcriptome Amplification, Qiagen). Phi 29 was UV-treated for one hour before use, in order to prevent any residual DNA contaminant.

**Example 11: High throughput sequencing**

Samples 117 (both random RT and HPV-specific RT) and 119 (both random RT and HPV-specific RT) were independently analyzed on two sequencing runs (300bp paired-end sequencing, TruSeq PCR-free library prep, 600 cycle kit) on a MiSeq apparatus (Illumina). fastQ data were generated and QC tests done following standard procedures. Total numbers of sequencing reads per sample are summarized in table 5.

**Example 12: Data analysis**

Quality-filtered reads were mapped to reference sequences using the following criteria: (i) alignment identity of at least 90% and (ii) Smith and Waterman score above 100. A selection of 10 human genes served as cellular controls (table 6). The analysis of the reads mapping HPV sequences relied on two strategies: first, at the genomic level, sequencing reads mapping HPV16 (NC_001526.2), HPV6 (HG793939.1) and HPV35 (JX129488.1) were count for each coding sequence (CDS), without adding any particular filter (table 7). In a second and more transcript-specific approach, reads mapping splice junctions of HPV16 were identified (table 8). This latter analysis was done for HPV16 only as a proof of principle, and relied on well-documented donor and acceptor splice sites, as described for example in Zheng et al. FrontBiosci 2006.

**Example 13: Sequence results**

Following the random reverse-transcription protocol, the sequencing of patient 117 resulted in a total of 1,455 and 126 reads (over 34,977,682) that were successfully mapped to the HPV16 (NC_001526.2) and HPV35 (JX129488.1) genomes, respectively. 15 reads (over 39675490) were mapped to the HPV16 genome for the mono-infected patient 119. Following the alternative procedure with HPV-specific RT primers, the sequencing of patient 117 resulted in a total of 2033, 69 and 14 reads (over 28598603) for HPV16, HPV35 and HPV6 genomes, respectively. 6 reads (over 19383833) were mapped to the HPV16 genome for patient 119.

Two lines of analysis were conducted in order to characterize finely different populations of HPV reads. First, at the genomic level, reads mapping CDS regions were counted, giving a broad view of phenomena such as the early vs late genes equilibrium (table 7). In addition to that, we sought to characterize deeply specific HPV16 transcripts by taking advantage of well-documented donor and acceptor splice sites described for HPV16. This led us to define 11 spliced transcripts which can be associated unambiguously to one specific RNA event (table 8). Together, these two analysis showed that (i) HPV sequences are reachable using HTS, (ii) it is possible to perform a gene-by-gene reads counting at the genomic level, (iii) reads associated to specific splice junctions exist and can be characterized and counted as well, confirming essentially the detection of transcripts over possible artefacts introduced by residual HPV DNA, (iv) discrepancies exist between samples, between HPV genus and between HPV genes and transcripts patterns, which reflect probably the diversity of HPV infections.

**Example 14: Examples of R scores**

These observations opened the possibly to define a score, referred to as R score, based on HPV CDS counts and/or specific transcripts within each genotype present in a given sample , to gain a fine molecular characterization of any individual HPV-positive samples. From this perspective, either one value or a combination of more than one ratio(s) could be considered. A non-restrictive list of R scores is given in table 9 in order to illustrate several possible combinations based either on CDS or specific transcripts. As an example, R scores based on a ratio E6 and/or E7 and/or E2 and/or L1 and/or L2 succeeded in generating high score values (highlighted) that should be associated of non- or lowly-productive HPV cycles typical of transformed cells. Of note, weighting coefficients such as $\alpha$E6 and/or $\beta$E7 and/or $\gamma$E2 and/or $\delta$L1 and/or $\varepsilon$L2 can be added as parameters, independently, in order to better discriminate, for instance, low risk and high risk lesions.

**Example 15: Random RT vs HPV-specific RT**

As an alternative to the conventional random RT upstream of random amplification, we attempted to define and to use HPV-specific RT primers, with the ultimate goal to achieve a specific HPV enrichment over non-HPV sequences. Such targeted (semi-random) approach may prove extremely important in the perspective of reducing the depth sequencing (that is dependent on the ratio of HPV to non HPV sequences), increasing multiplexing, and reducing costs required before being able to use HTS as a screening test. Although the number of HPV reads remains roughly comparable between the random RT and the HPV-specific RT approaches, a marked difference was observed regarding cellular genes, as exemplified by both PCR (table 4, average ACTB $\Delta$Ct -3,01 for HPV RT compared to E7 $\Delta$Ct -0,56) and HTS results (table 6, average 3,3 fold reduction for HPV RT, after total reads number correction). In addition to that, HPV6 reads were detectable in the poly-infected sample 117 only when applying the HPV RT approach, thus recovering the results of the Papillocheck gold standard genotyping test. These results, albeit based on a limited number of experimental evidences, suggest *a minima* that our innovative HPV-specific reverse transcription approach coupled with random amplification is able to reduce the cellular and other non-HPV ballasts, without deteriorate the detection of specific HPV targets. Optimizations of the technique are now required to achieved a strong HPV enrichment and to afford linear quantification.

**Table 1: Biological samples and associated HPV-genotyping**

| Sample | Year of birth | Lesion | HPV typing (Papillocheck) |
|---|---|---|---|
| 117 | 1969 | High grade (HSIL) | 6, 16, 35 |

(continued)

| Sample | Year of birth | Lesion | HPV typing (Papillocheck) |
|---|---|---|---|
| 119 | 1986 | High grade (HSIL) | 16 |

**Table 2:**

| Seq index | Virus name | Genus name | Species name | GenBank ID | HR α HPV (x) |
|---|---|---|---|---|---|
| 1 | HPV2 | Alpha | Alpha-4 | X55964 | |
| 2 | HPV3 | Alpha | Alpha-2 | X74462 | |
| 3 | HPV6 | Alpha | Alpha-10 | X00203 | |
| 4 | HPV7 | Alpha | Alpha-8 | X74463 | |
| 5 | HPV10 | Alpha | Alpha-2 | X74465 | |
| 6 | HPV11 | Alpha | Alpha-10 | M14119 | |
| 7 | HPV13 | Alpha | Alpha-10 | X62843 | |
| 8 | HPV16 | Alpha | Alpha-9 | K02718 | |
| 9 | HPV16 | Alpha | Alpha-9 | AF536179 | |
| 10 | HPV16 | Alpha | Alpha-9 | HQ644236 | |
| 11 | HPV16 | Alpha | Alpha-9 | AF534061 | |
| 12 | HPV16 | Alpha | Alpha-9 | AF536180 | |
| 13 | HPV16 | Alpha | Alpha-9 | HQ644298 | |
| 14 | HPV16 | Alpha | Alpha-9 | AF472509 | |
| 15 | HPV16 | Alpha | Alpha-9 | HQ644257 | |
| 16 | HPV16 | Alpha | Alpha-9 | AY686579 | x |
| 17 | HPV16 | Alpha | Alpha-9 | AF402678 | |
| 18 | HPV18 | Alpha | Alpha-7 | X05015 | x |
| 19 | HPV18 | Alpha | Alpha-7 | AY262282 | |
| 20 | HPV18 | Alpha | Alpha-7 | EF202146 | |
| 21 | HPV18 | Alpha | Alpha-7 | EF202147 | |
| 22 | HPV18 | Alpha | Alpha-7 | EF202151 | |
| 23 | HPV18 | Alpha | Alpha-7 | GQ180787 | |
| 24 | HPV18 | Alpha | Alpha-7 | EF202155 | |
| 25 | HPV18 | Alpha | Alpha-7 | KC470225 | |
| 26 | HPV18 | Alpha | Alpha-7 | EF202152 | |
| 27 | HPV18 | Alpha | Alpha-7 | KC470229 | |
| 28 | HPV26 | Alpha | Alpha-5 | X74472 | |
| 29 | HPV27 | Alpha | Alpha-4 | X74473 | |
| 30 | HPV28 | Alpha | Alpha-2 | U31783 | |
| 31 | HPV29 | Alpha | Alpha-2 | U31784 | x |
| 32 | HPV30 | Alpha | Alpha-6 | X74474 | |
| 33 | HPV31 | Alpha | Alpha-9 | J04353 | x |

(continued)

| Seq index | Virus name | Genus name | Species name | GenBank ID | HR α HPV (x) |
|---|---|---|---|---|---|
| 34 | HPV32 | Alpha | Alpha-1 | X74475 | |
| 35 | HPV33 | Alpha | Alpha-9 | M12732 | X |
| 36 | HPV34 | Alpha | Alpha-11 | X74476 | |
| 37 | HPV35 | Alpha | Alpha-9 | X74477 | |
| 38 | HPV39 | Alpha | Alpha-7 | M62849 | |
| 39 | HPV40 | Alpha | Alpha-8 | X74478 | x |
| 40 | HPV42 | Alpha | Alpha-1 | M73236 | |
| 41 | HPV43 | Alpha | Alpha-8 | AJ620205 | |
| 42 | HPV44 | Alpha | Alpha-10 | U31788 | |
| 43 | HPV45 | Alpha | Alpha-7 | X74479 | |
| 44 | HPV51 | Alpha | Alpha-5 | M62877 | x |
| 45 | HPV52 | Alpha | Alpha-9 | X74481 | |
| 46 | HPV53 | Alpha | Alpha-6 | X74482 | |
| 47 | HPV54 | Alpha | Alpha-13 | U37488 | |
| 48 | HPV56 | Alpha | Alpha-6 | X74483 | |
| 49 | HPV57 | Alpha | Alpha-4 | X55965 | |
| 50 | HPV58 | Alpha | Alpha-9 | D90400 | |
| 51 | HPV59 | Alpha | Alpha-7 | X77858 | x |
| 52 | HPV61 | Alpha | Alpha-3 | U31793 | x |
| 53 | HPV62 | Alpha | Alpha-3 | AY395706 | |
| 54 | HPV66 | Alpha | Alpha-6 | U31794 | |
| 55 | HPV67 | Alpha | Alpha-9 | D21208 | |
| 56 | HPV68 | Alpha | Alpha-7 | X67161 | x |
| 57 | HPV69 | Alpha | Alpha-5 | AB027020 | |
| 58 | HPV70 | Alpha | Alpha-7 | U21941 | x |
| 59 | HPV71 | Alpha | Alpha-14 | AB040456 | x |
| 60 | HPV72 | Alpha | Alpha-3 | X94164 | |
| 61 | HPV73 | Alpha | Alpha-11 | X94165 | |
| 62 | HPV74 | Alpha | Alpha-10 | AF436130 | |
| 63 | HPV77 | Alpha | Alpha-2 | Y15175 | |
| 64 | HPV78 | Alpha | Alpha-2 | AB793779 | |
| 65 | HPV81 | Alpha | Alpha-3 | AJ620209 | |
| 66 | HPV82 | Alpha | Alpha-5 | AB027021 | x |
| 67 | HPV83 | Alpha | Alpha-3 | AF151983 | |
| 68 | HPV84 | Alpha | Alpha-3 | AF293960 | x |
| 69 | HPV85 | Alpha | Alpha-7 | AF131950 | |
| 70 | HPV86 | Alpha | Alpha-3 | AF349909 | |
| 71 | HPV87 | Alpha | Alpha-3 | AJ400628 | |

(continued)

| Seq index | Virus name | Genus name | Species name | GenBank ID | HR α HPV (x) |
|---|---|---|---|---|---|
| 72 | HPV89 | Alpha | Alpha-3 | AF436128 | |
| 73 | HPV90 | Alpha | Alpha-14 | AY057438 | x |
| 74 | HPV91 | Alpha | Alpha-8 | AF419318 | |
| 75 | HPV94 | Alpha | Alpha-2 | AJ620211 | |
| 76 | HPV97 | Alpha | Alpha-7 | DQ080080 | |
| 77 | HPV102 | Alpha | Alpha-3 | DQ080083 | |
| 78 | HPV106 | Alpha | Alpha-14 | DQ080082 | |
| 79 | HPV114 | Alpha | Alpha-3 | GQ244463 | |
| 80 | HPV117 | Alpha | Alpha-2 | GQ246950 | |
| 81 | HPV125 | Alpha | Alpha-2 | FN547152 | |
| 82 | HPV160 | Alpha | Alpha-2 | AB745694 | x |

**Table 3: Primers used for HPV-specific reverse transcription**

| Primer name | Sequence (5'->3') | Length (bp) | %GC | Tm | SEQ ID NO. |
|---|---|---|---|---|---|
| HPV16-early | CAGCGGACGTATTAATAGG | 19 | 47 | 54.5 | **SEQ ID NO.153** |
| HPV16-late | TCATATTCCTCCCCATGTC | 19 | 47 | 54.5 | **SEQ ID NO.154** |
| HPV18-early-pop1 | AGGGGACGTTATTACCAC | 18 | 50 | 53.7 | **SEQ ID NO.155** |
| HPV18-early-pop2 | CAGGGGACGTTATTATCAC | 19 | 47 | 54.5 | **SEQ ID NO.156** |
| HPV18-late-pop1 | ATATTCCTCAACATGTCTGC | 20 | 40 | 53.2 | **SEQ ID NO.157** |
| HPV18-late-pop2 | CATATTCTTCAACATGTCTGC | 21 | 38 | 54.0 | **SEQ ID NO.158** |

**Table 4: Comparative Ct values obtained by PCR after random or HPV-specific reverse transcription**

| | Human ACTB | | | E7 HPV16 | | |
|---|---|---|---|---|---|---|
| | Random RT | HPV RT | ΔCt | Random RT | HPV RT | ΔCt |
| Sample 117 | 26.69 | 29.48 | -2.79 | 37.23 | 37.35 | -0.12 |
| Sample 119 | 23.97 | 27.20 | -3.23 | 35.25 | 36.25 | -1.00 |

**Table 5: Total number of sequencing reads**

| | Total reads # (raw) | Quality Filtering |
|---|---|---|
| **Sample 117 random RT** | 37,055,284 | 34,977,682 |
| **Sample 117 HPV RT** | 30,607,370 | 28,598,603 |
| **Sample 119 random RT** | 41,994,892 | 39,675,490 |
| **Sample 119 HPV RT** | 20,462,884 | 19,383,833 |

**Table 6: Number of sequencing reads mapping human cellular genes (GRCh37) after random or HPV-specific reverse transcription**

| | ACTB | GAPDH | G6PD | HPRT1 | RPLP0 | GUSB | PPIA | KRT19 | CDKN2A | MKI67P1 | TOTAL |
|---|---|---|---|---|---|---|---|---|---|---|---|
| **Sample 117 random RT** | 731 | 7 | 1 | 32 | 0 | 4 | 4 | 10 | 5 | 0 | **794** |
| **Sample 117 HPV RT** | 86 | 0 | 0 | 13 | 2 | 2 | 2 | 5 | 11 | 0 | **121** |
| **Sample 119 random RT** | 546 | 19 | 9 | 183 | 38 | 23 | 50 | 57 | 96 | 0 | **1,021** |
| **Sample 119 HPV RT** | 47 | 5 | 28 | 146 | 6 | 17 | 50 | 0 | 73 | 12 | **384** |

**Table 7: Number of sequencing reads mapping HPV CDS**

| HPV CDS | Sample 117 | | | | | | Sample 119 | |
|---|---|---|---|---|---|---|---|---|
| | HPV16 | | HPV35 | | HPV6 | | HPV16 | |
| | Rand. RT | HPV RT | Rand. RT | HPV RT | Rand. RT | HPV RT | Rand. RT | HPV RT |
| **E6** | 162 | 121 | 0 | 0 | 0 | 0 | 6 | 2 |
| **E7** | 91 | 91 | 7 | 0 | 0 | 14 | 9 | 0 |
| **E1** | 585 | 862 | 30 | 7 | 0 | 13 | 3 | 3 |
| **E2** | 366 | 684 | 22 | 0 | 0 | 0 | 1 | 0 |
| **E4** | 29 | 33 | 0 | 0 | 0 | 0 | 0 | 0 |
| **E5** | 71 | 70 | 0 | 0 | 0 | 0 | 1 | 0 |
| **L2** | 248 | 211 | 12 | 11 | 0 | 0 | 0 | 0 |
| **L1** | 305 | 535 | 24 | 47 | 0 | 0 | 0 | 1 |

**Table 8: Number of sequencing reads mapping spliced HPV16 transcripts**

| HPV16 splice transcripts | Genomic coordinates of HPV16 splice sites (NC_001526.2) | | Sample 117 Number of reads at the splice junction | | Sample 119 Number of reads at the splice junction | |
|---|---|---|---|---|---|---|
| | Spl. Donor | Spl. Accep. | Rand. RT | HPV RT | Rand. RT | HPV RT |
| E6*I | 226 | 409 | 33 | 25 | 0 | 0 |
| E6*II | 226 | 526 | 4 | 0 | 0 | 0 |
| E6*III, E5 | 226 | 3358 | 0 | 0 | 0 | 0 |
| E6*IV | 226 | 2709 | 36 | 23 | 0 | 0 |
| E6^E7 | 226 | 742 | 0 | 0 | 0 | 0 |
| E1C | 880 | 2582 | 0 | 0 | 0 | 0 |
| E1^E4 | 880 | 3358 | 12 | 4 | 0 | 0 |
| E2 | 880 | 2709 | 0 | 2 | 0 | 0 |
| E2C | 1302 | 3358 | 0 | 0 | 0 | 0 |
| L1 | 3632 | 5639 | 0 | 0 | 0 | 0 |
| L1* | 1302 | 5639 | 0 | 0 | 0 | 0 |

**Table 9: Examples of R scores established either from reads mapping CDS or specific transcripts**

| R score (examples) | Sample 117 | | | | | | Sample 119 | |
|---|---|---|---|---|---|---|---|---|
| | HPV16 | | HPV35 | | HPV6 | | HPV16 | |
| | Rand. RT | HPV RT | Rand. RT | HPV RT | Rand. RT | HPV RT | Rand. RT | HPV RT |
| **Genome (CDS)** | | | | | | | | |
| E6/L1 | 0,53 | 0,23 | 0,00 | 0,00 | +∞ | +∞ | +∞ | 2,00 |
| E7/L1 | 0,30 | 0,17 | 0,29 | 0,00 | +∞ | +∞ | +∞ | 0,00 |
| (E6+E7)/L1 | 0,83 | 0,40 | 0,29 | 0,00 | +∞ | +∞ | +∞ | 2,00 |
| (E6+E7)/(L1+L2) | 0,46 | 0,28 | 0,19 | 0,00 | +∞ | +∞ | +∞ | 2,00 |
| (E6+E7)/E2 | 0,69 | 0,31 | 0,32 | +∞ | +∞ | +∞ | 15,00 | +∞ |
| (E6+E7)/(E2*(L1+L2)) | 0,00 | 0,00 | 0,01 | +∞ | +∞ | +∞ | +∞ | +∞ |
| (E6+E7)/(E2+E4) | 0,64 | 0,30 | 0,32 | +∞ | +∞ | +∞ | 15,00 | +∞ |
| (E6+E7)/(E6+E7+L1+L2) | 0,31 | 0,22 | 0,16 | 0,00 | +∞ | 1,00 | 1,00 | 0,67 |
| (E6+E7+E2-E4)/(L1+L2) | 1,07 | 1,16 | 0,81 | 0,00 | +∞ | +∞ | +∞ | 2,00 |
| (E6+E7+E1+E2+E4)/(L1+L2) | 2,36 | 2,49 | 1,64 | 0,12 | +∞ | +∞ | +∞ | 5,00 |
| (E6+E7)/(E6+E7+E2+L1+L2) * E2/(E6+E7+E2+L1+L2) | 0,07 | 0,05 | 0,04 | 0,00 | +∞ | 0,00 | 0,06 | 0,00 |
| **Transcripts (splice junctions)** | | | | | | | | |
| E6*I/(L1+L1*) | +∞ | +∞ | no data | | no data | | not applicable (no reads) | |
| E6*II/(L1+L1*) | +∞ | +∞ | | | | | | |
| E6*III, E5/(L1+L1*) | +∞ | +∞ | | | | | | |
| E6*IV/(L1+L1*) | +∞ | +∞ | | | | | | |
| E6^7/(L1+L1*) | +∞ | +∞ | | | | | | |
| (E6*I+E6*II+E6*III, E5+E6*IV+E6^7) /(L1+L1*) | +∞ | +∞ | | | | | | |
| (E6*I+E6*II+E6*III, E5+E6*IV+E6^7) /(E2+E2C) | +∞ | 24,00 | | | | | | |
| (E6*I+E6*II+E6*III, E5+E6*IV+E6^7) /(E2+E2C+E1^E4) | 6,08 | 8,00 | | | | | | |
| (E6*I+E6*II+E6*III, E5+E6*IV+E6^7) /((E2+E2C)*(L1+L1*)) | +∞ | +∞ | | | | | | |
| (E6*I+E6*II+E6*III, E5+E6*IV+E6^7) /(E6*I+E6*II+E6*III, E5+E6*IV+E6^7+L1+L1*) | 1,00 | 1,00 | | | | | | |
| (E6*I+E6*II+E6*III, E5+E6*IV+E6^7+E2+E2C-E1^E4) /(L1+L1*) | +∞ | +∞ | | | | | | |

### Example 16: Summary of Examples 1-15

The method according to the present invention described in the Examples above comprises:

1. Extraction of viral RNAs (Example 6) from a biological sample (Example 3),
2. Reverse transcription of the RNAs into cDNA with random hexamers (Example 7) or primers specific for HPV (Example 8); the design of the primers being illustrated by Example 1 (consensus primer) and Example 5 (HPV16 and HPV18 specific primers). A cDNA quality control is carried out by quantitative PCR (Example 9).
3. Amplification of cDNA by MDA technology with random hexamers (Example 10) to generate a DNA sequence bank (Example 2),
4. High throughput sequencing of the DNA bank and generation of "sequencing reads" (Example 11),
5. Aligning reads (Example 12) with the sequences of the HPV genomes present in the database (Example 5). Two analytical strategies are possible (Example 12, results in Example 13):

   a. counting reads aligning with each CDS of interest, or
   b. enumeration of reads aligning only the known splice junctions of each CDS of interest.;

6. Computing R score (Example 14) whose the different possible computings are ratios described in Table 9. The ratio is defined as the ratio between the number of reads generated for at least 2 genes described in the present patent application.

### Example 17: Detection and quantification of HPV16 and human transcripts

#### 17.1 HPV database

Sixty four (64) genomic sequences representing the HPV alpha genus were retrieved from the International Human Papillomavirus Reference Center (http://www.hpvcenter.se/index.html; updated May 2014). Additional nine (9) sublineage sequences corresponding to HPV16, plus nine (9) sublineage sequences corresponding to HPV18 (described in Burk et al. Virology 2013) were added. The resulting eighty two (82) HPV genomes are referred to as the αHPV database (Table 2 above). A subgroup of the αHPV database composed of sixteen (16) sequences (16, 18, 31, 33, 35, 39, 45, 51, 52, 56, 58, 59, 66, 68, 73 and 82), corresponding to high risk or putative high risk Papillomaviruses, is referred to as the HR αHPV group.

**17.2 Delineation of transcription maps for HR αHPV**

For each sequences of the HR αHPV group, known and putative/predicted splice donor (SD) and splice acceptor (SA) sites were annotated. First, previously documented SD and SA sites for HPV16 and HPV18 were retrieved from Zheng et al. Front Biosci. 2006, Wang et al. Journal of Virology 2011 and Toots et al. PLoS ONE 2014 (Figure 7, light grey). Based on the idea that virtually all spliced sites are analogous to those previously described for other papillomaviruses (Wang et al. Journal of Virology 2011), the annotation of SA and SD was then expanded to the entire αHPV group by direct analogy (Figure 7, black numbers). In addition, SA/SD predictions were supported by online splice site prediction software.

**17.3 Transcripts database and primers design**

A database of short (150 to 500 bp) sequences reconstructed 5' from the splice donor site and 3'from the splice acceptor site was generated, gathering each transcript, part of transcript, or putative transcript for each HR αHPV, and is referred to as the αHPV transcripts database. This database served as a basis for the design of PCR primers. More precisely, a pair of primers was defined for each transcript, part of transcript or putative transcript when possible, with the objective of encompassing the splice junction, as defined in Figure 7 (the corresponding transcripts appear with suffix 'sp_'in Table 10).

**Table 10: Primer pairs for HPV16 and human transcripts**

| | Transcript targeted | Forward primer | SEQ ID NO. | Reverse primer | SEQ ID NO. |
|---|---|---|---|---|---|
| Human transcripts | ACTB.E4E5 | CCAGGTCATCACCATTGGCAAT | 159 | CGTACAGGTCTTTGCGGATGT | 160 |
| | AKT1.E2E3 | CCATGAGCGACGTGGCTATT | 161 | CTCACGTTGGTCCACATCCT | 162 |
| | B2M.E1E2 | CTGTGCTCGCGCTACTCT | 163 | CAACTTCAATGTCGGATGGATGAAAC | 164 |
| | BCL2.E2E3 | GTGGATGACTGAGTACCTGAACC | 165 | GGCCAAACTGAGCAGAGTCTT | 166 |
| | BRAF.E11E12 | CGGGACTCGAGTGATGATTGG | 167 | CTGAGGTGTAGGTGCTGTCA | 168 |
| | CDH1.E10E11 | CTCCTGAAAAGAGAGTGGAAGTGT | 169 | CCGGATTAATCTCCAGCCAGTT | 170 |
| | CDKN2A.E1E2 | AACGCACCGAATAGTTACGGT | 171 | ACGGGTCGGGTGAGAGT | 172 |
| | CDKN2B.E1E2 | CGGATCCCAACGGAGTCAA | 173 | ACCGGTCGGGTGAGAGT | 174 |
| | ERBB2.E11E12 | TCTTCCAGAACCTGCAAGTAATCC | 175 | GGTGGGTGTTATGGTGGATGA | 176 |
| | FOS.E3E4 | AGGAGAATCCGAAGGGAAAGGAATA | 177 | TCCTTCAGCAGGTTGGCAAT | 178 |
| | GAPDH.E5E6 | AGTCCACTGGCGTCTTCAC | 179 | TGATCTTGAGGCTGTTGTCATACTTC | 180 |
| | GUSB.E10E11 | GCGAGTATGGAGCAGAAACGA | 181 | AATTCCAAATGAGCTCTCCAACCA | 182 |
| | HRAS.E2E3 | CGGAATATAAGCTGGTGGTGGT | 183 | GCACGTCTCCCCATCAATGA | 184 |
| | KRAS.E3E4 | GTGCAATGAGGGACCAGTACA | 185 | CTACTAGGACCATAGGTACATCTTCAGA | 186 |
| | KRT10.E3E4 | GATGAGCTGACCCTGACCAA | 187 | GGCAGCATTCATTTCCACATTCAC | 188 |
| | KRT14.E3E4 | AGGAGCTGGCCTACCTGAA | 189 | CTTCTCATACTGGTCACGCATCT | 190 |
| | KRT17.E1E2 | AACACTGAGCTGGAGGTGAAG | 191 | CTGTAGCAGGATGTTGGCATTG | 192 |
| | MET.E2E3 | TGTGTGCATTCCCTATCAAATATGTCAA | 193 | GCGCTTCACAGCCTGATGA | 194 |
| | MKI67.E6E7 | CGTCGTGTCTCAAGATCTAGCTT | 195 | TGAGTCATCTGCGGTACTGTCT | 196 |
| | MYC.E1E2 | GCTTCTCTGAAAGGCTCTCCTT | 197 | AAATACGGCTGCACCGAGT | 198 |
| | NOTCH1.E31E32 | CCGACGCACAAGGTGTCTT | 199 | GTCGGCGTGTGAGTTGATGA | 200 |
| | PCNA.E4E5 | GACGGAGTGAAATTTTCTGCAAGT | 201 | GAAGTTCAGGTACCTCAGTGCAAA | 202 |
| | PTEN.E8E9 | AGCGTGCAGATAATGACAAGGAA | 203 | GATTTGACGGCTCCTCTACTGT | 204 |
| | RB1.E22E23 | CGGTCTTCATGCAGAGACTGA | 205 | GTGAAATATAGATGTTCCCTCCAGGAAT | 206 |
| | RPLP0.E7E8 | GACGGATTACACCTTCCCACTT | 207 | GACTCTTCCTTGGCTTCAACCTTA | 208 |
| | STAT1.E18E19 | CGATGGGCTCAGCTTTCAGA | 209 | ACAAAACCTCGTCCACGGAAT | 210 |
| | TERT.E10E11 | TCCTGCGTTTGGTGGATGAT | 211 | CCTCGTCTTCTACAGGGAAGTTCA | 212 |
| | TOP2A.E21E22 | TGGGTGGTCCTGCAAAATCC | 213 | ACATATTGATTTGGAGCCAGTTCTTCA | 214 |
| | TP53.E4E5 | CTGGCCCCTGTCATCTTCTG | 215 | CTTGGCCAGTTGGCAAAACAT | 216 |
| | WNT1.E2E3 | CTGGAACTGTCCCACTGCT | 217 | CAGGATTCGATGGAACCTTCTGA | 218 |
| HPV16 genomic and | unsp_226_227 | CACAGAGCTGCAAACAACTATACAT | 219 | CACATACAGCATATGGATTCCCATCTC | 220 |
| | unsp_408_409 | GGAACAACATTAGAACAGCAATACAAC A | 221 | TGTCCAGATGTCTTTGCTTTTCTTCA | 222 |
| | unsp_525_526 | CGGTGGACCGGTCGATG | 223 | TCAGTTGTCTCTGGTTGCAAATCT | 224 |
| | unsp_741_742 | CTCAGAGGAGGAGGATGAAATAGATG | 225 | CCATTAACAGGTCTTCCAAAGTACGA | 226 |
| | unsp_880_881 | GGAATTGTGTGCCCCATCTGT | 227 | CATCCATTACATCCCGTACCCT | 228 |
| | unsp_p997_998 | GGTTTTATGTAGAGGCTGTAGTGGAA | 229 | TGTGCAGTAAACAACGCATGTG | 230 |
| | unsp_1301_1302 | GCGGGTATGGCAATACTGAAGT | 231 | TGGTGTTTGGCATATAGTGTGTCTTT | 232 |
| | gen_1553_2056 | ATCAACGTGTTGCGATTGGT | 233 | CTAATAGTAACACAACCATTCCCCATGA | 234 |
| | unsp_p2307_2308 | GAGGTGATTGGAAGCAAATTGTTATGT | 235 | CAGACCCTTGCAGAAATTTCATTAAACT | 236 |

| | | | | | |
|---|---|---|---|---|---|
| | unsp_2580_2581 | GGATGTAAAGCATAGACCATTGGTACA | 237 | GTTTTCGTCAAATGGAAACTCATTAGGA | 238 |
| | unsp_2707_2708 | CGGAAATCCAGTGTATGAGCTTAATGAT | 239 | TGACACACATTTAAACGTTGGCAAAG | 240 |
| | unsp_3356_3357 | CATGCGGGTGGTCAGGTAA | 241 | AAGGCGACGGCTTTGGTAT | 242 |
| | unsp_3631_3632 | GCTCACACAAAGGACGGATTAAC | 243 | CCAATGCCATGTAGACGACACT | 244 |
| | gen_3883_4218 | GCGTGCTTTTTGCTTTGCTTTG | 245 | CAGAGGCTGCTGTTATCCACAATA | 246 |
| | unsp_4619_p4620 | TGGGCCCTTCTGATCCTTCTAT | 247 | GGTCAGTGAAAGTGGGATTATTATGTGT | 248 |
| | unsp_p5009_5010 | CTGCTTTTGTAACCACTCCCACTA | 249 | CCTAGAGGTTAATGCTGGCCTATG | 250 |
| | unsp_5408_p5409 | CTTCACATGCAGCCTCACCTA | 251 | GGAATATTGTATGCACCACCAAAAGG | 252 |
| | unsp_5636_5637 | CCTATAGTTCCAGGGTCTCCACAA | 253 | ATCCGTGCTTACAACCTTAGATACTG | 254 |
| | gen_5889_6779 | GGATGACACAGAAAATGCTAGTGCTTA | 255 | CACCTGGATTTACTGCAACATTGG | 256 |
| | unsp_7029_p7030 | ACCTCCAGCACCTAAAGAAGATGA | 257 | GGTGTAGCTTTTCGTTTTCCTAATGTAA | 258 |
| HPV16 spliced transcripts | sp_226_409 | CACAGAGCTGCAAACAACTATACAT | 259 | TGTCCAGATGTCTTTGCTTTTCTTCA | 260 |
| | sp_226_526 | CACAGAGCTGCAAACAACTATACAT | 261 | TCAGTTGTCTCTGGTTGCAAATCT | 262 |
| | sp_226_742 | CACAGAGCTGCAAACAACTATACAT | 263 | CCATTAACAGGTCTTCCAAAGTACGA | 264 |
| | sp_226_p1087 | CACAGAGCTGCAAACAACTATACAT | 265 | CACTAAGTGGACTACCAAATACTTTCGT | 266 |
| | sp_226_2581 | CACAGAGCTGCAAACAACTATACAT | 267 | GTTTTCGTCAAATGGAAACTCATTAGGA | 268 |
| | sp_226_2708 | CACAGAGCTGCAAACAACTATACAT | 269 | TGACACACATTTAAACGTTGGCAAAG | 270 |
| | sp_226_3357 | CACAGAGCTGCAAACAACTATACAT | 271 | AAGGCGACGGCTTTGGTAT | 272 |
| | sp_226_p4620 | CACAGAGCTGCAAACAACTATACAT | 273 | GGTCAGTGAAAGTGGGATTATTATGTGT | 274 |
| | sp_226_p5409 | CACAGAGCTGCAAACAACTATACAT | 275 | GGAATATTGTATGCACCACCAAAAGG | 276 |
| | sp_226_5637 | CACAGAGCTGCAAACAACTATACAT | 277 | ATCCGTGCTTACAACCTTAGATACTG | 278 |
| | sp_226_p7030 | CACAGAGCTGCAAACAACTATACAT | 279 | GGTGTAGCTTTTCGTTTTCCTAATGTAA | 280 |
| | sp_880_p1087 | GGAATTGTGTGCCCCATCTGT | 281 | CACTAAGTGGACTACCAAATACTTTCGT | 282 |
| | sp_880_2581 | GGAATTGTGTGCCCCATCTGT | 283 | GTTTTCGTCAAATGGAAACTCATTAGGA | 284 |
| | sp_880_2708 | GGAATTGTGTGCCCCATCTGT | 285 | TGACACACATTTAAACGTTGGCAAAG | 286 |
| | sp_880_3357 | GGAATTGTGTGCCCCATCTGT | 287 | AAGGCGACGGCTTTGGTAT | 288 |
| | sp_880_p4620 | GGAATTGTGTGCCCCATCTGT | 289 | GGTCAGTGAAAGTGGGATTATTATGTGT | 290 |
| | sp_880_p5409 | GGAATTGTGTGCCCCATCTGT | 291 | GGAATATTGTATGCACCACCAAAAGG | 292 |
| | sp_880_5637 | GGAATTGTGTGCCCCATCTGT | 293 | ATCCGTGCTTACAACCTTAGATACTG | 294 |
| | sp_880_p7030 | GGAATTGTGTGCCCCATCTGT | 295 | GGTGTAGCTTTTCGTTTTCCTAATGTAA | 296 |
| | sp_p997_p1087 | GGTTTTATGTAGAGGCTGTAGTGGAA | 297 | CACTAAGTGGACTACCAAATACTTTCGT | 298 |
| | sp_p997_2581 | GGTTTTATGTAGAGGCTGTAGTGGAA | 299 | GTTTTCGTCAAATGGAAACTCATTAGGA | 300 |
| | sp_p997_2708 | GGTTTTATGTAGAGGCTGTAGTGGAA | 301 | TGACACACATTTAAACGTTGGCAAAG | 302 |
| | sp_p997_3357 | GGTTTTATGTAGAGGCTGTAGTGGAA | 303 | AAGGCGACGGCTTTGGTAT | 304 |
| | sp_p997_p4620 | GGTTTTATGTAGAGGCTGTAGTGGAA | 305 | GGTCAGTGAAAGTGGGATTATTATGTGT | 306 |
| | sp_p997_p5409 | GGTTTTATGTAGAGGCTGTAGTGGAA | 307 | GGAATATTGTATGCACCACCAAAAGG | 308 |
| | sp_p997_5637 | GGTTTTATGTAGAGGCTGTAGTGGAA | 309 | ATCCGTGCTTACAACCTTAGATACTG | 310 |
| | sp_p997_p7030 | GGTTTTATGTAGAGGCTGTAGTGGAA | 311 | GGTGTAGCTTTTCGTTTTCCTAATGTAA | 312 |
| | sp_1301_2581 | GCGGGTATGGCAATACTGAAGT | 313 | GTTTTCGTCAAATGGAAACTCATTAGGA | 314 |
| | sp_1301_2708 | GCGGGTATGGCAATACTGAAGT | 315 | TGACACACATTTAAACGTTGGCAAAG | 316 |
| | sp_1301_3357 | GCGGGTATGGCAATACTGAAGT | 317 | AAGGCGACGGCTTTGGTAT | 318 |
| | sp_1301_5637 | GCGGGTATGGCAATACTGAAGT | 319 | ATCCGTGCTTACAACCTTAGATACTG | 320 |
| | sp_1301_p4620 | GCGGGTATGGCAATACTGAAGT | 321 | GGTCAGTGAAAGTGGGATTATTATGTGT | 322 |
| | sp_1301_p5409 | GCGGGTATGGCAATACTGAAGT | 323 | GGAATATTGTATGCACCACCAAAAGG | 324 |

| | | | | | |
|---|---|---|---|---|---|
| | sp_1301_p7030 | GCGGGTATGGCAATACTGAAGT | 325 | GGTGTAGCTTTTCGTTTTCCTAATGTAA | 326 |
| | sp_p2307_2708 | GAGGTGATTGGAAGCAAATTGTTATGT | 327 | TGACACACATTTAAACGTTGGCAAAG | 328 |
| | sp_p2307_3357 | GAGGTGATTGGAAGCAAATTGTTATGT | 329 | AAGGCGACGGCTTTGGTAT | 330 |
| | sp_p2307_5637 | GAGGTGATTGGAAGCAAATTGTTATGT | 331 | ATCCGTGCTTACAACCTTAGATACTG | 332 |
| | sp_p2307_p4620 | GAGGTGATTGGAAGCAAATTGTTATGT | 333 | GGTCAGTGAAAGTGGGATTATTATGTGT | 334 |
| | sp_p2307_p5409 | GAGGTGATTGGAAGCAAATTGTTATGT | 335 | GGAATATTGTATGCACCACCAAAAGG | 336 |
| | sp_p2307_p7030 | GAGGTGATTGGAAGCAAATTGTTATGT | 337 | GGTGTAGCTTTTCGTTTTCCTAATGTAA | 338 |
| | sp_3631_5637 | GCTCACACAAAGGACGGATTAAC | 339 | ATCCGTGCTTACAACCTTAGATACTG | 340 |
| | sp_3631_p4620 | GCTCACACAAAGGACGGATTAAC | 341 | GGTCAGTGAAAGTGGGATTATTATGTGT | 342 |
| | sp_3631_p5409 | GCTCACACAAAGGACGGATTAAC | 343 | GGAATATTGTATGCACCACCAAAAGG | 344 |
| | sp_3631_p7030 | GCTCACACAAAGGACGGATTAAC | 345 | GGTGTAGCTTTTCGTTTTCCTAATGTAA | 346 |
| | sp_p5009_p5409 | CTGCTTTTGTAACCACTCCCACTA | 347 | GGAATATTGTATGCACCACCAAAAGG | 348 |
| | sp_p5009_5637 | CTGCTTTTGTAACCACTCCCACTA | 349 | ATCCGTGCTTACAACCTTAGATACTG | 350 |
| | sp_p5009_p7030 | CTGCTTTTGTAACCACTCCCACTA | 351 | GGTGTAGCTTTTCGTTTTCCTAATGTAA | 352 |
| HPV16-human fusion transcripts | fus_880_MYC_001_ exon1 | GGAATTGTGTGCCCCATCTGT | 353 | CTGAGAAGCCCTGCCCTTC | 354 |
| | fus_880_MYC_001_ exon2 | GGAATTGTGTGCCCCATCTGT | 355 | AAATACGGCTGCACCGAGT | 356 |
| | fus_880_MYC_001_ exon3 | GGAATTGTGTGCCCCATCTGT | 357 | GGTGATCCAGACTCTGACCTTTTG | 358 |
| | fus_880_PVT1_002_ exon3 | GGAATTGTGTGCCCCATCTGT | 359 | ATCATGATGGCTGTATGTGCCA | 360 |
| | fus_880_PVT1_004_ exon1 | GGAATTGTGTGCCCCATCTGT | 361 | CATGGTTCCACCAGCGTTATT | 362 |
| | fus_880_PVT1_005_ exon1 | GGAATTGTGTGCCCCATCTGT | 363 | TCTTTGCTCGCAGCTCGT | 364 |
| | fus_2869_MYC_001 _exon1 | AGTACAGACCTACGTGACCATATAGAC | 365 | CTGAGAAGCCCTGCCCTTC | 366 |
| | fus_2869_MYC_001 _exon2 | AGTACAGACCTACGTGACCATATAGAC | 367 | AAATACGGCTGCACCGAGT | 368 |
| | fus_2869_MYC_001 _exon3 | AGTACAGACCTACGTGACCATATAGAC | 369 | GGTGATCCAGACTCTGACCTTTTG | 370 |
| | fus_2869_PVT1_002 _exon3 | AGTACAGACCTACGTGACCATATAGAC | 371 | ATCATGATGGCTGTATGTGCCA | 372 |
| | fus_2869_PVT1_004 _exon1 | AGTACAGACCTACGTGACCATATAGAC | 373 | CATGGTTCCACCAGCGTTATT | 374 |
| | fus_2869_PVT1_005 _exon1 | AGTACAGACCTACGTGACCATATAGAC | 375 | TCTTTGCTCGCAGCTCGT | 376 |

In particular, the nearest neighbor splice sites have been taken into consideration in order to minimize risks of co-amplifying several spliced isoforms with a given couple of primers. Additional primers pairs were defined, when possible, to amplify the boundaries at the 5'-SD-genomic and genomic-SA-3' positions (suffix 'unsp_') to allow for a better quantitative monitoring of concomitant spliced and/or genomic/unspliced transcription events and refine if necessary the description of transcripts equilibrium in the course of HPV infection. To complete this view and provide extra controls, primers were also designed within some HPV genomic regions lacking known SD/SA sites (suffix 'gen_'), meaning that the detection of such sequences could result only from locally unspliced transcription or DNA contamination. A selection of human transcripts has been included in the design as well for normalization purposes and/or to support or improve a combination of human and/or HPV transcripts being able to discriminate low grade vs high grade lesions of the cervix. Of note, extra fusion transcripts ('fus_') were investigated and primers were conceived following HPV breakpoint hypothesis in the context of HPV integration within the two human locus MYC and PTV1, as discussed for example in Lu et al. PLoS ONE 2014, Tang et al. Nature Communication 2013, Wentzensen et al. Oncogene 2002 or Peter et al. Oncogene 2006. In this case, forward primers were located 5' of HPV breakpoints (see Figure 7) and reverse primers designed within the targeted human exons, thus allowing for the detection and fine characterization of hybrid HPV-human transcripts.

### 17.4 Biological samples and cell line

Two high grade lesions (HSIL) samples of the cervix from two donor women, hereinafter referred to as 610 and 729, were collected in PreservCyt medium (Hologic) and kept at room temperature for a couple of days. After homogenization, 1mL aliquots were collected from the 20μL total liquid medium for HPV genotyping (Papillocheck, Greiner Bio-One). Results of HPV typing are given in Table 11.

**Table 11: HPV16 mono-infected samples from patients**

| Sample | Year of birth | Lesion | HPV typing (Papillocheck) |
|---|---|---|---|
| 610 | 1985 | High grade (HSIL) | 16 |
| 729 | 1950 | High grade (HSIL) | 16 |

The remaining samples were centrifuged at 4,500xg for 10 min and the pellets were stored at -80°C before RNA extraction. In addition, SiHa cells (HPV16 genomic integration) were cultured and harvested, providing another source of RNA.

### 17.5 RNA extraction and characterization

Total RNA from samples 610 and 729 were extracted using the PicoPure RNA isolation kit (Life Technologies), adding a DNAse treatment step directly on column (RNAse-free DNAse set, Qiagen) as recommended by the supplier. Elution was achieved in 30μL elution buffer. Assessment of RNA quantity and quality was done with a Nanodrop 1000 (Thermo Scientist) and a Bioanalyzer 2100 using the RNA Nano chips (Agilent).

### 17.6 Random reverse transcription

Random reverse transcription of total RNA was carried out using the SuperScript III First-Strand cDNA Synthesis kit (Invitrogen). Briefly, 2μL of total RNA was used for template and the reaction was performed in the presence of 50nM random hexamers (provided by Invitrogen), incubated 10min at 25°C, 60min at 50°C and 5min at 85°C before a final RNAse H treatment 20min at 37°C. The resulting cDNA were immediately amplified using the multiplex approach described below.

### 17.7 Multiplex amplification of specific transcripts

Amplification of HPV along with human transcripts was performed from the cDNA of samples 610, 729 and SiHa using a mixture of primers (appropriate for AmpliSeq™ technology; Life technologies) in a multiplex-manner, by a 20 cycles of amplification reaction. Following amplification, sequencing libraries were constructed (Life technologies) and validated on a Bioanalyzer 2100 before sequencing.

### 17.8 High throughput sequencing and data analysis

Samples 610, 729 and SiHa were sequenced on an Ion PGM apparatus using an Ion 118 chip (Life Technologies). FastQ data were generated and QC tests done following standard procedures. For each sample, sequencing reads were trimmed according to their Phred quality score then mapped to the HPV transcripts database using Bowtie 2 (Langmead et al. Nature Methods 2012). For spliced transcripts, alignments that did not encompass the splice junction were removed from the analysis. The number of reads for each sample is detailed in Table 12.

**Table 12: Reads number for HPV16 and human transcripts**

| | Transcript targeted | 610 | 729 | SiHa |
|---|---|---|---|---|
| Human transcripts | ACTB.E4E5 | 258790 | 81325 | 53371 |
| | AKT1.E2E3 | 837 | 3517 | 1412 |
| | B2M.E1E2 | 73613 | 101287 | 14808 |
| | BCL2.E2E3 | 80 | 937 | 1 |
| | BRAF.E11E12 | n.d. | 5509 | 1447 |
| | CDH1.E10E11 | n.d. | 37368 | 2018 |
| | CDKN2A.E1E2 | 137 | 300 | 365 |
| | CDKN2B.E1E2 | n.d. | 57289 | 2958 |
| | ERBB2.E11E12 | n.d. | 73226 | 2159 |
| | FOS.E3E4 | n.d. | 8966 | 9397 |
| | GAPDH.E5E6 | 79755 | 52860 | 70892 |

| | | | | |
|---|---|---|---|---|
| | GUSB.E10E11 | 137 | 226 | 112 |
| | HRAS.E2E3 | 65 | 700 | 181 |
| | KRAS.E3E4 | n.d. | 1006 | 474 |
| | KRT10.E3E4 | 374 | 412 | 0 |
| | KRT14.E3E4 | 38273 | 6019 | 86442 |
| | KRT17.E1E2 | n.d. | 21485 | 260630 |
| | MET.E2E3 | n.d. | 1361 | 308 |
| | MKI67.E6E7 | 1 | 4 | 1210 |
| | MYC.E1E2 | 0 | 112 | 223 |
| | NOTCH1.E31E32 | n.d. | 15301 | 1420 |
| | PCNA.E4E5 | 116 | 609 | 1262 |
| | PTEN.E8E9 | n.d. | 1568 | 1392 |
| | RB1.E22E23 | n.d. | 2807 | 2571 |
| | RPLP0.E7E8 | 32454 | 38380 | 5104 |
| | STAT1.E18E19 | n.d. | 12373 | 7993 |
| | TERT.E10E11 | n.d. | 0 | 628 |
| | TOP2A.E21E22 | 8 | 15 | 4267 |
| | TP53.E4E5 | n.d. | 11798 | 5551 |
| | WNT1.E2E3 | n.d. | 0 | 0 |
| HPV16 genomic and unspliced transcripts | unsp_226_227 | n.d. | 0 | 9180 |
| | unsp_408_409 | 61 | 0 | 18 |
| | unsp_525_526 | 1122 | 2 | 2871 |
| | unsp_741_742 | 2102 | 2 | 1651 |
| | unsp_880_881 | n.d. | 0 | 1079 |
| | unsp_p997_998 | 1610 | 0 | 383 |
| | unsp_1301_1302 | 140 | 2 | 592 |
| | gen_1553_2056 | 1740 | 1 | 297 |
| | unsp_p2307_2308 | 7923 | 1 | 608 |
| | unsp_2580_2581 | 11800 | 1 | 881 |
| | unsp_2707_2708 | 25162 | 2 | 828 |
| | unsp_3356_3357 | 2996 | 0 | 0 |
| | unsp_3631_3632 | 10497 | 2 | 0 |
| | gen_3883_4218 | 1661 | 0 | 2 |
| | unsp_4619_p4620 | 2685 | 0 | 0 |
| | unsp_p5009_5010 | 1619 | 1 | 0 |
| | unsp_5408_p5409 | 1690 | 0 | 1 |
| | unsp_5636_5637 | 3047 | 0 | 0 |
| | gen_5889_6779 | 1356 | 0 | 0 |
| | unsp_7029_p7030 | 5794 | 1 | 0 |
| HPV16 spliced transcripts | sp_226_409 | n.d. | 7 | 905 |
| | sp_226_526 | n.d. | 0 | 568 |
| | sp_226_742 | n.d. | 0 | 39 |
| | sp_226_p1087 | n.d. | 0 | 0 |
| | sp_226_2581 | n.d. | 0 | 0 |
| | sp_226_2708 | n.d. | 0 | 3 |

| | | | | |
|---|---|---|---|---|
| | sp_226_3357 | n.d. | 0 | 0 |
| | sp_226_p4620 | n.d. | 0 | 0 |
| | sp_226_p5409 | n.d. | 0 | 0 |
| | sp_226_5637 | n.d. | 0 | 0 |
| | sp_226_p7030 | n.d. | 0 | 0 |
| | sp_880_p1087 | n.d. | 0 | 0 |
| | sp_880_2581 | 0 | 0 | 4 |
| | sp_880_2708 | 29 | 0 | 92 |
| | sp_880_3357 | 11874 | 2 | 0 |
| | sp_880_p4620 | 0 | 0 | 0 |
| | sp_880_p5409 | 0 | 0 | 0 |
| | sp_880_5637 | 0 | 0 | 0 |
| | sp_880_p7030 | 0 | 0 | 0 |
| | sp_p997_p1087 | n.d. | 0 | 0 |
| | sp_p997_2581 | 0 | 0 | 0 |
| | sp_p997_2708 | 0 | 0 | 0 |
| | sp_p997_3357 | 0 | 0 | 0 |
| | sp_p997_p4620 | 0 | 0 | 0 |
| | sp_p997_p5409 | 0 | 0 | 0 |
| | sp_p997_5637 | 0 | 0 | 0 |
| | sp_p997_p7030 | 0 | 0 | 0 |
| | sp_1301_2581 | 0 | 0 | 0 |
| | sp_1301_2708 | 0 | 0 | 0 |
| | sp_1301_3357 | 0 | 0 | 0 |
| | sp_1301_5637 | 0 | 0 | 0 |
| | sp_1301_p4620 | 0 | 0 | 0 |
| | sp_1301_p5409 | 0 | 0 | 0 |
| | sp_1301_p7030 | 0 | 0 | 0 |
| | sp_p2307_2708 | 0 | 0 | 0 |
| | sp_p2307_3357 | 0 | 0 | 0 |
| | sp_p2307_5637 | 0 | 0 | 0 |
| | sp_p2307_p4620 | 0 | 0 | 0 |
| | sp_p2307_p5409 | 0 | 0 | 0 |
| | sp_p2307_p7030 | 0 | 0 | 0 |
| | sp_3631_5637 | 30 | 0 | 0 |
| | sp_3631_p4620 | 0 | 0 | 0 |
| | sp_3631_p5409 | 0 | 0 | 0 |
| | sp_3631_p7030 | 0 | 0 | 0 |
| | sp_p5009_p5409 | 0 | 0 | 0 |
| | sp_p5009_5637 | 0 | 0 | 0 |
| | sp_p5009_p7030 | 0 | 0 | 0 |
| HPV16-human | fus_880_MYC_001_exon1 | 0 | 0 | 0 |
| | fus_880_MYC_001_exon2 | 0 | 0 | 0 |
| | fus_880_MYC_001_exon3 | 0 | 0 | 0 |
| | fus_880_PVT1_002_exon3 | 0 | 0 | 0 |

| | | | | |
|---|---|---|---|---|
| | fus_880_PVT1_004_exon1 | 0 | 0 | 0 |
| | fus_880_PVT1_005_exon1 | 0 | 0 | 0 |
| | fus_2869_MYC_001_exon1 | 0 | 0 | 0 |
| | fus_2869_MYC_001_exon2 | 0 | 0 | 0 |
| | fus_2869_MYC_001_exon3 | 0 | 0 | 0 |
| | fus_2869_PVT1_002_exon3 | 0 | 0 | 0 |
| | fus_2869_PVT1_004_exon1 | 0 | 0 | 0 |
| | fus_2869_PVT1_005_exon1 | 0 | 0 | 0 |

**17.9 Multiplex amplification and quantification of HPV16 transcripts**

As a proof of principle, it was seeked to discriminate 47 spliced transcripts ('sp_'), 16 unspliced transcripts ('unsp_'), 3 genomic transcripts ('gen_'), 12 putative HPV-human fusion transcripts ('fus_'), plus additional 30 human transcripts, from mono-infected HPV16 samples (samples 610 and 729) and SiHa cells. To ensure amplification specificity, the design has been checked for its lack of cross-match against the HPV database in addition to the human genome and transcripts databases. Primers are detailed in Table 10. Reads number following QC, mapping and validation of the splice junction are detailed in Table 12.

**17.10 Results: detection and quantification of HPV16 and human transcripts**

The experiment showed that (i) specific human transcripts, as internal and/or normalization controls, were detected in samples 610, 729 and SiHa with expression levels varying between transcripts and from one sample to another, thus validating the integrity of starting RNA material and the effectiveness of subsequent multiplex amplification steps (ii) specific spliced ('sp_') and unspliced ('unsp_') HPV16 transcripts were successfully detected and characterized in samples 610, 729 and SiHa, albeit in a variable proportion between samples, supporting the quantitative variations of specific HPV16 transcripts or transcription events between biological samples, (iii) in particular, sample SiHa exhibited no or rare genomic ('gen_'), unspliced ('unsp_') and spliced ('sp_') transcripts reads beyond genomic position 3356, which appeared consistent with the loss of viral late genes following HPV16 integration into the genome of SiHa cells, and (iv) it thus demonstrated the capability of the method to accurately differentiate between non-replicative, integrative HPV16 infection stages often associated with higher levels of E6/E7 transcripts (in this particular case sp_226_409, sp_226_526 and sp_226_742, see Table 12), from other anterior, HPV16-induced transformation and/or proliferation steps which usually imply transcription of the E2 and/or L1 and/or L2 genes (see as an example sp_880_3357 in Table 12).Consequently, specific HPV16 spliced transcripts and/or HPV16 unspliced transcripts and/or HPV16 genomic transcripts and/or HPV16-human fusion transcripts can be weighted to compute a score, or score ratio, discriminating different stages of interaction of HPV16 with infected cells, in particular the early vs late stages of HPV16 cycle , and/or the integrative vs non integrative forms of the HPV16 genome into infected cells, which are events associated to cell transformation. More generally, these results suggest that the method can be extended and applied to all HR $\alpha$HPV.

**17.11 Examples of R scores**

These observations reinforce the possibly to define a score, referred to as R score, based on specific HPV transcripts counts as a molecular marker of any individual HPV-positive samples. From this perspective, either one value or a combination of more than one ratio(s) could be used as a marker of the viral-cell interactions that shapes the transformation process. A non-restrictive list of R scores is given in Table 13 in order to illustrate several possible combinations based on specific HPV16 transcripts.

**Table 13: Examples of R scores**

| R scores (examples) | 610 | 729 | SiHa |
|---|---|---|---|
| sp_226_409 / sp_880_2708 | n.d. | $+\infty$ | 9,83 |
| sp_880_2581 / sp_3631_5637 | 0 | n.a. | $+\infty$ |
| sp_880_2708 / sp_3631_5637 | 0,96 | n.a. | $+\infty$ |
| sp_880_3357 / sp_3631_5637 | 395,8 | $+\infty$ | n.a. |

(continued)

| R scores (examples) | 610 | 729 | SiHa |
|---|---|---|---|
| unsp_741_742 / unsp_p5009_5010 | 1,29 | 2 | +∞ |

As an example, R scores based on a ratio sp_226_409/sp_880_2708 and/or sp_880_2581/sp_3631_5637 and/or sp_880_2708/sp_3631_5637 and/or sp_880_3357/sp_3631_5637 and/or unsp_741_742/unsp_p5009_5010 succeeded in generating high score values (e.g.: +∞) that are associated with non - or lowly - productive HPV cycles typical of transformed cells. Of note, weighting coefficients such as α(sp_226_409/sp_880_2708) and/or β(sp_880_2581/sp_3631_5637) and/or y(sp_880_2708/sp_3631_5637) and/or δ(sp_880_3357/sp_3631_5637) and/or ε(unsp_741_742/unsp_p5009_5010) can be added as parameters, independently, in order to better discriminate, for instance, low risk and high risk lesions.

### 17.12 Extension of the method to the HR αHPV group

The method was extended to the entire HR αHPV group (i.e.: HPV16, 18, 31, 33, 35, 39, 45, 51, 52, 56, 58, 59, 66, 68, 73 and 82) based on the transcription map described in Figure 7. Primers resulting from this improved design are listed in Table 14.

**Table 14: Primer pairs for HR αHPV group and human transcripts**

| Virus name | Transcript targeted | Forward primer (5'-3') | SEQ ID NO° | Reverse primer (5'-3') | SEQ ID NO° |
|---|---|---|---|---|---|
| HVP16 | 16_fus_2869_PVT1_005 _exon1 | AGTACAGACCTACGTGACCATATAGAC | 377 | TCTTTGCTCGCAGCTCGT | 378 |
| HVP16 | 16_sp_1301_2581 | GCGGGTATGGCAATACTGAAGT | 379 | GTTTTCGTCAAATGGAAACTCAT TAGGA | 380 |
| HVP16 | 16_sp_226_742 | CACAGAGCTGCAAACAACTATACAT | 381 | CCATTAACAGGTCTTCCAAAGTA CGA | 382 |
| HVP16 | 16_unsp_525_526 | CGGTGGACCGGTCGATG | 383 | TCAGTTGTCTCTGGTTGCAAATC T | 384 |
| HVP16 | 16_unsp_1301_1302 | GCGGGTATGGCAATACTGAAGT | 385 | TGGTGTTTGGCATATAGTGTGTC TTT | 386 |
| HVP16 | 16_sp_880_2581 | GGAATTGTGTGCCCCATCTGT | 387 | GTTTTCGTCAAATGGAAACTCAT TAGGA | 388 |
| HVP16 | 16_fus_2869_MYC_001_ exon2 | AGTACAGACCTACGTGACCATATAGAC | 389 | AAATACGGCTGCACCGAGT | 390 |
| HVP16 | 16_fus_3619_PVT1_005 _exon1 | GCTCACACAAAGGACGGATTAAC | 391 | TCTTTGCTCGCAGCTCGT | 392 |
| HVP16 | 16_fus_880_PVT1_005_ exon1 | GGAATTGTGTGCCCCATCTGT | 393 | TCTTTGCTCGCAGCTCGT | 394 |
| HVP16 | 16_unsp_226_227 | CACAGAGCTGCAAACAACTATACAT | 395 | CACATACAGCATATGGATTCCCA TCTC | 396 |
| HVP16 | 16_sp_226_409 | CACAGAGCTGCAAACAACTATACAT | 397 | TGTCCAGATGTCTTTGCTTTTCTT CA | 398 |
| HVP16 | 16_sp_880_3357 | GGAATTGTGTGCCCCATCTGT | 399 | AAGGCGACGGCTTTGGTAT | 400 |
| HVP16 | 16_unsp_2580_2581 | GGATGTAAAGCATAGACCATTGGTACA | 401 | GTTTTCGTCAAATGGAAACTCAT TAGGA | 402 |
| HVP16 | 16_fus_2869_MYC_001_ exon3 | AGTACAGACCTACGTGACCATATAGAC | 403 | GGTGATCCAGACTCTGACCTTTT G | 404 |
| HVP16 | 16_fus_2869_PVT1_002 _exon3 | AGTACAGACCTACGTGACCATATAGAC | 405 | ATCATGATGGCTGTATGTGCCA | 406 |
| HVP16 | 16_unsp_3631_3632 | GCTCACACAAAGGACGGATTAAC | 407 | CCAATGCCATGTAGACGACACT | 408 |
| HVP16 | 16_fus_3619_PVT1_004 _exon1 | GCTCACACAAAGGACGGATTAAC | 409 | CATGGTTCCACCAGCGTTATT | 410 |

(continued)

| Virus name | Transcript targeted | Forward primer (5'-3') | SEQ ID NO° | Reverse primer (5'-3') | SEQ ID NO° |
|---|---|---|---|---|---|
| HVP16 | 16_fus_880_MYC_001_e xon3 | GGAATTGTGTGCCCCATCTGT | 411 | GGTGATCCAGACTCTGACCTTTT G | 412 |
| HVP16 | 16_sp_226_2581 | CACAGAGCTGCAAACAACTATACAT | 413 | GTTTTCGTCAAATGGAAACTCAT TAGGA | 414 |
| HVP16 | 16_sp_1301_2708 | GCGGGTATGGCAATACTGAAGT | 415 | TGACACACATTTAAACGTTGGCA AAG | 416 |
| HVP16 | 16_fus_880_PVT1_002_ exon3 | GGAATTGTGTGCCCCATCTGT | 417 | ATCATGATGGCTGTATGTGCCA | 418 |
| HVP16 | 16_sp_3631_5637 | GCTCACACAAAGGACGGATTAAC | 419 | ATCCGTGCTTACAACCTTAGATA CTG | 420 |
| HVP16 | 16_unsp_3356_3357 | CATGCGGGTGGTCAGGTAA | 421 | AAGGCGACGGCTTTGGTAT | 422 |
| HVP16 | 16_unsp_5636_5637 | CCTATAGTTCCAGGGTCTCCACAA | 423 | ATCCGTGCTTACAACCTTAGATA CTG | 424 |
| HVP16 | 16_fus_880_PVT1_004_ exon1 | GGAATTGTGTGCCCCATCTGT | 425 | CATGGTTCCACCAGCGTTATT | 426 |
| HVP16 | 16_fus_2869_MYC_001_ exon1 | AGTACAGACCTACGTGACCATATAGAC | 427 | CTGAGAAGCCCTGCCCTTC | 428 |
| HVP16 | 16_unsp_2707_2708 | CGGAAATCCAGTGTATGAGCTTAATGA T | 429 | TGACACACATTTAAACGTTGGCA AAG | 430 |
| HVP16 | 16_sp_226_3357 | CACAGAGCTGCAAACAACTATACAT | 431 | AAGGCGACGGCTTTGGTAT | 432 |
| HVP16 | 16_fus_3619_MYC_001_ exon1 | GCTCACACAAAGGACGGATTAAC | 433 | CTGAGAAGCCCTGCCCTTC | 434 |
| HVP16 | 16_sp_1301_3357 | GCGGGTATGGCAATACTGAAGT | 435 | AAGGCGACGGCTTTGGTAT | 436 |
| HVP16 | 16_sp_880_5637 | GGAATTGTGTGCCCCATCTGT | 437 | ATCCGTGCTTACAACCTTAGATA CTG | 438 |
| HVP16 | 16_unsp_741_742 | CTCAGAGGAGGAGGATGAAATAGATG | 439 | CCATTAACAGGTCTTCCAAAGTA CGA | 440 |
| HVP16 | 16_fus_880_MYC_001_e xon2 | GGAATTGTGTGCCCCATCTGT | 441 | AAATACGGCTGCACCGAGT | 442 |
| HVP16 | 16_fus_3619_MYC_001_ exon3 | GCTCACACAAAGGACGGATTAAC | 443 | GGTGATCCAGACTCTGACCTTTT G | 444 |
| HVP16 | 16_fus_880_MYC_001_e xon1 | GGAATTGTGTGCCCCATCTGT | 445 | CTGAGAAGCCCTGCCCTTC | 446 |

(continued)

| Virus name | Transcript targeted | Forward primer (5'-3') | SEQ ID NO° | Reverse primer (5'-3') | SEQ ID NO° |
|---|---|---|---|---|---|
| HVP16 | 16_unsp_408_409 | GGAACAACATTAGAACAGCAATACAAC A | 447 | TGTCCAGATGTCTTTGCTTTTCTT CA | 448 |
| HVP16 | 16_unsp_880_881 | GGAATTGTGTGCCCCATCTGT | 449 | CATCCATTACATCCCGTACCCT | 450 |
| HVP16 | 16_fus_3619_PVT1_002 _exon3 | GCTCACACAAAGGACGGATTAAC | 451 | ATCATGATGGCTGTATGTGCCA | 452 |
| HVP16 | 16_sp_226_2708 | CACAGAGCTGCAAACAACTATACAT | 453 | TGACACACATTTAAACGTTGGCA AAG | 454 |
| HVP16 | 16_sp_880_2708 | GGAATTGTGTGCCCCATCTGT | 455 | TGACACACATTTAAACGTTGGCA AAG | 456 |
| HVP16 | 16_fus_3619_MYC_001_ exon2 | GCTCACACAAAGGACGGATTAAC | 457 | AAATACGGCTGCACCGAGT | 458 |
| HVP16 | 16_sp_226_526 | CACAGAGCTGCAAACAACTATACAT | 459 | TCAGTTGTCTCTGGTTGCAAATC T | 460 |
| HVP16 | 16_fus_2869_PVT1_004 _exon1 | AGTACAGACCTACGTGACCATATAGAC | 461 | CATGGTTCCACCAGCGTTATT | 462 |
| HVP16 | 16_gen_3881_4212 | CGTGCTTTTTGCTTTGCTTTGT | 463 | GAGGCTGCTGTTATCCACAATA GTAAT | 464 |
| HVP16 | 16_gen_5887_7259 | CCTGTGTAGGTGTTGAGGTAGGT | 465 | TCTATTATCCACACCTGCATTTGC T | 466 |
| HVP16 | 16_gen_1551_2331 | AACGTGTTGCGATTGGTGTATTG | 467 | CATTCCCCATGAACATGCTAAAC TTTG | 468 |
| HVP16 | 16_gen_7266_7904 | CCAGGCCCATTTTGTAGCTT | 469 | AGGTCAGGAAAACAGGGATTTG G | 470 |
| HVP18 | 18_unsp_2650_2651 | CTAAAATGTCCTCCAATACTACTAACCA CAA | 471 | GTCATTTATTTCATATACTGGATT GCCA | 472 |
| HVP18 | 18_sp_929_2651 | TGCATCCCAGCAGTAAGCAA | 473 | GTCATTTATTTCATATACTGGATT GCCA | 474 |
| HVP18 | 18_unsp_3165_3166 | GGATTGGACACTGCAAGACACA | 475 | CCCATGCTACATAGGTCATACAA TTGTC | 476 |
| HVP18 | 18_sp_3165_3465 | GGATTGGACACTGCAAGACACA | 477 | ACGTCTGGCCGTAGGTCT | 478 |

(continued)

| Virus name | Transcript targeted | Forward primer (5'-3') | SEQ ID NO° | Reverse primer (5'-3') | SEQ ID NO° |
|---|---|---|---|---|---|
| HVP18 | 18_unsp_790_791 | CAGAGGAAGAAAACGATGAAATAGATGG | 479 | AGAAACAGCTGCTGGAATGCT | 480 |
| HVP18 | 18_unsp_5612_5613 | TCCTAAGAAACGTAAACGTGTTCCC | 481 | GTATTTACAACTCTTGCCACAGAAGGA | 482 |
| HVP18 | 18_sp_1357_2651 | TCAGATAGTGGCTATGGCTGTTCT | 483 | GTCATTTATTTCATATACTGGATTGCCA | 484 |
| HVP18 | 18_fus_3684_MYC_001_exon3 | CAGCTACACCTACAGGCAACAA | 485 | GGTGATCCAGACTCTGACCTTTTG | 486 |
| HVP18 | 18_fus_2943_MYC_001_exon2 | AATGACAGTAAAGACATAGACAGCCAAA | 487 | AAATACGGCTGCACCGAGT | 488 |
| HVP18 | 18_sp_233_3465 | TTCACTGCAAGACATAGAAATAACCTGT | 489 | ACGTCTGGCCGTAGGTCT | 490 |
| HVP18 | 18_fus_3684_PVT1_002_exon3 | CAGCTACACCTACAGGCAACAA | 491 | ATCATGATGGCTGTATGTGCCA | 492 |
| HVP18 | 18_sp_1357_2779 | TCAGATAGTGGCTATGGCTGTTCT | 493 | GGTTTCCTTCGGTGTCTGCAT | 494 |
| HVP18 | 18_sp_3696_5776 | CAGCTACACCTACAGGCAACAA | 495 | TCAGGTAACTGCACCCTAAATACTCTAT | 496 |
| HVP18 | 18_fus_2943_PVT1_004_exon1 | AATGACAGTAAAGACATAGACAGCCAAA | 497 | CATGGTTCCACCAGCGTTATT | 498 |
| HVP18 | 18_fus_3684_MYC_001_exon2 | CAGCTACACCTACAGGCAACAA | 499 | AAATACGGCTGCACCGAGT | 500 |
| HVP18 | 18_fus_3684_PVT1_005_exon1 | CAGCTACACCTACAGGCAACAA | 501 | TCTTTGCTCGCAGCTCGT | 502 |
| HVP18 | 18_unsp_5775_5776 | GCATATTTTATCATGCTGGCAGCTCTA | 503 | TCAGGTAACTGCACCCTAAATACTCTAT | 504 |
| HVP18 | 18_sp_929_5613 | TGCATCCCAGCAGTAAGCAA | 505 | GTATTTACAACTCTTGCCACAGAAGGA | 506 |
| HVP18 | 18_fus_929_PVT1_005_ exon1 | TGCATCCCAGCAGTAAGCAA | 507 | TCTTTGCTCGCAGCTCGT | 508 |
| HVP18 | 18_fus_2943_PVTI_002 _exon3 | AATGACAGTAAAGACATAGACAGCCAAA | 509 | ATCATGATGGCTGTATGTGCCA | 510 |

(continued)

| Virus name | Transcript targeted | Forward primer (5'-3') | SEQ ID NO° | Reverse primer (5'-3') | SEQ ID NO° |
|---|---|---|---|---|---|
| HVP18 | 18_sp_1357_3465 | TCAGATAGTGGCTATGGCTGTTCT | 511 | ACGTCTGGCCGTAGGTCT | 512 |
| HVP18 | 18_fus_929_MYC_001_e xon2 | TGCATCCCAGCAGTAAGCAA | 513 | AAATACGGCTGCACCGAGT | 514 |
| HVP18 | 18_fus_929_MYC_001_e xon1 | TGCATCCCAGCAGTAAGCAA | 515 | CTGAGAAGCCCTGCCCTTC | 516 |
| HVP18 | 18_fus_2943_PVT1_005 _exon1 | AATGACAGTAAAGACATAGACAGCCAA A | 517 | TCTTTGCTCGCAGCTCGT | 518 |
| HVP18 | 18_fus_2943_MYC_001_ exon3 | AATGACAGTAAAGACATAGACAGCCAA A | 519 | GGTGATCCAGACTCTGACCTTTT G | 520 |
| HVP18 | 18_sp_233_416 | TTCACTGCAAGACATAGAAATAACCTG T | 521 | CCCAGCTATGTTGTGAAATCGT | 522 |
| HVP18 | 18_fus_929_PVT1_004_ exon1 | TGCATCCCAGCAGTAAGCAA | 523 | CATGGTTCCACCAGCGTTATT | 524 |
| HVP18 | 18_unsp_1357_1358 | TCAGATAGTGGCTATGGCTGTTCT | 525 | CCGTTGTCTATAGCCTCCGT | 526 |
| HVP18 | 18_sp_3696_5613 | CAGCTACACCTACAGGCAACAA | 527 | GTATTTACAACTCTTGCCACAGA AGGA | 528 |
| HVP18 | 18_sp_929_2779 | TGCATCCCAGCAGTAAGCAA | 529 | GGTTTCCTTCGGTGTCTGCAT | 530 |
| HVP18 | 18_sp_929_3465 | TGCATCCCAGCAGTAAGCAA | 531 | ACGTCTGGCCGTAGGTCT | 532 |
| HVP18 | 18_unsp_233_234 | TTCACTGCAAGACATAGAAATAACCTG T | 533 | CTATACATTTATGGCATGCAGCA TGG | 534 |
| HVP18 | 18_unsp_415_416 | TCAGACTCTGTGTATGGAGACACAT | 535 | CCCAGCTATGTTGTGAAATCGT | 536 |
| HVP18 | 18_fus_2943_MYC_001_ exon1 | AATGACAGTAAAGACATAGACAGCCAA A | 537 | CTGAGAAGCCCTGCCCTTC | 538 |
| HVP18 | 18_fus_929_MYC_001_e xon3 | TGCATCCCAGCAGTAAGCAA | 539 | GGTGATCCAGACTCTGACCTTTT G | 540 |
| HVP18 | 18_sp_233_791 | TTCACTGCAAGACATAGAAATAACCTG T | 541 | AGAAACAGCTGCTGGAATGCT | 542 |
| HVP18 | 18_fus_3684_PVT1_004 _exon1 | CAGCTACACCTACAGGCAACAA | 543 | CATGGTTCCACCAGCGTTATT | 544 |
| HVP18 | 18_sp_929_5776 | TGCATCCCAGCAGTAAGCAA | 545 | TCAGGTAACTGCACCCTAAATAC TCTAT | 546 |
| HVP18 | 18_fus_929_PVT1_002_ exon3 | TGCATCCCAGCAGTAAGCAA | 547 | ATCATGATGGCTGTATGTGCCA | 548 |

(continued)

| Virus name | Transcript targeted | Forward primer (5'-3') | SEQ ID NO° | Reverse primer (5'-3') | SEQ ID NO° |
|---|---|---|---|---|---|
| HVP18 | 18_sp_233_2779 | TTCACTGCAAGACATAGAAATAACCTGT | 549 | GGTTTCCTTCGGTGTCTGCAT | 550 |
| HVP18 | 18_sp_233_2651 | TTCACTGCAAGACATAGAAATAACCTGT | 551 | GTCATTTATTTCATATACTGGATTGCCA | 552 |
| HVP18 | 18_fus_3684_MYC_001_exon1 | CAGCTACACCTACAGGCAACAA | 553 | CTGAGAAGCCCTGCCCTTC | 554 |
| HVP18 | 18_unsp_929_930 | TGCATCCCAGCAGTAAGCAA | 555 | CTCGTCATCTGATATTACATCTCCTGTT | 556 |
| HVP18 | 18_sp_3786_5776 | CGAAAACATAGCGACCACTATAGAGAT | 557 | TCAGGTAACTGCACCCTAAATACTCTAT | 558 |
| HVP18 | 18_unsp_3464_3465 | TGACGACACGGTATCCGCTA | 559 | ACGTCTGGCCGTAGGTCT | 560 |
| HVP18 | 18_unsp_3786_3787 | CGAAAACATAGCGACCACTATAGAGAT | 561 | TTGTACACTATCTGGAATTGCAACAGT | 562 |
| HVP18 | 18_unsp_3696_3697 | CAGCTACACCTACAGGCAACAA | 563 | GTCGCTATGTTTTCGCAATCTGTA | 564 |
| HVP18 | 18_gen_1607_2401 | TGGAGTAAACCCAACAATAGCAGAAG | 565 | CATTTGTAACGCAACAGGGCTAAT | 566 |
| HVP18 | 18_sp_3786_5613 | CGAAAACATAGCGACCACTATAGAGAT | 567 | GTATTTACAACTCTTGCCACAGAAGGA | 568 |
| HVP18 | 18_gen_7284_7857 | CGCCCTAGTGAGTAACAACTGTATTT | 569 | GGAGGATTGTAGGATAAAATGGATGCT | 570 |
| HVP18 | 18_gen_6026_7277 | GAGGACGTTAGGGACAATGTGT | 571 | CCCTGTGATAAAGGACGCGATTT | 572 |
| HVP18 | 18_gen_3946_4234 | CGTATGCATGGGTATTGGTATTTGTG | 573 | CATGTATATGCAATAGTAACATGGGCAA | 574 |
| HVP31 | 31_unsp_5551_5552 | GCCACAAGTGTCTATTTTTGTTGATG | 575 | TTTAGACACTGGGACAGGTGGTA | 576 |
| HVP31 | 31_unsp_739_740 | CAGATGAGGAGGATGTCATAGACAGT | 577 | CATTAACAGCTCTTGCAATATGCGAATA | 578 |
| HVP31 | 31_fus_3578_MYC_001_exon3 | CAGCTGCATGCACAAACCA | 579 | GGTGATCCAGACTCTGACCTTTTG | 580 |

(continued)

| Virus name | Transcript targeted | Forward primer (5'-3') | SEQ ID NO° | Reverse primer (5'-3') | SEQ ID NO° |
|---|---|---|---|---|---|
| HVP31 | 31_fus_2807_MYC_001_ exon2 | CAACGTTTAAATGTGTGTCAGGACAAA | 581 | AAATACGGCTGCACCGAGT | 582 |
| HVP31 | 31_fus_2807_PVT1_004 _exon1 | CAACGTTTAAATGTGTGTCAGGACAAA | 583 | CATGGTTCCACCAGCGTTATT | 584 |
| HVP31 | 31_fus_3578_PVT1_004 _exon1 | CAGCTGCATGCACAAACCA | 585 | CATGGTTCCACCAGCGTTATT | 586 |
| HVP31 | 31_sp_3590_5552 | CAGCTGCATGCACAAACCA | 587 | TTTAGACACTGGGACAGGTGGT A | 588 |
| HVP31 | 31_fus_2807_PVT1_002 _exon3 | CAACGTTTAAATGTGTGTCAGGACAAA | 589 | ATCATGATGGCTGTATGTGCCA | 590 |
| HVP31 | 31_unsp_1296_1297 | GCGGGTATGGCAATACTGAAGT | 591 | TGGAGTTTCATTCTCTCGTTCACT ATG | 592 |
| HVP31 | 31_sp_1296_2646 | GCGGGTATGGCAATACTGAAGT | 593 | CGTTGAGAAAGAGTCTCCATCG TTTT | 594 |
| HVP31 | 31_sp_230_3295 | CGGCATTGGAAATACCCTACGAT | 595 | GAATTCGATGTGGTGGTGTTGTT G | 596 |
| HVP31 | 31_fus_2807_MYC_001_ exon1 | CAACGTTTAAATGTGTGTCAGGACAAA | 597 | CTGAGAAGCCCTGCCCTTC | 598 |
| HVP31 | 31_sp_230_740 | CGGCATTGGAAATACCCTACGAT | 599 | CATTAACAGCTCTTGCAATATGC GAATA | 600 |
| HVP31 | 31_fus_2807_MYC_001_ exon3 | CAACGTTTAAATGTGTGTCAGGACAAA | 601 | GGTGATCCAGACTCTGACCTTTT G | 602 |
| HVP31 | 31_fus_877_PVT1_004_ exon1 | AATCGTGTGCCCCAACTGT | 603 | CATGGTTCCACCAGCGTTATT | 604 |
| HVP31 | 31_unsp_2645_2646 | CTGGTGGTTTTTACATTTCCAAATCCAT | 605 | CGTTGAGAAAGAGTCTCCATCG TTTT | 606 |
| HVP31 | 31_sp_877_5552 | AATCGTGTGCCCCAACTGT | 607 | TTTAGACACTGGGACAGGTGGT A | 608 |
| HVP31 | 31_sp_230_413 | CGGCATTGGAAATACCCTACGAT | 609 | TTTTCTTCTGGACACAACGGTCT T | 610 |
| HVP31 | 31_sp_1296_2518 | GCGGGTATGGCAATACTGAAGT | 611 | AATGTAAAAACCACCAGTCTGCT ATGTA | 612 |

(continued)

| Virus name | Transcript targeted | Forward primer (5'-3') | SEQ ID NO° | Reverse primer (5'-3') | SEQ ID NO° |
|---|---|---|---|---|---|
| HVP31 | 31_sp_230_2646 | CGGCATTGGAAATACCCTACGAT | 613 | CGTTGAGAAAGAGTCTCCATCG TTTT | 614 |
| HVP31 | 31_sp_877_2518 | AATCGTGTGCCCCAACTGT | 615 | AATGTAAAAACCACCAGTCTGCT ATGTA | 616 |
| HVP31 | 31_fus_3578_PVT1_005 _exon1 | CAGCTGCATGCACAAACCA | 617 | TCTTTGCTCGCAGCTCGT | 618 |
| HVP31 | 31_unsp_230_231 | CGGCATTGGAAATACCCTACGAT | 619 | TCTTAAACATTTTGTACACACTCC GTGT | 620 |
| HVP31 | 31_fus_3578_MYC_001_ exon2 | CAGCTGCATGCACAAACCA | 621 | AAATACGGCTGCACCGAGT | 622 |
| HVP31 | 31_fus_877_PVT1_002_ exon3 | AATCGTGTGCCCCAACTGT | 623 | ATCATGATGGCTGTATGTGCCA | 624 |
| HVP31 | 31_fus_877_MYC_001_e xon1 | AATCGTGTGCCCCAACTGT | 625 | CTGAGAAGCCCTGCCCTTC | 626 |
| HVP31 | 31_unsp_877_878 | AATCGTGTGCCCCAACTGT | 627 | CCCCTGTCTGTCTGTCAATTACT G | 628 |
| HVP31 | 31_sp_877_2646 | AATCGTGTGCCCCAACTGT | 629 | CGTTGAGAAAGAGTCTCCATCG TTTT | 630 |
| HVP31 | 31_unsp_3590_3591 | CAGCTGCATGCACAAACCA | 631 | GCCATGTAGATGACACTTGTTCA TACAA | 632 |
| HVP31 | 31_sp_230_530 | CGGCATTGGAAATACCCTACGAT | 633 | ACATAGTCTTGCAACGTAGGTGT TT | 634 |
| HVP31 | 31_unsp_412_413 | GGAACAACATTAGAAAAATTGACAAAC AAAGG | 635 | TTTTCTTCTGGACACAACGGTCT T | 636 |
| HVP31 | 31_sp_230_2518 | CGGCATTGGAAATACCCTACGAT | 637 | AATGTAAAAACCACCAGTCTGCT ATGTA | 638 |
| HVP31 | 31_fus_3578_PVT1_002 _exon3 | CAGCTGCATGCACAAACCA | 639 | ATCATGATGGCTGTATGTGCCA | 640 |
| HVP31 | 31_fus877_PVT1_005_ exon1 | AATCGTGTGCCCCAACTGT | 641 | TCTTTGCTCGCAGCTCGT | 642 |
| HVP31 | 31_unsp_2517_2518 | CACTAGATGGCAACCCTGTATCT | 643 | AATGTAAAAACCACCAGTCTGCT ATGTA | 644 |
| HVP31 | 31_sp_877_3295 | AATCGTGTGCCCCAACTGT | 645 | GAATTCGATGTGGTGGTGTTGTT G | 646 |

(continued)

| Virus name | Transcript targeted | Forward primer (5'-3') | SEQ ID NO° | Reverse primer (5'-3') | SEQ ID NO° |
|---|---|---|---|---|---|
| HVP31 | 31_fus_877_MYC_001_e xon3 | AATCGTGTGCCCCAACTGT | 647 | GGTGATCCAGACTCTGACCTTTT G | 648 |
| HVP31 | 31_unsp_3294_3295 | CATGCGGGTGGTCAGGTAA | 649 | GAATTCGATGTGGTGGTGTTGTT G | 650 |
| HVP31 | 31_fus_877_MYC_001_e xon2 | AATCGTGTGCCCCAACTGT | 651 | AAATACGGCTGCACCGAGT | 652 |
| HVP31 | 31_unsp_529_530 | GAAACGATTCCACAACATAGGAGGA | 653 | ACATAGTCTTGCAACGTAGGTGT TT | 654 |
| HVP31 | 31_sp_1296_3295 | GCGGGTATGGCAATACTGAAGT | 655 | GAATTCGATGTGGTGGTGTTGTT G | 656 |
| HVP31 | 31_fus_3578_MYC_001_ exon1 | CAGCTGCATGCACAAACCA | 657 | CTGAGAAGCCCTGCCCTTC | 658 |
| HVP31 | 31_fus_2807_PVT1_005 _exon1 | CAACGTTTAAATGTGTGTCAGGACAAA | 659 | TCTTTGCTCGCAGCTCGT | 660 |
| HVP31 | 31_gen_7233_7912 | TGTGTGTGTTGTGTATGTTGTCCTT | 661 | CAACTTTTACTATGGCGTGACAC CTA | 662 |
| HVP31 | 31_gen_5802_7226 | GCTTAGTTTGGGCCTGTGTT | 663 | ACCACCGGCATATCTATTAGAGT TTTC | 664 |
| HVP31 | 31_gen_3840_4137 | GCATTGTGCTATGCTTTTTGCTTTG | 665 | ACAACGTAATGGAGAGGTTGCA ATA | 666 |
| HVP31 | 31_gen_1546_2268 | GTGAAACACCAGAATGGATAGAAAGA C | 667 | TGCACATGCATTACTATCACTGT CA | 668 |
| HVP33 | 33_fus_3577_MYC_001_ exon3 | ACGTACTGCAACTAACTGCACAA | 669 | GGTGATCCAGACTCTGACCTTTT G | 670 |
| HVP33 | 33_fus_3577_PVT1_004 _exon1 | ACGTACTGCAACTAACTGCACAA | 671 | CATGGTTCCACCAGCGTTATT | 672 |
| HVP33 | 33_sp_1316_3351 | GATGAGCTAGAAGACAGCGGATATG | 673 | GTGGTGGTCGGTTATCGTTGT | 674 |
| HVP33 | 33_unsp_894_895 | GTGCCCTACCTGTGCACAA | 675 | TTCTTCTCTCTATGACTGCTTCTA CCT | 676 |
| HVP33 | 33_unsp_2574_2575 | TGTGAAACATAGGGCATTAGTGCAATT A | 677 | CATACACTGGGTTACCATTTTCA TCAAA | 678 |
| HVP33 | 33_fus_894_PVT1_002_ exon3 | GTGCCCTACCTGTGCACAA | 679 | ATCATGATGGCTGTATGTGCCA | 680 |

(continued)

| Virus name | Transcript targeted | Forward primer (5'-3') | SEQ ID NO° | Reverse primer (5'-3') | SEQ ID NO° |
|---|---|---|---|---|---|
| HVP33 | 33_fus_894_MYC_001_e xon1 | GTGCCCTACCTGTGCACAA | 681 | CTGAGAAGCCCTGCCCTTC | 682 |
| HVP33 | 33_sp_3589_5594 | ACGTACTGCAACTAACTGCACAA | 683 | ATCAGTGCTGACAACTTTAGATA CAGG | 684 |
| HVP33 | 33_fus_3577_PVT1_005 _exon1 | ACGTACTGCAACTAACTGCACAA | 685 | TCTTTGCTCGCAGCTCGT | 686 |
| HVP33 | 33_sp_231_531 | AGCATTGGAGACAACTATACACAACAT T | 687 | CATATTCCTTTAACGTTGGCTTG TGT | 688 |
| HVP33 | 33_unsp_413_414 | ATTCTGTATATGGAAATACATTAGAACA AACAG | 689 | TCGTTTGTTTAAATCCACATGTC GTTTT | 690 |
| HVP33 | 33_fus_3577_MYC_001_ exon1 | ACGTACTGCAACTAACTGCACAA | 691 | CTGAGAAGCCCTGCCCTTC | 692 |
| HVP33 | 33_fus_3577_MYC_001_ exon2 | ACGTACTGCAACTAACTGCACAA | 693 | AAATACGGCTGCACCGAGT | 694 |
| HVP33 | 33_sp_231_414 | AGCATTGGAGACAACTATACACAACAT T | 695 | TCGTTTGTTTAAATCCACATGTC GTTTT | 696 |
| HVP33 | 33_fus_2863_MYC_001_ exon1 | GTGCAGGAGAAAATACTAGATCTTTAC GA | 697 | CTGAGAAGCCCTGCCCTTC | 698 |
| HVP33 | 33_unsp_1316_1317 | GATGAGCTAGAAGACAGCGGATATG | 699 | CATCCCCCACCCCACTAGAT | 700 |
| HVP33 | 33_fus_3577_PVT1_002 _exon3 | ACGTACTGCAACTAACTGCACAA | 701 | ATCATGATGGCTGTATGTGCCA | 702 |
| HVP33 | 33_fus_2863_PVT1_002 _exon3 | GTGCAGGAGAAAATACTAGATCTTTAC GA | 703 | ATCATGATGGCTGTATGTGCCA | 704 |
| HVP33 | 33_unsp_231_232 | AGCATTGGAGACAACTATACACAACAT T | 705 | CGCAAACACAGTTTACATATTCC AAATG | 706 |
| HVP33 | 33_fus_894_PVT1_005_ exon1 | GTGCCCTACCTGTGCACAA | 707 | TCTTTGCTCGCAGCTCGT | 708 |
| HVP33 | 33_fus_894_MYC_001_e xon2 | GTGCCCTACCTGTGCACAA | 709 | AAATACGGCTGCACCGAGT | 710 |
| HVP33 | 33_fus_2863_MYC_001_ exon3 | GTGCAGGAGAAAATACTAGATCTTTAC GA | 711 | GGTGATCCAGACTCTGACCTTTT G | 712 |
| HVP33 | 33_sp_231_2575 | AGCATTGGAGACAACTATACACAACAT T | 713 | CATACACTGGGTTACCATTTTCA TCAAA | 714 |

(continued)

| Virus name | Transcript targeted | Forward primer (5'-3') | SEQ ID NO° | Reverse primer (5'-3') | SEQ ID NO° |
|---|---|---|---|---|---|
| HVP33 | 33_fus_2863_PVT1_004 _exon1 | GTGCAGGAGAAAATACTAGATCTTTAC GA | 715 | CATGGTTCCACCAGCGTTATT | 716 |
| HVP33 | 33_sp_894_2702 | GTGCCCTACCTGTGCACAA | 717 | TGATATTTCCTCCATGGTTTTCCT TGTC | 718 |
| HVP33 | 33_sp_231_3351 | AGCATTGGAGACAACTATACACAACAT T | 719 | GTGGTGGTCGGTTATCGTTGT | 720 |
| HVP33 | 33_sp_1316_2575 | GATGAGCTAGAAGACAGCGGATATG | 721 | CATACACTGGGTTACCATTTTCA TCAAA | 722 |
| HVP33 | 33_fus_894_MYC_001_e xon3 | GTGCCCTACCTGTGCACAA | 723 | GGTGATCCAGACTCTGACCTTTT G | 724 |
| HVP33 | 33_fus_2863_PVT1_005 _exon1 | GTGCAGGAGAAAATACTAGATCTTTAC GA | 725 | TCTTTGCTCGCAGCTCGT | 726 |
| HVP33 | 33_sp_894_3351 | GTGCCCTACCTGTGCACAA | 727 | GTGGTGGTCGGTTATCGTTGT | 728 |
| HVP33 | 33_fus_894_PVT1_004_ exon1 | GTGCCCTACCTGTGCACAA | 729 | CATGGTTCCACCAGCGTTATT | 730 |
| HVP33 | 33_fus_2863_MYC_001_ exon2 | GTGCAGGAGAAAATACTAGATCTTTAC GA | 731 | AAATACGGCTGCACCGAGT | 732 |
| HVP33 | 33_sp_1316_2702 | GATGAGCTAGAAGACAGCGGATATG | 733 | TGATATTTCCTCCATGGTTTTCCT TGTC | 734 |
| HVP33 | 33_unsp_3350_3351 | GGATGCTGCAAAGTATTCTAAAACACA A | 735 | GTGGTGGTCGGTTATCGTTGT | 736 |
| HVP33 | 33_unsp_530_531 | CGATTTCATAATATTTCGGGTCGTTGG | 737 | CATATTCCTTTAACGTTGGCTTG TGT | 738 |
| HVP33 | 33_sp_894_5594 | GTGCCCTACCTGTGCACAA | 739 | ATCAGTGCTGACAACTTTAGATA CAGG | 740 |
| HVP33 | 33_unsp_3589_3590 | ACGTACTGCAACTAACTGCACAA | 741 | GCCAGGTGGATGACATAGAACT ATACA | 742 |
| HVP33 | 33_unsp_5593_5594 | TTGTTGTAGACGGTGCTGACTTT | 743 | ATCAGTGCTGACAACTTTAGATA CAGG | 744 |
| HVP33 | 33_sp_894_2575 | GTGCCCTACCTGTGCACAA | 745 | CATACACTGGGTTACCATTTTCA TCAAA | 746 |

(continued)

| Virus name | Transcript targeted | Forward primer (5'-3') | SEQ ID NO° | Reverse primer (5'-3') | SEQ ID NO° |
|---|---|---|---|---|---|
| HVP33 | 33_sp_231_2702 | AGCATTGGAGACAACTATACACAACAT T | 747 | TGATATTTCCTCCATGGTTTTCCT TGTC | 748 |
| HVP33 | 33_gen_3839_4175 | CCATTTCTACCTATGCTTGGTTGCT | 749 | GTTGTGTCATATGCTGTGCATGA AA | 750 |
| HVP33 | 33_gen_7292_7909 | CTTGCCCTACCCTGCATTG | 751 | CGGTTAGGCATACAAAATGGAG GAAAT | 752 |
| HVP33 | 33_gen_1566_2325 | CGGAGCCAAACATGTGCATTG | 753 | CGTTATCATATGCCCACTGTACC ATT | 754 |
| HVP33 | 33_gen_5844_7285 | CATGTGTAGGCCTTGAAATAGGTAGAG | 755 | CCTATTATCAGCACCCGGTTGT | 756 |
| HVP35 | 35_unsp_232_233 | CGAGGTAGAAGAAAGCATCCATGAAAT | 757 | CATACTCCATATGGCTGGCCTTC | 758 |
| HVP35 | 35_unsp_3596_3597 | TCTACATCTGACTGCACAAACAAAGA | 759 | CCATCTCCATGTAGATGAAGCAT CTTG | 760 |
| HVP35 | 35_sp_232_2670 | CGAGGTAGAAGAAAGCATCCATGAAAT | 761 | GGAAAGCGTCTCCATCATTTTCT TTG | 762 |
| HVP35 | 35_fus_2831_MYC_001_ exon3 | ATTACGAGACTGATAGCACATGTTTGT | 763 | GGTGATCCAGACTCTGACCTTTT G | 764 |
| HVP35 | 35_fus_3584_MYC_001_ exon3 | TCTACATCTGACTGCACAAACAAAGA | 765 | GGTGATCCAGACTCTGACCTTTT G | 766 |
| HVP35 | 35_fus_883_MYC_001_e xon2 | CGGCTGTTCACAGAGAGCATAAT | 767 | AAATACGGCTGCACCGAGT | 768 |
| HVP35 | 35_fus_2831_PVT1_004 _exon1 | ATTACGAGACTGATAGCACATGTTTGT | 769 | CATGGTTCCACCAGCGTTATT | 770 |
| HVP35 | 35_unsp_5600_5601 | GGGTGACTTTTATTTACACCCTAGTT | 771 | CATCAGTGCTAACAACCTTAGAC ACT | 772 |
| HVP35 | 35_fus_2831_PVT1_005 _exon1 | ATTACGAGACTGATAGCACATGTTTGT | 773 | TCTTTGCTCGCAGCTCGT | 774 |
| HVP35 | 35_sp_3596_5601 | TCTACATCTGACTGCACAAACAAAGA | 775 | CATCAGTGCTAACAACCTTAGAC ACT | 776 |
| HVP35 | 35_sp_883_2670 | CGGCTGTTCACAGAGAGCATAAT | 777 | GGAAAGCGTCTCCATCATTTTCT TTG | 778 |
| HVP35 | 35_unsp_883_884 | CGGCTGTTCACAGAGAGCATAAT | 779 | CCCGTACGTCTACTAACTACTGC TT | 780 |

(continued)

| Virus name | Transcript targeted | Forward primer (5'-3') | SEQ ID NO° | Reverse primer (5'-3') | SEQ ID NO° |
|---|---|---|---|---|---|
| HVP35 | 35_fus_2831_PVT1_002 _exon3 | ATTACGAGACTGATAGCACATGTTTGT | 781 | ATCATGATGGCTGTATGTGCCA | 782 |
| HVP35 | 35_fus_883_MYC_001_e xon3 | CGGCTGTTCACAGAGAGCATAAT | 783 | GGTGATCCAGACTCTGACCTTTT G | 784 |
| HVP35 | 35_sp_883_5601 | CGGCTGTTCACAGAGAGCATAAT | 785 | CATCAGTGCTAACAACCTTAGAC ACT | 786 |
| HVP35 | 35_fus_883_MYC_001_e xon1 | CGGCTGTTCACAGAGAGCATAAT | 787 | CTGAGAAGCCCTGCCCTTC | 788 |
| HVP35 | 35_fus_3584_PVT1_005 _exon1 | TCTACATCTGACTGCACAAACAAAGA | 789 | TCTTTGCTCGCAGCTCGT | 790 |
| HVP35 | 35_fus_2831_MYC_001_ exon2 | ATTACGAGACTGATAGCACATGTTTGT | 791 | AAATACGGCTGCACCGAGT | 792 |
| HVP35 | 35_sp_232_2543 | CGAGGTAGAAGAAAGCATCCATGAAAT | 793 | TCATTGTGAAATGTAAAGACCAC TACCC | 794 |
| HVP35 | 35_fus_2831_MYC_001_ exon1 | ATTACGAGACTGATAGCACATGTTTGT | 795 | CTGAGAAGCCCTGCCCTTC | 796 |
| HVP35 | 35_unsp_5766_5767 | CATCTACTATCATGCAGGCAGTTCT | 797 | ACTCTGTATTGCAAACCAGATAC CTTG | 798 |
| HVP35 | 35_unsp_2669_2670 | GGAAACCCAGTGTATGGGCTTAAT | 799 | GGAAAGCGTCTCCATCATTTTCT TTG | 800 |
| HVP35 | 35_fus_883_PVT1_002_ exon3 | CGGCTGTTCACAGAGAGCATAAT | 801 | ATCATGATGGCTGTATGTGCCA | 802 |
| HVP35 | 35_fus_3584_PVT1_004 _exon1 | TCTACATCTGACTGCACAAACAAAGA | 803 | CATGGTTCCACCAGCGTTATT | 804 |
| HVP35 | 35_fus_3584_PVT1_002 _exon3 | TCTACATCTGACTGCACAAACAAAGA | 805 | ATCATGATGGCTGTATGTGCCA | 806 |
| HVP35 | 35_fus_883_PVT1_005_ exon1 | CGGCTGTTCACAGAGAGCATAAT | 807 | TCTTTGCTCGCAGCTCGT | 808 |
| HVP35 | 35_fus_3584_MYC_001_ exon1 | TCTACATCTGACTGCACAAACAAAGA | 809 | CTGAGAAGCCCTGCCCTTC | 810 |
| HVP35 | 35_sp_883_3319 | CGGCTGTTCACAGAGAGCATAAT | 811 | GCTTTGGTATGGGTCTCGGT | 812 |
| HVP35 | 35_sp_1305_3319 | ATTATTTGAACTACCAGACAGCGGTT | 813 | GCTTTGGTATGGGTCTCGGT | 814 |

(continued)

| Virus name | Transcript targeted | Forward primer (5'-3') | SEQ ID NO° | Reverse primer (5'-3') | SEQ ID NO° |
|---|---|---|---|---|---|
| HVP35 | 35_sp_883_2543 | CGGCTGTTCACAGAGAGCATAAT | 815 | TCATTGTGAAATGTAAAGACCAC TACCC | 816 |
| HVP35 | 35_sp_1305_2670 | ATTATTTGAACTACCAGACAGCGGTT | 817 | GGAAAGCGTCTCCATCATTTTCT TTG | 818 |
| HVP35 | 35_sp_232_415 | CGAGGTAGAAGAAAGCATCCATGAAAT | 819 | TCCACCGATGTTATGGAATCGTT TT | 820 |
| HVP35 | 35_fus_3584_MYC_001_ exon2 | TCTACATCTGACTGCACAAACAAAGA | 821 | AAATACGGCTGCACCGAGT | 822 |
| HVP35 | 35_fus_883_PVT1_004_ exon1 | CGGCTGTTCACAGAGAGCATAAT | 823 | CATGGTTCCACCAGCGTTATT | 824 |
| HVP35 | 35_unsp_414_415 | GGAGAAACGTTAGAAAAACAATGCAAC A | 825 | TCCACCGATGTTATGGAATCGTT TT | 826 |
| HVP35 | 35_sp_232_3319 | CGAGGTAGAAGAAAGCATCCATGAAAT | 827 | GCTTTGGTATGGGTCTCGGT | 828 |
| HVP35 | 35_unsp_1305_1306 | ATTATTTGAACTACCAGACAGCGGTT | 829 | GCTACTAGAGGTTATACTATCCC CACT | 830 |
| HVP35 | 35_sp_883_5767 | CGGCTGTTCACAGAGAGCATAAT | 831 | ACTCTGTATTGCAAACCAGATAC CTTG | 832 |
| HVP35 | 35_sp_1305_2543 | ATTATTTGAACTACCAGACAGCGGTT | 833 | TCATTGTGAAATGTAAAGACCAC TACCC | 834 |
| HVP35 | 35_unsp_2542_2543 | CATTAGTGCAATTAAAATGCCCACCTT | 835 | TCATTGTGAAATGTAAAGACCAC TACCC | 836 |
| HVP35 | 35_sp_3596_5767 | TCTACATCTGACTGCACAAACAAAGA | 837 | ACTCTGTATTGCAAACCAGATAC CTTG | 838 |
| HVP35 | 35_unsp_3318_3319 | AAAATATATGGGAAGTGCATGTGGGT | 839 | GCTTTGGTATGGGTCTCGGT | 840 |
| HVP35 | 35_gen_3846_4185 | CGTTCGCTATTGCTATCTGTGTCATTA | 841 | GCCAAATATTGTGCATGAGCGTT AATC | 842 |
| HVP35 | 35_gen_7293_7879 | AACATTCCTACCTCAGCAGAACAC | 843 | TGGGTGGACCACAAGTATGAAA A | 844 |
| HVP35 | 35_gen_6017_7286 | GGTACAGATAACAGGGAATGCATTTCT | 845 | GACATTCTCCTGCTTTTACCTGG TTA | 846 |
| HVP35 | 35_gen_1555_2293 | GCTATGTATTTCAGCTGCAAGTATGCT | 847 | CATTCTGGTGTTTCTCCATCAAC CT | 848 |

(continued)

| Virus name | Transcript targeted | Forward primer (5'-3') | SEQ ID NO° | Reverse primer (5'-3') | SEQ ID NO° |
|---|---|---|---|---|---|
| HVP39 | 39_sp_943_2636 | CGTGGTGTGCAACTGCAA | 849 | CTGTTTTGGTCAAATGGAAATGC ATTAG | 850 |
| HVP39 | 39_unsp_5642_5643 | GCAATAACCATTCAGGGTTCCAATT | 851 | AGTATTGACAACCTTCGCCACA | 852 |
| HVP39 | 39_unsp_1368_1369 | GGTGTATTCCGTGCCAGACA | 853 | GTACACTGCCGCCATGTTC | 854 |
| HVP39 | 39_sp_1368_3424 | GGTGTATTCCGTGCCAGACA | 855 | GGTCGCGGTGGTGTTTGATAA | 856 |
| HVP39 | 39_sp_235_2636 | CACCACCTTGCAGGACATTACAATA | 857 | CTGTTTTGGTCAAATGGAAATGC ATTAG | 858 |
| HVP39 | 39_fus_3677_PVT1_004 _exon1 | CACAGTAACAGTACAGGCCACA | 859 | CATGGTTCCACCAGCGTTATT | 860 |
| HVP39 | 39_fus_3677_PVT1_002 _exon3 | CACAGTAACAGTACAGGCCACA | 861 | ATCATGATGGCTGTATGTGCCA | 862 |
| HVP39 | 39_unsp_801_802 | CATGCAGTTAATCACCAACATCAACT | 863 | TGCTGTAGTTGTCGCAGAGTATC | 864 |
| HVP39 | 39_sp_235_418 | CACCACCTTGCAGGACATTACAATA | 865 | CTGTCCTGTATAGCTTCCTGCTA TTTT | 866 |
| HVP39 | 39_unsp_943_944 | CGTGGTGTGCAACTGCAA | 867 | CACTGTGTCGCCTGTTTGTTTAT | 868 |
| HVP39 | 39_fus_943_MYC_001_e xon2 | CGTGGTGTGCAACTGCAA | 869 | AAATACGGCTGCACCGAGT | 870 |
| HVP39 | 39_unsp_2635_2636 | ATTAGATGGGTATGCAATAAGTTTAGA TAGG | 871 | CTGTTTTGGTCAAATGGAAATGC ATTAG | 872 |
| HVP39 | 39_fus_2927_MYC_001_ exon2 | ACAACGTTTAAATGTGTTACAGGACA | 873 | AAATACGGCTGCACCGAGT | 874 |
| HVP39 | 39_fus_2927_PVT1_002 _exon3 | ACAACGTTTAAATGTGTTACAGGACA | 875 | ATCATGATGGCTGTATGTGCCA | 876 |
| HVP39 | 39_fus_943_PVT1__002_ exon3 | CGTGGTGTGCAACTGCAA | 877 | ATCATGATGGCTGTATGTGCCA | 878 |
| HVP39 | 39_fus_943_PVTI_005_ exon1 | CGTGGTGTGCAACTGCAA | 879 | TCTTTGCTCGCAGCTCGT | 880 |
| HVP39 | 39_fus_943_MYC_001_e xon3 | CGTGGTGTGCAACTGCAA | 881 | GGTGATCCAGACTCTGACCTTTT G | 882 |
| HVP39 | 39_fus_2927_PVT1_005 _exon1 | ACAACGTTTAAATGTGTTACAGGACA | 883 | TCTTTGCTCGCAGCTCGT | 884 |

(continued)

| Virus name | Transcript targeted | Forward primer (5'-3') | SEQ ID NO° | Reverse primer (5'-3') | SEQ ID NO° |
|---|---|---|---|---|---|
| HVP39 | 39_fus_943_MYC_001_e xon1 | CGTGGTGTGCAACTGCAA | 885 | CTGAGAAGCCCTGCCCTTC | 886 |
| HVP39 | 39_sp_943_5643 | CGTGGTGTGCAACTGCAA | 887 | AGTATTGACAACCTTCGCCACA | 888 |
| HVP39 | 39_unsp_3689_3690 | CACAGTAACAGTACAGGCCACA | 889 | CGTATCCAATGCCAGGTACATG AAA | 890 |
| HVP39 | 39_sp_3689_5643 | CACAGTAACAGTACAGGCCACA | 891 | AGTATTGACAACCTTCGCCACA | 892 |
| HVP39 | 39_fus_3677_PVT1_005 _exon1 | CACAGTAACAGTACAGGCCACA | 893 | TCTTTGCTCGCAGCTCGT | 894 |
| HVP39 | 39_unsp_417_418 | CTCGGACTCGGTGTATGCAA | 895 | CTGTCCTGTATAGCTTCCTGCTA TTTT | 896 |
| HVP39 | 39_fus_943_PVT1_004_ exon1 | CGTGGTGTGCAACTGCAA | 897 | CATGGTTCCACCAGCGTTATT | 898 |
| HVP39 | 39_sp_943_3424 | CGTGGTGTGCAACTGCAA | 899 | GGTCGCGGTGGTGTTTGATAA | 900 |
| HVP39 | 39_unsp_235_236 | CACCACCTTGCAGGACATTACAATA | 901 | GATTGGCATGCAGCTAGTGG | 902 |
| HVP39 | 39_sp_235_802 | CACCACCTTGCAGGACATTACAATA | 903 | TGCTGTAGTTGTCGCAGAGTATC | 904 |
| HVP39 | 39_fus_2927_MYC_001_ exon3 | ACAACGTTTAAATGTGTTACAGGACA | 905 | GGTGATCCAGACTCTGACCTTTT G | 906 |
| HVP39 | 39_fus_2927_MYC_001_ exon1 | ACAACGTTTAAATGTGTTACAGGACA | 907 | CTGAGAAGCCCTGCCCTTC | 908 |
| HVP39 | 39_fus_3677_MYC_001_ exon2 | CACAGTAACAGTACAGGCCACA | 909 | AAATACGGCTGCACCGAGT | 910 |
| HVP39 | 39_sp_1368_2636 | GGTGTATTCCGTGCCAGACA | 911 | CTGTTTTGGTCAAATGGAAATGC ATTAG | 912 |
| HVP39 | 39_fus_3677_MYC_001_ exon1 | CACAGTAACAGTACAGGCCACA | 913 | CTGAGAAGCCCTGCCCTTC | 914 |
| HVP39 | 39_fus_2927_PVT1_004 _exon1 | ACAACGTTTAAATGTGTTACAGGACA | 915 | CATGGTTCCACCAGCGTTATT | 916 |
| HVP39 | 39_fus_3677_MYC_001_ exon3 | CACAGTAACAGTACAGGCCACA | 917 | GGTGATCCAGACTCTGACCTTTT G | 918 |
| HVP39 | 39_sp_235_3424 | CACCACCTTGCAGGACATTACAATA | 919 | GGTCGCGGTGGTGTTTGATAA | 920 |

(continued)

| Virus name | Transcript targeted | Forward primer (5'-3') | SEQ ID NO° | Reverse primer (5'-3') | SEQ ID NO° |
|---|---|---|---|---|---|
| HVP39 | 39_gen_3939_4242 | TTGGTGTGGTTTGGTGTGTGTATAT | 921 | CTCCAATGGTGTGGTACGTATAA GAA | 922 |
| HVP39 | 39_gen_7267_7833 | CATTTTGTGGCGACCGAAGT | 923 | CCTGGACAGGATGATGAGTAAT AAGG | 924 |
| HVP39 | 39_gen_1618_2386 | AGGGTTACTGTAGGAAAGGGATTAAGT | 925 | CGTATCCCCTGTTACCACACTAA TATTG | 926 |
| HVP39 | 39_gen_5893_7260 | CCAGCCATTGGGTGTTGGTA | 927 | GCCTATAATGCACAACTGTGTCT GTT | 928 |
| HVP45 | 45_fus_929_MYC_001_e xon3 | AGCACCTTGTCCTTTGTGTGT | 929 | GGTGATCCAGACTCTGACCTTTT G | 930 |
| HVP45 | 45_fus_2901_PVT1_004 _exon1 | CGTTACAGGACAAAATACTAGACCACT A | 931 | CATGGTTCCACCAGCGTTATT | 932 |
| HVP45 | 45_sp_929_2610 | AGCACCTTGTCCTTTGTGTGT | 933 | GAAATGCATGTGGAAATGTAAA TACCGT | 934 |
| HVP45 | 45_fus_3648_MYC_001_ exon2 | TCCTGTGTTCAAGTACAAGTAACAACA A | 935 | AAATACGGCTGCACCGAGT | 936 |
| HVP45 | 45_sp_1357_2610 | TCAGATAGTGGCTATGGCTGTTCT | 937 | GAAATGCATGTGGAAATGTAAA TACCGT | 938 |
| HVP45 | 45_fus_3648_MYC_001_ exon3 | TCCTGTGTTCAAGTACAAGTAACAACA A | 939 | GGTGATCCAGACTCTGACCTTTT G | 940 |
| HVP45 | 45_sp_230_791 | CTACAAGACGTATCTATTGCCTGTGT | 941 | TCAAAAACAGCTGCTGTAGTGTT CT | 942 |
| HVP45 | 45_sp_929_3423 | AGCACCTTGTCCTTTGTGTGT | 943 | CCCACGGATGCGGTTTTG | 944 |
| HVP45 | 45_unsp_412_413 | AAACTCTGTATATGGAGAGACACTGGA | 945 | CGTTTGTCCTTAAGGTGTCTACG TTTT | 946 |
| HVP45 | 45_sp_230_413 | CTACAAGACGTATCTATTGCCTGTGT | 947 | CGTTTGTCCTTAAGGTGTCTACG TTTT | 948 |
| HVP45 | 45_sp_230_2737 | CTACAAGACGTATCTATTGCCTGTGT | 949 | GGATTCCTTCGGTGTCTGCAT | 950 |
| HVP45 | 45_fus_2901_MYC_001_ exon1 | CGTTACAGGACAAAATACTAGACCACT A | 951 | CTGAGAAGCCCTGCCCTTC | 952 |

(continued)

| Virus name | Transcript targeted | Forward primer (5'-3') | SEQ ID NO° | Reverse primer (5'-3') | SEQ ID NO° |
|---|---|---|---|---|---|
| HVP45 | 45_unsp_230_231 | CTACAAGACGTATCTATTGCCTGTGT | 953 | AAGTCTATACATTTATGGCATGC AGCATA | 954 |
| HVP45 | 45_fus_929_PVT1_004_ exon1 | AGCACCTTGTCCTTTGTGTGT | 955 | CATGGTTCCACCAGCGTTATT | 956 |
| HVP45 | 45_sp_1357_3423 | TCAGATAGTGGCTATGGCTGTTCT | 957 | CCCACGGATGCGGTTTTG | 958 |
| HVP45 | 45_fus_929_MYC_001_e xon2 | AGCACCTTGTCCTTTGTGTGT | 959 | AAATACGGCTGCACCGAGT | 960 |
| HVP45 | 45_fus_929_PVT1_005_ exon1 | AGCACCTTGTCCTTTGTGTGT | 961 | TCTTTGCTCGCAGCTCGT | 962 |
| HVP45 | 45_sp_230_3423 | CTACAAGACGTATCTATTGCCTGTGT | 963 | CCCACGGATGCGGTTTTG | 964 |
| HVP45 | 45_unsp_2609_2610 | CATTATTACAGCTAAAATGTCCTCCAAT CC | 965 | GAAATGCATGTGGAAATGTAAA TACCGT | 966 |
| HVP45 | 45_fus_3648_PVT1_002 _exon3 | TCCTGTGTTCAAGTACAAGTAACAACA A | 967 | ATCATGATGGCTGTATGTGCCA | 968 |
| HVP45 | 45_fus_2901_PVT1_002 _exon3 | CGTTACAGGACAAAATACTAGACCACT A | 969 | ATCATGATGGCTGTATGTGCCA | 970 |
| HVP45 | 45_fus_2901_MYC_001_ exon2 | CGTTACAGGACAAAATACTAGACCACT A | 971 | AAATACGGCTGCACCGAGT | 972 |
| HVP45 | 45_unsp_790_791 | GGAGTTAGTCATGCACAACTACCA | 973 | TCAAAAACAGCTGCTGTAGTGTT CT | 974 |
| HVP45 | 45_sp_929_2737 | AGCACCTTGTCCTTTGTGTGT | 975 | GGATTCCTTCGGTGTCTGCAT | 976 |
| HVP45 | 45_fus_3648_PVT1_005 _exon1 | TCCTGTGTTCAAGTACAAGTAACAACA A | 977 | TCTTTGCTCGCAGCTCGT | 978 |
| HVP45 | 45_sp_929_5608 | AGCACCTTGTCCTTTGTGTGT | 979 | GCTGACAACTCTGGCCACA | 980 |
| HVP45 | 45_sp_230_2610 | CTACAAGACGTATCTATTGCCTGTGT | 981 | GAAATGCATGTGGAAATGTAAA TACCGT | 982 |
| HVP45 | 45_unsp_5607_5608 | GCACACAATATTATTTATGGCCATGGTA | 983 | GCTGACAACTCTGGCCACA | 984 |
| HVP45 | 45_fus_2901_PVT1_005 _exon1 | CGTTACAGGACAAAATACTAGACCACT A | 985 | TCTTTGCTCGCAGCTCGT | 986 |
| HVP45 | 45_unsp_929_930 | AGCACCTTGTCCTTTGTGTGT | 987 | CAATTGTTTCTACAAAGAACCAG CCATT | 988 |

(continued)

| Virus name | Transcript targeted | Forward primer (5'-3') | SEQ ID NO° | Reverse primer (5'-3') | SEQ ID NO° |
|---|---|---|---|---|---|
| HVP45 | 45_fus_2901_MYC_001_ exon3 | CGTTACAGGACAAAATACTAGACCACT A | 989 | GGTGATCCAGACTCTGACCTTTT G | 990 |
| HVP45 | 45_fus_929_MYC_001_e xon1 | AGCACCTTGTCCTTTGTGTGT | 991 | CTGAGAAGCCCTGCCCTTC | 992 |
| HVP45 | 45_sp_1357_2737 | TCAGATAGTGGCTATGGCTGTTCT | 993 | GGATTCCTTCGGTGTCTGCAT | 994 |
| HVP45 | 45_unsp_1357_1358 | TCAGATAGTGGCTATGGCTGTTCT | 995 | ACTATCCCCACCACTACTTTGTG TA | 996 |
| HVP45 | 45_fus_3648_PVT1_004 _exon1 | TCCTGTGTTCAAGTACAAGTAACAACA A | 997 | CATGGTTCCACCAGCGTTATT | 998 |
| HVP45 | 45_sp_3660_5608 | TCCTGTGTTCAAGTACAAGTAACAACA A | 999 | GCTGACAACTCTGGCCACA | 1000 |
| HVP45 | 45_fus_3648_MYC_001_ exon1 | TCCTGTGTTCAAGTACAAGTAACAACA A | 1001 | CTGAGAAGCCCTGCCCTTC | 1002 |
| HVP45 | 45_fus_929_PVT1_002_ exon3 | AGCACCTTGTCCTTTGTGTGT | 1003 | ATCATGATGGCTGTATGTGCCA | 1004 |
| HVP45 | 45_gen_3910_4227 | TGCTTTTGCTTGGTTGTTGGT | 1005 | CATCACAGGTATGTTACACTGTA CTGT | 1006 |
| HVP45 | 45_unsp_3660_3661 | TCCTGTGTTCAAGTACAAGTAACAACA A | 1007 | GGTCTGCATATTTGCGTAGCCTA TA | 1008 |
| HVP45 | 45_gen_7316_7858 | ATTTCGGTTGCCTGTGGCTTATA | 1009 | CAGTTGTGCAAGCCATTGTTTTA GT | 1010 |
| HVP45 | 45_sp_3750_5608 | CGCAAATATGCAGACCATTACTCAGAA | 1011 | GCTGACAACTCTGGCCACA | 1012 |
| HVP45 | 45_gen_1607_2360 | GCAACGTTATACGCCCATATCCAAT | 1013 | GGTACGTGCAACAATGTGCTTA A | 1014 |
| HVP45 | 45_unsp_3750_3751 | CGCAAATATGCAGACCATTACTCAGAA | 1015 | CCCACCGAGATTTGTACACTGTT A | 1016 |
| HVP45 | 45_gen_5858_7309 | GGCATGTGTAGGTATGGAAATTGGT | 1017 | ACATCCTGCGTAATAACAGCTGT AG | 1018 |
| HVP45 | 45_unsp_3422_3423 | TGACGACACGGTATCCGCTA | 1019 | CCCACGGATGCGGTTTTG | 1020 |
| HVP51 | 51_unsp_2547_2548 | AGTATGTCCACCATTACTAATAACGTCA AAC | 1021 | TCATTCAATGTATACACAGCATT CCCAT | 1022 |

(continued)

| Virus name | Transcript targeted | Forward primer (5'-3') | SEQ ID NO° | Reverse primer (5'-3') | SEQ ID NO° |
|---|---|---|---|---|---|
| HVP51 | 51_fus_3572_MYC_001_ exon2 | CTAACACTGGAGGGCACCAAA | 1023 | AAATACGGCTGCACCGAGT | 1024 |
| HVP51 | 51_fus_886_PVT1_002_ exon3 | GGGCGAACTAAGCCTGGTTT | 1025 | ATCATGATGGCTGTATGTGCCA | 1026 |
| HVP51 | 51_fus_886_MYC_001_e xon2 | GGGCGAACTAAGCCTGGTTT | 1027 | AAATACGGCTGCACCGAGT | 1028 |
| HVP51 | 51_fus_3572_PVT1_005 _exon1 | CTAACACTGGAGGGCACCAAA | 1029 | TCTTTGCTCGCAGCTCGT | 1030 |
| HVP51 | 51_fus_2834_MYC_001_ exon1 | GTGCCAGGAGAAAATACTAGACTGTTA T | 1031 | CTGAGAAGCCCTGCCCTTC | 1032 |
| HVP51 | 51_unsp_3584_3585 | CTAACACTGGAGGGCACCAAA | 1033 | ATGCCAGGTTGAGGATACGTTTT TAT | 1034 |
| HVP51 | 51_fus_2834_PVT1_004 _exon1 | GTGCCAGGAGAAAATACTAGACTGTTA T | 1035 | CATGGTTCCACCAGCGTTATT | 1036 |
| HVP51 | 51_unsp_1302_1303 | CGGACAGCGGATATGGCAATA | 1037 | TCTGTTGTTTCCACATCCATAAC ACT | 1038 |
| HVP51 | 51_fus_3572_PVT1_002 _exon3 | CTAACACTGGAGGGCACCAAA | 1039 | ATCATGATGGCTGTATGTGCCA | 1040 |
| HVP51 | 51_sp_3584_5521 | CTAACACTGGAGGGCACCAAA | 1041 | CAATTCGAGACACAGGTGCAG | 1042 |
| HVP51 | 51_unsp_401_402 | GAGAGTATAGACGTTATAGCAGGTCTG T | 1043 | TCCCGCTATTTCATGGAACCTTTT | 1044 |
| HVP51 | 51_sp_1302_3319 | CGGACAGCGGATATGGCAATA | 1045 | CCACGCAGGTGGTAAGGG | 1046 |
| HVP51 | 51_sp_217_751 | CTGCATGAATTATGTGAAGCTTTGAAC | 1047 | CATCTGCTGTACAACGCGAAG | 1048 |
| HVP51 | 51_fus_886_MYC_001_e xon3 | GGGCGAACTAAGCCTGGTTT | 1049 | GGTGATCCAGACTCTGACCTTTT G | 1050 |
| HVP51 | 51_fus_3572_PVT1_004 _exon1 | CTAACACTGGAGGGCACCAAA | 1051 | CATGGTTCCACCAGCGTTATT | 1052 |
| HVP51 | 51_sp_1302_2548 | CGGACAGCGGATATGGCAATA | 1053 | TCATTCAATGTATACACAGCATT CCCAT | 1054 |
| HVP51 | 51_unsp_886_887 | GGGCGAACTAAGCCTGGTTT | 1055 | CTCATCATCCGAAACATTATCTC CTGT | 1056 |

(continued)

| Virus name | Transcript targeted | Forward primer (5'-3') | SEQ ID NO° | Reverse primer (5'-3') | SEQ ID NO° |
|---|---|---|---|---|---|
| HVP51 | 51_fus_2834_PVT1_002 _exon3 | GTGCCAGGAGAAAATACTAGACTGTTA T | 1057 | ATCATGATGGCTGTATGTGCCA | 1058 |
| HVP51 | 51_unsp_3318_3319 | GCACAACAGTGGGAGGTCTATATG | 1059 | CCACGCAGGTGGTAAGGG | 1060 |
| HVP51 | 51_fus_2834_MYC_001_ exon2 | GTGCCAGGAGAAAATACTAGACTGTTA T | 1061 | AAATACGGCTGCACCGAGT | 1062 |
| HVP51 | 51_sp_886_5521 | GGGCGAACTAAGCCTGGTTT | 1063 | CAATTCGAGACACAGGTGCAG | 1064 |
| HVP51 | 51_fus_3572_MYC_001_ exon3 | CTAACACTGGAGGGCACCAAA | 1065 | GGTGATCCAGACTCTGACCTTTT G | 1066 |
| HVP51 | 51_sp_217_402 | CTGCATGAATTATGTGAAGCTTTGAAC | 1067 | TCCCGCTATTTCATGGAACCTTTT | 1068 |
| HVP51 | 51_unsp_750_751 | GCGTGACCAGCTACCAGAAA | 1069 | CATCTGCTGTACAACGCGAAG | 1070 |
| HVP51 | 51_fus_886_PVT1_004_ exon1 | GGGCGAACTAAGCCTGGTTT | 1071 | CATGGTTCCACCAGCGTTATT | 1072 |
| HVP51 | 51_fus_3572_MYC_001_ exon1 | CTAACACTGGAGGGCACCAAA | 1073 | CTGAGAAGCCCTGCCCTTC | 1074 |
| HVP51 | 51_unsp_217_218 | CTGCATGAATTATGTGAAGCTTTGAAC | 1075 | GTAAACATTGTTTGCATACTGCA TATGGA | 1076 |
| HVP51 | 51_fus_886_MYC_001_e xon1 | GGGCGAACTAAGCCTGGTTT | 1077 | CTGAGAAGCCCTGCCCTTC | 1078 |
| HVP51 | 51_sp_886_3319 | GGGCGAACTAAGCCTGGTTT | 1079 | CCACGCAGGTGGTAAGGG | 1080 |
| HVP51 | 51_fus_2834_MYC_001_ exon3 | GTGCCAGGAGAAAATACTAGACTGTTA T | 1081 | GGTGATCCAGACTCTGACCTTTT G | 1082 |
| HVP51 | 51_fus_2834_PVT1_005 _exon1 | GTGCCAGGAGAAAATACTAGACTGTTA T | 1083 | TCTTTGCTCGCAGCTCGT | 1084 |
| HVP51 | 51_fus_886_PVT1_005_ exon1 | GGGCGAACTAAGCCTGGTTT | 1085 | TCTTTGCTCGCAGCTCGT | 1086 |
| HVP51 | 51_unsp_5520_5521 | GGCCCTATACACATTTACTACGCAAA | 1087 | CAATTCGAGACACAGGTGCAG | 1088 |
| HVP51 | 51_sp_217_3319 | CTGCATGAATTATGTGAAGCTTTGAAC | 1089 | CCACGCAGGTGGTAAGGG | 1090 |
| HVP51 | 51_sp_886_2548 | GGGCGAACTAAGCCTGGTTT | 1091 | TCATTCAATGTATACACAGCATT CCCAT | 1092 |
| HVP51 | 51_sp_217_2548 | CTGCATGAATTATGTGAAGCTTTGAAC | 1093 | TCATTCAATGTATACACAGCATT CCCAT | 1094 |

(continued)

| Virus name | Transcript targeted | Forward primer (5'-3') | SEQ ID NO° | Reverse primer (5'-3') | SEQ ID NO° |
|---|---|---|---|---|---|
| HVP51 | 51_gen_7175_7808 | GGGTATTACATTATCCCCGTAGGTCAA | 1095 | GCTGCAGCTGTAACAAAATGGA A | 1096 |
| HVP51 | 51_gen_3834_4103 | AAGCCAATATGTGCTGCTAATTGTA | 1097 | AACACGTATTGGGACAGCAGTA G | 1098 |
| HVP51 | 51_gen_1552_2298 | GATGGAGGCAACTGGAGAGAAATT | 1099 | GTGTTTGGTGGGCCATATATGA CTAT | 1100 |
| HVP51 | 51_gen_5771_7168 | ACACCCCTCCACAGGCTAA | 1101 | TGTACGCCAACCTGCAACAA | 1102 |
| HVP52 | 52_unsp_523_524 | GACATGTTAATGCAAACAAGCGATTTC | 1103 | TCAGTTGTTTCAGGTTGCAGATC TAATA | 1104 |
| HVP52 | 52_fus_879_MYC_001_e xon1 | GCTGTTGGGCACATTACAAGTT | 1105 | CTGAGAAGCCCTGCCCTTC | 1106 |
| HVP52 | 52_fus_2857_PVT1_005 _exon1 | GCTGATAGTAATGACCTAAACGCACAA A | 1107 | TCTTTGCTCGCAGCTCGT | 1108 |
| HVP52 | 52_fus_879_PVT1_004_ exon1 | GCTGTTGGGCACATTACAAGTT | 1109 | CATGGTTCCACCAGCGTTATT | 1110 |
| HVP52 | 52_sp_224_524 | AGAATCGGTGCATGAAATAAGGCT | 1111 | TCAGTTGTTTCAGGTTGCAGATC TAATA | 1112 |
| HVP52 | 52_sp_3625_5643 | TCACTGCAACTGAGTGCACAA | 1113 | TGCTTACAACCTTAGAGACAGGT ACA | 1114 |
| HVP52 | 52_fus_3613_PVT1_005 _exon1 | TCACTGCAACTGAGTGCACAA | 1115 | TCTTTGCTCGCAGCTCGT | 1116 |
| HVP52 | 52_unsp_5642_5643 | TTTTACTACGTCGCAGGCGTAA | 1117 | TGCTTACAACCTTAGAGACAGGT ACA | 1118 |
| HVP52 | 52_sp_224_407 | AGAATCGGTGCATGAAATAAGGCT | 1119 | CGCTTGTTTGCATTAACATGTCT TTCT | 1120 |
| HVP52 | 52_unsp_3625_3626 | TCACTGCAACTGAGTGCACAA | 1121 | TGCCAGGTAGATGAAATTTGAA CATACA | 1122 |
| HVP52 | 52_fus_879_MYC_001_e xon3 | GCTGTTGGGCACATTACAAGTT | 1123 | GGTGATCCAGACTCTGACCTTTT G | 1124 |
| HVP52 | 52_fus_879_MYC_001_e xon2 | GCTGTTGGGCACATTACAAGTT | 1125 | AAATACGGCTGCACCGAGT | 1126 |
| HVP52 | 52_sp_879_3345 | GCTGTTGGGCACATTACAAGTT | 1127 | GCGGAGGTCTTGGAGGTTT | 1128 |

(continued)

| Virus name | Transcript targeted | Forward primer (5'-3') | SEQ ID NO° | Reverse primer (5'-3') | SEQ ID NO° |
|---|---|---|---|---|---|
| HVP52 | 52_fus_3613_PVT1_002_exon3 | TCACTGCAACTGAGTGCACAA | 1129 | ATCATGATGGCTGTATGTGCCA | 1130 |
| HVP52 | 52_fus_2857_MYC_001_exon3 | GCTGATAGTAATGACCTAAACGCACAAA | 1131 | GGTGATCCAGACTCTGACCTTTTG | 1132 |
| HVP52 | 52_fus_879_PVT1_002_exon3 | GCTGTTGGGCACATTACAAGTT | 1133 | ATCATGATGGCTGTATGTGCCA | 1134 |
| HVP52 | 52_sp_224_738 | AGAATCGGTGCATGAAATAAGGCT | 1135 | GCATTTGCTGTAGAGTACGAAGGT | 1136 |
| HVP52 | 52_sp_224_3345 | AGAATCGGTGCATGAAATAAGGCT | 1137 | GCGGAGGTCTTGGAGGTTT | 1138 |
| HVP52 | 52_sp_1301_2569 | CAAACCATGTCACGTAGAAGACAG | 1139 | GGGTTTTTGAAATGAAACACAACCAATC | 1140 |
| HVP52 | 52_unsp_737_738 | GATGAGGAGGATACAGATGGTGTG | 1141 | GCATTTGCTGTAGAGTACGAAGGT | 1142 |
| HVP52 | 52_unsp_1301_1302 | CAAACCATGTCACGTAGAAGACAG | 1143 | CCCCACCCCACTTGATTGA | 1144 |
| HVP52 | 52_sp_1301_2696 | CAAACCATGTCACGTAGAAGACAG | 1145 | CGGTATCGACTCCATCGTTTTCC | 1146 |
| HVP52 | 52_fus_2857_MYC_001_exon1 | GCTGATAGTAATGACCTAAACGCACAAA | 1147 | CTGAGAAGCCCTGCCCTTC | 1148 |
| HVP52 | 52_sp_879_5810 | GCTGTTGGGCACATTACAAGTT | 1149 | CCTGTATTGCAGGCCAGACA | 1150 |
| HVP52 | 52_unsp_224_225 | AGAATCGGTGCATGAAATAAGGCT | 1151 | CACACGCCATATGGATTATTGTCTCTA | 1152 |
| HVP52 | 52_sp_879_2696 | GCTGTTGGGCACATTACAAGTT | 1153 | CGGTATCGACTCCATCGTTTTCC | 1154 |
| HVP52 | 52_fus_3613_MYC_001_exon3 | TCACTGCAACTGAGTGCACAA | 1155 | GGTGATCCAGACTCTGACCTTTTG | 1156 |
| HVP52 | 52_unsp_879_880 | GCTGTTGGGCACATTACAAGTT | 1157 | TCCTCTGAAATGTTATCTCCTGTTTGTT | 1158 |
| HVP52 | 52_unsp_2568_2569 | CCTTAGTACAAATAAAATGCCCACCAT | 1159 | GGGTTTTTGAAATGAAACACAACCAATC | 1160 |
| HVP52 | 52_fus_2857_PVT1_002_exon3 | GCTGATAGTAATGACCTAAACGCACAAA | 1161 | ATCATGATGGCTGTATGTGCCA | 1162 |
| HVP52 | 52_sp_224_2696 | AGAATCGGTGCATGAAATAAGGCT | 1163 | CGGTATCGACTCCATCGTTTTCC | 1164 |

(continued)

| Virus name | Transcript targeted | Forward primer (5'-3') | SEQ ID NO° | Reverse primer (5'-3') | SEQ ID NO° |
|---|---|---|---|---|---|
| HVP52 | 52_fus_2857_MYC_001_exon2 | GCTGATAGTAATGACCTAAACGCACAA A | 1165 | AAATACGGCTGCACCGAGT | 1166 |
| HVP52 | 52_fus_879_PVT1_005_exon1 | GCTGTTGGGCACATTACAAGTT | 1167 | TCTTTGCTCGCAGCTCGT | 1168 |
| HVP52 | 52_unsp_3344_3345 | GTAACAGGAGTATGGGAAGTACATGT G | 1169 | GCGGAGGTCTTGGAGGTTT | 1170 |
| HVP52 | 52_unsp_406_407 | GTATGGGAAAACATTAGAAGAGAGGG T | 1171 | CGCTTGTTTGCATTAACATGTCT TTCT | 1172 |
| HVP52 | 52_sp_1301_3345 | CAAACCATGTCACGTAGAAGACAG | 1173 | GCGGAGGTCTTGGAGGTTT | 1174 |
| HVP52 | 52_fus_3613_MYC_001_exon2 | TCACTGCAACTGAGTGCACAA | 1175 | AAATACGGCTGCACCGAGT | 1176 |
| HVP52 | 52_sp_879_2569 | GCTGTTGGGCACATTACAAGTT | 1177 | GGGTTTTTGAAATGAAACACAA CCAATC | 1178 |
| HVP52 | 52_sp_224_2569 | AGAATCGGTGCATGAAATAAGGCT | 1179 | GGGTTTTTGAAATGAAACACAA CCAATC | 1180 |
| HVP52 | 52_fus_ 3613_PVT1_004_exon1 | TCACTGCAACTGAGTGCACAA | 1181 | CATGGTTCCACCAGCGTTATT | 1182 |
| HVP52 | 52_sp_3625_5810 | TCACTGCAACTGAGTGCACAA | 1183 | CCTGTATTGCAGGCCAGACA | 1184 |
| HVP52 | 52_sp_879_5643 | GCTGTTGGGCACATTACAAGTT | 1185 | TGCTTACAACCTTAGAGACAGGT ACA | 1186 |
| HVP52 | 52_unsp_5809_5810 | AAGCATCTATTATTATGCAGGCAGTTCT | 1187 | CCTGTATTGCAGGCCAGACA | 1188 |
| HVP52 | 52_fus_3613_MYC_001_exon1 | TCACTGCAACTGAGTGCACAA | 1189 | CTGAGAAGCCCTGCCCTTC | 1190 |
| HVP52 | 52_fus_ 2857_PVT1_004_exon1 | GCTGATAGTAATGACCTAAACGCACAA A | 1191 | CATGGTTCCACCAGCGTTATT | 1192 |
| HVP52 | 52_gen_6060_7338 | GGACTATATGTTTTGGGAGGTGGATTT | 1193 | GATGCAGGGCGTTTTAGTTTGG | 1194 |
| HVP52 | 52_gen_1551_2319 | CACCATCAGTTGCAGAAGGATTAAAAG | 1195 | CTGTGACATTAGTTTGGACACTG TT | 1196 |
| HVP52 | 52_gen_7345_7942 | TCGGTTGGTCTTGGCACAA | 1197 | TTTAGGCGGGACAACAAGTGT | 1198 |
| HVP52 | 52_gen_3875_4225 | CAACACAAGCCAATATTGCTGCTA | 1199 | CCTGCGCATACACCGATATAGAT | 1200 |
| HVP56 | 56_unsp_910_911 | GTTAACAGTAACGTGCCCACTCT | 1201 | TTCTACAATTGCCTCTACTTCAAA CCAT | 1202 |

(continued)

| Virus name | Transcript targeted | Forward primer (5'-3') | SEQ ID NO° | Reverse primer (5'-3') | SEQ ID NO° |
|---|---|---|---|---|---|
| HVP56 | 56_fus_3629_MYC_001_exon2 | ACAACAACCACCCTGGTGATAAG | 1203 | AAATACGGCTGCACCGAGT | 1204 |
| HVP56 | 56_fus_ 2861_PVT1_002_exon3 | GTGCCAGAACAAAATACTAGACTGTTT | 1205 | ATCATGATGGCTGTATGTGCCA | 1206 |
| HVP56 | 56_fus_910_MYC_001_exon3 | GTTAACAGTAACGTGCCCACTCT | 1207 | GGTGATCCAGACTCTGACCTTTT G | 1208 |
| HVP56 | 56_sp_910_2572 | GTTAACAGTAACGTGCCCACTCT | 1209 | TGAAACTGAAACACTAACATTCT ACTGTGT | 1210 |
| HVP56 | 56_fus_2861_PVT1_005 _exon1 | GTGCCAGAACAAAATACTAGACTGTTT | 1211 | TCTTTGCTCGCAGCTCGT | 1212 |
| HVP56 | 56_fus_2861_PVT1_004 _exon1 | GTGCCAGAACAAAATACTAGACTGTTT | 1213 | CATGGTTCCACCAGCGTTATT | 1214 |
| HVP56 | 56_fus_3629_MYC_001_ exon1 | ACAACAACCACCCTGGTGATAAG | 1215 | CTGAGAAGCCCTGCCCTTC | 1216 |
| HVP56 | 56_unsp_3641_3642 | ACAACAACCACCCTGGTGATAAG | 1217 | TATTGTCTGTACTTGTCCAATGA TATGT | 1218 |
| HVP56 | 56_unsp_532_533 | TGCATTGTGACAGAAAAAGACGATTTC | 1219 | ACGTCTTGCAGCGTTGGTA | 1220 |
| HVP56 | 56_unsp_2698_2699 | AGAATGTTAGTGTTTCAGTTTCAAAATC C | 1221 | TTTTCTTTGTCCTCGTCGTTATCC AA | 1222 |
| HVP56 | 56_fus_3629_PVT1_002 _exon3 | ACAACAACCACCCTGGTGATAAG | 1223 | ATCATGATGGCTGTATGTGCCA | 1224 |
| HVP56 | 56_fus_2861_MYC_001_ exon1 | GTGCCAGAACAAAATACTAGACTGTTT | 1225 | CTGAGAAGCCCTGCCCTTC | 1226 |
| HVP56 | 56_sp_233_2572 | GCACCACTTGAGTGAGGTATTAGAA | 1227 | TGAAACTGAAACACTAACATTCT ACTGTGT | 1228 |
| HVP56 | 56_unsp_772_773 | ACAGCAAGCTAGACAAGCTAAACAA | 1229 | TGTACAACACGCAGGTCCTC | 1230 |
| HVP56 | 56_fus_910_MYC_001_e xon1 | GTTAACAGTAACGTGCCCACTCT | 1231 | CTGAGAAGCCCTGCCCTTC | 1232 |
| HVP56 | 56_sp_910_5597 | GTTAACAGTAACGTGCCCACTCT | 1233 | ACAACCTTTGAAACAGGTGTTG GA | 1234 |
| HVP56 | 56_sp_233_533 | GCACCACTTGAGTGAGGTATTAGAA | 1235 | ACGTCTTGCAGCGTTGGTA | 1236 |

(continued)

| Virus name | Transcript targeted | Forward primer (5'-3') | SEQ ID NO° | Reverse primer (5'-3') | SEQ ID NO° |
|---|---|---|---|---|---|
| HVP56 | 56_fus_2861_MYC_001_ exon3 | GTGCCAGAACAAAATACTAGACTGTTT | 1237 | GGTGATCCAGACTCTGACCTTTT G | 1238 |
| HVP56 | 56_unsp_5758_5759 | ATCATGCAGGCAGTTCACGA | 1239 | CAACCGTACCCTAAATACCCTAT ATTGA | 1240 |
| HVP56 | 56_sp_1295_3349 | CAAGACAGCGGGTATGGCAATA | 1241 | GGTGGTGGTGGTGGTCTT | 1242 |
| HVP56 | 56_sp_1295_2699 | CAAGACAGCGGGTATGGCAATA | 1243 | TTTTCTTTGTCCTCGTCGTTATCC AA | 1244 |
| HVP56 | 56_unsp_233_234 | GCACCACTTGAGTGAGGTATTAGAA | 1245 | ACAATAAACATACTCTGCACACT GCATA | 1246 |
| HVP56 | 56_sp_3641_5597 | ACAACAACCACCCTGGTGATAAG | 1247 | ACAACCTTTGAAACAGGTGTTG GA | 1248 |
| HVP56 | 56_sp_233_2699 | GCACCACTTGAGTGAGGTATTAGAA | 1249 | TTTTCTTTGTCCTCGTCGTTATCC AA | 1250 |
| HVP56 | 56_sp_1295_2572 | CAAGACAGCGGGTATGGCAATA | 1251 | TGAAACTGAAACACTAACATTCT ACTGTGT | 1252 |
| HVP56 | 56_sp_910_3349 | GTTAACAGTAACGTGCCCACTCT | 1253 | GGTGGTGGTGGTGGTCTT | 1254 |
| HVP56 | 56_unsp_1295_1296 | CAAGACAGCGGGTATGGCAATA | 1255 | GGTACTGTTTTGTGAGCCTCCAT TT | 1256 |
| HVP56 | 56_fus_910_MYC_001_e xon2 | GTTAACAGTAACGTGCCCACTCT | 1257 | AAATACGGCTGCACCGAGT | 1258 |
| HVP56 | 56_fus_3629_MYC_001_ exon3 | ACAACAACCACCCTGGTGATAAG | 1259 | GGTGATCCAGACTCTGACCTTTT G | 1260 |
| HVP56 | 56_fus_3629_PVTI_004 _exon1 | ACAACAACCACCCTGGTGATAAG | 1261 | CATGGTTCCACCAGCGTTATT | 1262 |
| HVP56 | 56_fus_2861_MYC_001_ exon2 | GTGCCAGAACAAAATACTAGACTGTTT | 1263 | AAATACGGCTGCACCGAGT | 1264 |
| HVP56 | 56_unsp_5596_5597 | AGGGATCCTCCTTTGCATTATGG | 1265 | ACAACCTTTGAAACAGGTGTTG GA | 1266 |
| HVP56 | 56_sp_233_416 | GCACCACTTGAGTGAGGTATTAGAA | 1267 | CAATTGCTTTTCCTCCGGAGTTA A | 1268 |
| HVP56 | 56_sp_910_5759 | GTTAACAGTAACGTGCCCACTCT | 1269 | CAACCGTACCCTAAATACCCTAT ATTGA | 1270 |

(continued)

| Virus name | Transcript targeted | Forward primer (5'-3') | SEQ ID NO° | Reverse primer (5'-3') | SEQ ID NO° |
|---|---|---|---|---|---|
| HVP56 | 56_fus_910_PVT1_002_ exon3 | GTTAACAGTAACGTGCCCACTCT | 1271 | ATCATGATGGCTGTATGTGCCA | 1272 |
| HVP56 | 56_sp_910_2699 | GTTAACAGTAACGTGCCCACTCT | 1273 | TTTTCTTTGTCCTCGTCGTTATCC AA | 1274 |
| HVP56 | 56_unsp_415_416 | TCAGTGTATGGAGCTACACTAGAAAGT | 1275 | CAATTGCTTTTCCTCCGGAGTTA A | 1276 |
| HVP56 | 56_fus_910_PVT1_005_ exon1 | GTTAACAGTAACGTGCCCACTCT | 1277 | TCTTTGCTCGCAGCTCGT | 1278 |
| HVP56 | 56_sp_3641_5759 | ACAACAACCACCCTGGTGATAAG | 1279 | CAACCGTACCCTAAATACCCTAT ATTGA | 1280 |
| HVP56 | 56_fus_910_PVT1_004_ exon1 | GTTAACAGTAACGTGCCCACTCT | 1281 | CATGGTTCCACCAGCGTTATT | 1282 |
| HVP56 | 56_fus_3629_PVT1_005 _exon1 | ACAACAACCACCCTGGTGATAAG | 1283 | TCTTTGCTCGCAGCTCGT | 1284 |
| HVP56 | 56_sp_233_3349 | GCACCACTTGAGTGAGGTATTAGAA | 1285 | GGTGGTGGTGGTGGTCTT | 1286 |
| HVP56 | 56_sp_233_773 | GCACCACTTGAGTGAGGTATTAGAA | 1287 | TGTACAACACGCAGGTCCTC | 1288 |
| HVP56 | 56_gen_3891_4183 | TGCTACGCATATATATTGCAACCATTGA | 1289 | GGATGTGGCTATAACAAACCAA AACAAT | 1290 |
| HVP56 | 56_gen_7283_7844 | AATTCGGTTGCATGGCCTAGT | 1291 | GGGTGCGGTACTGTACATAATT CAAG | 1292 |
| HVP56 | 56_gen_6009_7276 | TGTACTCCCGCTATGGGTGAA | 1293 | GTGTCTATCATGTCCCCATCCTCT A | 1294 |
| HVP56 | 56_gen_1545_2322 | CAGATGATAGCCAAATTGCGTTTCA | 1295 | GCTGTTGTGCCCTTTTATAATGT CTAC | 1296 |
| HVP58 | 58_sp_898_5643 | TGCTTATGGGCACATGTACCATT | 1297 | GCTTACAACCTTAGACACAGGC A | 1298 |
| HVP58 | 58_fus_3596_PVT1_002 _exon3 | GAGGAGGACTACACAGTACAACTAACT | 1299 | ATCATGATGGCTGTATGTGCCA | 1300 |
| HVP58 | 58_fus_898_MYC_001_e xon3 | TGCTTATGGGCACATGTACCATT | 1301 | GGTGATCCAGACTCTGACCTTTT G | 1302 |
| HVP58 | 58_sp_1320_3355 | AAAATTATTGAGCTAGAAGACAGCGGA T | 1303 | CCCTGTGTACTTTCGTTGTTGGT | 1304 |

(continued)

| Virus name | Transcript targeted | Forward primer (5'-3') | SEQ ID NO° | Reverse primer (5'-3') | SEQ ID NO° |
|---|---|---|---|---|---|
| HVP58 | 58_unsp_1320_1321 | AAAATTATTGAGCTAGAAGACAGCGGAT | 1305 | CCCCACTAGACTCCGAGTCATTTAA | 1306 |
| HVP58 | 58_fus_3596_MYC_001_exon3 | GAGGAGGACTACACAGTACAACTAACT | 1307 | GGTGATCCAGACTCTGACCTTTTG | 1308 |
| HVP58 | 58_fus_2867_PVT1_005_exon1 | GCAGGACAAAATCCTAGACATATACGAA | 1309 | TCTTTGCTCGCAGCTCGT | 1310 |
| HVP58 | 58_fus_3596_PVT1_004_exon1 | GAGGAGGACTACACAGTACAACTAACT | 1311 | CATGGTTCCACCAGCGTTATT | 1312 |
| HVP58 | 58_unsp_898_899 | TGCTTATGGGCACATGTACCATT | 1313 | CTGTTCTTCGTTCTATTACCGCTTCTA | 1314 |
| HVP58 | 58_fus_898_PVT1_002_ exon3 | TGCTTATGGGCACATGTACCATT | 1315 | ATCATGATGGCTGTATGTGCCA | 1316 |
| HVP58 | 58_unsp_2578_2579 | ATTAGATGGTAACGACATTTCAATAGATGT | 1317 | TGCATCAAATGGAAATGGATTGTTAAATTCA | 1318 |
| HVP58 | 58_sp_232_2579 | GTCAGGCGTTGGAGACATCT | 1319 | TGCATCAAATGGAAATGGATTGTTAAATTCA | 1320 |
| HVP58 | 58_fus_2867_MYC_001_exon3 | GCAGGACAAAATCCTAGACATATACGAA | 1321 | GGTGATCCAGACTCTGACCTTTTG | 1322 |
| HVP58 | 58_fus_898_MYC_001_e xon2 | TGCTTATGGGCACATGTACCATT | 1323 | AAATACGGCTGCACCGAGT | 1324 |
| HVP58 | 58_sp_232_415 | GTCAGGCGTTGGAGACATCT | 1325 | CGACCCGAAATATTATGAAACCTTTTGT | 1326 |
| HVP58 | 58_fus_898_PVT1_005_ exon1 | TGCTTATGGGCACATGTACCATT | 1327 | TCTTTGCTCGCAGCTCGT | 1328 |
| HVP58 | 58_sp_232_2706 | GTCAGGCGTTGGAGACATCT | 1329 | TGATATTTCCTCCATCGTTTTCCTTGTC | 1330 |
| HVP58 | 58_unsp_414_415 | CGCTATATGGAGACACATTAGAACAAACA | 1331 | CGACCCGAAATATTATGAAACCTTTTGT | 1332 |
| HVP58 | 58_unsp_3354_3355 | ACAATTATGGGAGGTACATGTGGGTA | 1333 | CCCTGTGTACTTTCGTTGTTGGT | 1334 |
| HVP58 | 58_sp_1320_2579 | AAAATTATTGAGCTAGAAGACAGCGGAT | 1335 | TGCATCAAATGGAAATGGATTGTTAAATTCA | 1336 |
| HVP58 | 58_sp_1320_2706 | AAAATTATTGAGCTAGAAGACAGCGGAT | 1337 | TGATATTTCCTCCATCGTTTTCCTTGTC | 1338 |

(continued)

| Virus name | Transcript targeted | Forward primer (5'-3') | SEQ ID NO° | Reverse primer (5'-3') | SEQ ID NO° |
|---|---|---|---|---|---|
| HVP58 | 58_fus_2867_MYC_001_ exon2 | GCAGGACAAAATCCTAGACATATACGA A | 1339 | AAATACGGCTGCACCGAGT | 1340 |
| HVP58 | 58_sp_232_532 | GTCAGGCGTTGGAGACATCT | 1341 | GCGTTGGGTTGTTTCCTCTCA | 1342 |
| HVP58 | 58_fus_898_PVT1_004_ exon1 | TGCTTATGGGCACATGTACCATT | 1343 | CATGGTTCCACCAGCGTTATT | 1344 |
| HVP58 | 58_fus_898_MYC_001_e xon1 | TGCTTATGGGCACATGTACCATT | 1345 | CTGAGAAGCCCTGCCCTTC | 1346 |
| HVP58 | 58_sp_898_2706 | TGCTTATGGGCACATGTACCATT | 1347 | TGATATTTCCTCCATCGTTTTCCT TGTC | 1348 |
| HVP58 | 58_fus_3596_PVT1_005 _exon1 | GAGGAGGACTACACAGTACAACTAACT | 1349 | TCTTTGCTCGCAGCTCGT | 1350 |
| HVP58 | 58_fus_2867_PVT1_004 _exon1 | GCAGGACAAAATCCTAGACATATACGA A | 1351 | CATGGTTCCACCAGCGTTATT | 1352 |
| HVP58 | 58_sp_232_3355 | GTCAGGCGTTGGAGACATCT | 1353 | CCCTGTGTACTTTCGTTGTTGGT | 1354 |
| HVP58 | 58_fus_2867_PVT1_002 _exon3 | GCAGGACAAAATCCTAGACATATACGA A | 1355 | ATCATGATGGCTGTATGTGCCA | 1356 |
| HVP58 | 58_fus_3596_MYC_001_ exon2 | GAGGAGGACTACACAGTACAACTAACT | 1357 | AAATACGGCTGCACCGAGT | 1358 |
| HVP58 | 58_unsp_3608_3609 | GAGGAGGACTACACAGTACAACTAACT | 1359 | CCAATGCCATGTGGATGACATAT TACA | 1360 |
| HVP58 | 58_unsp_5642_5643 | CTGATTTTATGTTGCACCCTAGCTATTT | 1361 | GCTTACAACCTTAGACACAGGC A | 1362 |
| HVP58 | 58_fus_3596_MYC_001_ exon1 | GAGGAGGACTACACAGTACAACTAACT | 1363 | CTGAGAAGCCCTGCCCTTC | 1364 |
| HVP58 | 58_unsp_232_233 | GTCAGGCGTTGGAGACATCT | 1365 | TCGTAAGCACACTTTACATACTG CAAA | 1366 |
| HVP58 | 58_sp_898_2579 | TGCTTATGGGCACATGTACCATT | 1367 | TGCATCAAATGGAAATGGATTG TTAAATTCA | 1368 |
| HVP58 | 58_sp_898_3355 | TGCTTATGGGCACATGTACCATT | 1369 | CCCTGTGTACTTTCGTTGTTGGT | 1370 |
| HVP58 | 58_sp_3608_5643 | GAGGAGGACTACACAGTACAACTAACT | 1371 | GCTTACAACCTTAGACACAGGC A | 1372 |

(continued)

| Virus name | Transcript targeted | Forward primer (5'-3') | SEQ ID NO° | Reverse primer (5'-3') | SEQ ID NO° |
|---|---|---|---|---|---|
| HVP58 | 58_fus_2867_MYC_001_ exon1 | GCAGGACAAAATCCTAGACATATACGA A | 1373 | CTGAGAAGCCCTGCCCTTC | 1374 |
| HVP58 | 58_gen_5893_7295 | CGTTTGGTCTGGGCATGTGTA | 1375 | GCTGTGCGGGATATCTGTTACTG | 1376 |
| HVP58 | 58_gen_3858_4208 | TCTATATATGCTTGGTTGCTGGTGTTG | 1377 | CATGTGCAGAACCAGTATACAG TTAGT | 1378 |
| HVP58 | 58_gen_1570_2329 | CAATGGGACAATGGATACAAAGTAGGT | 1379 | GGGCCACACAGTAACATACAAC T | 1380 |
| HVP58 | 58_gen_7302_7824 | TCTATGAGTAAGGTGCTGTCCCTAAAT | 1381 | GGAGGTAAAGTAAAATGGAGG CAGTA | 1382 |
| HVP59 | 59_unsp_3627_3628 | TCCGTTTGCATCCAGGCAA | 1383 | CCAATGCCAGGTAGAGGAAATA TTTTCA | 1384 |
| HVP59 | 59_fus_2862_MYC_001_ exon1 | GCGTTTAAGTGTGTTACAGGATCAAAT | 1385 | CTGAGAAGCCCTGCCCTTC | 1386 |
| HVP59 | 59_sp_1306_3359 | AAAGAAGGTTAATAACAGTGCCAGACA | 1387 | CCCAAGTACGTGGCTTCGG | 1388 |
| HVP59 | 59_unsp_2570_2571 | AGATAGAAAGCATAGGCACCTAGTACA A | 1389 | TCTATTTTTGTCAAATGGCAATTT GTTTGGA | 1390 |
| HVP59 | 59_unsp_748_749 | CAGATGGAGTTAATCATCCTTTGCTACT | 1391 | TGTAAGGCTCGCAATCCGT | 1392 |
| HVP59 | 59_fus_887_PVT1_004_ exon1 | ACTATCCTTTGTGTGTCCTTTGTGT | 1393 | CATGGTTCCACCAGCGTTATT | 1394 |
| HVP59 | 59_fus_2862_PVT1_004 _exon1 | GCGTTTAAGTGTGTTACAGGATCAAAT | 1395 | CATGGTTCCACCAGCGTTATT | 1396 |
| HVP59 | 59_fus_887_PVT1_002_ exon3 | ACTATCCTTTGTGTGTCCTTTGTGT | 1397 | ATCATGATGGCTGTATGTGCCA | 1398 |
| HVP59 | 59_fus_3615_MYC_001_ exon1 | TCCGTTTGCATCCAGGCAA | 1399 | CTGAGAAGCCCTGCCCTTC | 1400 |
| HVP59 | 59_sp_1306_2698 | AAAGAAGGTTAATAACAGTGCCAGACA | 1401 | GGTGTCCATCACTGTCTGCAT | 1402 |
| HVP59 | 59_sp_3627_5606 | TCCGTTTGCATCCAGGCAA | 1403 | TGACATACTCATCAGTGCTGACA AC | 1404 |
| HVP59 | 59_fus_3615_MYC_001_ exon2 | TCCGTTTGCATCCAGGCAA | 1405 | AAATACGGCTGCACCGAGT | 1406 |
| HVP59 | 59_fus_2862_MYC_001_ exon3 | GCGTTTAAGTGTGTTACAGGATCAAAT | 1407 | GGTGATCCAGACTCTGACCTTTT G | 1408 |

(continued)

| Virus name | Transcript targeted | Forward primer (5'-3') | SEQ ID NO° | Reverse primer (5'-3') | SEQ ID NO° |
|---|---|---|---|---|---|
| HVP59 | 59_unsp_5768_5769 | GTATGTCACCCGTACCAGTATTTTCTAC | 1409 | GCCAAATTTATTGGGATCAGGT AACTT | 1410 |
| HVP59 | 59_sp_887_3359 | ACTATCCTTTGTGTGTCCTTTGTGT | 1411 | CCCAAGTACGTGGCTTCGG | 1412 |
| HVP59 | 59_sp_183_2571 | GCATCAATTGTGTGTTTTGCAAAGG | 1413 | TCTATTTTTGTCAAATGGCAATTT GTTTGGA | 1414 |
| HVP59 | 59_fus_3615_MYC_001_ exon3 | TCCGTTTGCATCCAGGCAA | 1415 | GGTGATCCAGACTCTGACCTTTT G | 1416 |
| HVP59 | 59_fus_887_MYC_001_e xon3 | ACTATCCTTTGTGTGTCCTTTGTGT | 1417 | GGTGATCCAGACTCTGACCTTTT G | 1418 |
| HVP59 | 59_fus_3615_PVT1_005 _exon1 | TCCGTTTGCATCCAGGCAA | 1419 | TCTTTGCTCGCAGCTCGT | 1420 |
| HVP59 | 59_fus_2862_MYC_001_ exon2 | GCGTTTAAGTGTGTTACAGGATCAAAT | 1421 | AAATACGGCTGCACCGAGT | 1422 |
| HVP59 | 59_sp_887_5606 | ACTATCCTTTGTGTGTCCTTTGTGT | 1423 | TGACATACTCATCAGTGCTGACA AC | 1424 |
| HVP59 | 59_fus_887_MYC_001_e xon2 | ACTATCCTTTGTGTGTCCTTTGTGT | 1425 | AAATACGGCTGCACCGAGT | 1426 |
| HVP59 | 59_sp_887_5769 | ACTATCCTTTGTGTGTCCTTTGTGT | 1427 | GCCAAATTTATTGGGATCAGGT AACTT | 1428 |
| HVP59 | 59_unsp_887_888 | ACTATCCTTTGTGTGTCCTTTGTGT | 1429 | CGTCATCTGAAATTTTGTCACCT GTTTT | 1430 |
| HVP59 | 59_fus_2862_PVT1_002 _exon3 | GCGTTTAAGTGTGTTACAGGATCAAAT | 1431 | ATCATGATGGCTGTATGTGCCA | 1432 |
| HVP59 | 59_sp_3627_5769 | TCCGTTTGCATCCAGGCAA | 1433 | GCCAAATTTATTGGGATCAGGT AACTT | 1434 |
| HVP59 | 59_sp_1306_2571 | AAAGAAGGTTAATAACAGTGCCAGACA | 1435 | TCTATTTTTGTCAAATGGCAATTT GTTTGGA | 1436 |
| HVP59 | 59_sp_887_2571 | ACTATCCTTTGTGTGTCCTTTGTGT | 1437 | TCTATTTTTGTCAAATGGCAATTT GTTTGGA | 1438 |
| HVP59 | 59_unsp_1306_1307 | AAAGAAGGTTAATAACAGTGCCAGACA | 1439 | GTCTATTTGACTGTCGCTACAAA CAC | 1440 |

(continued)

| Virus name | Transcript targeted | Forward primer (5'-3') | SEQ ID NO° | Reverse primer (5'-3') | SEQ ID NO° |
|---|---|---|---|---|---|
| HVP59 | 59_unsp_5605_5606 | CCTCGTAAACGTAAACGTGTTCC | 1441 | TGACATACTCATCAGTGCTGACA AC | 1442 |
| HVP59 | 59_fus_3615_PVT1_002 _exon3 | TCCGTTTGCATCCAGGCAA | 1443 | ATCATGATGGCTGTATGTGCCA | 1444 |
| HVP59 | 59_fus_3615_PVT1_004 _exon1 | TCCGTTTGCATCCAGGCAA | 1445 | CATGGTTCCACCAGCGTTATT | 1446 |
| HVP59 | 59_fus_2862_PVT1_005 _exon1 | GCGTTTAAGTGTGTTACAGGATCAAAT | 1447 | TCTTTGCTCGCAGCTCGT | 1448 |
| HVP59 | 59_sp_183_3359 | GCATCAATTGTGTGTTTTGCAAAGG | 1449 | CCCAAGTACGTGGCTTCGG | 1450 |
| HVP59 | 59_sp_887_2698 | ACTATCCTTTGTGTGTCCTTTGTGT | 1451 | GGTGTCCATCACTGTCTGCAT | 1452 |
| HVP59 | 59_sp_183_2698 | GCATCAATTGTGTGTTTTGCAAAGG | 1453 | GGTGTCCATCACTGTCTGCAT | 1454 |
| HVP59 | 59_fus_887_MYC_001_e xon1 | ACTATCCTTTGTGTGTCCTTTGTGT | 1455 | CTGAGAAGCCCTGCCCTTC | 1456 |
| HVP59 | 59_fus_887_PVT1_005_ exon1 | ACTATCCTTTGTGTGTCCTTTGTGT | 1457 | TCTTTGCTCGCAGCTCGT | 1458 |
| HVP59 | 59_sp_183_749 | GCATCAATTGTGTGTTTTGCAAAGG | 1459 | TGTAAGGCTCGCAATCCGT | 1460 |
| HVP59 | 59_unsp_183_184 | GCATCAATTGTGTGTTTTGCAAAGG | 1461 | GCATTTCAGACACGCTGCATAC | 1462 |
| HVP59 | 59_gen_3877_4222 | GTTGCAATGTCCCGCTTCTG | 1463 | CATGGGCATATAGTAGTAACAG TGGAA | 1464 |
| HVP59 | 59_gen_7261_7896 | GGTTGCACCCAATGAGTAAGGTA | 1465 | GCAAAACTGGACATTCAGGACA AAA | 1466 |
| HVP59 | 59_gen_6019_7254 | GCTGTGTACCTGCCATTGGA | 1467 | CTGTGTCTACCATATCACCATCTT CA | 1468 |
| HVP59 | 59_gen_1556_2321 | GTGCATGTTAATTGAACCACCCAAA | 1469 | TCAAACACGCTATCATCAACTCC AT | 1470 |
| HVP66 | 66_fus_2843_PVT1_002 _exon3 | CGTGCCAGAACAAAATACTAGACTGT | 1471 | ATCATGATGGCTGTATGTGCCA | 1472 |
| HVP66 | 66_sp_233_773 | CACCATCTGAGCGAGGTATTACA | 1473 | TGTACCACACGTAGCTCCTCT | 1474 |
| HVP66 | 66_fus_910_MYC_001_e xon1 | GTTAACAGTAACGTGCCCACTCT | 1475 | CTGAGAAGCCCTGCCCTTC | 1476 |
| HVP66 | 66_unsp_233_234 | CACCATCTGAGCGAGGTATTACA | 1477 | ACAATAAACATACCCTACATACT GCATATGG | 1478 |

(continued)

| Virus name | Transcript targeted | Forward primer (5'-3') | SEQ ID NO° | Reverse primer (5'-3') | SEQ ID NO° |
|---|---|---|---|---|---|
| HVP66 | 66_fus_910_MYC_001_e xon3 | GTTAACAGTAACGTGCCCACTCT | 1479 | GGTGATCCAGACTCTGACCTTTT G | 1480 |
| HVP66 | 66_fus_2843_MYC_001_ exon1 | CGTGCCAGAACAAAATACTAGACTGT | 1481 | CTGAGAAGCCCTGCCCTTC | 1482 |
| HVP66 | 66_sp_1290_2682 | GAAGACAGCGGGTATGGCAATA | 1483 | TTTTCTTTGTCCTCGTCGTTATCC AA | 1484 |
| HVP66 | 66_fus_2843_MYC_001_ exon3 | CGTGCCAGAACAAAATACTAGACTGT | 1485 | GGTGATCCAGACTCTGACCTTTT G | 1486 |
| HVP66 | 66_sp_1290_2555 | GAAGACAGCGGGTATGGCAATA | 1487 | CATTACTTAATTCATACACAGGA TTACCATT | 1488 |
| HVP66 | 66_fus_3605_PVT1_005 _exon1 | GTATCAACACACAAAGCCACTGT | 1489 | TCTTTGCTCGCAGCTCGT | 1490 |
| HVP66 | 66_sp_910_5647 | GTTAACAGTAACGTGCCCACTCT | 1491 | ACAACCTTTGAAACAGGTGTTG GA | 1492 |
| HVP66 | 66_fus_3605_MYC_001_ exon3 | GTATCAACACACAAAGCCACTGT | 1493 | GGTGATCCAGACTCTGACCTTTT G | 1494 |
| HVP66 | 66_sp_910_3362 | GTTAACAGTAACGTGCCCACTCT | 1495 | GGTGGTGGTGGTCCTGTG | 1496 |
| HVP66 | 66_fus_3605_PVT1_004 _exon1 | GTATCAACACACAAAGCCACTGT | 1497 | CATGGTTCCACCAGCGTTATT | 1498 |
| HVP66 | 66_fus_2843_MYC_001_ exon2 | CGTGCCAGAACAAAATACTAGACTGT | 1499 | AAATACGGCTGCACCGAGT | 1500 |
| HVP66 | 66_sp_233_2682 | CACCATCTGAGCGAGGTATTACA | 1501 | TTTTCTTTGTCCTCGTCGTTATCC AA | 1502 |
| HVP66 | 66_sp_233_533 | CACCATCTGAGCGAGGTATTACA | 1503 | AACCTCTTGCAACGTTGGTACT | 1504 |
| HVP66 | 66_unsp_772_773 | ACAGCAAGCTAGACAAGCTGAA | 1505 | TGTACCACACGTAGCTCCTCT | 1506 |
| HVP66 | 66_sp_233_416 | CACCATCTGAGCGAGGTATTACA | 1507 | GAAATCGTCTTTTATGTTCACAG TGCAA | 1508 |
| HVP66 | 66_sp_233_2555 | CACCATCTGAGCGAGGTATTACA | 1509 | CATTACTTAATTCATACACAGGA TTACCATT | 1510 |

(continued)

| Virus name | Transcript targeted | Forward primer (5'-3') | SEQ ID NO° | Reverse primer (5'-3') | SEQ ID NO° |
|---|---|---|---|---|---|
| HVP66 | 66_sp_3617_5647 | GTATCAACACACAAAGCCACTGT | 1511 | ACAACCTTTGAAACAGGTGTTG GA | 1512 |
| HVP66 | 66_fus_2843_PVT1_004 _exon1 | CGTGCCAGAACAAAATACTAGACTGT | 1513 | CATGGTTCCACCAGCGTTATT | 1514 |
| HVP66 | 66_fus_910_PVT1_002_ exon3 | GTTAACAGTAACGTGCCCACTCT | 1515 | ATCATGATGGCTGTATGTGCCA | 1516 |
| HVP66 | 66_unsp_910_911 | GTTAACAGTAACGTGCCCACTCT | 1517 | TTCTACAATTGCTTCTACCTGAA ACCAT | 1518 |
| HVP66 | 66_sp_233_3362 | CACCATCTGAGCGAGGTATTACA | 1519 | GGTGGTGGTGGTCCTGTG | 1520 |
| HVP66 | 66_sp_910_2555 | GTTAACAGTAACGTGCCCACTCT | 1521 | CATTACTTAATTCATACACAGGA TTACCATT | 1522 |
| HVP66 | 66_fus_3605_MYC_001_ exon2 | GTATCAACACACAAAGCCACTGT | 1523 | AAATACGGCTGCACCGAGT | 1524 |
| HVP66 | 66_fus_2843_PVT1_005 _exon1 | CGTGCCAGAACAAAATACTAGACTGT | 1525 | TCTTTGCTCGCAGCTCGT | 1526 |
| HVP66 | 66_fus_3605_PVT1_002 _exon3 | GTATCAACACACAAAGCCACTGT | 1527 | ATCATGATGGCTGTATGTGCCA | 1528 |
| HVP66 | 66_sp_1290_3362 | GAAGACAGCGGGTATGGCAATA | 1529 | GGTGGTGGTGGTCCTGTG | 1530 |
| HVP66 | 66_sp_910_2682 | GTTAACAGTAACGTGCCCACTCT | 1531 | TTTTCTTTGTCCTCGTCGTTATCC AA | 1532 |
| HVP66 | 66_fus_910_MYC_001_e xon2 | GTTAACAGTAACGTGCCCACTCT | 1533 | AAATACGGCTGCACCGAGT | 1534 |
| HVP66 | 66_fus_3605_MYC_001_ exon1 | GTATCAACACACAAAGCCACTGT | 1535 | CTGAGAAGCCCTGCCCTTC | 1536 |
| HVP66 | 66_unsp_1290_1291 | GAAGACAGCGGGTATGGCAATA | 1537 | GATACCGAGTGCTCACTACAATT ACTG | 1538 |
| HVP66 | 66_fus_910_PVT1_004_ exon1 | GTTAACAGTAACGTGCCCACTCT | 1539 | CATGGTTCCACCAGCGTTATT | 1540 |
| HVP66 | 66_fus_910_PVT1_005_ exon1 | GTTAACAGTAACGTGCCCACTCT | 1541 | TCTTTGCTCGCAGCTCGT | 1542 |
| HVP66 | 66_unsp_3617_3618 | GTATCAACACACAAAGCCACTGT | 1543 | TCTGTACTTGTCCAATGATATGT TGTTGT | 1544 |
| HVP66 | 66_unsp_5646_5647 | GCTACATTTGCACTATGGCCTGTA | 1545 | ACAACCTTTGAAACAGGTGTTG GA | 1546 |

(continued)

| Virus name | Transcript targeted | Forward primer (5'-3') | SEQ ID NO° | Reverse primer (5'-3') | SEQ ID NO° |
|---|---|---|---|---|---|
| HVP66 | 66_unsp_415_416 | GGGCAACATTAGAAAGTATAACTAAAA AACA | 1547 | GAAATCGTCTTTTATGTTCACAG TGCAA | 1548 |
| HVP66 | 66_gen_7301_7824 | GGTTAGGTGGTGTTCCTTACTGTTTA | 1549 | CAAAAGGCTAGGCAACCGAATT | 1550 |
| HVP66 | 66_gen_1540_2305 | AGACATAGATAGCAATGCACAAGCA | 1551 | ATCACCCCCTTCATCTACTTTACT ACA | 1552 |
| HVP66 | 66_gen_3867_4235 | GTTTGTCTGTGTGTGTGCCATT | 1553 | GCATGGCAATATATACACAGTG TAGGT | 1554 |
| HVP66 | 66_gen_5897_7294 | GTAGGCCGAGGTCAACCTTTA | 1555 | GTGCACATCCCACAATACATAAC TG | 1556 |
| HVP66 | 66_sp_1290_3331 | GACAGGGAGACAGCTCAACAATTATT | 1557 | CTCTCGGTACACAGTTTGCTGAT TA | 1558 |
| HVP66 | 66_unsp_3330_3331 | GTGGGTGGTGTAAAGTGTCATCA | 1559 | GGACAGTAAATACTCTCGGTTTC CAT | 1560 |
| HVP68 | 68_sp_129_3292 | GACATTGGACACTACATTGCATGAC | 1561 | TCGCGGTGGTGTTCTGTAG | 1562 |
| HVP68 | 68_sp_129_2510 | GACATTGGACACTACATTGCATGAC | 1563 | CTGTTTTGGTCAAATGGAAATGC ATTAG | 1564 |
| HVP68 | 68_fus_2801_MYC_001_ exon1 | ACAGGACAGTAAATGTATACAGGACCA T | 1565 | CTGAGAAGCCCTGCCCTTC | 1566 |
| HVP68 | 68_unsp_5487_5488 | TACAACCTTTGCCATAACTATATATGGT | 1567 | ATTGACAACCTTCGCCACTGA | 1568 |
| HVP68 | 68_fus_3551_PVT1_004 _exon1 | AGTAGAAGTGCAGGCCAAAACAA | 1569 | CATGGTTCCACCAGCGTTATT | 1570 |
| HVP68 | 68_fus_838_MYC_001_e xon1 | TCCGTGGTGTGCAACTGAA | 1571 | CTGAGAAGCCCTGCCCTTC | 1572 |
| HVP68 | 68_fus_3551_MYC_001_ exon3 | AGTAGAAGTGCAGGCCAAAACAA | 1573 | GGTGATCCAGACTCTGACCTTTT G | 1574 |
| HVP68 | 68_fus_2801_PVT1_002 _exon3 | ACAGGACAGTAAATGTATACAGGACCA T | 1575 | ATCATGATGGCTGTATGTGCCA | 1576 |
| HVP68 | 68_fus_2801_MYC_001_ exon2 | ACAGGACAGTAAATGTATACAGGACCA T | 1577 | AAATACGGCTGCACCGAGT | 1578 |
| HVP68 | 68_fus_838_PVT1_004_ exon1 | TCCGTGGTGTGCAACTGAA | 1579 | CATGGTTCCACCAGCGTTATT | 1580 |

(continued)

| Virus name | Transcript targeted | Forward primer (5'-3') | SEQ ID NO° | Reverse primer (5'-3') | SEQ ID NO° |
|---|---|---|---|---|---|
| HVP68 | 68_unsp_1233_1234 | AGACAACCGGCGTATACAGTG | 1581 | CACACTACTACAGTCCTCCCGTA T | 1582 |
| HVP68 | 68_fus_3551_MYC_001_ exon1 | AGTAGAAGTGCAGGCCAAAACAA | 1583 | CTGAGAAGCCCTGCCCTTC | 1584 |
| HVP68 | 68_fus_2801_PVT1_004 _exon1 | ACAGGACAGTAAATGTATACAGGACCA T | 1585 | CATGGTTCCACCAGCGTTATT | 1586 |
| HVP68 | 68_unsp_129_130 | GACATTGGACACTACATTGCATGAC | 1587 | GATTGGCATGCAGCAAATGGTA | 1588 |
| HVP68 | 68_fus_2801_MYC_001_ exon3 | ACAGGACAGTAAATGTATACAGGACCA T | 1589 | GGTGATCCAGACTCTGACCTTTT G | 1590 |
| HVP68 | 68_sp_1233_2510 | AGACAACCGGCGTATACAGTG | 1591 | CTGTTTTGGTCAAATGGAAATGC ATTAG | 1592 |
| HVP68 | 68_unsp_696_697 | CCACCAACATCTACTACTAGCCAGA | 1593 | CTGTTGTAGTGTCCGCAGGTT | 1594 |
| HVP68 | 68_unsp_2509_2510 | CCTAATACAAATAAAGTGTCCACCAAT GCT | 1595 | CTGTTTTGGTCAAATGGAAATGC ATTAG | 1596 |
| HVP68 | 68_sp_129_312 | GACATTGGACACTACATTGCATGAC | 1597 | CTTCGTTTTGTTGTTAGGTGCCT TAG | 1598 |
| HVP68 | 68_fus_3551_PVT1_002 _exon3 | AGTAGAAGTGCAGGCCAAAACAA | 1599 | ATCATGATGGCTGTATGTGCCA | 1600 |
| HVP68 | 68_fus_3551_PVT1_005 _exon1 | AGTAGAAGTGCAGGCCAAAACAA | 1601 | TCTTTGCTCGCAGCTCGT | 1602 |
| HVP68 | 68_sp_838_2510 | TCCGTGGTGTGCAACTGAA | 1603 | CTGTTTTGGTCAAATGGAAATGC ATTAG | 1604 |
| HVP68 | 68_unsp_838_839 | TCCGTGGTGTGCAACTGAA | 1605 | GACTGTGTCACCTGTTTGTTTAT CTACT | 1606 |
| HVP68 | 68_sp_838_5488 | TCCGTGGTGTGCAACTGAA | 1607 | ATTGACAACCTTCGCCACTGA | 1608 |
| HVP68 | 68_fus_3551_MYC_001_ exon2 | AGTAGAAGTGCAGGCCAAAACAA | 1609 | AAATACGGCTGCACCGAGT | 1610 |
| HVP68 | 68_fus_838_PVT1_002_ exon3 | TCCGTGGTGTGCAACTGAA | 1611 | ATCATGATGGCTGTATGTGCCA | 1612 |
| HVP68 | 68_fus_838_PVT1_005_ exon1 | TCCGTGGTGTGCAACTGAA | 1613 | TCTTTGCTCGCAGCTCGT | 1614 |

(continued)

| Virus name | Transcript targeted | Forward primer (5'-3') | SEQ ID NO° | Reverse primer (5'-3') | SEQ ID NO° |
|---|---|---|---|---|---|
| HVP68 | 68_sp_838_3292 | TCCGTGGTGTGCAACTGAA | 1615 | TCGCGGTGGTGTTCTGTAG | 1616 |
| HVP68 | 68_sp_3563_5488 | AGTAGAAGTGCAGGCCAAAACAA | 1617 | ATTGACAACCTTCGCCACTGA | 1618 |
| HVP68 | 68_fus_838_MYC_001_e xon2 | TCCGTGGTGTGCAACTGAA | 1619 | AAATACGGCTGCACCGAGT | 1620 |
| HVP68 | 68_sp_129_697 | GACATTGGACACTACATTGCATGAC | 1621 | CTGTTGTAGTGTCCGCAGGTT | 1622 |
| HVP68 | 68_unsp_3291_3292 | CTAGTGGAAAATGGGACGTGCATTATA | 1623 | TCGCGGTGGTGTTCTGTAG | 1624 |
| HVP68 | 68_unsp_3563_3564 | AGTAGAAGTGCAGGCCAAAACAA | 1625 | AAGCGTTATGTTTTTGCAACCTA TACC | 1626 |
| HVP68 | 68_sp_1233_3292 | AGACAACCGGCGTATACAGTG | 1627 | TCGCGGTGGTGTTCTGTAG | 1628 |
| HVP68 | 68_fus_838_MYC_001_e xon3 | TCCGTGGTGTGCAACTGAA | 1629 | GGTGATCCAGACTCTGACCTTTT G | 1630 |
| HVP68 | 68_fus_2801_PVT1_005 _exon1 | ACAGGACAGTAAATGTATACAGGACCA T | 1631 | TCTTTGCTCGCAGCTCGT | 1632 |
| HVP68 | 68_unsp_311_312 | GGAATCGGTGTATGCAACTACATTAGA A | 1633 | CTTCGTTTTGTTGTTAGGTGCCT TAG | 1634 |
| HVP68 | 68_gen_7154_7822 | CCCTGTGACTAACATATGTCCTTGT | 1635 | CCACACGGTATAGTTTGCAACCA T | 1636 |
| HVP68 | 68_gen_5738_7147 | GCCTGTGTTGGTGTTGAAATAGGTA | 1637 | TGCAACATTGTCCCTACTGTCTTT AG | 1638 |
| HVP68 | 68_gen_3813_4090 | GGTGTGGTTTTGTGTATGCATGT | 1639 | GGTATACAGCAAACACCTCAAA TGGT | 1640 |
| HVP68 | 68_gen_1483_2260 | CGACACGCCGGAATGGATAA | 1641 | CGCTGCAGCATTACTATTACAAT CTG | 1642 |
| HVP73 | 73_unsp_5493_5494 | TGGGTCAGGTTTTATATTACACCCTAGT | 1643 | GCTTACAACCTTAGACACAGACA CA | 1644 |
| HVP73 | 73_fus_2858_MYC_001_ exon1 | GTATGAACGTGACAGTGTACACCTAA | 1645 | CTGAGAAGCCCTGCCCTTC | 1646 |
| HVP73 | 73_sp_1287_3346 | AAACGAAGACTGTTTGAGGAGCA | 1647 | TGGTGTTGGTGGTTGTGGT | 1648 |
| HVP73 | 73_fus_3560_MYC_001_ exon2 | ACCTACATCCCACCACAGAGT | 1649 | AAATACGGCTGCACCGAGT | 1650 |

(continued)

| Virus name | Transcript targeted | Forward primer (5'-3') | SEQ ID NO° | Reverse primer (5'-3') | SEQ ID NO° |
|---|---|---|---|---|---|
| HVP73 | 73_sp_862_2570 | TGCTTATGGGTACACTAGGTATTGTGT | 1651 | GGGTTCCCATTACTGTCAAATGGA | 1652 |
| HVP73 | 73_fus_862_MYC_001_e xon3 | TGCTTATGGGTACACTAGGTATTGTGT | 1653 | GGTGATCCAGACTCTGACCTTTTG | 1654 |
| HVP73 | 73_sp_227_410 | AGCGTTATGTGACGAAGTGAATATTTCT | 1655 | CTGTTCTGCTATTTGATGAAACCGTTTT | 1656 |
| HVP73 | 73_sp_227_527 | AGCGTTATGTGACGAAGTGAATATTTCT | 1657 | TTCGGTTGTTGGTTTCAGGTCTAA | 1658 |
| HVP73 | 73_sp_862_3346 | TGCTTATGGGTACACTAGGTATTGTGT | 1659 | TGGTGTTGGTGGTTGTGGT | 1660 |
| HVP73 | 73_unsp_3345_3346 | GGGTAAAAGGCATATGGGAAGTACAT | 1661 | TGGTGTTGGTGGTTGTGGT | 1662 |
| HVP73 | 73_unsp_2569_2570 | CAAGTTAAATGCCCTCCATTACTGATAAC | 1663 | GGGTTCCCATTACTGTCAAATGGA | 1664 |
| HVP73 | 73_fus_862_PVT1_004_ exon1 | TGCTTATGGGTACACTAGGTATTGTGT | 1665 | CATGGTTCCACCAGCGTTATT | 1666 |
| HVP73 | 73_unsp_3572_3573 | ACCTACATCCCACCACAGAGT | 1667 | GTCCAATGCCATGTTGTTGTTACA | 1668 |
| HVP73 | 73_fus_3560_PVT1_002 _exon3 | ACCTACATCCCACCACAGAGT | 1669 | ATCATGATGGCTGTATGTGCCA | 1670 |
| HVP73 | 73_sp_862_5647 | TGCTTATGGGTACACTAGGTATTGTGT | 1671 | ACGAAGCCTAAACACCCTGTATTG | 1672 |
| HVP73 | 73_fus_2858_PVT1_002 _exon3 | GTATGAACGTGACAGTGTACACCTAA | 1673 | ATCATGATGGCTGTATGTGCCA | 1674 |
| HVP73 | 73_unsp_726_727 | ACTCAGAGGATGAGGATGAAACAGA | 1675 | CCTAGTGTACCCATAAGCAACTCTTCTA | 1676 |
| HVP73 | 73_sp_3572_5494 | ACCTACATCCCACCACAGAGT | 1677 | GCTTACAACCTTAGACACAGACACA | 1678 |
| HVP73 | 73_unsp_1287_1288 | AAACGAAGACTGTTTGAGGAGCA | 1679 | GACACAATTTGGTTGCCTTCTTCATTAA | 1680 |
| HVP73 | 73_fus_862_MYC_001_e xon2 | TGCTTATGGGTACACTAGGTATTGTGT | 1681 | AAATACGGCTGCACCGAGT | 1682 |
| HVP73 | 73_unsp_5646_5647 | TGCAGGTAGCACACGTTTGT | 1683 | ACGAAGCCTAAACACCCTGTATTG | 1684 |

(continued)

| Virus name | Transcript targeted | Forward primer (5'-3') | SEQ ID NO° | Reverse primer (5'-3') | SEQ ID NO° |
|---|---|---|---|---|---|
| HVP73 | 73_fus_3560_PVT1_005 _exon1 | ACCTACATCCCACCACAGAGT | 1685 | TCTTTGCTCGCAGCTCGT | 1686 |
| HVP73 | 73_unsp_862_863 | TGCTTATGGGTACACTAGGTATTGTGT | 1687 | TGGAATTGGATCCCCTGTTTTTC TTT | 1688 |
| HVP73 | 73_fus_862_PVT1_005_ exon1 | TGCTTATGGGTACACTAGGTATTGTGT | 1689 | TCTTTGCTCGCAGCTCGT | 1690 |
| HVP73 | 73_fus_862_MYC_001_e xon1 | TGCTTATGGGTACACTAGGTATTGTGT | 1691 | CTGAGAAGCCCTGCCCTTC | 1692 |
| HVP73 | 73_sp_862_5494 | TGCTTATGGGTACACTAGGTATTGTGT | 1693 | GCTTACAACCTTAGACACAGACA CA | 1694 |
| HVP73 | 73_fus_2858_PVT1_004 _exon1 | GTATGAACGTGACAGTGTACACCTAA | 1695 | CATGGTTCCACCAGCGTTATT | 1696 |
| HVP73 | 73_unsp_227_228 | AGCGTTATGTGACGAAGTGAATATTTC T | 1697 | AAAATTTTAAACACGGTTGACAT ACAC | 1698 |
| HVP73 | 73_fus_2858_MYC_001_ exon2 | GTATGAACGTGACAGTGTACACCTAA | 1699 | AAATACGGCTGCACCGAGT | 1700 |
| HVP73 | 73_fus_2858_PVT1_005 _exon1 | GTATGAACGTGACAGTGTACACCTAA | 1701 | TCTTTGCTCGCAGCTCGT | 1702 |
| HVP73 | 73_fus_862_PVT1_002_ exon3 | TGCTTATGGGTACACTAGGTATTGTGT | 1703 | ATCATGATGGCTGTATGTGCCA | 1704 |
| HVP73 | 73_unsp_409_410 | AGACAATCAGTATATGGCACTACGTTA GA | 1705 | CTGTTCTGCTATTTGATGAAACC GTTTT | 1706 |
| HVP73 | 73_fus_3560_PVT1_004 _exon1 | ACCTACATCCCACCACAGAGT | 1707 | CATGGTTCCACCAGCGTTATT | 1708 |
| HVP73 | 73_sp_227_2570 | AGCGTTATGTGACGAAGTGAATATTTC T | 1709 | GGGTTCCCATTACTGTCAAATGG A | 1710 |
| HVP73 | 73_fus_3560_MYC_001_ exon1 | ACCTACATCCCACCACAGAGT | 1711 | CTGAGAAGCCCTGCCCTTC | 1712 |
| HVP73 | 73_fus_3560_MYC_001_ exon3 | ACCTACATCCCACCACAGAGT | 1713 | GGTGATCCAGACTCTGACCTTTT G | 1714 |
| HVP73 | 73_sp_1287_2570 | AAACGAAGACTGTTTGAGGAGCA | 1715 | GGGTTCCCATTACTGTCAAATGG A | 1716 |

(continued)

| Virus name | Transcript targeted | Forward primer (5'-3') | SEQ ID NO° | Reverse primer (5'-3') | SEQ ID NO° |
|---|---|---|---|---|---|
| HVP73 | 73_sp_227_727 | AGCGTTATGTGACGAAGTGAATATTTCT | 1717 | CCTAGTGTACCCATAAGCAACTCTTCTA | 1718 |
| HVP73 | 73_sp_227_3346 | AGCGTTATGTGACGAAGTGAATATTTCT | 1719 | TGGTGTTGGTGGTTGTGGT | 1720 |
| HVP73 | 73_fus_2858_MYC_001_exon3 | GTATGAACGTGACAGTGTACACCTAA | 1721 | GGTGATCCAGACTCTGACCTTTTG | 1722 |
| HVP73 | 73_sp_3572_5647 | ACCTACATCCCACCACAGAGT | 1723 | ACGAAGCCTAAACACCCTGTATTG | 1724 |
| HVP73 | 73_gen_3822_4053 | TCGCTTGCAGTGTCTGTGTATATTT | 1725 | CATGGTAATGTACAAGTGCCATAGGA | 1726 |
| HVP73 | 73_gen_1537_2320 | GAACGCATGTTAATTGAACCTCCAA | 1727 | GCTGCACTAACGTTTGTCTTTTAATCC | 1728 |
| HVP73 | 73_gen_5897_7198 | TGTATTTTAGGTTGTAGGCCTCCCTTA | 1729 | CTCCAAAGCCAACATCTATCATATCAC | 1730 |
| HVP73 | 73_gen_7205_7700 | GTCGCCATTTTACATGCATTAAGGT | 1731 | AGGAAACAAACCCTGCCAAGTT | 1732 |
| HVP82 | 82_sp_3613_5571 | TGCGACCACCAAATACACTGT | 1733 | GTGTTGACAATGCGTGACACT | 1734 |
| HVP82 | 82_fus_3601_MYC_001_exon1 | TGCGACCACCAAATACACTGT | 1735 | CTGAGAAGCCCTGCCCTTC | 1736 |
| HVP82 | 82_fus_2860_MYC_001_exon3 | GTGCCAGGAGAAAATACTAGACTGTTAT | 1737 | GGTGATCCAGACTCTGACCTTTTG | 1738 |
| HVP82 | 82_fus_2860_PVT1_005_exon1 | GTGCCAGGAGAAAATACTAGACTGTTAT | 1739 | TCTTTGCTCGCAGCTCGT | 1740 |
| HVP82 | 82_fus_2860_MYC_001_exon1 | GTGCCAGGAGAAAATACTAGACTGTTAT | 1741 | CTGAGAAGCCCTGCCCTTC | 1742 |
| HVP82 | 82_unsp_1316_1317 | CCGGACAGTGGATATGGCAATA | 1743 | GGTCTATCTCTGTACTTCTGTCGCT | 1744 |
| HVP82 | 82_sp_222_753 | CCTGCAATACGTCTATGCACAAT | 1745 | CCAGTAACATTTGCTGAAATATGCGAA | 1746 |
| HVP82 | 82_unsp_752_753 | GGAGGATGAAGTAGATAATATGCGTGAC | 1747 | CCAGTAACATTTGCTGAAATATGCGAA | 1748 |
| HVP82 | 82_unsp_3344_3345 | GGGCACAACAATGGGAGGTA | 1749 | GGGTGTTCGATAGCTGTTCAA | 1750 |

(continued)

| Virus name | Transcript targeted | Forward primer (5'-3') | SEQ ID NO° | Reverse primer (5'-3') | SEQ ID NO° |
|---|---|---|---|---|---|
| HVP82 | 82_unsp_3613_3614 | TGCGACCACCAAATACACTGT | 1751 | CAATGCCAGGTAGATGACACTT CTTTAA | 1752 |
| HVP82 | 82_unsp_5570_5571 | GGGATTACTACTTTGTGGCCGTATA | 1753 | GTGTTGACAATGCGTGACACT | 1754 |
| HVP82 | 82_sp_222_407 | CCTGCAATACGTCTATGCACAAT | 1755 | TTTTTTGTCGTCCACCACCTTTTG | 1756 |
| HVP82 | 82_fus_3601_MYC_001_ exon3 | TGCGACCACCAAATACACTGT | 1757 | GGTGATCCAGACTCTGACCTTTT G | 1758 |
| HVP82 | 82_fus_888_MYC_001_e xon2 | CGTGGTGTGCGACCAACTAA | 1759 | AAATACGGCTGCACCGAGT | 1760 |
| HVP82 | 82_fus_888_MYC_001_e xon1 | CGTGGTGTGCGACCAACTAA | 1761 | CTGAGAAGCCCTGCCCTTC | 1762 |
| HVP82 | 82_sp_222_3345 | CCTGCAATACGTCTATGCACAAT | 1763 | GGGTGTTCGATAGCTGTTCAA | 1764 |
| HVP82 | 82_unsp_222_223 | CCTGCAATACGTCTATGCACAAT | 1765 | CATGCTGCATATGGCGTATTGTC | 1766 |
| HVP82 | 82_unsp_406_407 | GTAGGTCTGTGTATGGTGCTACATT | 1767 | TTTTTTGTCGTCCACCACCTTTTG | 1768 |
| HVP82 | 82_fus_3601_PVT1_002 _exon3 | TGCGACCACCAAATACACTGT | 1769 | ATCATGATGGCTGTATGTGCCA | 1770 |
| HVP82 | 82_fus_3601_PVT1_005 _exon1 | TGCGACCACCAAATACACTGT | 1771 | TCTTTGCTCGCAGCTCGT | 1772 |
| HVP82 | 82_sp_1316_3345 | CCGGACAGTGGATATGGCAATA | 1773 | GGGTGTTCGATAGCTGTTCAA | 1774 |
| HVP82 | 82_sp_888_5571 | CGTGGTGTGCGACCAACTAA | 1775 | GTGTTGACAATGCGTGACACT | 1776 |
| HVP82 | 82_fus_888_PVT1_004_ exon1 | CGTGGTGTGCGACCAACTAA | 1777 | CATGGTTCCACCAGCGTTATT | 1778 |
| HVP82 | 82_sp_888_2575 | CGTGGTGTGCGACCAACTAA | 1779 | CATCATTTAGTGCATATACAGGA TTCCC | 1780 |
| HVP82 | 82_sp_888_3345 | CGTGGTGTGCGACCAACTAA | 1781 | GGGTGTTCGATAGCTGTTCAA | 1782 |
| HVP82 | 82_fus_2860_PVT1_004 _exon1 | GTGCCAGGAGAAAATACTAGACTGTTA T | 1783 | CATGGTTCCACCAGCGTTATT | 1784 |
| HVP82 | 82_sp_1316_2575 | CCGGACAGTGGATATGGCAATA | 1785 | CATCATTTAGTGCATATACAGGA TTCCC | 1786 |
| HVP82 | 82_fus_3601_PVT1_004 _exon1 | TGCGACCACCAAATACACTGT | 1787 | CATGGTTCCACCAGCGTTATT | 1788 |
| HVP82 | 82_fus_888_PVT1_005_ exon1 | CGTGGTGTGCGACCAACTAA | 1789 | TCTTTGCTCGCAGCTCGT | 1790 |

(continued)

| Virus name | Transcript targeted | Forward primer (5'-3') | SEQ ID NO° | Reverse primer (5'-3') | SEQ ID NO° |
|---|---|---|---|---|---|
| HVP82 | 82_sp_222_2575 | CCTGCAATACGTCTATGCACAAT | 1791 | CATCATTTAGTGCATATACAGGA TTCCC | 1792 |
| HVP82 | 82_fus_888_MYC_001_e xon3 | CGTGGTGTGCGACCAACTAA | 1793 | GGTGATCCAGACTCTGACCTTTT G | 1794 |
| HVP82 | 82_unsp_888_889 | CGTGGTGTGCGACCAACTAA | 1795 | TTGTCAACTACTGCCTCCACATA AAA | 1796 |
| HVP82 | 82_sp_222_521 | CCTGCAATACGTCTATGCACAAT | 1797 | TCCAACACTATGTCCTTTAATTGT GGT | 1798 |
| HVP82 | 82_fus_3601_MYC_001_ exon2 | TGCGACCACCAAATACACTGT | 1799 | AAATACGGCTGCACCGAGT | 1800 |
| HVP82 | 82_fus_2860_MYC_001_ exon2 | GTGCCAGGAGAAAATACTAGACTGTTA T | 1801 | AAATACGGCTGCACCGAGT | 1802 |
| HVP82 | 82_fus_2860_PVT1_002 _exon3 | GTGCCAGGAGAAAATACTAGACTGTTA T | 1803 | ATCATGATGGCTGTATGTGCCA | 1804 |
| HVP82 | 82_fus_888_PVT1_002_ exon3 | CGTGGTGTGCGACCAACTAA | 1805 | ATCATGATGGCTGTATGTGCCA | 1806 |
| HVP82 | 82_unsp_2574_2575 | ACACAGAAGCCTGCTGCAAA | 1807 | CATCATTTAGTGCATATACAGGA TTCCC | 1808 |
| HVP82 | 82_gen_7220_7871 | CCTGTAGGTTAAGGGTGGTGTT | 1809 | AAATCGGTCGCCACAAAATGG | 1810 |
| HVP82 | 82_gen_1566_2325 | CGTAGTACAGCCGTTGCATTG | 1811 | CCCATTGTACCATTTGCGATAGT T | 1812 |
| HVP82 | 82_gen_5821_7213 | GGATGTGTTGGTGTTGAAGTAGGTA | 1813 | TCCTGTTGGTCGTTGCCATT | 1814 |
| HVP82 | 82_gen_3863_4134 | GCTGCTAAGTGTATATAGTTACTCGCA | 1815 | CTGCTGCAAACACATATTGGGAT T | 1816 |

**17.13 Summary of Example 17**

The method according to the present invention described in Example 17 comprises:

1. Extraction of the viral RNA (Example 17.5) from a biological sample (Example 17.4),
2. Reverse transcription of the RNAs into cDNA with random hexamers (Example 17.6),
3. Amplification of the cDNA by multiplex PCR (Example 7) to generate a DNA sequence database. The multiplex amplification is performed with HPV-specific primer pairs (Example 17.3)
The primers are designed specifically for each of the HPV genomes present in the database (Example 17.2). The primers are modified to make them compatible with the high-throughput sequencing technique that is used.
4. High throughput sequencing of the DNA library and generation of "sequencing reads" (Example 17.8),
5. Aligning reads (Example 8) with the sequences of the HPV genomes present in the database (Examples 17.3 and 17.8).
6. Computing a score R (Example 17.11) whose the different possible computing are ratios described in Table 13. In this case, the ratio is defined as the ratio between the number of reads generated by at least 2 of pairs of primers described herein.

## Claims

**1.** A method for determining a patient risk of developing oncogenic virus induced cancer, such as group alpha HPV, comprising:

a) enrichment of the viral RNAs in a sample using random or consensus pre-amplification and/or specific reverse transcriptase reaction, preferably consensus pre-amplification,
b) sequencing cDNA produced in step a), and generating reads of said cDNA,
c) determining the number of reads matching said viruses based on species discrimination and determining the most prevalent high risk species present in the sample relative to other species,
d) determining within said most prevalent high risk species the relative number of reads matching at least one oncogenic gene compared to at least one non oncogenic gene, preferably oncogenic genes compared to non oncogenic genes,
e) computing ratios within said high risk species of reads matching at least one oncogenic gene versus corresponding interspecies structural or regulatory gene, preferably oncogenic genes versus corresponding interspecies structural or regulatory genes,

f) determining risk of developing oncogenic virus induced cancer in patients in which said ratio tend towards infinity,

wherein the enrichment of the viral RNAs is performed by a reverse transcription of the viral RNAs, and an amplification of the produced cDNA by multiplex-PCR with a group alpha HPV-specific composition of primers comprising at least one of the following groups of pairs of primers:

- SD1-SA1 group consisting of the pairs of primers of SEQ ID NO: 397-398; 521-522; 609-610; 695-696; 819-820; 865-866; 947-948; 1067-1068; 1119-1120; 1267-1268; 1325-1326; 1507-1508; 1597-1598; 1655-1656; 1755-1756; and/or,
- SD1-SA2 group consisting of the pairs of primers of SEQ ID NO: 459-460; 633-634; 687-688; 1111-1112; 1235-1236; 1341-1342; 1503-1504; 1657-1658; 1797-1798; and/or,
- SD1-SA3 group consisting of the pairs of primers of SEQ ID NO: 381-382; 541-542; 599-600; 903-904; 941-942; 1047-1048; 1135-1136; 1287-1288; 1459-1460; 1473-1474; 1621-1622; 1717-1718; 1745-1746; and/or,
- SD1-SA4 group consisting of the pairs of primers of SEQ ID NO: 413-414; 551-552; 637-638; 713-714; 793-794; 857-858; 981-982; 1093-1094; 1179-1180; 1227-1228; 1319-1320; 1413-1414; 1509-1510; 1563-1564; 1709-1710; 1791-1792 ; and/or,
- SD1-SA5 group consisting of the pairs of primers of SEQ ID NO: 453-454; 549-550; 613-614; 747-748; 761-762; 949-950; 1163-1164; 1249-1250; 1329-1330; 1453-1454; 1501-1502; and/or,
- SD1-SA6 group consisting of the pairs of primers of SEQ ID NO: 431-432; 595-596; 719-720; 827-828; 1089-1090; 1137-1138; 1285-1286; 1353-1354; 1561-1562; 1719-1720; 1763-1764; and/or,
- SD1-SA7 group consisting of the pairs of primers of SEQ ID NO: 919-920; 1449-1450; and/or,
- SD1-SA8 group consisting of the pairs of primers of SEQ ID NO: 489-490; 963-964; 1519-1520 ; and/or,
- SD2-SA4 group consisting of the pairs of primers of SEQ ID NO: 387-388; 473-474; 615-616; 745-746; 815-816; 849-850; 933-934; 1091-1092; 1177-1178; 1209-1210; 1367-1368; 1437-1438; 1521-1522; 1603-1604; 1651-1652; 1779-1780 ; and/or,
- SD2-SA5 group consisting of the pairs of primers of SEQ ID NO: 455-456; 529-530; 629-630; 717-718; 777-778; 975-976; 1153-1154; 1273-1274; 1347-1348; 1451-1452; 1531-1532; and/or,
- SD2-SA6 group consisting of the pairs of primers of SEQ ID NO: 399-400; 645-646; 727-728; 811-812; 1079-1080; 1127-1128; 1253-1254; 1369-1370; 1615-1616; 1659-1660; 1781-1782; and/or,
- SD2-SA7 group consisting of the pairs of primers of SEQ ID NO: 531-532; 899-900; 943-944; 1411-1412; 1495-1496; and/or,
- SD2-SA9 group consisting of the pairs of primers of SEQ ID NO: 437-438; 505-506; 607-608; 739-740; 785-786; 887-888; 979-980; 1063-1064; 1185-1186; 1233-1234; 1297-1298; 1423-1424; 1491-1492; 1607-1608; 1693-1694; 1775-1776 ; and/or,
- SD2-SA10 group consisting of the pairs of primers of SEQ ID NO: 545-546; 831-832; 1149-1150; 1269-1270; 1427-1428; 1671-1672 ; and/or,
- SD3-SA4 group consisting of the pairs of primers of SEQ ID NO: 379-380; 483-484; 611-612; 721-722; 833-834; 911-912; 937-938; 1053-1054; 1139-1140; 1251-1252; 1335-1336; 1435-1436; 1487-1488; 1591-1592; 1715-1716; 1785-1786 ; and/or,
- SD3-SA5 group consisting of the pairs of primers of SEQ ID NO: 415-416; 493-494; 593-594; 733-734; 817-818; 993-994; 1145-1146; 1243-1244; 1337-1338; 1401-1402; 1483-1484 ; and/or,
- SD3-SA6 group consisting of the pairs of primers of SEQ ID NO: 435-436; 655-656; 673-674; 813-814; 1045-1046; 1173-1174; 1241-1242; 1303-1304; 1557-1558; 1627-1628; 1647-1648; 1773-1774; and/or,
- SD3-SA7 group consisting of the pairs of primers of SEQ ID NO: 855-856; 1387-1388 ; and/or,
- SD3-SA8 group consisting of the pairs of primers of SEQ ID NO: 511-512; 957-958; 1529-1530; and/or,
- SD5-SA9 group consisting of the pairs of primers of SEQ ID NO: 419-420; 527-528; 567-568; 587-588; 683-684; 775-776; 891-892; 999-1000; 1041-1042; 1113-1114; 1247-1248; 1371-1372; 1403-1404; 1511-1512; 1617-1618; 1677-1678; 1733-1734; and/or,
- SD5-SA10 group consisting of the pairs of primers of SEQ ID NO: 495-496; 837-838; 1183-1184; 1279-1280; 1433-1434; 1723-1724.

**2.** The method according to claim 1 wherein the group alpha HPV-specific composition of primers comprises the following groups of pairs of primers:
SD1-SA1 group consisting of the pairs of primers of SEQ ID NO: 397-398; 521-522; 609-610; 695-696; 819-820; 865-866; 947-948; 1067-1068; 1119-1120; 1267-1268; 1325-1326; 1507-1508; 1597-1598; 1655-1656; 1755-1756.

**3.** The method according to any of claims 1 to 2, wherein the a group alpha HPV-specific composition of primers encompasses splice junctions, genomic and unspliced regions, and human fusion transcript regions of each HPV transcript.

**4.** The method according to any of claims 1 to 3, wherein the group alpha HPV-specific composition of primers comprises at least one of, preferably all, the following groups of pairs of primers:

- the HPV16-specific primers comprising or consisting of the primers of SEQ ID NOs: 219-258 for HPV16 genomic and unspliced transcripts, SEQ ID NOs: 259-352 for HPV16 spliced transcripts and SEQ ID NOs: 353-376 for HPV16-human fusion transcripts (including the pairs of primers of SEQ ID NO: 219-220; 221-222; 223-224; 225-226; 227-228; 229-230; 231-232; 233-234; 235-236; 237-238; 239-240; 241-242; 243-244; 245-246; 247-248; 249-250; 251-252; 253-254; 255-256; 257-258; 259-260; 261-262; 263-264; 265-266; 267-268; 269-270; 271-272; 273-274; 275-276; 277-278; 279-280; 281-282; 283-284; 285-286; 287-288; 289-290; 291-292; 293-294; 295-296; 297-298; 299-300; 301-302; 303-304; 305-306; 307-308; 309-310; 311-312; 313-314; 315-316; 317-318; 319-320; 321-322; 323-324; 325-326; 327-328; 329-330; 331-332; 333-334; 335-336; 337-338; 339-340; 341-342; 343-344; 345-346; 347-348; 349-350; 351-352; 353-354; 355-356; 357-358; 359-360; 361-362; 363-364; 365-366; 367-368; 369-370; 371-372; 373-374; 375-376) or 377-470 (including the pairs of primers of SEQ ID NO. 377-378; 379-380; 381-382; 383-384; 385- 386; 387-388; 389-390; 391-392; 393-394; 395-396; 397-398; 399-400; 401-402; 403-404; 405-406; 407-408; 409-410; 411-412; 413-414; 415-416; 417-418; 419-420; 421-422; 423-424; 425-426; 427-428; 429-430; 431-432; 433-434; 435-436; 437-438; 439-440; 441-442; 443-444; 445-446; 447-448; 449-450; 451-452; 453-454; 455-456; 457-458; 459-460; 461-462; 463-464; 465-466; 467-468 and; 469-470); and/or,
- the HPV18-specific primers comprising or consisting of the primers of SEQ ID NO. 471-574 (including the pairs of primers of SEQ ID NO.: 471-472; 473-474; 475-476; 477-478; 479-480; 481-482; 483-484; 485-486; 487-488; 489-490; 491-492; 493-494; 495-496; 497-498; 499-500; 501-502; 503-504; 505-506; 507-508; 509-510; 511-512; 513-514; 515-516; 517-518; 519-520; 521-522; 523-524; 525-526; 527-528; 529-530; 531-532; 533-534; 535-536; 537-538; 539-540; 541-542; 543-544; 545-546; 547-548; 549-550; 551-552; 553-554; 555-556; 557-558; 559-560; 561-562; 563-564; 565-566; 567-568; 569-570; 571-572; 573-574); and/or,
- the HPV31-specific primers comprising or consisting of the primers of SEQ ID NO. 575-668 (including the pairs of primers of SEQ ID NO.: 575-576; 577-578; 579-580; 581-582; 583-584; 585-586; 587-588; 589-590; 591-592; 593-594; 595-596; 597-598; 599-600; 601-602; 603-604; 605-606; 607-608; 609-610; 611-612; 613-614; 615-616; 617-618; 619-620; 621-622; 623-624; 625-626; 627-628; 629-630; 631-632; 633-634; 635-636; 637-638; 639-640; 641-642; 643-644; 645-646; 647-648; 649-650; 651-652; 653-654; 655-656; 657-658; 659-660; 661-662; 663-664; 665-666; 667-668); and/or,
- the HPV33-specific primers comprising or consisting of SEQ ID NO. 669-756 (including the pairs of primers of SEQ ID NO.: 669-670; 671-672; 673-674; 675-676; 677-678; 679-680; 681-682; 683-684; 685-686; 687-688; 689-690; 691-692; 693-694; 695-696; 697-698; 699-700; 701-702; 703-704; 705-706; 707-708; 709-710; 711-712; 713-714; 715-716; 717-718; 719-720; 721-722; 723-724; 725-726; 727-728; 729-730; 731-732; 733-734; 735-736; 737-738; 739-740; 741-742; 743-744; 745-746; 747-748; 749-750; 751-752; 753-754; 755-756); and/or,
- the HPV35-specific primers comprising or consisting of the primers of SEQ ID NO. 757-848 (including the pairs of primers of SEQ ID NO.: 757-758; 759-760; 761-762; 763-764; 765-766; 767-768; 769-770; 771-772; 773-774; 775-776; 777-778; 779-780; 781-782; 783-784; 785-786; 787-788; 789-790; 791-792; 793-794; 795-796; 797-798; 799-800; 801-802; 803-804; 805-806; 807-808; 809-810; 811-812; 813-814; 815-816; 817-818; 819-820; 821-822; 823-824; 825-826; 827-828; 829-830; 831-832; 833-834; 835-836; 837-838; 839-840; 841-842; 843-844; 845-846; 847-848); and/or,
- the HPV39-specific primers comprising or consisting of the primers of SEQ ID NO. 849-928 (including the pairs of primers of SEQ ID NO.: 849-850; 851-852; 853-854; 855-856; 857-858; 859-860; 861-862; 863-864; 865-866; 867-868; 869-870; 871-872; 873-874; 875-876; 877-878; 879-880; 881-882; 883-884; 885-886; 887-888; 889-890; 891-892; 893-894; 895-896; 897-898; 899-900; 901-902; 903-904; 905-906; 907-908; 909-910; 911-912; 913-914; 915-916; 917-918; 919-920; 921-922; 923-924; 925-926; 927-928); and/or,
- the HPV45-specific primers comprising or consisting of the primers of SEQ ID NO. 929-1020 (including the pairs of primers of SEQ ID NO.: 929-930; 931-932; 933-934; 935-936; 937-938; 939-940; 941-942; 943-944; 945-946; 947-948; 949-950; 951-952; 953-954; 955-956; 957-958; 959-960; 961-962; 963-964; 965-966; 967-968; 969-970; 971-972; 973-974; 975-976; 977-978; 979-980; 981-982; 983-984; 985-986; 987-988; 989-990; 991-992; 993-994; 995-996; 997-998; 999-1000; 1001-1002; 1003-1004; 1005-1006; 1007-1008; 1009-1010; 1011-1012; 1013-1014; 1015-1016; 1017-1018; 1019-1020); and/or,
- the HPV51-specific primers comprising or consisting of the primers of SEQ ID NO. 1021-1102 (including the pairs of primers of SEQ ID NO.: 1021-1022; 1023-1024; 1025-1026; 1027-1028; 1029-1030; 1031-1032; 1033-1034; 1035-1036; 1037-1038; 1039-1040; 1041-1042; 1043-1044; 1045-1046; 1047-1048; 1049-1050; 1051-1052; 1053-1054; 1055-1056; 1057-1058; 1059-1060; 1061-1062; 1063-1064; 1065-1066; 1067-1068; 1069-1070; 1071-1072; 1073-1074; 1075-1076; 1077-1078; 1079-1080; 1081-1082; 1083-1084; 1085-1086; 1087-1088; 1089-1090; 1091-1092; 1093-1094; 1095-1096; 1097-1098; 1099-1100; 1101-1102); and/or,

- the HPV52-specific primers comprising or consisting of the primers of SEQ ID NO. 1103-1200 (including the pairs of primers of SEQ ID NO.: 1103-1104; 1105-1106; 1107-1108; 1109-1110; 1111-1112; 1113-1114; 1115-1116; 1117-1118; 1119-1120; 1121-1122; 1123-1124; 1125-1126; 1127-1128; 1129-1130; 1131-1132; 1133-1134; 1135-1136; 1137-1138; 1139-1140; 1141-1142; 1143-1144; 1145-1146; 1147-1148; 1149-1150; 1151-1152; 1153-1154; 1155-1156; 1157-1158; 1159-1160; 1161-1162; 1163-1164; 1165-1166; 1167-1168; 1169-1170; 1171-1172; 1173-1174; 1175-1176; 1177-1178; 1179-1180; 1181-1182; 1183-1184; 1185-1186; 1187-1188; 1189-1190; 1191-1192; 1193-1194; 1195-1196; 1197-1198; 1199-1200); and/or,

- the HPV56-specific primers comprising or consisting of the primers of SEQ ID NO. 1201-1296 (including the pairs of primers of SEQ ID NO.: 1201-1202; 1203-1204; 1205-1206; 1207-1208; 1209-1210; 1211-1212; 1213-1214; 1215-1216; 1217-1218; 1219-1220; 1221-1222; 1223-1224; 1225-1226; 1227-1228; 1229-1230; 1231-1232; 1233-1234; 1235-1236; 1237-1238; 1239-1240; 1241-1242; 1243-1244; 1245-1246; 1247-1248; 1249-1250; 1251-1252; 1253-1254; 1255-1256; 1257-1258; 1259-1260; 1261-1262; 1263-1264; 1265-1266; 1267-1268; 1269-1270; 1271-1272; 1273-1274; 1275-1276; 1277-1278; 1279-1280; 1281-1282; 1283-1284; 1285-1286; 1287-1288; 1289-1290; 1291-1292; 1293-1294; 1295-1296); and/or,

- the HPV58-specific primers comprising or consisting of the primers of SEQ ID NO. 1297-1382 (including the pairs of primers of SEQ ID NO.: 1297-1298; 1299-1300; 1301-1302; 1303-1304; 1305-1306; 1307-1308; 1309-1310; 1311-1312; 1313-1314; 1315-1316; 1317-1318; 1319-1320; 1321-1322; 1323-1324; 1325-1326; 1327-1328; 1329-1330; 1331-1332; 1333-1334; 1335-1336; 1337-1338; 1339-1340; 1341-1342; 1343-1344; 1345-1346; 1347-1348; 1349-1350; 1351-1352; 1353-1354; 1355-1356; 1357-1358; 1359-1360; 1361-1362; 1363-1364; 1365-1366; 1367-1368; 1369-1370; 1371-1372; 1373-1374; 1375-1376; 1377-1378; 1379-1380; 1381-1382); and/or,

- the HPV59-specific primers comprising or consisting of the primers of SEQ ID NO. 1383-1470 (including the pairs of primers of SEQ ID NO.: 1383-1384; 1385-1386; 1387-1388; 1389-1390; 1391-1392; 1393-1394; 1395-1396; 1397-1398; 1399-1400; 1401-1402; 1403-1404; 1405-1406; 1407-1408; 1409-1410; 1411-1412; 1413-1414; 1415-1416; 1417-1418; 1419-1420; 1421-1422; 1423-1424; 1425-1426; 1427-1428; 1429-1430; 1431-1432; 1433-1434; 1435-1436; 1437-1438; 1439-1440; 1441-1442; 1443-1444; 1445-1446; 1447-1448; 1449-1450; 1451-1452; 1453-1454; 1455-1456; 1457-1458; 1459-1460; 1461-1462; 1463-1464; 1465-1466; 1467-1468; 1469-1470); and/or,

- the HPV66-specific primers comprising or consisting of the primers of SEQ ID NO. 1471-1560 (including the pairs of primers of SEQ ID NO.: 1471-1472; 1473-1474; 1475-1476; 1477-1478; 1479-1480; 1481-1482; 1483-1484; 1485-1486; 1487-1488; 1489-1490; 1491-1492; 1493-1494; 1495-1496; 1497-1498; 1499-1500; 1501-1502; 1503-1504; 1505-1506; 1507-1508; 1509-1510; 1511-1512; 1513-1514; 1515-1516; 1517-1518; 1519-1520; 1521-1522; 1523-1524; 1525-1526; 1527-1528; 1529-1530; 1531-1532; 1533-1534; 1535-1536; 1537-1538; 1539-1540; 1541-1542; 1543-1544; 1545-1546; 1547-1548; 1549-1550; 1551-1552; 1553-1554; 1555-1556; 1557-1558; 1559-1560; and/or,

- the HPV68-specific primers comprising or consisting of the primers of SEQ ID NO. 1561-1642 (including the pairs of primers of SEQ ID NO.: 1561-1562; 1563-1564; 1565-1566; 1567-1568; 1569-1570; 1571-1572; 1573-1574; 1575-1576; 1577-1578; 1579-1580; 1581-1582; 1583-1584; 1585-1586; 1587-1588; 1589-1590; 1591-1592; 1593-1594; 1595-1596; 1597-1598; 1599-1600; 1601-1602; 1603-1604; 1605-1606; 1607-1608; 1609-1610; 1611-1612; 1613-1614; 1615-1616; 1617-1618; 1619-1620; 1621-1622; 1623-1624; 1625-1626; 1627-1628; 1629-1630; 1631-1632; 1633-1634; 1635-1636; 1637-1638; 1639-1640; 1641-1642); and/or,

- the HPV73-specific primers comprising or consisting of the primers of SEQ ID NO. 1643-1732 (including the pairs of primers of SEQ ID NO.: 1643-1644; 1645-1646; 1647-1648; 1649-1650; 1651-1652; 1653-1654; 1655-1656; 1657-1658; 1659-1660; 1661-1662; 1663-1664; 1665-1666; 1667-1668; 1669-1670; 1671-1672; 1673-1674; 1675-1676; 1677-1678; 1679-1680; 1681-1682; 1683-1684; 1685-1686; 1687-1688; 1689-1690; 1691-1692; 1693-1694; 1695-1696; 1697-1698; 1699-1700; 1701-1702; 1703-1704; 1705-1706; 1707-1708; 1709-1710; 1711-1712; 1713-1714; 1715-1716; 1717-1718; 1719-1720; 1721-1722; 1723-1724; 1725-1726; 1727-1728; 1729-1730; 1731-1732); and/or,

- the HPV82-specific primers comprising or consisting of the primers of SEQ ID NO. 1733-1816 (including the pairs of primers of SEQ ID NO.: 1733-1734; 1735-1736; 1737-1738; 1739-1740; 1741-1742; 1743-1744; 1745-1746; 1747-1748; 1749-1750; 1751-1752; 1753-1754; 1755-1756; 1757-1758; 1759-1760; 1761-1762; 1763-1764; 1765-1766; 1767-1768; 1769-1770; 1771-1772; 1773-1774; 1775-1776; 1777-1778; 1779-1780; 1781-1782; 1783-1784; 1785-1786; 1787-1788; 1789-1790; 1791-1792; 1793-1794; 1795-1796; 1797-1798;

1799-1800; 1801-1802; 1803-1804; 1805-1806; 1807-1808; 1809-1810; 1811-1812; 1813-1814; 1815-1816).

**5.** The method according to claim 1 or 2, wherein the group alpha HPV-specific composition of primers comprises one of the following groups of pairs of primers:

- the group of pairs of primers of SEQ ID NO: 397-398; 521-522; 609-610; 695-696; 819-820; 865-866; 947-948; 1067-1068; 1119-1120; 1267-1268; 1325-1326; 1507-1508; 1597-1598; 1655-1656; 1755-1756; 459-460; 633-634; 687-688; 1111-1112; 1235-1236; 1341-1342; 1503-1504; 1657-1658; 1797-1798; 381-382; 541-542; 599-600; 903-904; 941-942; 1047-1048; 1135-1136; 1287-1288; 1459-1460; 1473-1474; 1621-1622; 1717-1718; 1745-1746; 413-414; 551-552; 637-638; 713-714; 793-794; 857-858; 981-982; 1093-1094; 1179-1180; 1227-1228; 1319-1320; 1413-1414; 1509-1510; 1563-1564; 1709-1710; 1791-1792; 453-454; 549-550; 613-614; 747-748; 761-762; 949-950; 1163-1164; 1249-1250; 1329-1330; 1453-1454; 1501-1502; 431-432; 595-596; 719-720; 827-828; 1089-1090; 1137-1138; 1285-1286; 1353-1354; 1561-1562; 1719-1720; 1763-1764; 919-920; 1449-1450; 489-490; 963-964; 1519-1520; 387-388; 473-474; 615-616; 745-746; 815-816; 849-850; 933-934; 1091-1092; 1177-1178; 1209-1210; 1367-1368; 1437-1438; 1521-1522; 1603-1604; 1651-1652; 1779-1780; 455-456; 529-530; 629-630; 717-718; 777-778; 975-976; 1153-1154; 1273-1274; 1347-1348; 1451-1452; 1531-1532; 399-400; 645-646; 727-728; 811-812; 1079-1080; 1127-1128; 1253-1254; 1369-1370; 1615-1616; 1659-1660; 1781-1782; 531-532; 899-900; 943-944; 1411-1412; 1495-1496; 437-438; 505-506; 607-608; 739-740; 785-786; 887-888; 979-980; 1063-1064; 1185-1186; 1233-1234; 1297-1298; 1423-1424; 1491-1492; 1607-1608; 1693-1694; 1775-1776; 545-546; 831-832; 1149-1150; 1269-1270; 1427-1428; 1671-1672; 379-380; 483-484; 611-612; 721-722; 833-834; 911-912; 937-938; 1053-1054; 1139-1140; 1251-1252; 1335-1336; 1435-1436; 1487-1488; 1591-1592; 1715-1716; 1785-1786; 415-416; 493-494; 593-594; 733-734; 817-818; 993-994; 1145-1146; 1243-1244; 1337-1338; 1401-1402; 1483-1484; 435-436; 655-656; 673-674; 813-814; 1045-1046; 1173-1174; 1241-1242; 1303-1304; 1557-1558; 1627-1628; 1647-1648; 1773-1774; 855-856; 1387-1388; 511-512; 957-958; 1529-1530; 477-478; 419-420; 527-528; 567-568; 587-588; 683-684; 775-776; 891-892; 999-1000; 1041-1042; 1113-1114; 1247-1248; 1371-1372; 1403-1404; 1511-1512; 1617-1618; 1677-1678; 1733-1734; 495-496; 837-838; 1183-1184; 1279-1280; 1433-1434; 1723-1724; 1011-1012; 557-558; or,
- the group of pairs of primers of SEQ ID NO: 229-230; 233-234; 235-236; 245-246; 247-248; 249-250; 251-252; 255-256; 257-258; 265-266; 273-274; 275-276; 277-278; 279-280; 281-282; 289-290; 291-292; 295-296; 297-298; 299-300; 301-302; 303-304; 305-306; 307-308; 309-310; 311-312; 319-320; 321-322; 323-324; 325-326; 327-328; 329-330; 331-332; 333-334; 335-336; 337-338; 341-342; 343-344; 345-346; 347-348; 349-350; 351-352; 377-378; 379-380; 381-382; 383-384; 385-386; 387-388; 389-390; 391-392; 393-394; 395-396; 397-398; 399-400; 401-402; 403-404; 405-406; 407-408; 409-410; 411-412; 413-414; 415-416; 417-418; 419-420; 421-422; 423-424; 425-426; 427-428; 429-430; 431-432; 433-434; 435-436; 437-438; 439-440; 441-442; 443-444; 445-446; 447-448; 449-450; 451-452; 453-454; 455-456; 457-458; 459-460; 461-462; 463-464; 465-466; 467-468; 469-470; 471-472; 473-474; 475-476; 477-478; 479-480; 481-482; 483-484; 485-486; 487-488; 489-490; 491-492; 493-494; 495-496; 497-498; 499-500; 501-502; 503-504; 505-506; 507-508; 509-510; 511-512; 513-514; 515-516; 517-518; 519-520; 521-522; 523-524; 525-526; 527-528; 529-530; 531-532; 533-534; 535-536; 537-538; 539-540; 541-542; 543-544; 545-546; 547-548; 549-550; 551-552; 553-554; 555-556; 557-558; 559-560; 561-562; 563-564; 565-566; 567-568; 569-570; 571-572; 573-574; 575-576; 577-578; 579-580; 581-582; 583-584; 585-586; 587-588; 589-590; 591-592; 593-594; 595-596; 597-598; 599-600; 601-602; 603-604; 605-606; 607-608; 609-610; 611-612; 613-614; 615-616; 617-618; 619-620; 621-622; 623-624; 625-626; 627-628; 629-630; 631-632; 633-634; 635-636; 637-638; 639-640; 641-642; 643-644; 645-646; 647-648; 649-650; 651-652; 653-654; 655-656; 657-658; 659-660; 661-662; 663-664; 665-666; 667-668; 669-670; 671-672; 673-674; 675-676; 677-678; 679-680; 681-682; 683-684; 685-686; 687-688; 689-690; 691-692; 693-694; 695-696; 697-698; 699-700; 701-702; 703-704; 705-706; 707-708; 709-710; 711-712; 713-714; 715-716; 717-718; 719-720; 721-722; 723-724; 725-726; 727-728; 729-730; 731-732; 733-734; 735-736; 737-738; 739-740; 741-742; 743-744; 745-746; 747-748; 749-750; 751-752; 753-754; 755-756; 757-758; 759-760; 761-762; 763-764; 765-766; 767-768; 769-770; 771-772; 773-774; 775-776; 777-778; 779-780; 781-782; 783-784; 785-786; 787-788; 789-790; 791-792; 793-794; 795-796; 797-798; 799-800; 801-802; 803-804; 805-806; 807-808; 809-810; 811-812; 813-814; 815-816; 817-818; 819-820; 821-822; 823-824; 825-826; 827-828; 829-830; 831-832; 833-834; 835-836; 837-838; 839-840; 841-842; 843-844; 845-846; 847-848; 849-850; 851-852; 853-854; 855-856; 857-858; 859-860; 861-862; 863-864; 865-866; 867-868; 869-870; 871-872; 873-874; 875-876; 877-878; 879-880; 881-882; 883-884; 885-886; 887-888; 889-890; 891-892; 893-894; 895-896; 897-898; 899-900; 901-902; 903-904; 905-906; 907-908; 909-910; 911-912; 913-914; 915-916; 917-918; 919-920; 921-922; 923-924; 925-926; 927-928; 929-930; 931-932; 933-934; 935-936; 937-938; 939-940; 941-942; 943-944;

945-946; 947-948; 949-950; 951-952; 953-954; 955-956; 957-958; 959-960; 961-962; 963-964; 965-966; 967-968; 969-970; 971-972; 973-974; 975-976; 977-978; 979-980; 981-982; 983-984; 985-986; 987-988; 989-990; 991-992; 993-994; 995-996; 997-998; 999-1000; 1001-1002; 1003-1004; 1005-1006; 1007-1008; 1009-1010; 1011-1012; 1013-1014; 1015-1016; 1017-1018; 1019-1020; 1021-1022; 1023-1024; 1025-1026; 1027-1028; 1029-1030; 1031-1032; 1033-1034; 1035-1036; 1037-1038; 1039-1040; 1041-1042; 1043-1044; 1045-1046; 1047-1048; 1049-1050; 1051-1052; 1053-1054; 1055-1056; 1057-1058; 1059-1060; 1061-1062; 1063-1064; 1065-1066; 1067-1068; 1069-1070; 1071-1072; 1073-1074; 1075-1076; 1077-1078; 1079-1080; 1081-1082; 1083-1084; 1085-1086; 1087-1088; 1089-1090; 1091-1092; 1093-1094; 1095-1096; 1097-1098; 1099-1100; 1101-1102; 1103-1104; 1105-1106; 1107-1108; 1109-1110; 1111-1112; 1113-1114; 1115-1116; 1117-1118; 1119-1120; 1121-1122; 1123-1124; 1125-1126; 1127-1128; 1129-1130; 1131-1132; 1133-1134; 1135-1136; 1137-1138; 1139-1140; 1141-1142; 1143-1144; 1145-1146; 1147-1148; 1149-1150; 1151-1152; 1153-1154; 1155-1156; 1157-1158; 1159-1160; 1161-1162; 1163-1164; 1165-1166; 1167-1168; 1169-1170; 1171-1172; 1173-1174; 1175-1176; 1177-1178; 1179-1180; 1181-1182; 1183-1184; 1185-1186; 1187-1188; 1189-1190; 1191-1192; 1193-1194; 1195-1196; 1197-1198; 1199-1200; 1201-1202; 1203-1204; 1205-1206; 1207-1208; 1209-1210; 1211-1212; 1213-1214; 1215-1216; 1217-1218; 1219-1220; 1221-1222; 1223-1224; 1225-1226; 1227-1228; 1229-1230; 1231-1232; 1233-1234; 1235-1236; 1237-1238; 1239-1240; 1241-1242; 1243-1244; 1245-1246; 1247-1248; 1249-1250; 1251-1252; 1253-1254; 1255-1256; 1257-1258; 1259-1260; 1261-1262; 1263-1264; 1265-1266; 1267-1268; 1269-1270; 1271-1272; 1273-1274; 1275-1276; 1277-1278; 1279-1280; 1281-1282; 1283-1284; 1285-1286; 1287-1288; 1289-1290; 1291-1292; 1293-1294; 1295-1296; 1297-1298; 1299-1300; 1301-1302; 1303-1304; 1305-1306; 1307-1308; 1309-1310; 1311-1312; 1313-1314; 1315-1316; 1317-1318; 1319-1320; 1321-1322; 1323-1324; 1325-1326; 1327-1328; 1329-1330; 1331-1332; 1333-1334; 1335-1336; 1337-1338; 1339-1340; 1341-1342; 1343-1344; 1345-1346; 1347-1348; 1349-1350; 1351-1352; 1353-1354; 1355-1356; 1357-1358; 1359-1360; 1361-1362; 1363-1364; 1365-1366; 1367-1368; 1369-1370; 1371-1372; 1373-1374; 1375-1376; 1377-1378; 1379-1380; 1381-1382; 1383-1384; 1385-1386; 1387-1388; 1389-1390; 1391-1392; 1393-1394; 1395-1396; 1397-1398; 1399-1400; 1401-1402; 1403-1404; 1405-1406; 1407-1408; 1409-1410; 1411-1412; 1413-1414; 1415-1416; 1417-1418; 1419-1420; 1421-1422; 1423-1424; 1425-1426; 1427-1428; 1429-1430; 1431-1432; 1433-1434; 1435-1436; 1437-1438; 1439-1440; 1441-1442; 1443-1444; 1445-1446; 1447-1448; 1449-1450; 1451-1452; 1453-1454; 1455-1456; 1457-1458; 1459-1460; 1461-1462; 1463-1464; 1465-1466; 1467-1468; 1469-1470; 1471-1472; 1473-1474; 1475-1476; 1477-1478; 1479-1480; 1481-1482; 1483-1484; 1485-1486; 1487-1488; 1489-1490; 1491-1492; 1493-1494; 1495-1496; 1497-1498; 1499-1500; 1501-1502; 1503-1504; 1505-1506; 1507-1508; 1509-1510; 1511-1512; 1513-1514; 1515-1516; 1517-1518; 1519-1520; 1521-1522; 1523-1524; 1525-1526; 1527-1528; 1529-1530; 1531-1532; 1533-1534; 1535-1536; 1537-1538; 1539-1540; 1541-1542; 1543-1544; 1545-1546; 1547-1548; 1549-1550; 1551-1552; 1553-1554; 1555-1556; 1557-1558; 1559-1560; 1561-1562; 1563-1564; 1565-1566; 1567-1568; 1569-1570; 1571-1572; 1573-1574; 1575-1576; 1577-1578; 1579-1580; 1581-1582; 1583-1584; 1585-1586; 1587-1588; 1589-1590; 1591-1592; 1593-1594; 1595-1596; 1597-1598; 1599-1600; 1601-1602; 1603-1604; 1605-1606; 1607-1608; 1609-1610; 1611-1612; 1613-1614; 1615-1616; 1617-1618; 1619-1620; 1621-1622; 1623-1624; 1625-1626; 1627-1628; 1629-1630; 1631-1632; 1633-1634; 1635-1636; 1637-1638; 1639-1640; 1641-1642; 1643-1644; 1645-1646; 1647-1648; 1649-1650; 1651-1652; 1653-1654; 1655-1656; 1657-1658; 1659-1660; 1661-1662; 1663-1664; 1665-1666; 1667-1668; 1669-1670; 1671-1672; 1673-1674; 1675-1676; 1677-1678; 1679-1680; 1681-1682; 1683-1684; 1685-1686; 1687-1688; 1689-1690; 1691-1692; 1693-1694; 1695-1696; 1697-1698; 1699-1700; 1701-1702; 1703-1704; 1705-1706; 1707-1708; 1709-1710; 1711-1712; 1713-1714; 1715-1716; 1717-1718; 1719-1720; 1721-1722; 1723-1724; 1725-1726; 1727-1728; 1729-1730; 1731-1732; 1733-1734; 1735-1736; 1737-1738; 1739-1740; 1741-1742; 1743-1744; 1745-1746; 1747-1748; 1749-1750; 1751-1752; 1753-1754; 1755-1756; 1757-1758; 1759-1760; 1761-1762; 1763-1764; 1765-1766; 1767-1768; 1769-1770; 1771-1772; 1773-1774; 1775-1776; 1777-1778; 1779-1780; 1781-1782; 1783-1784; 1785-1786; 1787-1788; 1789-1790; 1791-1792; 1793-1794; 1795-1796; 1797-1798; 1799-1800; 1801-1802; 1803-1804; 1805-1806; 1807-1808; 1809-1810; 1811-1812; 1813-1814; 1815-1816.

**6.** The method of any one of claims 1 to 5, comprising:

a) optionally, pretreating nucleic acids to remove human genomic DNA,
b) optionally, pre-amplify viral mRNAs, wherein said viral mRNAs comprises oncogenic mRNAs and at least one other mRNA,
c) sequencing mRNAs, or cDNAs thereof, obtained after steps a) and b), in the sample of a human subject,
d) identifying the reads corresponding to said oncogenic mRNAs,
e) identifying to which species or genotypes said oncogenic mRNAs of step d) belong to,
f) sorting the reads corresponding to said at least one other viral mRNAs, or cDNAs thereof obtained after steps a) and b), of the same genotype or species identified in step e),
g) optionally, identifying fusion transcripts as a signature of viral DNA integrations events in the host chromosome and/or additional human cancer cell biomarkers,
h) optionally, deleting all other sequences including human sequences which are not sequences identified and sorted following steps d), e), f) and g),
i) computing ratios R defining molecular abundance of said oncogenic mRNAs relative to said at least one other viral mRNAs of the same genotype or species of step f).

**7.** A method for diagnosis or prognosis risk to develop HPV induced cancer comprising:

(a) determining the level of at least a first marker selected from E6 mRNAs of group alpha HPVs, E7 mRNAs of group alpha HPVs, or both, in the sample of a patient or in the sample of an individual suspected to be infected by HPV, wherein said level in HPV species selective,
(b) comparing the levels determined in step (a) to a reference value of E6 mRNAs of group alpha HPVs, E7 mRNAs of group alpha HPVs, or both in low risk individuals infected with HPVs,

wherein an increased level as determined in step a) compared to the reference level in step b) is indicative of higher risk to develop HPV induced cancer,
wherein the method comprises a step of amplification with a group alpha HPV-specific composition of primers as defined in any of claims 1-2.

**8.** The method according to one of claims 6 and 7, wherein E6 or E7 mRNAs of HPVs belong to genus alpha HPVs, and wherein it comprises amplification and/or sequencing covering HPVs of at least groups α5, 6, 7, and 10.

**9.** The method of claim 8, wherein amplification or determination of the levels of E6 and L1 biomarkers comprises using a composition of primers comprising for E6: α5: both SEQ ID NO: 44 and SEQ ID NO: 45, and all three SEQ ID NO: 46, SEQ ID NO: 47 and SEQ ID NO: 48 and; α6: SEQ ID NO: 58 or both SEQ ID NO: 59 and SEQ ID NO: 60, and both SEQ ID NO: 61 and SEQ ID NO: 62 and; α7: all three SEQ ID NO: 73, SEQ ID NO: 75 and SEQ ID NO: 76 or all three SEQ ID NO: 74, SEQ ID NO: 75 and SEQ ID NO: 76, and all five SEQ ID NO: 77, SEQ ID NO: 78, SEQ ID NO: 79, SEQ ID NO: 80 and SEQ ID NO: 81 and ; α10: all three SEQ ID NO: 115, SEQ ID NO: 116, SEQ ID NO: 117 and all four SEQ ID NO: 118, SEQ ID NO: 119, SEQ ID NO: 120, SEQ ID NO: 121;
and comprising for L1 α5: SEQ ID NO: 53 or both SEQ ID NO: 54 and SEQ ID NO: 55, and both SEQ ID NO: 56 and SEQ ID NO: 57; and α6: SEQ ID NO: 71 and SEQ ID NO: 72; and α7: both SEQ ID NO: 87 and SEQ ID NO: 88 ,and both SEQ ID NO: 89 and SEQ ID NO: 90; and α10: both SEQ ID NO: 128, SEQ ID NO: 129 and SEQ ID NO: 130 or all four SEQ ID NO: 131, SEQ ID NO: 132, SEQ ID NO: 133, SEQ ID NO: 134.

**10.** The method of claim 8, wherein amplification or determination of the levels of E7 and L1 biomarkers comprises using a composition of primers comprising for E7 : α5: both SEQ ID NO: 49, SEQ ID NO: 50 and both SEQ ID NO: 51, SEQ ID NO: 52; and α6: SEQ ID NO: 63 or SEQ ID NO: 64 or SEQ ID NO: 65 SEQ ID NO: 66 or both SEQ ID NO: 67 and SEQ ID NO: 68, and both SEQ ID NO: 69 and SEQ ID NO: 70; and α7: SEQ ID NO: 82 or both SEQ ID NO: 83, SEQ ID NO: 84, and both SEQ ID NO: 85, SEQ ID NO: 86; and α10: all three SEQ ID NO: 122, SEQ ID NO: 123 and SEQ ID NO: 124, and all three SEQ ID NO: 125, SEQ ID NO: 126 and SEQ ID NO: 127; and α9: all three SEQ ID NO: 105, SEQ ID NO: 106 and SEQ ID NO: 107, and all three SEQ ID NO: 108, SEQ ID NO: 109 and 110; and α8: SEQ ID NO: 94, and both SEQ ID NO: 95 and SEQ ID NO: 96; and α1: SEQ ID NO: 3 and SEQ ID NO: 4;and α3: both SEQ ID NO: 28 and SEQ ID NO: 29, and all three SEQ ID NO: 30, SEQ ID NO: 31, SEQ ID NO: 32; and α2: all three SEQ ID NO: 12, SEQ ID NO: 13 and SEQ ID NO: 14, and all three SEQ ID NO: 15, SEQ ID NO: 16 and SEQ ID NO: 17;, and α4: SEQ ID NO: 38, and SEQ ID NO: 39; and α11 SEQ ID NO: 137, and SEQ ID NO: 138; and α13: SEQ ID NO: 143, and SEQ ID NO: 144; and α14: SEQ ID NO: 149,and SEQ ID NO: 150;
and comprising for L1 α5: SEQ ID NO: 53 or both SEQ ID NO: 54 and SEQ ID NO: 55, and both SEQ ID NO: 56 and SEQ ID NO: 57; and α6: SEQ ID NO: 71 and SEQ ID NO: 72; and α7: both SEQ ID NO: 87 and SEQ ID NO: 88 ,and both SEQ ID NO: 89 and SEQ ID NO: 90; and α10: both SEQ ID NO: 128, SEQ ID NO: 129 and SEQ ID NO: 130 or all four SEQ ID NO: 131, SEQ ID NO: 132, SEQ ID NO: 133, SEQ ID NO: 134; and α9: both SEQ ID NO: 111 and SEQ ID NO: 112, and both SEQ ID NO: 113 and SEQ ID NO: 114; and α8: SEQ ID NO: 97, and both SEQ ID NO: 98 and SEQ ID NO: 99; and α1: SEQ ID NO: 5 and SEQ ID NO: 6; and α3 SEQ ID NO: 33 and both SEQ ID NO: 34 and SEQ ID NO: 35; and α2 SEQ ID NO: 18, and both SEQ ID NO: 19 and SEQ ID NO: 20; and α4: SEQ ID NO: 40 or SEQ ID NO: 41, and SEQ ID NO: 42 or SEQ ID NO: 43; and α11: SEQ ID NO: 139, and SEQ ID NO: 140; and α13 SEQ ID NO: 145, and SEQ ID NO: 146; and α14: SEQ ID NO: 151 and SEQ ID NO: 152.

**11.** The method of claim 8, wherein amplification or determination of the levels of E6 and L1 biomarkers comprises using a composition of primers comprising for E6: α5: both SEQ ID NO: 44 and SEQ ID NO: 45, and all three SEQ ID NO: 46, SEQ ID NO: 47 and SEQ ID NO: 48 and; α6: SEQ ID NO: 58 or both SEQ ID NO: 59 and SEQ ID NO: 60, and both SEQ ID NO: 61 and SEQ ID NO: 62and; α7: all three SEQ ID NO: 73, SEQ ID NO: 75 and SEQ ID NO: 76 or all three SEQ ID NO: 74, SEQ ID NO: 75 and SEQ ID NO: 76, and all five SEQ ID NO: 77, SEQ ID NO: 78, SEQ ID NO: 79, SEQ ID NO: 80 and SEQ ID NO: 81 and ; α10: all three SEQ ID NO: 115, SEQ ID NO: 116, SEQ ID NO: 117 and all four SEQ ID NO: 118, SEQ ID NO: 119, SEQ ID NO: 120, SEQ ID NO: 121,
and comprising for L1 α5: SEQ ID NO: 53 or both SEQ ID NO: 54 and SEQ ID NO: 55, and both SEQ ID NO: 56 and SEQ ID NO: 57; and α6: SEQ ID NO: 71 and SEQ ID NO: 72; and α7: both SEQ ID NO: 87 and SEQ ID NO: 88 ,and both SEQ ID NO: 89 and SEQ ID NO: 90; and α10: both SEQ ID NO: 128, SEQ ID NO: 129 and SEQ ID NO: 130 or all four SEQ ID NO: 131, SEQ ID NO: 132, SEQ ID NO: 133, SEQ ID NO: 134.

**12.** The method of one of claims 8 to 11, wherein amplification or determination of the levels of E6 and L1 biomarkers comprises using a composition of primers comprising for E6: α5: both SEQ ID NO: 44 and SEQ ID NO: 45, and all three SEQ ID NO: 46, SEQ ID NO: 47 and SEQ ID NO: 48 and; α6: SEQ ID NO: 58 or both SEQ ID NO: 59 and SEQ ID NO: 60, and both SEQ ID NO: 61 and SEQ ID NO: 62 and; α7: all three SEQ ID NO: 73, SEQ ID NO: 75 and SEQ ID NO: 76 or all three SEQ ID NO: 74, SEQ ID NO: 75 and SEQ ID NO: 76, and all five SEQ ID NO: 77, SEQ ID NO: 78, SEQ ID NO: 79, SEQ ID NO: 80 and SEQ ID NO: 81 and ; α10: all three SEQ ID NO: 115, SEQ ID NO: 116, SEQ ID NO: 117 and all four SEQ ID NO: 118, SEQ ID NO: 119, SEQ ID NO: 120, SEQ ID NO: 121; and α9: both SEQ ID NO: 100 and SEQ ID NO: 101 and all three SEQ ID NO: 102, SEQ ID NO: 103 and SEQ ID NO: 104; and α8: SEQ ID NO: 91, and both SEQ ID NO: 92 and SEQ ID NO: 93; and α1: SEQ ID NO: 1 and SEQ ID NO: 2; and α3: all three SEQ ID NO: 21, SEQ ID NO: 22 and SEQ ID NO: 23, and all four SEQ ID NO: 24, SEQ ID NO: 25, SEQ ID NO: 26 and SEQ ID NO: 27; and α2 and both SEQ ID NO: 7 and SEQ ID NO: 8, and all three SEQ ID NO: 9, SEQ ID NO: 10 and SEQ ID NO: 11; and α4: SEQ ID NO: 36, and SEQ ID NO: 37; and α11: SEQ ID NO: 135, and SEQ ID NO: 136; and α13 SEQ ID NO: 141, and SEQ ID NO: 142; and α14: SEQ ID NO: 147, and SEQ ID NO: 148,
and comprising for L1, α5: SEQ ID NO: 53 or both SEQ ID NO: 54 and SEQ ID NO: 55, and both SEQ ID NO: 56 and SEQ ID NO: 57; and α6: SEQ ID NO: 71 and SEQ ID NO: 72; and α7: both SEQ ID NO: 87 and SEQ ID NO: 88 ,and both SEQ ID NO: 89 and SEQ ID NO: 90; and α10: both SEQ ID NO: 128, SEQ ID NO: 129 and SEQ ID NO: 130 or all four SEQ ID NO: 131, SEQ ID NO: 132, SEQ ID NO: 133, SEQ ID NO: 134; and α9: both SEQ ID NO: 111 and SEQ ID NO: 112, and both SEQ ID NO: 113 and SEQ ID NO: 114; and α8: SEQ ID NO: 97, and both SEQ ID NO: 98 and SEQ ID NO: 99; and α1: SEQ ID NO: 5 and SEQ ID NO: 6; and α3 SEQ ID NO: 33 and both SEQ ID NO: 34 and SEQ ID NO: 35;and α2 SEQ ID NO: 18, and both SEQ ID NO: 19 and SEQ ID NO: 20; and α4: SEQ ID NO: 40 or SEQ ID NO: 41, and SEQ ID NO: 42 or SEQ ID NO: 43; and α11: SEQ ID NO: 139, and SEQ ID NO: 140; and α13 SEQ ID NO: 145, and SEQ ID NO: 146; and α14: SEQ ID NO: 151 and SEQ ID NO: 152.

**13.** A composition of primers as defined in any one of claims 1, 2, 4, 5 and 9 to 12.

**14.** A kit for diagnosis or prognosis risk to develop HPV induced cancer comprising:

a) a composition of primers according to claim 13,
b) reagents to detect or sequence amplification products.

**15.** A kit for diagnosis or prognosis risk to develop HPV induced cancer comprising:

a) a composition of primers for detecting at least a first marker selected from E6 mRNAs of group alpha HPVs, E7 mRNAs of group alpha HPVs, or both,
b) a composition of primers for detecting at least a second marker selected from L1 mRNAs of group alpha HPVs, L2 mRNAs of group alpha HPVs, or both,
wherein said E6, E7, L1 and L2 mRNAs have corresponding intragenetic sequences,
c) and optionally, primers or probes for detecting at least one host cellular marker indicative of neoplasia or cancer,

wherein the composition of primers is as defined in claim 1 or 2.

## Patentansprüche

**1.** Verfahren zum Bestimmen eines Patientenrisikos des Entwickelns von durch onkogenes Virus induziertem Krebs, wie Gruppe-Alpha-HPV, umfassend:

a) Anreicherung der viralen RNAs in einer Probe unter Verwendung einer zufälligen oder Konsensus-Voramplifikation und/oder einer spezifischen reversen Transkriptase-Reaktion, vorzugsweise einer Konsensus-Voramplifikation,
b) Sequenzieren der in Schritt a) hergestellten cDNA und Erzeugen von Lesevorgängen der cDNA,
c) Bestimmen der Anzahl von Lesevorgängen, die mit den Viren übereinstimmen, basierend auf Speziesunterscheidung, und Bestimmen der am häufigsten in der Probe vorhandenen Hochrisikospezies relativ zu anderen Spezies,
d) Bestimmen der relativen Anzahl von Lesevorgängen, die mit mindestens einem onkogenen Gen im Vergleich zu mindestens einem nicht onkogenen Gen, vorzugsweise onkogenen Genen im Vergleich zu nicht onkogenen Genen, übereinstimmen, innerhalb der am weitesten verbreiteten Hochrisikospezies
e) Berechnen von Verhältnissen von Lesevorgängen innerhalb der Hochrisikospezies, die mit mindestens einem onkogenen Gen gegenüber entsprechenden strukturellen oder regulatorischen Interspeziesgenen, vorzugsweise onkogenen Genen gegenüber entsprechenden strukturellen oder regulatorischen Interspeziesgenen, übereinstimmen,
f) Bestimmen des Risikos des Entwickelns von durch onkogenes Virus induziertem Krebs bei Patienten, bei denen das Verhältnis gegen unendlich tendiert,

wobei die Anreicherung der viralen RNAs durch eine reverse Transkription der viralen RNAs und eine Amplifikation der hergestellten cDNA durch Multiplex-PCR mit einer Gruppe-Alpha-HPV-spezifischen Zusammensetzung von Primern durchgeführt wird, die mindestens eines der folgenden Gruppen von Primerpaaren umfasst:

- SD1-SA1-Gruppe, bestehend aus den Primerpaaren von SEQ ID NO: 397-398; 521-522; 609-610; 695-696; 819-820; 865-866; 947-948; 1067-1068; 1119-1120; 1267-1268; 1325-1326; 1507-1508; 1597-1598; 1655-1656; 1755-1756; und/oder
- SD1-SA2-Gruppe, bestehend aus den Primerpaaren von SEQ ID NO: 459-460; 633-634; 687-688; 1111-1112; 1235-1236; 1341 - 1342; 1503-1504; 1657-1658; 1797-1798; und/oder
- SD1-SA3-Gruppe, bestehend aus den Primerpaaren von SEQ ID NO: 381-382; 541-542; 599-600; 903-904; 941-942; 1047-1048; 1135-1136; 1287-1288; 1459-1460; 1473-1474; 1621-1622; 1717-1718; 1745-1746; und/oder
- SD1-SA4-Gruppe, bestehend aus den Primerpaaren von SEQ ID NO: 413-414; 551-552; 637-638; 713-714; 793-794; 857-858; 981-982; 1093-1094; 1179-1180; 1227-1228; 1319-1320; 1413-1414; 1509-1510; 1563-1564; 1709-1710; 1791-1792; und/oder
- SD1-SA5-Gruppe, bestehend aus den Primerpaaren von SEQ ID NO: 453-454; 549-550; 613-614; 747-748; 761-762; 949-950; 1163-1164; 1249-1250; 1329-1330; 1453-1454; 1501-1502; und/oder
- SD1-SA6-Gruppe, bestehend aus den Primerpaaren von SEQ ID NO: 431-432; 595-596; 719-720; 827-828; 1089-1090; 1137-1138; 1285-1286; 1353-1354; 1561-1562; 1719-1720; 1763-1764; und/oder
- SD1-SA7-Gruppe, bestehend aus den Primerpaaren von SEQ ID NO: 919-920; 1449-1450; und/oder
- SD1-SA8-Gruppe, bestehend aus den Primerpaaren von SEQ ID NO: 489-490; 963-964; 1519-1520; und/oder
- SD2-SA4-Gruppe, bestehend aus den Primerpaaren von SEQ ID NO: 387-388; 473-474; 615-616; 745-746; 815-816; 849-850; 933-934; 1091-1092; 1177-1178; 1209-1210; 1367-1368; 1437-1438; 1521-1522; 1603-1604; 1651-1652; 1779-1780; und/oder
- SD2-SA5-Gruppe, bestehend aus den Primerpaaren von SEQ ID NO: 455-456; 529-530; 629-630; 717-718; 777-778; 975-976; 1153-1154; 1273-1274; 1347-1348; 1451-1452; 1531-1532; und/oder
- SD2-SA6-Gruppe, bestehend aus den Primerpaaren von SEQ ID NO: 399-400; 645-646; 727-728; 811-812; 1079-1080; 1127-1128; 1253-1254; 1369-1370; 1615-1616; 1659-1660; 1781-1782; und/oder
- SD2-SA7-Gruppe, bestehend aus den Primerpaaren von SEQ ID NO: 531-532; 899-900; 943-944; 1411-1412; 1495-1496; und/oder
- SD2-SA9-Gruppe, bestehend aus den Primerpaaren von SEQ ID NO: 437-438; 505-506; 607-608; 739-740; 785-786; 887-888; 979-980; 1063-1064; 1185-1186; 1233-1234; 1297-1298; 1423-1424; 1491-1492; 1607-1608; 1693-1694; 1775-1776; und/oder
- SD2-SA10-Gruppe, bestehend aus den Primerpaaren von SEQ ID NO: 545-546; 831-832; 1149-1150; 1269-1270; 1427-1428; 1671-1672; und/oder
- SD3-SA4-Gruppe, bestehend aus den Primerpaaren von SEQ ID NO: 379-380; 483-484; 611-612; 721-722; 833-834; 911-912; 937-938; 1053-1054; 1139-1140; 1251-1252; 1335-1336; 1435-1436; 1487-1488; 1591-1592; 1715-1716; 1785-1786; und/oder
- SD3-SA5-Gruppe, bestehend aus den Primerpaaren von SEQ ID NO: 415-416; 493-494; 593-594; 733-734;

817-818; 993-994; 1145-1146; 1243-1244; 1337-1338; 1401-1402; 1483-1484; und/oder

- SD3-SA6-Gruppe, bestehend aus den Primerpaaren von SEQ ID NO: 435-436; 655-656; 673-674; 813-814; 1045-1046; 1173-1174; 1241-1242; 1303-1304; 1557-1558; 1627-1628; 1647-1648; 1773-1774; und/oder
- SD3-SA7-Gruppe, bestehend aus den Primerpaaren von SEQ ID NO: 855-856; 1387-1388; und/oder
- SD3-SA8-Gruppe, bestehend aus den Primerpaaren von SEQ ID NO: 511-512; 957-958; 1529-1530; und/oder
- SD5-SA9-Gruppe, bestehend aus den Primerpaaren von SEQ ID NO: 419-420; 527-528; 567-568; 587-588; 683-684; 775-776; 891-892; 999-1000; 1041-1042; 1113-1114; 1247-1248; 1371-1372; 1403-1404; 1511-1512; 1617-1618; 1677-1678; 1733-1734; und/oder
- SD5-SA10-Gruppe, bestehend aus den Primerpaaren von SEQ ID NO: 495-496; 837-838; 1183-1184; 1279-1280; 1433-1434; 1723-1724.

**2.** Verfahren nach Anspruch 1, wobei die Gruppe-Alpha-HPV-spezifische Zusammensetzung von Primern die folgenden Gruppen von Primerpaaren umfasst:
SD1-SA1-Gruppe, bestehend aus den Primerpaaren von SEQ ID NO: 397-398; 521-522; 609-610; 695-696; 819-820; 865-866; 947-948; 1067-1068; 1119-1120; 1267-1268; 1325-1326; 1507-1508; 1597-1598; 1655-1656; 1755-1756.

**3.** Verfahren nach einem der Ansprüche 1 bis 2, wobei die Gruppe-Alpha-HPV-spezifische Zusammensetzung von Primern Spleißverbindungen, genomische und ungespleißte Regionen und humane Fusionstranskriptregionen jedes HPV-Transkripts umschließt.

**4.** Verfahren nach einem der Ansprüche 1 bis 3, wobei die Gruppe-Alpha-HPV-spezifische Zusammensetzung von Primern mindestens eine, vorzugsweise alle, der folgenden Gruppen von Primerpaaren umfasst:

- die HPV16-spezifischen Primer, umfassend oder bestehend aus den Primern von SEQ ID NO: 219-258 für genomische und ungespleißte HPV16-Transkripte, SEQ ID NO: 259-352 für gespleißte HPV16-Transkripte und SEQ ID NO: 353-376 für humane HPV16-Fusionstranskripte (einschließlich der Primerpaare von SEQ ID NO: 219-220; 221-222; 223-224; 225-226; 227-228; 229-230; 231-232; 233-234; 235-236; 237-238; 239-240; 241-242; 243-244; 245-246; 247-248; 249-250; 251-252; 253-254; 255-256; 257-258; 259-260; 261-262; 263-264; 265-266; 267-268; 269-270; 271-272; 273-274; 275-276; 277-278; 279-280; 281-282; 283-284; 285-286; 287-288; 289-290; 291-292; 293-294; 295-296; 297-298; 299-300; 301-302; 303-304; 305-306; 307-308; 309-310; 311-312; 313-314; 315-316; 317-318; 319-320; 321-322; 323-324; 325-326; 327-328; 329-330; 331-332; 333-334; 335-336; 337-338; 339-340; 341-342; 343-344; 345-346; 347-348; 349-350; 351-352; 353-354; 355-356; 357-358; 359-360; 361-362; 363-364; 365-366; 367-368; 369-370; 371-372; 373-374; 375-376) oder 377-470 (einschließlich der Primerpaare von SEQ ID NO. 377-378; 379-380; 381-382; 383-384; 385-386; 387-388; 389-390; 391-392; 393-394; 395-396; 397-398; 399-400; 401-402; 403-404; 405-406; 407-408; 409-410; 411-412; 413-414; 415-416; 417-418; 419-420; 421-422; 423-424; 425-426; 427-428; 429-430; 431-432; 433-434; 435-436; 437-438; 439-440; 441-442; 443-444; 445-446; 447-448; 449-450; 451-452; 453-454; 455-456; 457-458; 459-460; 461-462; 463-464; 465-466; 467-468 und; 469-470); und/oder
- die HPV18-spezifischen Primer, umfassend oder bestehend aus den Primern von SEQ ID NO. 471-574 (einschließlich der Primerpaare von SEQ ID NO.: 471-472; 473-474; 475-476; 477-478; 479-480; 481-482; 483-484; 485-486; 487-488; 489-490; 491-492; 493-494; 495-496; 497-498; 499-500; 501-502; 503-504; 505-506; 507-508; 509-510; 511-512; 513-514; 515-516; 517-518; 519-520; 521-522; 523-524; 525-526; 527-528; 529-530; 531-532; 533-534; 535-536; 537-538; 539-540; 541-542; 543-544; 545-546; 547-548; 549-550; 551-552; 553-554; 555-556; 557-558; 559-560; 561-562; 563-564; 565-566; 567-568; 569-570; 571-572; 573-574); und/oder
- die HPV31-spezifischen Primer, umfassend oder bestehend aus den Primern von SEQ ID NO. 575-668 (einschließlich der Primerpaare von SEQ ID NO.: 575-576; 577-578; 579-580; 581-582; 583-584; 585-586; 587-588; 589-590; 591-592; 593-594; 595-596; 597-598; 599-600; 601-602; 603-604; 605-606; 607-608; 609-610; 611-612; 613-614; 615-616; 617-618; 619-620; 621-622; 623-624; 625-626; 627-628; 629-630; 631-632; 633-634; 635-636; 637-638; 639-640; 641-642; 643-644; 645-646; 647-648; 649-650; 651-652; 653-654; 655-656; 657-658; 659-660; 661-662; 663-664; 665-666; 667-668); und/oder
- die HPV33-spezifischen Primer, umfassend oder bestehend aus SEQ ID NO. 669-756 (einschließlich der Primerpaare von SEQ ID NO.: 669-670; 671-672; 673-674; 675-676; 677-678; 679-680; 681-682; 683-684; 685-686; 687-688; 689-690; 691-692; 693-694; 695-696; 697-698; 699-700; 701-702; 703-704; 705-706; 707-708; 709-710; 711-712; 713-714; 715-716; 717-718; 719-720; 721-722; 723-724; 725-726; 727-728; 729-730; 731-732; 733-734; 735-736; 737-738; 739-740; 741-742; 743-744; 745-746; 747-748; 749-750;

751-752; 753-754; 755-756); und/oder

- die HPV35-spezifischen Primer, umfassend oder bestehend aus den Primern von SEQ ID NO. 757-848 (einschließlich der Primerpaare von SEQ ID NO.: 757-758; 759-760; 761-762; 763-764; 765-766; 767-768; 769-770; 771-772; 773-774; 775-776; 777-778; 779-780; 781-782; 783-784; 785-786; 787-788; 789-790; 791-792; 793-794; 795-796; 797-798; 799-800; 801-802; 803-804; 805-806; 807-808; 809-810; 811-812; 813-814; 815-816; 817-818; 819-820; 821-822; 823-824; 825-826; 827-828; 829-830; 831-832; 833-834; 835-836; 837-838; 839-840; 841-842; 843-844; 845-846; 847-848); und/oder

- die HPV39-spezifischen Primer, umfassend oder bestehend aus den Primern von SEQ ID NO. 849-928 (einschließlich der Primerpaare von SEQ ID NO.: 849-850; 851-852; 853-854; 855-856; 857-858; 859-860; 861-862; 863-864; 865-866; 867-868; 869-870; 871-872; 873-874; 875-876; 877-878; 879-880; 881-882; 883-884; 885-886; 887-888; 889-890; 891-892; 893-894; 895-896; 897-898; 899-900; 901-902; 903-904; 905-906; 907-908; 909-910; 911-912; 913-914; 915-916; 917-918; 919-920; 921-922; 923-924; 925-926; 927-928); und/oder

- die HPV45-spezifischen Primer, umfassend oder bestehend aus den Primern von SEQ ID NO. 929-1020 (einschließlich der Primerpaare von SEQ ID NO.: 929-930; 931-932; 933-934; 935-936; 937-938; 939-940; 941-942; 943-944; 945-946; 947-948; 949-950; 951-952; 953-954; 955-956; 957-958; 959-960; 961-962; 963-964; 965-966; 967-968; 969-970; 971-972; 973-974; 975-976; 977-978; 979-980; 981-982; 983-984; 985-986; 987-988; 989-990; 991-992; 993-994; 995-996; 997-998; 999-1000; 1001-1002; 1003-1004; 1005-1006; 1007-1008; 1009-1010; 1011-1012; 1013-1014; 1015-1016; 1017-1018; 1019-1020); und/oder

- die HPV51-spezifischen Primer, umfassend oder bestehend aus den Primern von SEQ ID NO. 1021-1102 (einschließlich der Primerpaare von SEQ ID NO.: 1021-1022; 1023-1024; 1025-1026; 1027-1028; 1029-1030; 1031-1032; 1033-1034; 1035-1036; 1037-1038; 1039-1040; 1041-1042; 1043-1044; 1045-1046; 1047-1048; 1049-1050; 1051-1052; 1053-1054; 1055-1056; 1057-1058; 1059-1060; 1061-1062; 1063-1064; 1065-1066; 1067-1068; 1069-1070; 1071-1072; 1073-1074; 1075-1076; 1077-1078; 1079-1080; 1081-1082; 1083-1084; 1085-1086; 1087-1088; 1089-1090; 1091-1092; 1093-1094; 1095-1096; 1097-1098; 1099-1100; 1101-1102); und/oder

- die HPV52-spezifischen Primer, umfassend oder bestehend aus den Primern von SEQ ID NO. 1103-1200 (einschließlich der Primerpaare von SEQ ID NO.: 1103-1104; 1105-1106; 1107-1108; 1109-1110; 1111-1112; 1113-1114; 1115-1116; 1117-1118; 1119-1120; 1121-1122; 1123-1124; 1125-1126; 1127-1128; 1129-1130; 1131-1132; 1133-1134; 1135-1136; 1137-1138; 1139-1140; 1141-1142; 1143-1144; 1145-1146; 1147-1148; 1149-1150; 1151-1152; 1153-1154; 1155-1156; 1157-1158; 1159-1160; 1161-1162; 1163-1164; 1165-1166; 1167-1168; 1169-1170; 1171-1172; 1173-1174; 1175-1176; 1177-1178; 1179-1180; 1181-1182; 1183-1184; 1185-1186; 1187-1188; 1189-1190; 1191-1192; 1193-1194; 1195-1196; 1197-1198; 1199-1200); und/oder

- die HPV56-spezifischen Primer, umfassend oder bestehend aus den Primern von SEQ ID NO. 1201-1296 (einschließlich der Primerpaare von SEQ ID NO.: 1201-1202; 1203-1204; 1205-1206; 1207-1208; 1209-1210; 1211-1212; 1213-1214; 1215-1216; 1217-1218; 1219-1220; 1221-1222; 1223-1224; 1225-1226; 1227-1228; 1229-1230; 1231-1232; 1233-1234; 1235-1236; 1237-1238; 1239-1240; 1241-1242; 1243-1244; 1245-1246; 1247-1248; 1249-1250; 1251-1252; 1253-1254; 1255-1256; 1257-1258; 1259-1260; 1261-1262; 1263-1264; 1265-1266; 1267-1268; 1269-1270; 1271-1272; 1273-1274; 1275-1276; 1277-1278; 1279-1280; 1281-1282; 1283-1284; 1285-1286; 1287-1288; 1289-1290; 1291-1292; 1293-1294; 1295-1296); und/oder

- die HPV58-spezifischen Primer, umfassend oder bestehend aus den Primern von SEQ ID NO. 1297-1382 (einschließlich der Primerpaare von SEQ ID NO.: 1297-1298; 1299-1300; 1301-1302; 1303-1304; 1305-1306; 1307-1308; 1309-1310; 1311-1312; 1313-1314; 1315-1316; 1317-1318; 1319-1320; 1321-1322; 1323-1324; 1325-1326; 1327-1328; 1329-1330; 1331-1332; 1333-1334; 1335-1336; 1337-1338; 1339-1340; 1341-1342; 1343-1344; 1345-1346; 1347-1348; 1349-1350; 1351-1352; 1353-1354; 1355-1356; 1357-1358; 1359-1360; 1361-1362; 1363-1364; 1365-1366; 1367-1368; 1369-1370; 1371-1372; 1373-1374; 1375-1376; 1377-1378; 1379-1380; 1381-1382); und/oder

- die HPV59-spezifischen Primer, umfassend oder bestehend aus den Primern von SEQ ID NO. 1383-1470 (einschließlich der Primerpaare von SEQ ID NO.: 1383-1384; 1385-1386; 1387-1388; 1389-1390; 1391-1392; 1393-1394; 1395-1396; 1397-1398; 1399-1400; 1401-1402; 1403-1404; 1405-1406; 1407-1408; 1409-1410; 1411-1412; 1413-1414; 1415-1416; 1417-1418; 1419-1420; 1421-1422; 1423-1424; 1425-1426; 1427-1428; 1429-1430; 1431-1432; 1433-1434; 1435-1436; 1437-1438; 1439-1440; 1441-1442; 1443-1444; 1445-1446; 1447-1448; 1449-1450; 1451-1452; 1453-1454; 1455-1456; 1457-1458; 1459-1460; 1461-1462; 1463-1464; 1465-1466; 1467-1468; 1469-1470); und/oder

- die HPV66-spezifischen Primer, umfassend oder bestehend aus den Primern von SEQ ID NO. 1471-1560 (einschließlich der Primerpaare von SEQ ID NO.: 1471-1472; 1473-1474; 1475-1476; 1477-1478; 1479-1480; 1481-1482; 1483-1484; 1485-1486; 1487-1488; 1489-1490; 1491-1492; 1493-1494; 1495-1496; 1497-1498; 1499-1500; 1501-1502; 1503-1504; 1505-1506; 1507-1508; 1509-1510; 1511-1512; 1513-1514; 1515-1516;

1517-1518; 1519-1520; 1521-1522; 1523-1524; 1525-1526; 1527-1528; 1529-1530;1531-1532; 1533-1534; 1535-1536; 1537-1538; 1539-1540; 1541-1542; 1543-1544; 1545-1546; 1547-1548; 1549-1550; 1551-1552; 1553-1554; 1555-1556; 1557-1558; 1559-1560; und/oder

- die HPV68-spezifischen Primer, umfassend oder bestehend aus den Primern von SEQ ID NO. 1561-1642 (einschließlich der Primerpaare von SEQ ID NO.: 1561-1562; 1563-1564; 1565-1566; 1567-1568; 1569-1570; 1571-1572; 1573-1574; 1575-1576; 1577-1578; 1579-1580; 1581-1582; 1583-1584; 1585-1586; 1587-1588; 1589-1590; 1591-1592; 1593-1594; 1595-1596; 1597-1598; 1599-1600; 1601-1602; 1603-1604; 1605-1606; 1607-1608; 1609-1610; 1611-1612; 1613-1614; 1615-1616; 1617-1618; 1619-1620; 1621-1622; 1623-1624; 1625-1626; 1627-1628; 1629-1630; 1631-1632; 1633-1634; 1635-1636; 1637-1638; 1639-1640; 1641-1642); und/oder
- die HPV73-spezifischen Primer, umfassend oder bestehend aus den Primern von SEQ ID NO. 1643-1732 (einschließlich der Primerpaare von SEQ ID NO.: 1643-1644; 1645-1646; 1647-1648; 1649-1650; 1651-1652; 1653-1654; 1655-1656; 1657-1658; 1659-1660; 1661-1662; 1663-1664; 1665-1666; 1667-1668; 1669-1670; 1671-1672; 1673-1674; 1675-1676; 1677-1678; 1679-1680; 1681-1682; 1683-1684; 1685-1686; 1687-1688; 1689-1690; 1691-1692; 1693-1694; 1695-1696; 1697-1698; 1699-1700; 1701-1702; 1703-1704; 1705-1706; 1707-1708; 1709-1710; 1711-1712; 1713-1714; 1715-1716; 1717-1718; 1719-1720; 1721-1722; 1723-1724; 1725-1726; 1727-1728; 1729-1730; 1731-1732); und/oder
- die HPV82-spezifischen Primer, umfassend oder bestehend aus den Primern von SEQ ID NO. 1733-1816 (einschließlich der Primerpaare von SEQ ID NO.: 1733-1734; 1735-1736; 1737-1738; 1739-1740; 1741-1742; 1743-1744; 1745-1746; 1747-1748; 1749-1750; 1751-1752; 1753-1754; 1755-1756; 1757-1758; 1759-1760; 1761-1762; 1763-1764; 1765-1766; 1767-1768; 1769-1770; 1771-1772; 1773-1774; 1775-1776; 1777-1778; 1779-1780; 1781-1782; 1783-1784; 1785-1786; 1787-1788; 1789-1790; 1791-1792; 1793-1794; 1795-1796; 1797-1798; 1799-1800; 1801-1802; 1803-1804; 1805-1806; 1807-1808; 1809-1810; 1811-1812; 1813-1814; 1815-1816).

**5.** Verfahren nach Anspruch 1 oder 2, wobei die Gruppe-Alpha-HPV-spezifische Zusammensetzung von Primern eine der folgenden Gruppen von Primerpaaren umfasst:

- die Gruppe von Primerpaaren von SEQ ID NO: 397-398; 521-522; 609-610; 695-696; 819-820; 865-866; 947-948; 1067-1068; 1119-1120; 1267-1268; 1325-1326; 1507-1508; 1597-1598; 1655-1656; 1755-1756; 459-460; 633-634; 687-688; 1111-1112; 1235-1236; 1341-1342; 1503-1504; 1657-1658; 1797-1798; 381-382; 541-542; 599-600; 903-904; 941-942; 1047-1048; 1135-1136; 1287-1288; 1459-1460; 1473-1474; 1621-1622; 1717-1718; 1745-1746; 413-414; 551-552; 637-638; 713-714; 793-794; 857-858; 981-982; 1093-1094; 1179-1180; 1227-1228; 1319-1320; 1413-1414; 1509-1510; 1563-1564; 1709-1710; 1791-1792; 453-454; 549-550; 613-614; 747-748; 761-762; 949-950; 1163-1164; 1249-1250; 1329-1330; 1453-1454; 1501-1502; 431-432; 595-596; 719-720; 827-828; 1089-1090; 1137-1138; 1285-1286; 1353-1354; 1561-1562; 1719-1720; 1763-1764; 919-920; 1449-1450; 489-490; 963-964; 1519-1520; 387-388; 473-474; 615-616; 745-746; 815-816; 849-850; 933-934; 1091-1092; 1177-1178; 1209-1210; 1367-1368; 1437-1438; 1521-1522; 1603-1604; 1651-1652; 1779-1780; 455-456; 529-530; 629-630; 717-718; 777-778; 975-976; 1153-1154; 1273-1274; 1347-1348; 1451-1452; 1531-1532; 399-400; 645-646; 727-728; 811-812; 1079-1080; 1127-1128; 1253-1254; 1369-1370; 1615-1616; 1659-1660; 1781-1782; 531-532; 899-900; 943-944; 1411-1412; 1495-1496; 437-438; 505-506; 607-608; 739-740; 785-786; 887-888; 979-980; 1063-1064; 1185-1186; 1233-1234; 1297-1298; 1423-1424; 1491-1492; 1607-1608; 1693-1694; 1775-1776; 545-546; 831-832; 1149-1150; 1269-1270; 1427-1428; 1671-1672; 379-380; 483-484; 611-612; 721-722; 833-834; 911-912; 937-938; 1053-1054; 1139-1140; 1251-1252; 1335-1336; 1435-1436; 1487-1488; 1591-1592; 1715-1716; 1785-1786; 415-416; 493-494; 593-594; 733-734; 817-818; 993-994; 1145-1146; 1243-1244; 1337-1338; 1401-1402; 1483-1484; 435-436; 655-656; 673-674; 813-814; 1045-1046; 1173-1174; 1241-1242; 1303-1304; 1557-1558; 1627-1628; 1647-1648; 1773-1774; 855-856; 1387-1388; 511-512; 957-958; 1529-1530; 477-478; 419-420; 527-528; 567-568; 587-588; 683-684; 775-776; 891-892; 999-1000; 1041-1042; 1113-1114; 1247-1248; 1371-1372; 1403-1404; 1511-1512; 1617-1618; 1677-1678; 1733-1734; 495-496; 837-838; 1183-1184; 1279-1280; 1433-1434; 1723-1724; 1011-1012; 557-558; oder
- die Gruppe von Primerpaaren von SEQ ID NO: 229-230; 233-234; 235-236; 245-246; 247-248; 249-250; 251-252; 255-256; 257-258; 265-266; 273-274; 275-276; 277-278; 279-280; 281-282; 289-290; 291-292; 295-296; 297-298; 299-300; 301-302; 303-304; 305-306; 307-308; 309-310; 311-312; 319-320; 321-322; 323-324; 325-326; 327-328; 329-330; 331-332; 333-334; 335-336; 337-338; 341-342; 343-344; 345-346; 347-348; 349-350; 351-352; 377-378; 379-380; 381-382; 383-384; 385-386; 387-388; 389-390; 391-392; 393-394; 395-396; 397-398; 399-400; 401-402; 403-404; 405-406; 407-408; 409-410; 411-412; 413-414; 415-416; 417-418; 419-420; 421-422; 423-424; 425-426; 427-428; 429-430; 431-432; 433-434; 435-436;

437-438; 439-440; 441-442; 443-444; 445-446; 447-448; 449-450; 451-452; 453-454; 455-456; 457-458; 459-460; 461-462; 463-464; 465-466; 467-468; 469-470; 471-472; 473-474; 475-476; 477-478; 479-480; 481-482; 483-484; 485-486; 487-488; 489-490; 491-492; 493-494; 495-496; 497-498; 499-500; 501-502; 503-504; 505-506; 507-508; 509-510; 511-512; 513-514; 515-516; 517-518; 519-520; 521-522; 523-524; 525-526; 527-528; 529-530; 531-532; 533-534; 535-536; 537-538; 539-540; 541-542; 543-544; 545-546; 547-548; 549-550; 551-552; 553-554; 555-556; 557-558; 559-560; 561-562; 563-564; 565-566; 567-568; 569-570; 571-572; 573-574; 575-576; 577-578; 579-580; 581-582; 583-584; 585-586; 587-588; 589-590; 591-592; 593-594; 595-596; 597-598; 599-600; 601-602; 603-604; 605-606; 607-608; 609-610; 611-612; 613-614; 615-616; 617-618; 619-620; 621-622; 623-624; 625-626; 627-628; 629-630; 631-632; 633-634; 635-636; 637-638; 639-640; 641-642; 643-644; 645-646; 647-648; 649-650; 651-652; 653-654; 655-656; 657-658; 659-660; 661-662; 663-664; 665-666; 667-668; 669-670; 671-672; 673-674; 675-676; 677-678; 679-680; 681-682; 683-684; 685-686; 687-688; 689-690; 691-692; 693-694; 695-696; 697-698; 699-700; 701-702; 703-704; 705-706; 707-708; 709-710; 711-712; 713-714; 715-716; 717-718; 719-720; 721-722; 723-724; 725-726; 727-728; 729-730; 731-732; 733-734; 735-736; 737-738; 739-740; 741-742; 743-744; 745-746; 747-748; 749-750; 751-752; 753-754; 755-756; 757-758; 759-760; 761-762; 763-764; 765-766; 767-768; 769-770; 771-772; 773-774; 775-776; 777-778; 779-780; 781-782; 783-784; 785-786; 787-788; 789-790; 791-792; 793-794; 795-796; 797-798; 799-800; 801-802; 803-804; 805-806; 807-808; 809-810; 811-812; 813-814; 815-816; 817-818; 819-820; 821-822; 823-824; 825-826; 827-828; 829-830; 831-832; 833-834; 835-836; 837-838; 839-840; 841-842; 843-844; 845-846; 847-848; 849-850; 851-852; 853-854; 855-856; 857-858; 859-860; 861-862; 863-864; 865-866; 867-868; 869-870; 871-872; 873-874; 875-876; 877-878; 879-880; 881-882; 883-884; 885-886; 887-888; 889-890; 891-892; 893-894; 895-896; 897-898; 899-900; 901-902; 903-904; 905-906; 907-908; 909-910; 911-912; 913-914; 915-916; 917-918; 919-920; 921-922; 923-924; 925-926; 927-928; 929-930; 931-932; 933-934; 935-936; 937-938; 939-940; 941-942; 943-944; 945-946; 947-948; 949-950; 951-952; 953-954; 955-956; 957-958; 959-960; 961-962; 963-964; 965-966; 967-968; 969-970; 971-972; 973-974; 975-976; 977-978; 979-980; 981-982; 983-984; 985-986; 987-988; 989-990; 991-992; 993-994; 995-996; 997-998; 999-1000; 1001-1002; 1003-1004; 1005-1006; 1007-1008; 1009-1010; 1011-1012; 1013-1014; 1015-1016; 1017-1018; 1019-1020; 1021-1022; 1023-1024; 1025-1026; 1027-1028; 1029-1030; 1031-1032; 1033-1034; 1035-1036; 1037-1038; 1039-1040; 1041-1042; 1043-1044; 1045-1046; 1047-1048; 1049-1050; 1051-1052; 1053-1054; 1055-1056; 1057-1058; 1059-1060; 1061-1062; 1063-1064; 1065-1066; 1067-1068; 1069-1070; 1071-1072; 1073-1074; 1075-1076; 1077-1078; 1079-1080; 1081-1082; 1083-1084; 1085-1086; 1087-1088; 1089-1090; 1091-1092; 1093-1094; 1095-1096; 1097-1098; 1099-1100; 1101-1102; 1103-1104; 1105-1106; 1107-1108; 1109-1110; 1111-1112; 1113-1114; 1115-1116; 1117-1118; 1119-1120; 1121-1122; 1123-1124; 1125-1126; 1127-1128; 1129-1130; 1131-1132; 1133-1134; 1135-1136; 1137-1138; 1139-1140; 1141-1142; 1143-1144; 1145-1146; 1147-1148; 1149-1150; 1151-1152; 1153-1154; 1155-1156; 1157-1158; 1159-1160; 1161-1162; 1163-1164; 1165-1166; 1167-1168; 1169-1170; 1171-1172; 1173-1174; 1175-1176; 1177-1178; 1179-1180; 1181-1182; 1183-1184; 1185-1186; 1187-1188; 1189-1190; 1191-1192; 1193-1194; 1195-1196; 1197-1198; 1199-1200; 1201-1202; 1203-1204; 1205-1206; 1207-1208; 1209-1210; 1211-1212; 1213-1214; 1215-1216; 1217-1218; 1219-1220; 1221-1222; 1223-1224; 1225-1226; 1227-1228; 1229-1230; 1231-1232; 1233-1234; 1235-1236; 1237-1238; 1239-1240; 1241-1242; 1243-1244; 1245-1246; 1247-1248; 1249-1250; 1251-1252; 1253-1254; 1255-1256; 1257-1258; 1259-1260; 1261-1262; 1263-1264; 1265-1266; 1267-1268; 1269-1270; 1271-1272; 1273-1274; 1275-1276; 1277-1278; 1279-1280; 1281-1282; 1283-1284; 1285-1286; 1287-1288; 1289-1290; 1291-1292; 1293-1294; 1295-1296; 1297-1298; 1299-1300; 1301-1302; 1303-1304; 1305-1306; 1307-1308; 1309-1310; 1311-1312; 1313-1314; 1315-1316; 1317-1318; 1319-1320; 1321-1322; 1323-1324; 1325-1326; 1327-1328; 1329-1330; 1331-1332; 1333-1334; 1335-1336; 1337-1338; 1339-1340; 1341-1342; 1343-1344; 1345-1346; 1347-1348; 1349-1350; 1351-1352; 1353-1354; 1355-1356; 1357-1358; 1359-1360; 1361-1362; 1363-1364; 1365-1366; 1367-1368; 1369-1370; 1371-1372; 1373-1374; 1375-1376; 1377-1378; 1379-1380; 1381-1382; 1383-1384; 1385-1386; 1387-1388; 1389-1390; 1391-1392; 1393-1394; 1395-1396; 1397-1398; 1399-1400; 1401-1402; 1403-1404; 1405-1406; 1407-1408; 1409-1410; 1411-1412; 1413-1414; 1415-1416; 1417-1418; 1419-1420; 1421-1422; 1423-1424; 1425-1426; 1427-1428; 1429-1430; 1431-1432; 1433-1434; 1435-1436; 1437-1438; 1439-1440; 1441-1442; 1443-1444; 1445-1446; 1447-1448; 1449-1450; 1451-1452; 1453-1454; 1455-1456; 1457-1458; 1459-1460; 1461-1462; 1463-1464; 1465-1466; 1467-1468; 1469-1470; 1471-1472; 1473-1474; 1475-1476; 1477-1478; 1479-1480; 1481-1482; 1483-1484; 1485-1486; 1487-1488; 1489-1490; 1491-1492; 1493-1494; 1495-1496; 1497-1498; 1499-1500; 1501-1502; 1503-1504; 1505-1506; 1507-1508; 1509-1510; 1511-1512; 1513-1514; 1515-1516; 1517-1518; 1519-1520; 1521-1522; 1523-1524; 1525-1526; 1527-1528; 1529-1530; 1531-1532; 1533-1534; 1535-1536; 1537-1538; 1539-1540; 1541-1542; 1543-1544; 1545-1546; 1547-1548; 1549-1550; 1551-1552; 1553-1554; 1555-1556; 1557-1558; 1559-1560; 1561-1562; 1563-1564; 1565-1566; 1567-1568; 1569-1570; 1571-1572; 1573-1574; 1575-1576; 1577-1578; 1579-1580; 1581-1582;

1583-1584; 1585-1586; 1587-1588; 1589-1590; 1591-1592; 1593-1594; 1595-1596; 1597-1598; 1599-1600; 1601-1602; 1603-1604; 1605-1606; 1607-1608; 1609-1610; 1611-1612; 1613-1614; 1615-1616; 1617-1618; 1619-1620; 1621-1622; 1623-1624; 1625-1626; 1627-1628; 1629-1630; 1631-1632; 1633-1634; 1635-1636; 1637-1638; 1639-1640; 1641-1642; 1643-1644; 1645-1646; 1647-1648; 1649-1650; 1651-1652; 1653-1654; 1655-1656; 1657-1658; 1659-1660; 1661-1662; 1663-1664; 1665-1666; 1667-1668; 1669-1670; 1671-1672; 1673-1674; 1675-1676; 1677-1678; 1679-1680; 1681-1682; 1683-1684; 1685-1686; 1687-1688; 1689-1690; 1691-1692; 1693-1694; 1695-1696; 1697-1698; 1699-1700; 1701-1702; 1703-1704; 1705-1706; 1707-1708; 1709-1710; 1711-1712; 1713-1714; 1715-1716; 1717-1718; 1719-1720; 1721-1722; 1723-1724; 1725-1726; 1727-1728; 1729-1730; 1731-1732; 1733-1734; 1735-1736; 1737-1738; 1739-1740; 1741-1742; 1743-1744; 1745-1746; 1747-1748; 1749-1750; 1751-1752; 1753-1754; 1755-1756; 1757-1758; 1759-1760; 1761-1762; 1763-1764; 1765-1766; 1767-1768; 1769-1770; 1771-1772; 1773-1774; 1775-1776; 1777-1778; 1779-1780; 1781-1782; 1783-1784; 1785-1786; 1787-1788; 1789-1790; 1791-1792; 1793-1794; 1795-1796; 1797-1798; 1799-1800; 1801-1802; 1803-1804; 1805-1806; 1807-1808; 1809-1810; 1811-1812; 1813-1814; 1815-1816.

**6.** Verfahren nach einem der Ansprüche 1 bis 5, umfassend:

a) wahlweise Vorbehandeln von Nukleinsäuren, um humane genomische DNA zu entfernen,
b) wahlweise Voramplifizieren von viralen mRNAs, wobei die viralen mRNAs onkogene mRNAs und mindestens eine andere mRNA umfassen,
c) Sequenzieren von mRNAs oder cDNAs davon, die nach Schritten a) und b) erhalten wurden, in der Probe eines humanen Subjekts,
d) Identifizieren der Lesevorgänge, die den onkogenen mRNAs entsprechen,
e) Identifizieren, zu welchen Spezies oder Genotypen die onkogenen mRNAs von Schritt d) gehören,
f) Sortieren der Lesevorgänge, die der mindestens einen anderen viralen mRNA oder deren nach Schritt a) und b) erhaltenen cDNAs des gleichen Genotyps oder der gleichen Spezies entsprechen, die in Schritt e) identifiziert wurden,
g) wahlweise Identifizieren von Fusions-Transkripten als eine Signatur von viralen DNA-Integrationsereignissen im Wirtschromosom und/oder zusätzlichen humanen Krebszell-Biomarkern,
h) wahlweise Deletieren aller anderen Sequenzen, einschließlich humaner Sequenzen, die keine Sequenzen sind, die Schritten d), e), f) und g) folgend identifiziert und sortiert wurden,
i) Berechnen von Verhältnissen R, die eine molekulare Häufigkeit der onkogenen mRNAs relativ zu der mindestens einen anderen viralen mRNA des gleichen Genotyps oder der gleichen Spezies von Schritt f) definieren.

**7.** Verfahren zur Diagnose oder zum Prognoserisiko, HPV-induzierten Krebs zu entwickeln, umfassend:

(a) Bestimmen des Niveaus von mindestens einem ersten Marker, ausgewählt aus E6-mRNAs von Gruppe-Alpha-HPVs, E7-mRNAs von Gruppe-Alpha-HPVs oder beiden, in der Probe eines Patienten oder in der Probe einer Person, von denen vermutet wird, mit HPV infiziert zu sein, wobei das Niveau an HPV speziesselektiv ist,
(b) Vergleichen des in Schritt (a) bestimmten Niveaus mit einem Referenzwert von E6-mRNAs von Gruppe-Alpha-HPVs, E7-mRNAs von Gruppe-Alpha-HPVs oder beiden bei mit HPVs infizierten Personen mit geringem Risiko,

wobei ein erhöhtes Niveau, wie in Schritt a) bestimmt, im Vergleich zu dem Referenzniveau in Schritt b) ein höheres Risiko anzeigt, HPV-induzierten Krebs zu entwickeln,
wobei das Verfahren einen Amplifikationsschritt mit einer Gruppe-Alpha-HPV-spezifischen Zusammensetzung von Primern, wie in einem der Ansprüche 1-2 definiert, umfasst.

**8.** Verfahren nach einem der Ansprüche 6 und 7, wobei E6-oder E7-mRNAs von HPVs zur Gattung Alpha-HPVs gehören und wobei es eine Amplifikation und/oder Sequenzierung umfasst, die HPVs von mindestens den Gruppen a5, 6, 7 und 10 abdeckt.

**9.** Verfahren nach Anspruch 8, wobei die Amplifikation oder Bestimmung der Niveaus an E6- und L1-Biomarkern das Verwenden einer Zusammensetzung von Primern umfasst, umfassend für E6: $\alpha$5: sowohl SEQ ID NO: 44 als auch SEQ ID NO: 45 und alle drei SEQ ID NO: 46, SEQ ID NO: 47 und SEQ ID NO: 48 und; $\alpha$6: SEQ ID NO: 58 oder sowohl SEQ ID NO: 59 als auch SEQ ID NO: 60 und sowohl SEQ ID NO: 61 als auch SEQ ID NO: 62 und; $\alpha$7: alle drei SEQ ID NO: 73, SEQ ID NO: 75 und SEQ ID NO: 76 oder alle drei SEQ ID NO: 74, SEQ ID NO: 75 und SEQ ID NO: 76 und alle fünf SEQ ID NO: 77, SEQ ID NO: 78, SEQ ID NO: 79, SEQ ID NO: 80 und SEQ ID NO: 81 und; $\alpha$10: alle drei SEQ ID NO: 115, SEQ ID NO: 116, SEQ ID NO: 117 und alle vier SEQ ID NO: 118, SEQ ID NO: 119, SEQ ID NO: 120, SEQ ID NO: 121;
und umfassend für L1 α5: SEQ ID NO: 53 oder sowohl SEQ ID NO: 54 als auch SEQ ID NO: 55 und sowohl SEQ ID NO: 56 als auch SEQ ID NO: 57; und α6: SEQ ID NO: 71 und SEQ ID NO: 72; und α7: sowohl SEQ ID NO: 87 als auch SEQ ID NO: 88 und sowohl SEQ ID NO: 89 als auch SEQ ID NO: 90; und α10: sowohl SEQ ID NO: 128, SEQ ID NO: 129 als auch SEQ ID NO: 130 oder alle vier SEQ ID NO: 131, SEQ ID NO: 132, SEQ ID NO: 133, SEQ ID NO: 134.

**10.** Verfahren nach Anspruch 8, wobei die Amplifikation oder Bestimmung der Niveaus an E7- und L1-Biomarkern das Verwenden einer Zusammensetzung von Primern umfasst, umfassend für E7: α5: sowohl SEQ ID NO: 49, SEQ ID NO: 50 und sowohl SEQ ID NO: 51, SEQ ID NO: 52; und α6: SEQ ID NO: 63 oder SEQ ID NO: 64 oder SEQ ID NO: 65 SEQ ID NO: 66 oder sowohl SEQ ID NO: 67 als auch SEQ ID NO: 68 und sowohl SEQ ID NO: 69 als auch SEQ ID NO: 70; und α7: SEQ ID NO: 82 oder sowohl SEQ ID NO: 83, SEQ ID NO: 84 und sowohl SEQ ID NO: 85, SEQ ID NO: 86; und; α10: alle drei SEQ ID NO: 122, SEQ ID NO: 123 und SEQ ID NO: 124 und alle drei SEQ ID NO: 125, SEQ ID NO: 126 und SEQ ID NO: 127; und; a9: alle drei SEQ ID NO: 105, SEQ ID NO: 106 und SEQ ID NO: 107 und alle drei SEQ ID NO: 108, SEQ ID NO: 109 und 110; und α8: SEQ ID NO: 94; und sowohl SEQ ID NO: 95 als auch SEQ ID NO: 96; und α1: SEQ ID NO: 3 und SEQ ID NO: 4; und α3: sowohl SEQ ID NO: 28 als auch SEQ ID NO: 29 und alle drei SEQ ID NO: 30, SEQ ID NO: 31, SEQ ID NO: 32; und α2: alle drei SEQ ID NO: 12, SEQ ID NO: 13 und SEQ ID NO: 14 und alle drei SEQ ID NO: 15, SEQ ID NO: 16 und SEQ ID NO: 17;, und α4: SEQ ID NO: 38 und SEQ ID NO: 39; und α11 SEQ ID NO: 137 und SEQ ID NO: 138; und α13: SEQ ID NO: 143 und SEQ ID NO: 144; und α14: SEQ ID NO: 149 und SEQ ID NO: 150;
und umfassend für L1 α5: SEQ ID NO: 53 oder sowohl SEQ ID NO: 54 als auch SEQ ID NO: 55 und sowohl SEQ ID NO: 56 als auch SEQ ID NO: 57; und α6: SEQ ID NO: 71 und SEQ ID NO: 72; und α7: sowohl SEQ ID NO: 87 als auch SEQ ID NO: 88 und sowohl SEQ ID NO: 89 als auch SEQ ID NO: 90; und α10: sowohl SEQ ID NO: 128, SEQ ID NO: 129 als auch SEQ ID NO: 130 oder alle vier SEQ ID NO: 131, SEQ ID NO: 132, SEQ ID NO: 133, SEQ ID NO: 134; und α9: sowohl SEQ ID NO: 111 als auch SEQ ID NO: 112 und sowohl SEQ ID NO: 113 als auch SEQ ID NO: 114; und α8: SEQ ID NO: 97 und sowohl SEQ ID NO: 98 als auch SEQ ID NO: 99; und α1: SEQ ID NO: 5 und SEQ ID NO: 6; und α3 SEQ ID NO: 33 und sowohl SEQ ID NO: 34 als auch SEQ ID NO: 35; und α2 SEQ ID NO: 18 und sowohl SEQ ID NO: 19 als auch SEQ ID NO: 20; und α4: SEQ ID NO: 40 oder SEQ ID NO: 41 und SEQ ID NO: 42 oder SEQ ID NO: 43; und α11: SEQ ID NO: 139 und SEQ ID NO: 140; und α13 SEQ ID NO: 145 und SEQ ID NO: 146; und α14: SEQ ID NO: 151 und SEQ ID NO: 152.

**11.** Verfahren nach Anspruch 8, wobei die Amplifikation oder Bestimmung der Niveaus an E6- und L1-Biomarkern das Verwenden einer Zusammensetzung von Primern umfasst, umfassend für E6: α5: sowohl SEQ ID NO: 44 als auch SEQ ID NO: 45 und alle drei SEQ ID NO: 46, SEQ ID NO: 47 und SEQ ID NO: 48 und; α6: SEQ ID NO: 58 oder sowohl SEQ ID NO: 59 als auch SEQ ID NO: 60 und sowohl SEQ ID NO: 61 als auch SEQ ID NO: 62 und; α7: alle drei SEQ ID NO: 73, SEQ ID NO: 75 und SEQ ID NO: 76 oder alle drei SEQ ID NO: 74, SEQ ID NO: 75 und SEQ ID NO: 76 und alle fünf SEQ ID NO: 77, SEQ ID NO: 78, SEQ ID NO: 79, SEQ ID NO: 80 und SEQ ID NO: 81 und; α10: alle drei SEQ ID NO: 115, SEQ ID NO: 116, SEQ ID NO: 117 und alle vier SEQ ID NO: 118, SEQ ID NO: 119, SEQ ID NO: 120, SEQ ID NO: 121,
und umfassend für L1 α5: SEQ ID NO: 53 oder sowohl SEQ ID NO: 54 als auch SEQ ID NO: 55 und sowohl SEQ ID NO: 56 als auch SEQ ID NO: 57; und α6: SEQ ID NO: 71 und SEQ ID NO: 72; und α7: sowohl SEQ ID NO: 87 als auch SEQ ID NO: 88 und sowohl SEQ ID NO: 89 als auch SEQ ID NO: 90; und α10: sowohl SEQ ID NO: 128, SEQ ID NO: 129 als auch SEQ ID NO: 130 oder alle vier SEQ ID NO: 131, SEQ ID NO: 132, SEQ ID NO: 133, SEQ ID NO: 134.

**12.** Verfahren nach einem der Ansprüche 8 bis 11, wobei die Amplifikation oder Bestimmung der Niveaus an E6- und L1-Biomarkern das Verwenden einer Zusammensetzung von Primern umfasst, umfassend für E6: α5: sowohl SEQ ID NO: 44 als auch SEQ ID NO: 45 und alle drei SEQ ID NO: 46, SEQ ID NO: 47 und SEQ ID NO: 48 und; α6: SEQ ID NO: 58 oder sowohl SEQ ID NO: 59 als auch SEQ ID NO: 60 und sowohl SEQ ID NO: 61 als auch SEQ ID NO: 62 und; α7: alle drei SEQ ID NO: 73, SEQ ID NO: 75 und SEQ ID NO: 76 oder alle drei SEQ ID NO: 74, SEQ ID NO: 75 und SEQ ID NO: 76 und alle fünf SEQ ID NO: 77, SEQ ID NO: 78, SEQ ID NO: 79, SEQ ID NO: 80 und SEQ ID NO: 81 und; α10: alle drei SEQ ID NO: 115, SEQ ID NO: 116, SEQ ID NO: 117 und alle vier SEQ ID NO: 118, SEQ ID NO: 119, SEQ ID NO: 120, SEQ ID NO: 121; und α9: sowohl SEQ ID NO: 100 als auch SEQ ID NO: 101 und alle drei SEQ ID NO: 102, SEQ ID NO: 103 und SEQ ID NO: 104; und α8: SEQ ID NO: 91 und sowohl SEQ ID NO: 92 als auch SEQ ID NO: 93; und α1: SEQ ID NO: 1 und SEQ ID NO: 2; und α3: alle drei SEQ ID NO: 21, SEQ ID NO: 22 und SEQ ID NO: 23 und alle vier SEQ ID NO: 24, SEQ ID NO: 25, SEQ ID NO: 26 und SEQ ID NO: 27; und α2 und sowohl SEQ ID NO: 7 als auch SEQ ID NO: 8 und alle drei SEQ ID NO: 9, SEQ ID NO: 10 und SEQ ID NO: 11; und α4: SEQ ID NO: 36 und SEQ ID NO: 37; und α11: SEQ ID NO: 135 und SEQ ID NO: 136; und α13 SEQ ID NO: 141 und SEQ ID NO: 142; und α14: SEQ ID NO: 147 und SEQ ID NO: 148, und umfassend für L1, α5: SEQ ID NO: 53 oder sowohl SEQ ID NO: 54 als auch SEQ ID NO: 55 und sowohl SEQ ID NO: 56 als auch SEQ ID NO: 57; und α6: SEQ ID NO: 71 und SEQ ID NO: 72; und α7: sowohl SEQ ID NO: 87 als auch SEQ ID NO: 88 und sowohl SEQ ID NO: 89 als auch SEQ ID NO: 90; und α10: sowohl SEQ ID NO: 128, SEQ ID NO: 129 als auch SEQ ID NO: 130 oder alle vier SEQ ID NO: 131, SEQ ID NO: 132, SEQ ID NO: 133, SEQ ID NO: 134; und α9: sowohl SEQ ID NO: 111 als auch SEQ ID NO: 112 und sowohl SEQ ID NO: 113 als auch SEQ ID NO: 114; und α8: SEQ ID NO: 97 und sowohl SEQ ID NO: 98 als auch SEQ ID NO: 99; und α1: SEQ ID NO: 5 und SEQ ID NO: 6; und α3 SEQ ID NO: 33 und sowohl SEQ ID NO: 34 als auch SEQ ID NO: 35;und α2 SEQ ID NO: 18 und sowohl SEQ ID NO: 19 als auch SEQ ID NO: 20; und α4: SEQ ID NO: 40 oder SEQ ID NO: 41 und SEQ ID NO: 42 oder SEQ ID NO: 43; und α11: SEQ ID NO: 139 und SEQ ID NO: 140; und α13 SEQ ID NO: 145 und SEQ ID NO: 146; und α14: SEQ ID NO: 151 und SEQ ID NO: 152.

**13.** Zusammensetzung von Primern nach einem der Ansprüche 1, 2, 4, 5 und 9 bis 12.

**14.** Kit zur Diagnose oder zum Prognoserisiko, HPVinduzierten Krebs zu entwickeln, umfassend:

a) eine Zusammensetzung von Primern nach Anspruch 13,
b) Reagenzien, um Amplifikationsprodukte nachzuweisen oder zu sequenzieren.

**15.** Kit zur Diagnose oder zum Prognoserisiko, HPVinduzierten Krebs zu entwickeln, umfassend:

a) eine Zusammensetzung von Primern zum Nachweisen mindestens eines ersten Markers, ausgewählt aus E6-mRNAs von Gruppe-Alpha-HPVs, E7-mRNAs von Gruppe-Alpha-HPVs oder beiden,
b) eine Zusammensetzung von Primern zum Nachweisen mindestens eines zweiten Markers, ausgewählt aus L1-mRNAs von Gruppe-Alpha-HPVs, L2-mRNAs von Gruppe-Alpha-HPVs oder beiden, wobei die E6-, E7-, L1- und L2-mRNAs entsprechende intragenetische Sequenzen aufweisen,
c) und wahlweise Primer oder Sonden zum Nachweisen mindestens eines Wirtszellmarker, der Neoplasie oder Krebs anzeigt, wobei die Zusammensetzung der Primer wie in Anspruch 1 oder 2 definiert ist.

## Revendications

**1.** Procédé pour déterminer un risque pour un patient de développer un cancer induit par un virus oncogène, tel qu'un HPV de groupe alpha, comprenant:

a) l'enrichissement des ARN viraux dans un échantillon en utilisant une pré-amplification aléatoire ou consensus et/ou une réaction de transcriptase inverse spécifique, de préférence une pré-amplification consensus,
b) le séquençage de l'ADNc produit à l'étape a), et la génération de séquences de fragments dudit ADNc,
c) la détermination du nombre de séquences de fragments correspondant auxdits virus sur la base d'une discrimination d'espèces et déterminer les espèces à haut risque les plus répandues présentes dans l'échantillon par rapport aux autres espèces,
d) la détermination au sein desdites espèces à haut risque les plus répandues du nombre relatif de séquences de fragments correspondant à au moins un gène oncogène par rapport à au moins un gène non oncogène, de préférence des gènes oncogènes par rapport à des gènes non oncogènes,
e) le calcul des rapports au sein desdites espèces à haut risque de séquences de fragments correspondant à au moins un gène oncogène par rapport à un gène structurel ou régulateur inter-espèce correspondant, de préférence des gènes oncogènes par rapport à des gènes structurels ou régulateurs inter-espèce correspondants,
f) la détermination du risque de développer un cancer induit par un virus oncogène chez des patients pour lesquels ledit rapport tend vers l'infini,

dans lequel l'enrichissement des ARN viraux est effectué par une transcription inverse des ARN viraux, et une amplification de l'ADNc produit par PCR multiplex avec une composition d'amorces spécifique au HPV de groupe alpha comprenant au moins l'un des groupes suivants de paires d'amorces :

- groupe SD1-SA1 constitué des paires d'amorces de SEQ ID NO : 397-398 ; 521-522 ; 609-610 ; 695-696 ; 819-820 ; 865-866 ; 947-948 ; 1067-1068 ; 1119-1120 ; 1267-1268 ; 1325-1326 ; 1507-1508 ; 1597-1598 ; 1655-1656 ; 1755-1756 ; et/ou,

- groupe SD1-SA2 constitué des paires d'amorces de SEQ ID NO : 459-460 ; 633-634; 687-688; 1111-1112; 1235-1236; 1341-1342 ; 1503-1504 ; 1657-1658 ; 1797-1798 ; et/ou,
- groupe SD1-SA3 constitué des paires d'amorces de SEQ ID NO : 381-382 ; 541-542; 599-600; 903-904; 941-942; 1047-1048; 1135-1136; 1287-1288; 1459-1460; 1473-1474; 1621-1622; 1717-1718; 1745-1746 ; et/ou,
- groupe SD1-SA4 constitué des paires d'amorces de SEQ ID NO : 413-414 ; 551-552 ; 637-638 ; 713-714 ; 793-794 ; 857-858 ; 981-982 ; 1093-1094 ; 1179-1180 ; 1227-1228 ; 1319-1320 ; 1413-1414 ; 1509-1510 ; 1563-1564 ; 1709-1710 ; 1791-1792 ; et/ou,
- groupe SD1-SA5 constitué des paires d'amorces de SEQ ID NO : 453-454 ; 549-550 ; 613-614 ; 747-748 ; 761-762 ; 949-950 ; 1163-1164 ; 1249-1250 ; 1329-1330 ; 1453-1454 ; 1501-1502 ; et/ou,
- groupe SD1-SA6 constitué des paires d'amorces de SEQ ID NO : 431-432 ; 595-596 ; 719-720 ; 827-828 ; 1089-1090 ; 1137-1138 ; 1285-1286 ; 1353-1354 ; 1561-1562 ; 1719-1720 ; 1763-1764 ; et/ou,
- groupe SD1-SA7 constitué des paires d'amorces de SEQ ID NO : 919-920 ; 1449-1450 ; et/ou,
- groupe SD1-SA8 constitué des paires d'amorces de SEQ ID NO : 489-490 ; 963-964 ; 1519-1520 ; et/ou,
- groupe SD2-SA4 constitué des paires d'amorces de SEQ ID NO : 387-388 ; 473-474 ; 615-616 ; 745-746 ; 815-816 ; 849-850 ; 933-934 ; 1091-1092 ; 1177-1178 ; 1209-1210 ; 1367-1368 ; 1437-1438 ; 1521-1522 ; 1603-1604 ; 1651-1652 ; 1779-1780 ; et/ou,
- groupe SD2-SA5 constitué des paires d'amorces de SEQ ID NO : 455-456 ; 529-530 ; 629-630 ; 717-718 ; 777-778 ; 975-976 ; 1153-1154 ; 1273-1274 ; 1347-1348 ; 1451-1452 ; 1531-1532 ; et/ou,
- groupe SD2-SA6 constitué des paires d'amorces de SEQ ID NO : 399-400 ; 645-646 ; 727-728 ; 811-812 ; 1079-1080 ; 1127-1128 ; 1253-1254 ; 1369-1370 ; 1615-1616 ; 1659-1660 ; 1781-1782 ; et/ou,
- groupe SD2-SA7 constitué des paires d'amorces de SEQ ID NO : 531-532 ; 899-900 ; 943-944 ; 1411-1412 ; 1495-1496 ; et/ou,
- groupe SD2-SA9 constitué des paires d'amorces de SEQ ID NO : 437-438 ; 505-506 ; 607-608 ; 739-740 ; 785-786 ; 887-888 ; 979-980 ; 1063-1064 ; 1185-1186 ; 1233-1234 ; 1297-1298 ; 1423-1424 ; 1491-1492 ; 1607-1608 ; 1693-1694 ; 1775-1776 ; et/ou,
- groupe SD2-SA10 constitué des paires d'amorces de SEQ ID NO : 545-546 ; 831-832; 1149-1150; 1269-1270; 1427-1428; 1671-1672 ; et/ou,
- groupe SD3-SA4 constitué des paires d'amorces de SEQ ID NO : 379-380 ; 483-484 ; 611-612 ; 721-722 ; 833-834 ; 911-912 ; 937-938 ; 1053-1054 ; 1139-1140 ; 1251-1252 ; 1335-1336 ; 1435-1436 ; 1487-1488 ; 1591-1592 ; 1715-1716 ; 1785-1786 ; et/ou,
- groupe SD3-SA5 constitué des paires d'amorces de SEQ ID NO : 415-416 ; 493-494 ; 593-594 ; 733-734 ; 817-818 ; 993-994 ; 1145-1146 ; 1243-1244 ; 1337-1338 ; 1401-1402 ; 1483-1484 ; et/ou,
- groupe SD3-SA6 constitué des paires d'amorces de SEQ ID NO : 435-436 ; 655-656 ; 673-674 ; 813-814 ; 1045-1046 ; 1173-1174 ; 1241-1242 ; 1303-1304 ; 1557-1558 ; 1627-1628 ; 1647-1648 ; 1773-1774 ; et/ou,
- groupe SD3-SA7 constitué des paires d'amorces de SEQ ID NO : 855-856 ; 1387-1388 ; et/ou,
- groupe SD3-SA8 constitué des paires d'amorces de SEQ ID NO : 511-512 ; 957-958 ; 1529-1530 ; et/ou,
- groupe SD5-SA9 constitué des paires d'amorces de SEQ ID NO : 419-420 ; 527-528 ; 567-568 ; 587-588 ; 683-684 ; 775-776 ; 891-892 ; 999-1000 ; 1041-1042 ; 1113-1114 ; 1247-1248 ; 1371-1372 ; 1403-1404 ; 1511-1512 ; 1617-1618 ; 1677-1678 ; 1733-1734 ; et/ou,
- groupe SD5-SA10 constitué des paires d'amorces de SEQ ID NO : 495-496 ; 837-838; 1183-1184; 1279-1280; 1433-1434; 1723-1724.

**2.** Procédé selon la revendication 1, dans lequel la composition d'amorces spécifique au HPV de groupe alpha comprend les groupes suivants de paires d'amorces :

- groupe SD1-SA1 constitué des paires d'amorces de SEQ ID NO : 397-398 ; 521-522 ; 609-610 ; 695-696 ; 819-820 ; 865-866 ; 947-948 ; 1067-1068 ; 1119-1120 ; 1267-1268 ; 1325-1326 ; 1507-1508 ; 1597-1598 ; 1655-1656 ; 1755-1756.

**3.** Procédé selon l'une quelconque des revendications 1 à 2, dans lequel la composition d'amorces spécifique au HPV de groupe alpha englobe des jonctions d'épissage, des régions génomiques et non épissées, et des régions de transcrit de fusion humaine de chaque trancrit de HPV.

**4.** Procédé selon l'une quelconque des revendications 1 à 3, dans lequel la composition d'amorces spécifique au HPV groupe alpha comprend au moins l'un des, de préférence tous les, groupes de paires d'amorces suivants :

- les amorces spécifiques au HPV16 comprenant ou consistant en les amorces des SEQ ID NO : 219-258 pour des transcrits génomiques et non épissés de HPV16, SEQ ID NO : 259-352 pour des transcrits épissés de HPV16 et SEQ ID NO : 353-376 pour des transcrits de fusion humaine de HPV16 (y compris les paires d'amorces de SEQ ID NO : 219-220 ; 221-222 ; 223-224 ; 225-226 ; 227-228 ; 229-230 ; 231-232 ; 233-234 ; 235-236 ; 237-238 ; 239-240 ; 241-242 ; 243-244 ; 245-246 ; 247-248 ; 249-250 ; 251-252 ; 253-254 ; 255-256 ; 257-258 ; 259-260 ; 261-262 ; 263-264 ; 265-266 ; 267-268 ; 269-270 ; 271-272 ; 273-274 ; 275-276 ; 277-278 ; 279-280 ; 281-282 ; 283-284 ; 285-286 ; 287-288 ; 289-290 ; 291-292 ; 293-294 ; 295-296 ; 297-298 ; 299-300 ; 301-302 ; 303-304 ; 305-306 ; 307-308 ; 309-310 ; 311-312 ; 313-314 ; 315-316 ; 317-318 ; 319-320 ; 321-322 ; 323-324 ; 325-326 ; 327-328 ; 329-330 ; 331-332 ; 333-334 ; 335-336 ; 337-338 ; 339-340 ; 341-342 ; 343-344 ; 345-346 ; 347-348 ; 349-350 ; 351-352 ; 353-354 ; 355-356 ; 357-358 ; 359-360 ; 361-362 ; 363-364 ; 365-366 ; 367-368 ; 369-370; 371-372; 373-374; 375-376) ou 377-470 (y compris les paires d'amorces de SEQ ID NO. 377-378 ; 379-380 ; 381-382 ; 383-384 ; 385- 386 ; 387-388 ; 389-390 ; 391-392 ; 393-394 ; 395-396 ; 397-398 ; 399-400 ; 401-402 ; 403-404 ; 405-406 ; 407-408 ; 409-410 ; 411-412 ; 413-414 ; 415-416 ; 417-418 ; 419-420 ; 421-422 ; 423-424 ; 425-426 ; 427-428 ; 429-430 ; 431-432 ; 433-434 ; 435-436 ; 437-438 ; 439-440 ; 441-442 ; 443-444 ; 445-446 ; 447-448 ; 449-450 ; 451-452 ; 453-454 ; 455-456 ; 457-458 ; 459-460 ; 461-462 ; 463-464 ; 465-466 ; 467-468 and ; 469-470) ; et/ou,

- les amorces spécifiques au HPV18 comprenant ou consistant en les amorces de SEQ ID NO : 471-574 (y compris les paires d'amorces de SEQ ID NO : 471-472 ; 473-474 ; 475-476 ; 477-478 ; 479-480 ; 481-482 ; 483-484 ; 485-486 ; 487-488 ; 489-490 ; 491-492 ; 493-494 ; 495-496 ; 497-498 ; 499-500 ; 501-502 ; 503-504 ; 505-506 ; 507-508 ; 509-510 ; 511-512 ; 513-514 ; 515-516 ; 517-518 ; 519-520 ; 521-522 ; 523-524 ; 525-526 ; 527-528 ; 529-530 ; 531-532 ; 533-534 ; 535-536 ; 537-538 ; 539-540 ; 541-542 ; 543-544 ; 545-546 ; 547-548 ; 549-550 ; 551-552 ; 553-554 ; 555-556 ; 557-558 ; 559-560 ; 561-562 ; 563-564 ; 565-566 ; 567-568 ; 569-570 ; 571-572 ; 573-574) ; et/ou,

- les amorces spécifiques au HPV31 comprenant ou consistant en les amorces de SEQ ID NO : 575-668 (y compris les paires d'amorces de SEQ ID NO : 575-576 ; 577-578 ; 579-580 ; 581-582 ; 583-584 ; 585-586 ; 587-588; 589-590; 591-592; 593-594; 595-596; 597-598; 599-600; 601-602; 603-604 ; 605-606 ; 607-608 ; 609-610 ; 611-612 ; 613-614 ; 615-616 ; 617-618 ; 619-620 ; 621-622 ; 623-624 ; 625-626 ; 627-628 ; 629-630 ; 631-632 ; 633-634 ; 635-636 ; 637-638 ; 639-640 ; 641-642 ; 643-644 ; 645-646 ; 647-648 ; 649-650 ; 651-652 ; 653-654 ; 655-656 ; 657-658 ; 659-660 ; 661-662 ; 663-664 ; 665-666 ; 667-668) ; et/ou,

- les amorces spécifiques au HPV33 comprenant ou consistant en SEQ ID NO : 669-756 (y compris les paires d'amorces de SEQ ID NO : 669-670 ; 671-672 ; 673-674 ; 675-676 ; 677-678 ; 679-680 ; 681-682 ; 683-684 ; 685-686 ; 687-688 ; 689-690 ; 691-692 ; 693-694 ; 695-696 ; 697-698 ; 699-700 ; 701-702 ; 703- 704; 705-706; 707-708; 709-710; 711-712; 713-714; 715-716; 717-718; 719-720; 721-722; 723-724; 725-726; 727-728; 729-730; 731-732; 733-734 ; 735-736 ; 737-738 ; 739-740 ; 741-742 ; 743-744 ; 745-746 ; 747-748 ; 749-750 ; 751-752 ; 753-754 ; 755-756) ; et/ou,

- les amorces spécifiques au HPV35 comprenant ou consistant en les amorces de SEQ ID NO : 757-848 (y compris les paires d'amorces de SEQ ID NO : 757-758 ; 759-760 ; 761-762 ; 763-764 ; 765-766 ; 767-768 ; 769-770; 771-772; 773-774; 775-776; 777-778; 779-780; 781-782; 783-784; 785-786 ; 787-788 ; 789-790 ; 791-792 ; 793-794 ; 795-796 ; 797-798 ; 799-800 ; 801-802 ; 803-804 ; 805-806 ; 807-808 ; 809-810 ; 811-812 ; 813-814 ; 815-816 ; 817-818 ; 819-820 ; 821-822 ; 823-824 ; 825-826 ; 827-828 ; 829-830 ; 831-832 ; 833-834; 835-836; 837-838; 839-840; 841-842; 843-844; 845-846; 847-848) ; et/ou,

- les amorces spécifiques au HPV39 comprenant ou consistant en les amorces de SEQ ID NO : 849-928 (y compris les paires d'amorces de SEQ ID NO : 849-850 ; 851-852 ; 853-854 ; 855-856 ; 857-858 ; 859-860 ; 861-862; 863-864; 865-866; 867-868; 869-870; 871-872; 873-874; 875-876; 877-878 ; 879-880 ; 881-882 ; 883-884 ; 885-886 ; 887-888 ; 889-890 ; 891-892 ; 893-894 ; 895-896 ; 897-898 ; 899-900 ; 901-902 ; 903-904 ; 905-906 ; 907-908 ; 909-910 ; 911-912 ; 913-914 ; 915-916 ; 917-918 ; 919-920 ; 921-922 ; 923-924 ; 925-926 ; 927-928) ; et/ou,

- les amorces spécifiques au HPV45 comprenant ou consistant en les amorces de SEQ ID NO : 929-1020 (y compris les paires d'amorces de SEQ ID NO : 929-930 ; 931-932 ; 933-934 ; 935-936 ; 937-938 ; 939-940 ; 941-942; 943-944; 945-946; 947-948; 949-950; 951-952; 953-954; 955-956; 957-958 ; 959-960 ; 961-962 ; 963-964 ; 965-966 ; 967-968 ; 969-970 ; 971-972 ; 973-974 ; 975-976 ; 977-978 ; 979-980 ; 981-982 ; 983-984 ; 985-986 ; 987-988 ; 989-990 ; 991-992 ; 993-994 ; 995-996 ; 997-998 ; 999-1000 ; 1001-1002 ; 1003-1004; 1005-1006; 1007-1008; 1009-1010; 1011-1012; 1013-1014; 1015-1016 ; 1017-1018 ; 1019-1020) ; et/ou,

- les amorces spécifiques au HPV51 comprenant ou consistant en les amorces de SEQ ID NO : 1021-1102 (y compris les paires d'amorces de SEQ ID NO : 1021-1022 ; 1023-1024 ; 1025-1026 ; 1027-1028 ; 1029-1030 ; 1031-1032; 1033-1034; 1035-1036; 1037-1038; 1039-1040; 1041-1042; 1043-1044 ; 1045-1046 ; 1047-1048 ; 1049-1050 ; 1051-1052 ; 1053-1054 ; 1055-1056; 1057-1058; 1059-1060; 1061-1062; 1063-1064; 1065-1066; 1067-1068 ; 1069-1070 ; 1071-1072; 1073-1074 ; 1075-1076 ; 1077-1078 ; 1079-1080; 1081-1082; 1083-1084; 1085-1086; 1087-1088; 1089-1090; 1091-1092; 1093-1094; 1095-1096; 1097-1098; 1099-1100; 1101-1102) ;

et/ou,

- les amorces spécifiques au HPV52 comprenant ou consistant en les amorces de SEQ ID NO : 1103-1200 (y compris les paires d'amorces de SEQ ID NO : 1103-1104 ; 1105-1106; 1107-1108; 1109-1110; 1111-1112; 1113-1114; 1115-1116; 1117-1118; 1119-1120; 1121-1122; 1123-1124 ; 1125-1126; 1127-1128; 1129-1130; 1131-1132; 1133-1134; 1135-1136 ; 1137-1138; 1139-1140; 1141-1142; 1143-1144; 1145-1146; 1147-1148; 1149-1150; 1151-1152; 1153-1154; 1155-1156; 1157-1158; 1159-1160; 1161-1162; 1163-1164; 1165-1166; 1167-1168; 1169-1170; 1171-1172; 1173-1174; 1175-1176; 1177-1178; 1179-1180; 1181-1182; 1183-1184; 1185-1186; 1187-1188; 1189-1190; 1191-1192; 1193-1194; 1195-1196; 1197-1198 ; 1199-1200) ; et/ou,
- les amorces spécifiques au HPV56 comprenant ou consistant en les amorces de SEQ ID NO : 1201-1296 (y compris les paires d'amorces de SEQ ID NO : 1201-1202 ; 1203-1204 ; 1205-1206 ; 1207-1208 ; 1209-1210 ; 1211-1212; 1213-1214; 1215-1216; 1217-1218; 1219-1220; 1221-1222; 1223-1224 ; 1225-1226 ; 1227-1228 ; 1229-1230 ; 1231-1232 ; 1233-1234 ; 1235-1236 ; 1237-1238 ; 1239-1240 ; 1241-1242; 1243-1244 ; 1245-1246 ; 1247-1248; 1249-1250; 1251-1252; 1253-1254; 1255-1256; 1257-1258; 1259-1260; 1261-1262; 1263-1264; 1265-1266; 1267-1268; 1269-1270; 1271-1272 ; 1273-1274 ; 1275-1276 ; 1277-1278 ; 1279-1280 ; 1281-1282 ; 1283-1284 ; 1285-1286 ; 1287-1288 ; 1289-1290 ; 1291-1292 ; 1293-1294 ; 1295-1296) ; et/ou,
- les amorces spécifiques au HPV58 comprenant ou consistant en les amorces de SEQ ID NO : 1297-1382 (y compris les paires d'amorces de SEQ ID NO : 1297-1298 ; 1299-1300; 1301-1302; 1303-1304; 1305-1306; 1307-1308 ; 1309-1310 ; 1311-1312 ; 1313-1314 ; 1315-1316 ; 1317-1318 ; 1319-1320 ; 1321-1322 ; 1323-1324 ; 1325-1326 ; 1327-1328 ; 1329-1330 ; 1331-1332 ; 1333-1334 ; 1335-1336 ; 1337-1338 ; 1339-1340 ; 1341-1342; 1343-1344 ; 1345-1346 ; 1347-1348 ; 1349-1350 ; 1351-1352 ; 1353-1354 ; 1355-1356; 1357-1358; 1359-1360; 1361-1362; 1363-1364; 1365-1366; 1367-1368 ; 1369-1370 ; 1371-1372; 1373-1374 ; 1375-1376 ; 1377-1378 ; 1379-1380 ; 1381-1382) ; et/ou,
- les amorces spécifiques au HPV59 comprenant ou consistant en les amorces de SEQ ID NO : 1383-1470 (y compris les paires d'amorces de SEQ ID NO. : 1383-1384 ; 1385-1386 ; 1387-1388 ; 1389-1390 ; 1391-1392 ; 1393-1394; 1395-1396; 1397-1398; 1399-1400; 1401-1402; 1403-1404; 1405-1406; 1407-1408; 1409-1410; 1411-1412; 1413-1414; 1415-1416; 1417-1418 ; 1419-1420 ; 1421-1422 ; 1423-1424 ; 1425-1426 ; 1427-1428 ; 1429-1430; 1431-1432; 1433-1434; 1435-1436; 1437-1438; 1439-1440; 1441-1442; 1443-1444; 1445-1446; 1447-1448; 1449-1450; 1451-1452 ; 1453-1454; 1455-1456; 1457-1458; 1459-1460; 1461-1462; 1463-1464; 1465-1466 ; 1467-1468 ; 1469-1470) ; et/ou,
- les amorces spécifiques au HPV66 comprenant ou consistant en les amorces de SEQ ID NO : 1471-1560 (y compris les paires d'amorces de SEQ ID NO : 1471-1472 ; 1473-1474; 1475-1476; 1477-1478; 1479- 1480 ; 1481-1482; 1483-1484; 1485-1486; 1487-1488; 1489-1490; 1491-1492; 1493-1494; 1495-1496; 1497-1498; 1499-1500; 1501-1502; 1503-1504; 1505-1506; 1507-1508; 1509-1510; 1511-1512; 1513- 1514; 1515-1516; 1517-1518 ; 1519-1520 ; 1521-1522 ; 1523-1524 ; 1525-1526 ; 1527-1528 ; 1529-1530; 1531-1532; 1533-1534; 1535-1536; 1537-1538; 1539-1540; 1541-1542; 1543-1544; 1545-1546; 1547- 1548 ; 1549-1550 ; 1551-1552 ; 1553-1554 ; 1555-1556 ; 1557-1558 ; 1559-1560 ; et/ou,
- les amorces spécifiques au HPV68 comprenant ou consistant en les amorces de SEQ ID NO : 1561-1642 (y compris les paires d'amorces de SEQ ID NO : 1561-1562 ; 1563-1564; 1565-1566; 1567-1568 ; 1569-1570 ; 1571-1572 ; 1573-1574 ; 1575-1576 ; 1577-1578 ; 1579-1580 ; 1581-1582 ; 1583-1584 ; 1585-1586 ; 1587-1588 ; 1589-1590 ; 1591-1592 ; 1593-1594 ; 1595-1596 ; 1597-1598 ; 1599-1600 ; 1601-1602 ; 1603-1604 ; 1605-1606 ; 1607-1608 ; 1609-1610 ; 1611-1612 ; 1613-1614 ; 1615-1616 ; 1617-1618 ; 1619-1620 ; 1621-1622 ; 1623-1624 ; 1625-1626 ; 1627-1628 ; 1629-1630 ; 1631-1632; 1633-1634 ; 1635-1636 ; 1637-1638 ; 1639-1640 ; 1641-1642); et/ou,
- les amorces spécifiques au HPV73 comprenant ou consistant en les amorces de SEQ ID NO : 1643-1732 (y compris les paires d'amorces de SEQ ID NO : 1643-1644 ; 1645-1646 ; 1647-1648 ; 1649-1650 ; 1651-1652 ; 1653-1654; 1655-1656; 1657-1658; 1659-1660; 1661-1662; 1663-1664; 1665-1666 ; 1667-1668 ; 1669-1670 ; 1671-1672 ; 1673-1674 ; 1675-1676 ; 1677-1678; 1679-1680; 1681-1682; 1683-1684; 1685-1686; 1687-1688; 1689-1690; 1691-1692; 1693-1694; 1695-1696; 1697-1698; 1699-1700; 1701-1702 ; 1703-1704 ; 1705-1706 ; 1707-1708 ; 1709-1710 ; 1711-1712; 1713-1714; 1715-1716; 1717-1718; 1719-1720; 1721-1722; 1723-1724; 1725-1726 ; 1727-1728 ; 1729-1730 ; 1731-1732) ; et/ou,
- les amorces spécifiques au HPV82 comprenant ou consistant en les amorces de SEQ ID NO : 1733-1816 (y compris les paires d'amorces de SEQ ID NO : 1733-1734 ; 1735-1736 ; 1737-1738 ; 1739-1740 ; 1741-1742 ; 1743-1744 ; 1745-1746 ; 1747-1748 ; 1749-1750 ; 1751-1752 ; 1753-1754 ; 1755-1756 ; 1757-1758 ; 1759-1760 ; 1761-1762 ; 1763-1764 ; 1765-1766 ; 1767-1768 ; 1769-1770 ; 1771-1772 ; 1773-1774 ; 1775-1776 ; 1777-1778 ; 1779-1780 ; 1781-1782 ; 1783-1784 ; 1785-1786 ; 1787-1788 ; 1789-1790 ; 1791-1792 ; 1793-1794 ; 1795-1796 ; 1797-1798 ; 1799-1800 ; 1801-1802 ; 1803-1804; 1805-1806; 1807-1808; 1809-1810; 1811-1812; 1813-1814; 1815-1816).

**5.** Procédé selon la revendication 1 ou 2, dans lequel la composition d'amorces spécifique au HPV groupe alpha comprend l'un des groupes suivants de paires d'amorces :

- le groupe de paires d'amorces de SEQ ID NO : 397-398 ; 521-522 ; 609-610 ; 695-696 ; 819-820 ; 865-866 ; 947-948 ; 1067-1068 ; 1119-1120 ; 1267-1268 ; 1325-1326 ; 1507-1508 ; 1597-1598 ; 1655-1656 ; 1755-1756 ; 459-460 ; 633-634 ; 687-688 ; 1111-1112 ; 1235-1236 ; 1341-1342 ; 1503-1504 ; 1657-1658 ; 1797-1798 ; 381-382 ; 541-542 ; 599-600 ; 903-904 ; 941-942 ; 1047-1048; 1135-1136; 1287-1288; 1459-1460; 1473-1474; 1621-1622; 1717-1718 ; 1745-1746 ; 413-414 ; 551-552 ; 637-638 ; 713-714 ; 793-794 ; 857-858 ; 981-982 ; 1093-1094 ; 1179-1180 ; 1227-1228 ; 1319-1320 ; 1413-1414; 1509-1510 ; 1563-1564 ; 1709-1710 ; 1791-1792 ; 453-454 ; 549-550 ; 613-614 ; 747-748 ; 761-762 ; 949-950 ; 1163-1164 ; 1249-1250 ; 1329-1330 ; 1453-1454 ; 1501-1502; 431-432; 595-596; 719-720; 827-828; 1089-1090; 1137-1138; 1285-1286 ; 1353-1354 ; 1561-1562 ; 1719-1720 ; 1763-1764 ; 919-920 ; 1449-1450 ; 489-490 ; 963-964 ; 1519-1520 ; 387-388 ; 473-474 ; 615-616 ; 745-746 ; 815-816 ; 849-850 ; 933-934 ; 1091-1092 ; 1177-1178 ; 1209-1210 ; 1367-1368 ; 1437-1438; 1521-1522; 1603-1604; 1651-1652; 1779-1780; 455-456; 529-530; 629-630; 717-718; 777-778; 975-976; 1153-1154; 1273-1274; 1347-1348 ; 1451-1452 ; 1531-1532 ; 399-400 ; 645-646 ; 727-728 ; 811-812 ; 1079-1080; 1127-1128; 1253-1254; 1369-1370; 1615-1616; 1659-1660; 1781-1782 ; 531-532 ; 899-900 ; 943-944 ; 1411-1412 ; 1495-1496 ; 437-438 ; 505-506 ; 607-608 ; 739-740 ; 785-786 ; 887-888 ; 979-980 ; 1063-1064 ; 1185-1186 ; 1233-1234 ; 1297-1298 ; 1423-1424 ; 1491-1492 ; 1607-1608 ; 1693-1694 ; 1775-1776; 545-546; 831-832; 1149-1150; 1269-1270; 1427-1428 ; 1671-1672 ; 379-380 ; 483-484 ; 611-612 ; 721-722 ; 833-834 ; 911-912 ; 937-938 ; 1053-1054; 1139-1140; 1251-1252; 1335-1336; 1435-1436; 1487-1488; 1591-1592; 1715-1716; 1785-1786; 415-416; 493-494; 593-594; 733-734; 817-818 ; 993-994; 1145-1146; 1243-1244 ; 1337-1338; 1401-1402; 1483-1484 ; 435-436 ; 655-656 ; 673-674 ; 813-814 ; 1045-1046 ; 1173-1174 ; 1241 - 1242 ; 1303-1304 ; 1557-1558 ; 1627-1628 ; 1647-1648 ; 1773-1774 ; 855-856 ; 1387-1388 ; 511-512 ; 957-958 ; 1529-1530 ; 477-478 ; 419-420 ; 527-528 ; 567-568 ; 587-588 ; 683-684 ; 775-776 ; 891-892 ; 999-1000 ; 1041-1042 ; 1113-1114 ; 1247-1248 ; 1371-1372 ; 1403-1404 ; 1511-1512 ; 1617-1618 ; 1677-1678 ; 1733-1734 ; 495-496 ; 837-838 ; 1183-1184 ; 1279-1280 ; 1433-1434 ; 1723-1724 ; 1011-1012 ; 557-558 ; ou,
- le groupe de paires d'amorces de SEQ ID NO : 229-230 ; 233-234 ; 235-236 ; 245-246 ; 247-248 ; 249-250 ; 251-252 ; 255-256 ; 257-258 ; 265-266 ; 273-274 ; 275-276 ; 277-278 ; 279-280 ; 281-282 ; 289-290 ; 291-292 ; 295-296 ; 297-298 ; 299-300 ; 301-302 ; 303-304 ; 305-306 ; 307-308 ; 309-310 ; 311-312 ; 319-320 ; 321-322 ; 323-324 ; 325-326 ; 327-328 ; 329-330 ; 331-332 ; 333-334 ; 335-336 ; 337-338 ; 341-342 ; 343-344 ; 345-346 ; 347-348 ; 349-350 ; 351-352 ; 377-378 ; 379-380 ; 381-382 ; 383-384 ; 385-386 ; 387-388 ; 389-390 ; 391-392 ; 393-394 ; 395-396 ; 397-398 ; 399-400 ; 401-402 ; 403-404 ; 405-406 ; 407-408 ; 409-410 ; 411-412 ; 413-414 ; 415-416 ; 417-418 ; 419-420 ; 421-422 ; 423-424 ; 425-426 ; 427-428 ; 429-430 ; 431-432 ; 433-434 ; 435-436 ; 437-438 ; 439-440 ; 441-442 ; 443-444 ; 445-446 ; 447-448 ; 449-450 ; 451-452 ; 453-454 ; 455-456 ; 457-458 ; 459-460 ; 461-462 ; 463-464 ; 465-466 ; 467-468 ; 469-470 ; 471-472 ; 473-474 ; 475-476 ; 477-478 ; 479-480 ; 481-482 ; 483-484 ; 485-486 ; 487-488 ; 489-490 ; 491-492 ; 493-494 ; 495-496 ; 497-498 ; 499-500 ; 501-502 ; 503-504 ; 505-506 ; 507-508 ; 509-510 ; 511-512 ; 513-514 ; 515-516 ; 517-518 ; 519-520 ; 521-522 ; 523-524 ; 525-526 ; 527-528 ; 529-530 ; 531-532 ; 533-534 ; 535-536 ; 537-538 ; 539-540 ; 541-542 ; 543-544 ; 545-546 ; 547-548 ; 549-550 ; 551-552 ; 553-554 ; 555-556 ; 557-558 ; 559-560 ; 561-562 ; 563-564 ; 565-566 ; 567-568 ; 569-570 ; 571-572 ; 573-574 ; 575-576 ; 577-578 ; 579-580 ; 581-582 ; 583-584 ; 585-586 ; 587-588 ; 589-590 ; 591-592 ; 593-594 ; 595-596 ; 597-598 ; 599-600 ; 601-602 ; 603-604 ; 605-606 ; 607-608 ; 609-610 ; 611-612 ; 613-614 ; 615-616 ; 617-618 ; 619-620 ; 621-622 ; 623-624 ; 625-626 ; 627-628 ; 629-630 ; 631-632 ; 633-634 ; 635-636 ; 637-638 ; 639-640 ; 641-642 ; 643-644 ; 645-646 ; 647-648 ; 649-650 ; 651-652 ; 653-654 ; 655-656 ; 657-658 ; 659-660 ; 661-662 ; 663-664 ; 665-666 ; 667-668 ; 669-670 ; 671-672 ; 673-674 ; 675-676 ; 677-678 ; 679-680 ; 681-682 ; 683-684 ; 685-686 ; 687-688 ; 689-690 ; 691-692 ; 693-694 ; 695-696 ; 697-698 ; 699-700 ; 701-702 ; 703-704 ; 705-706 ; 707-708 ; 709-710 ; 711-712 ; 713-714 ; 715-716 ; 717-718 ; 719-720 ; 721-722 ; 723-724 ; 725-726 ; 727-728 ; 729-730 ; 731-732 ; 733-734 ; 735-736 ; 737-738 ; 739-740 ; 741-742 ; 743-744 ; 745-746 ; 747-748 ; 749-750 ; 751-752 ; 753-754 ; 755-756 ; 757-758 ; 759-760 ; 761-762 ; 763-764 ; 765-766 ; 767-768 ; 769-770 ; 771-772 ; 773-774 ; 775-776 ; 777-778 ; 779-780 ; 781-782 ; 783-784 ; 785-786 ; 787-788 ; 789-790 ; 791-792 ; 793-794 ; 795-796 ; 797-798 ; 799-800 ; 801-802 ; 803-804 ; 805-806 ; 807-808 ; 809-810 ; 811-812 ; 813-814 ; 815-816 ; 817-818 ; 819-820 ; 821-822 ; 823-824 ; 825-826 ; 827-828 ; 829-830 ; 831-832 ; 833-834 ; 835-836 ; 837-838 ; 839-840 ; 841-842 ; 843-844 ; 845-846 ; 847-848 ; 849-850 ; 851-852 ; 853-854 ; 855-856 ; 857-858 ; 859-860 ; 861-862 ; 863-864 ; 865-866 ; 867-868 ; 869-870 ; 871-872 ; 873-874 ; 875-876 ; 877-878 ; 879-880 ; 881-882 ; 883-884 ; 885-886 ; 887-888 ; 889-890 ; 891-892 ; 893-894 ; 895-896 ; 897-898 ; 899-900 ; 901-902 ; 903-904 ; 905-906 ; 907-908 ; 909-910 ; 911-912 ; 913-914 ; 915-916 ; 917-918 ; 919-920 ; 921-922 ; 923-924 ; 925-926 ; 927-928 ; 929-930 ; 931-932 ; 933-934 ; 935-936 ; 937-938 ; 939-940 ; 941-942 ; 943-944 ; 945-946 ; 947-948 ; 949-950 ; 951-952 ; 953-954 ; 955-956 ; 957-958 ; 959-960 ; 961-962 ; 963-964 ;

965-966 ; 967-968 ; 969-970 ; 971-972 ; 973-974 ; 975-976 ; 977-978 ; 979-980 ; 981-982 ; 983-984 ; 985-986 ; 987-988 ; 989-990 ; 991-992 ; 993-994 ; 995-996 ; 997-998 ; 999-1000 ; 1001-1002 ; 1003-1004 ; 1005-1006 ; 1007-1008 ; 1009-1010 ; 1011-1012 ; 1013-1014 ; 1015-1016 ; 1017-1018 ; 1019-1020 ; 1021-1022; 1023-1024; 1025-1026; 1027-1028; 1029-1030; 1031-1032; 1033-1034; 1035-1036; 1037-1038; 1039-1040; 1041-1042; 1043-1044; 1045-1046; 1047-1048; 1049-1050; 1051-1052; 1053-1054; 1055-1056; 1057-1058; 1059-1060; 1061-1062; 1063-1064; 1065-1066; 1067-1068; 1069-1070; 1071-1072 ; 1073-1074; 1075-1076; 1077-1078; 1079-1080; 1081-1082; 1083-1084; 1085-1086; 1087-1088; 1089-1090; 1091-1092; 1093-1094; 1095-1096; 1097-1098; 1099-1100; 1101-1102; 1103-1104; 1105-1106; 1107-1108; 1109-1110; 1111-1112; 1113-1114; 1115-1116; 1117-1118; 1119-1120; 1121-1122; 1123-1124; 1125-1126; 1127-1128; 1129-1130; 1131-1132 ; 1133-1134; 1135-1136; 1137-1138; 1139-1140; 1141-1142; 1143-1144 ; 1145-1146; 1147-1148; 1149-1150; 1151-1152; 1153-1154; 1155-1156 ; 1157-1158; 1159-1160; 1161-1162; 1163-1164; 1165-1166; 1167-1168 ; 1169-1170; 1171-1172; 1173-1174; 1175-1176; 1177-1178; 1179-1180 ; 1181-1182; 1183-1184; 1185-1186; 1187-1188; 1189-1190; 1191-1192 ; 1193-1194; 1195-1196; 1197-1198; 1199-1200; 1201-1202; 1203-1204 ; 1205-1206; 1207-1208; 1209-1210; 1211-1212; 1213-1214; 1215-1216 ; 1217-1218; 1219-1220; 1221-1222; 1223-1224; 1225-1226; 1227-1228 ; 1229-1230; 1231-1232; 1233-1234; 1235-1236; 1237-1238; 1239-1240 ; 1241-1242; 1243-1244; 1245-1246; 1247-1248; 1249-1250; 1251-1252 ; 1253-1254; 1255-1256; 1257-1258; 1259-1260; 1261-1262; 1263-1264 ; 1265-1266; 1267-1268; 1269-1270; 1271-1272; 1273-1274; 1275-1276 ; 1277-1278 ; 1279-1280 ; 1281-1282 ; 1283-1284; 1285-1286 ; 1287-1288 ; 1289-1290; 1291-1292; 1293-1294; 1295-1296; 1297-1298; 1299-1300 ; 1301-1302; 1303-1304; 1305-1306; 1307-1308; 1309-1310; 1311-1312 ; 1313-1314; 1315-1316; 1317-1318; 1319-1320; 1321-1322; 1323-1324 ; 1325-1326; 1327-1328; 1329-1330; 1331-1332; 1333-1334; 1335-1336 ; 1337-1338; 1339-1340; 1341-1342; 1343-1344; 1345-1346; 1347-1348 ; 1349-1350; 1351-1352; 1353-1354; 1355-1356; 1357-1358; 1359-1360 ; 1361-1362; 1363-1364; 1365-1366; 1367-1368; 1369-1370; 1371-1372 ; 1373-1374; 1375-1376; 1377-1378; 1379-1380; 1381-1382; 1383-1384 ; 1385-1386; 1387-1388; 1389-1390; 1391-1392; 1393-1394; 1395-1396 ; 1397-1398; 1399-1400; 1401-1402; 1403-1404; 1405-1406; 1407-1408 ; 1409-1410; 1411-1412; 1413-1414; 1415-1416; 1417-1418; 1419-1420 ; 1421-1422; 1423-1424; 1425-1426; 1427-1428; 1429-1430; 1431-1432 ; 1433-1434; 1435-1436; 1437-1438; 1439-1440; 1441-1442; 1443-1444 ; 1445-1446; 1447-1448; 1449-1450; 1451-1452; 1453-1454; 1455-1456 ; 1457-1458; 1459-1460; 1461-1462; 1463-1464; 1465-1466; 1467-1468 ; 1469-1470; 1471-1472; 1473-1474; 1475-1476; 1477-1478; 1479-1480 ; 1481-1482; 1483-1484; 1485-1486; 1487-1488; 1489-1490; 1491-1492 ; 1493-1494; 1495-1496; 1497-1498; 1499-1500; 1501-1502; 1503-1504 ; 1505-1506; 1507-1508; 1509-1510; 1511-1512; 1513-1514; 1515-1516 ; 1517-1518; 1519-1520; 1521-1522; 1523-1524; 1525-1526; 1527-1528 ; 1529-1530; 1531-1532; 1533-1534; 1535-1536; 1537-1538; 1539-1540 ; 1541-1542; 1543-1544; 1545-1546; 1547-1548; 1549-1550; 1551-1552 ; 1553-1554; 1555-1556; 1557-1558; 1559-1560; 1561-1562; 1563-1564 ; 1565-1566; 1567-1568; 1569-1570; 1571-1572; 1573-1574; 1575-1576 ; 1577-1578; 1579-1580; 1581-1582; 1583-1584; 1585-1586; 1587-1588 ; 1589-1590; 1591-1592; 1593-1594; 1595-1596; 1597-1598; 1599-1600 ; 1601-1602; 1603-1604; 1605-1606; 1607-1608; 1609-1610; 1611-1612 ; 1613-1614; 1615-1616; 1617-1618; 1619-1620; 1621-1622; 1623-1624 ; 1625-1626; 1627-1628; 1629-1630; 1631-1632; 1633-1634; 1635-1636 ; 1637-1638; 1639-1640; 1641-1642; 1643-1644; 1645-1646; 1647-1648 ; 1649-1650; 1651-1652; 1653-1654; 1655-1656; 1657-1658; 1659-1660 ; 1661-1662 ; 1663-1664 ; 1665-1666 ; 1667-1668 ; 1669-1670 ; 1671-1672 ; 1673-1674; 1675-1676; 1677-1678; 1679-1680; 1681-1682; 1683-1684 ; 1685-1686; 1687-1688; 1689-1690; 1691-1692; 1693-1694; 1695-1696 ; 1697-1698; 1699-1700; 1701-1702; 1703-1704; 1705-1706; 1707-1708 ; 1709-1710; 1711-1712; 1713-1714; 1715-1716; 1717-1718; 1719-1720 ; 1721-1722; 1723-1724; 1725-1726; 1727-1728; 1729-1730; 1731-1732 ; 1733-1734; 1735-1736; 1737-1738; 1739-1740; 1741-1742; 1743-1744 ; 1745-1746; 1747-1748; 1749-1750; 1751-1752; 1753-1754; 1755-1756 ; 1757-1758; 1759-1760; 1761-1762; 1763-1764; 1765-1766; 1767-1768 ; 1769-1770; 1771-1772; 1773-1774; 1775-1776; 1777-1778; 1779-1780 ; 1781-1782; 1783-1784; 1785-1786; 1787-1788; 1789-1790; 1791-1792 ; 1793-1794; 1795-1796; 1797-1798; 1799-1800; 1801-1802; 1803-1804 ; 1805-1806; 1807-1808; 1809-1810; 1811-1812; 1813-1814; 1815-1816.

**6.** Procédé selon l'une quelconque des revendications 1 à 5, comprenant:

a) facultativement, le prétraitement des acides nucléiques pour éliminer de l'ADN génomique humain,
b) facultativement, la pré-amplification des ARNm viraux, dans lequel lesdits ARNm viraux comprennent des ARNm oncogènes et au moins un autre ARNm,
c) le séquençage des ARNm, ou des ADNc de ceux-ci, obtenus après les étapes a) et b), dans l'échantillon d'un sujet humain,
d) l'identification des séquences de fragments correspondant auxdits ARNm oncogènes,
e) l'identification d'à quel(le)s espèces ou génotypes appartiennent lesdits ARNm oncogènes de l'étape d),

f) le tri des séquences de fragments correspondant audit au moins un autre ARNm viral, ou aux ADNc de celui-ci obtenus après les étapes a) et b), du même génotype ou de la même espèce identifié(e) à l'étape e),
g) facultativement, l'identification des transcrits de fusion comme signature d'événements d'intégration d'ADN viral dans le chromosome hôte et/ou des biomarqueurs de cellules cancéreuses humaines supplémentaires,
h) facultativement, la délétion de toutes les autres séquences, y compris des séquences humaines qui ne sont pas des séquences identifiées et triées à la suite des étapes d), e), f) et g),
i) le calcul des rapports R définissant une abondance moléculaire desdits ARNm oncogènes par rapport audit au moins un autre ARNm viral du même génotype ou de la même espèce de l'étape f).

**7.** Procédé de diagnostic ou de pronostic du risque de développer un cancer induit par HPV comprenant:

(a) la détermination du niveau d'au moins un premier marqueur choisi parmi des ARNm E6 de HPV groupe alpha, de ARNm E7 de HPV groupe alpha, ou les deux, dans l'échantillon d'un patient ou dans l'échantillon d'un individu suspecté d'être infecté par le HPV, dans lequel ledit niveau dans les espèces de HPV est sélectif,
(b) la comparaison des niveaux déterminés à l'étape (a) à une valeur de référence d'ARNm E6 de HPV groupe alpha, d'ARNm E7 de HPV groupe alpha, ou des deux chez des individus à faible risque infectés par les HPV,

dans lequel un niveau accru tel que déterminé à l'étape a) par rapport au niveau de référence de l'étape b) est indicatif d'un risque plus élevé de développer un cancer induit par VPH,
dans lequel le procédé comprend une étape d'amplification avec une composition d'amorces spécifique à un HPV groupe alpha telle que définie dans l'une quelconque des revendications 1-2.

**8.** Procédé selon l'une des revendications 6 et 7, dans lequel les ARNm E6 ou E7 de HPV appartiennent aux HPV genre alpha, et dans lequel il comprend une amplification et/ou un séquençage couvrant des HPV d'au moins des groupes a5, 6, 7 et 10.

**9.** Procédé selon la revendication 8, dans lequel une amplification ou une détermination des niveaux de biomarqueurs E6 et L1 comprend une utilisation d'une composition d'amorces comprenant pour E6 : α5 : à la fois SEQ ID NO : 44 et SEQ ID NO : 45, et les trois SEQ ID NO : 46, SEQ ID NO : 47 et SEQ ID NO : 48 et ; α6 : SEQ ID NO : 58 ou à la fois SEQ ID NO : 59 et SEQ ID NO : 60, et à la fois SEQ ID NO : 61 et SEQ ID NO : 62 et ; α7 : les trois SEQ ID NO : 73, SEQ ID NO : 75 et SEQ ID NO : 76 ou les trois SEQ ID NO : 74, SEQ ID NO : 75 et SEQ ID NO : 76, et les cinq SEQ ID NO : 77, SEQ ID NO : 78, SEQ ID NO : 79, SEQ ID NO : 80 et SEQ ID NO : 81 et ; α10 : les trois SEQ ID NO : 115, SEQ ID NO : 116, SEQ ID NO : 117 et les quatre SEQ ID NO : 118, SEQ ID NO : 119, SEQ ID NO : 120, SEQ ID NO : 121 ;
et comprenant pour L1 α5 : SEQ ID NO : 53 ou à la fois SEQ ID NO : 54 et SEQ ID NO : 55, et à la fois SEQ ID NO : 56 et SEQ ID NO : 57 ; et α6 : SEQ ID NO : 71 et SEQ ID NO : 72 ; et α7: à la fois SEQ ID NO : 87 et SEQ ID NO : 88, et SEQ ID NO : 89 et à la fois SEQ ID NO : 90 ; et α10 : à la fois SEQ ID NO : 128, SEQ ID NO : 129 et SEQ ID NO : 130 ou les quatre SEQ ID NO : 131, SEQ ID NO : 132, SEQ ID NO : 133, SEQ ID NO : 134.

**10.** Procédé selon la revendication 8, dans lequel une amplification ou une détermination des niveaux de biomarqueurs E7 et L1 comprend une utilisation d'une composition d'amorces comprenant pour E7 : α5 : à la fois SEQ ID NO : 49, SEQ ID NO : 50 et à la fois SEQ ID NO : 51, SEQ ID NO : 52 ; et α6 : SEQ ID NO : 63 ou SEQ ID NO : 64 ou SEQ ID NO : 65 SEQ ID NO : 66 ou à la fois SEQ ID NO : 67 et SEQ ID NO : 68, et à la fois SEQ ID NO : 69 et SEQ ID NO : 70; et α7 : SEQ ID NO : 82 ou à la fois SEQ ID NO : 83, SEQ ID NO : 84 et à la fois SEQ ID NO : 85, SEQ ID NO : 86 ; et a10 : les trois SEQ ID NO : 122, SEQ ID NO : 123 et SEQ ID NO : 124, et les trois SEQ ID NO: 125, SEQ ID NO: 126 et SEQ ID NO: 127 ; et α9 : les trois SEQ ID NO : 105, SEQ ID NO : 106 et SEQ ID NO : 107, et les trois SEQ ID NO : 108, SEQ ID NO : 109 et 110 ; et α8 : SEQ ID NO : 94, et à la fois SEQ ID NO : 95 et SEQ ID NO : 96 ; et α1 : SEQ ID NO : 3 et SEQ ID NO : 4 ; et α3 : à la fois SEQ ID NO : 28 et SEQ ID NO : 29, et les trois SEQ ID NO : 30, SEQ ID NO : 31, SEQ ID NO : 32 ; et α2 : les trois SEQ ID NO : 12, SEQ ID NO : 13 et SEQ ID NO : 14, et les trois SEQ ID NO : 15, SEQ ID NO : 16 et SEQ ID NO : 17 ; et α4 : SEQ ID NO : 38, et SEQ ID NO : 39 ; et toutes les SEQ ID NO : 137 et SEQ ID NO: 138 ; et α13 : SEQ ID NO: 143, et SEQ ID NO : 144 ; et 14 : SEQ ID NO : 149, et SEQ ID NO : 150 ;
et comprenant pour L1 α5 : SEQ ID NO : 53 ou à la fois SEQ ID NO : 54 et SEQ ID NO : 55, et à la fois SEQ ID NO : 56 et SEQ ID NO : 57 ; et α6 : SEQ ID NO : 71 et SEQ ID NO : 72 ; et α7 : à la fois SEQ ID NO : 87 et SEQ ID NO : 88, et à la fois SEQ ID NO : 89 et SEQ ID NO: 90; et α10 : à la fois SEQ ID NO: 128, SEQ ID NO: 129 et SEQ ID NO : 130 ou les quatre SEQ ID NO : 131, SEQ ID NO : 132, SEQ ID NO : 133, SEQ ID NO : 134 ; et α9 : à la fois SEQ ID NO : 111 et SEQ ID NO : 112, et à la fois SEQ ID NO : 113 et SEQ ID NO : 114 ; et α8 : SEQ ID NO : 97, et à la fois SEQ ID NO : 98 et SEQ ID NO : 99 ; et α1 : SEQ ID NO : 5 et SEQ ID NO : 6 ; et α3 SEQ ID NO : 33 et à la fois SEQ ID NO: 34 et SEQ ID NO : 35 ; et a2 SEQ ID NO : 18, et à la fois SEQ ID NO : 19 et SEQ ID NO : 20 ; et α4 : SEQ ID NO : 40 ou SEQ ID NO : 41, et SEQ ID NO : 42 ou SEQ ID NO : 43 ; et α11 : SEQ ID NO : 139, et SEQ ID NO: 140 ; et a13 SEQ ID NO : 145, et SEQ ID NO : 146 ; et α14 : SEQ ID NO : 151 et SEQ ID NO : 152.

**11.** Procédé selon la revendication 8, dans lequel une amplification ou une détermination des niveaux de biomarqueurs E6 et L1 comprend une utilisation d'une composition d'amorces comprenant pour E6 : α5 : à la fois SEQ ID NO : 44 et SEQ ID NO : 45, et les trois SEQ ID NO : 46, SEQ ID NO : 47 et SEQ ID NO : 48 et ; α6 : SEQ ID NO : 58 ou à la fois SEQ ID NO : 59 et SEQ ID NO : 60, et à la fois SEQ ID NO : 61 et SEQ ID NO : 62 et ; α7 : les trois SEQ ID NO : 73, SEQ ID NO : 75 et SEQ ID NO : 76 ou les trois SEQ ID NO : 74, SEQ ID NO : 75 et SEQ ID NO : 76, et les cinq SEQ ID NO : 77, SEQ ID NO : 78, SEQ ID NO : 79, SEQ ID NO : 80 et SEQ ID NO : 81 et ; α10 : les trois SEQ ID NO : 115, SEQ ID NO : 116, SEQ ID NO : 117 et les quatre SEQ ID NO : 118, SEQ ID NO : 119, SEQ ID NO : 120, SEQ ID NO : 121,
et comprenant pour L1 α5 : SEQ ID NO : 53 ou à la fois SEQ ID NO : 54 et SEQ ID NO : 55, et à la fois SEQ ID NO : 56 et SEQ ID NO : 57 ; et α6 : SEQ ID NO : 71 et SEQ ID NO : 72 ; et α7: à la fois SEQ ID NO : 87 et SEQ ID NO : 88, et SEQ ID NO : 89 et SEQ ID NO : 90 ; et α10 : à la fois SEQ ID NO : 128, SEQ ID NO : 129 et SEQ ID NO : 130 ou les quatre SEQ ID NO : 131, SEQ ID NO : 132, SEQ ID NO : 133, SEQ ID NO : 134.

**12.** Procédé selon l'une des revendications 8 à 11, dans lequel une amplification ou une détermination des niveaux de biomarqueurs E6 et L1 comprend une utilisation d'une composition d'amorces comprenant pour E6 : α5 : à la fois SEQ ID NO : 44 et SEQ ID NO : 45, et les trois SEQ ID NO : 46, SEQ ID NO : 47 et SEQ ID NO : 48 et; α6 : SEQ ID NO : 58 ou à la fois SEQ ID NO : 59 et SEQ ID NO : 60, et à la fois SEQ ID NO : 61 et SEQ ID NO : 62 et; α7 : les trois SEQ ID NO : 73, SEQ ID NO : 75 et SEQ ID NO : 76 ou les trois SEQ ID NO : 74, SEQ ID NO : 75 et SEQ ID NO : 76, et les cinq SEQ ID NO : 77, SEQ ID NO : 78, SEQ ID NO : 79, SEQ ID NO: 80 et SEQ ID NO : 81 et; α10 : les trois SEQ ID NO: 115, SEQ ID NO : 116, SEQ ID NO : 117 et les quatre SEQ ID NO : 118, SEQ ID NO : 119, SEQ ID NO: 120, SEQ ID NO: 121 ; et α9 : à la fois SEQ ID NO : 100 et SEQ ID NO : 101 et les trois SEQ ID NO : 102, SEQ ID NO : 103 et SEQ ID NO : 104 ; et α8 : SEQ ID NO : 91, et à la fois SEQ ID NO : 92 et SEQ ID NO : 93 ; et α1 : SEQ ID NO : 1 et SEQ ID NO : 2 ; et α3 : les trois SEQ ID NO : 21, SEQ ID NO : 22 et SEQ ID NO : 23, et les quatre SEQ ID NO : 24, SEQ ID NO : 25, SEQ ID NO : 26 et SEQ ID NO : 27 ; et α2 et à la fois SEQ ID NO : 7 et SEQ ID NO : 8, et les trois SEQ ID NO : 9, SEQ ID α11 : SEQ ID NO : 135, et SEQ ID NO : 136 ; et α13 : SEQ ID NO : 141, et SEQ ID NO : 142 ; et α14 : SEQ ID NO : 147, et SEQ ID NO : 148,
et comprenant pour L1, α5 : SEQ ID NO : 53 ou à la fois SEQ ID NO : 54 et SEQ ID NO : 55, et à la fois SEQ ID NO : 56 et SEQ ID NO : 57 ; et α6 : SEQ ID NO : 71 et SEQ ID NO : 72 ; et α7 : à la fois SEQ ID NO : 87 et SEQ ID NO : 88, et à la fois SEQ ID NO : 89 et SEQ ID NO: 90; et α10 : à la fois SEQ ID NO: 128, SEQ ID NO: 129 et SEQ ID NO : 130 ou les quatre SEQ ID NO : 131, SEQ ID NO : 132, SEQ ID NO : 133, SEQ ID NO : 134 ; et α9 : à la fois SEQ ID NO : 111 et SEQ ID NO : 112, et à la fois SEQ ID NO : 113 et SEQ ID NO : 114 ; et α8 : SEQ ID NO : 97, et à la fois SEQ ID NO : 98 et SEQ ID NO : 99 ; et α1 : SEQ ID NO : 5 et SEQ ID NO : 6 ; et a3 SEQ ID NO : 33 et à la fois SEQ ID NO : 34 et SEQ ID NO : 35, et a2 SEO ID NO : 18, et à la fois SEQ ID NO : 19 et SEQ ID NO : 20 ; et α4 : SEQ ID NO : 40 ou SEQ ID NO : 41, et SEQ ID NO : 42 ou SEQ ID NO : 43 ; et α11 : SEQ ID NO : 139, et SEQ ID NO: 140 ; et a13 SEQ ID NO : 145, et SEQ ID NO : 146 ; et α14 : SEQ ID NO : 151 et SEQ ID NO : 152.

**13.** Composition d'amorces telle que définies dans l'une quelconque des revendications 1, 2, 4, 5 et 9 à 12.

**14.** Kit de diagnostic ou de pronostic du risque de développer un cancer induit par HPV comprenant :

a) une composition d'amorces selon la revendication 13,
b) des réactifs pour détecter ou séquencer des produits d'amplification.

**15.** Kit de diagnostic ou de pronostic du risque de développer un cancer induit par le HPV comprenant :

a) une composition d'amorces pour détecter au moins un premier marqueur choisi parmi les ARNm E6 de HPV groupe alpha, les ARNm E7 de HPV groupe alpha, ou les deux,
b) une composition d'amorces pour détecter au moins un second marqueur choisi parmi les ARNm L1 de HPV de groupe alpha, les ARNm L2 de HPV groupe alpha, ou les deux,
dans lequel lesdits ARNm E6, E7, L1 et L2 ont des séquences intragénétiques correspondantes,
c) et facultativement, des amorces ou sondes pour détecter au moins un marqueur cellulaire hôte indicatif d'une néoplasie ou d'un cancer,

dans lequel la composition d'amorces est telle que définie dans la revendication 1 ou 2.

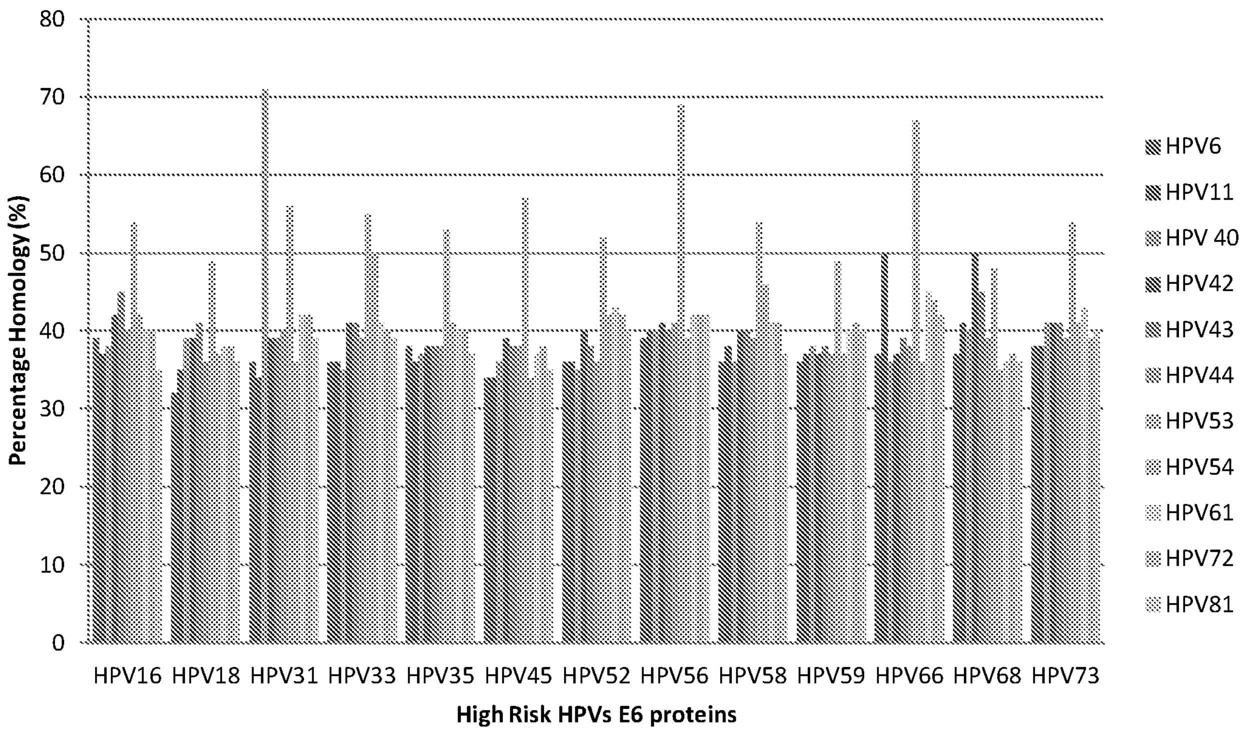
80
70
60
50
40
30
20
10
0
Percentage Homology (%)
HPV16
HPV18
HPV31
HPV33
HPV35
HPV45
HPV52
HPV56
HPV58
HPV59
HPV66
HPV68
HPV73
High Risk HPVs E6 proteins
HPV6
HPV11
HPV 40
HPV42
HPV43
HPV44
HPV53
HPV54
HPV61
HPV72
HPV81

FIGURE 1

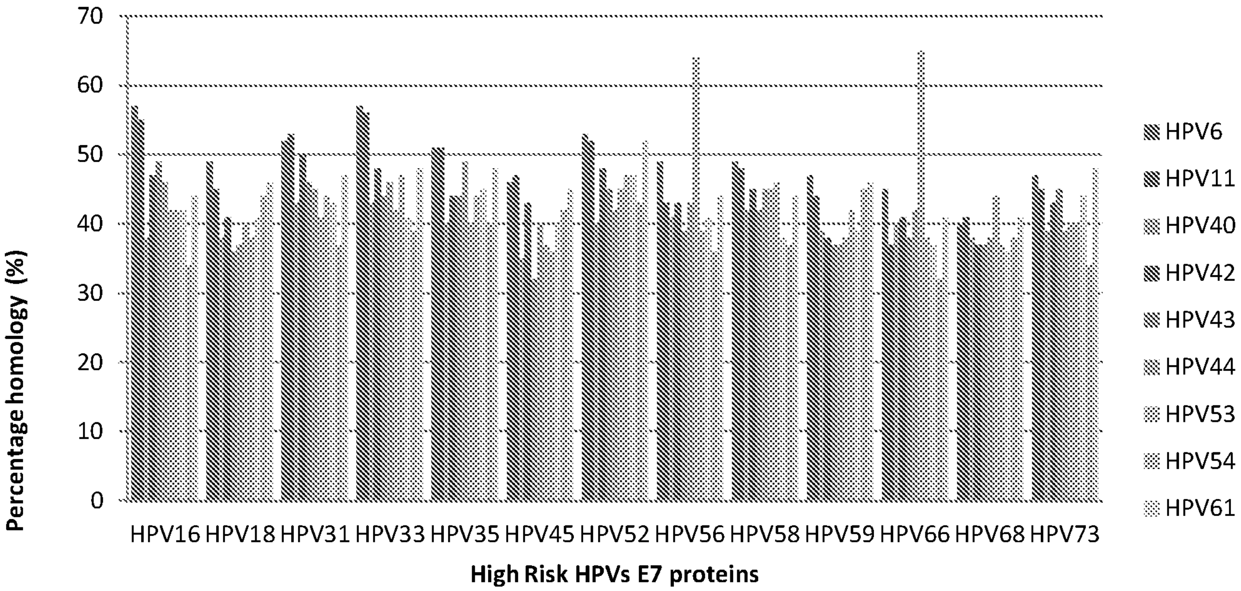
70
60
50
40
30
20
10
0
Percentage homology (%)
HPV16 HPV18 HPV31 HPV33 HPV35 HPV45 HPV52 HPV56 HPV58 HPV59 HPV66 HPV68 HPV73
High Risk HPVs E7 proteins
HPV6
HPV11
HPV40
HPV42
HPV43
HPV44
HPV53
HPV54
HPV61

FIGURE 2

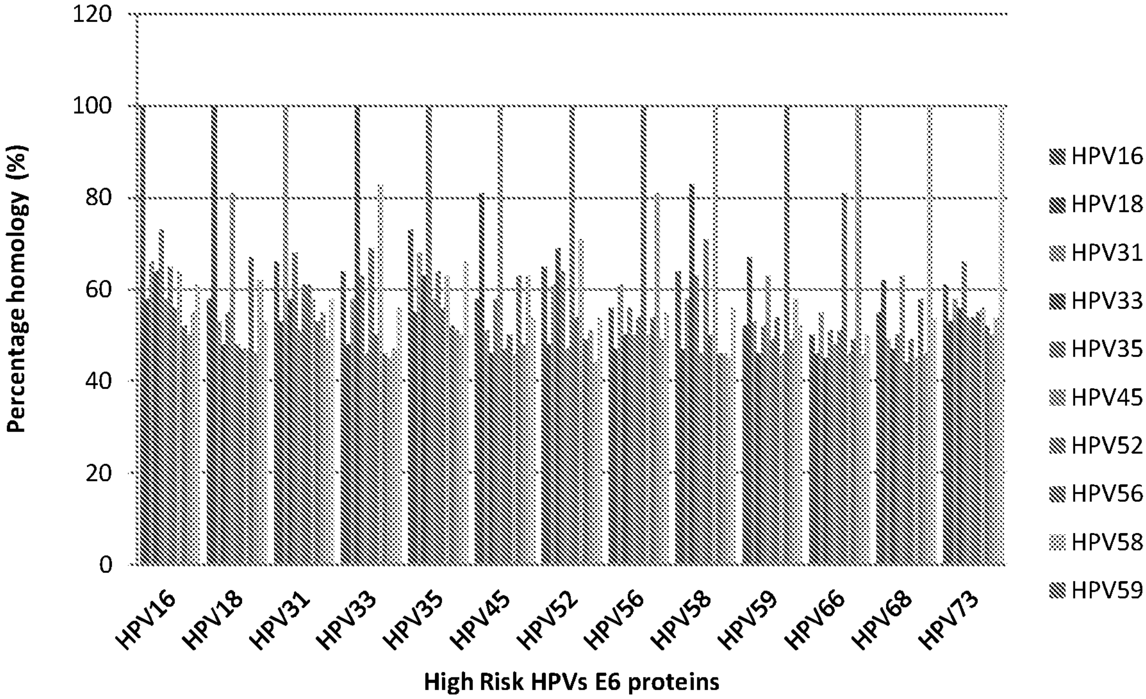
120
100
80
60
40
20
0
Percentage homology (%)
HPV16
HPV18
HPV31
HPV33
HPV35
HPV45
HPV52
HPV56
HPV58
HPV59
HPV66
HPV68
HPV73
High Risk HPVs E6 proteins
HPV16
HPV18
HPV31
HPV33
HPV35
HPV45
HPV52
HPV56
HPV58
HPV59

FIGURE 3

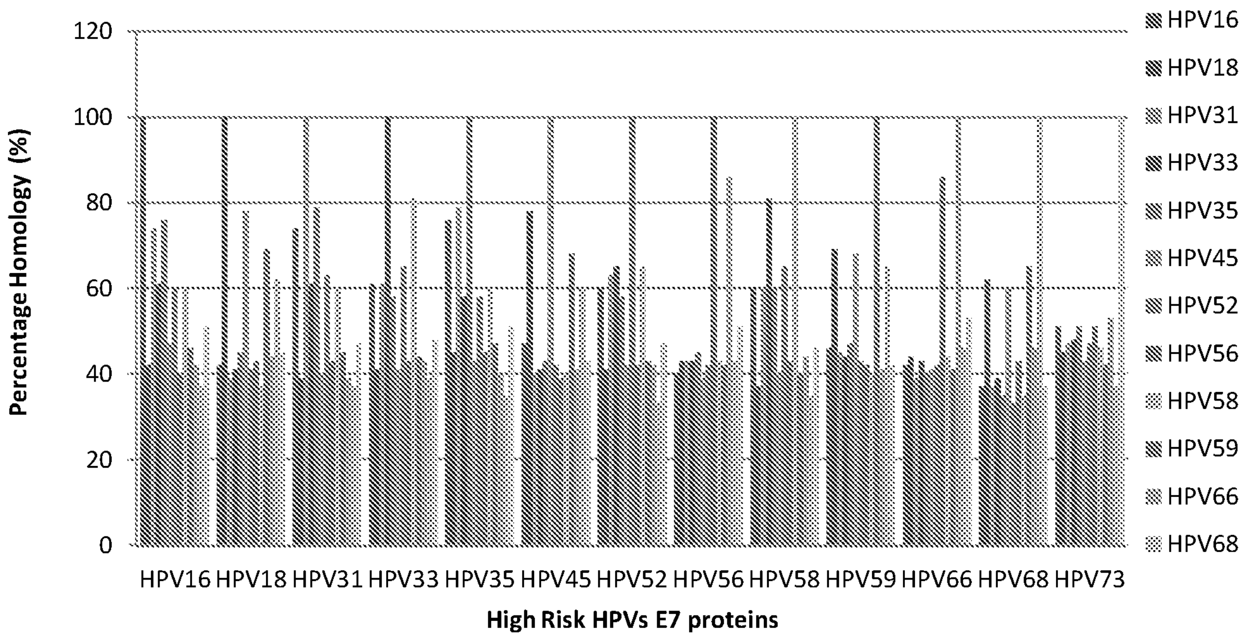

Percentage Homology (%)
120
100
80
60
40
20
0
HPV16
HPV18
HPV31
HPV33
HPV35
HPV45
HPV52
HPV56
HPV58
HPV59
HPV66
HPV68
HPV73
High Risk HPVs E7 proteins
HPV16
HPV18
HPV31
HPV33
HPV35
HPV45
HPV52
HPV56
HPV58
HPV59
HPV66
HPV68


FIGURE 4

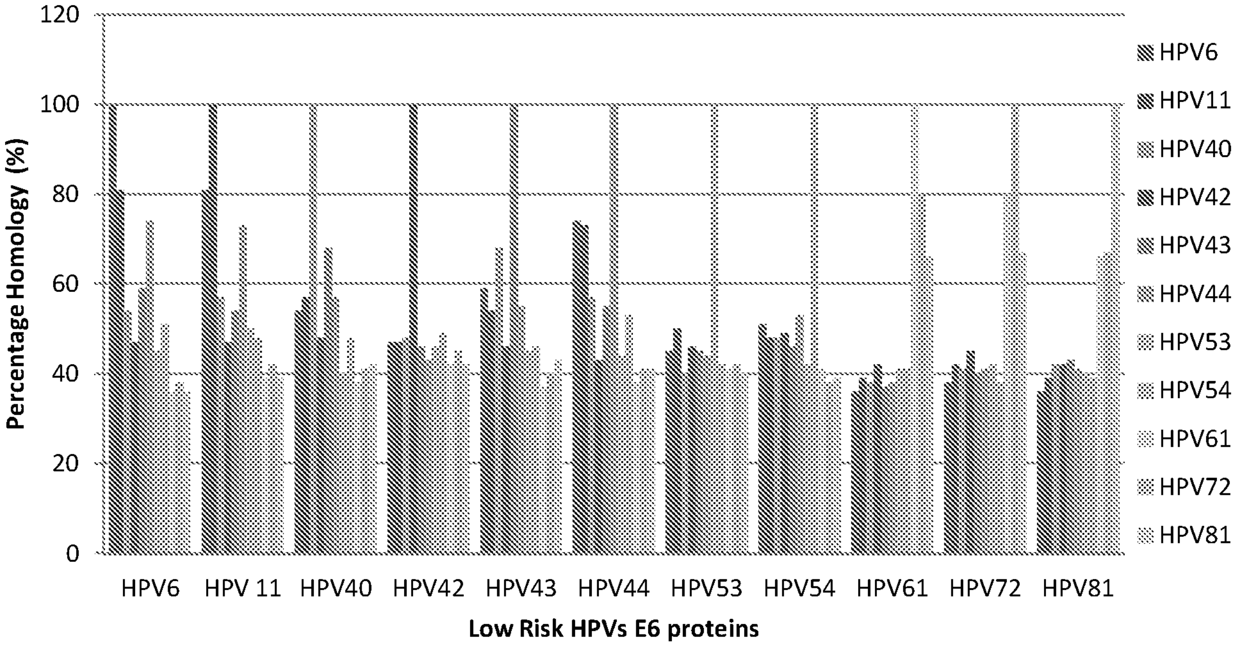
120
100
80
60
40
20
0
Percentage Homology (%)
HPV6
HPV 11
HPV40
HPV42
HPV43
HPV44
HPV53
HPV54
HPV61
HPV72
HPV81
Low Risk HPVs E6 proteins
HPV6
HPV11
HPV40
HPV42
HPV43
HPV44
HPV53
HPV54
HPV61
HPV72
HPV81

FIGURE 5

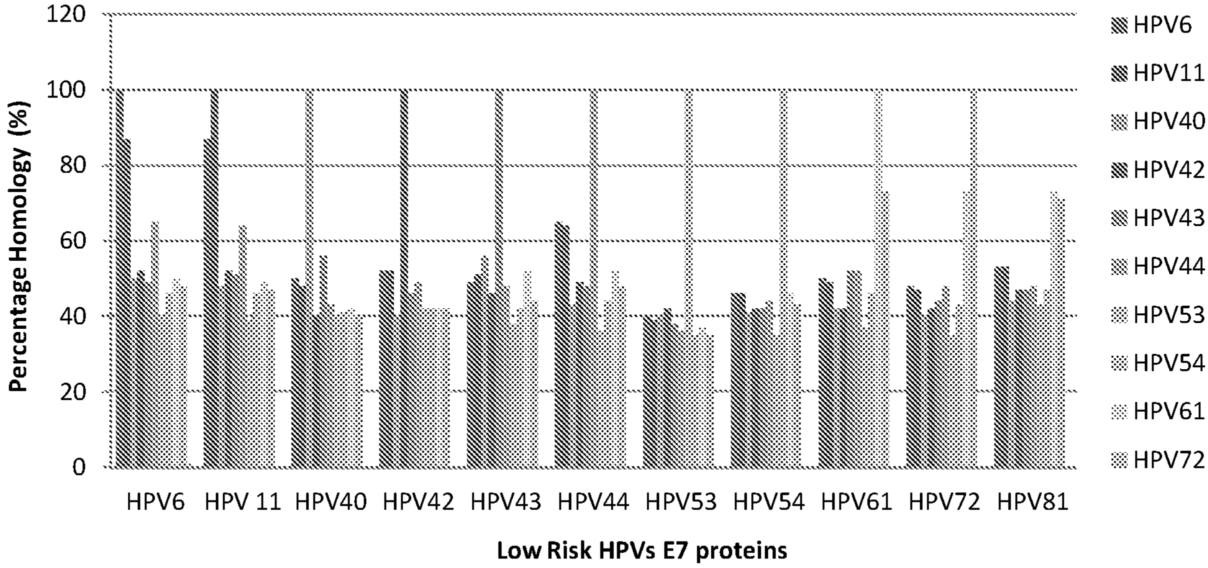
120
100
80
60
40
20
0
Percentage Homology (%)
HPV6
HPV 11
HPV40
HPV42
HPV43
HPV44
HPV53
HPV54
HPV61
HPV72
HPV81
Low Risk HPVs E7 proteins
HPV6
HPV11
HPV40
HPV42
HPV43
HPV44
HPV53
HPV54
HPV61
HPV72

FIGURE 6

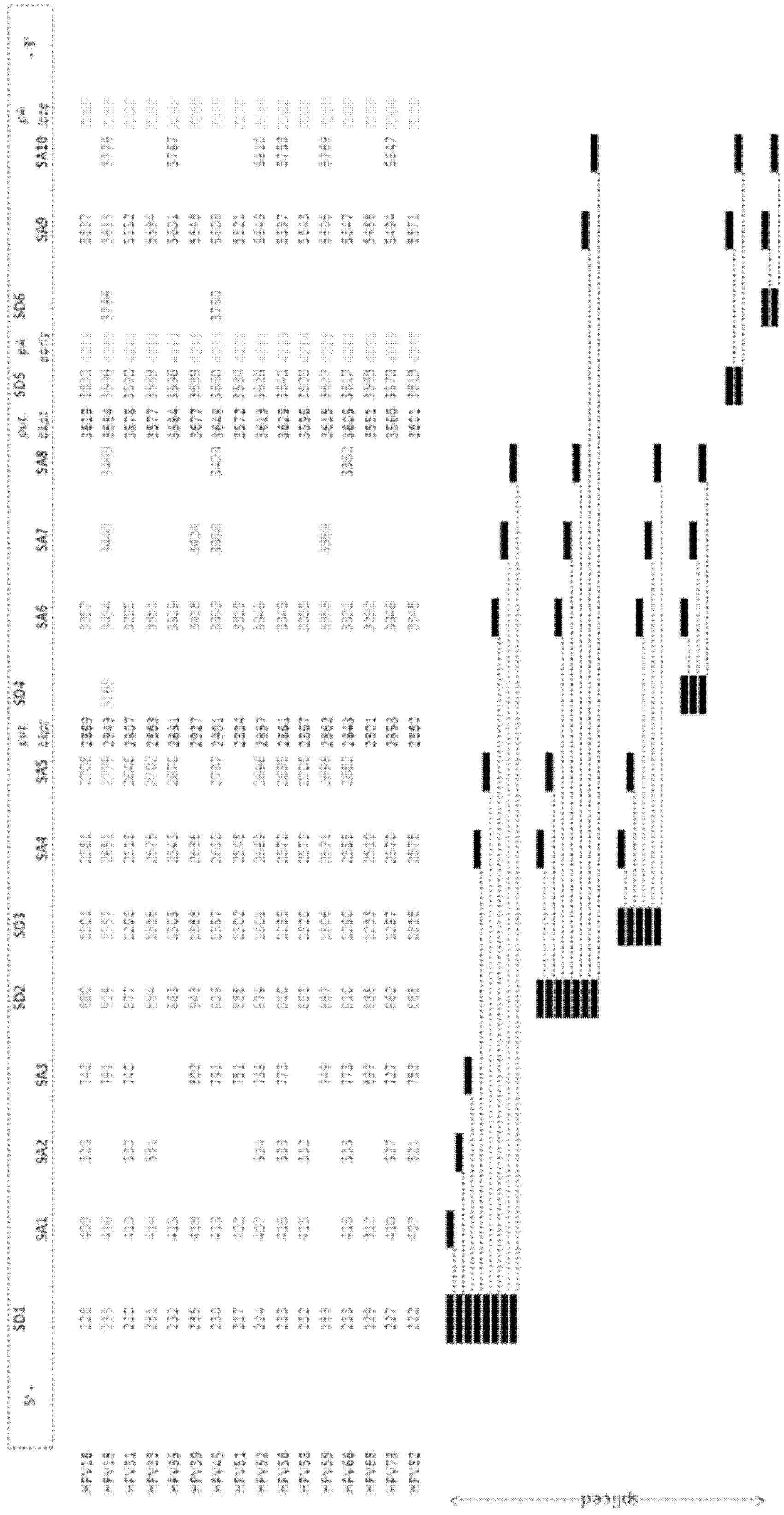
5'
SD1
SA1
SA2
SA3
SD2
SD3
SA4
SA5
put. bkpt
SD4
SA6
SA7
SA8
put. bkpt
SD5
pA early
SD6
SA9
SA10
pA late
3'
HPV16
HPV18
HPV31
HPV33
HPV35
HPV39
HPV45
HPV51
HPV52
HPV56
HPV58
HPV59
HPV66
HPV68
HPV73
HPV82
spliced

FIGURE 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011088573 A
- US 2006222656 A
- WO 2010129941 A


### Non-patent literature cited in the description


- **J. SAMBROOK ; D.W. RUSSELL.** Molecular Cloning: A Laboratory Manual. Cold Spring Harbor Laboratory Press, 2001
- Short Protocols in Molecular Biology, Current Protocols. 2002
- **ZHENG et al.** *FrontBiosci,* 2006
- **BURK et al.** *Virology,* 2013
- **ZHENG et al.** *Front Biosci,* 2006
- **WANG et al.** *Journal of Virology,* 2011
- **TOOTS et al.** *PLoS ONE,* 2014
- **LU et al.** *PLoS ONE,* 2014
- **TANG et al.** *Nature Communication,* 2013
- **WENTZENSEN et al.** *Oncogene,* 2002
- **PETER et al.** *Oncogene,* 2006
- **LANGMEAD et al.** *Nature Methods,* 2012